# EUROPEAN PATENT APPLICATION

(11) **EP 4 512 426 A1**
(43) Date of publication of application: **26.02.2025**
(21) Application number: 23791308.2
(22) Date of filing: 20.04.2023
(51) Int. Cl.: A61K 47/54, A61K 47/64, A61K 47/65, A61P 35/00, A61P 37/02

(54) **COMPOUND AND USE THEREOF**

(30) Priority: 20.04.2022 WO PCT/CN2022/088021; 02.12.2022 WO PCT/CN2022/136395; 06.04.2023 CN 202310361865
(71) Applicant: Coherent Biopharma (Suzhou), Limited, Industrial Park Suzhou Jiangsu 215123 (CN)
(72) Inventor: ZHANG, Zhiguang, Suzhou, Jiangsu 215123 (CN); WANG, Guitao, Suzhou, Jiangsu 215123 (CN); SHA, Fei, Suzhou, Jiangsu 215123 (CN); YAN, Liuliu, Suzhou, Jiangsu 215123 (CN); WEI, Zhigang, Suzhou, Jiangsu 215123 (CN); QIAN, Gang, Suzhou, Jiangsu 215123 (CN); JIANG, Shanjun, Suzhou, Jiangsu 215123 (CN); HUANG, Baohua Robert, Suzhou, Jiangsu 215123 (CN); CHEN, Lianyong, Suzhou, Jiangsu 215123 (CN)
(74) Representative: Maiwald GmbH
(86) International application number: PCT/CN2023/089422
(87) International publication number: WO 2023/202654

(57) **Abstract**

A conjugate compound capable of targeting and binding to cells and degrading abnormal or unwanted proteins, use thereof, and a linker compound useful for the preparation of said conjugate compound.

## Description

### TECHNICAL FIELD

The present application relates to the field of biomedical chemistry. Specifically, the present application relates to a conjugate compound capable of specifically binding to a cell and degrading a target protein and use thereof, and a linker compound for preparing the conjugate compound.

### BACKGROUND

The proteolysis targeting chimera (PROTAC) was proposed and verified by Raymond Deshaies and Craig Crews in 2001, and is a novel drug development technique for degrading a protein of interest (POI) using the ubiquitin-proteasome system. It is an improved and universal platform technology for induced protein degradation techniques. The chimeric bifunctional small molecules generated by the platform contain a target protein ligand for binding to the target protein at one end and an E3 ligase ligand for binding to E3 ligase at the other end. The two ligands are linked via a linking moiety. After entering the cell, the chimera can bring the target protein and E3 ligase closer and form a ternary complex, target protein-PROTAC-E3 ligase. Meanwhile, ubiquitin approaches E3 ligase due to the action of ubiquitin-conjugating enzyme E2 and is further attached to the target protein depending on E3 ligase (ubiquitination of the target protein). The ubiquitinated target protein will be recognized and degraded by proteasome, and thus the level of the target protein is selectively reduced. In such a process, the target protein ligand in the PROTAC requires no long-term occupation of the binding site but the transient formation of the ternary complex to instantly accomplish the ubiquitination of the target protein, and the PROTAC can be recycled in cells. Theoretically, a catalytic amount of a PROTAC drug can clear any overexpressed and mutated pathogenic protein, thereby treating the disease.

The PROTAC technology has various advantages: 1) PROTACs can overcome tumor drug resistance: the resistance against cancer therapies such as chemotherapeutics, kinase inhibitors, immunotherapeutics, and the like hinders their clinical use and increases the difficulties of drug development, while PROTAC at a lower dose can degrade the whole target protein and delete all functions of the target protein; the PROTAC technology is prospective in addressing the drug resistance challenges at present; 2) PROTACs are also prospective in degrading undruggable targets: currently, only 20-25% of protein targets are undergoing drug exploration, and most small molecule drugs or macromolecular antibodies require binding to active sites of enzymes or receptors for action; however, 80% of proteins in human cells lack such sites, while PROTACs require no such sites but binding activity to trigger the approach target proteins to E3 enzyme, thereby mediating the ubiquitination of the target protein and causing the degradation of the target protein.

In 2013, Arvinas advanced the first-in-class PROTAC drug (ARV-110) to the clinical study stage in 2019. Pharmaceutical enterprises throughout the world strive to utilize the PROTAC technology to develop targeted proteolysis medicines. By 2021, 16 PROTAC drugs have been in or are about to enter the clinical study stage. However, PROTACs also have limitations. For example, PROTACs possess poor druggability. The molecular weight of PROTACs is typically between 700 and 1200, and such a relatively large size often compromises their membrane permeability, physicochemical properties (e.g., low water solubility), and pharmacokinetics, which may complicate their *in vivo* applications. In addition, PROTACs feature poor targeting properties. Although the high efficiency of PROTACs to various targets has been demonstrated, the presence of various reported degradants so far suggests the limited selectivity to tissues and cells of different types.

By conjugating PROTACs to targeted ligands with targeting properties, the present invention combines the advantages of ligand-drug conjugates and the PROTAC technology, and improves the targeting property and the druggability of PROTACs while overcoming the resistance to antitumor drugs and the undruggability of conventional targets via PROTAC drugs and utilizing the low toxicity of PROTAC drugs.

### SUMMARY

In one aspect, the present application provides a conjugate compound or a pharmaceutically acceptable salt thereof, comprising: a targeted molecule comprising at least two ligands specifically binding to a cell surface protein and a payload linked to the targeted molecule, wherein the payload is a proteolysis targeting chimera.

In some embodiments, the targeted molecule binds to the following cell surface proteins: FOLR1, TRPV6, PSMA, LHRH, EGFR, Her2, Trop2, Her3, Claudin18.2, c-Met, or any combination thereof. In some embodiments, the targeted molecule binds to the following cell surface proteins: FOLR1, TRPV6, PSMA, c-Met, or any combination thereof.

In some embodiments, the targeted molecule binds to the following cell surface protein combinations: FOLR1 and TRPV6; FOLR1 and PSMA; TRPV6 and PSMA; TRPV6 and c-Met; FOLR1 and c-Met; PSMA and c-Met; FOLR1, TRPV6, and PSMA; FOLR1, TRPV6, and c-Met; FOLR1, PSMA, and c-Met; TRPV6, PSMA, and c-Met; or FOLR1, PSMA, TRPV6, and c-Met. In some embodiments, the targeted molecule comprises two ligands respectively binding to FOLR1 and TRPV6, FOLR1 and PSMA, TRPV6 and PSMA, FOLR1 and c-Met, PSMA and c-Met, or TRPV6 and c-Met.

In some embodiments, the ligand binding to FOLR1 comprises folic acid or an analog thereof and pteroic acid; preferably the folic acid analog is selected from: 5-methyltetrahydrofolic acid, 5-formyltetrahydrofolic acid, 10-formyltetrahydrofolic acid, methotrexate, 5,10-formyltetrahydrofolic acid, 5,10-methenyltetrahydrofolic acid, aminopterin, and raltitrexed.

In some embodiments, the ligand binding to TRPV6 comprises the amino acid sequence set forth in SEQ ID NO: 1, the amino acid sequence set forth in SEQ ID NO: 2, or an amino acid sequence having at least 80%, 85%, 90%, 95%, 96%, 97%, 98%, or 99% sequence identity to SEQ ID NO: 1 or SEQ ID NO: 2.

In some embodiments, the ligand binding to PSMA comprises a peptide, an antibody, or a small molecule. In some embodiments, the ligand binding to PSMA is selected from the following structures: 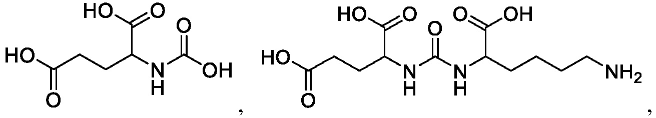 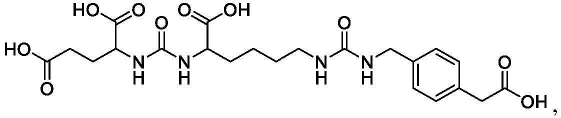 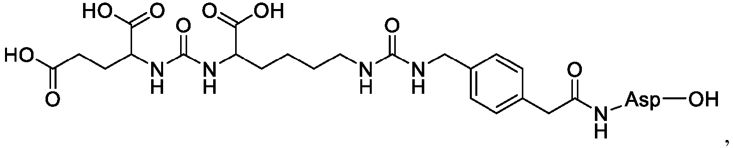 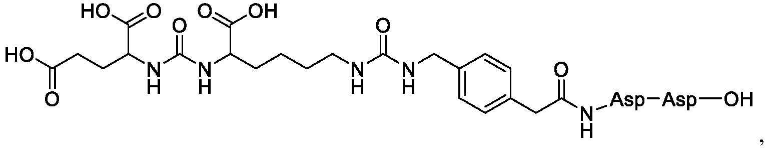 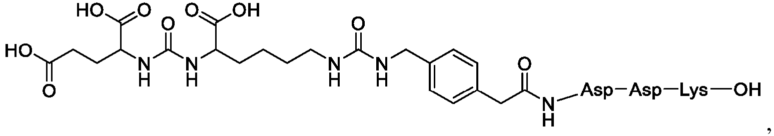 and

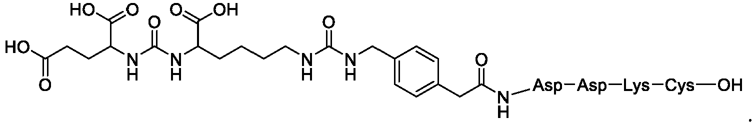

In some embodiments, the ligand binding to c-MET comprises the amino acid sequence set forth in SEQ ID NO: 3: Cys^{a}-X1-Cys^{c}-X2-Gly-Pro-Pro-X3-Phe-Glu-Cys^{d}-Trp-Cys^{b}-Tyr-X4-X5-X6; X1 is Asn, His, or Tyr; X2 is Gly, Ser, Thr, or Asn; X3 is Thr or Arg; X4 is Ala, Asp, Glu, Gly, or Ser; X5 is Ser or Thr; X6 is Asp or Glu; Cys^{a-d} are cysteine residues; preferably, residues Cys^{a} and Cys^{b}, as well as residues Cys^{c} and Cys^{d}, are separately cyclized to form two independent disulfide bonds. In some embodiments, the carboxyl of the terminal lysine of the c-MET ligand may be amidated. In some embodiments, the N-terminus of the c-MET ligand may be acetylated. In some embodiments, the ligand binding to c-MET comprises the amino acid sequence set forth in SEQ ID NO: 4: Ala-Gly-Ser-Cys^{a}-Tyr-Cys^{c}-Ser-Gly-Pro-Pro-Arg-Phe-Glu-Cys^{d}-Trp-Cys^{b}-Tyr-Glu-Thr-Glu-Gly-Thr-Gly-Gly-Gly-Lys; Cys^{a-d} are cysteine residues; preferably, residues Cys^{a} and Cys^{b}, as well as residues Cys^{c} and Cys^{d}, are separately cyclized to form two independent disulfide bonds; preferably, the amino of the terminal Ala is acetylated. In some embodiments, the ligand binding to c-MET comprises an amino acid sequence having at least 80%, 85%, 90%, 95%, 96%, 97%, 98%, or 99% sequence identity to SEQ ID NO: 4; Cys^{a-d} are cysteine residues; preferably, residues Cys^{a} and Cys^{b}, as well as residues Cys^{c} and Cys^{d}, are separately cyclized to form two independent disulfide bonds.

In some embodiments, the ligands in the targeted molecule are linked to each other directly or via a spacer region; preferably the linkage is by means of a dehydration reaction. In some embodiments, the spacer region comprises an amino acid or an amino acid combination selected from the following group: Lys, Cys, Lys-Cys, Cys-Cys, Lys-Cys-Lys, Arg-Arg, Ala-Ser-Asn, Ala-Ala-Ala, Ser-Ser-Arg, Pro-Arg, Asp-Asp-Lys-Cys, and Pro-Leu-Gly; optionally, the amino acid may be amidated. In some embodiments, Lys is amidated, e.g., to 2,6-diaminohexanamide. In some embodiments, the spacer region is formed by one or more identical or different compounds selected from the following group by means of a dehydration reaction: 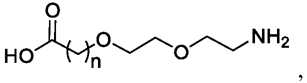 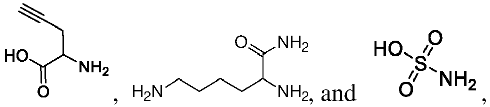 wherein n is 0 or 1.

In some embodiments, the targeted molecule having the following structural moiety is linked to the payload to form the conjugate compound: 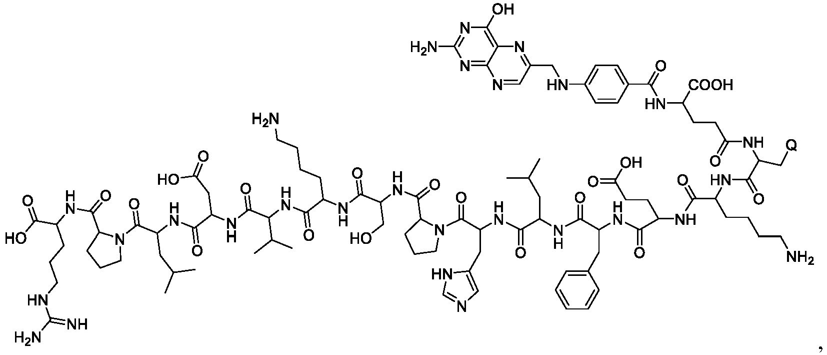 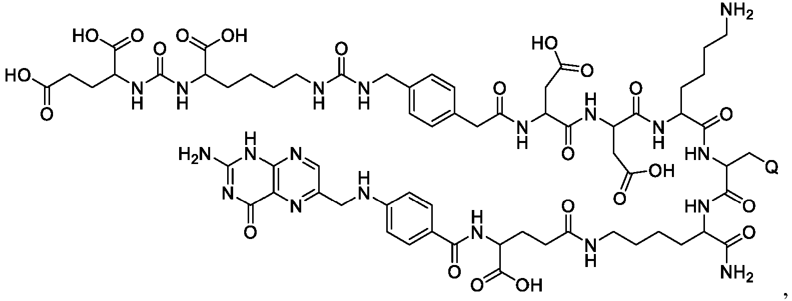 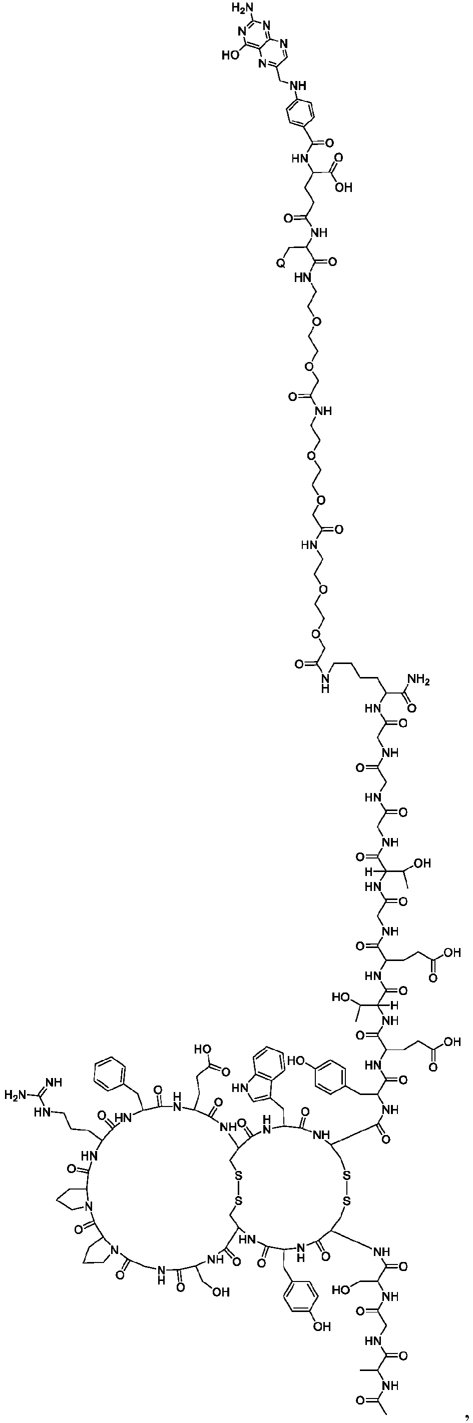 or 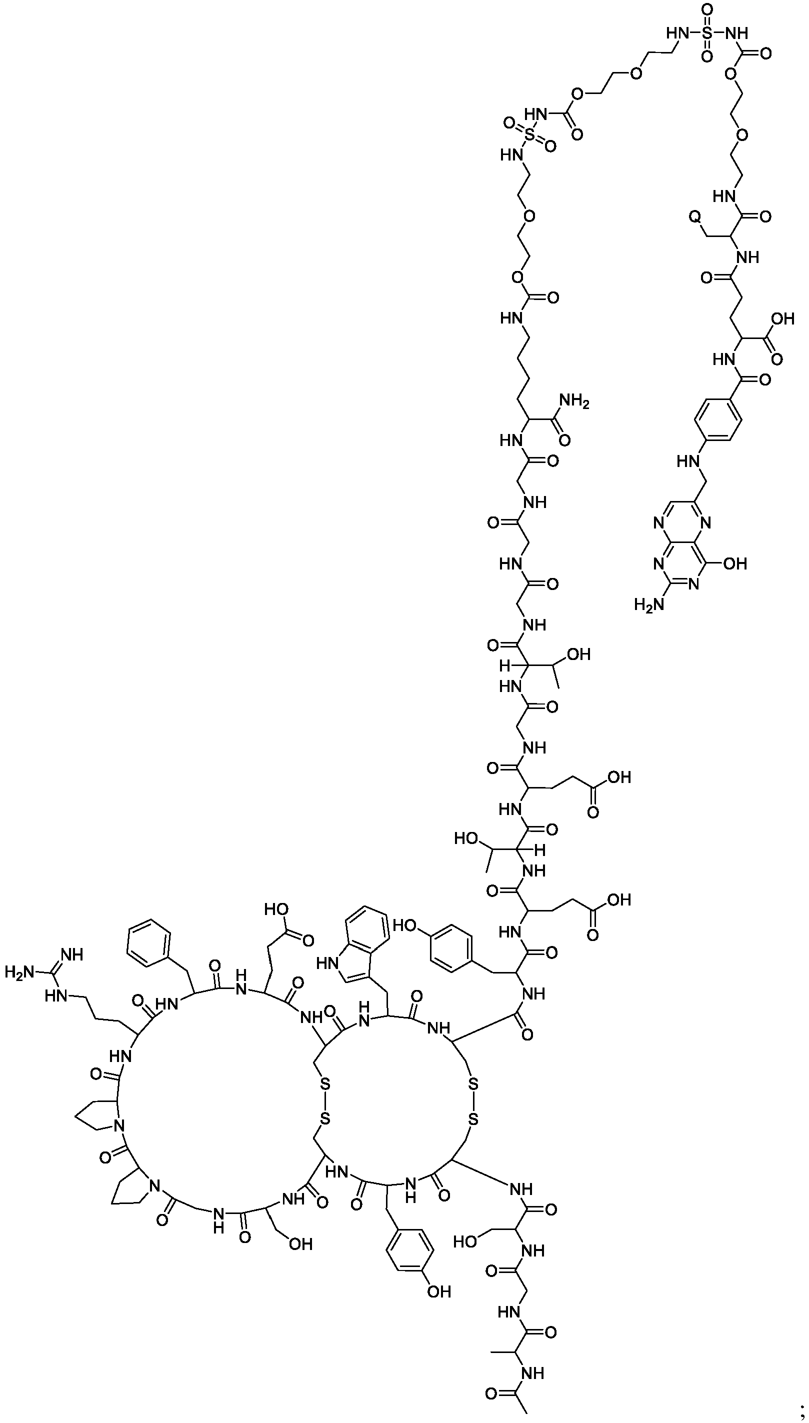 wherein, Q is an active group. In some embodiments, the aforementioned targeted molecule is linked to the payload through the active group Q; preferably, the active group Q is alkynyl or sulfhydryl.

In some embodiments, the targeted molecule having the following structures is linked to the payload to form the conjugate compound: 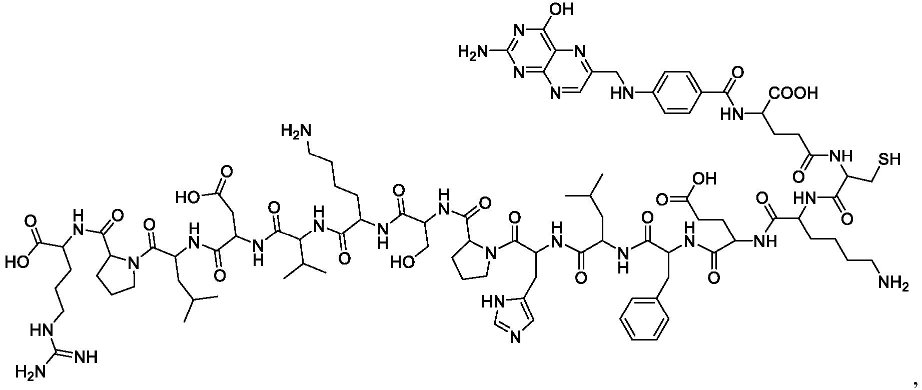 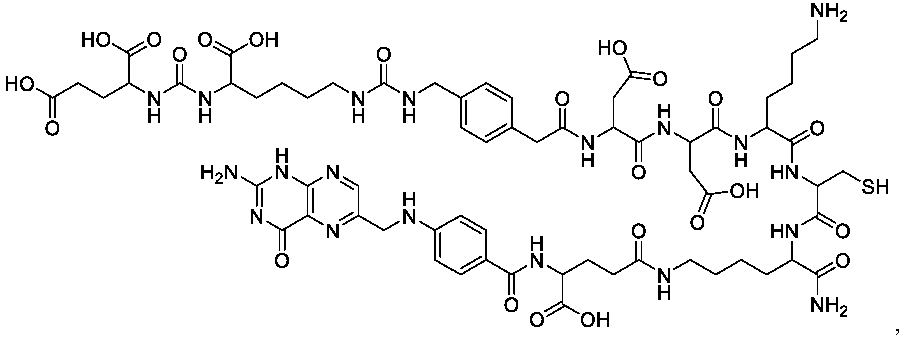 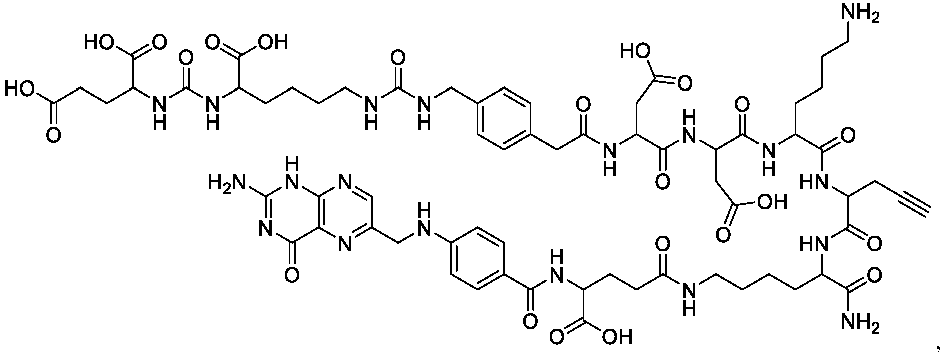 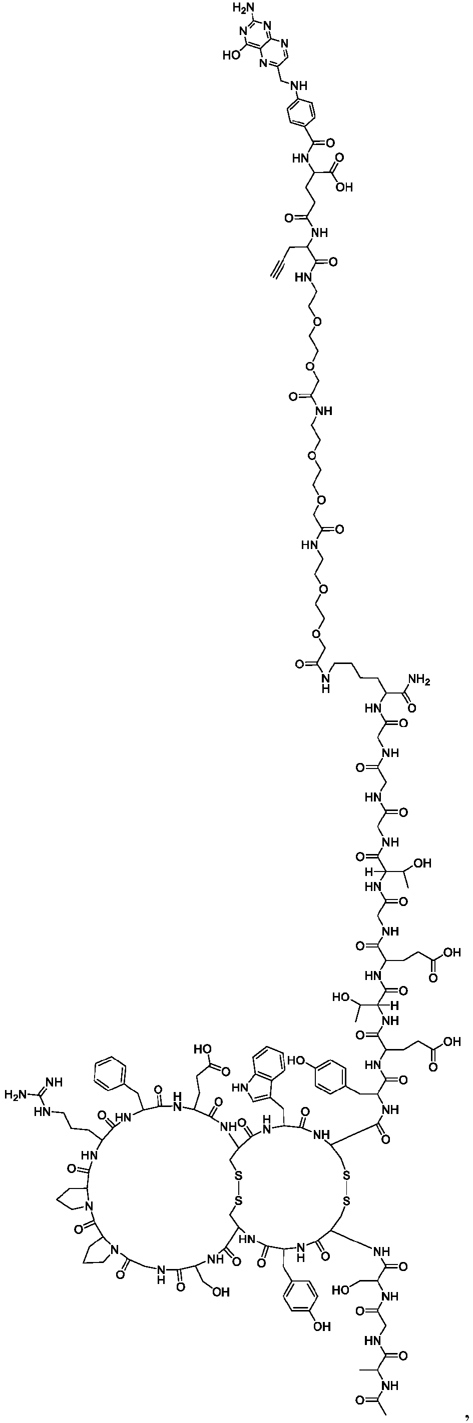 or

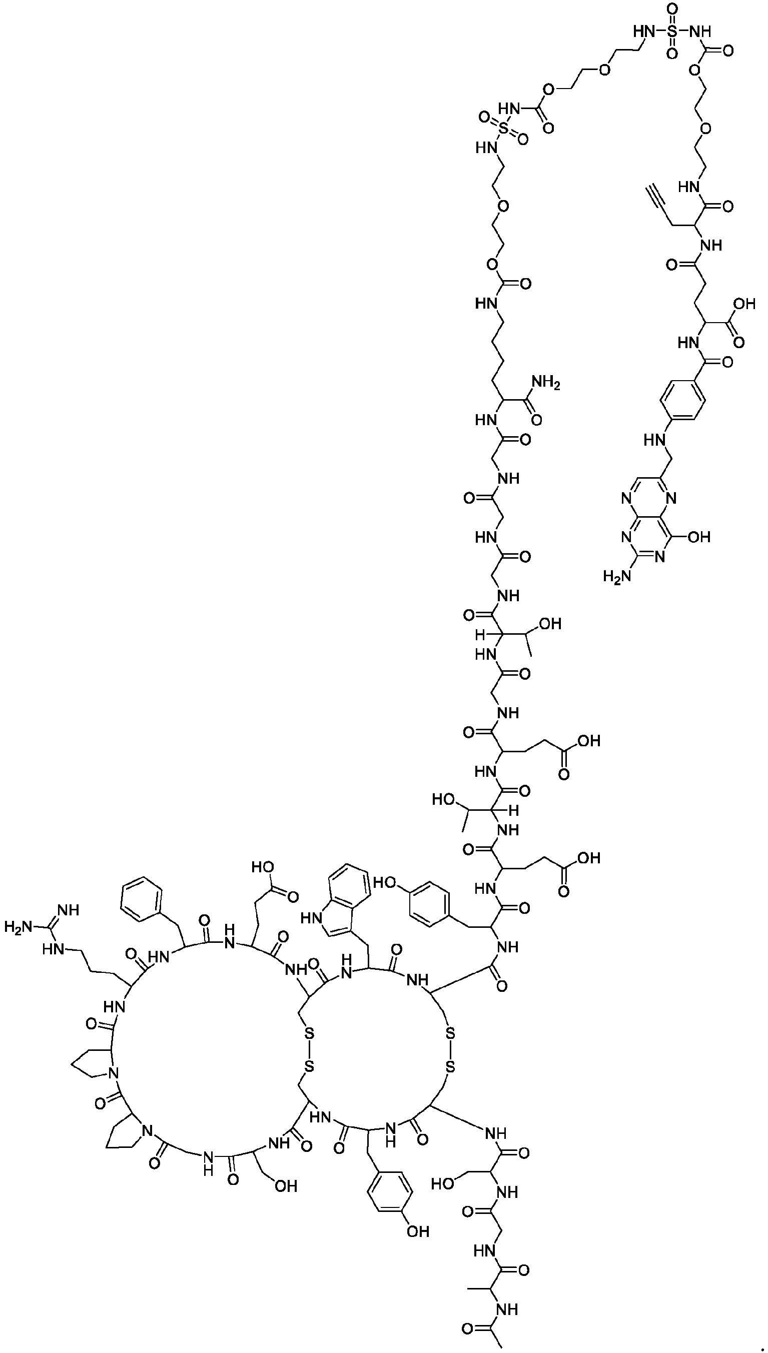

In some embodiments, the proteolysis targeting chimera having the structure represented by formula (I) is linked to the targeted molecule to form the conjugate compound:

(T_{P})ₙ-L-T_{E3} (I),

wherein,
T_{P} is a target protein ligand moiety for binding to a target protein, wherein n is 1, 2, or 3;
L is a linking moiety for linking T_{P} to T_{E3};
T_{E3} is an E3 ligase ligand moiety for binding to E3 ligase.

In some embodiments, the E3 ligase ligand binds to VHL (Von Hippel-Lindau) protein, CRBN (cereblon) protein, MDM2 protein, or XIAP protein; preferably, the E3 ligase ligand is a VHL ligand or a CRBN ligand.

In some embodiments, the VHL ligand is VHL-L or an analog thereof, wherein VHL-L has a structure selected from the following group: 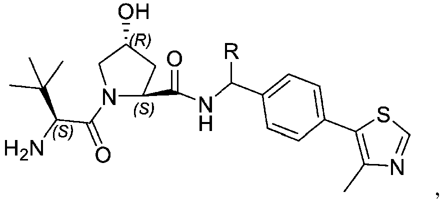 wherein R is hydrogen or methyl; and

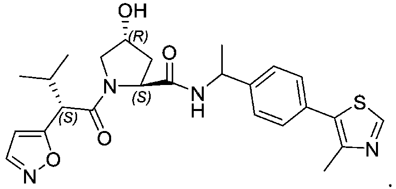

In some embodiments, the CRBN ligand is lenalidomide, pomalidomide, thalidomide, or an analog thereof.

In some embodiments, the proteolysis targeting chimera has a structure selected from the following group: 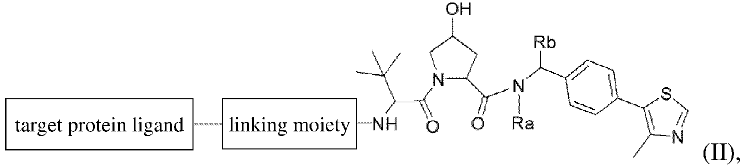 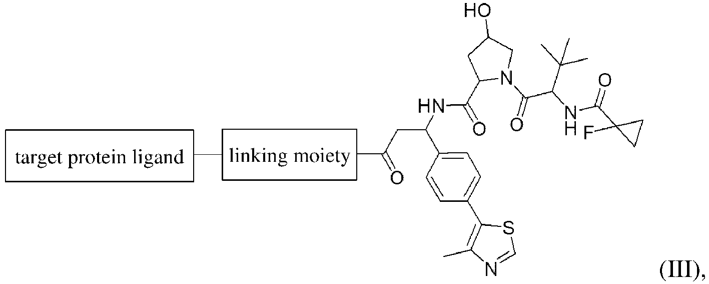 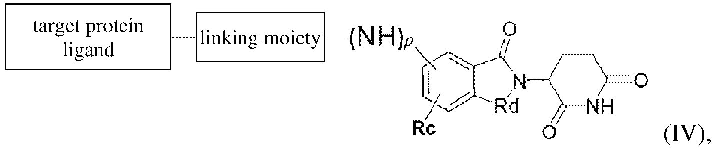 or 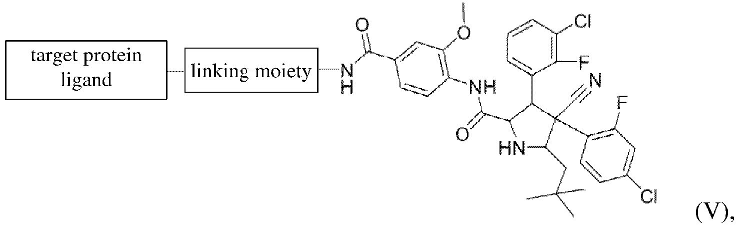 wherein,
Ra, Rb and Rc are each independently hydrogen, alkyl, NH₂, OH, or halogen; preferably the alkyl is methyl, and the halogen is fluorine;
Rd is CH₂, C=O, or C=S;
p is 0 or 1;
the target protein ligand can bind to a specific target protein;
the target protein ligand is linked to the E3 ligase group through the linking moiety.

In some embodiments, the target protein is a nuclear receptor (e.g., AR and ER), a kinase-like protein (e.g., RIPK2, BCR-ABL, EGFR, HER2, c-Met, TBK1, CDK2/4/6/9, ALK, Akt, CK2, ERK1/2, FLT3, PI3K, BTK, and FAK), AKT, PAN-BET protein, BET protein (e.g., BRD2, BRD3, BRD4, and BRD6), BLK, BRAF1, BCL-xl, ERRα, FKBP12, FRS2α, JAK1, JAK3, IKZF1, IRAK4, MDM2, MetAP2, PLK1, PSK-J3, RAS, TACC3, Tau, TrkB, MDM2, or any combination thereof. In some embodiments, the target protein is AR protein, ER protein, RAS protein, BRD4 protein, PLK1 protein, FAK protein, MDM2 protein, or any combination thereof.

In some embodiments, the target protein ligand moiety has a structure selected from:

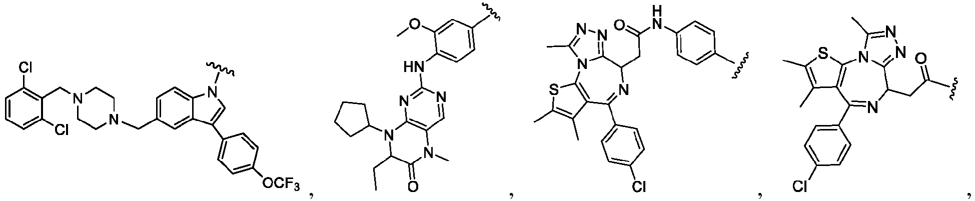

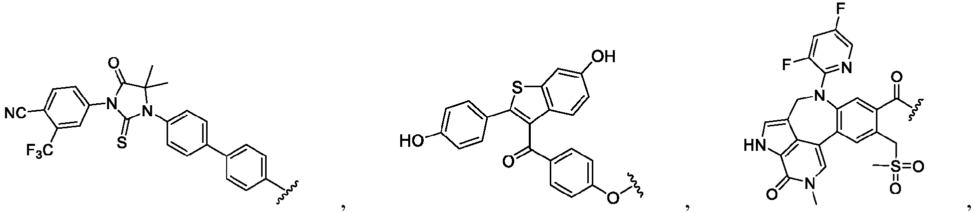

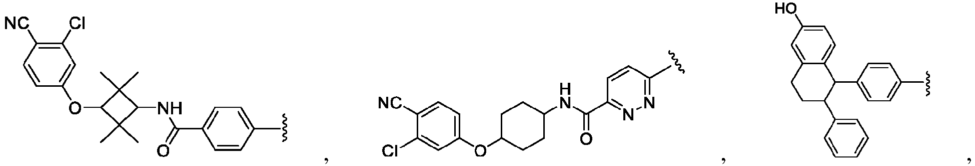

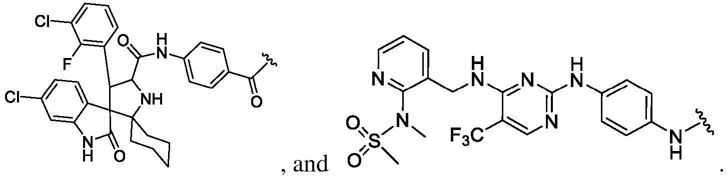

In some embodiments, the linking moiety in the proteolysis targeting chimera has a structure selected from the following group: 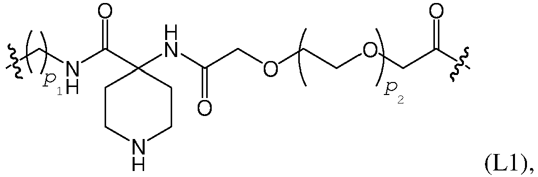 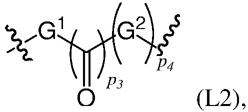 or 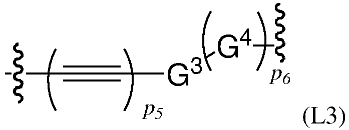 wherein,
p₁ is an integer of 0-4;
p₂ is an integer of 0-6;
p₃, p₄, p₅, and p₆ are each independently 0 or 1;
G¹ is a 4-14 membered linear alkylene, optionally 2-5 carbon atoms of the 4-14 membered linear alkylene may be substituted by -O-, -NH-, -N(C₁₋₆ alkyl)-, or -C≡C-;
G² is an amino acid residue, wherein preferably G² is a Lys residue;
G³ is selected from the following group: 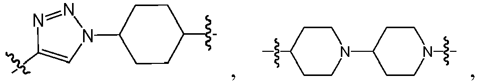 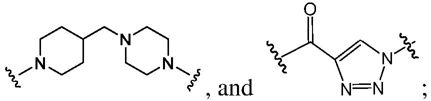
G⁴ is a 5-9 membered linear alkylene, optionally 2-4 carbon atoms of the 5-9 membered linear alkylene may be substituted by -O-, -NH-, -N(C₁₋₆ alkyl)-, or -C≡C-.

In some embodiments, the linking moiety in the proteolysis targeting chimera has a structure selected from the following group

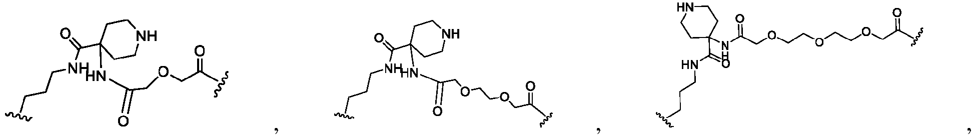

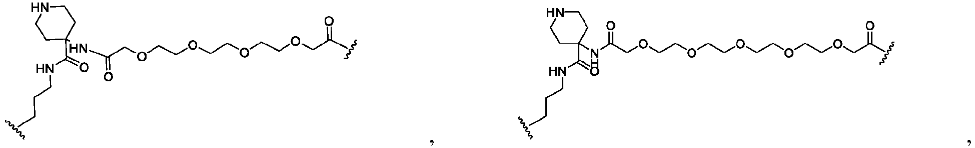

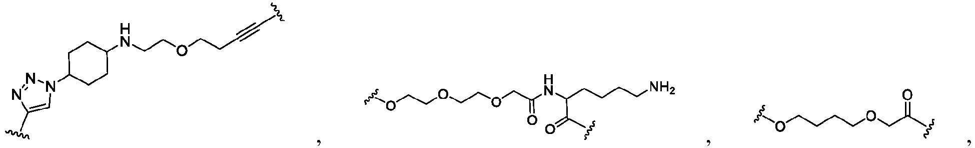

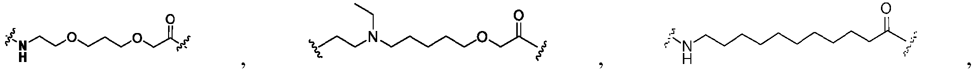

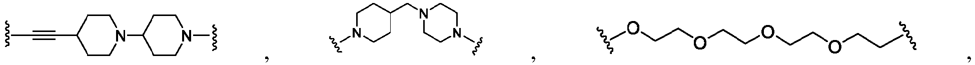

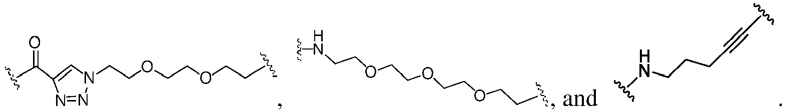

In some embodiments, the proteolysis targeting chimera has the structures shown below:

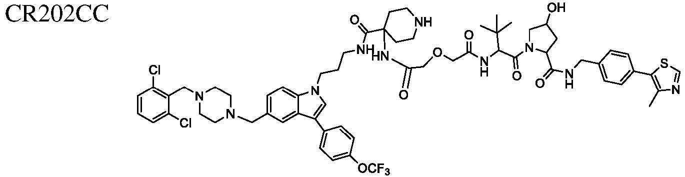

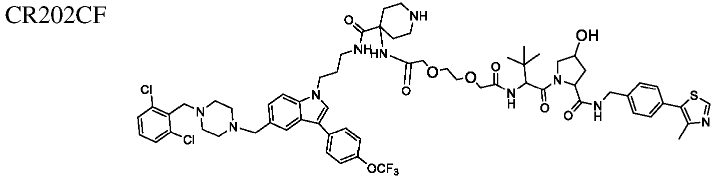

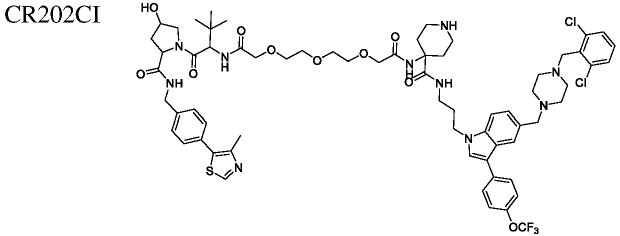

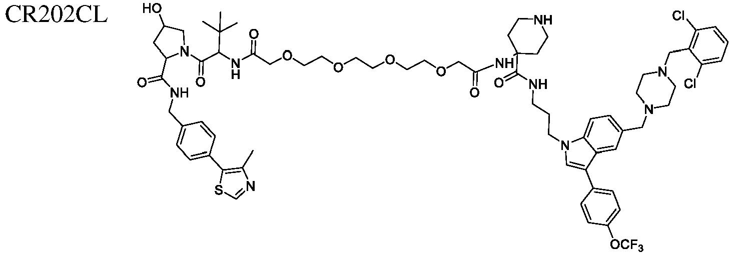

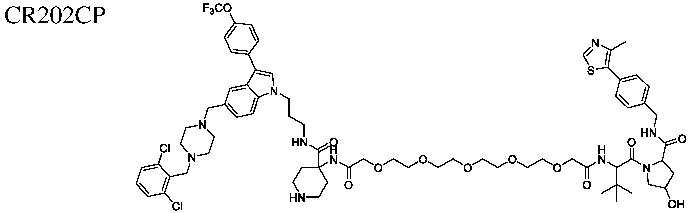

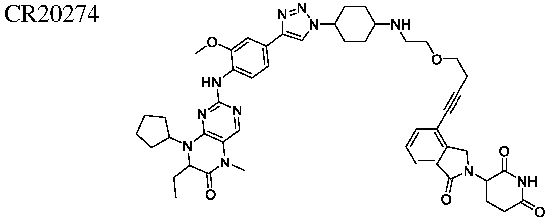

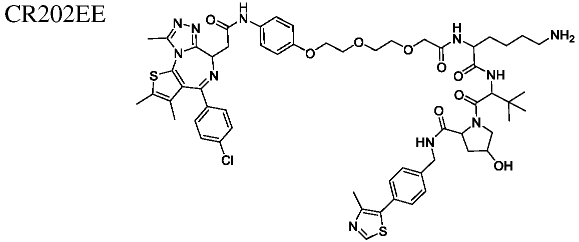

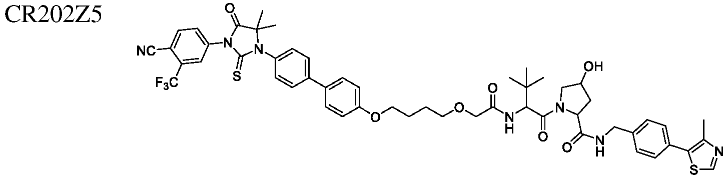

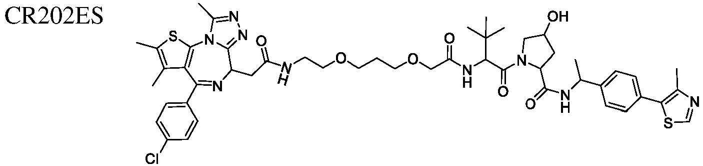

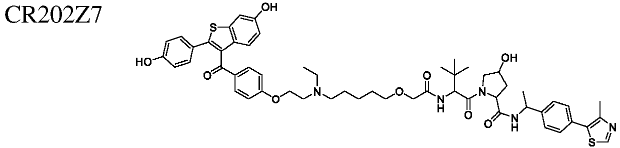

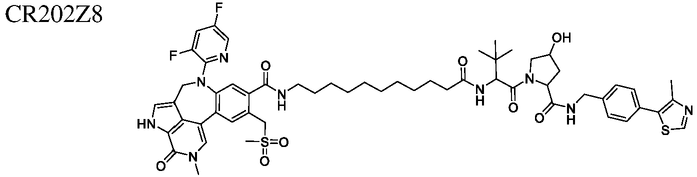

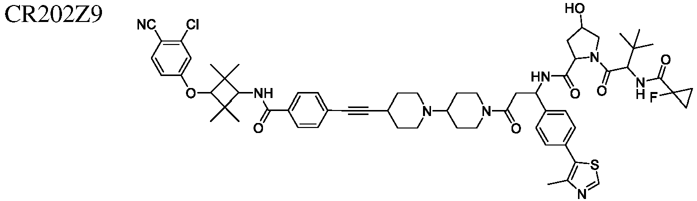

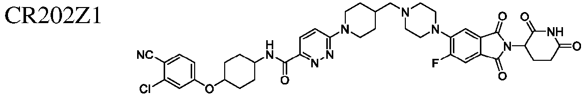

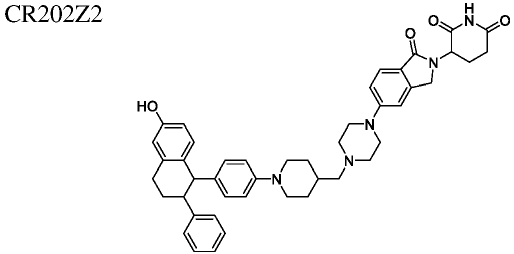

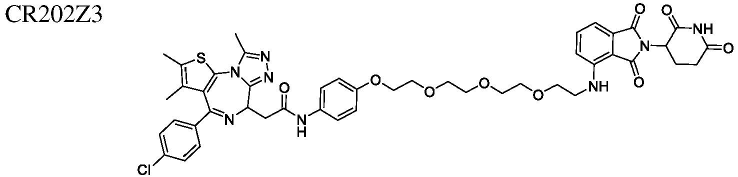

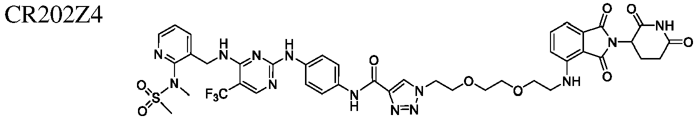

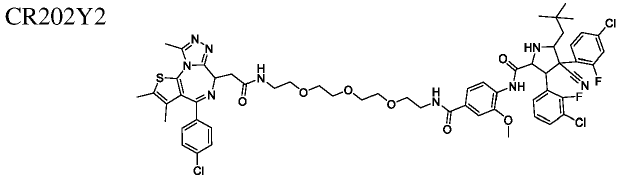

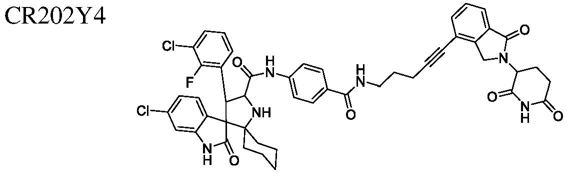

In some embodiments, the payload is linked to the targeted molecule directly, or the payload is linked to the targeted molecule via a linker. In some embodiments, the linkage between the payload and the targeted molecule, the payload and the linker, or the linker and the targeted molecule is accomplished by a chemical reaction. In some preferred embodiments, the linkage between the targeted molecule and the linker is accomplished by a click reaction.

In some embodiments, the linker comprises a linking module 1 and a linking module 2.

In some embodiments, the linker comprises a linking module 2.

In some embodiments, the linker comprises a linking module 1 and the following groups binding to the linking module 1: a PEG chain, 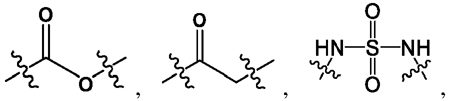 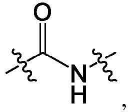 C₁-C₈ alkyl chain, -NH-, -O-, -C(O)-, or any combination thereof. In some preferred embodiments, the PEG chain is 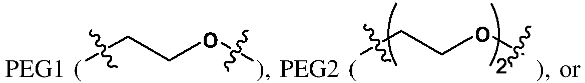 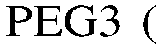 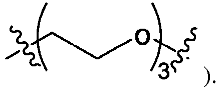 In some preferred embodiments, the linker comprises a linking module 1 and the following group binding to the linking module 1: 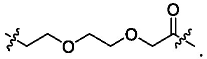 Those skilled in the art will appreciate that such groups may be bonded to the linking moiety in the linking module 1 through the left linking site and to the payload through the right linking site, or bonded to the linking moiety in the linking module 1 through the right linking site and to the payload through the left linking site.

In some embodiments, the linking module 1 is linked to the linking module 2 via a bond or a chemical group. In some embodiments, the chemical group is a PEG chain, 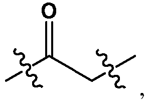 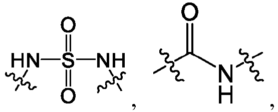 C₁-C₈ alkyl chain, -NH-, -O-, -C(O)-, 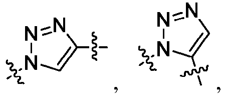 or any combination thereof. In some preferred embodiments, the PEG chain is PEG1, PEG2, or PEG3. In some preferred embodiments, the chemical group is 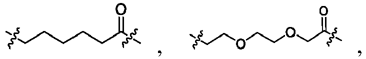 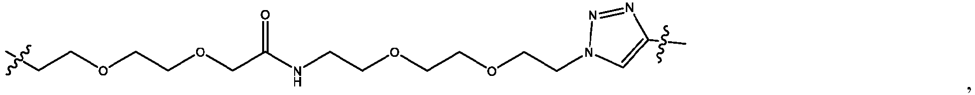 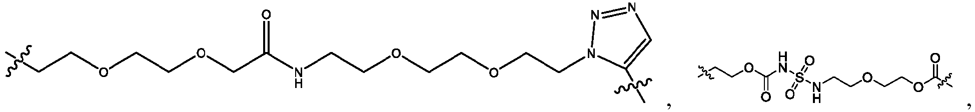 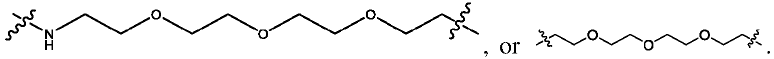 Those skilled in the art will appreciate that such chemical groups may be bonded to the linking module 1 through the left linking site and to the linking module 2 through the right linking site, or bonded to the linking module 1 through the right linking site and to the linking module 2 through the left linking site.

In some embodiments, the linking module 1 comprises maleimidocaproyl 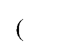 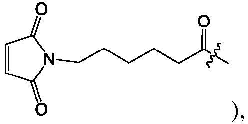 maleimido 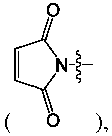 or azido

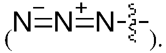

In some embodiments, the linking module 2 comprises a cathepsin-cleavable group, a plasmin-cleavable group, a legumain-cleavable group, a β-glucuronidase-cleavable group, a sulfatase-cleavable group, a phosphatase-cleavable group, a β-galactosidase-cleavable group, a glutathione enzyme-cleavable group, or an acid-labile group.

In some embodiments, the cathepsin-cleavable group is a CB enzyme-cleavable group.

In some preferred embodiments, the cathepsin-cleavable group comprises an amino acid or a combination of an amino acid and a self-decomposing fragment. In some preferred embodiments, the amino acid is Val-Cit, Gly-Gly-Phe-Gly, CycloBut-Cit, Phe-Cit, Val-Ala, Glu-Val-Cit, Ala-Cit, Val-Cit-Pro, or Val-Cit-Pro-Gly.

In some preferred embodiments, the plasmin-cleavable group comprises an amino acid or a combination of an amino acid and a self-decomposing fragment. In some preferred embodiments, the amino acid is Val-Leu-Lys.

In some preferred embodiments, the legumain-cleavable group comprises an amino acid or a combination of an amino acid and a self-decomposing fragment. In some preferred embodiments, the amino acid is selected from Gly-Asn-Asn or Ala-Ala-Asn.

In some preferred embodiments, the self-decomposing fragment is PAB and a derivative thereof, 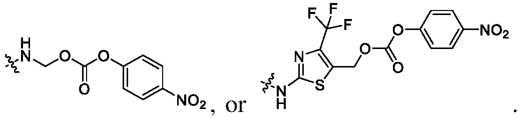 In some preferred embodiments, the PAB is 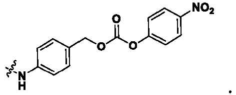 In some preferred embodiments, the PAB derivative is

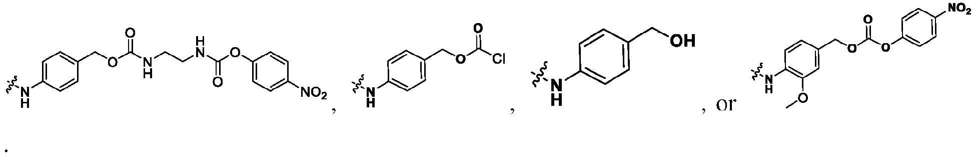

In some preferred embodiments, the β-glucuronidase-cleavable group is 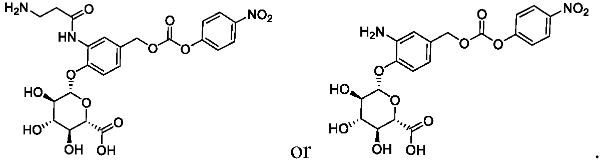 In some preferred embodiments, the sulfatase-cleavable group is 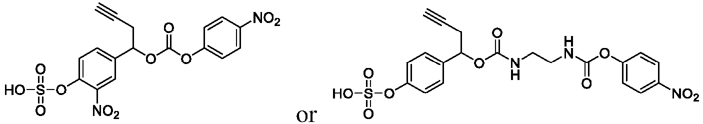 . In some preferred embodiments, the phosphatase-cleavable group is 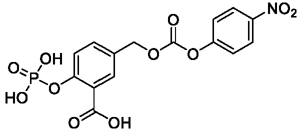 or 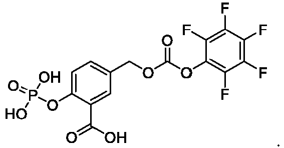 In some preferred embodiments, the β-galactosidase-cleavable group is 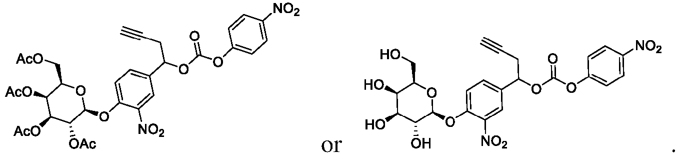 In some preferred embodiments, the glutathione enzyme-cleavable group is 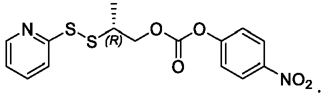 In some preferred embodiments, the acid-labile group is

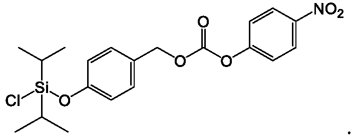

It will be appreciated by those skilled in the art that the active site of the cleavable groups described above may optionally be linked to the linking module 1, to the chemical group, to the targeted molecule, or to the payload after a proper reaction. For example, the β-glucuronidase-cleavable group, upon operative linkage to the linking module 1, the chemical group, the targeted molecule, or the payload, has the following structures 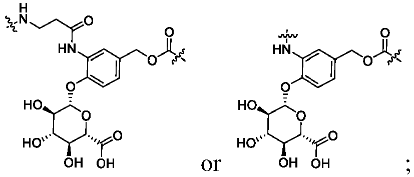 the sulfatase-cleavable group, upon operative linkage to the linking module 1, the chemical group, the targeted molecule, or the payload, has the following structures 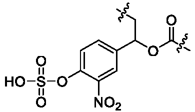 or 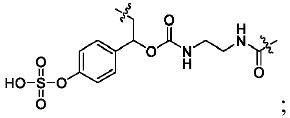 the phosphatase-cleavable group, upon operative linkage to the linking module 1, the chemical group, the targeted molecule, or the payload, has the following structure 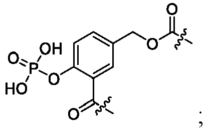 the β-galactosidase-cleavable group, upon operative linkage to the linking module 1, the chemical group, the targeted molecule, or the payload, has the following structures 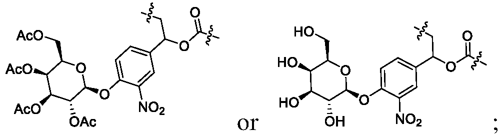 the glutathione enzyme-cleavable group, upon operative linkage to the linking module 1, the chemical group, the targeted molecule, or the payload, has the following structure 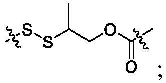 the acid-labile group, upon operative linkage to the linking module 1, the chemical group, the targeted molecule, or the payload, has the following structure

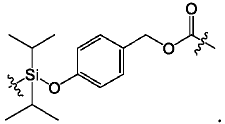

In some preferred embodiments, when the linker comprises the linking module 2, the linking module 2 is the glutathione enzyme-cleavable group. In some preferred embodiments, the glutathione enzyme-cleavable group is

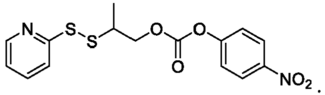

In some embodiments, the conjugate compound has the following structure:

W¹-L¹-(EG)n¹-(D)n⁵-(CH₂)n²-(B)n⁶-(AA)n³-(CH₂CH₂-Q)n⁴-L²-W²,

wherein,
**L¹** is 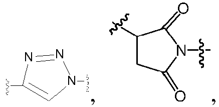 -S-S-, or a bond;
**EG** is -CH₂CH₂O- or -OCH₂CH₂-; n¹ is an integer of 0-6;
n² is an integer of 0-8;
**D** is -C(=O)-NH-S(=O)₂-NH-; n⁵ is 0 or 1;
**B** is each independently -C(=O)-, 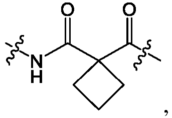 -NH-CH₂CH₂-C(=O)-, -C(=O)-NH-, -CH(CH₃)-CH₂-O-, -CH₂CH₂O-, -NH-C(=O)-, or -NH-; n⁶ is an integer of 0-3;
**AA** is each independently an amino acid residue; n³ is an integer of 0-5;
**Q** is each independently -C(=O)-, -O-, or a bond; n⁴ is an integer of 0-3;
**L²** is 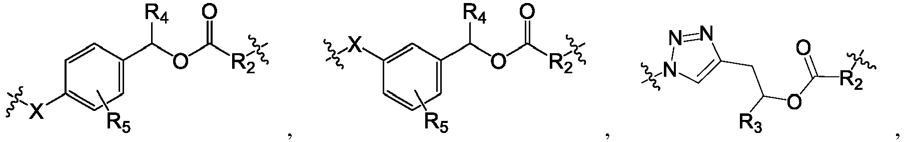 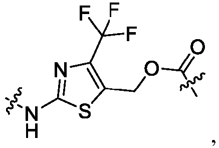 -NH-CH₂-O-, or a bond;
wherein X is a bond, -NH-, -C(=O)-, oxygen, or 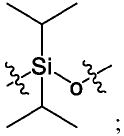
R₂ is a bond, -O-CH₂-, or -NH-CH₂CH₂-NH-C(=O)-;
R₃ is hydrogen or 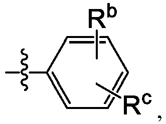 wherein R^{b} and R^{c} are each independently hydrogen, nitro, 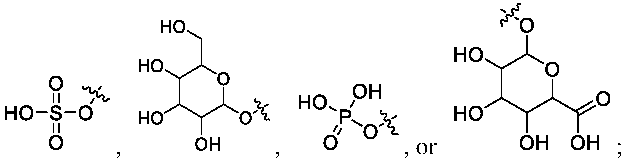
R₄ is hydrogen or C₁₋₈ alkyl;
R₅ is hydrogen, amino, nitro, phosphate group, -OR^{a}, or 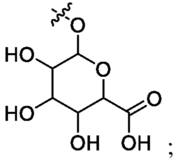 wherein R^{a} is methyl, ethyl, propyl, or isopropyl;
W¹ is the targeted molecule capable of binding to the cell surface protein, and
W² is the payload.

In some embodiments, EG is -CH₂CH₂O-. In some embodiments, n¹ is 2. In some embodiments, n¹ is 0. In some embodiments, n² is 1. In some embodiments, n² is 4. In some embodiments, n² is 5. In some embodiments, n⁵ is 0. In some embodiments, n⁶ is 1. In some embodiments, n⁶ is 3. In some embodiments, n¹ is 2, n² is 1, n³ and n⁴ are 0, and L² is a bond.

In some embodiments, AA is each independently an amino acid residue formed by Val, Cit, Glu, Leu, Lys, Ala, Gly, Phe, Gln, Pro, or Asn. In some embodiments, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Cit-, -Val-Leu-Lys-, -Val-Cit-Pro-Gly-, -Gly-Asn-Asn-, and -Ala-Ala-Asn-.

In some embodiments, when n⁴ is 1, Q is -C(=O)-; when n⁴ is 2, Q are -O- and -C(=O)-, respectively; when n⁴ is 3, Q are -O-, -O-, and a bond, respectively. In some embodiments, n⁴ is 0.

In some embodiments, L² is a bond, 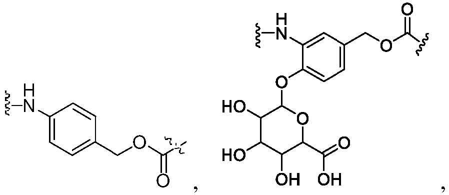 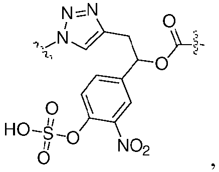 -NH-CH₂-O-, 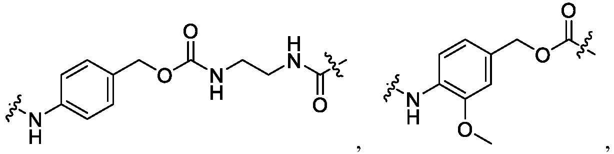 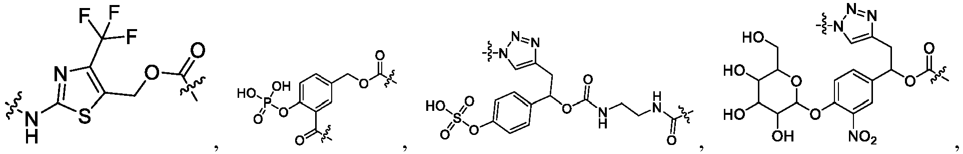 or

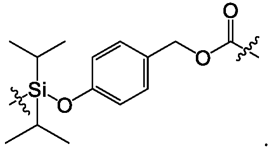

In some embodiments, the conjugate compound has the following structure:

W¹-L¹-(EG)n¹-(D)n⁵-(CH₂)n²-(B)n⁶-(AA)n³-(CH₂CH₂-Q)n⁴-L²-W²

wherein,
L¹ is 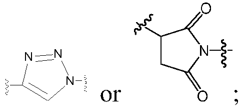
EG is -CH₂CH₂O-; n¹ is an integer of 0-4;
D is -C(=O)-NH-S(=O)₂-NH-; n⁵ is 0 or 1;
n² is an integer of 0-5;
B is -C(=O)-, -C(=O)-NH-, -CH₂CH₂O-, -NH-CH₂CH₂-C(=O)-, or -NH-; n⁶ is an integer of 0-3;
AA is each independently an amino acid residue; n³ is an integer of 0-3;
Q is each independently -C(=O)-, -O-, or a bond; n⁴ is an integer of 0-3;
L² is 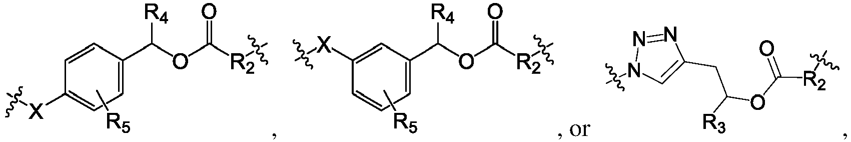
wherein X is a bond, -NH-, -C(=O)-, or 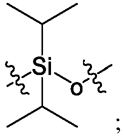
R₂ is a bond;
R₃ is 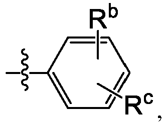 wherein R^{b} and R^{c} are each independently hydrogen, nitro, or 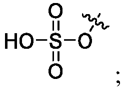
R₄ is hydrogen;
R₅ is hydrogen, -OCH₃, or 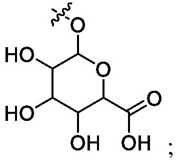
W¹ is the targeted molecule capable of binding to the cell surface protein, and W² is the payload.

In some embodiments, L² is

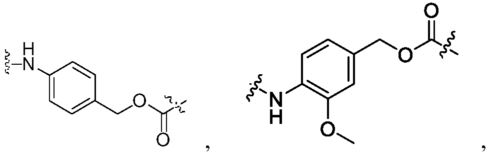

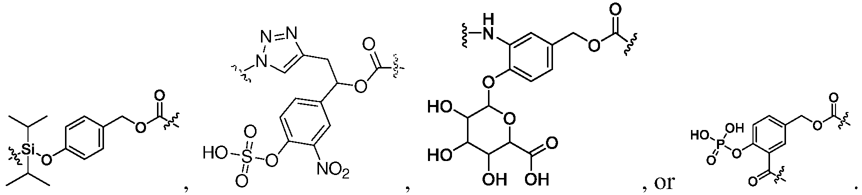

In some embodiments, L¹ is 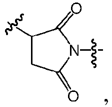 and n¹, n⁵, and n⁴ are all 0.

In some embodiments, L¹ is 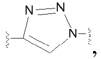 and n² is 1 or 2.

In some embodiments, when n⁵ is 1, n² and n⁴ are both 0.

In some embodiments, AA is each independently an amino acid residue formed by Val, Cit, Glu, or Ala. In some embodiments, -(AA)n³- is a peptide residue selected from the following group: -Val-Cit-, -Glu-Val-Cit-, -Ala-Cit-, and -Val-Ala-.

In some embodiments, the conjugate compound has the following structure:

W¹-L¹-(EG)n¹-(CH₂)n²-(C=O)-(AA)n³-L²-W²

wherein,
L¹ is 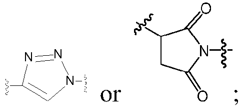
EG is -CH₂CH₂O-; n¹ is an integer of 0-4;
n² is an integer of 0-5;
AA is each independently an amino acid residue; n³ is an integer of 1-4;
L² is 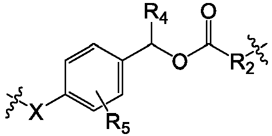 or a bond,
wherein X is -NH-;
R₂ is a bond;
R₄ is hydrogen;
R₅ is hydrogen or -OCH₃;
W¹ is the targeted molecule capable of binding to the cell surface protein, and W² is the payload.

In some embodiments, L¹ is 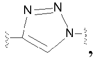 n¹ is 2, n² is 1, and L² is

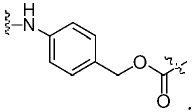

In some embodiments, L¹ is 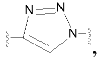 n¹ is 2, n² is 1, and L² is a bond.

In some embodiments, L¹ is 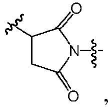 n¹ is 0, n² is 5, and L² is

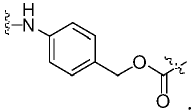

In some embodiments, L¹ is 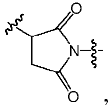 n¹ is 0, n² is 5, and L² is a bond.

In some embodiments, AA is each independently Val, Cit, Glu, or Ala amino acid residue. In some embodiments, -(AA)n³- is a peptide residue selected from the following group: - Val-Cit-, -Glu-Val-Cit-, -Ala-Cit-, -Phe-Cit-, -Val-Ala-, -Gly-Gly-Phe-Gly-, -Val-Leu-Lys-, and - Gly-Asn-Asn-.

In some embodiments, the conjugate compound has the following structures: 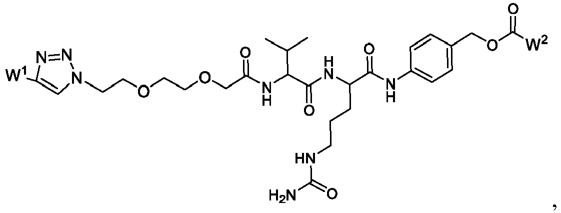 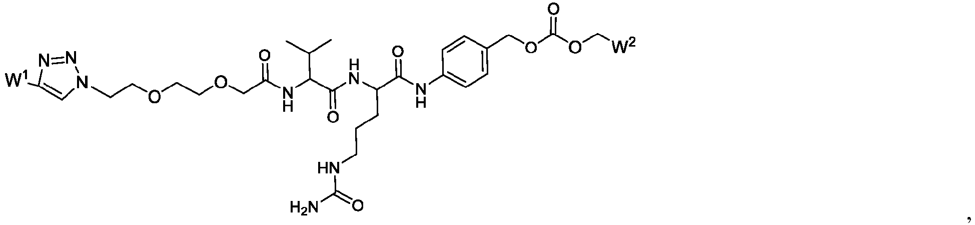 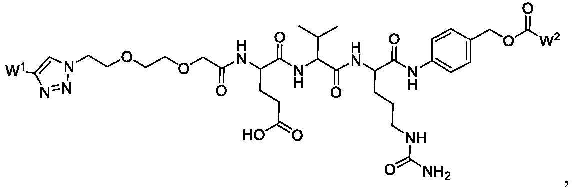 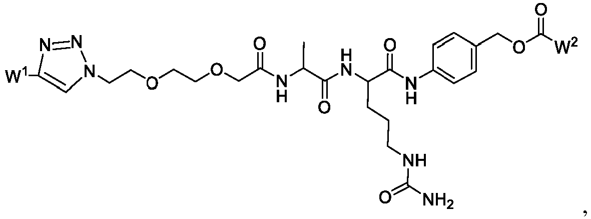 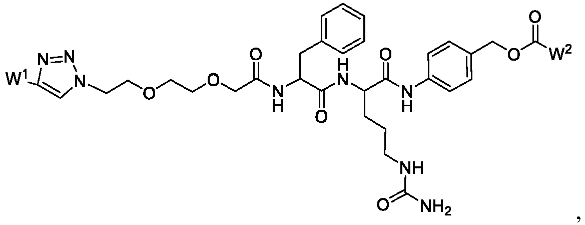 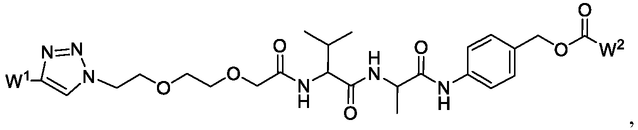 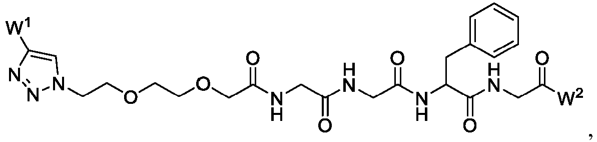 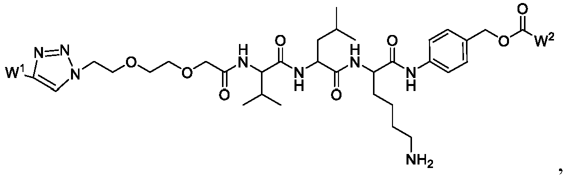 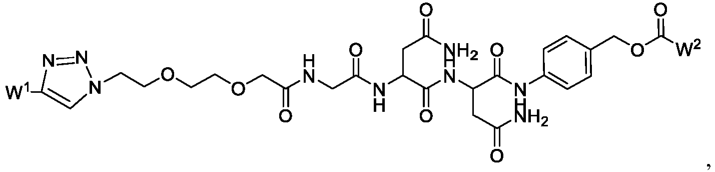 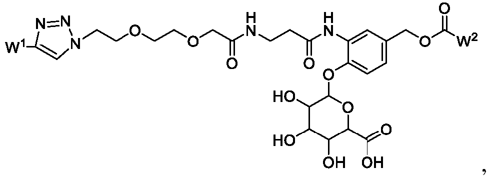 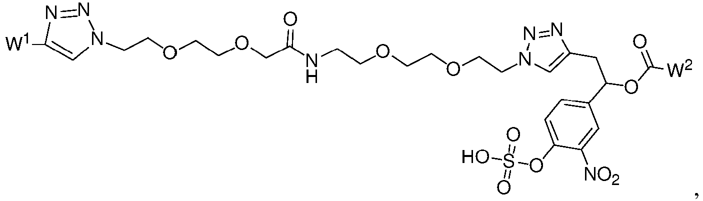 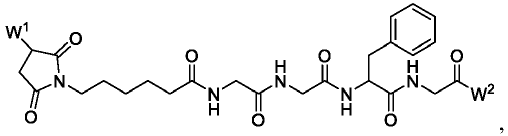 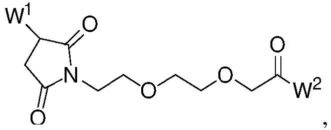 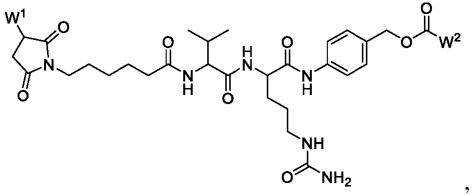 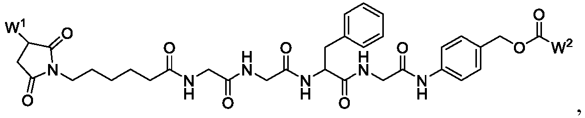 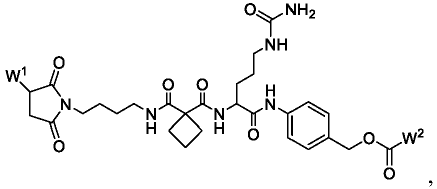 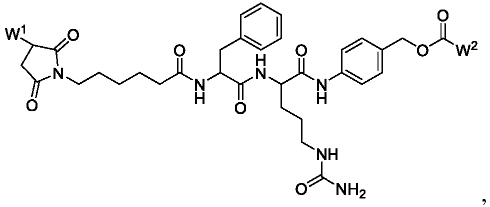 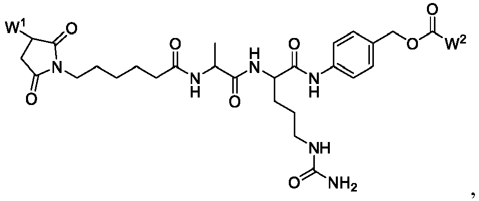 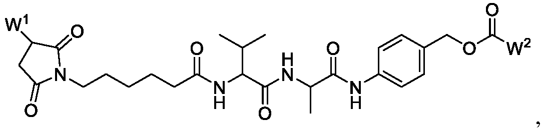 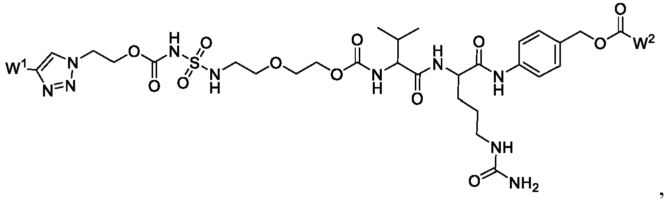 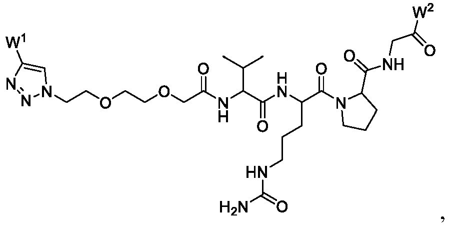 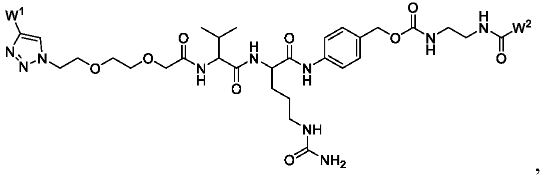 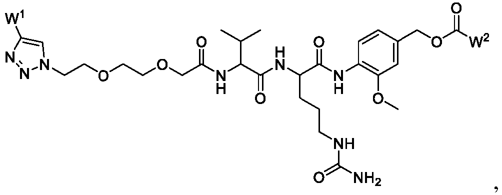 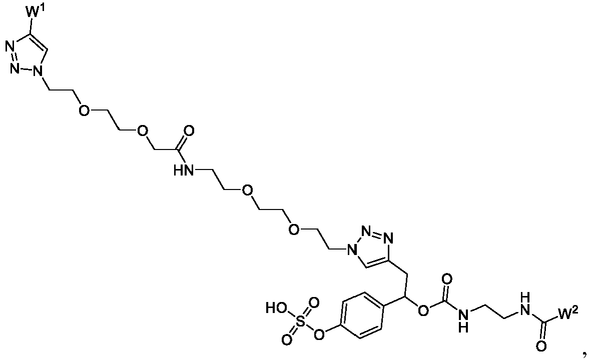 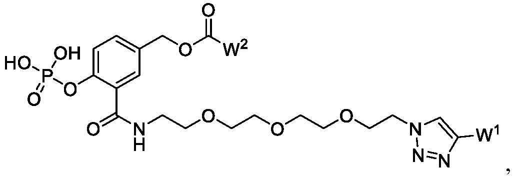 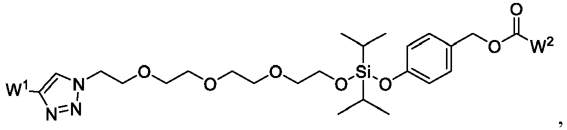 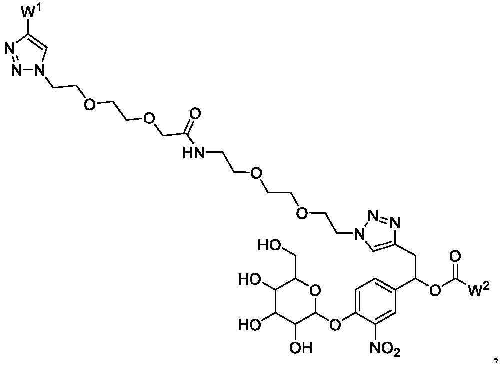 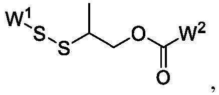 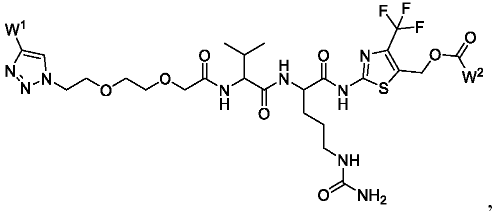 or 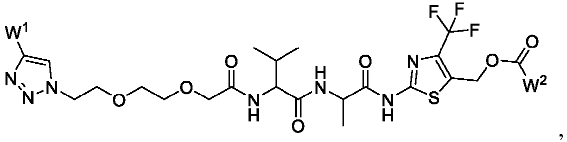 wherein W¹ is the targeted molecule capable of binding to the cell surface protein, and W² is the payload.

In some embodiments, the conjugate compound has the following structures:

| | |
|---|---|
| CR202CE | 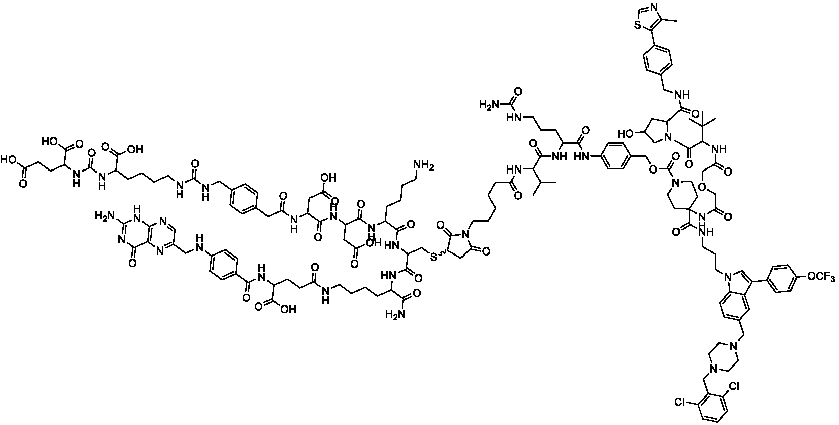 |
| CR202CH | 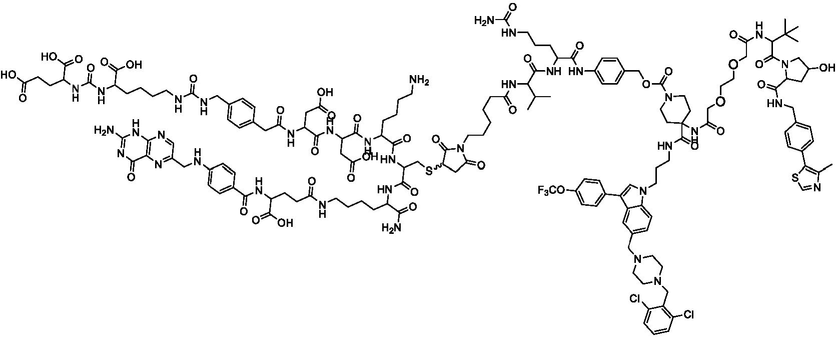 |
| CR202CK | 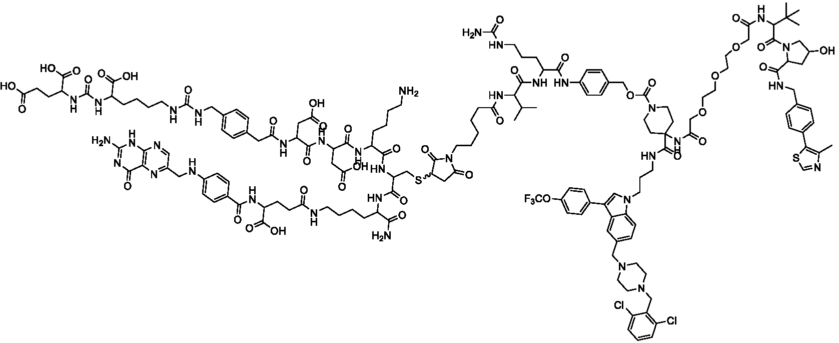 |
| CR202CN | 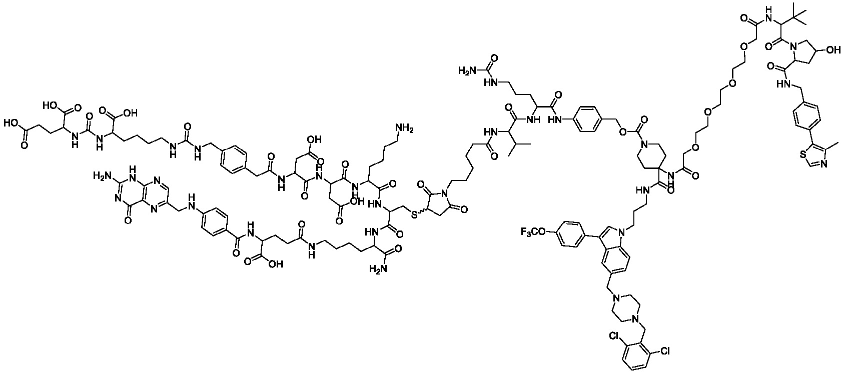 |
| CR202CR | 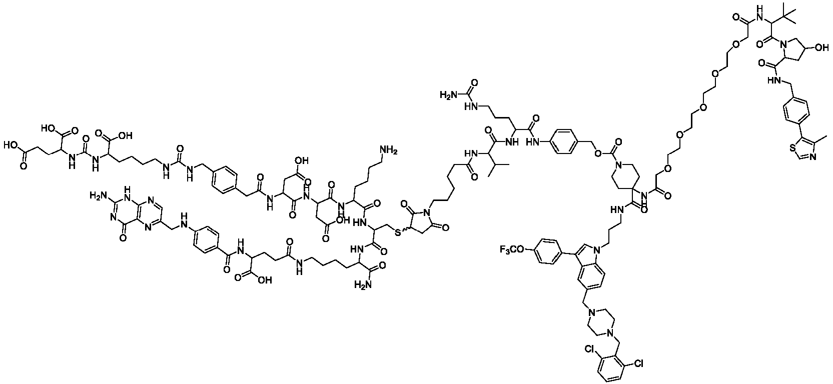 |
| CR202F0 | 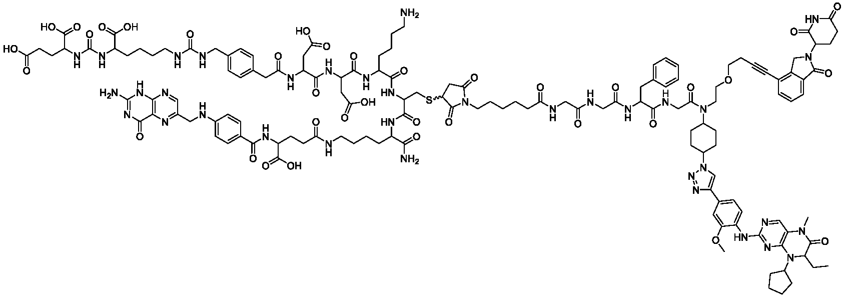 |
| CR202F2 | 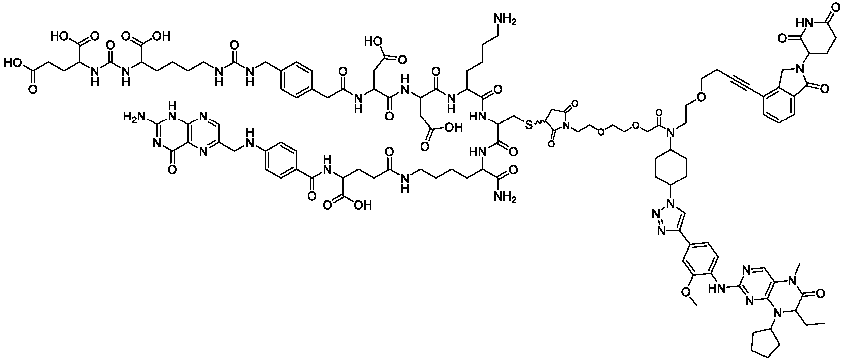 |
| CR20214 | 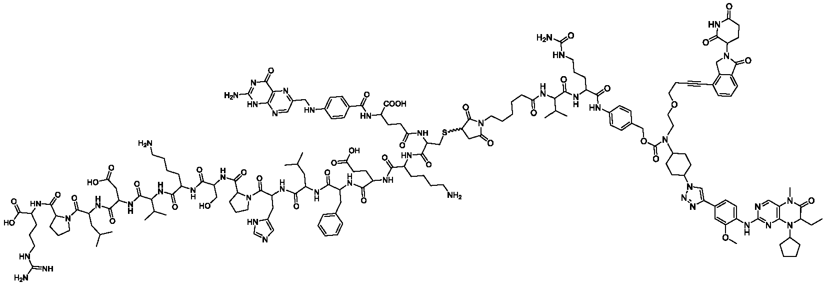 |
| CR20282 | 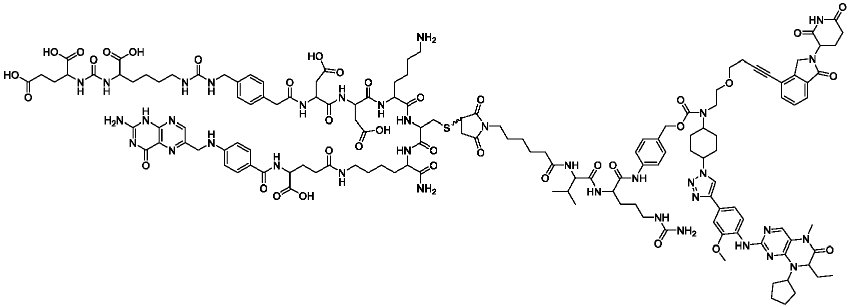 |
| CR20285 | 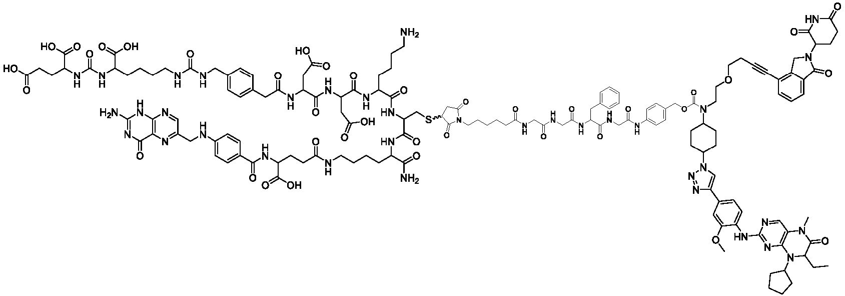 |
| CR20287 | 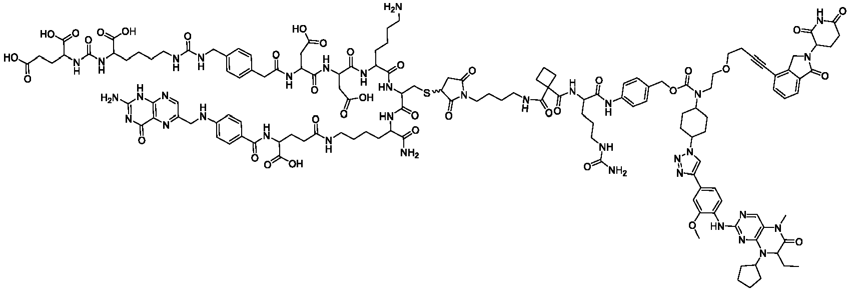 |
| CR20293 | 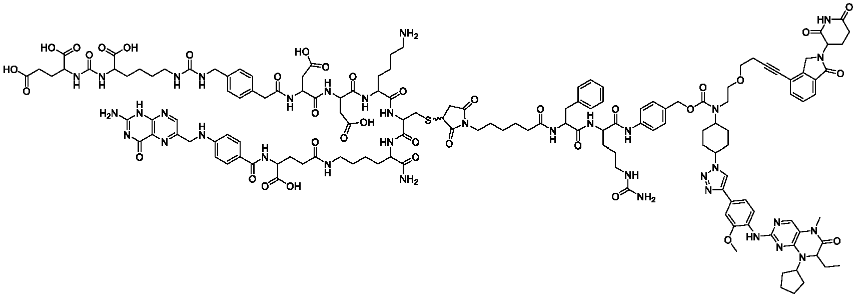 |
| CR20295 | 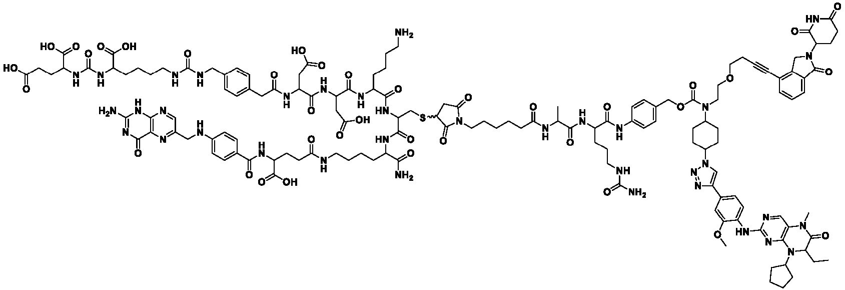 |
| CR20297 | 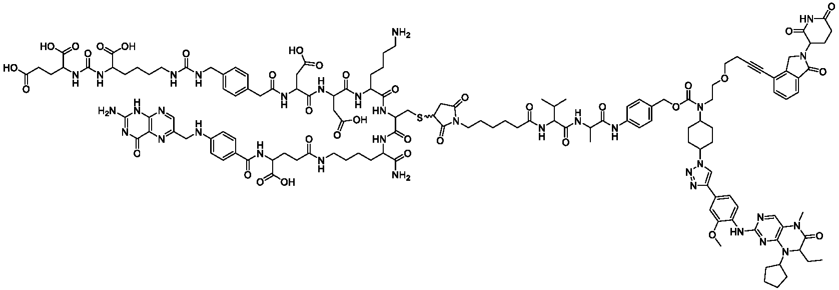 |
| CR202EF | 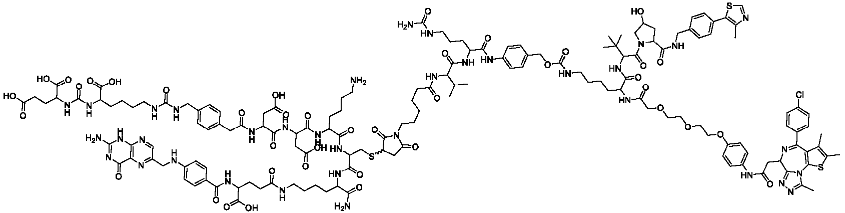 |
| CR202W5 | 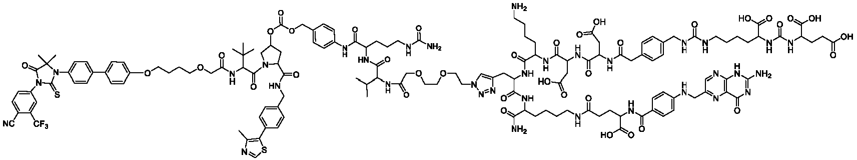 |
| CR202W6 | 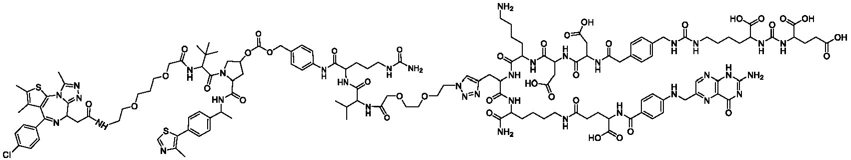 |
| CR202W7 | 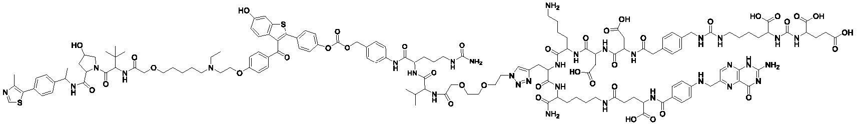 |
| CR202W8 | 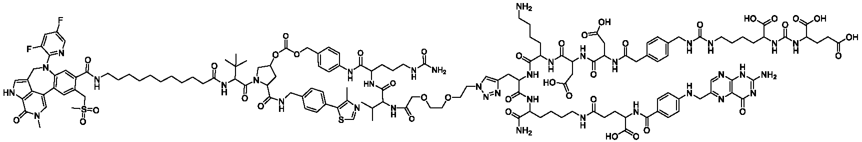 |
| CR202W9 | 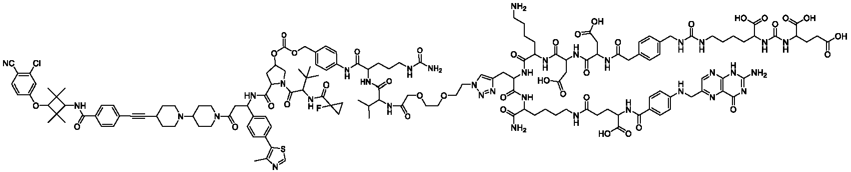 |
| CR202X0 | 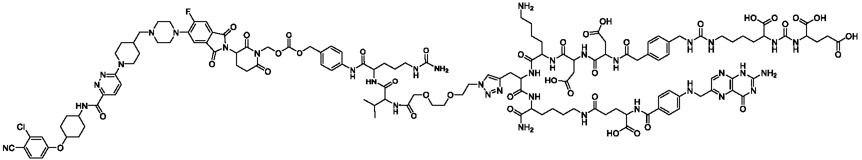 |
| CR202X1 | 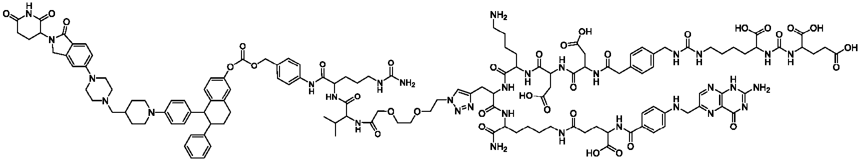 |
| CR202X2 | 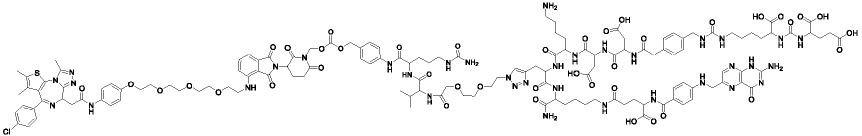 |
| CR202X3 | 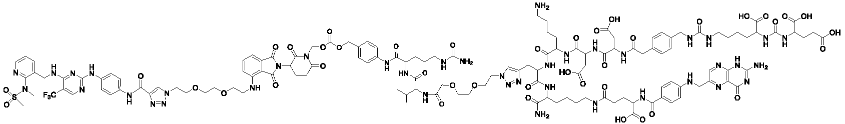 |
| CR202Y3 | 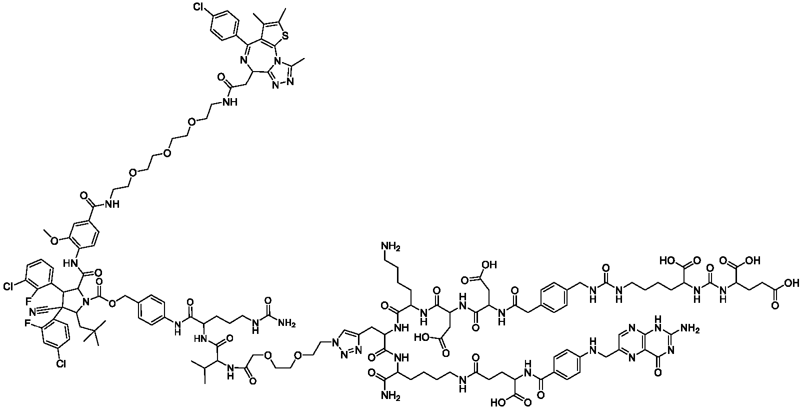 |
| CR202Y5 | 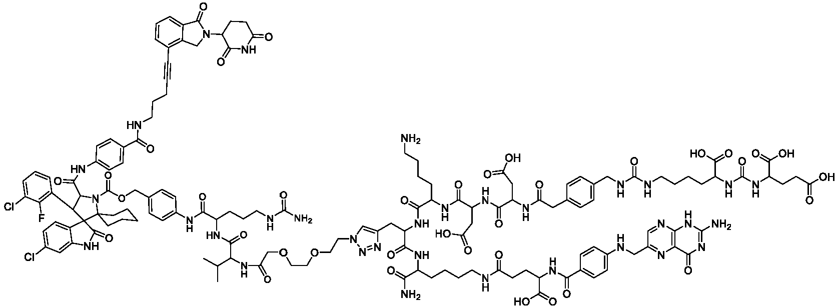 |
| CR202V4 | 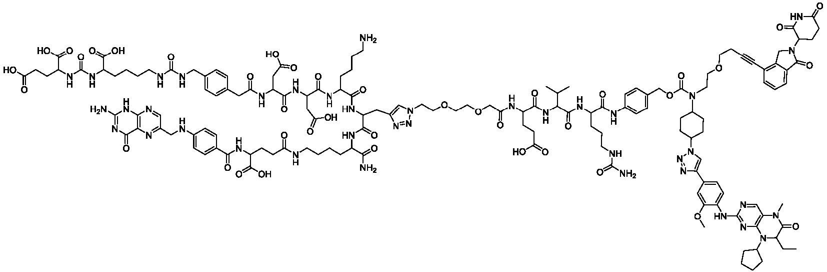 |
| CR202V5 | 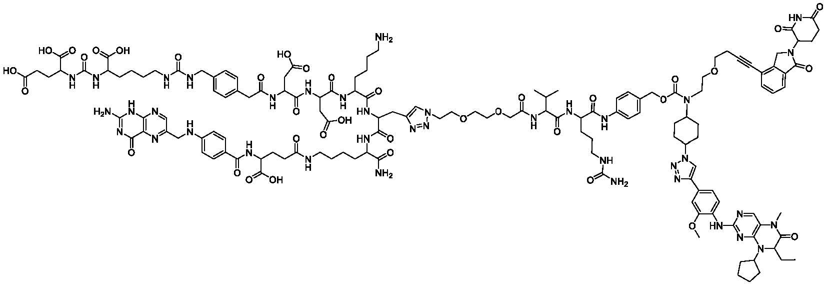 |
| CR202V6 | 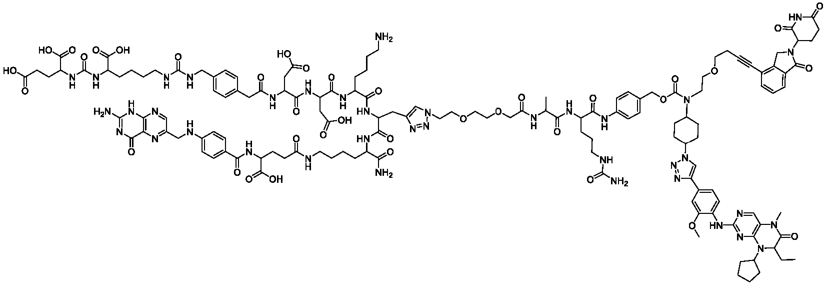 |
| CR202V7 | 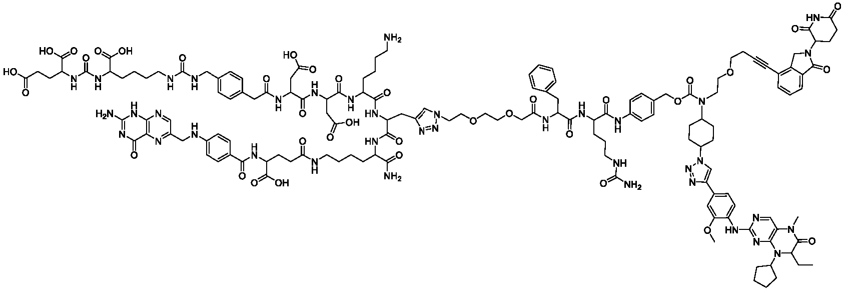 |
| CR202Z6 | 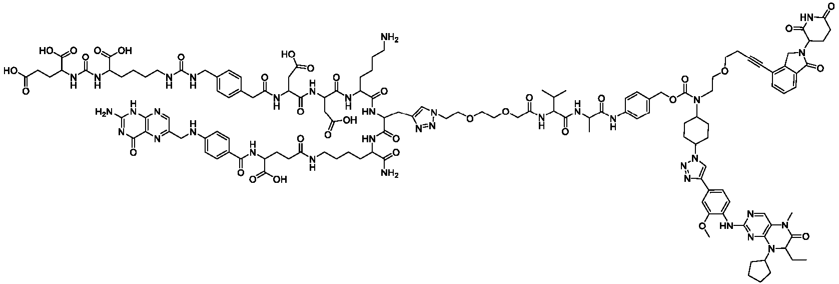 |
| CR202V8 | 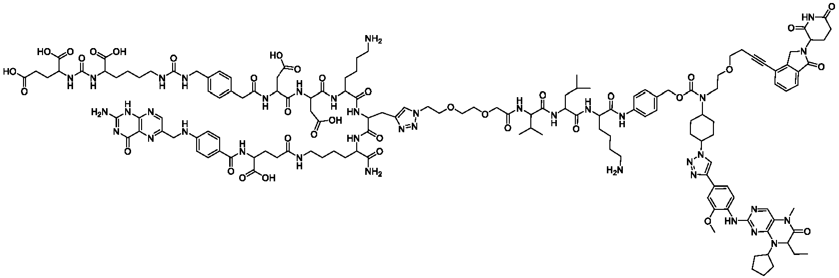 |
| CR202V9 | 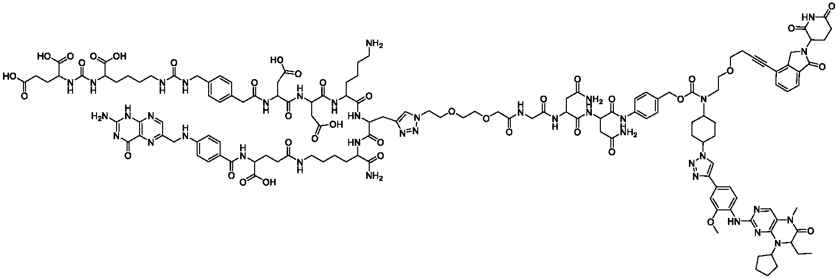 |
| CR202AL | 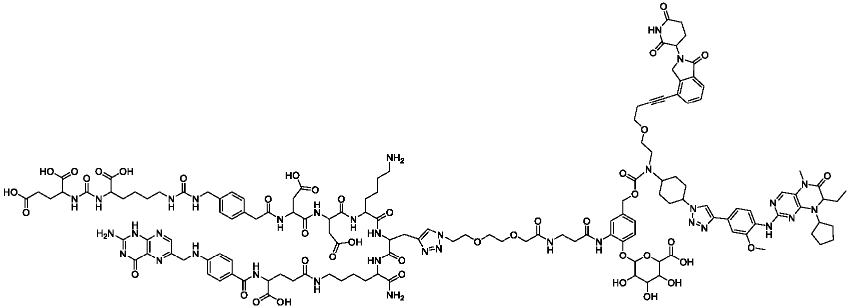 |
| CR202W1 | 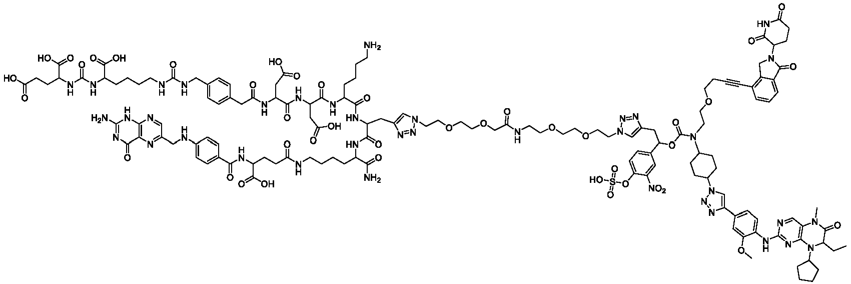 |
| CR202GM | 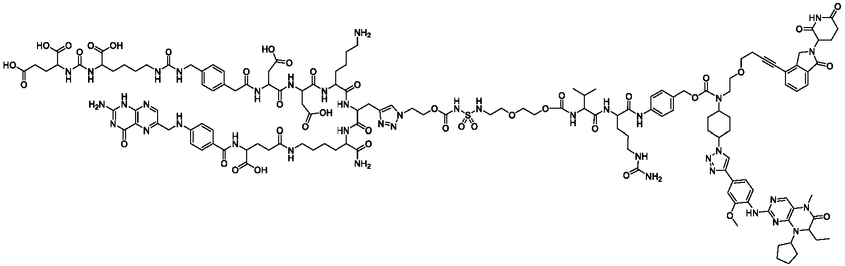 |
| CR202GQ | 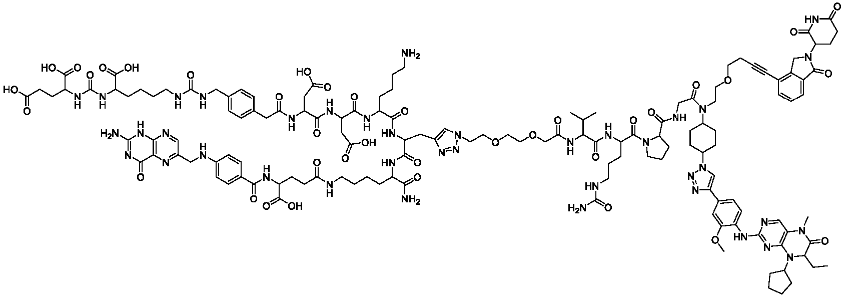 |
| CR202HJ | 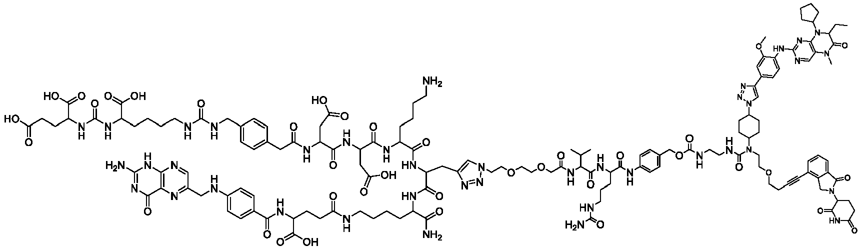 |
| CR202FU | 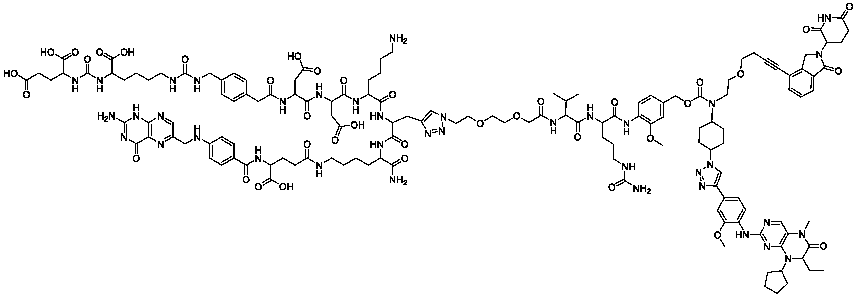 |
| CR202JG | 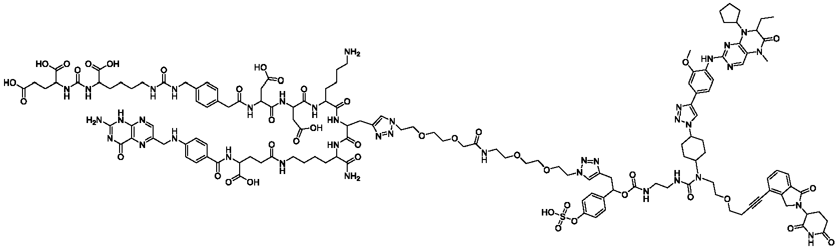 |
| CR202LB | 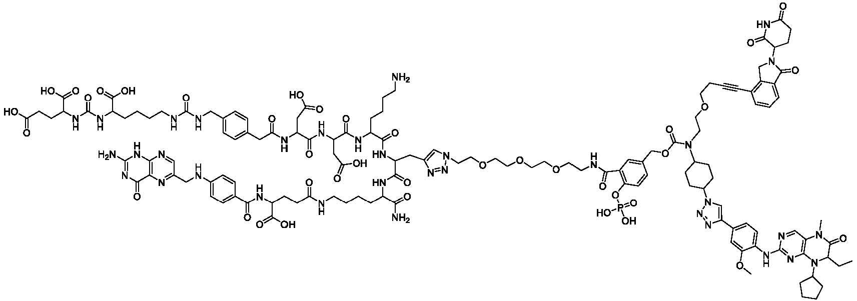 |
| CR202HT | 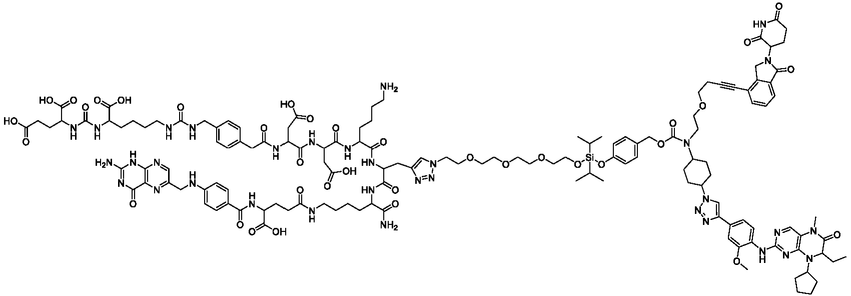 |
| CR202HU | 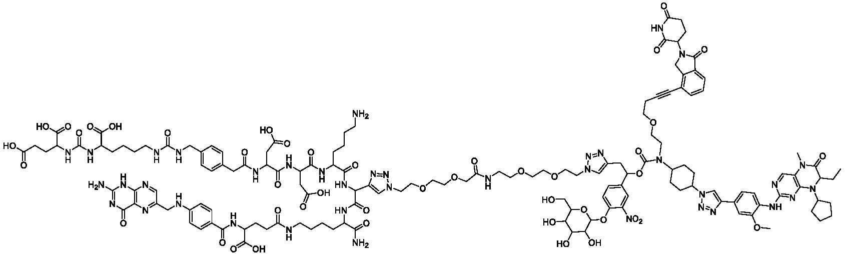 |
| CR202HV | 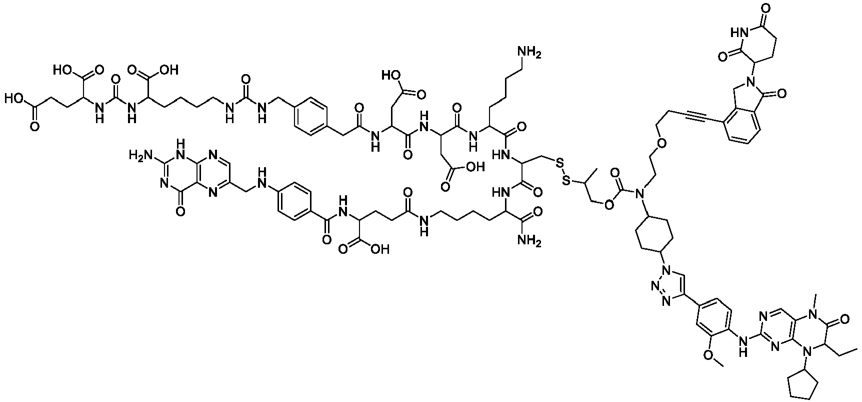 |
| CR202FX | 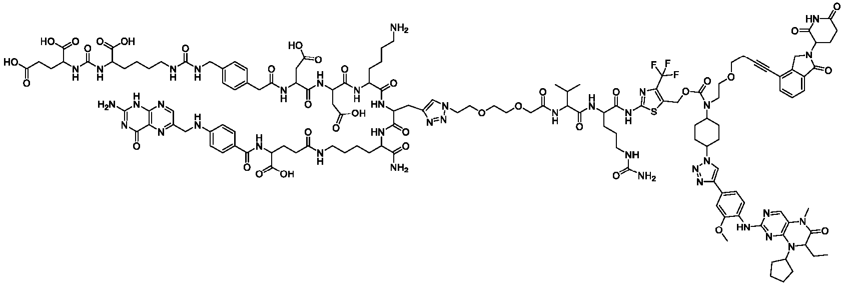 |
| CR202HR | 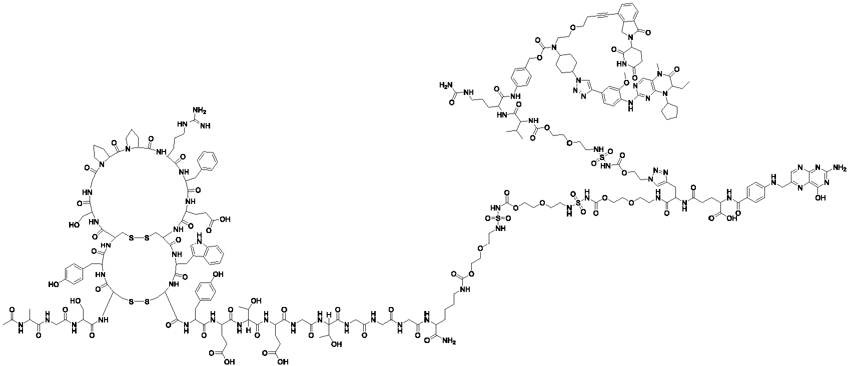 |
| CR202GC | 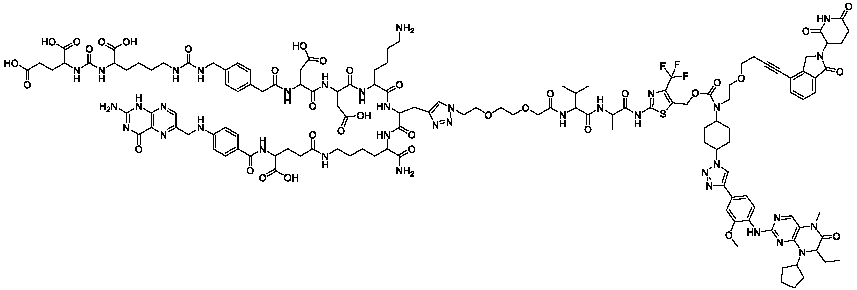 |
| CR202MT | 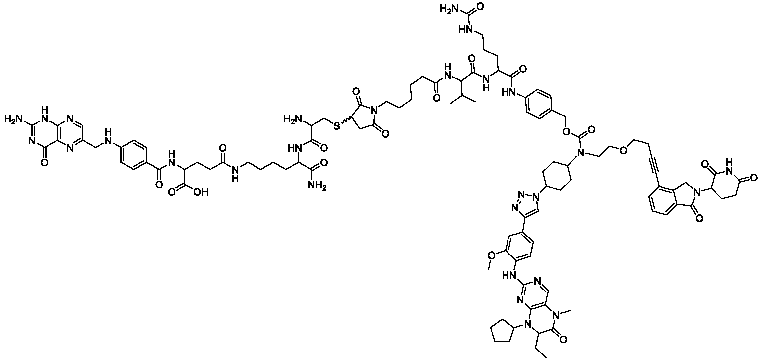 |
| CR202EZ | 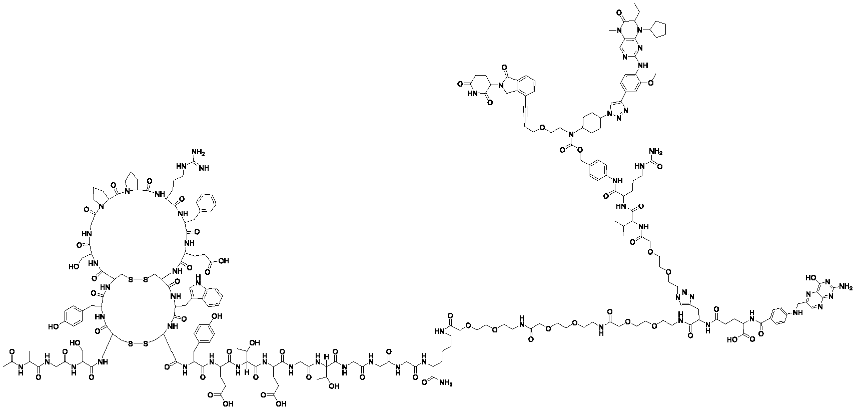 |

In another aspect, the present application provides a pharmaceutical composition, comprising the conjugate compound or the pharmaceutically acceptable salt thereof disclosed herein, and a pharmaceutically acceptable carrier. In some embodiments, the pharmaceutical composition is for intravenous, subcutaneous, oral, intramuscular, or intraventricular administration.

In another aspect, the present application provides a method for treating a disease in a subject, comprising administering to the subject a therapeutically effective amount of the conjugate compound or the pharmaceutically acceptable salt thereof disclosed herein or the pharmaceutical composition disclosed herein. In some embodiments, the disease is selected from the following group: a cancer, an immune disease, a cardiovascular disease, a metabolic disease, and a neurological disease. In some embodiments, the disease is characterized by the expression of the target protein or abnormal expression of the target protein in the subject. In some embodiments, the method further comprises administering one or more therapeutic agents in combination with the conjugate compound or the pharmaceutically acceptable salt thereof or the pharmaceutical composition.

In some embodiments, the cancer is characterized by cancer cells overexpressing FOLR1, TRPV6, PSMA, or c-MET. In some embodiments, the cancer is characterized by cancer cells overexpressing FOLR1 and TRPV6, FOLR1 and c-MET, c-MET and TRPV6, c-MET and PSMA, PSMA and TRPV6, or FOLR1 and PSMA. In some embodiments, the cancer is selected from the following group: human breast ductal carcinoma, human brain astroblastoma, transitional cell papilloma of the bladder, prostate cancer, breast cancer, lung cancer, kidney cancer, leukemia, ovarian cancer, gastric cancer, cervical cancer, uterine cancer, endometrial cancer, liver cancer, colon cancer, thyroid cancer, pancreatic cancer, colorectal cancer, esophageal cancer, testicular cancer, skin cancer, lymphoma, and multiple myeloma. In some embodiments, the lung cancer is non-small cell lung cancer or small cell lung cancer.

In some embodiments, the immune disease is an autoimmune disease. In some embodiments, the autoimmune disease is selected from the following group: connective tissue disorder, systemic sclerosis, rheumatoid arthritis, and systemic lupus erythematosus.

In some embodiments, the cardiovascular disease is selected from the following group: angina pectoris, myocardial infarction, stroke, heart attack, hypertensive heart disease, rheumatic heart disease, cardiomyopathy, cardiac arrhythmia, and congenital heart disease.

In some embodiments, the metabolic disease is selected from the following group: diabetes, gout, obesity, hypoglycemia, hyperglycemia, and dyslipidemia.

In some embodiments, the neurological disease is selected from the following group: Alzheimer's disease, Parkinson's disease, Huntington's disease, head injury, multiple sclerosis, vertigo, coma, and epilepsy.

In another aspect, the present application provides use of the conjugate compound or the pharmaceutically acceptable salt thereof disclosed herein, or the pharmaceutical composition disclosed herein in preparing a medicament for treating a disease in a subject.

In another aspect, the present application provides a compound or a salt thereof, wherein the compound has the following structure:

L^{a}-(EG)n¹-(D)n⁵-(CH₂)n²-(B)n⁶-(AA)n³-(CH₂CH₂-Q)n⁴-L^{b},

wherein,
L^{a} is 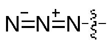 , 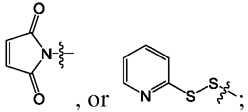
EG is -CH₂CH₂O- or -OCH₂CH₂-; n¹ is an integer of 0-6;
n² is an integer of 0-8;
D is -C(=O)-NH-S(=O)₂-NH-; n⁵ is 0 or 1;
B is -C(=O)-, 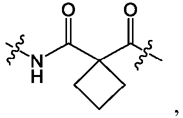 -NH-CH₂CH₂-C(=O)-, -C(=O)-NH-, -CH(CH₃)-CH₂-O-, - CH₂CH₂O-, -NH-C(=O)-, or -NH-; n⁶ is an integer of 0-3;
AA is each independently an amino acid residue; n³ is an integer of 0-5;
Q is each independently -C(=O)-, -O-, or a bond; n⁴ is an integer of 0-3;
L^{b} is 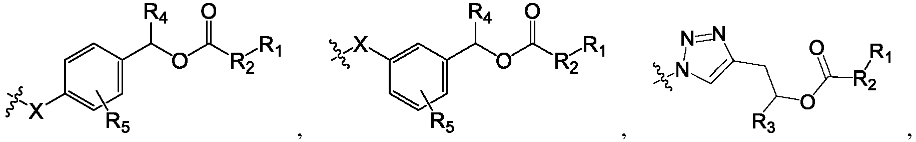 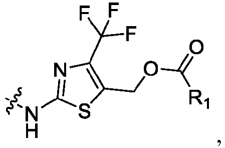 -OH, or -NH-CH₂-OH;
wherein X is a bond, -NH-, -C(=O)-, oxygen, or 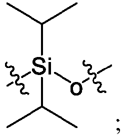
R₂ is a bond, -O-CH₂-, or -NH-CH₂CH₂-NH-C(=O)-;
R₃ is hydrogen or 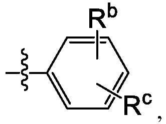 wherein R^{b} and R^{c} are each independently hydrogen, nitro, 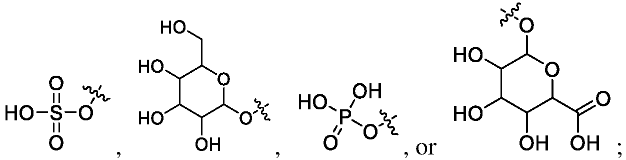
R₄ is hydrogen or C₁₋₈ alkyl;
R₅ is hydrogen, amino, nitro, phosphate group, -OR^{a}, or 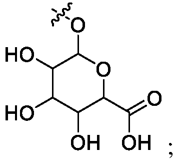 wherein R^{a} is methyl, ethyl, propyl, or isopropyl;
R₁ is a leaving group, preferably R₁ is halogen (e.g., chlorine),

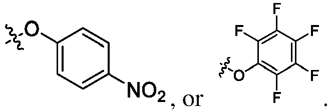

In some embodiments, EG is -CH₂CH₂O-. In some embodiments, n¹ is 2. In some embodiments, n¹ is 0. In some embodiments, n² is 1. In some embodiments, n² is 4. In some embodiments, n² is 5. In some embodiments, n⁵ is 0. In some embodiments, n⁶ is 1. In some embodiments, n⁶ is 3. In some embodiments, n¹ is 2, n² is 1, n³ and n⁴ are 0, and L^{b} is -OH.

In some embodiments, AA is each independently an amino acid residue formed by Val, Cit, Glu, Leu, Lys, Ala, Gly, Phe, Gln, Pro, or Asn. In some embodiments, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Cit-, -Val-Leu-Lys, -Val-Cit-Pro-Gly-, -Gly-Asn-Asn-, and -Ala-Ala-Asn-. In some embodiments, when n⁴ is 1, Q is -C(=O)-; when n⁴ is 2, Q are -O- and -C(=O)-, respectively; when n⁴ is 3, Q are -O-, -O-, and a bond, respectively. In some embodiments, n⁴ is 0.

In some embodiments, L^{b} is 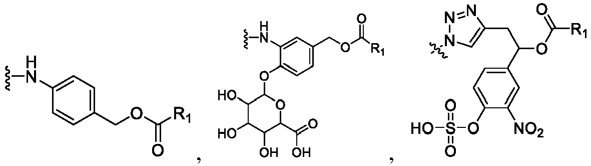 , -OH, -NH-CH₂-OH,

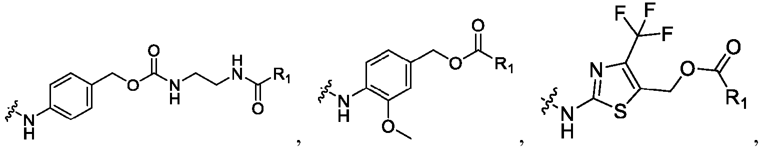

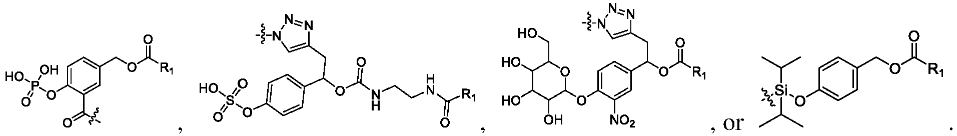

In some embodiments, the compound has the following structure:

L^{a}-(EG)n¹-(D)n⁵-(CH₂)n²-(B)n⁶-(AA)n³-(CH₂CH₂-Q)n⁴-L^{b}

wherein,
L^{a} is 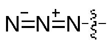 or 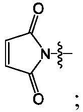
EG is -CH₂CH₂O-; n¹ is an integer of 2-4;
D is -C(=O)-NH-S(=O)₂-NH-; n⁵ is 0 or 1;
n² is an integer of 0-5;
B is -C(=O)-, -C(=O)-NH-, -CH₂CH₂O-, -NH-CH₂CH₂-C(=O)-, or -NH-; n⁶ is an integer of 0-3;
AA is each independently an amino acid residue; n³ is an integer of 0-3;
Q is each independently -C(=O)-, -O-, or a bond; n⁴ is an integer of 0-3;
L^{b} is -OH, 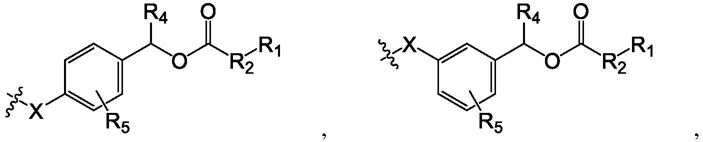 or 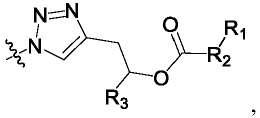
wherein X is a bond, -NH-, -C(=O)-, or 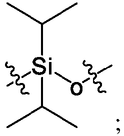
R₂ is a bond;
R₃ is 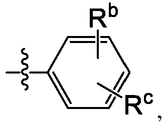 wherein R^{b} and R^{c} are each independently hydrogen, nitro, or 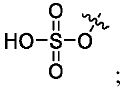
R₄ is hydrogen;
R₅ is hydrogen, -OCH₃, or 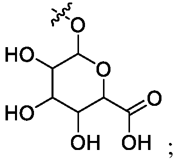
R₁ is a leaving group, preferably R₁ is halogen (e.g., chlorine),

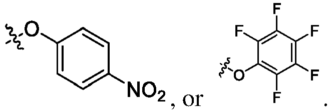

In some embodiments, L^{b} is -OH,

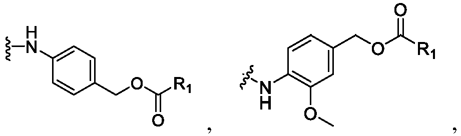

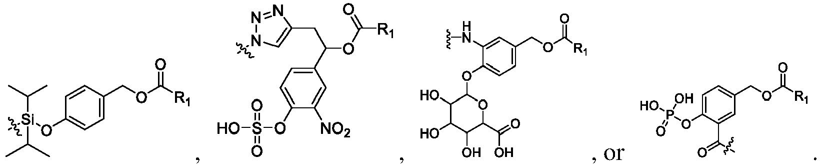

In some embodiments, when n⁵ is 1, n² and n⁴ are both 0.

In some embodiments, L^{a} is 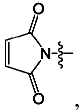 and n¹, n⁵, and n⁴ are all 0.

In some embodiments, L^{a} is 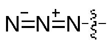 , and n² is 1 or 2.

In some embodiments, AA is each independently an amino acid residue formed by Val, Cit, Glu, or Ala. In some embodiments, -(AA)n³- is a peptide residue selected from the following group: -Val-Cit-, -Glu-Val-Cit-, -Ala-Cit-, and -Val-Ala-.

In some embodiments, the compound has the following structure:

L^{a}-(EG)n¹-(CH₂)n²-(C=O)-(AA)n³-L^{b}

wherein,
L^{a} is 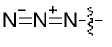 or 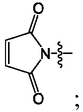
EG is -CH₂CH₂O-; n¹ is an integer of 0-4;
n² is an integer of 0-5;
AA is each independently an amino acid residue; n³ is an integer of 0-3;
L^{b} is 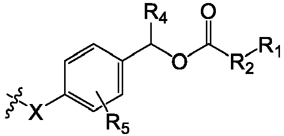 or -OH,
wherein X is -NH-;
R₂ is a bond;
R₄ is hydrogen;
R₅ is hydrogen or -OCH₃;
R₁ is a leaving group, preferably R₁ is halogen (e.g., chlorine),

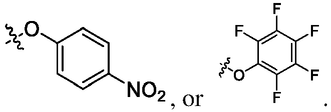

In some embodiments, L^{a} is 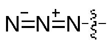 , n¹ is 2, n² is 1, and L^{b} is

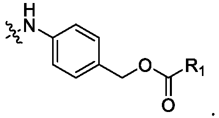

In some embodiments, L^{a} is 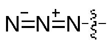 , n¹ is 2, n² is 1, and L^{b} is -OH.

In some embodiments, L^{a} is 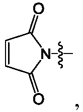 n¹ is 0, n² is 5, and L^{b} is

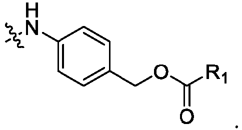

In some embodiments, L^{a} is 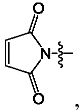 n¹ is 0, n² is 5, and L^{b} is -OH.

In some embodiments, AA is each independently Val, Cit, Glu, or Ala amino acid residue. In some embodiments, -(AA)n³- is a peptide residue selected from the following group: - Val-Cit-, -Glu-Val-Cit-, -Ala-Cit-, -Phe-Cit-, -Val-Ala-, -Gly-Gly-Phe-Gly-, -Val-Leu-Lys-, and - Gly-Asn-Asn-.

In some embodiments, the compound has a structure selected from the following group: 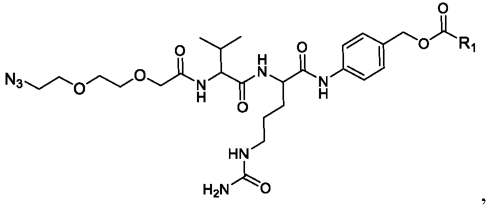 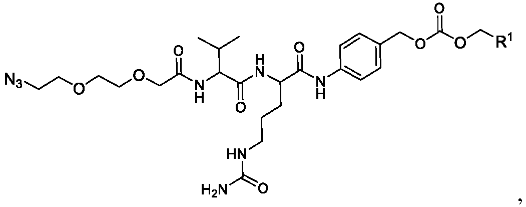 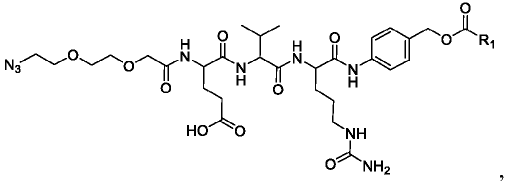 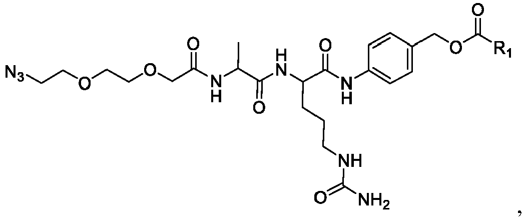 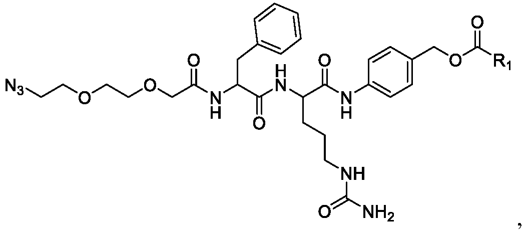 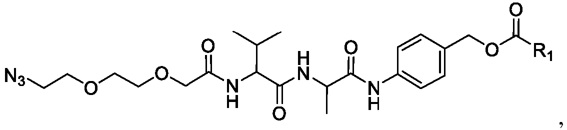 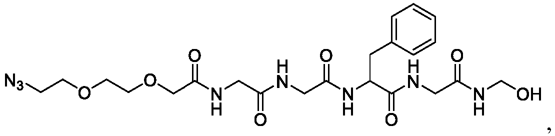 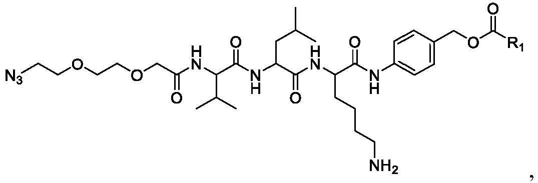 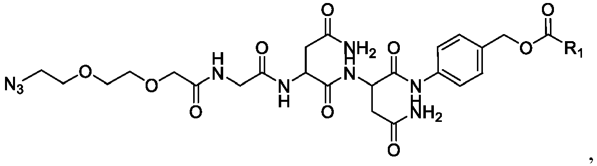 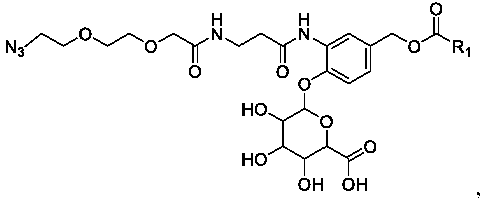 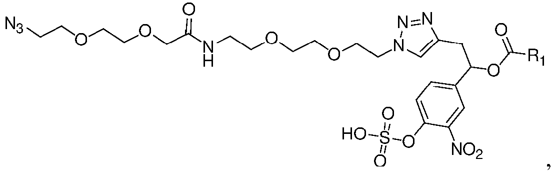 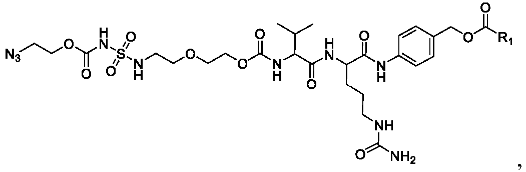 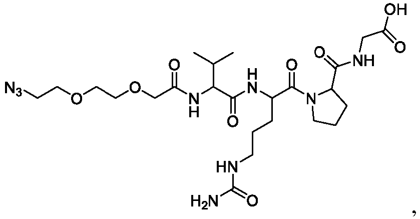 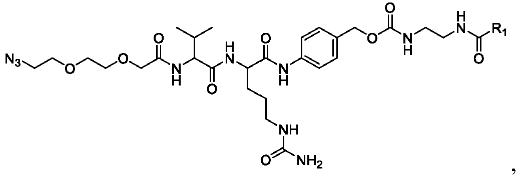 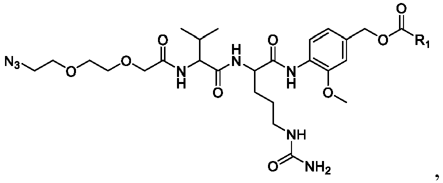 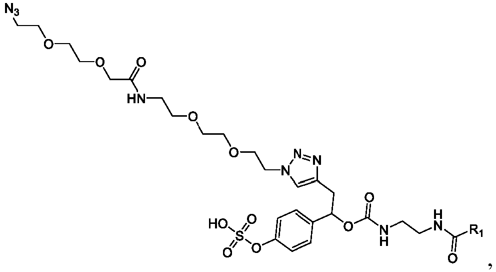 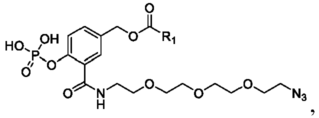 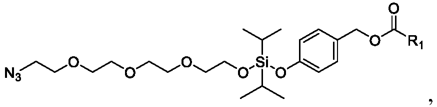 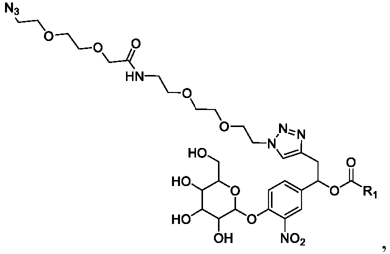 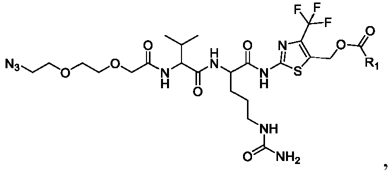 and 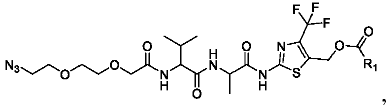 wherein R₁ is a leaving group, preferably R₁ is halogen (e.g., chlorine),

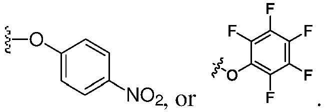

In another aspect, the present application provides a compound 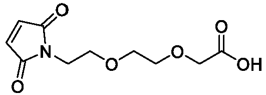 or a salt thereof.

In another aspect, the present application provides a conjugate compound or a pharmaceutically acceptable salt thereof, wherein the conjugate compound has the following structure:

W^{a}-L¹-(EG)n¹-(D)n⁵-(CH₂)n²-(B)n⁶-(AA)n³-(CH₂CH₂-Q)n⁴-L²-W^{b},

wherein,
L¹ is 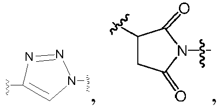 -S-S-, or a bond;
EG is -CH₂CH₂O- or -OCH₂CH₂-; n¹ is an integer of 0-6;
n² is an integer of 0-8;
D is -C(=O)-NH-S(=O)₂-NH-; n⁵ is 0 or 1;
B is each independently -C(=O)-, 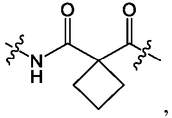 -NH-CH₂CH₂-C(=O)-, -C(=O)-NH-, -CH(CH₃)-CH₂-O-, -CH₂CH₂O-, -NH-C(=O)-, or -NH-; n⁶ is an integer of 0-3;
AA is each independently an amino acid residue; n³ is an integer of 0-5;
Q is each independently -C(=O)-, -O-, or a bond; n⁴ is an integer of 0-3;
L² is 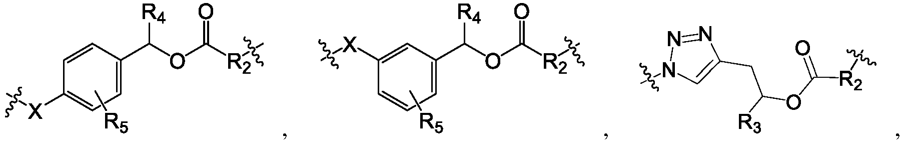 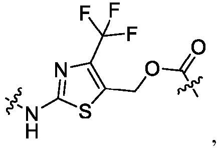 -NH-CH₂-OH, or a bond;
wherein X is a bond, -NH-, -C(=O)-, oxygen, or 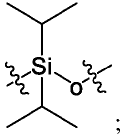
R₂ is a bond, -O-CH₂-, or -NH-CH₂CH₂-NH-C(=O)-;
R₃ is hydrogen or 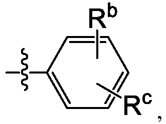 wherein R^{b} and R^{c} are each independently hydrogen, nitro, 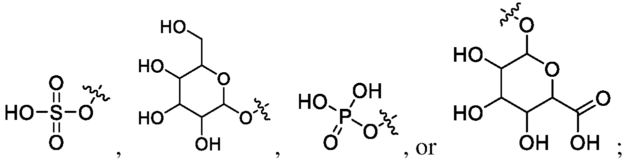
R₄ is hydrogen or C₁₋₈ alkyl;
R₅ is hydrogen, amino, nitro, phosphate group, -OR^{a}, or 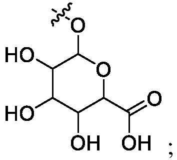 wherein R^{a} is methyl, ethyl, propyl, or isopropyl;
W^{a} and W^{b} are each independently a payload or a targeted molecule capable of binding to a cell surface protein.

In some embodiments, EG is -CH₂CH₂O-. In some embodiments, n¹ is 2. In some embodiments, n¹ is 0. In some embodiments, n² is 1. In some embodiments, n² is 4. In some embodiments, n² is 5. In some embodiments, n⁵ is 0. In some embodiments, n⁶ is 1. In some embodiments, n⁶ is 3. In some embodiments, n¹ is 2, n² is 1, n³ and n⁴ are 0, and L² is a bond.

In some embodiments, AA is each independently an amino acid residue formed by Val, Cit, Glu, Leu, Lys, Ala, Gly, Phe, Gln, Pro, or Asn. In some embodiments, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Cit-, -Val-Leu-Lys-, -Val-Cit-Pro-Gly-, -Gly-Asn-Asn-, and Ala-Ala-Asn-.

In some embodiments, when n⁴ is 1, Q is -C(=O)-; when n⁴ is 2, Q are -O- and -C(=O)-, respectively; when n⁴ is 3, Q are -O-, -O-, and a bond, respectively. In some embodiments, n⁴ is 0.

In some embodiments, L² is a bond, 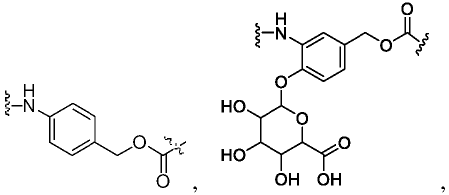 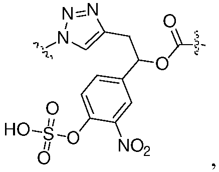 -NH-CH₂-O-, 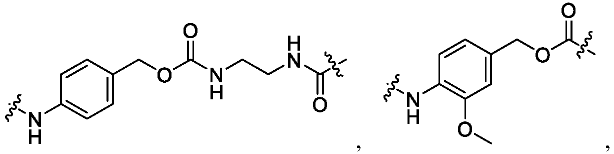 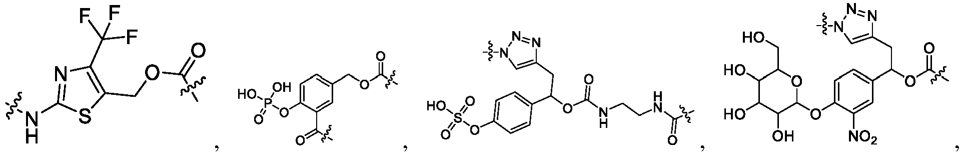 or

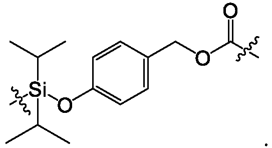

In some embodiments, the compound has the following structure:

W^{a}-L¹-(EG)n¹-(D)n⁵-(CH₂)n²-(B)n⁶-(AA)n³-(CH₂CH₂-Q)n⁴-L²-W^{b}

wherein,
L¹ is 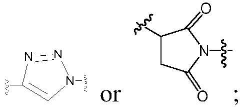
EG is -CH₂CH₂O-; n¹ is an integer of 2-4;
D is -C(=O)-NH-S(=O)₂-NH-; n⁵ is 0 or 1;
n² is an integer of 0-5;
B is -C(=O)-, -C(=O)-NH-, -CH₂CH₂O-, -NH-CH₂CH₂-C(=O)-, or -NH-; n⁶ is an integer of 0-3;
AA is each independently an amino acid residue; n³ is an integer of 0-3;
Q is each independently -C(=O)-, -O-, or a bond; n⁴ is an integer of 0-3;
L² is 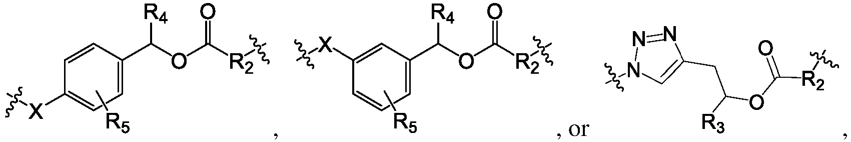
wherein X is a bond, -NH-, -C(=O)-, or 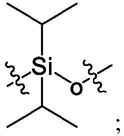
R₂ is a bond;
R₃ is 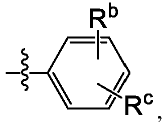 wherein R^{b} and R^{c} are each independently hydrogen, nitro, or 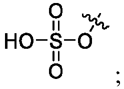
R₄ is hydrogen;
R₅ is hydrogen, -OCH₃, or 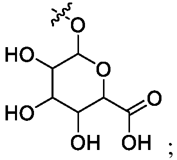
W^{a} and W^{b} are each independently a payload or a targeted molecule capable of binding to a cell surface protein.

In some embodiments, L² is

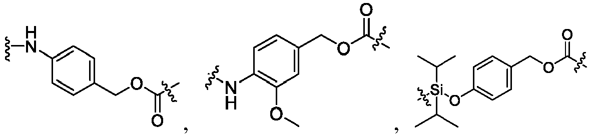

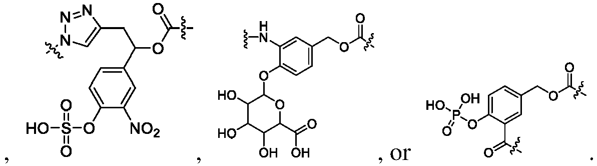

In some embodiments, when n⁵ is 1, n² and n⁴ are both 0.

In some embodiments, L¹ is 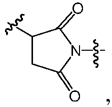 and n¹, n⁵, and n⁴ are all 0.

In some embodiments, L¹ is 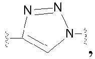 and n² is 1 or 2.

In some embodiments, AA is each independently an amino acid residue formed by Val, Cit, Glu, or Ala. In some embodiments, -(AA)n³- is a peptide residue selected from the following group: -Val-Cit-, -Glu-Val-Cit-, -Ala-Cit-, and -Val-Ala-.

In some embodiments, the compound has the following structure:

W^{a}-L¹-(EG)n¹-(CH₂)n²-(C=O)-(AA)n³-L²-W^{b}

wherein,
L¹ is 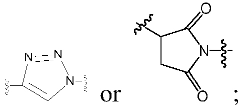
EG is -CH₂CH₂O-; n¹ is an integer of 0-4;
n² is an integer of 0-5;
AA is each independently an amino acid residue; n³ is an integer of 1-4;
L² is 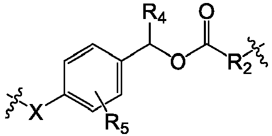 or a bond,
wherein X is -NH-;
R₂ is a bond;
R₄ is hydrogen;
R₅ is hydrogen or -OCH₃;
W^{a} and W^{b} are each independently a payload or a targeted molecule capable of binding to a cell surface protein.

In some embodiments, L¹ is 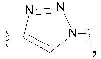 n¹ is 2, n² is 1, and L² is

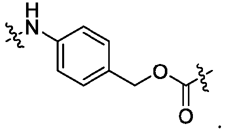

In some embodiments, L¹ is 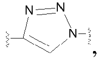 n¹ is 2, n² is 1, and L² is a bond.

In some embodiments, L¹ is 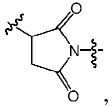 n¹ is 0, n² is 5, and L² is

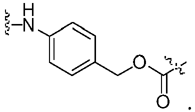

In some embodiments, L¹ is 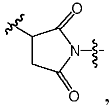 n¹ is 0, n² is 5, and L² is a bond.

In some embodiments, the conjugate compound has a structure selected from the following group: 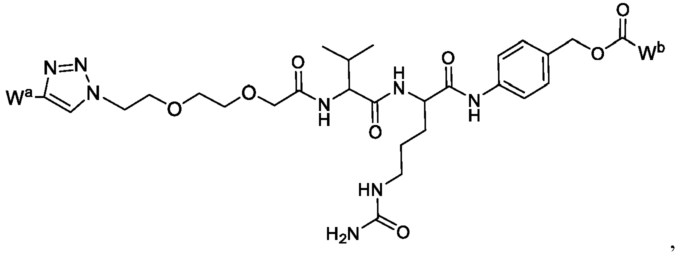 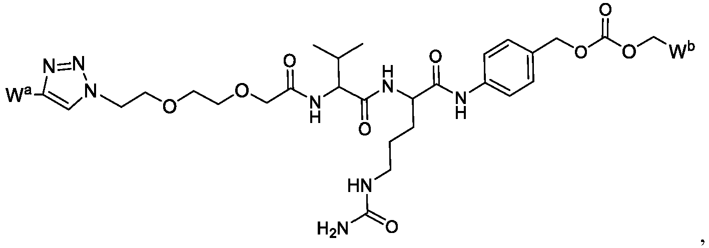 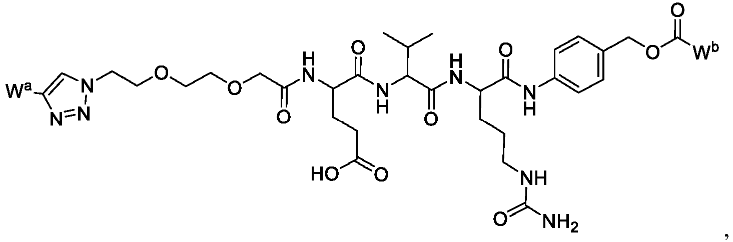 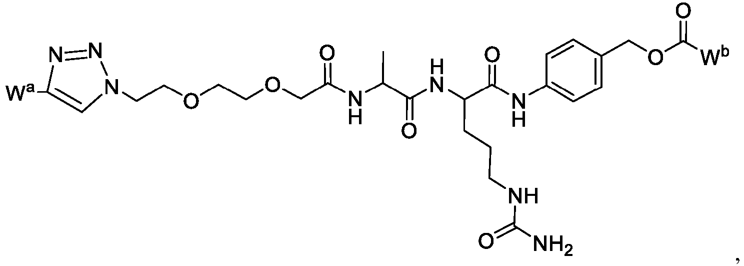 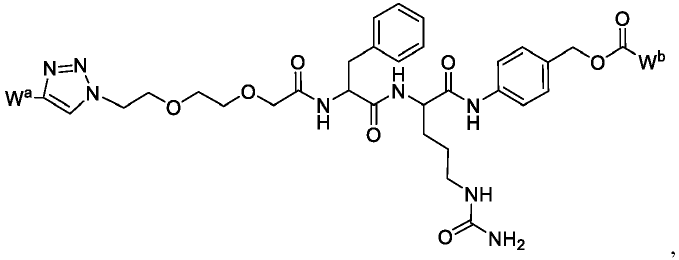 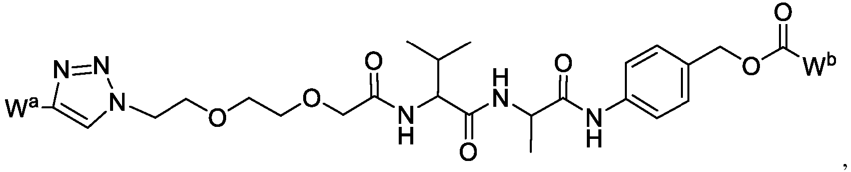 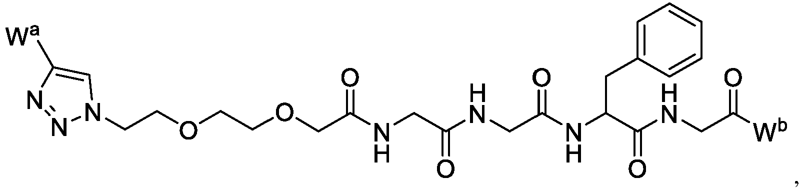 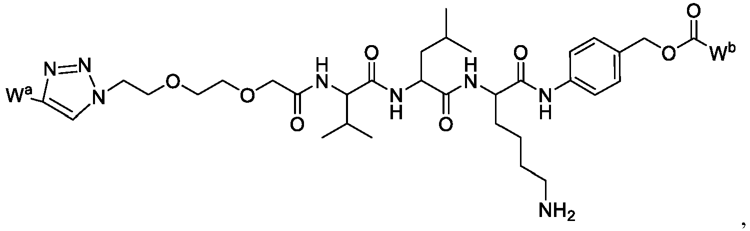 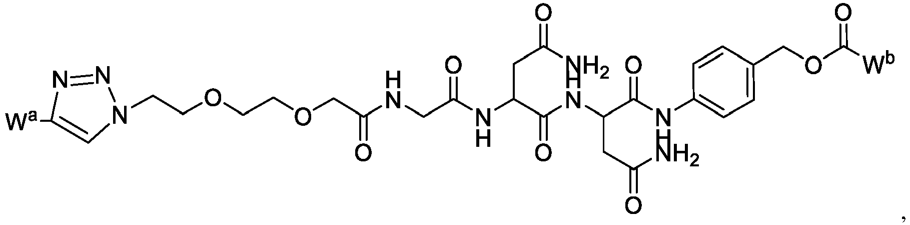 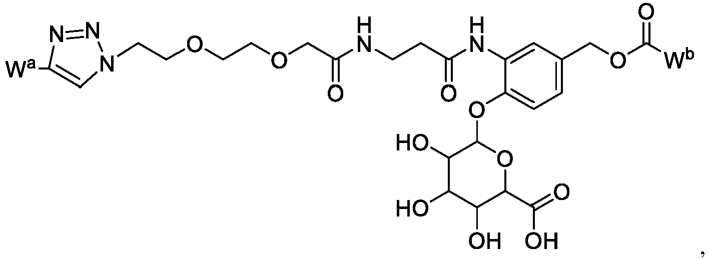 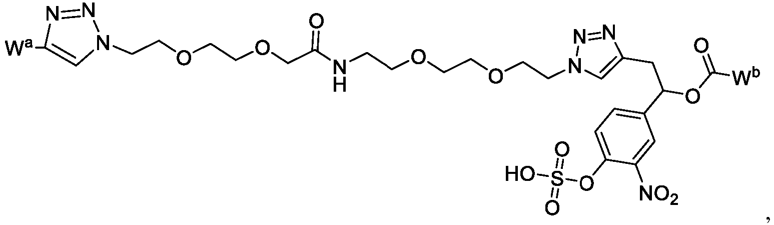 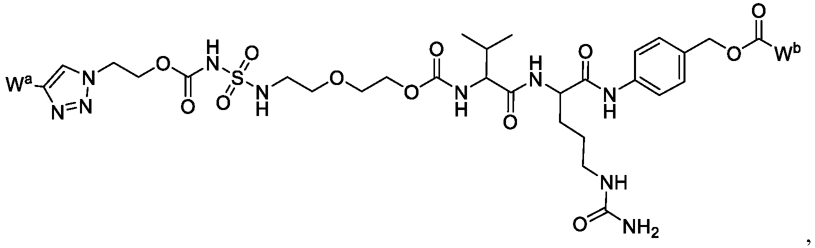 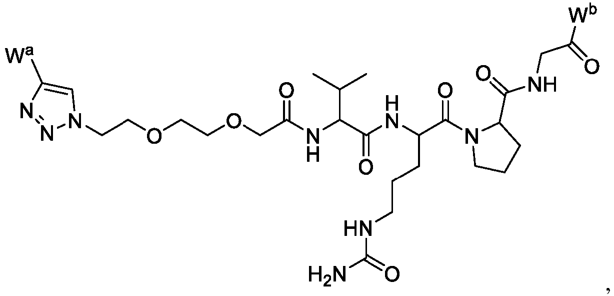 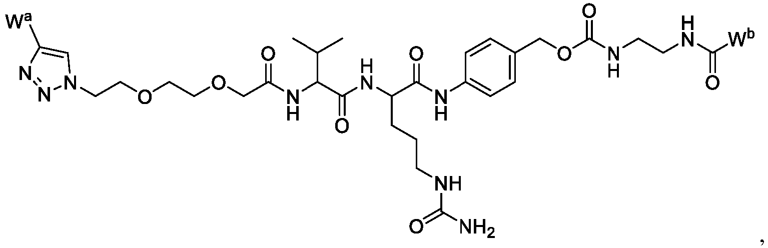 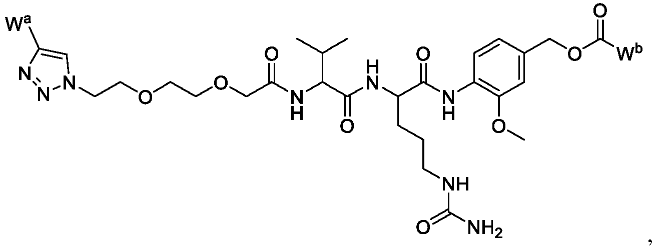 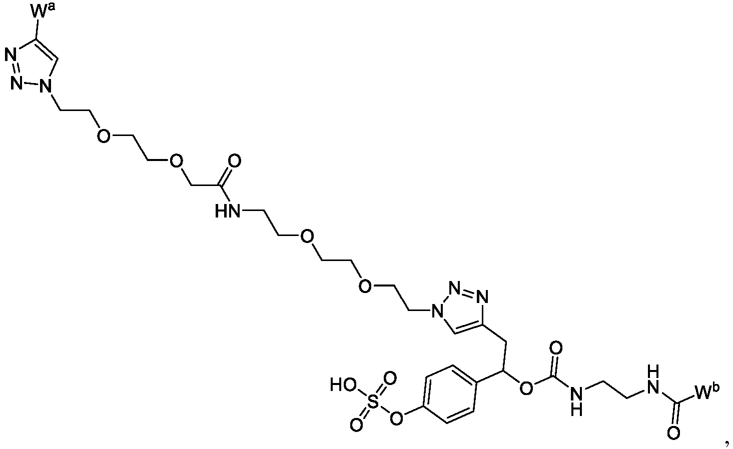 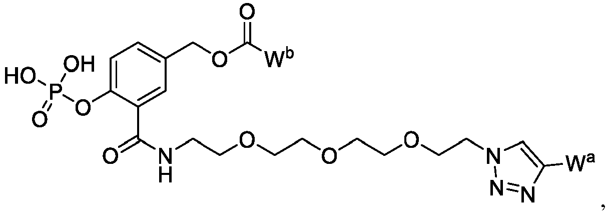 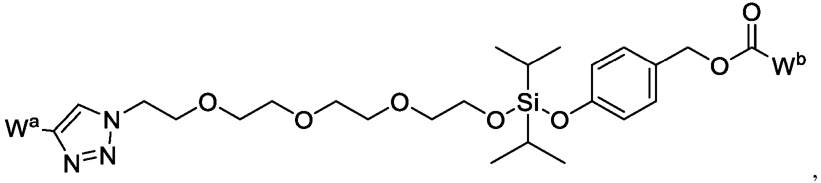 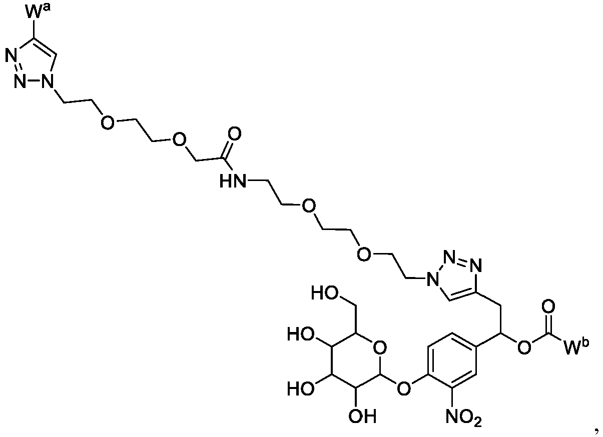 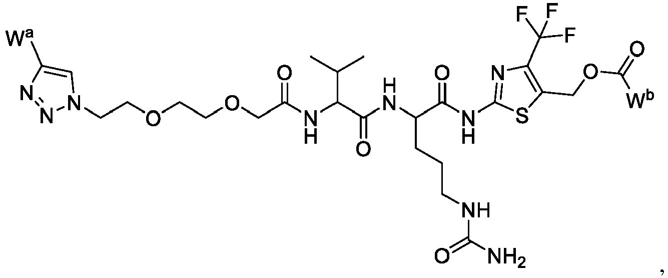 and 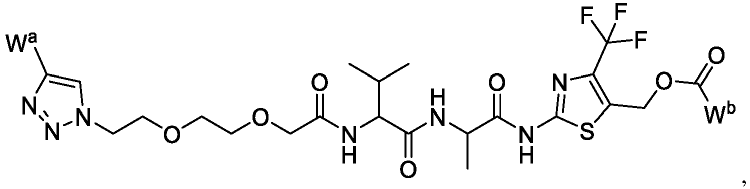 wherein W^{a} and W^{b} are each independently a payload or a targeted molecule capable of binding to a cell surface protein.

In another aspect, the present application discloses a conjugate compound or a pharmaceutically acceptable salt thereof, wherein the conjugate compound is 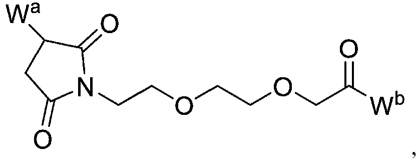 wherein W^{a} and W^{b} are each independently a payload or a targeted molecule capable of binding to a cell surface protein.

In another aspect, the present application discloses a conjugate compound or a pharmaceutically acceptable salt thereof, wherein the conjugate compound is 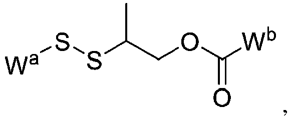 wherein W^{a} and W^{b} are each independently a payload or a targeted molecule capable of binding to a cell surface protein.

In another aspect, the present application provides a proteolysis targeting chimera or a pharmaceutically acceptable salt thereof, wherein the proteolysis targeting chimera compound has the following structures: 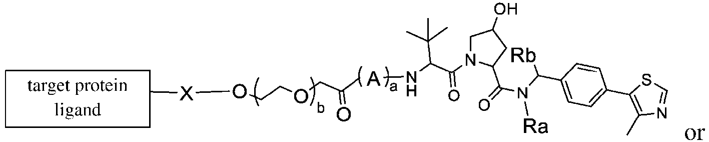 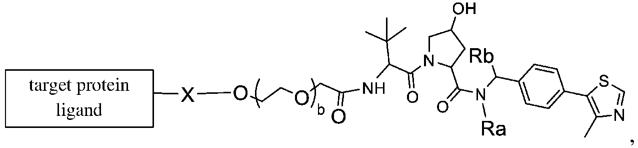 wherein,
Ra and Rb are each independently hydrogen, alkyl, NH₂, OH, or halogen;
A is each independently an amino acid residue;
a is 0 or 1;
b is an integer of 0-5; 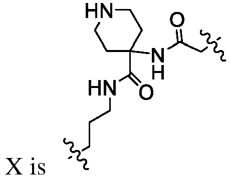 or a bond;
the target protein ligand is

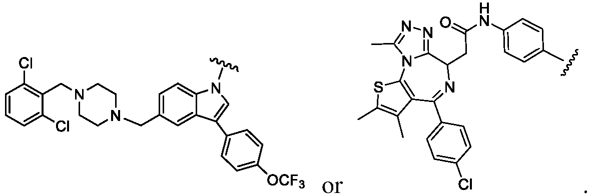

In some embodiments, Ra is hydrogen. In some embodiments, Rb is hydrogen. In some embodiments, A is an amino acid residue formed by Lys. In some embodiments, a is 1. In some embodiments, a is 0. In some embodiments, b is 1. In some embodiments, b is 2. In some embodiments, b is 3. In some embodiments, b is 4. In some embodiments, b is 5.

In some embodiments, the proteolysis targeting chimera has a structure selected from the following group: 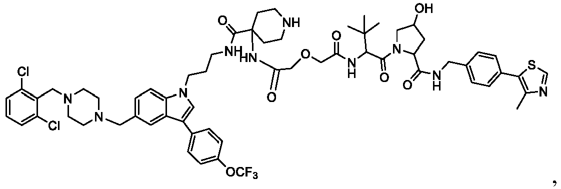 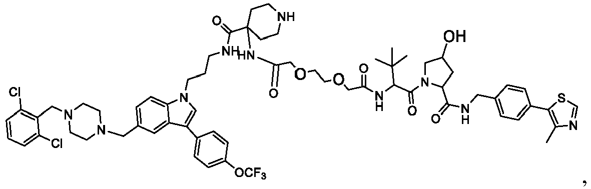 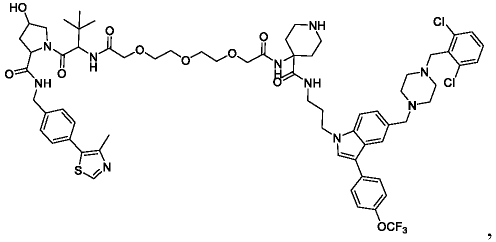 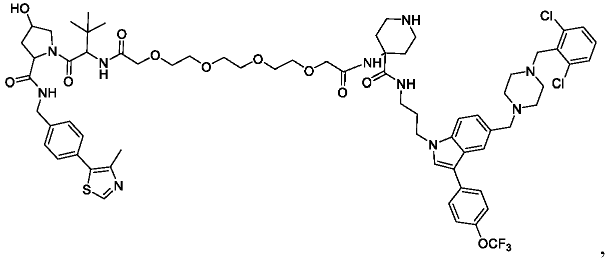 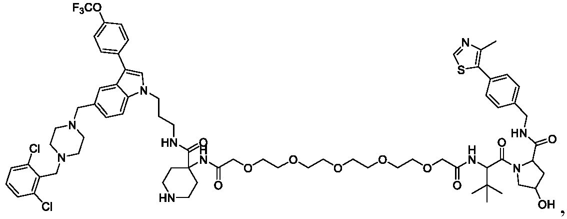 and

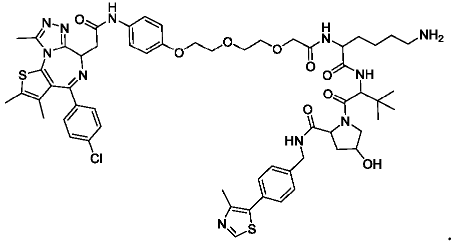

### BRIEF DESCRIPTION OF THE DRAWINGS

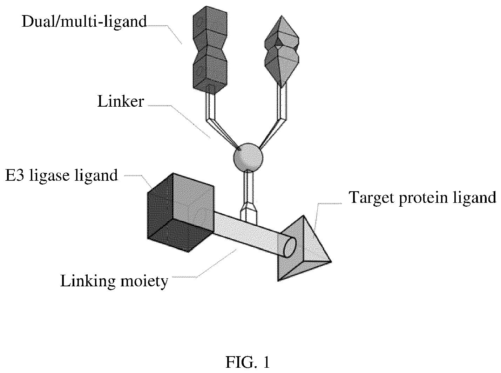

FIG. 1 illustrates an exemplary conjugate compound of the present application.

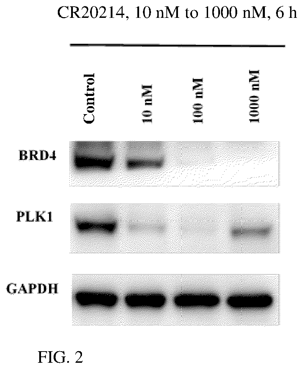

FIG. 2 illustrates the results of Western Blot assay for degradation of target protein by compound CR20214.

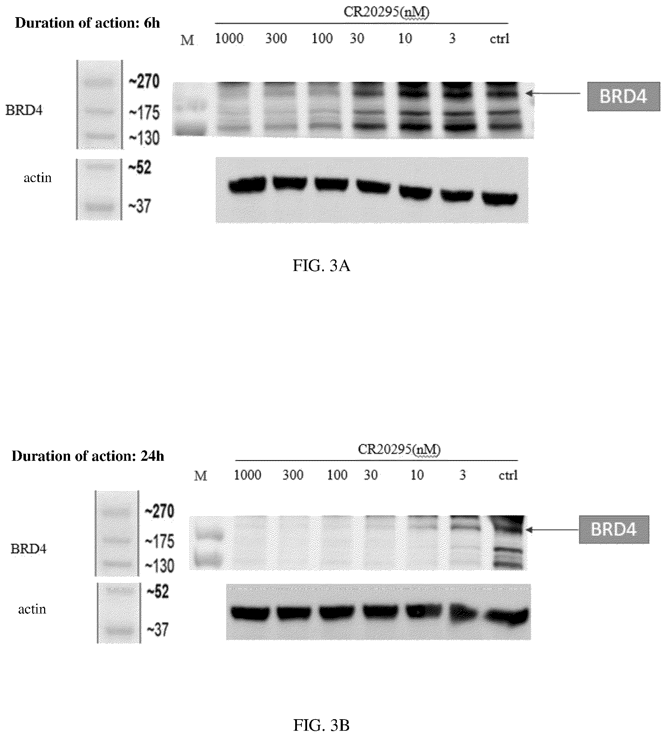

FIG. 3 illustrates the results of Western Blot assay for degradation of target protein by compound CR20295.

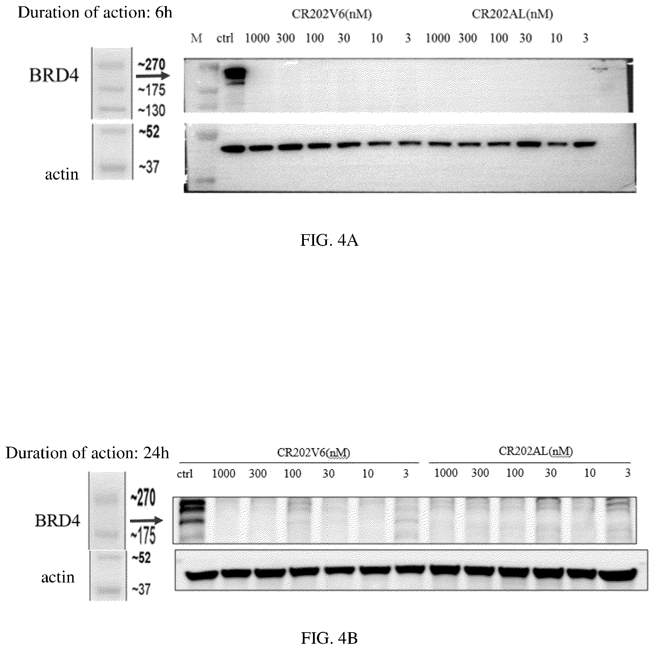

FIG. 4 illustrates the results of Western Blot assay for degradation of target protein by compounds CR202V6 and CR202AL.

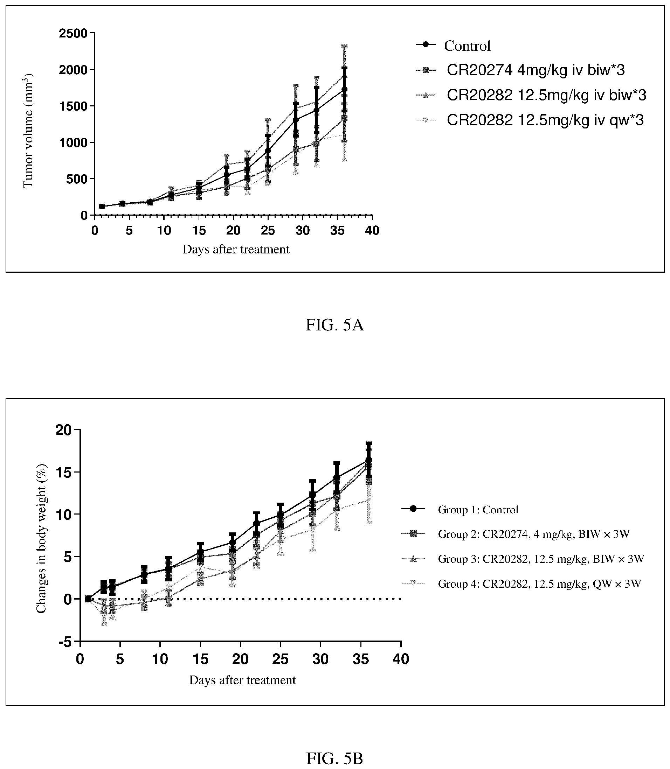

FIG. 5 illustrates the change in tumor volume in human liver cancer HepG2 mouse model (FIG. 5A) and the change from baseline over time in body weight in human liver cancer HepG2 mouse model (FIG. 5B) after the administration of reference, CR20274, and the conjugate compound of the present application.

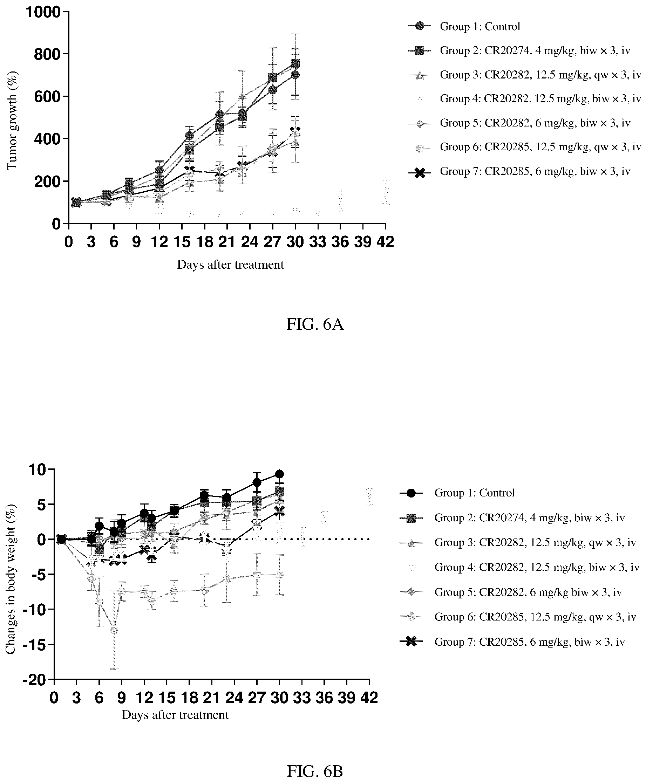

FIG. 6 illustrates the change in tumor volume in human breast cancer MDA-MB-231 mouse model (FIG. 6A) and the change from baseline over time in body weight in human breast cancer MDA-MB-231 mouse model (FIG. 6B) after the administration of reference, CR20274, and the conjugate compound of the present application.

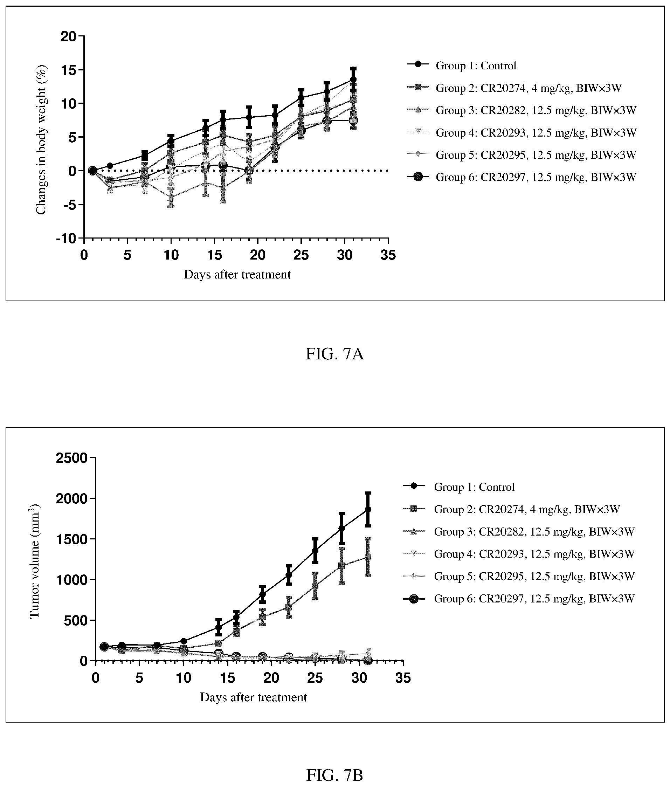

FIG. 7 illustrates the change from baseline over time in body weight in human prostate cancer 22RV1 mouse model (FIG. 7A) and the change in tumor volume in human prostate cancer 22RV1 mouse model (FIG. 7B) after the administration of reference, CR20274, and the conjugate compound of the present application.

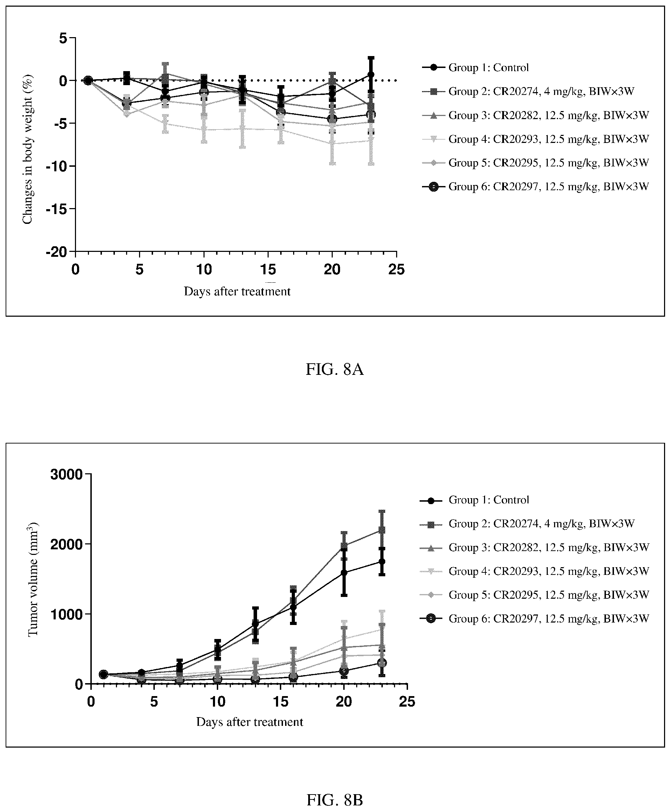

FIG. 8 illustrates the change from baseline over time in body weight in human prostate cancer C4-2 mouse model (FIG. 8A) and the change in tumor volume in human prostate cancer C4-2 mouse model (FIG. 8B) after the administration of reference, CR20274, and the conjugate compound of the present application.

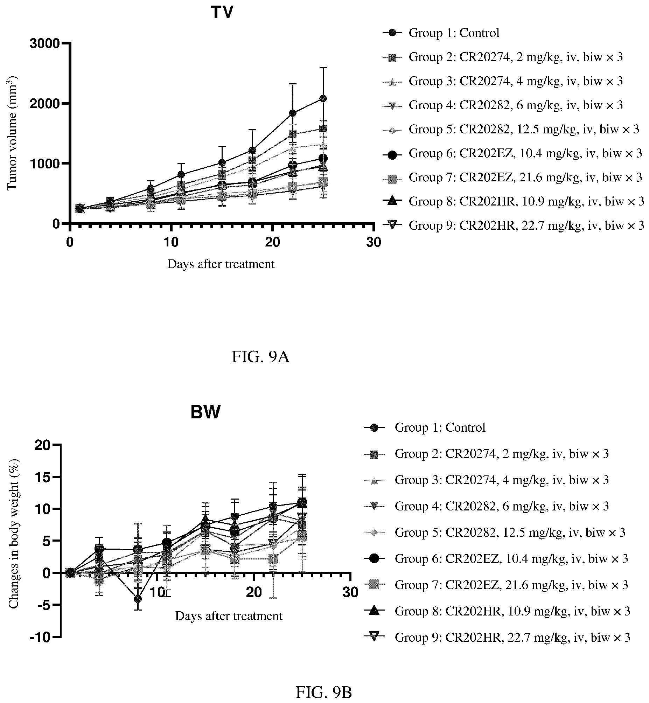

FIG. 9 illustrates the change in tumor volume in human colon cancer HT-29 mouse model (FIG. 9A) and the change from baseline over time in body weight in human colon cancer HT-29 mouse model (FIG. 9B) after the administration of reference, CR20274, and the conjugate compound of the present application.

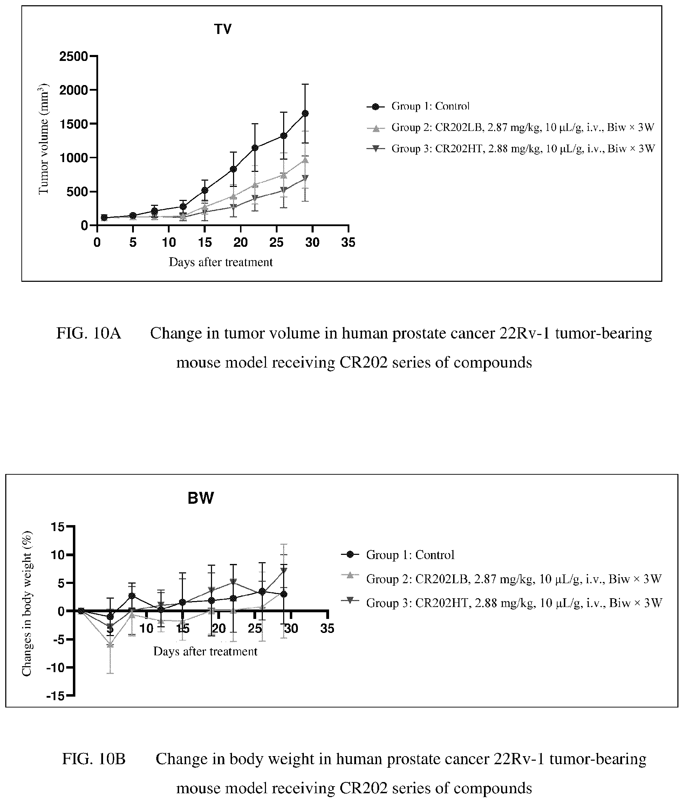

FIG. 10 illustrates the change in tumor volume in human prostate cancer 22RV1 mouse model (FIG. 10A) and the change from baseline over time in body weight in human prostate cancer 22RV1 mouse model (FIG. 10B) after the administration of reference, CR20274, and the conjugate compound of the present application.

### DETAILED DESCRIPTION

The following description is merely illustrative of various embodiments of the present application. Therefore, the specific embodiments herein should not be construed as limiting the scope of the present application. It will be appreciated that various equivalents and modifications readily attainable by those skilled in the art from the spirit and description of the present invention shall fall within the scope of the present invention. All documents, including publications, patents, and patent applications, cited in the present application are herein incorporated by reference in their entireties.

As used herein, the singular forms "a," "an," and "the" include plural references, unless otherwise explicitly indicated in the context.

As used herein, the terms such as "comprise," "include," "contain," and "having" are inclusive or open-ended and do not exclude additional, unrecited elements or procedures. The term "consisting of" is closed-ended.

In one aspect, the present application discloses a conjugate compound or a pharmaceutically acceptable salt thereof, comprising: a targeted molecule comprising at least two ligands specifically binding to a cell surface protein and a payload linked to the targeted molecule, wherein the payload is a proteolysis targeting chimera.

The term "targeted molecule" used herein refers to any molecule or moiety capable of targeting a target site, target tissue, target organ, target cell, or target intracellular region. In some embodiments, the targeted molecule is such that a moiety linked to the targeted molecule is distributed more at the target site, target tissue, target organ, target cell, or target intracellular region, e.g., at least 10%, 20%, 50%, 80%, 100%, 150%, 200%, 300%, 400%, 500% or greater, than a non-target site, non-target tissue, non-target organ, non-target cell, or non-target intracellular region. In some embodiments, the targeted molecule is such that a conjugate compound or agent carrying the targeted molecule is distributed more at the target site, target tissue, target organ, target cell, or target intracellular region, e.g., at least 10%, 20%, 50%, 80%, 100%, 150%, 200%, 300%, 400%, 500% or greater, than one without the targeted molecule. In some embodiments, the targeted molecule is capable of triggering or promoting the specific binding of a conjugate compound containing the targeted molecule to a target molecule, triggering or promoting the endocytosis of the conjugate compound in a target cell, or triggering or promoting the enrichment of the conjugate compound around and/or into a target cell.

The term "ligand" used herein may include a wide variety of chemical molecules or polypeptides having specific binding affinity for a selected target which may be a cell surface protein (e.g., a cell surface receptor or cell surface antigen), a specific protein, cell, tissue, organ, etc. In some embodiments, the ligand can specifically bind to a cell surface receptor. In some embodiments, the ligand can specifically bind to a cell surface antigen. In some embodiments, the ligand can specifically bind to a specific protein which may cause a disease. In some embodiments, the overexpression of the specific protein may cause a disease or the specific protein is a mutant protein that may cause a disease. In some embodiments, the ligand of the present application binds to a target with an affinity of 10⁻⁶ to 10⁻¹¹ M (K_{d} value). In some embodiments, the ligand of the present application binds to a target with an affinity of at least 10⁻⁶, at least 10⁻⁷, at least 10⁻⁸, or at least 10⁻⁹ M (K_{d} value). In some embodiments, the ligand of the present application binds to a target with an affinity that is at least two, three, four, five, six, eight, ten, twenty, fifty, one hundred, or more times greater than the affinity of the ligand for a non-target (e.g., other cell surface receptors, cell surface antigens, or specific proteins, etc.). In some embodiments, the expression level of the cell surface receptor, cell surface antigen, or specific protein of the present application on the surface of or within a target cell (e.g., a cancer cell or a physiologically dysfunctional cell) is significantly higher than the expression level in a normal cell. The term "significant" used herein refers to statistically significant differences, or significant differences as may be recognized by those skilled in the art.

In some embodiments, the expression level of the cell surface receptor, cell surface antigen, or specific protein of the present application on the surface of or within a target cell (e.g., a cancer cell or a physiologically abnormal cell) is 2-fold to 1,000,000-fold greater than the expression level in a normal cell; for example, the expression level in a target cell (e.g., a cancer cell or a physiologically abnormal cell) is 2-fold to 10-fold, 2-fold to 100-fold, 2-fold to 1,000-fold, 2-fold to 10,000-fold, 2-fold to 100,000-fold, or 2-fold to 1,000,000-fold greater than the expression level in a normal cell (which may be equal to any value within the above numerical range, including the endpoints of the range). In some embodiments, the expression level of the cell surface receptor in a target cell (e.g., a cancer cell or a physiologically dysfunctional cell) is at least 10-fold, at least 100-fold, at least 1,000-fold, at least 10,000-fold, or at least 100,000-fold greater than the expression level in a normal cell. In some embodiments, the expression level of the cell surface receptor, cell surface antigen, or specific protein on a normal cell is reduced by at least 50%, 60%, 70%, 80%, 90%, 95%, or 99% as compared to the expression level of the cell surface receptor, cell surface antigen, or specific protein on a target cell (e.g., a cancer cell or a physiologically dysfunctional cell). In some embodiments, the cell surface receptor, cell surface antigen, or specific protein described herein is not detected in normal cells.

In some embodiments, the cell surface receptor is selected from FOLR1, TRPV6, or c-Met. In some embodiments, the cell surface antigen is selected from PSMA.

In some embodiments, the specific protein is selected from a nuclear receptor (e.g., AR and ER), a kinase-like protein (e.g., RIPK2, BCR-ABL, EGFR, HER2, c-Met, TBK1, CDK2/4/6/9, ALK, Akt, CK2, ERK1/2, FLT3, PI3K, BTK, and FAK), AKT, PAN-BET protein, BET protein (e.g., BRD2, BRD3, BRD4, and BRD6), BLK, BRAF1, BCL-xl, ERRα, FKBP12, FRS2α, JAK1, JAK3, IKZF1, IRAK4, MDM2, MetAP2, PLK1, PSK-J3, RAS, TACC3, Tau, TrkB, MDM2, or any combination thereof. In some embodiments, the specific protein is selected from AR protein, ER protein, RAS protein, BRD4 protein, PLK1 protein, FAK protein, MDM2 protein, or any combination thereof. In some embodiments, the target protein ligand moiety in the proteolysis targeting chimera targets the specific protein.

Folate receptor 1 (FOLR1) is a glycosylphosphatidylinositol (GPI)-anchored glycoprotein that binds to folates with a nanomolar affinity and thus promotes the receptor-mediated endocytosis. Rapidly growing solid malignancies, including ovarian and lung cancers, depend on folate for metabolism and nucleic acid synthesis. In some embodiments, the targeted molecule described herein comprises a ligand targeting FOLR1.

The ligand specifically binding to FOLR1 or "FOLR1 ligand" refers to an antibody, peptide, aptamer, small molecule, etc., that specifically recognizes and binds to FOLR1. The FOLR1 ligand of the present application includes FOLR1 ligands that exist at present or may be developed in the future. In some embodiments, the FOLR1 ligand includes folic acid or an analog thereof and pteroic acid. The term "analog" used herein includes both structural and functional analogs. Structural analogs refer to a class of compounds having similar chemical structures, between which one or more different atoms, or one or more different functional groups, may be present. Functional analogs refer to a class of compounds having the same or similar chemical, biological, or pharmacological effects. In some embodiments, the folic acid analog is selected from the following group: 5-methyltetrahydrofolic acid, 5-formyltetrahydrofolic acid, 10-formyltetrahydrofolic acid, methotrexate, 5,10-formyltetrahydrofolic acid, 5,10-methenyltetrahydrofolic acid, aminopterin, and raltitrexed.

Transient receptor potential channel subfamily V member 6, or TRPV6, is a highly selective calcium ion transmembrane transport channel that mediates the active transport of calcium ions from the outside to the inside of cells. TRPV6 is expressed in human normal kidneys, gastrointestinal tracts, pancreas, breasts, salivary glands, etc., but is mainly expressed in intestinal epithelial cells. It is involved in the transport of calcium ions into cells, and thus when the quantity or functionality of the TRPV6 channel is changed, causes changes in calcium ion regulation, further resulting in structural or functional abnormality in associated tissues and organs. Compared with normal tissues, the expression of TRPV6 is significantly increased in malignant tumors such as breast cancer, bile duct cancer, ovarian cancer, squamous cell lung cancer, prostatic cancer, and the like, and the abnormal expression of TRPV6 is possibly related to tumorigenesis and the progression of tumors.

The ligand specifically binding to TRPV6 or "TRPV6 ligand" refers to an antibody, peptide, aptamer, small molecule, etc., that specifically recognizes and binds to TRPV6. The TRPV6 ligand of the present application includes TRPV6 ligands that exist at present or may be developed in the future. In some embodiments, the TRPV6 ligand comprises the amino acid sequence set forth in SEQ ID NO: 1 (KEFLHPSKVDLPR), the amino acid sequence set forth in SEQ ID NO: 2 (EGKLSSNDTEGGLCKEFLHPSKVDLPR), or an amino acid sequence having at least 80%, 85%, 90%, 95%, 96%, 97%, 98%, or 99% sequence identity to SEQ ID NO: 1 or SEQ ID NO: 2.

Prostate-specific membrane antigen (PSMA), a type II transmembrane glycoprotein present on prostate epithelial cell membranes, consists of 750 amino acids with 19 intracellular amino acids, 24 transmembrane amino acids, and 707 extracellular amino acids. Prostate-specific membrane antigen is expressed in normal prostate epithelial cells, but at much higher levels in prostate cancer cells. Compared with the conventional prostate-specific antigen used for clinical assays, prostate-specific membrane antigen is a more sensitive and specific prostate cancer tumor marker. It is especially highly expressed in hormone-refractory prostate cancer and prostate cancer metastasis, and has high sensitivity and specificity in distinguishing prostate cancer from other malignant tumors. Meanwhile, prostate-specific membrane antigen is also specifically expressed in tumor vascular endothelial cells in a variety of solid tumors of non-prostate origin (e.g., lung, bladder, gastric, pancreatic, renal, and colorectal cancers, etc.).

The ligand specifically binding to prostate-specific membrane antigen or "prostate-specific membrane antigen ligand" refers to an antibody, peptide, aptamer, small molecule, etc., that specifically recognizes and binds to prostate-specific membrane antigen. The prostate-specific membrane antigen ligand of the present application includes prostate-specific membrane antigen ligands that exist at present or may be developed in the future, as well as fragments of the foregoing ligands, so long as the fragments retain the ability to bind to prostate-specific membrane antigen. Antibody ligands are the most common prostate-specific membrane antigen ligands, including but not limited to monoclonal antibodies J591, J533, J415, and E99 (see, e.g., Liu H, Rajasekaran AK, Moy P, et al, Constitutive and antibody-induced internalization of prostate-specific membrane antigen [J]. Cancer Res., 1998, 58 (18):4055-4060). The aptamer is single-stranded DNA or RNA obtained by technical screening through an exponential enrichment ligand system and capable of binding to the prostate-specific membrane antigen with high affinity and high specificity, and the prostate-specific membrane antigen ligand includes but is not limited to xPSM-A10 aptamer and derivatives thereof and xPSM-A9 aptamer and derivatives thereof (see, e.g., Lupoid SE, et al, Identification and Characterization of nuclease-stabilized RNA molecules that bind human prostate cancer cells via the prostate-specific membrane antigen, Cancer Res., 2002, 62(14):4029-4033). Compared with antibody ligands and aptamer ligands, small molecule prostate-specific membrane antigen ligands possess the advantages of low molecular weight, high permeability, low immunogenicity, ease of synthesis, and the like, and such ligands include, but are not limited to, small molecule glutamine urea ligands and small molecule phosphoramidate ligands.

The terms "polypeptide", "protein", and "peptide" used herein in the present application may be a monomer or a polymer of amino acids. The polypeptide, protein, or peptide as described herein may comprise native amino acids, and non-native amino acids, or analogs and mimetics of amino acids. The polypeptide, protein, or peptide may be obtained by any method well known in the art, for example, without limitation, isolation and purification from natural substances, recombinant expression, chemical synthesis, and the like. In some embodiments, the polypeptide comprises 2-50, 2-40, 2-30, 2-25, 2-22, 2-20, 2-18, 2-15, 2-12, 2-10, 2-8, 4-50, 5-50, 5-40, 5-30, 5-25, 5-22, 5-20, 5-18, 5-15, 5-12, 5-10, 6, 7, 8, or 9 amino acids.

As used herein, the "small molecule ligand" refers to a compound having a molecular weight of about 3 kDa or less. In some embodiments, the micormolecular compound has a molecular weight of about 2 kDa or less. In some embodiments, the micormolecular compound has a molecular weight of about 1.5 kDa or less. In some preferred embodiments, the micormolecular compound has a molecular weight of about 1 kDa or less, 800 Da or less, 700 Da or less, 600 Da or less, or 500 Da or less.

In some embodiments, the small molecule prostate-specific membrane antigen ligand of the present application may be selected from the group consisting of: 2-[[methylhydroxyphosphinyl]methyl]pentanedioic acid; 2-[[ethylhydroxyphosphinyl]methyl]pentanedioic acid; 2-[[propylhydroxyphosphinyl]methyl]pentanedioic acid; 2-[[butylhydroxyphosphinyl]methyl]pentanedioic acid; 2-[[cyclohexylhydroxyphosphinyl]methyl]pentanedioic acid; 2-[[phenylhydroxyphosphinyl]methyl]pentanedioic acid; 2-[[2-(tetrahydrofuryl)hydroxyphosphinyl]methyl]pentanedioic acid; 2-[[(2-tetrahydropyranyl)hydroxyphosphinyl]methyl]pentanedioic acid; 2-[[((4-pyridyl)methyl)hydroxyphosphinyl]methyl]pentanedioic acid; 2-[[((2-pyridyl)methyl)hydroxyphosphinyl]methyl]pentanedioic acid; 2-[[(phenylmethyl)hydroxyphosphinyl]methyl]pentanedioic acid; 2-[[((2-phenylethyl)methyl)hydroxyphosphinyl]methyl]pentanedioic acid; 2-[[((3-phenylpropyl)methyl)hydroxyphosphinyl]methyl]pentanedioic acid; 2-[[((3-phenylbutyl)methyl)hydroxyphosphinyl]methyl]pentanedioic acid; 2-[[((2-phenylbutyl)methyl)hydroxyphosphinyl]methyl]pentanedioic acid; 2-[[(4-phenylbutyl)hydroxyphosphinyl]methyl]pentanedioic acid; 2-[[(aminomethyl)hydroxyphosphinyl]methyl]pentanedioic acid; 2-[[methylhydroxyphosphinyl]oxo]pentanedioic acid; 2-[[ethylhydroxyphosphinyl]oxo]pentanedioic acid; 2-[[propylhydroxyphosphinyl]oxo]pentanedioic acid; 2-[[butylhydroxyphosphinyl]oxo]pentanedioic acid; 2-[[phenylhydroxyphosphinyl]oxo]pentanedioic acid; 2-[[((4-pyridyl)methyl)hydroxyphosphinyl]oxo]pentanedioic acid; 2-[[((2-pyridyl)methyl)hydroxyphosphinyl]oxo]pentanedioic acid; 2-[[(phenylmethyl)hydroxyphosphinyl]oxo]pentanedioic acid; 2[[((2-phenylethyl)methyl)hydroxyphosphinyl]oxo]pentanedioic acid; 2-[[(N-hydroxy)carbamoyl]methyl]pentanedioic acid; 2-[[(N-hydroxy-N-methyl)carbamoyl]methyl]pentanedioic acid; 2-[[(N-butyl-N-hydroxy)carbamoyl]methyl]pentanedioic acid; 2-[[(N-benzyl-N-hydroxy)carbamoyl]methyl]pentanedioic acid; 2-[[(N-hydroxy-N-phenyl)carbamoyl]methyl]pentanedioic acid; 2-[[(N-hydroxy-N-2-phenylethyl)carbamoyl]methyl]pentanedioic acid; 2-[[(N-ethyl-N-hydroxy)carbamoyl]methyl]pentanedioic acid; 2-[[(N-hydroxy-N-propyl)carbamoyl]methyl]pentanedioic acid; 2-[[(N-hydroxy-N-3-phenylpropyl)carbamoyl]methyl]pentanedioic acid; 2-[[(N-hydroxy-N-4-pyridyl)carbamoyl]methyl]pentanedioic acid; 2-[[(N-hydroxy)carbamoyl]methyl]pentanedioic acid; 2-[[N-hydroxy(methyl)carbamoyl]methyl]pentanedioic acid; 2-[[N-hydroxy(benzyl)carbamoyl]methyl]pentanedioic acid; 2-[[N-hydroxy(phenyl)carbamoyl]methyl]pentanedioic acid; 2-[[N-hydroxy(2-phenylethyl)carbamoyl]methyl]pentanedioic acid; 2-[[N-hydroxy(ethyl)carbamoyl]methyl]pentanedioic acid; 2-[[N-hydroxy(propyl)carbamoyl]methyl]pentanedioic acid; 2-[[N-hydroxy(3-phenylpropyl)carbamoyl]methyl]pentanedioic acid; 2-[[N-hydroxy(4-pyridyl)carbamoyl]methyl]pentanedioic acid; 2-[(sulfinyl)methyl]pentanedioic acid; 2-[(methylsulfinyl)methyl]pentanedioic acid; 2-[(ethylsulfinyl)methyl]pentanedioic acid; 2-[(propylsulfinyl)methyl]pentanedioic acid; 2-[(butylsulfinyl)methyl]pentanedioic acid; 2-[(phenylsulfinyl]methyl]pentanedioic acid; 2-[[(2-phenylethyl)sulfinyl]methyl]pentanedioic acid; 2-[[(3-phenylpropyl)sulfinyl]methyl]pentanedioic acid; 2-[[(4-pyridyl)sulfinyl]methyl]pentanedioic acid; 2-[(benzylsulfinyl)methyl]pentanedioic acid; 2-[(sulfonyl)methyl]pentanedioic acid; 2-[(methylsulfonyl)methyl]pentanedioic acid; 2-[(ethylsulfonyl)methyl]pentanedioic acid; 2-[(propylsulfonyl)methyl]pentanedioic acid; 2-[(butylsulfonyl)methyl]pentanedioic acid; 2-[(phenylsulfonyl]methyl]pentanedioic acid; 2-[[(2-phenylethyl)sulfonyl]methyl]pentanedioic acid; 2-[[(3-phenylpropyl)sulfonyl]methyl]pentanedioic acid; 2-[[(4-pyridyl)sulfonyl]methyl]pentanedioic acid; 2-[(benzylsulfonyl)methyl]pentanedioic acid; 2-[(sulfoximinyl)methyl]pentanedioic acid; 2-[(methylsulfoximinyl)methyl]pentanedioic acid; 2-[(ethylsulfoximinyl)methyl]pentanedioic acid; 2-[(propylsulfoximinyl)methyl]pentanedioic acid; 2-[(butylsulfoximinyl)methyl]pentanedioic acid; 2-[(phenylsulfoximinyl]methyl]pentanedioic acid; 2-[[(2-phenylethyl)sulfoximinyl]methyl]pentanedioic acid; 2-[[(3-phenylpropyl)sulfoximinyl]methyl]pentanedioic acid; 2-[[(4-pyridyl)sulfoximinyl]methyl]pentanedioic acid; 2-[(benzylsulfoximinyl)methyl]pentanedioic acid; N-[methylhydroxyphosphinyl]glutamic acid; N-[ethylhydroxyphosphinyl]glutamic acid; N-[propylhydroxyphosphinyl]glutamic acid; N-[butylhydroxyphosphinyl]glutamic acid; N-[phenylhydroxyphosphinyl]glutamic acid; N-[(phenylmethyl)hydroxyphosphinyl]glutamic acid; N-[((2-phenylethyl)methyl)hydroxyphosphinyl]glutamic acid; and N-methyl-N-[phenylhydroxyphosphinyl]glutamic acid. The prostate-specific membrane antigen ligands of the present application also include all small molecule prostate-specific membrane antigen ligands disclosed in PCT Application Nos WO2010/108125 and WO2006/093991, which are incorporated herein in their entireties.

In some embodiments, the prostate-specific membrane antigen ligand of the present application is a pentanedioic acid derivative. In some embodiments, the prostate-specific membrane antigen ligand of the present application is an aminocarbonyl derivative of pentanedioic acid.

In some embodiments, the prostate-specific membrane antigen ligand of the present application comprises the following structure:

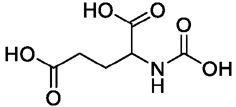

In some embodiments, the conjugate compound or the pharmaceutically acceptable salt thereof comprises a prostate-specific membrane antigen ligand comprising the following structure:

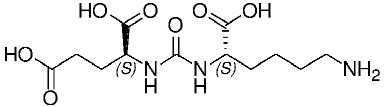

In some embodiments, the conjugate compound or the pharmaceutically acceptable salt thereof comprises a prostate-specific membrane antigen ligand comprising the following structure:

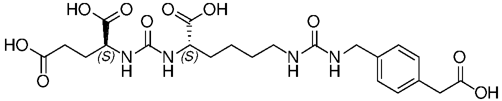

In some embodiments, the conjugate compound or the pharmaceutically acceptable salt thereof comprises a prostate-specific membrane antigen ligand comprising the following structure:

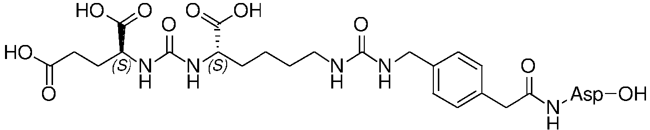

In some embodiments, the conjugate compound or the pharmaceutically acceptable salt thereof comprises a prostate-specific membrane antigen ligand comprising the following structure:

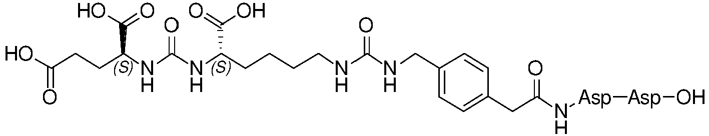

In some embodiments, the conjugate compound or the pharmaceutically acceptable salt thereof comprises a prostate-specific membrane antigen ligand comprising the following structure:

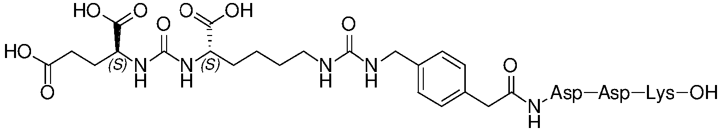

In some embodiments, the conjugate compound or the pharmaceutically acceptable salt thereof comprises a prostate-specific membrane antigen ligand comprising the following structure:

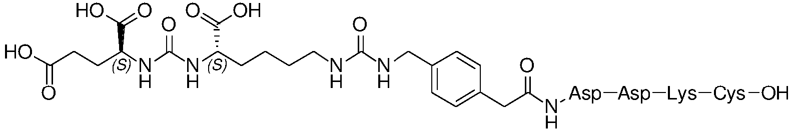

C-MET, a protein product encoded by the C-MET protooncogene, is a receptor of hepatocyte growth factor (HGF) having tyrosine kinase activity. It is associated with various oncogene products and regulatory proteins and involved in the regulation of cell signaling and cytoskeleton rearrangement, and is thus an important factor for cell proliferation, differentiation, and migration. C-MET is closely associated with the development and metastasis of a variety of cancers. Research has shown that many tumor patients have C-MET over-expression and gene amplification during their tumorigenesis and metastasis. Since C-MET may have different substrates in different cells and different differentiation stages, it exhibits a variety of functions in specific conditions, such as promoting the division of hepatocytes, endothelial cells and melanocytes, causing the dispersion of epithelial cells, and inducing morphological changes in cells. In some embodiments, the targeted molecule described herein comprises a ligand targeting C-MET.

The ligand specifically binding to C-MET or "C-MET ligand" refers to an antibody, peptide, aptamer, small molecule, etc., that specifically recognizes and binds to C-MET. The C-MET ligand of the present application includes C-MET ligands that exist at present or may be developed in the future. In some embodiments, the C-MET ligand comprises the amino acid sequence set forth in SEQ ID NO: 3: Cys^{a}-X1-Cys^{c}-X2-Gly-Pro-Pro-X3-Phe-Glu-Cys^{d}-Trp-Cys^{b}-Tyr-X4-X5-X6; X1 is Asn, His, or Tyr; X2 is Gly, Ser, Thr, or Asn; X3 is Thr or Arg; X4 is Ala, Asp, Glu, Gly, or Ser; X5 is Ser or Thr; X6 is Asp or Glu; Cys^{a-d} are cysteine residues. In some preferred embodiments, residues Cys^{a} and Cys^{b}, as well as residues Cys^{c} and Cys^{d}, are separately cyclized to form two independent disulfide bonds. In some embodiments, the ligand of c-MET comprises the amino acid sequence set forth in SEQ ID NO: 4: Ala-Gly-Ser-Cys^{a}-Tyr-Cys^{c}-Ser-Gly-Pro-Pro-Arg-Phe-Glu-Cys^{d}-Trp-Cys^{b}-Tyr-Glu-Thr-Glu-Gly-Thr-Gly-Gly-Gly-Lys; Cys^{a-d} are cysteine residues. In some preferred embodiments, residues Cys^{a} and Cys^{b}, as well as residues Cys^{c} and Cys^{d}, are separately cyclized to form two independent disulfide bonds. In some preferred embodiments, the carboxyl of the terminal lysine may be amidated. The C-MET ligand of the present application also includes all C-MET ligands disclosed in PCT Application No WO2008139207A3, which is incorporated herein by reference in its entirety.

The corresponding names and abbreviations of the amino acids used herein are shown in the following table. The term "amino acid" or the name or abbreviation of a specific amino acid used herein is intended to include all stereoisomers thereof, such as L-configuration and D-configuration. For example, "Arg" used herein may be L-Arg or D-Arg. For example, amidated Lys may be L-2,6-diaminohexanamide or D-2,6-diaminohexanamide.

| **Reagent** | **Symbols and abbreviations** |
|---|---|
| Alanine | A or Ala |
| Arginine | R or Arg |
| Asparagine | N or Asn |
| Aspartic acid | D or Asp |
| Cysteine | C or Cys |
| Glutamic acid | E or Glu |
| Glutamine | Q or Gln |
| Glycine | G or Gly |
| Histidine | H or His |
| Leucine | L or Leu |
| Isoleucine | I or Ile |
| Lysine | K or Lys |
| Methionine | M or Met |
| Phenylalanine | F or Phe |
| Proline | P or Pro |
| Serine | S or Ser |
| Threonine | T or Thr |
| Tryptophan | W or Trp |
| Tyrosine | Y or Tyr |
| Valine | V or Val |

In some embodiments, the ligands in the targeted molecule are linked to each other directly or via a spacer region. In some preferred embodiments, the linkage between the ligands or between the ligand and the spacer region is by means of a dehydration reaction. As used herein, the "dehydration reaction" refers to a process of reaction between two groups by the loss of one water molecule and the formation of a new chemical bond. For example, the amino group (-NH₂) of one amino acid reacts with the carboxyl group (-COOH) of another amino acid, releasing one water molecule and forming a new peptide bond.

In some embodiments, the spacer region comprises an amino acid or an amino acid combination selected from the following group: Lys, Cys, Lys-Cys, Cys-Cys, Lys-Cys-Lys, Arg-Arg, Ala-Ser-Asn, Ala-Ala-Ala, Ser-Ser-Arg, Pro-Arg, Asp-Asp-Lys-Cys, and Pro-Leu-Gly. Optionally, the amino acid in the spacer region may be amidated. In some preferred embodiments, lysine (Lys) is amidated, e.g., to 2,6-diaminohexanamide.

In some embodiments, the spacer region is formed by one or more identical or different compounds selected from the following group by means of a dehydration reaction: 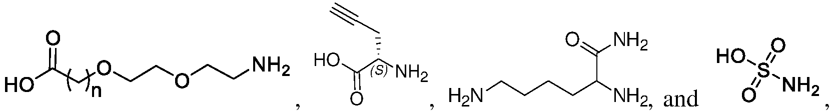 wherein n is 0 or 1. In some embodiments, the spacer region may be 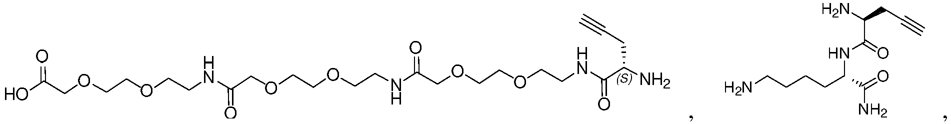 or

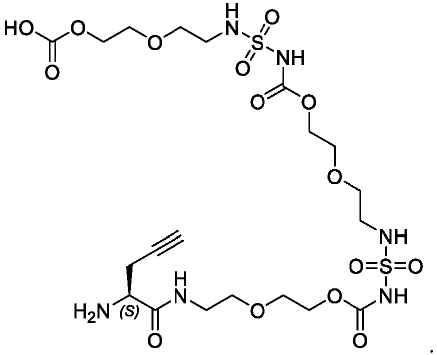

In some embodiments, the targeted molecule having the following structural moiety is linked to the payload to form the conjugate compound: 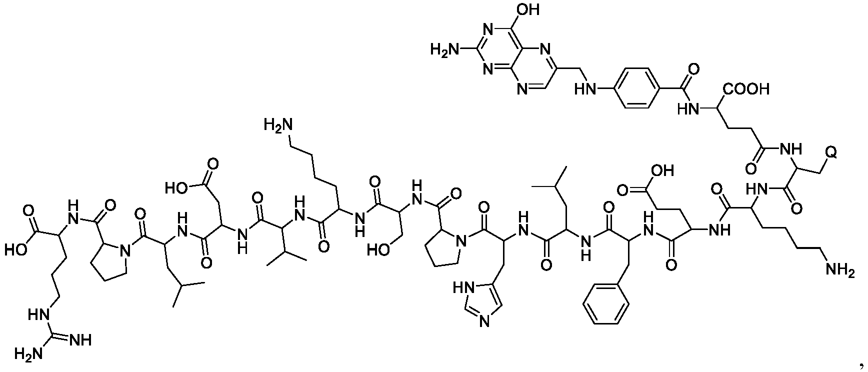 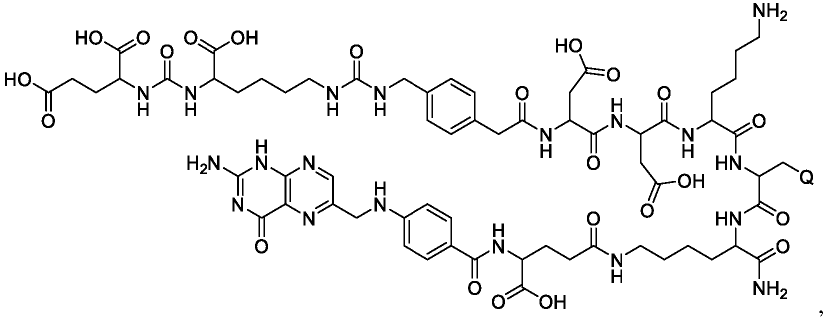 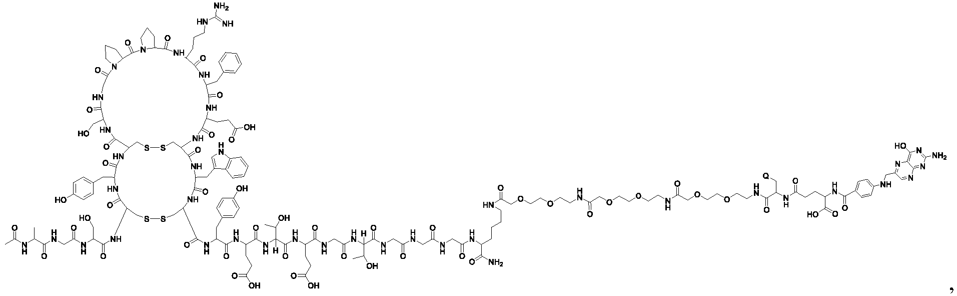 or 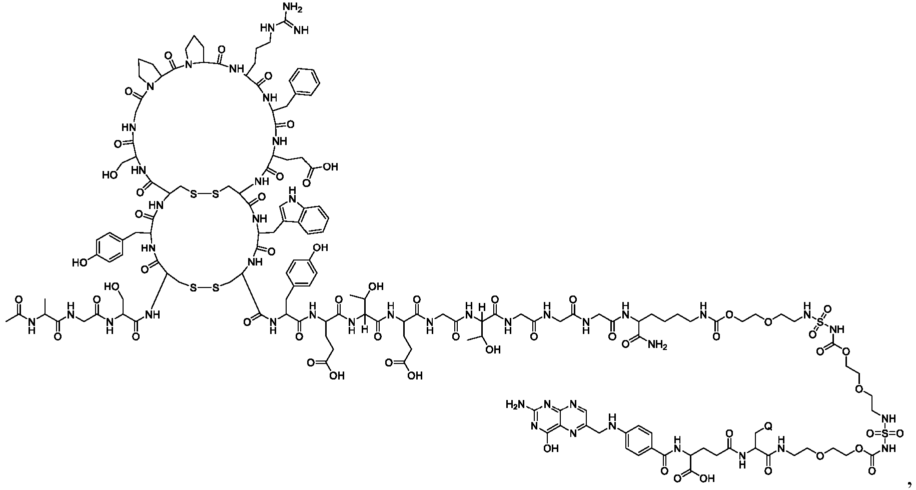 wherein, Q is an active group.

In some embodiments, the aforementioned targeted molecule is linked to the payload through the active group Q, wherein the active group Q is alkynyl or sulfhydryl.

In some embodiments, the targeted molecule having the following structures is linked to the payload to form the conjugate compound: 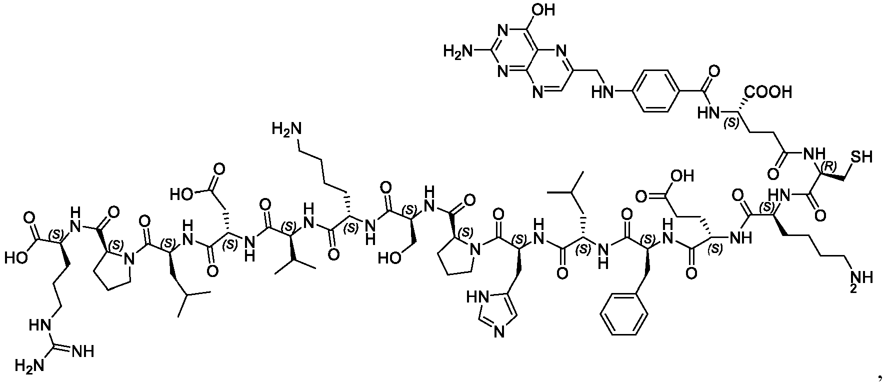 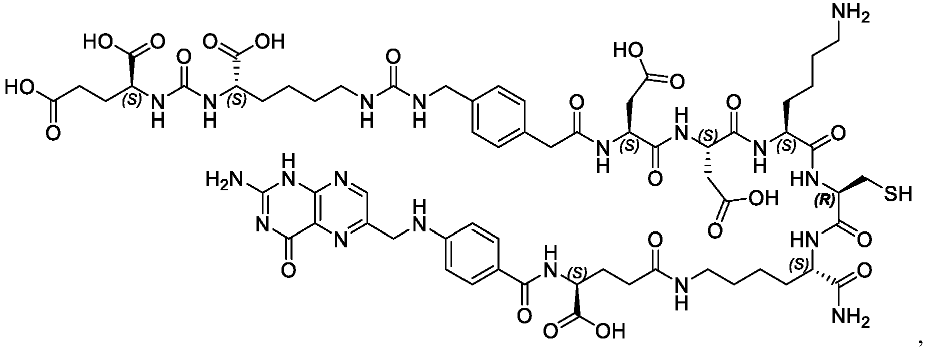 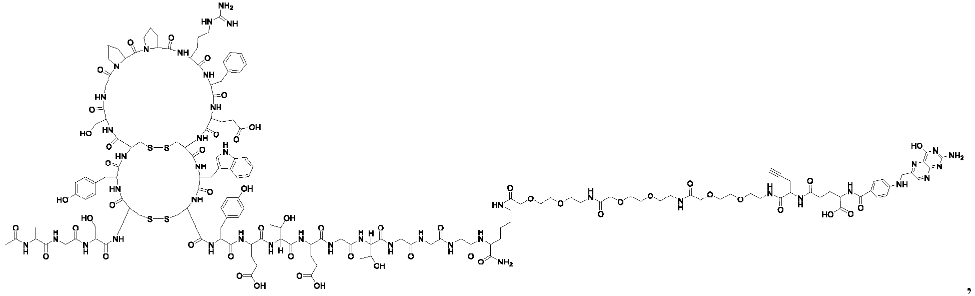 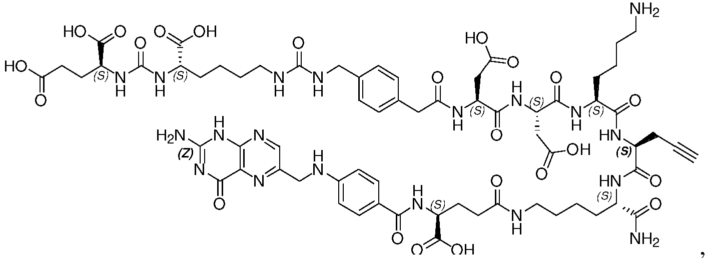 or

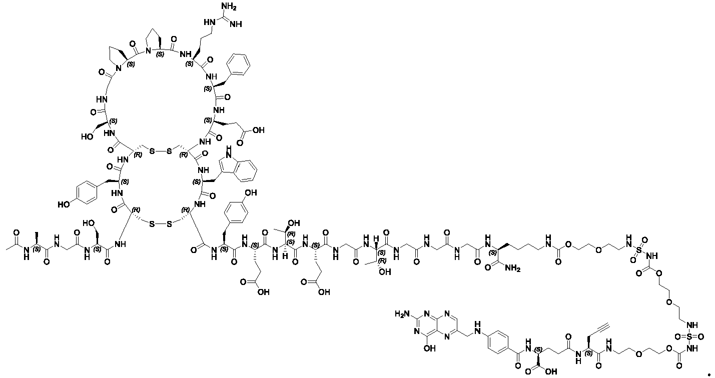

The term "payload" used herein refers to a molecule or substance to be delivered to a target cell or tissue. In some embodiments, the payload may be a molecule or substance for diagnosing, treating, or preventing a disease in a subject. Without limitation, the payload may be a proteolysis targeting chimera intended for use in diagnosing, treating, or preventing a disease in a subject.

In some embodiments, the proteolysis targeting chimera has a target protein ligand moiety for binding to a target protein (specific protein), an E3 ligase ligand moiety for binding to E3 ligase, and a linking moiety for linking the target protein ligand moiety and the E3 ligase ligand moiety. The proteolysis targeting chimera of the present application includes proteolysis targeting chimeras that exist at present or may be developed in the future. The proteolysis targeting chimera of the present application also includes all proteolysis targeting chimeras disclosed in PCT Application No WO2021205391A1, US Application No US20190151457A1, and Chinese Application Nos CN113563414, CN104736569A, and CN108366992A, which are incorporated herein by reference in their entireties.

In some embodiments, the proteolysis targeting chimera having the structure represented by formula (I) is linked to the targeted molecule to form the conjugate compound:

(T_{P})ₙ-L-T_{E3} (I),

wherein,
T_{P} is a target protein ligand moiety for binding to a target protein, wherein n is 1, 2, or 3;
L is a linking moiety for linking T_{P} to T_{E3};
T_{E3} is an E3 ligase ligand moiety for binding to E3 ligase.

In some embodiments, the proteolysis targeting chimera has a structure selected from the following group: 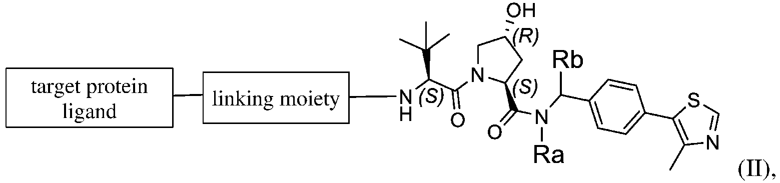 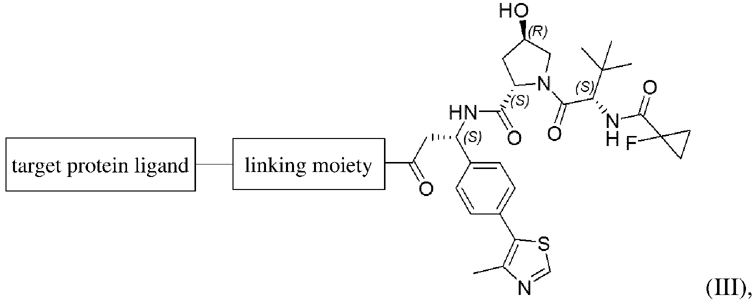 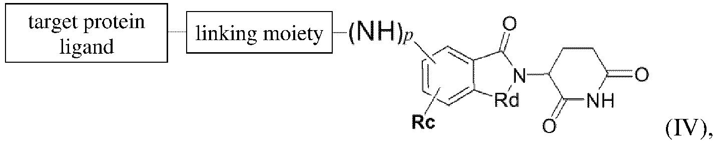 or 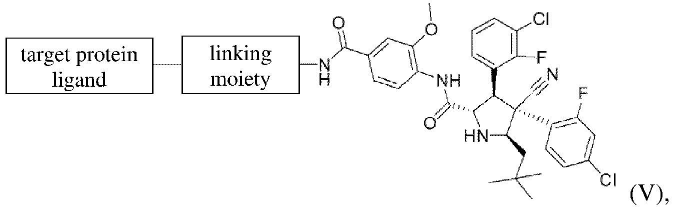 wherein,
Ra, Rb and Rc are each independently hydrogen, alkyl, NH₂, OH, or halogen; preferably the alkyl is methyl, and the halogen is fluorine;
Rd is CH₂, C=O, or C=S;
p is 0 or 1;
the target protein ligand can bind to a specific target protein;
the target protein ligand is linked to the E3 ligase group through the linking moiety.

In some embodiments, the target protein is the specific protein described herein. In some embodiments, the target protein is selected from a nuclear receptor (e.g., AR and ER), a kinase-like protein (e.g., RIPK2, BCR-ABL, EGFR, HER2, c-Met, TBK1, CDK2/4/6/9, ALK, Akt, CK2, ERK1/2, FLT3, PI3K, BTK, and FAK), AKT, PAN-BET protein, BET protein (e.g., BRD2, BRD3, BRD4, and BRD6), BLK, BRAF1, BCL-xl, ERRα, FKBP12, FRS2α, JAK1, JAK3, IKZF1, IRAK4, MDM2, MetAP2, PLK1, PSK-J3, RAS, TACC3, Tau, TrkB, MDM2, or any combination thereof. In some preferred embodiments, the target protein is selected from AR protein, ER protein, RAS protein, BRD4 protein, PLK1 protein, FAK protein, MDM2 protein, or any combination sthereof.

In some embodiments, the target protein ligand moiety disclosed herein has a structure selected from:

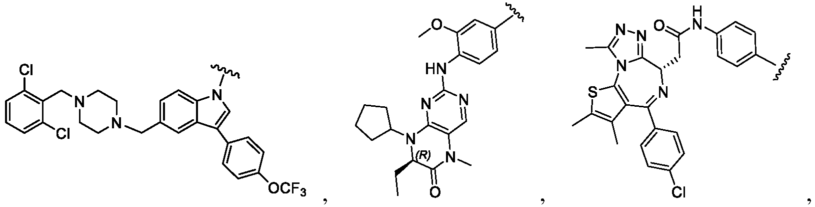

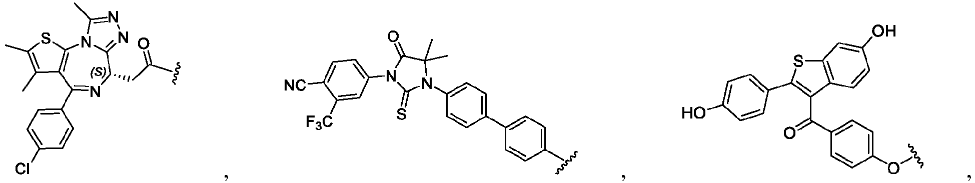

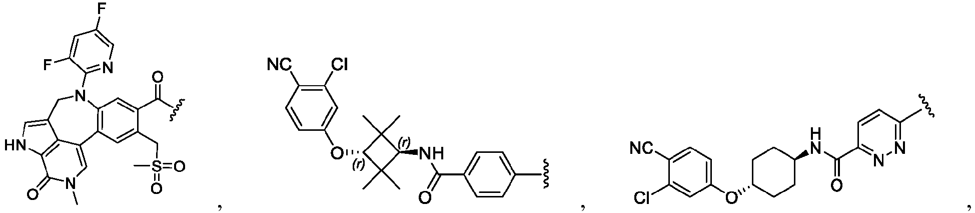

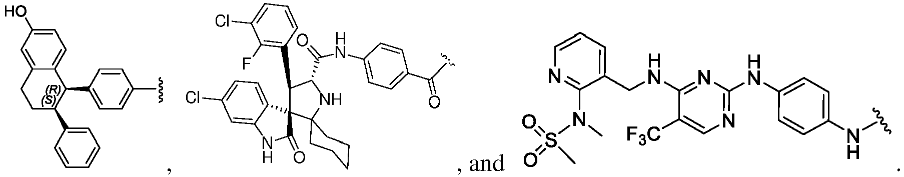

In some embodiments, the linking moiety in the proteolysis targeting chimera has a structure selected from the following group: 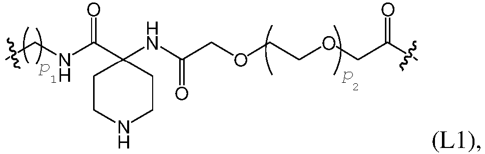 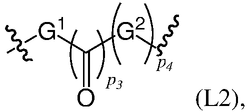 or 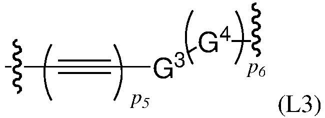 wherein,
p₁ is an integer of 0-4;
p₂ is an integer of 0-6;
p₃, p₄, p₅, and p₆ are each independently 0 or 1;
G¹ is a 4-14 membered linear alkylene, optionally 2-5 carbon atoms of the 4-14 membered linear alkylene may be substituted by -O-, -NH-, -N(C₁₋₆ alkyl)-, or -C≡C-;
G² is an amino acid residue, wherein preferably G² is a Lys residue;
G³ is selected from the following group: 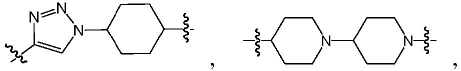 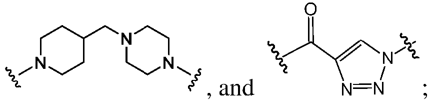
G⁴ is a 5-9 membered linear alkylene, optionally 2-4 carbon atoms of the 5-9 membered linear alkylene may be substituted by -O-, -NH-, -N(C₁₋₆ alkyl)-, or -C≡C-.

In some embodiments, the linking moiety in the proteolysis targeting chimera disclosed herein has a structure selected from the following group:

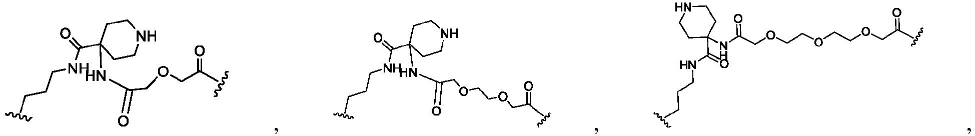

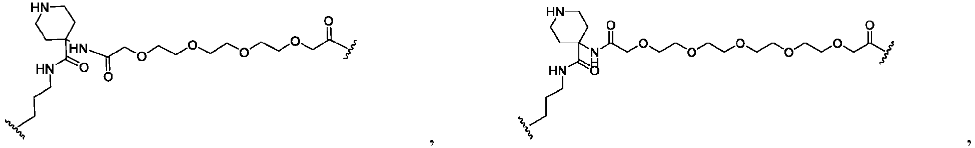

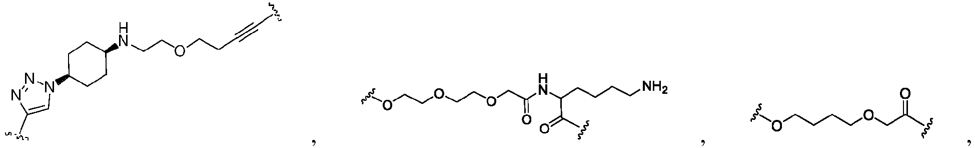

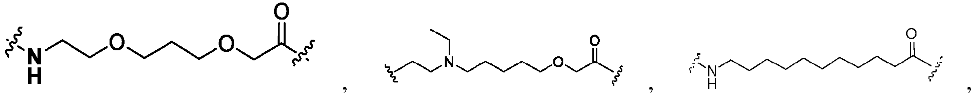

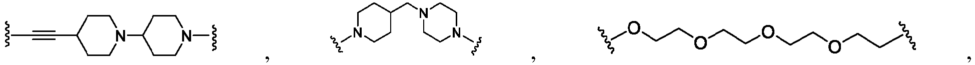

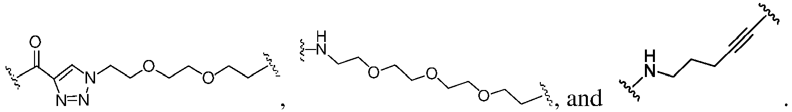

In some embodiments, the proteolysis targeting chimera disclosed herein has the structures shown below:

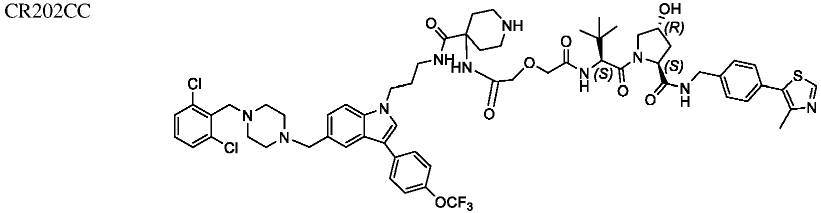

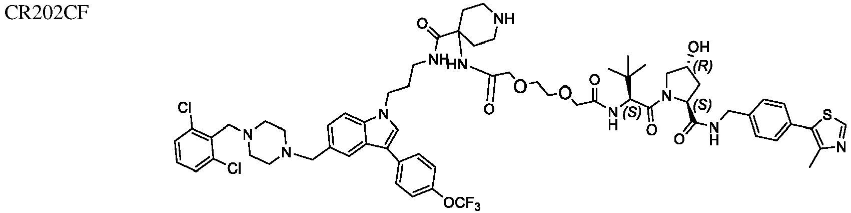

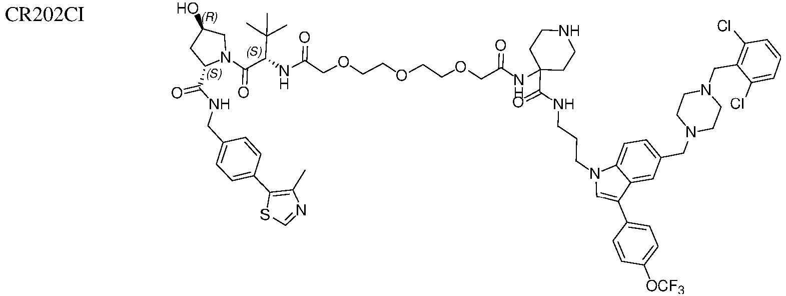

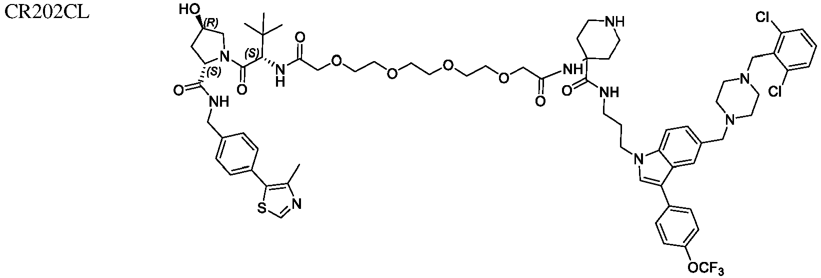

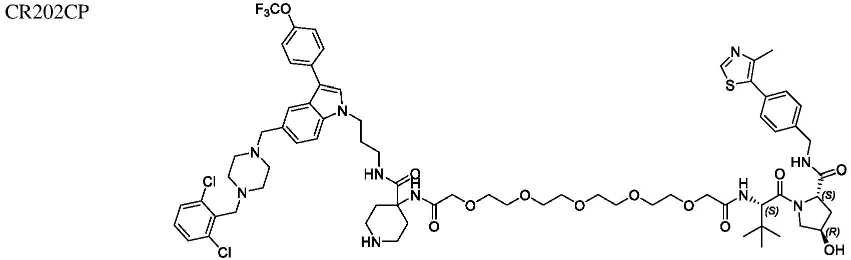

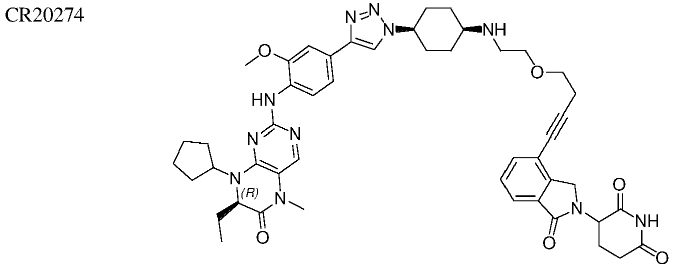

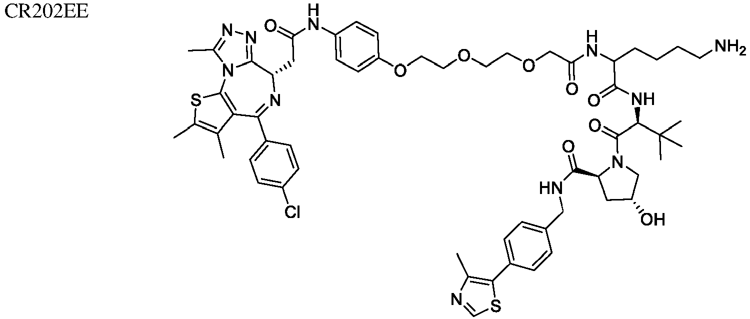

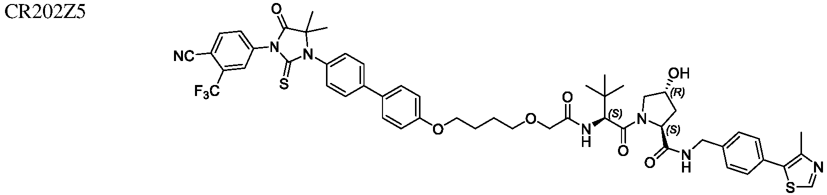

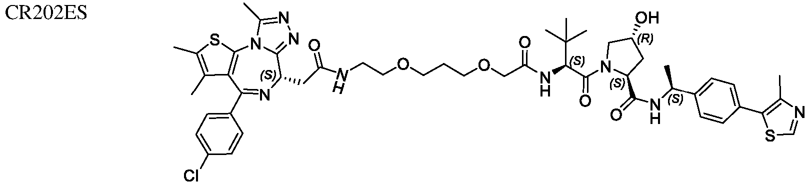

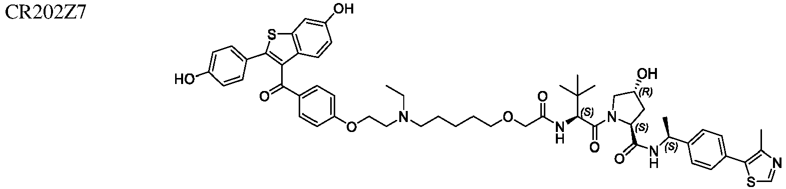

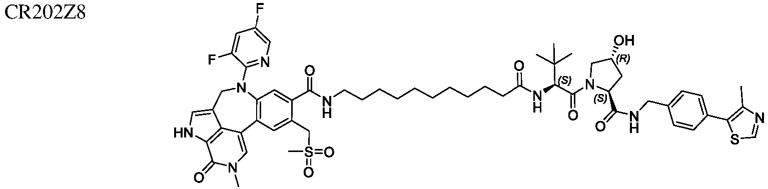

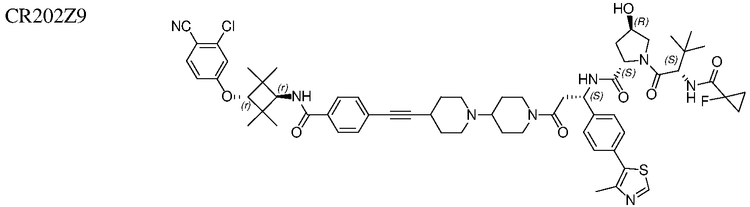

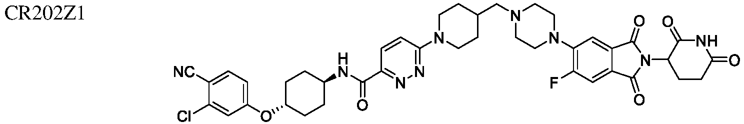

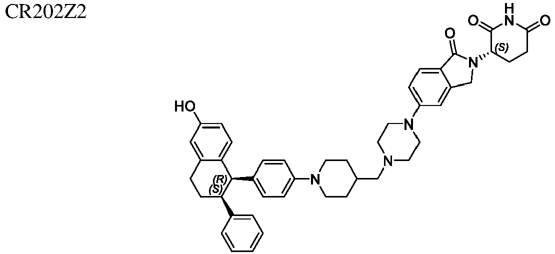

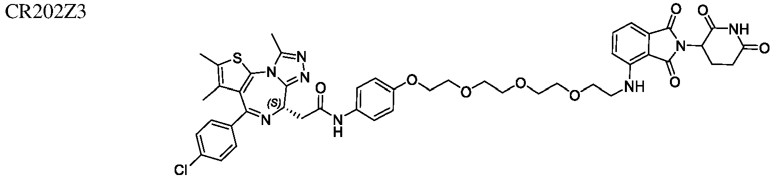

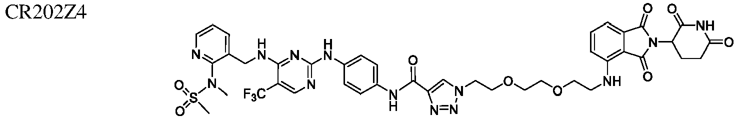

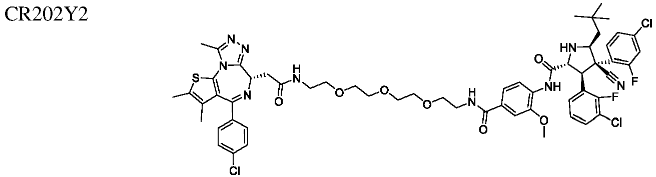

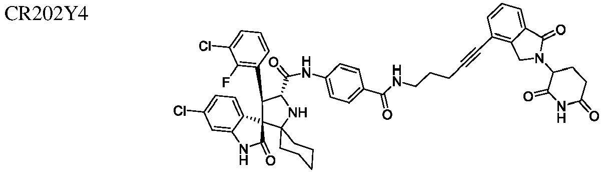

In some embodiments, the conjugate compound or the pharmaceutically acceptable salt thereof comprises one, two, three, four, or more payloads.

In some embodiments, the payload of the present application, prior to the linkage to the targeted molecule or the linker of the present application (i.e., prior to the formation of the conjugate compound), has a free amino or carboxyl group, and the payload is conjugated to the conjugate compound by the acylation of free amino or carboxyl group with a group in a corresponding moiety (e.g., the linker) of the conjugate compound.

In some embodiments, the payload is linked to the targeted molecule directly, or the payload is linked to the targeted molecule via a linker. In some embodiments, the linkage between the payload and the targeted molecule, the payload and the linker, or the linker and the targeted molecule is accomplished by a chemical reaction. In some preferred embodiments, the linkage between the targeted molecule and the linker is accomplished by a click reaction.

The click reaction described herein is a chemical synthesis method with high efficiency and high selectivity. The click reaction splices several molecular fragments by forming efficient connection units, and the whole reaction process is simple and rapid and has few byproducts. Examples of some click reactions include, but are not limited to, the addition reaction of thiols to alkenes, and cyclization of azides and alkynes.

The term "linker" used herein refers to a molecule or moiety that covalently links the payload to the targeted molecule. The linker comprises functional groups for linking the payload to at least one targeted molecule. In some embodiments, the functional groups may comprise two reactive moieties, one for linkage to the payload and the other for linkage to the targeted molecule. In some embodiments, the functional groups are different from each other. In some embodiments, the functional groups comprise groups comprising a thiol-reactive moiety and an amine-reactive moiety. In some embodiments, the functional groups are identical to each other. In some embodiments, the carboxylic acid in the amino acid in the linker is amidated. In some embodiments, the linker comprises a short-chain polyethylene glycol (e.g., comprising 2-10, 2-8, 3-8, 4-8, 4-7, 4-6, or 5 repeats). In some embodiments, the linker comprises an azido. In some embodiments, the linker comprises an alkynyl. In some embodiments, the linker is sufficiently stable to avoid accidental release of the payload in the blood circulation, thus increasing the effective amount of the payload to the target cell or tissue and avoiding toxicity. In some embodiments, the linker is capable of releasing the payload around or within the target cell to effectively kill the target cell or block the functionality of the target cell.

In some embodiments, the linker comprises a linking module 1 and a linking module 2.

In some embodiments, the linker comprises a linking module 2.

In some embodiments, the linker comprises a linking module 1 and the following groups binding to the linking module 1: a PEG chain, 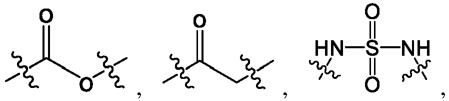 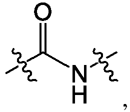 C₁-C₈ alkyl chain, -NH-, -O-, -C(O)-, or any combination thereof. In some preferred embodiments, the PEG chain is PEG1 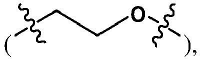 PEG2 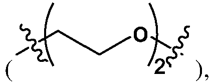 or PEG3 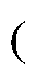 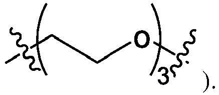 In some preferred embodiments, the linker comprises a linking module 1 and the following group binding to the linking module 1: 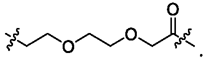 Those skilled in the art will appreciate that such groups may be bonded to the linking moiety in the linking module 1 through the left linking site and to the payload through the right linking site, or bonded to the linking moiety in the linking module 1 through the right linking site and to the payload through the left linking site.

In some embodiments, the linking module 1 is linked to the linking module 2 via a bond or a chemical group. In some embodiments, the chemical group is a PEG chain, 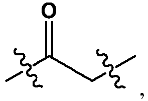 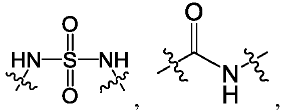 C₁-C₈ alkyl chain, -NH-, -O-, -C(O)-, 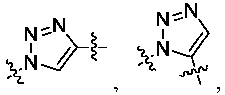 or any combination thereof. In some preferred embodiments, the PEG chain is PEG1, PEG2, or PEG3. In some preferred embodiments, the chemical group is 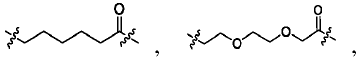 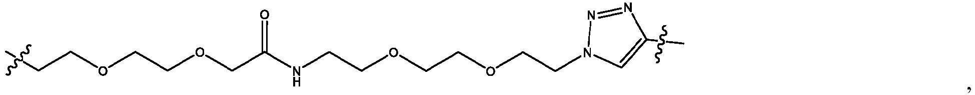 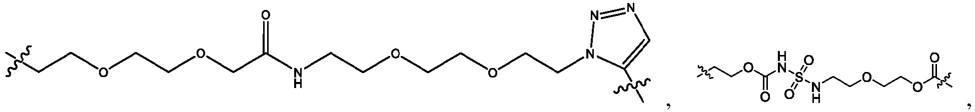 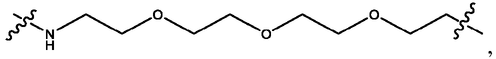 or 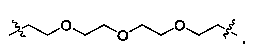 Those skilled in the art will appreciate that such chemical groups may be bonded to the linking module 1 through the left linking site and to the linking module 2 through the right linking site, or bonded to the linking module 1 through the right linking site and to the linking module 2 through the left linking site.

In some embodiments, the linking module 1 comprises maleimidocaproyl ( 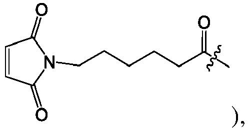 maleimido 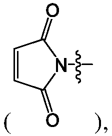 or azido

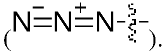

In some embodiments, the linking module 2 comprises a CB enzyme-cleavable group, a plasmin-cleavable group, a legumain-cleavable group, a β-glucuronidase-cleavable group, a sulfatase-cleavable group, a phosphatase-cleavable group, a β-galactosidase-cleavable group, a glutathione enzyme-cleavable group, or an acid-labile group.

In some preferred embodiments, the CB enzyme-cleavable group comprises an amino acid or a combination of an amino acid and a self-decomposing fragment. In some preferred embodiments, the amino acid is Val-Cit, Gly-Gly-Phe-Gly, CycloBut-Cit, Phe-Cit, Val-Ala, Glu-Val-Cit, Ala-Cit, Val-Cit-Pro, or Val-Cit-Pro-Gly.

In some preferred embodiments, the plasmin-cleavable group comprises an amino acid or a combination of an amino acid and a self-decomposing fragment. In some preferred embodiments, the amino acid is Val-Leu-Lys.

In some preferred embodiments, the legumain-cleavable group comprises an amino acid or a combination of an amino acid and a self-decomposing fragment. In some preferred embodiments, the amino acid is selected from Gly-Asn-Asn or Ala-Ala-Asn.

In some preferred embodiments, the self-decomposing fragment is PAB and a derivative thereof, 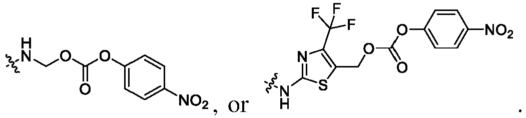 In some preferred embodiments, the PAB is 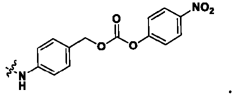 In some preferred embodiments, the PAB derivative is

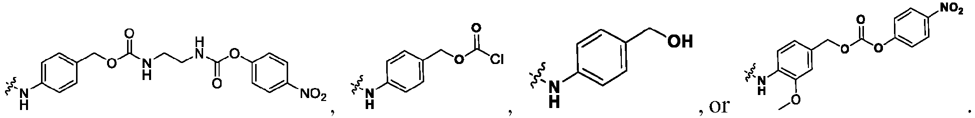

In some preferred embodiments, the β-glucuronidase-cleavable group is 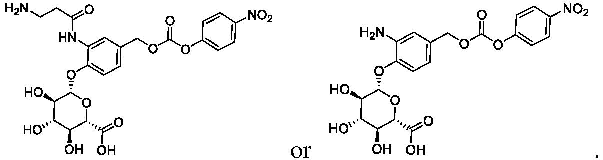 In some preferred embodiments, the sulfatase-cleavable group is 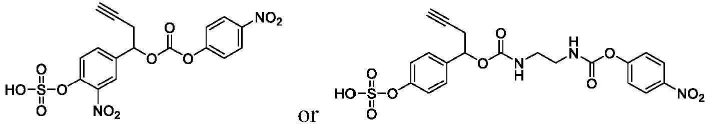 . In some preferred embodiments, the phosphatase-cleavable group is 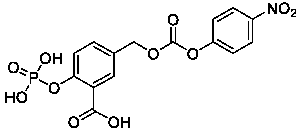 or 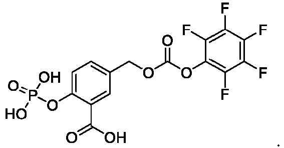 In some preferred embodiments, the β-galactosidase-cleavable group is 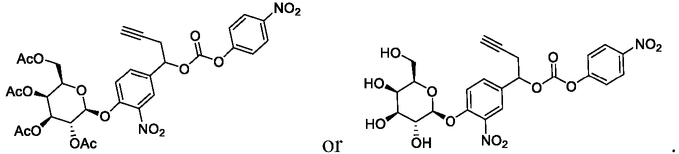 In some preferred embodiments, the glutathione enzyme-cleavable group is 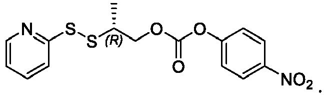 In some preferred embodiments, the acid-labile group is

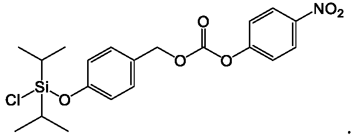

It will be appreciated by those skilled in the art that the active site of the cleavable groups described above may optionally be linked to the linking module 1, to the chemical group, to the targeted molecule, or to the payload after a proper reaction. For example, the β-glucuronidase-cleavable group, upon operative linkage to the linking module 1, the chemical group, the targeted molecule, or the payload, has the following structures 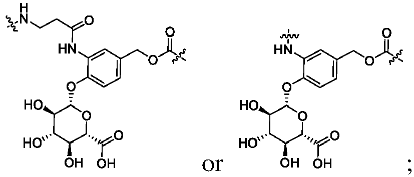 the sulfatase-cleavable group, upon operative linkage to the linking module 1, the chemical group, the targeted molecule, or the payload, has the following structures 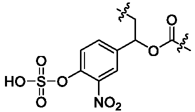 or 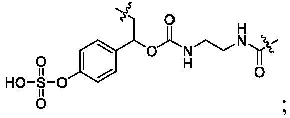 the phosphatase-cleavable group, upon operative linkage to the linking module 1, the chemical group, the targeted molecule, or the payload, has the following structure 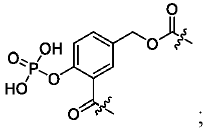 the β-galactosidase-cleavable group, upon operative linkage to the linking module 1, the chemical group, the targeted molecule, or the payload, has the following structures 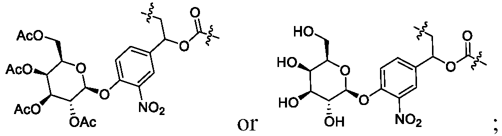 the glutathione enzyme-cleavable group, upon operative linkage to the linking module 1, the chemical group, the targeted molecule, or the payload, has the following structure 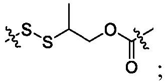 the acid-labile group, upon operative linkage to the linking module 1, the chemical group, the targeted molecule, or the payload, has the following structure

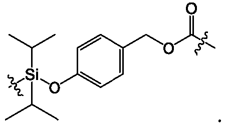

In some preferred embodiments, when the linker comprises the linking module 2, the linking module 2 is the glutathione enzyme-cleavable group. In some preferred embodiments, the glutathione enzyme-cleavable group is

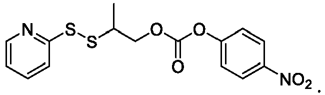

CB enzyme refers to cathepsin B, a cysteine protease commonly found in lysosomes. Plasmin refers to a serine protease commonly found in plasma. The legumain, or asparagine endopeptidase, is a peptidase in lysosomes capable of specifically cleaving peptide fragments containing asparagine residues (-Asn-).

In some embodiments, the conjugate compound has the following structure:

W¹-L¹-(EG)n¹-(D)n⁵-(CH₂)n²-(B)n⁶-(AA)n³-(CH₂CH₂-Q)n⁴-L²-W²,

wherein,
**L¹** is 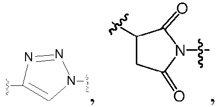 -S-S-, or a bond;
**EG** is -CH₂CH₂O- or -OCH₂CH₂-; n¹ is an integer of 0-6;
n² is an integer of 0-8;
**D** is -C(=O)-NH-S(=O)₂-NH-; n⁵ is 0 or 1;
**B** is each independently -C(=O)-, 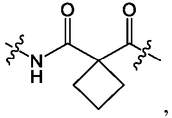 -NH-CH₂CH₂-C(=O)-, -C(=O)-NH-, -CH(CH₃)-CH₂-O-, -CH₂CH₂O-, -NH-C(=O)-, or -NH-; n⁶ is an integer of 0-3;
AA is each independently an amino acid residue; n³ is an integer of 0-5;
**Q** is each independently -C(=O)-, -O-, or a bond; n⁴ is an integer of 0-3;
**L²** is 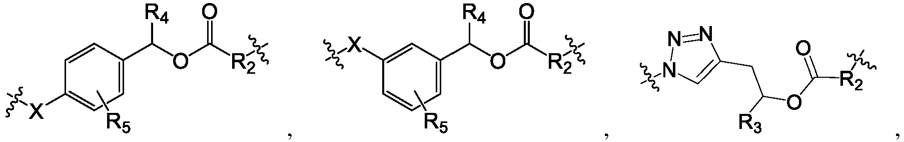 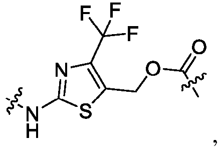 -NH-CH₂-O-, or a bond;
wherein X is a bond, -NH-, -C(=O)-, oxygen, or 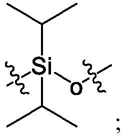
R₂ is a bond, -O-CH₂-, or -NH-CH₂CH₂-NH-C(=O)-;
R₃ is hydrogen or 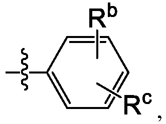 wherein R^{b} and R^{c} are each independently hydrogen, nitro, 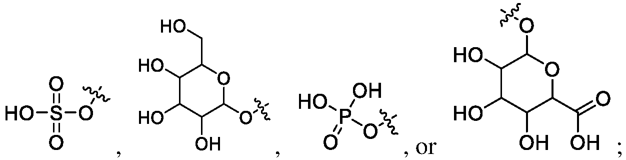
R₄ is hydrogen or C₁₋₈ alkyl;
R₅ is hydrogen, amino, nitro, phosphate group, -OR^{a}, or 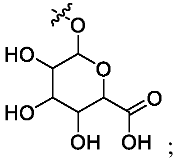 wherein R^{a} is methyl, ethyl, propyl, or isopropyl;
**W¹** is the targeted molecule capable of binding to the cell surface protein, and
**W²** is the payload.

In some embodiments, EG is -CH₂CH₂O-. In some embodiments, n¹ is 2. In some embodiments, n¹ is 0. In some embodiments, n² is 1. In some embodiments, n² is 4. In some embodiments, n² is 5. In some embodiments, n⁵ is 0. In some embodiments, n⁶ is 1. In some embodiments, n⁶ is 3. In some embodiments, n¹ is 2, n² is 1, n³ and n⁴ are 0, and L² is a bond.

In some embodiments, AA is each independently an amino acid residue formed by Val, Cit, Glu, Leu, Lys, Ala, Gly, Phe, Gln, Pro, or Asn. In some embodiments, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Cit-, -Val-Leu-Lys-, -Val-Cit-Pro-Gly-, -Gly-Asn-Asn-, and -Ala-Ala-Asn-.

In some embodiments, when n⁴ is 1, Q is -C(=O)-; when n⁴ is 2, Q are -O- and -C(=O)-, respectively; when n⁴ is 3, Q are -O-, -O-, and a bond, respectively. In some embodiments, n⁴ is 0.

In some embodiments, L² is a bond, 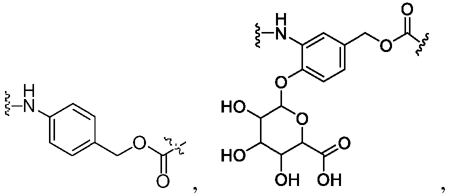 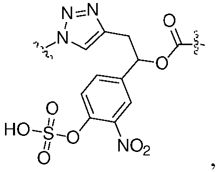 -NH-CH₂-O-, 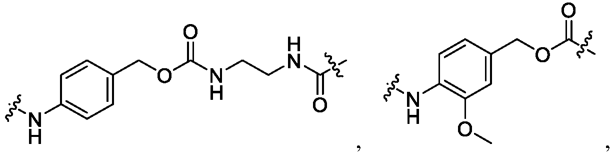 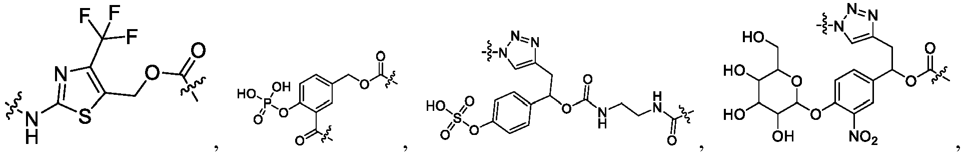 or

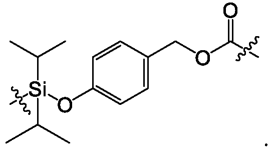

In some embodiments, the conjugate compound has the following structure:

W¹-L¹-(EG)n¹-(D)n⁵-(CH₂)n²-(B)n⁶-(AA)n³-(CH₂CH₂-Q)n⁴-L²-W²

wherein,
L¹ is 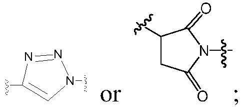
EG is -CH₂CH₂O-; n¹ is an integer of 0-4;
D is -C(=O)-NH-S(=O)₂-NH-; n⁵ is 0 or 1;
n² is an integer of 0-5;
B is -C(=O)-, -C(=O)-NH-, -CH₂CH₂O-, -NH-CH₂CH₂-C(=O)-, or -NH-; n⁶ is an integer of 0-3;
AA is each independently an amino acid residue; n³ is an integer of 0-3;
Q is each independently -C(=O)-, -O-, or a bond; n⁴ is an integer of 0-3;
L² is 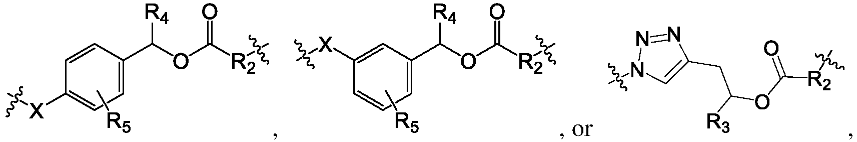
wherein X is a bond, -NH-, -C(=O)-, or 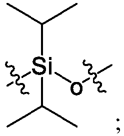
R₂ is a bond;
R₃ is 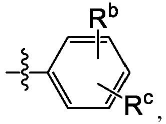 wherein R^{b} and R^{c} are each independently hydrogen, nitro, or 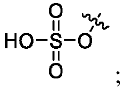
R₄ is hydrogen;
R₅ is hydrogen, -OCH₃, or 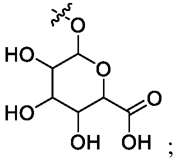
**W¹** is the targeted molecule capable of binding to the cell surface protein, and
**W²** is the payload.

In some embodiments, L² is

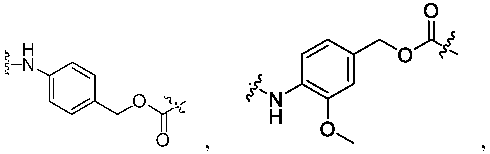

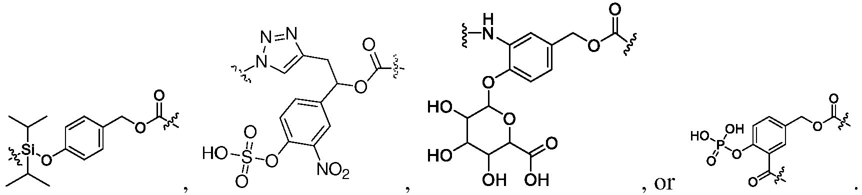

In some embodiments, L¹ is 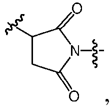 and n¹, n⁵, and n⁴ are all 0.

In some embodiments, L¹ is 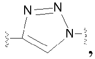 and n² is 1 or 2.

In some embodiments, when n⁵ is 1, n² and n⁴ are both 0.

In some embodiments, AA is each independently an amino acid residue formed by Val, Cit, Glu, or Ala. In some embodiments, -(AA)n³- is a peptide residue selected from the following group: -Val-Cit-, -Glu-Val-Cit-, -Ala-Cit-, and -Val-Ala-.

In some embodiments, the conjugate compound has the following structure:

W¹-L¹-(EG)n¹-(CH₂)n²-(C=O)-(AA)n³-L²-W²

wherein,
L¹ is 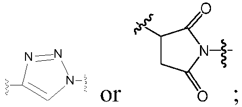
EG is -CH₂CH₂O-; n¹ is an integer of 0-4;
n² is an integer of 0-5;
AA is each independently an amino acid residue; n³ is an integer of 1-4;
L² is 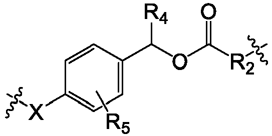 or a bond,
wherein X is -NH-;
R₂ is a bond;
R₄ is hydrogen;
R₅ is hydrogen or -OCH₃;
**W¹** is the targeted molecule capable of binding to the cell surface protein, and
**W²** is the payload.

In some embodiments, L¹ is 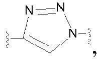 n¹ is 2, n² is 1, and L² is

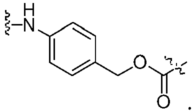

In some embodiments, L¹ is 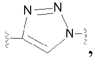 n¹ is 2, n² is 1, and L² is a bond.

In some embodiments, L¹ is 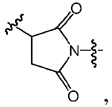 n¹ is 0, n² is 5, and L² is

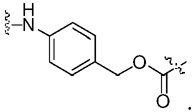

In some embodiments, L¹ is 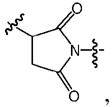 n¹ is 0, n² is 5, and L² is a bond.

In some embodiments, AA is each independently Val, Cit, Glu, or Ala amino acid residue. In some embodiments, -(AA)n³- is a peptide residue selected from the following group: - Val-Cit-, -Glu-Val-Cit-, -Ala-Cit-, -Phe-Cit-, -Val-Ala-, -Gly-Gly-Phe-Gly-, -Val-Leu-Lys-, and - Gly-Asn-Asn-.

In some embodiments, the conjugate compound disclosed herein has the following structures: 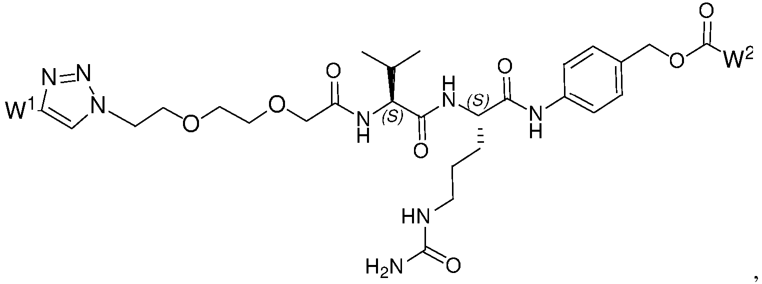 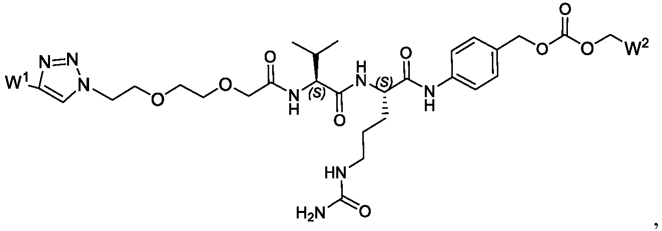 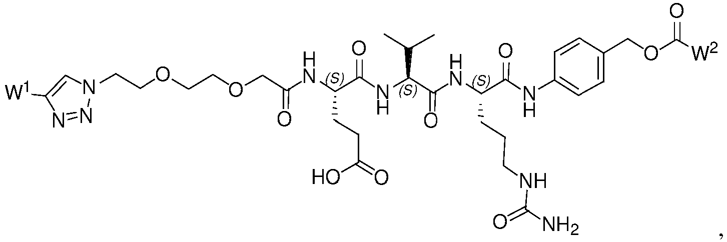 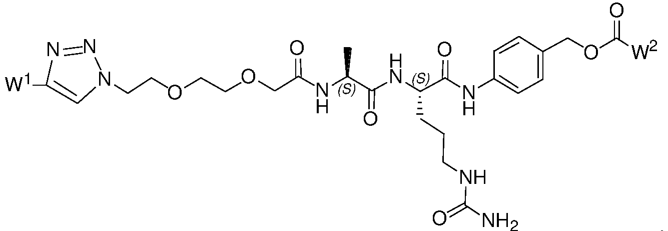 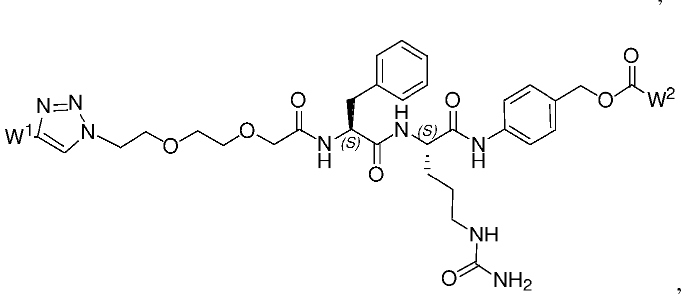 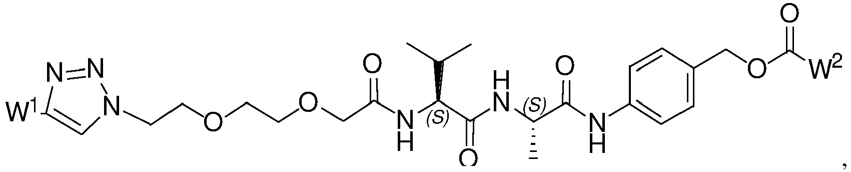 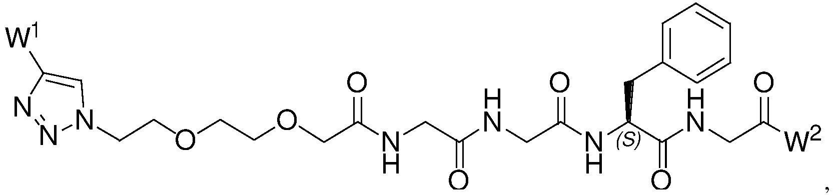 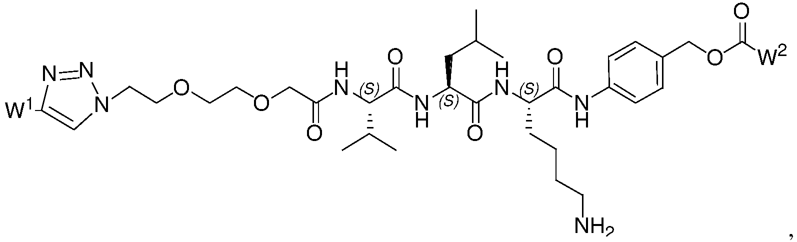 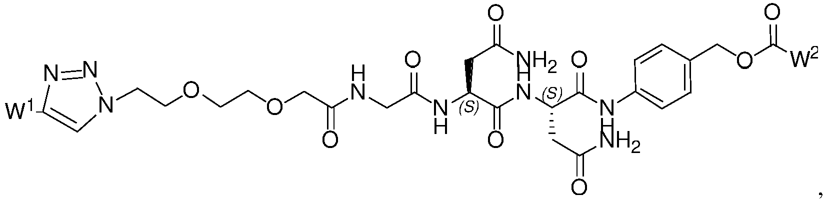 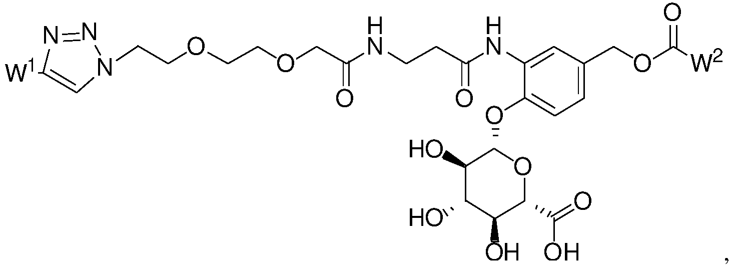 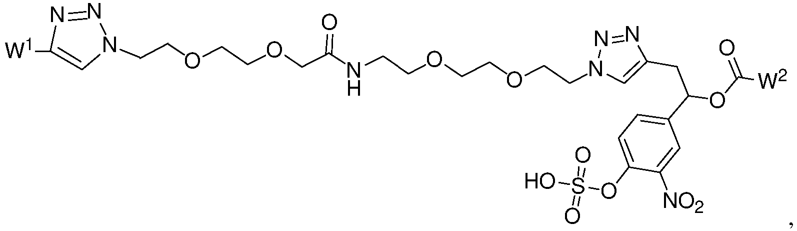 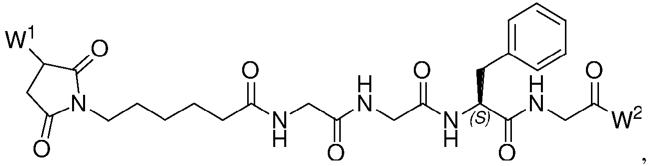 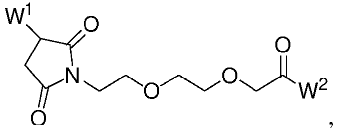 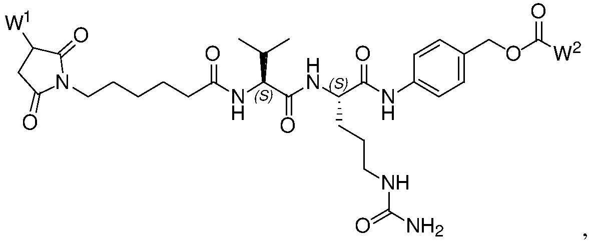 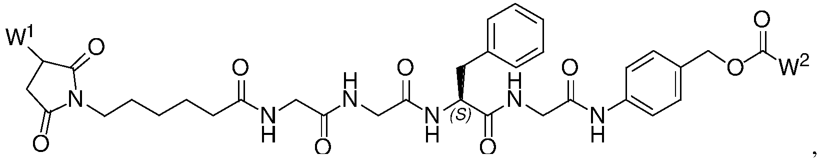 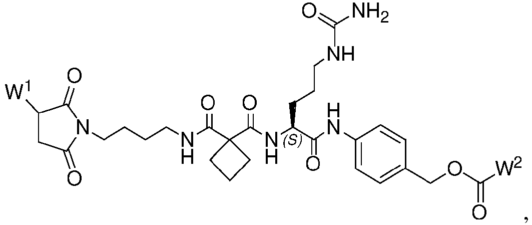 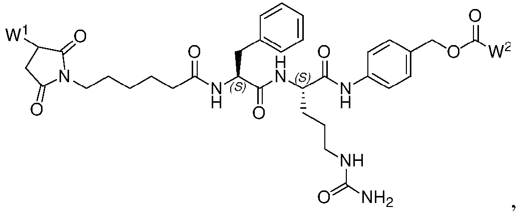 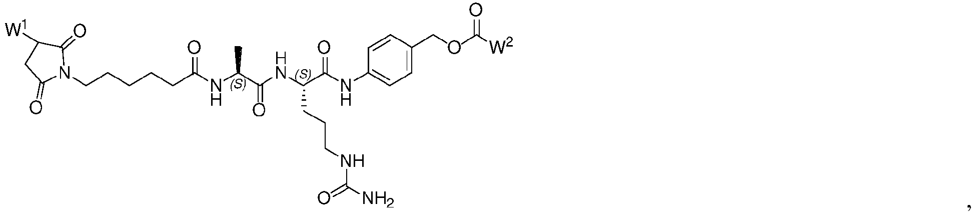 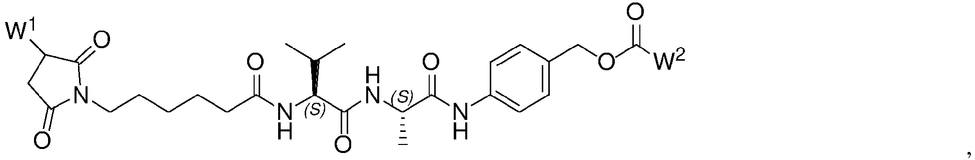 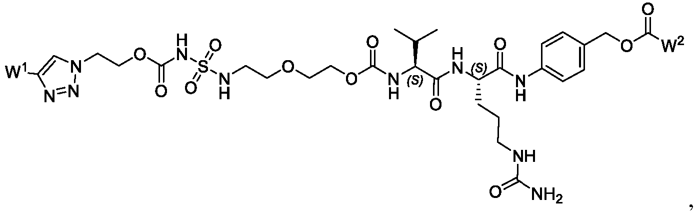 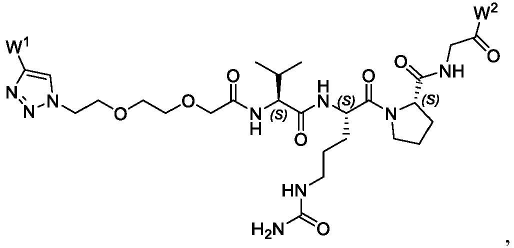 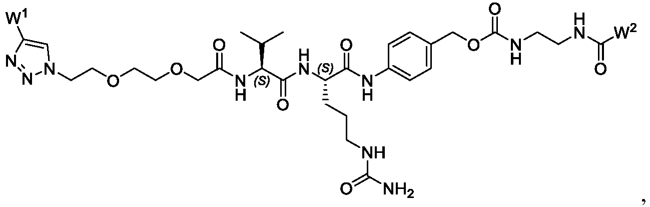 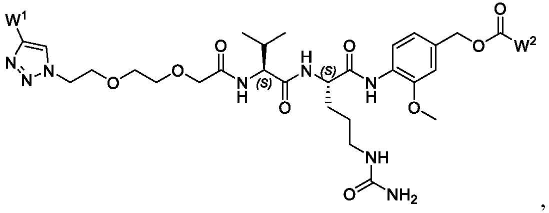 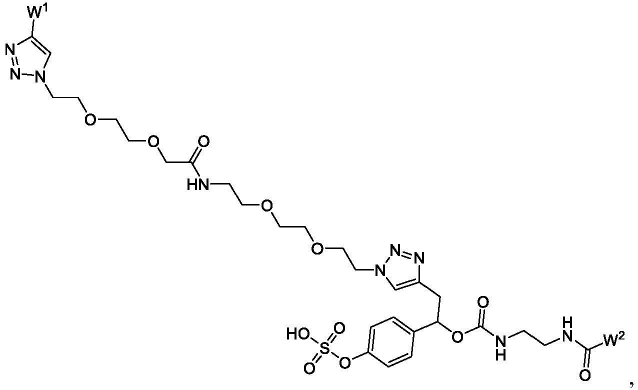 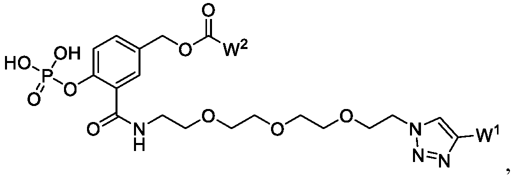 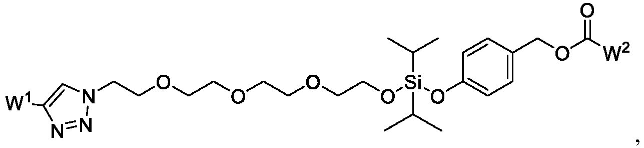 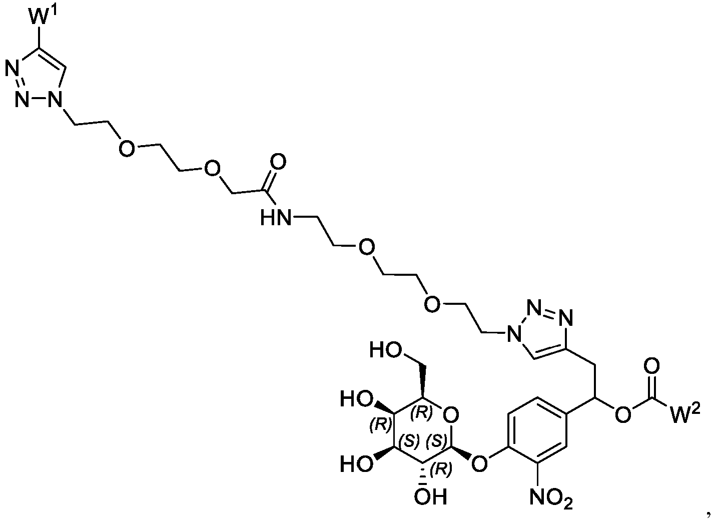 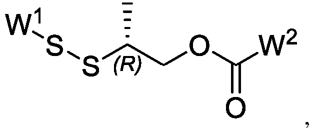 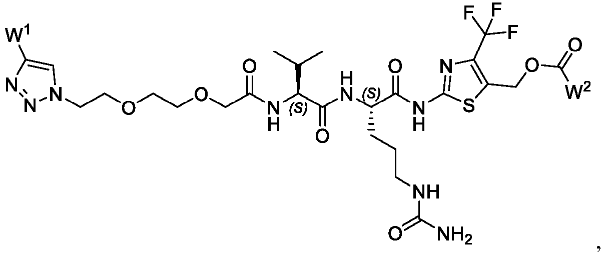 or 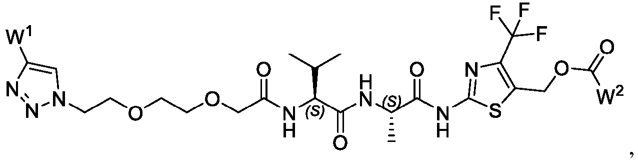 wherein W¹ is the targeted molecule capable of binding to the cell surface protein, and W² is the payload.

In some embodiments, disclosed herein are the following conjugate compounds:

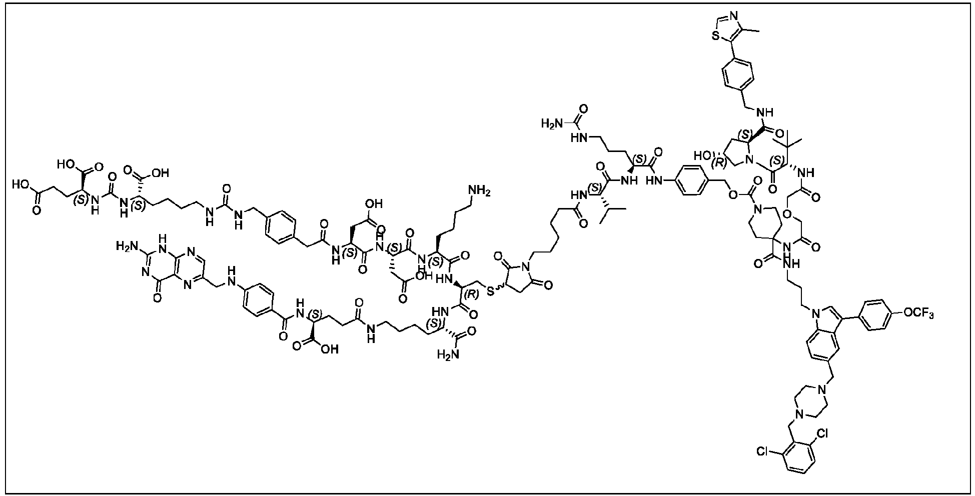

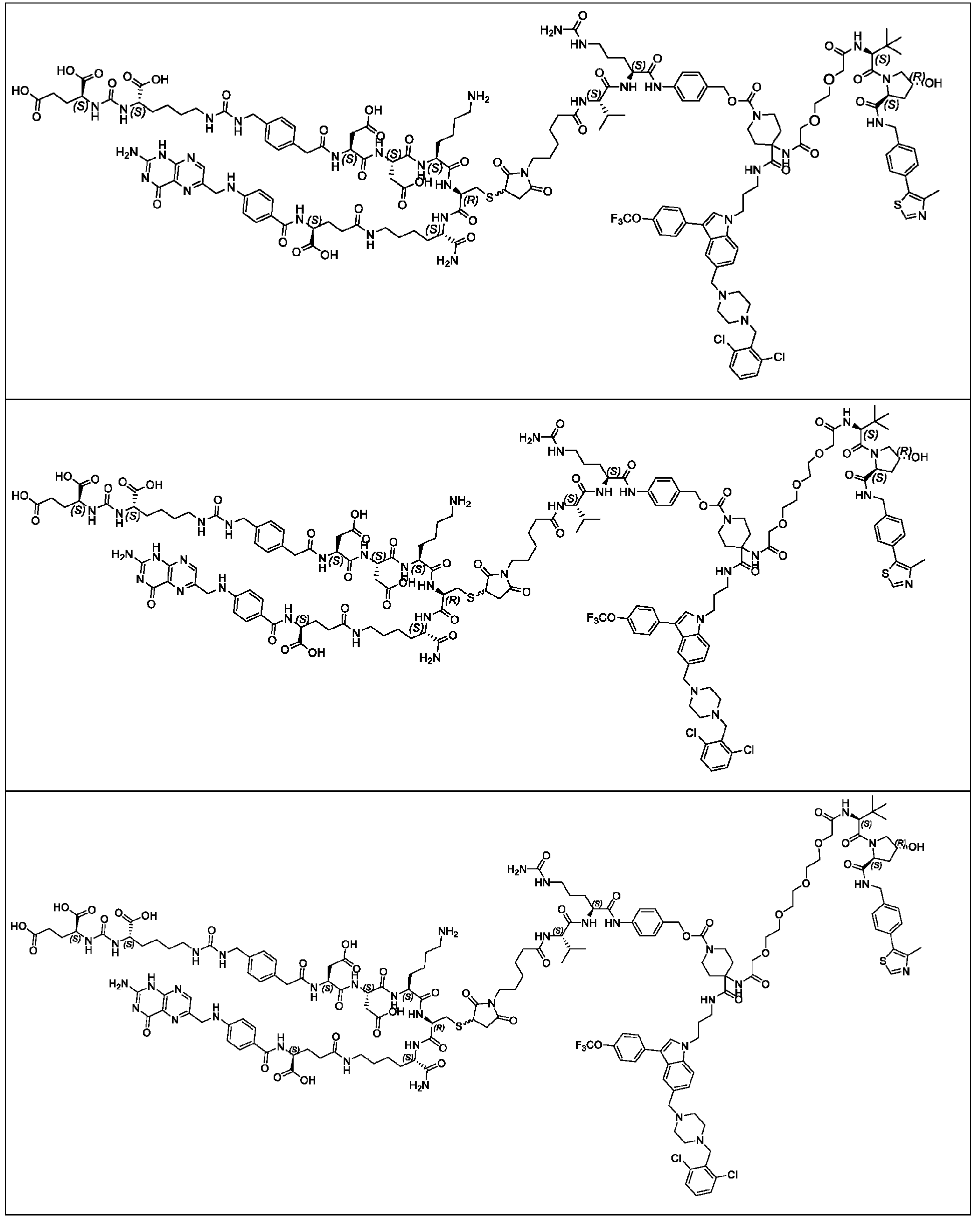

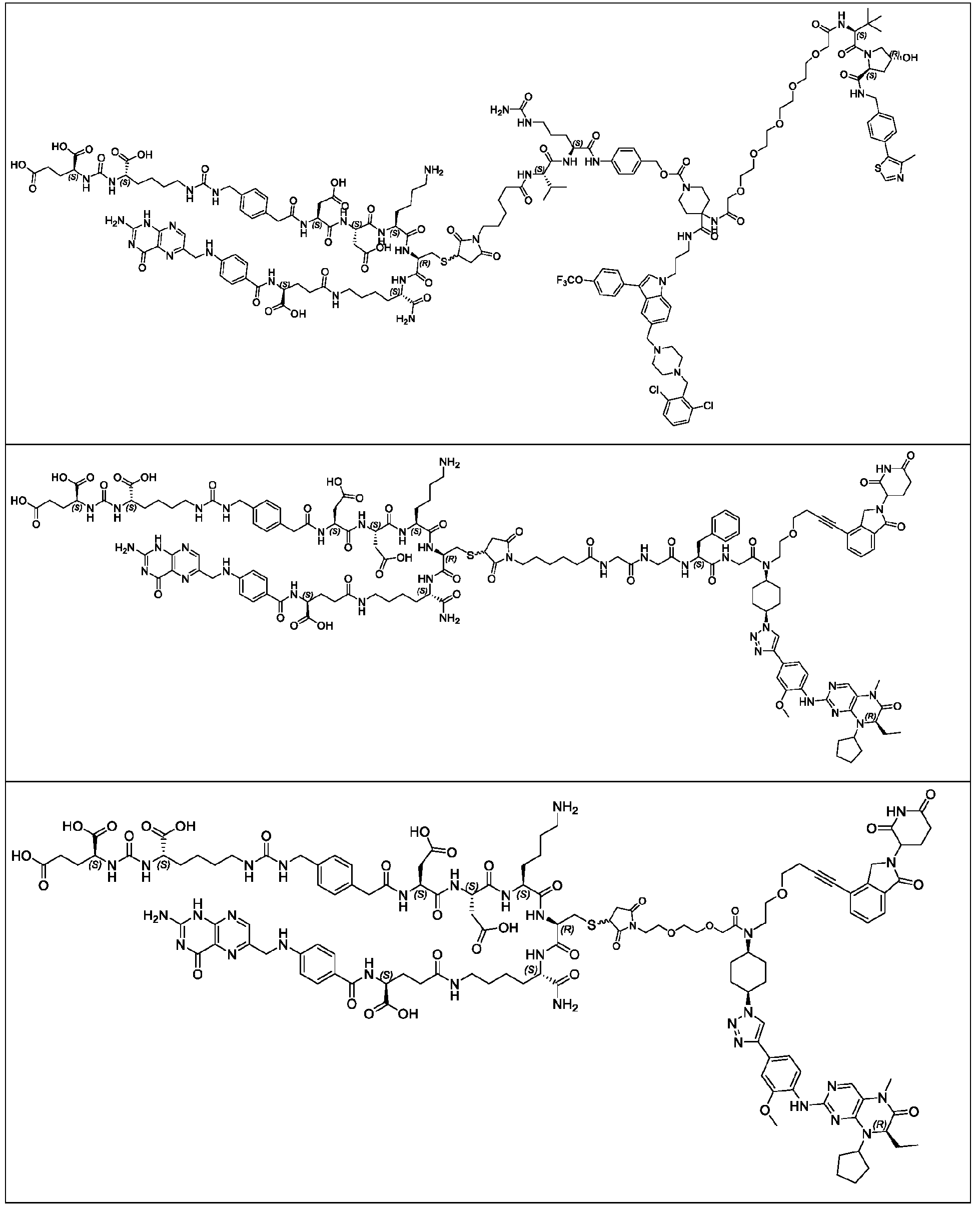

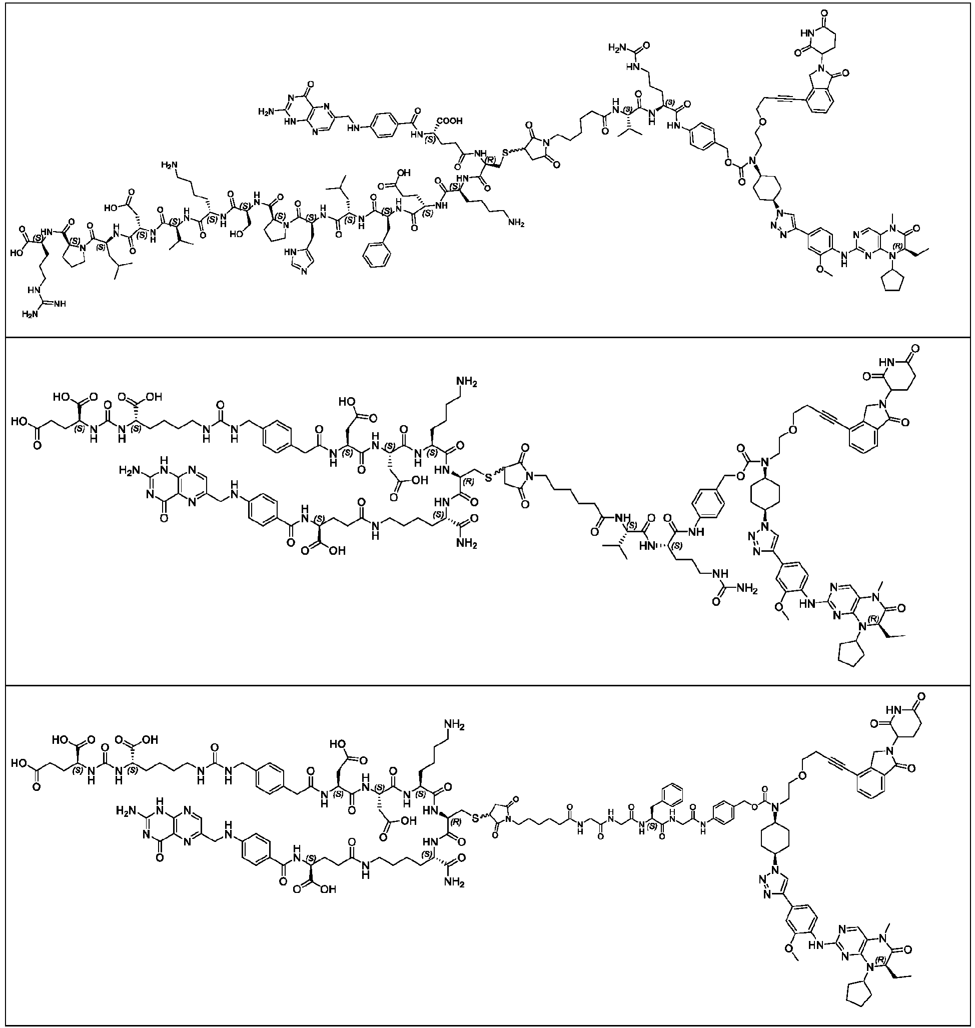

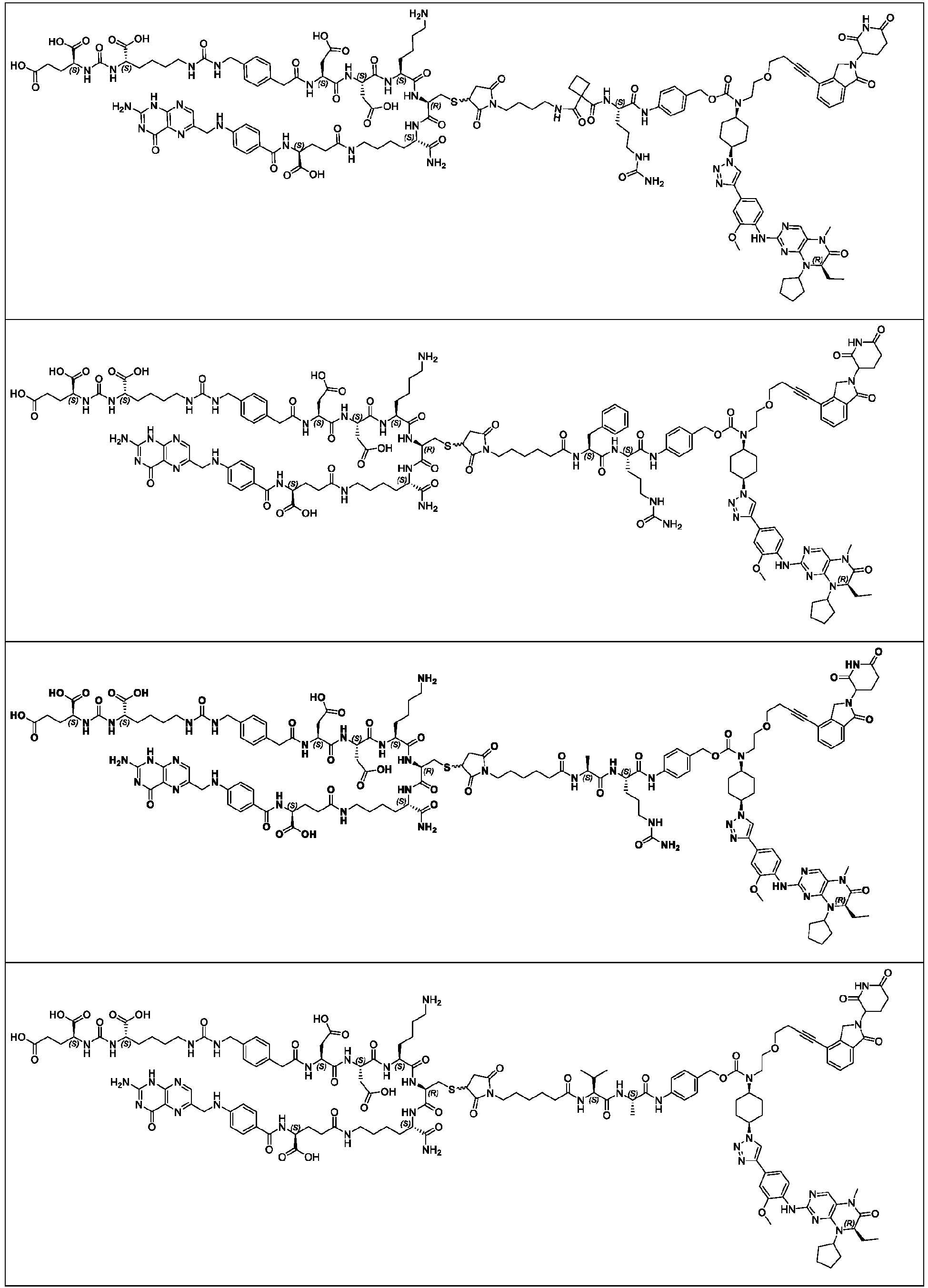

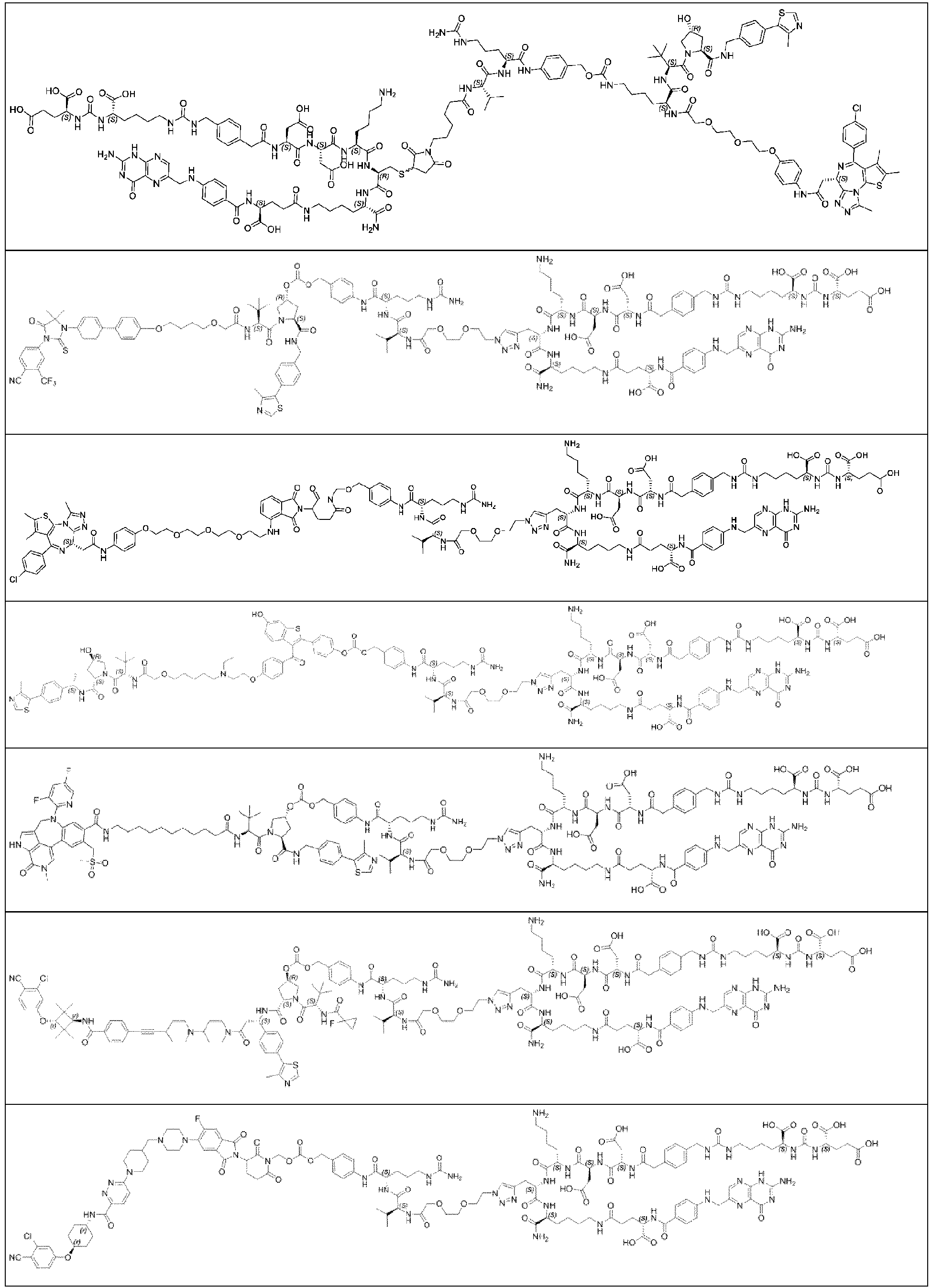

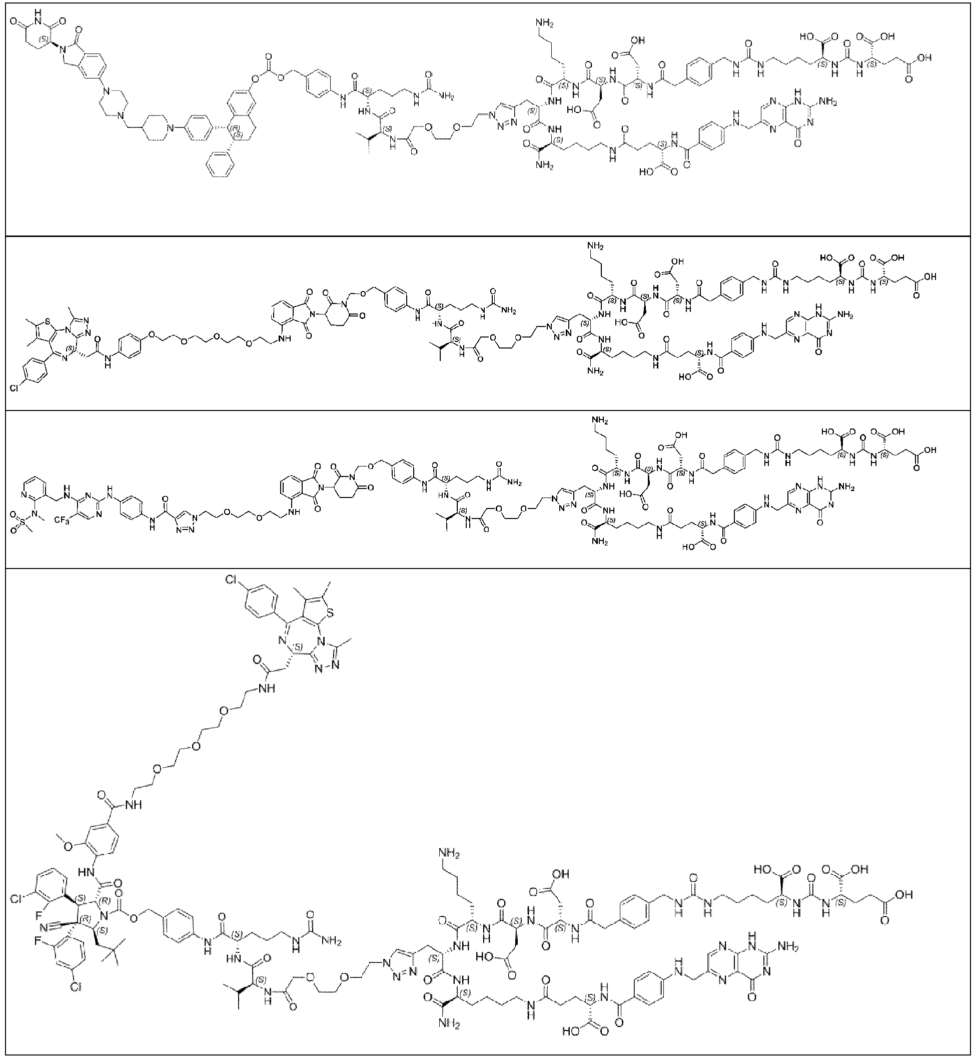

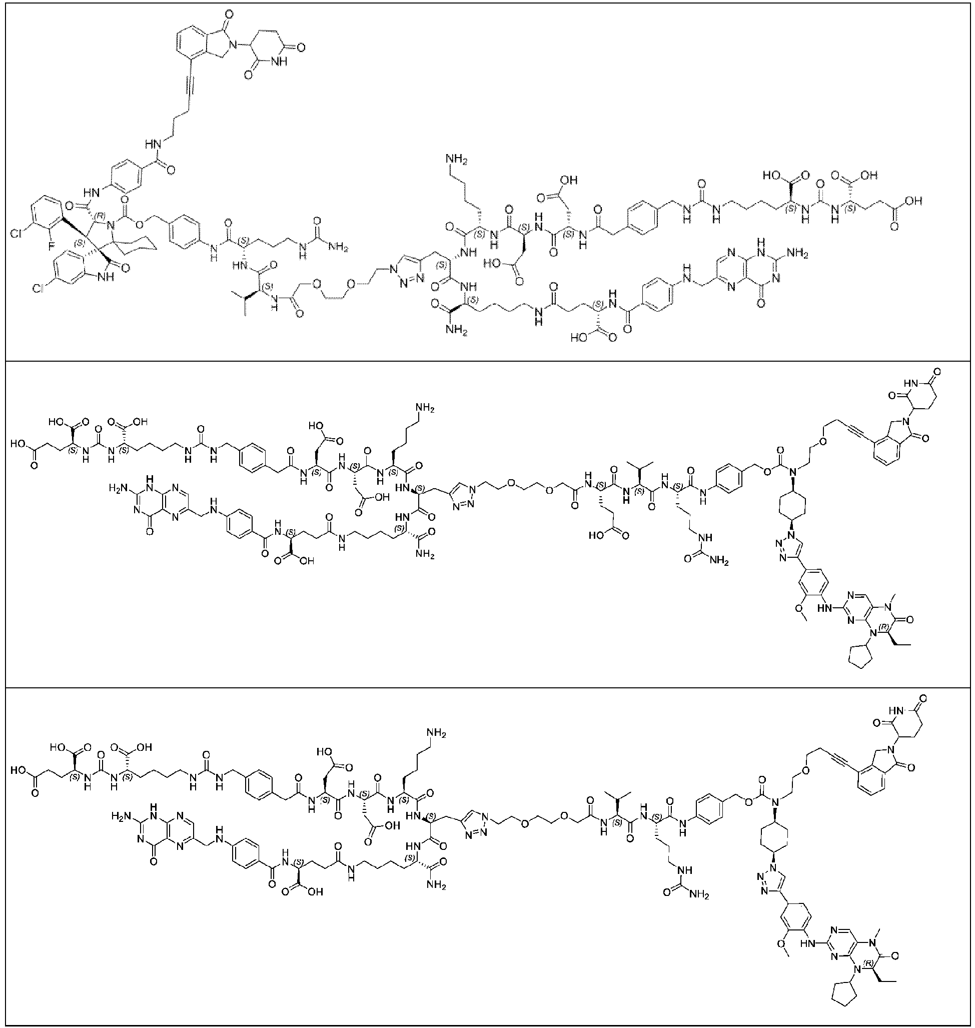

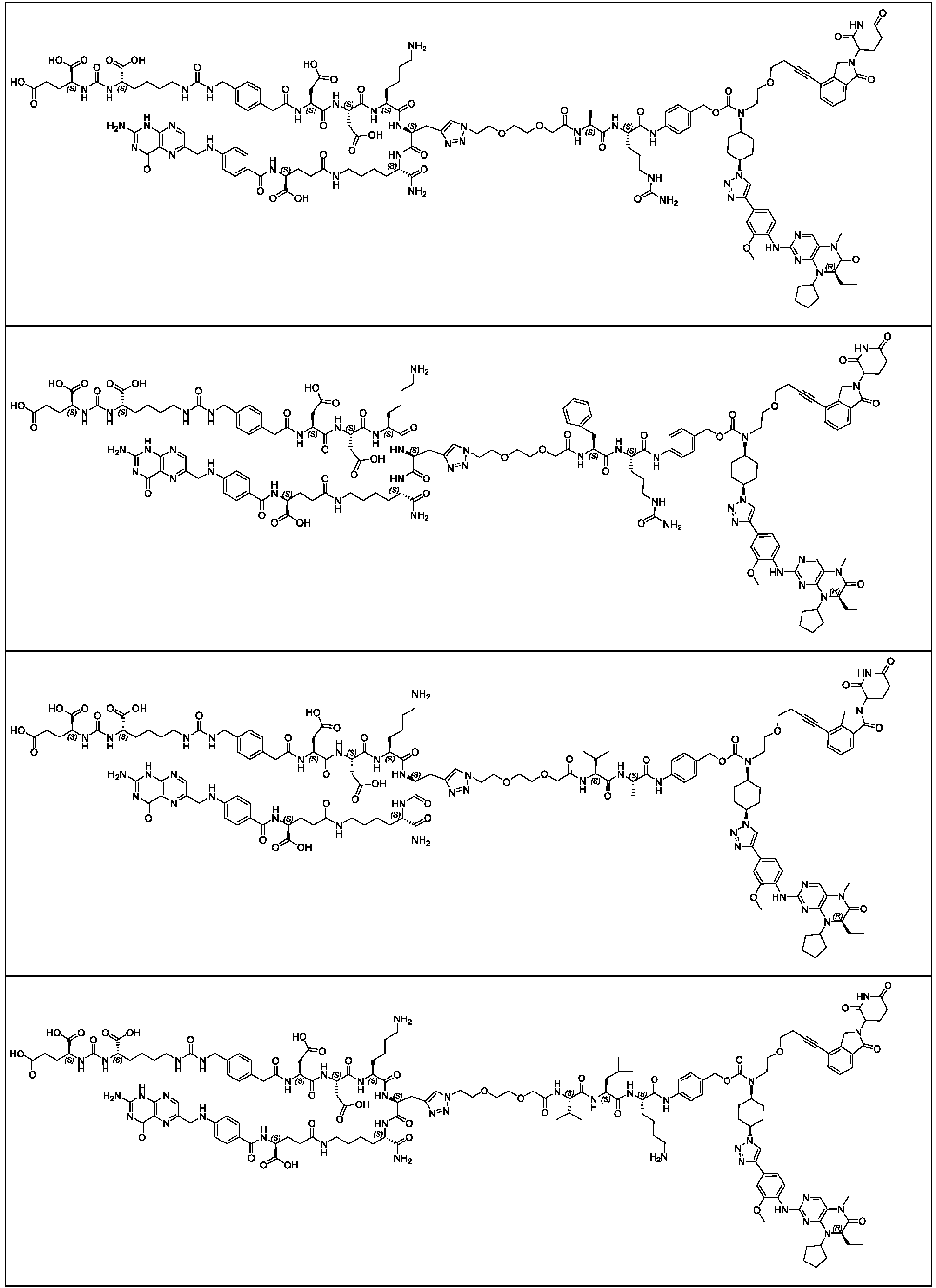

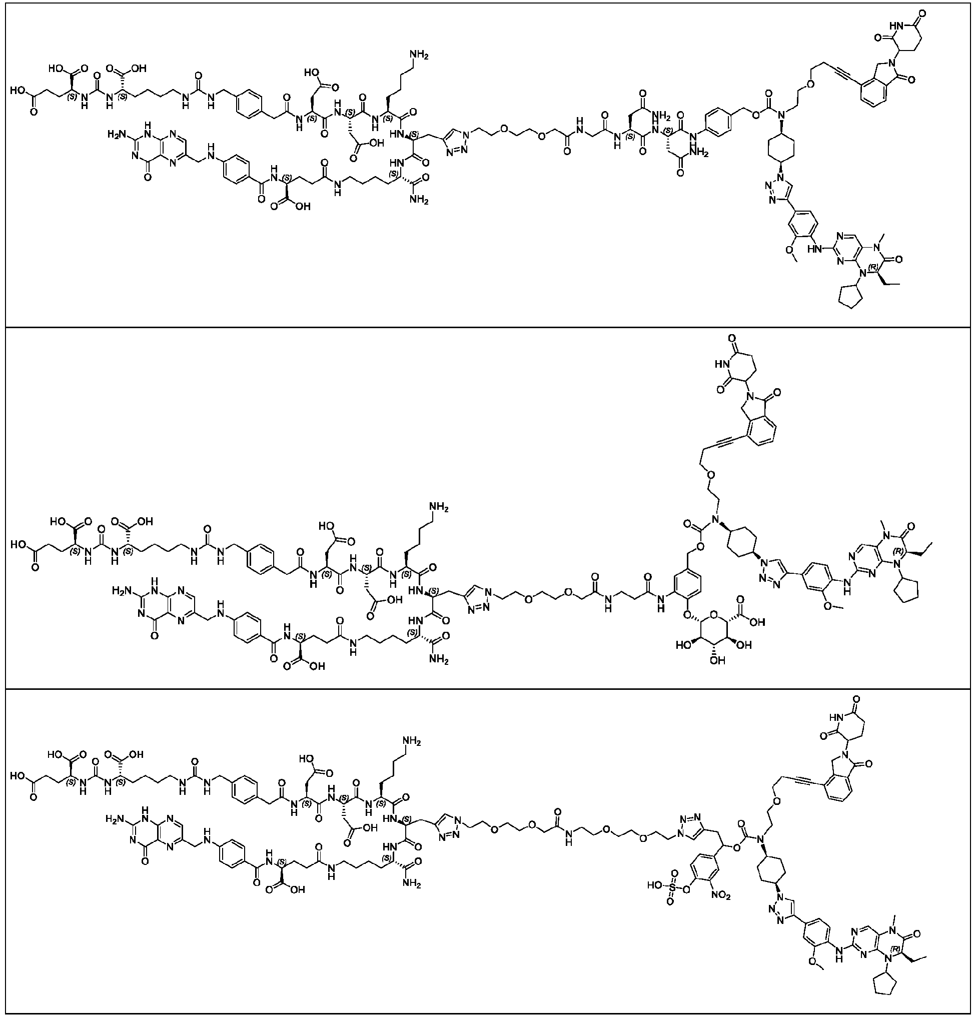

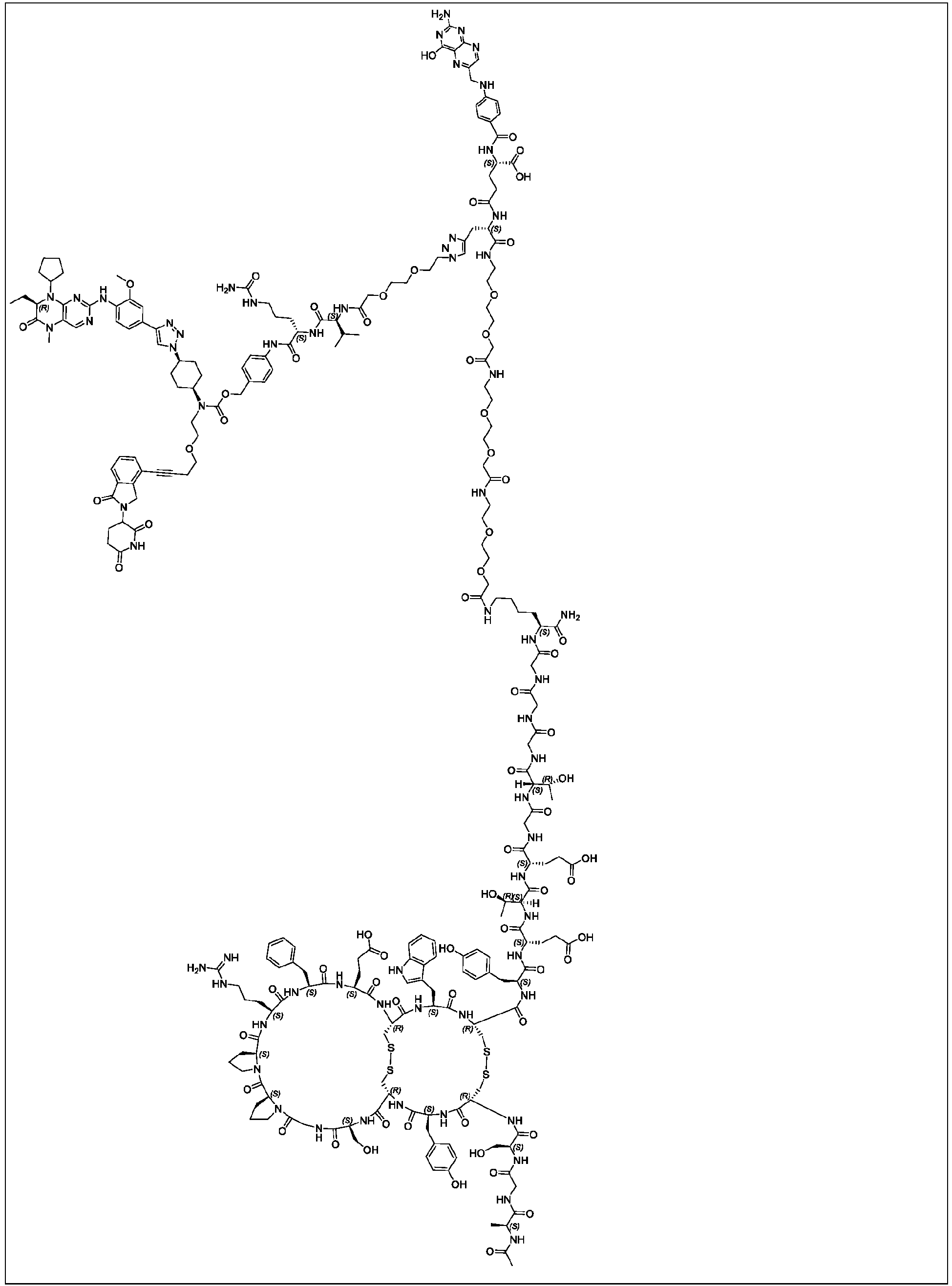

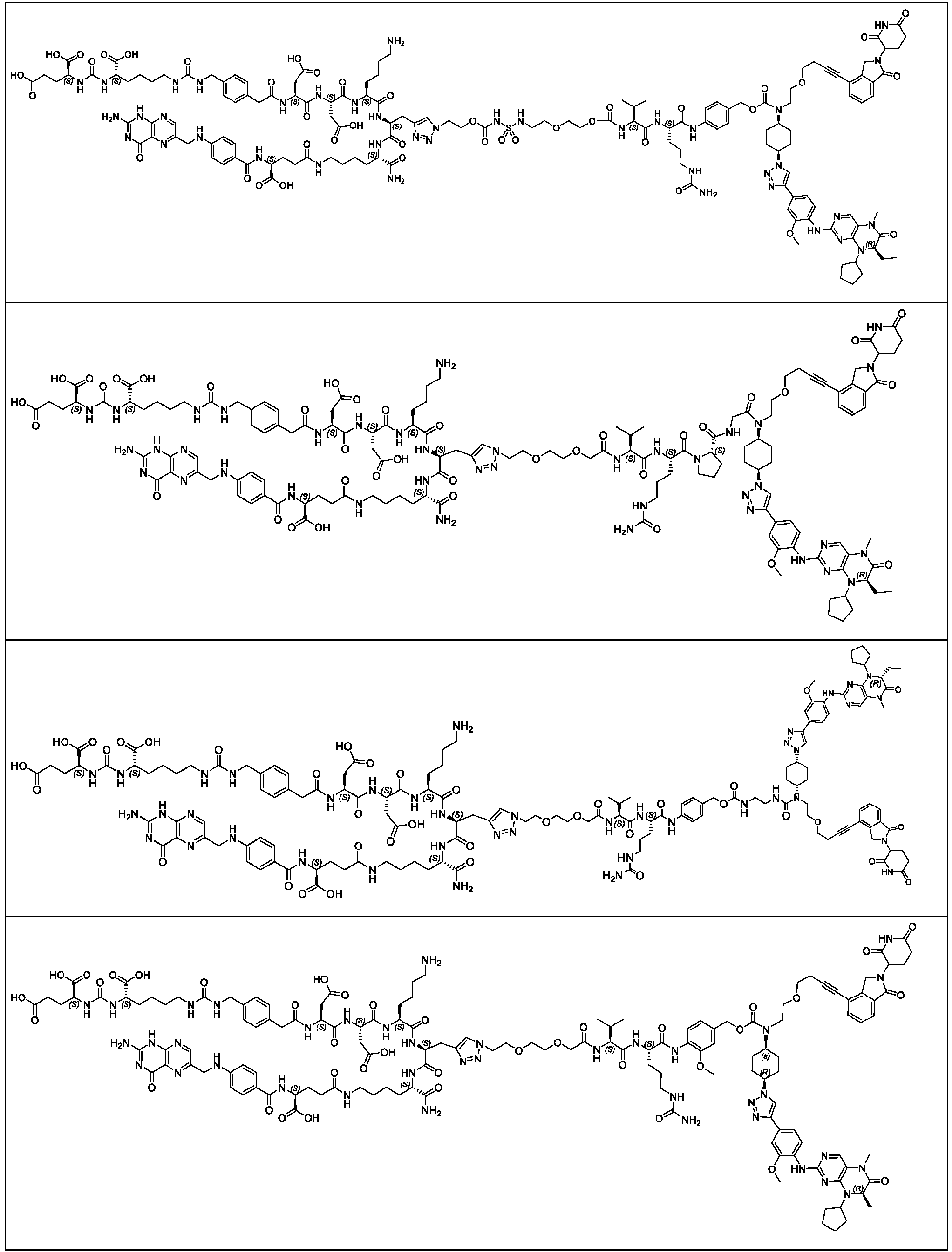

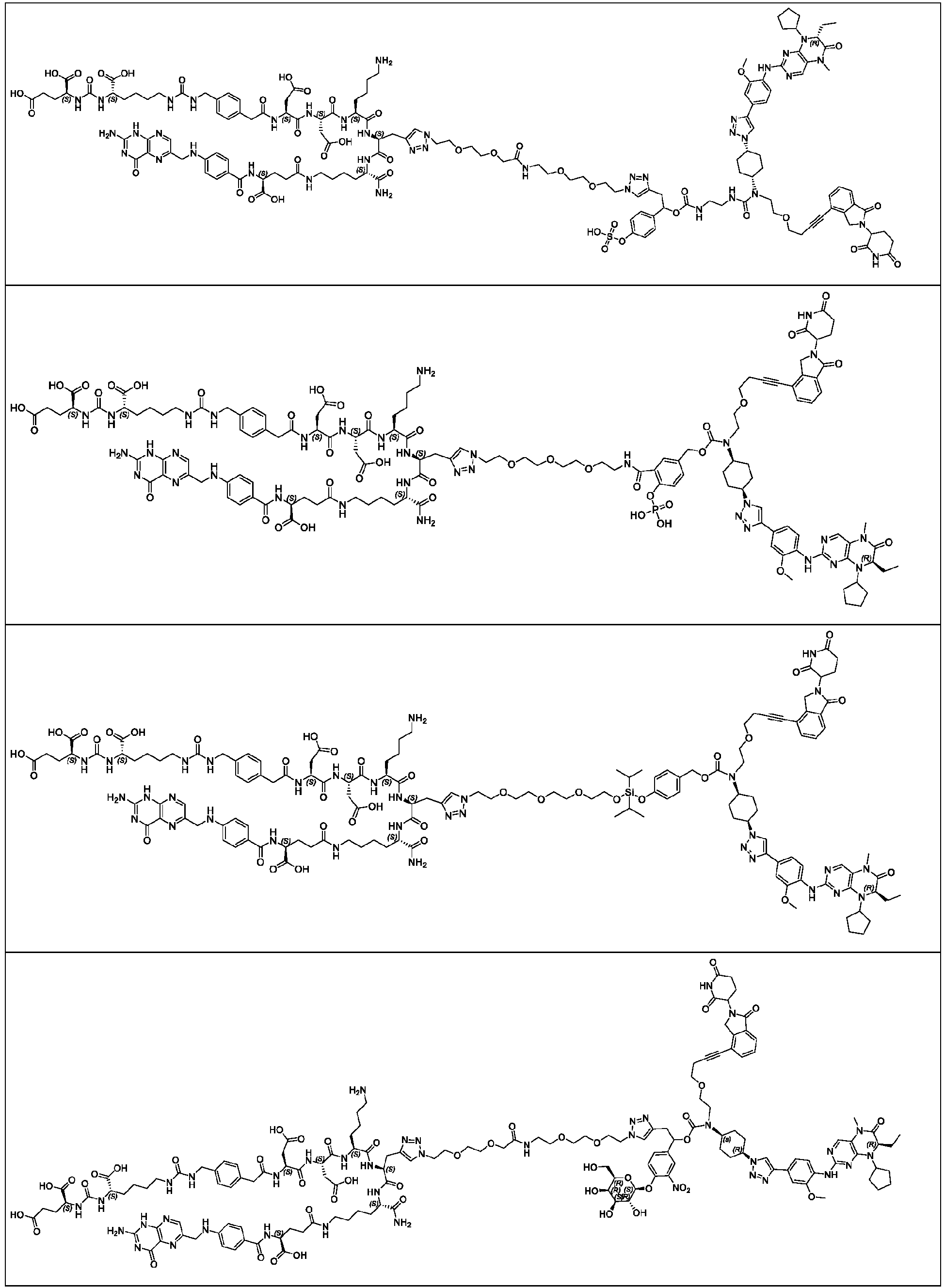

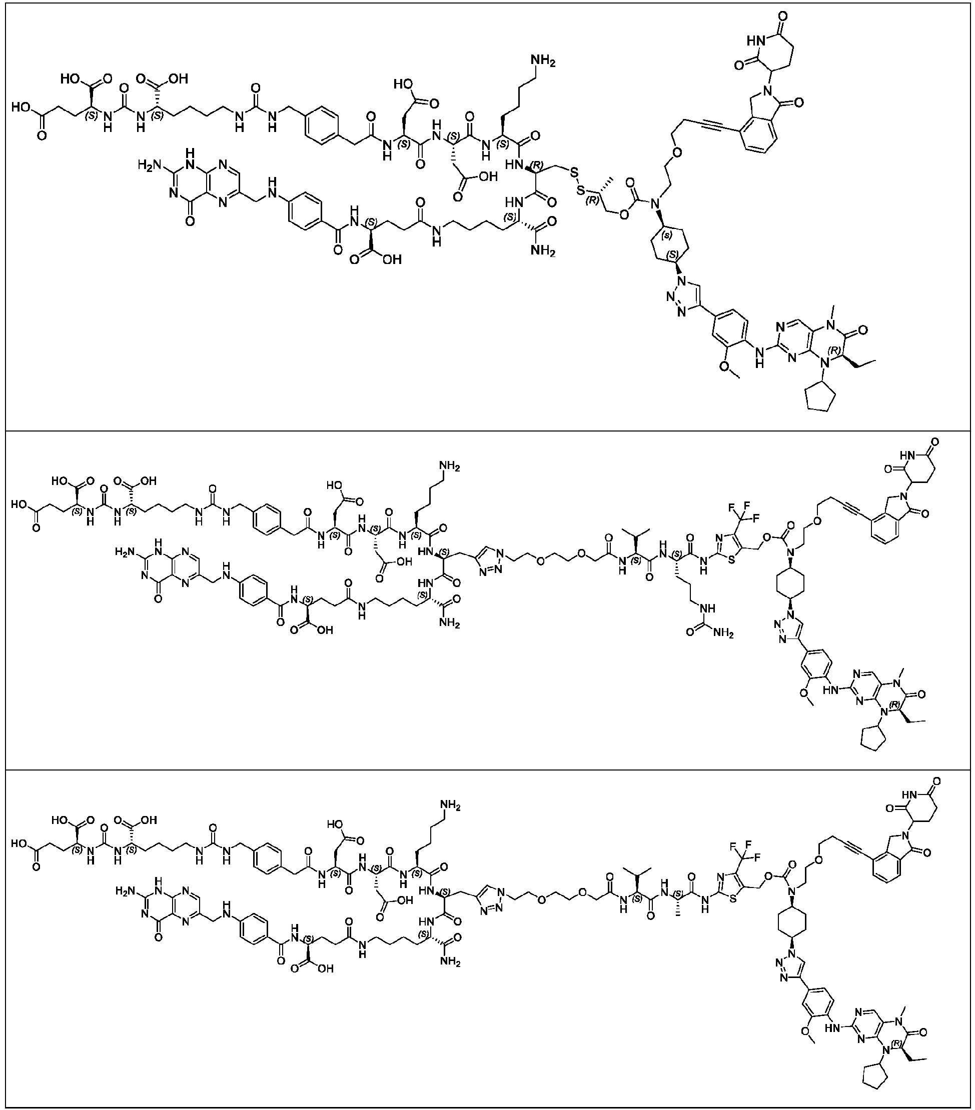

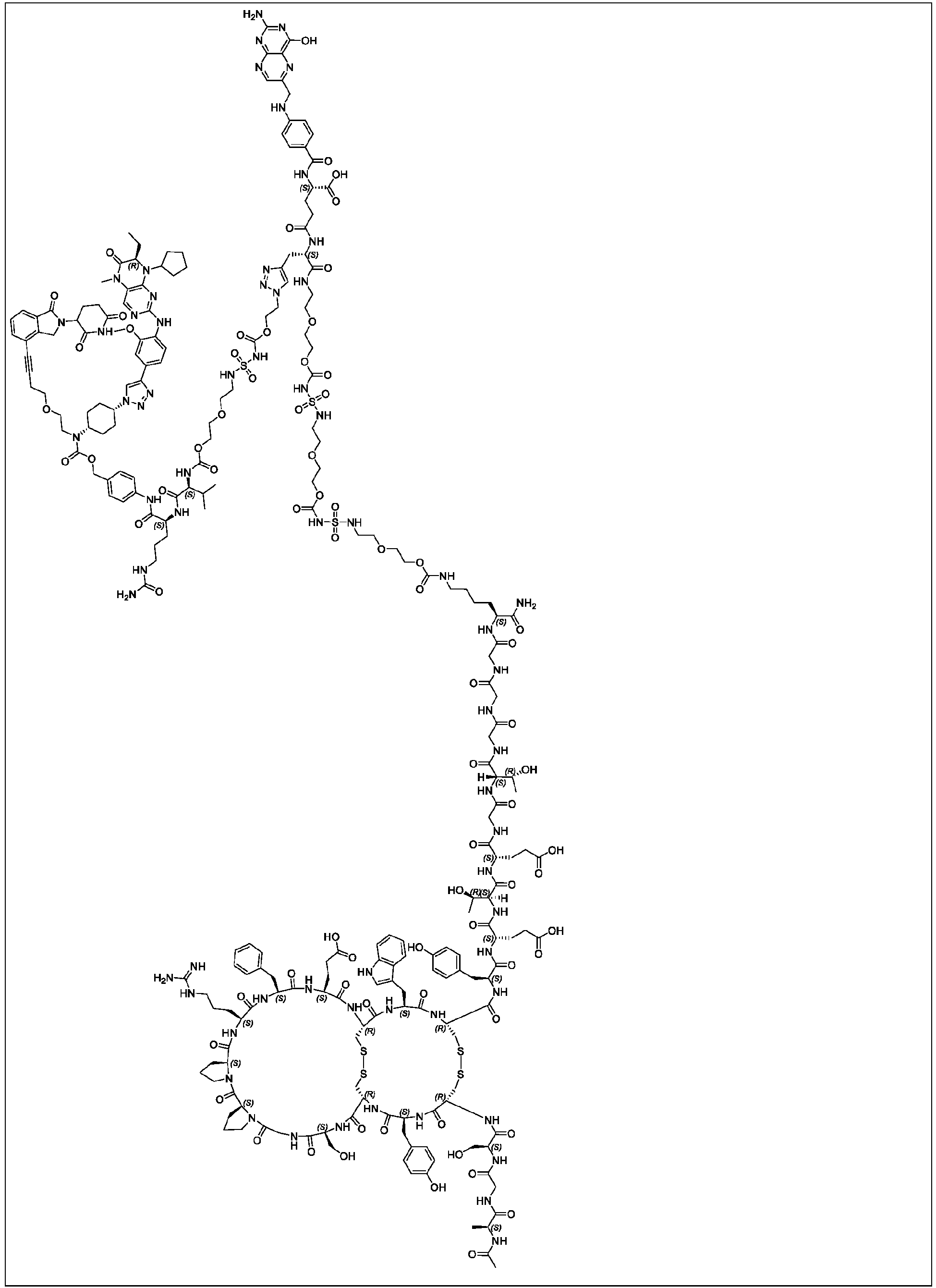

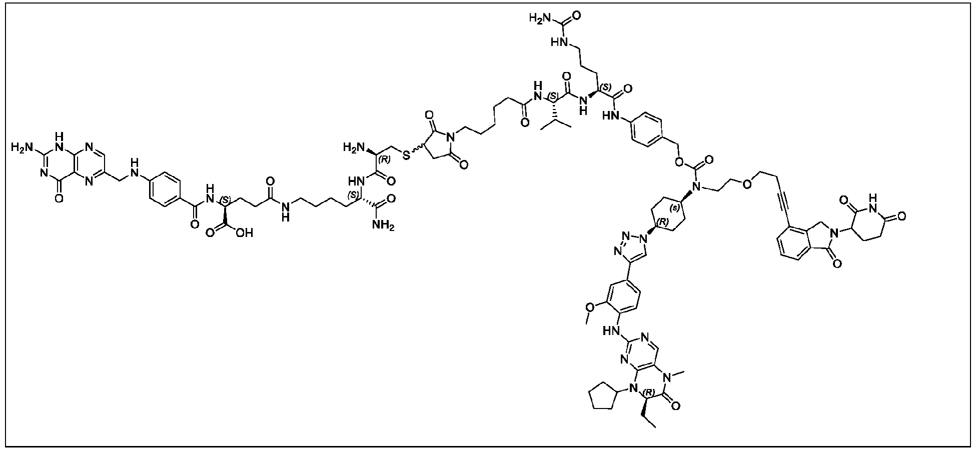

The term "conjugate compound" or "compound" used herein is intended to encompass all stereoisomers (e.g., enantiomers and diastereomers), geometric isomers, tautomers, and isotopic variants of the structures shown.

The conjugate compound or the compound described herein may be asymmetric (e.g., having one or more stereogenic centers). Unless otherwise indicated, all stereoisomers, such as enantiomers and diastereomers, are intended to be included. The conjugate compound or the compound of the present application containing asymmetrically substituted carbon atoms can be isolated in optically activated or racemic forms. Methods of preparing optically active forms from starting materials that are not optically active are well known in the art, such as by resolution of racemic mixtures or by stereoselective synthesis. Various geometric isomers of olefins, carbon-carbon double bonds, and the like may also be present in the conjugate compound or the compound described herein, and all such stable isomers have been contemplated in the present application. The present application describes the cis and trans geometric isomers of the conjugate compound or the compound, which may be isolated as mixtures of isomers or as individual isomers.

The racemic mixture of the compound can be resolved by any of a variety of methods well known in the art. An exemplary method comprises fractional crystallization using a chiral acid, which is an optically active salt-forming organic acid. Suitable resolving agents for the fractional recrystallization process are, for example, optically active acids (e.g., tartaric acid, diacetyltartaric acid, dibenzoyltartaric acid, mandelic acid, malic acid, the D and L forms of lactic acid) or various optically active camphorsulfonic acids. Other resolving agents suitable for the fractional crystallization process include stereoisomerically pure forms of N-methylbenzylamine, 2-phenylglycinol, norephedrine, ephedrine, N-methylephedrine, cyclohexylethylamine, 1,2-diaminocyclohexane, and the like.

The resolution of the racemic mixture can also be conducted by elution on a column containing an optically active resolving agent, such as dinitrobenzoylphenylglycine. Suitable eluent compositions can be determined by those skilled in the art.

The conjugate compound or the compound of the present application also includes tautomeric forms. Tautomeric forms result from the exchange of a single bond with an adjacent double bond accompanied by the migration of protons. The tautomeric forms include prototropic tautomers in isomeric protonated states with the same chemical formula and total charge. Examples of prototropic tautomers include keto-enol pairs, amide-imidic acid pairs, lactam-lactim pairs, enamine-imine pairs, and cyclic forms in which protons can occupy two or more positions of a heterocyclic ring system, such as 1H- and 3H-imidazole, 1H-, 2H- and 4H-1,2,4-triazole, 1H- and 2H-isoindole, and 1H- and 2H-pyrazole. The tautomeric forms can be balanced or sterically locked into one form by appropriate substitution.

The conjugate compound or the compound of the present application may also include all isotopic variants of atoms found in the intermediate or final compounds. Isotopes include those atoms having the same atomic number but different mass numbers. For example, isotopes of hydrogen include protium, deuterium, and tritium.

In some certain embodiments, the conjugate compound or the compound of the present application may be obtained by organic synthesis. The conjugate compound or the compound of the present application, including salts, esters, hydrates, or solvates thereof, may be prepared using any known organic synthesis techniques and may be synthesized according to a variety of possible synthetic routes.

The reaction for producing the conjugate compound or the compound of the present application may be conducted in an appropriate solvent, and the solvent can be easily selected by those skilled in the art of organic synthesis. Suitable solvents are substantially unreactive with the starting materials (reactants), intermediates, or products at a temperature for conducting the reaction (which may range, for example, from the freezing point of the solvent to the boiling point of the solvent). A given reaction may be conducted in one solvent or a mixture of more than one solvent. Depending on the particular reaction procedures, those skilled in the art will be able to select a suitable solvent for a particular reaction procedure.

The preparation of the conjugate compound or the compound of the present application may involve protection and deprotection of various chemical groups. Those skilled in the art can readily determine whether protection and deprotection are required and select appropriate protecting groups. Protecting group chemistry can be found, for example, in T. W. Greene and P. G. M. Wuts, Protective Groups in Organic Synthesis, 3rd Ed., Wiley & Sons, Inc., New York (1999), which is incorporated herein by reference in its entirety.

The reaction may be monitored according to any suitable methods known in the art. For example, the formation of the product may be monitored by spectroscopy, such as nuclear magnetic resonance spectroscopy (e.g., ¹H or ¹³C), infrared spectroscopy, spectrophotometry (e.g., UV-visible), mass spectrometry, or by chromatography, such as high-performance liquid chromatography (HPLC), liquid chromatography-mass spectrometry (LCMS), or thin layer chromatography (TLC). Those skilled in the art can purify the compounds by a variety of methods including high-performance liquid chromatography (HPLC; see, for example, "Preparative LC-MS Purification: Improved Compound Specific Method Optimization" Karl F. Blom, Brian Glass, Richard Sparks, Andrew P. Combs J. Combi. Chem. 2004, 6(6), 874-883, which is incorporated herein by reference in its entirety) and normal-phase silica gel column chromatography.

In one aspect, the present application discloses a pharmaceutical composition, comprising the conjugate compound or the pharmaceutically acceptable salt thereof disclosed herein, and a pharmaceutically acceptable carrier. In some embodiments, the pharmaceutical composition disclosed herein is for intravenous, subcutaneous, oral, intramuscular, or intraventricular administration.

The term "pharmaceutically acceptable" used herein refers to those, within the scope of sound medical judgment, suitable for use in contact with cells of human and other animals without undue toxicity, irritation, allergic response, and the like, and commensurate with a proper benefit/risk ratio.

The term "pharmaceutically acceptable salt" used herein refers to relatively non-toxic, inorganic and organic acid addition and base addition salts of the conjugate compound of the present application. Representative acid addition salts include hydrobromide, hydrochloride, sulfate, bisulfate, phosphate, nitrate, acetate, oxalate, valerate, oleate, palmitate, stearate, laurate, borate, benzoate, lactate, phosphate, tosylate, citrate, maleate, fumarate, succinate, tartrate, naphthenate, mesylate, glucoheptonate, lactobionate, sulfamate, malonate, salicylate, propionate, methylene-bis-*b*-hydroxynaphthoate, gentisate, isethionate, di-*p*-toluoyltartrate, methanesulfonate, ethanesulfonate, benzenesulfonate, *p*-toluenesulfonate, cyclohexylsulfamate, and quinate laurylsulfonate, and the like. Base addition salts include pharmaceutically acceptable metal and amine salts. Suitable metal salts include sodium, potassium, calcium, barium, zinc, magnesium, and aluminum salts. In some embodiments, sodium and potassium salts are preferred. Suitable inorganic base addition salts are prepared from metal bases including, for example, sodium hydride, sodium hydroxide, potassium hydroxide, calcium hydroxide, aluminum hydroxide, lithium hydroxide, magnesium hydroxide, and zinc hydroxide. Suitable amine base addition salts are prepared from amines which are sufficiently basic to form stable salts, and preferably include the following amines which are commonly used in pharmaceutical chemistry due to their low toxicity and acceptable for medical use: ammonia, ethylenediamine, N-methylglucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, tetramethylammonium hydroxide, triethylamine, dibenzylamine, diphenylmethylamine, dehydroabietylamine, N-ethylpiperidine, benzylamine, tetramethylammonium, tetraethylammonium, methylamine, dimethylamine, trimethylamine, ethylamine, basic amino acids (e.g., lysine and arginine), dicyclohexylamine, and the like.

The term "pharmaceutically acceptable carrier" used herein refers to a pharmaceutically acceptable solvent, suspension, or any other pharmaceutically inert carrier for delivering the conjugate compound provided herein to a subject that does not interfere with the structure and properties of the conjugate compound. Some such carriers enable the conjugate compound to be formulated, for example, as tablets, pills, capsules, liquids, gels, syrups, slurries, suspensions, and pastilles, for oral ingestion by a subject. Some such carriers enable the conjugate compound to be formulated for injection, infusion, or topical administration.

Pharmaceutically acceptable carriers for use in the pharmaceutical compositions provided herein include, but are not limited to, for example, pharmaceutically acceptable liquid, gel or solid carriers, aqueous carriers (e.g., sodium chloride injection, Ringer's injection, isotonic dextrose injection, sterile water injection, or dextrosed in lactated Ringer's injection), non-aqueous carriers (e.g., fixed oils of vegetable origin, cottonseed oil, corn oil, sesame oil, or peanut oil), antimicrobials, isotonic agents (e.g., sodium chloride or dextrose), buffers (e.g., phosphoric acid or citric acid buffers), antioxidants (e.g., sodium bisulfate), anesthetics (e.g., procaine hydrochloride), suspending/dispersing agents (e.g., sodium carboxymethylcellulose, hydroxypropylmethylcellulose, or polyvinylpyrrolidone), chelating agents (e.g., EDTA (ethylenediaminetetraacetic acid) or EGTA (ethylene glycol tetraacetic acid)), emulsifiers (e.g., polysorbate 80 (Tween-80)), diluents, adjuvants, excipients, or nontoxic auxiliary substances, other ingredients well known in the art, or various combinations thereof. Suitable ingredients may include, for example, fillers, binders, buffers, preservatives, lubricants, flavorings, thickeners, colorants, or emulsifiers.

In some embodiments, the pharmaceutical composition is an injectable formulation. The injectable formulation includes sterile aqueous solutions or dispersions, suspensions, or emulsions. In all cases, the injectable formulation should be sterile and should be fluid to facilitate injection. The injectable formulation should remain stable in the conditions of manufacture and storage and must be protected from microbial contamination by, e.g., bacteria and fungi. The carrier may be a solvent or dispersing medium containing, for example, water, ethanol, polyol (for example, glycerol, propylene glycol, and liquid polyethylene glycol), and suitable mixtures thereof and/or a vegetable oil. The injectable formulation should maintain proper fluidity. For example, proper fluidity can be maintained by the use of coatings such as lecithin, by the use of surfactants, and the like. The action of microorganisms can be prevented by various antibacterial and antifungal agents, for example, parabens, chlorobutanol, phenol, sorbic acid, and thimerosal.

In some embodiments, the pharmaceutical composition is an oral formulation. The oral formulation includes, but is not limited to, capsules, cachets, pills, tablets, lozenges (using a flavored medium, typically sucrose and acacia or tragacanth), powders, granules, or solutions or suspensions in aqueous or non-aqueous liquids, or as oil-in-water or water-in-oil liquid emulsions, or as elixirs or syrups, or as pastilles (using an inert medium, such as gelatin and glycerin, or sucrose and acacia), and/or as mouthwashes, and the like.

In solid dosage forms for oral administration (e.g., capsules, tablets, pills, dragees, pulvis, granules, etc.), the conjugate compound is mixed with one or more pharmaceutically acceptable carriers, such as sodium citrate or dicalcium phosphate, and/or any one of the following: (1) fillers or extenders, e.g., starches, lactose, sucrose, glucose, mannitol, and/or silicic acid; (2) binders, e.g., carboxymethylcellulose, alginates, gelatin, polyvinylpyrrolidone, sucrose, and/or acacia; (3) humectants, e.g., glycerol; (4) disintegrating agents, e.g., agar, calcium carbonate, potato or tapioca starch, alginic acid, certain silicates, and sodium carbonate; (5) solution retarding agents, e.g., paraffin; (6) absorption promoters, e.g., quaternary ammonium compounds; (7) wetting agents, e.g., acetol and glycerol monostearate; (8) absorbents, e.g., kaolin and bentonite; (9) lubricants, e.g., talc, calcium stearate, magnesium stearate, solid polyethylene glycols, sodium lauryl sulfate, and mixtures thereof; and (10) colorants.

In liquid dosage forms for oral administration, the conjugate compound is mixed with any one of the following: pharmaceutically acceptable emulsions, microemulsions, solutions, suspensions, syrups, and elixirs. In addition to the conjugate compound, the liquid dosage forms may contain inert diluents commonly used in the art such as water or other solvents, and solubilizing agents and emulsifiers such as ethanol, isopropanol, ethyl carbonate, ethyl acetate, benzyl alcohol, benzyl benzoate, isopropanol, 1,3-butylene glycol, oils (in particular, cottonseed, peanut, corn, olive, castor, and sesame oils), glycerol, tetrahydrofurfuryl alcohol, polyethylene glycols, and fatty acid esters of sorbitan, and mixtures thereof. In addition to inert diluents, the oral compositions may also contain adjuvants such as wetting agents, emulsifying and suspending agents, sweeteners, flavorings, colorants, fragrances, and preservatives.

In some embodiments, the pharmaceutical composition is an oral spray formulation or a nasal spray formulation. Spray formulations include, but are not limited to, aqueous aerosols, non-aqueous suspensions, liposomal formulations, solid granule formulations, and the like. Aqueous aerosols are prepared by mixing an aqueous solution or suspension of the agent with a conventional pharmaceutically acceptable carrier and stabilizer. The carrier and stabilizer may vary according to the requirements of the particular compound, but in general, may include non-ionic surfactants (Tween or polyethylene glycol), oleic acid, lecithin, amino acids such as glycine, buffered solutions, salts, saccharides, or sugar alcohols. Aerosols are typically prepared from isotonic solutions and can be delivered by spraying.

In some embodiments, the pharmaceutical composition may be used by mixing with one or more additional therapeutics. In some embodiments, the pharmaceutical composition comprises at least one additional therapeutic. In some embodiments, the additional therapeutic is an anti-tumor therapeutic, a cardiovascular therapeutic, an anti-inflammatory therapeutic, an antiviral therapeutic, a digestive system therapeutic, a nervous system therapeutic, a respiratory system therapeutic, an immune system therapeutic, a dermatological therapeutic, a metabolic therapeutic, or the like.

In some embodiments, the pharmaceutical composition may be administered to a subject in need via a suitable route, including but not limited to oral, injection (e.g., intravenous, intramuscular, subcutaneous, intradermal, intracardiac, intrathecal, intrapleural, intraperitoneal injection, etc.), transmucosal (e.g., intranasal, intraoral administration, etc.), sublingual, rectal, transdermal, intraocular, and pulmonary administration. In some embodiments, the pharmaceutical composition may be administered intravenously, subcutaneously, orally, intramuscularly, or intraventricularly.

Due to the nature of some payloads, e.g., high toxicity or high hydrophilicity, it is desirable to deliver the payload more specifically and efficiently to a subject in need. For example, in cancer therapies, it is desirable to deliver chemotherapeutic agents specifically to cancer cells without harming normal cells. Thus, in another aspect, the present application discloses a method for delivering a payload to a subject in need, comprising: administering to the subject a therapeutically effective amount of the conjugate compound or the pharmaceutically acceptable salt thereof provided herein or the pharmaceutical composition provided herein. The payload described herein can be any therapeutic that elicits a biological or medical response in a tissue, system, animal subject, or human and is being sought by an investigator, veterinarian, physician, or other healthcare provider to prevent, inhibit, ameliorate, or treat a disease.

In one aspect, the present application discloses a method for treating a disease in a subject, comprising administering to the subject a therapeutically effective amount of the conjugate compound or the pharmaceutically acceptable salt thereof disclosed herein, or the pharmaceutical composition disclosed herein.

The term "therapeutically effective amount" used herein refers to an amount of the conjugate compound or the pharmaceutically acceptable salt thereof or the pharmaceutical composition that alleviates to some extent one or more symptoms of a disease or disorder in a subject; an amount that partially or completely normalizes one or more physiological or biochemical indicators associated with or resulting in a disease or disorder; and/or an amount that reduces the possibility of developing a disease or disorder. Such amounts will generally vary depending on a variety of factors that can be determined and explained by those of ordinary skills in the art based on the scope of the description provided herein. These include, but are not limited to: the specific subject and the age, weight, height, general physical condition and medical history of the subject, the specific compound used, the carrier of the formulation, and the chosen route of administration, as well as the nature and severity of the condition.

In some embodiments, the conjugate compound or the pharmaceutically acceptable salt thereof, or the pharmaceutical composition, is in an amount sufficient to inhibit a disease or disorder in a subject, or prophylactically inhibit or prevent the development of a disease or disorder. Although the therapeutically effective amount may vary from subject to subject, it typically ranges from 0.01 to 100 mg/kg, for example, 0.01 to 90 mg/kg, 0.01 to 80 mg/kg, 0.01 to 70 mg/kg, 0.01 to 60 mg/kg, 0.01 to 50 mg/kg, 0.01 to 40 mg/kg, 0.01 to 30 mg/kg, 0.01 to 20 mg/kg, 0.01 to 10 mg/kg, 0.01 to 5 mg/kg, 0.01 to 4 mg/kg, 0.01 to 3 mg/kg, 0.01 to 2 mg/kg, 0.01 to 1 mg/kg, or 0.01 to 0.1 mg/kg. The therapeutically effective amount described herein may be equivalent to any value within the above numerical ranges, including the endpoints of the ranges.

In another aspect, the present application discloses a method for treating a disease in a subject, comprising administering to the subject a therapeutically effective amount of the conjugate compound or the pharmaceutically acceptable salt thereof disclosed herein or the pharmaceutical composition disclosed herein. In some embodiments, the method further comprises administering one or more therapeutic agents in combination with the conjugate compound or the pharmaceutically acceptable salt thereof or the pharmaceutical composition.

In yet another aspect, the present application discloses use of the conjugate compound or the pharmaceutically acceptable salt thereof disclosed herein, or the pharmaceutical composition disclosed herein in preparing a medicament for treating a disease in a subject.

The term "subject" used herein refers to both humans and non-human animals. Non-human animals include all vertebrates, such as mammals and non-mammals. The subject may also be a livestock, e.g., cattle, pigs, sheep, poultry and horses, or a domestic animal, e.g., dogs and cats. The subject may be male (e.g., man) or female (e.g., woman), and may be a senior, an adult, a juvenile, a child, or an infant. The human may be caucasian, African, Asian, semite, or a person of other ethnic background or mixed-race background.

In some embodiments, the disease described herein is selected from the following group: a cancer, an immune disease, a metabolic disease, and a neurological disease. In some embodiments, the disease is characterized by high or abnormal expression of a cell surface receptor, a cell surface antigen, or a specific protein by cells in an organ or tissue in the subject. In some embodiments, the cell surface receptor is selected from FOLR1, TRPV6, and c-Met. In some embodiments, the cell surface antigen is selected from PSMA. In some embodiments, the specific protein is selected from a nuclear receptor (e.g., AR and ER), a kinase-like protein (e.g., RIPK2, BCR-ABL, EGFR, HER2, c-Met, TBK1, CDK2/4/6/9, ALK, Akt, CK2, ERK1/2, FLT3, PI3K, BTK, and FAK), AKT, PAN-BET protein, BET protein (e.g., BRD2, BRD3, BRD4, and BRD6), BLK, BRAF1, BCL-xl, ERRα, FKBP12, FRS2α, JAK1, JAK3, IKZF1, IRAK4, MDM2, MetAP2, PLK1, PSK-J3, RAS, TACC3, Tau, TrkB, MDM2, or any combination thereof.

In some embodiments, the cancer cell or other physiologically dysfunctional cell possesses the expression of the cell surface receptor or antigen described herein. In some embodiments, the cancer cell or other physiologically dysfunctional cell possesses high expression of the cell surface receptor or antigen described herein (e.g., according to Depmap data (see https://depmap.org/portal/), the corresponding gene expression is at least 0, 0.01, 0.05, 0.1, 0.5, 1, 2, 3, 4, 5, 6, 7, 8, 9, 10, or 10 or greater). In some embodiments, the cancer cell or other physiologically dysfunctional cell possesses high expression of FOLR1, TRPV6, PSMA, c-Met, or any combination thereof.

In another aspect, the present application discloses a compound or a salt thereof, wherein the compound has the following structure:

L^{a}-(EG)n¹-(D)n⁵-(CH₂)n²-(B)n⁶-(AA)n³-(CH₂CH₂-Q)n⁴-L^{b},

wherein,
L^{a} is 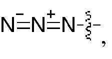 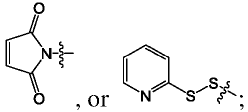
EG is -CH₂CH₂O- or -OCH₂CH₂-; n¹ is an integer of 0-6;
n² is an integer of 0-8;
D is -C(=O)-NH-S(=O)₂-NH-; n⁵ is 0 or 1;
B is -C(=O)-, 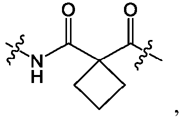 -NH-CH₂CH₂-C(=O)-, -C(=O)-NH-, -CH(CH₃)-CH₂-O-, - CH₂CH₂O-, -NH-C(=O)-, or -NH-; n⁶ is an integer of 0-3;
AA is each independently an amino acid residue; n³ is an integer of 0-5;
Q is each independently -C(=O)-, -O-, or a bond; n⁴ is an integer of 0-3;
L^{b} is 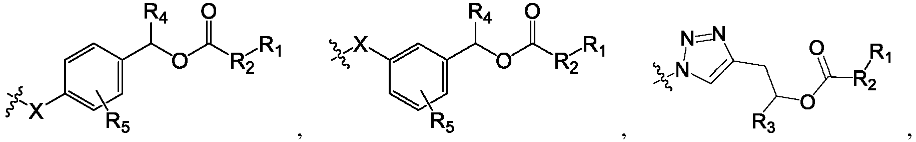 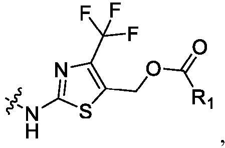 -OH, or -NH-CH₂-OH;
wherein X is a bond, -NH-, -C(=O)-, oxygen, or 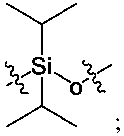
R₂ is a bond, -O-CH₂-, or -NH-CH₂CH₂-NH-C(=O)-;
R₃ is hydrogen or 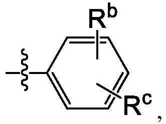 wherein R^{b} and R^{c} are each independently hydrogen, nitro, 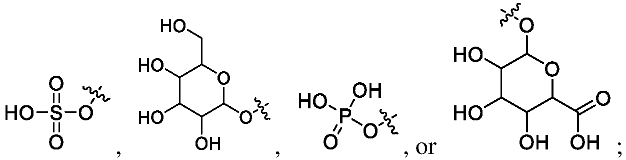
R₄ is hydrogen or C₁₋₈ alkyl;
R₅ is hydrogen, amino, nitro, phosphate group, -OR^{a}, or 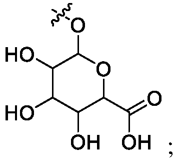 wherein R^{a} is methyl, ethyl, propyl, or isopropyl;
R₁ is a leaving group, preferably R₁ is halogen (e.g., chlorine),

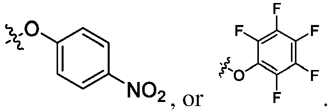

In some embodiments, EG is -CH₂CH₂O-. In some embodiments, n¹ is 2. In some embodiments, n¹ is 0. In some embodiments, n² is 1. In some embodiments, n² is 4. In some embodiments, n² is 5. In some embodiments, n⁵ is 0. In some embodiments, n⁶ is 1. In some embodiments, n⁶ is 3. In some embodiments, n¹ is 2, n² is 1, n³ and n⁴ are 0, and L^{b} is -OH.

In some embodiments, AA is each independently an amino acid residue formed by Val, Cit, Glu, Leu, Lys, Ala, Gly, Phe, Gln, Pro, or Asn. In some embodiments, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Cit-, -Val-Leu-Lys, -Val-Cit-Pro-Gly-, -Gly-Asn-Asn-, and -Ala-Ala-Asn-. In some embodiments, when n⁴ is 1, Q is -C(=O)-; when n⁴ is 2, Q are -O- and -C(=O)-, respectively; when n⁴ is 3, Q are -O-, -O-, and a bond, respectively. In some embodiments, n⁴ is 0.

In some embodiments, L^{b} is 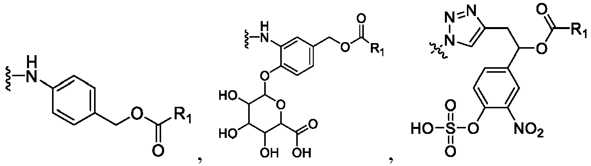 , -OH, -NH-CH₂-OH,

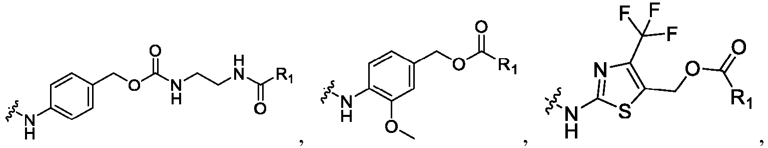

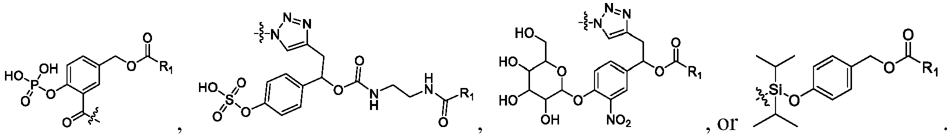

In some embodiments, the compound has the following structure:

L^{a}-(EG)n¹-(D)n⁵-(CH₂)n²-(B)n⁶-(AA)n³-(CH₂CH₂-Q)n⁴-L^{b}

wherein,
L^{a} is 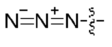 or 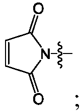
EG is -CH₂CH₂O-; n¹ is an integer of 2-4;
D is -C(=O)-NH-S(=O)₂-NH-; n⁵ is 0 or 1;
n² is an integer of 0-5;
B is -C(=O)-, -C(=O)-NH-, -CH₂CH₂O-, -NH-CH₂CH₂-C(=O)-, or -NH-; n⁶ is an integer of 0-3;
AA is each independently an amino acid residue; n³ is an integer of 0-3;
Q is each independently -C(=O)-, -O-, or a bond; n⁴ is an integer of 0-3;
L^{b} is -OH, 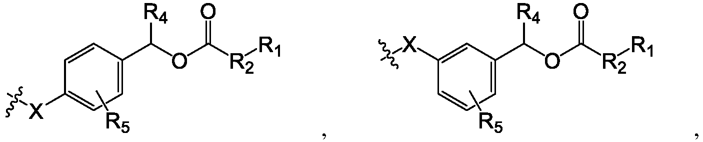 or 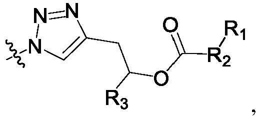
wherein X is a bond, -NH-, -C(=O)-, or 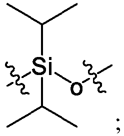
R₂ is a bond;
R₃ is 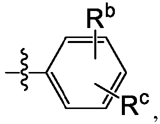 wherein R^{b} and R^{c} are each independently hydrogen, nitro, or 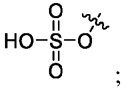
R₄ is hydrogen;
R₅ is hydrogen, -OCH₃, or 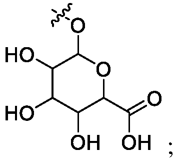
R₁ is a leaving group, preferably R₁ is halogen (e.g., chlorine),

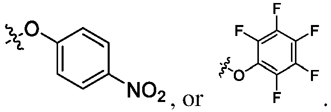

In some embodiments, L^{b} is -OH,

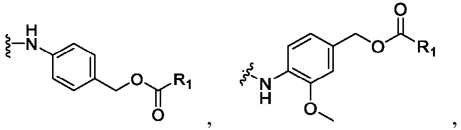

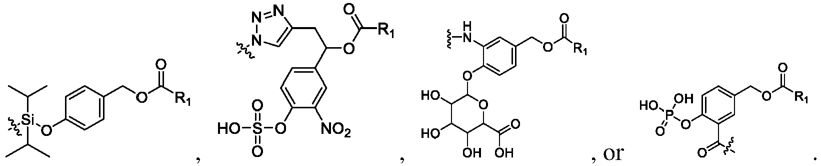

In some embodiments, when n⁵ is 1, n² and n⁴ are both 0.

In some embodiments, L^{a} is 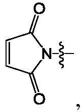 and n¹, n⁵, and n⁴ are all 0.

In some embodiments, L^{a} is 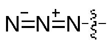 , and n² is 1 or 2.

In some embodiments, AA is each independently an amino acid residue formed by Val, Cit, Glu, or Ala. In some embodiments, -(AA)n³- is a peptide residue selected from the following group: -Val-Cit-, -Glu-Val-Cit-, -Ala-Cit-, and -Val-Ala-.

In some embodiments, the compound has the following structure:

L^{a}-(EG)n¹-(CH₂)n²-(C=O)-(AA)n³-L^{b}

wherein,
L^{a} is 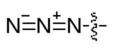 or 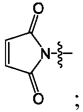
EG is -CH₂CH₂O-; n¹ is an integer of 0-4;
n² is an integer of 0-5;
AA is each independently an amino acid residue; n³ is an integer of 0-3;
L^{b} is 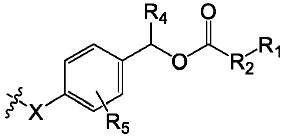 or -OH,
wherein X is -NH-;
R₂ is a bond;
R₄ is hydrogen;
R₅ is hydrogen or -OCH₃;
R₁ is a leaving group, preferably R₁ is halogen (e.g., chlorine),

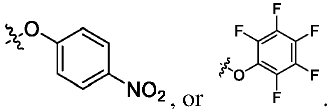

In some embodiments, L^{a} is 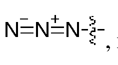 n¹ is 2, n² is 1, and L^{b} is

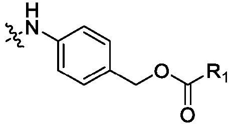

In some embodiments, L^{a} is 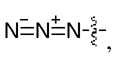 n¹ is 2, n² is is 1, and L^{b} is -OH.

In some embodiments, L^{a} is 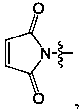 n¹ is 0, n² is 5, and L^{b} is

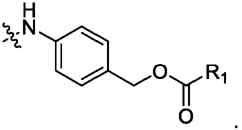

In some embodiments, L^{a} is 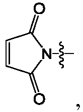 n¹ is 0, n² is 5, and L^{b} is -OH.

In some embodiments, AA is each independently Val, Cit, Glu, or Ala amino acid residue. In some embodiments, -(AA)n³- is a peptide residue selected from the following group: - Val-Cit-, -Glu-Val-Cit-, -Ala-Cit-, -Phe-Cit-, -Val-Ala-, -Gly-Gly-Phe-Gly-, -Val-Leu-Lys-, and - Gly-Asn-Asn-.

In some embodiments, the compound disclosed herein has a structure selected from the following group: 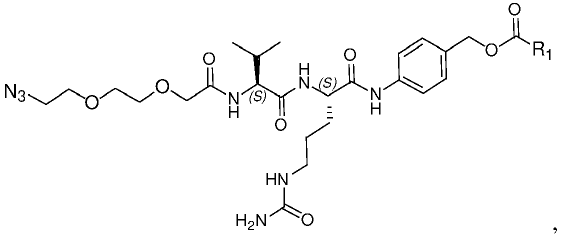 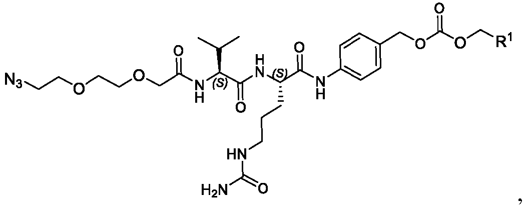 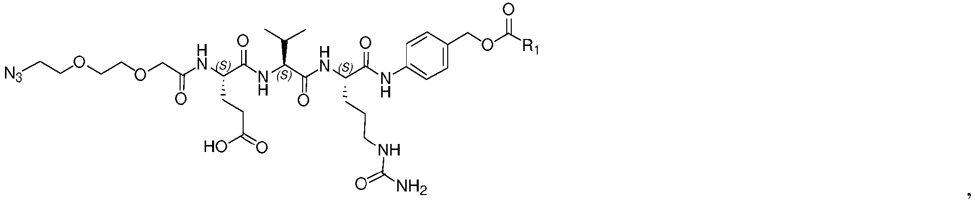 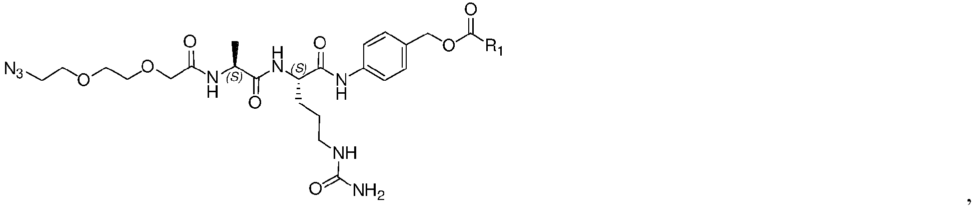 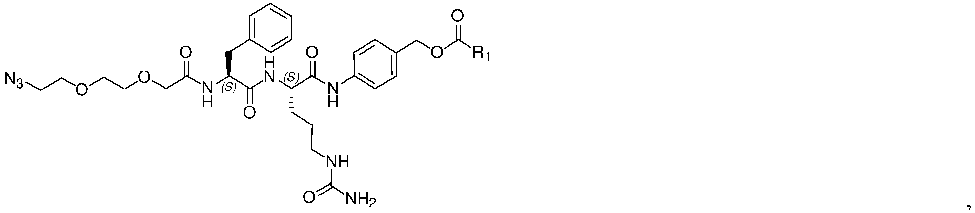 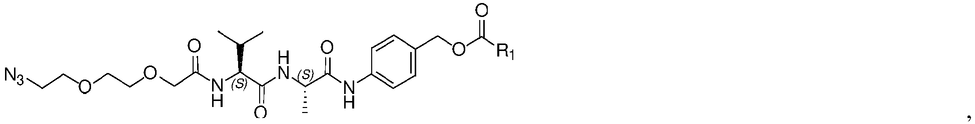 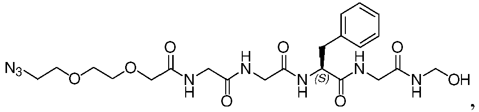 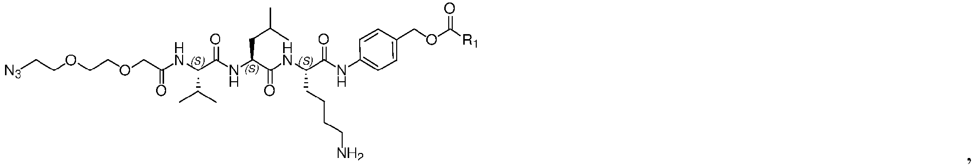 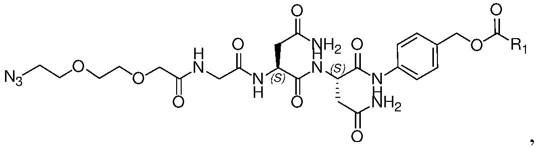 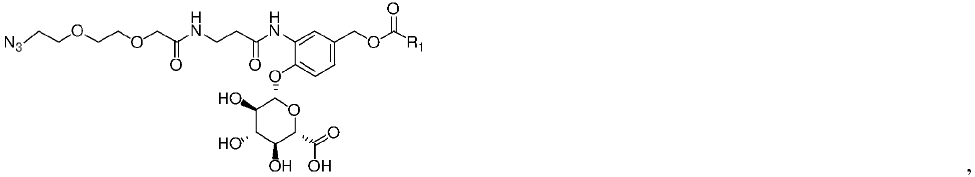 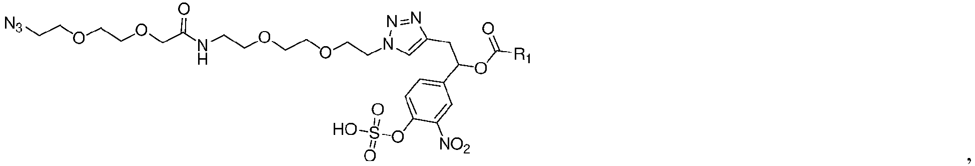 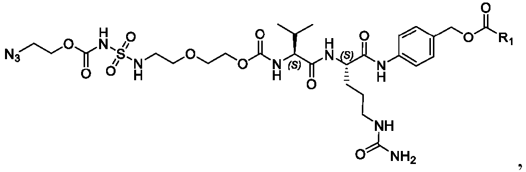 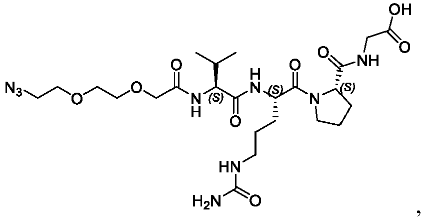 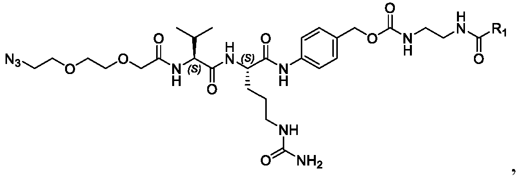 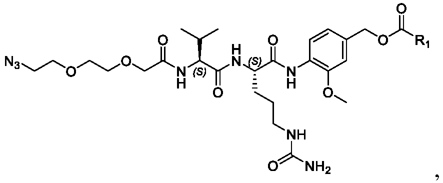 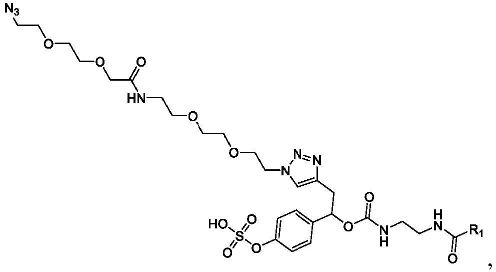 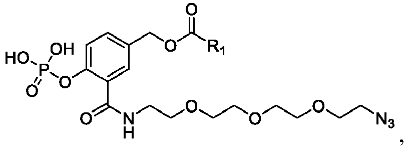 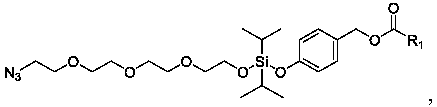 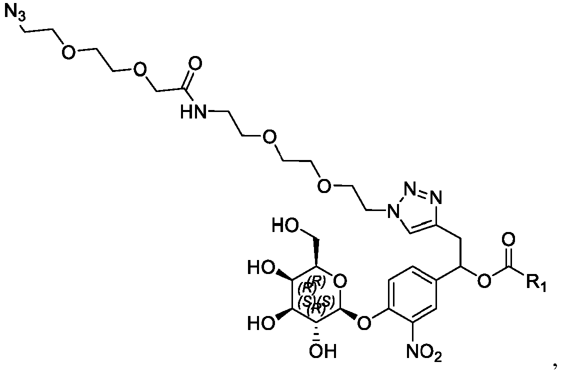 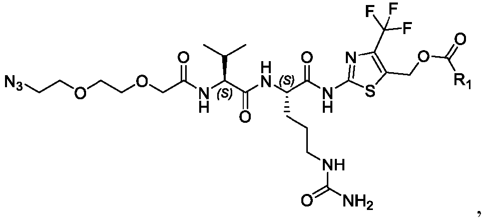 and 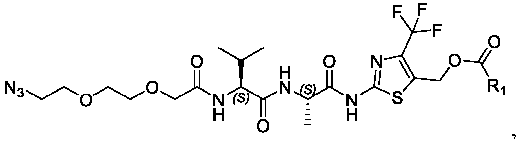 wherein R₁ is a leaving group, preferably R₁ is halogen (e.g., chlorine) or 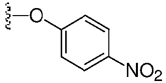 and a derivative thereof.

In another aspect, the present application discloses a compound 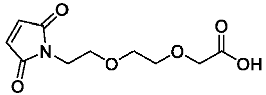 or a salt thereof.

In some embodiments, the leaving group is a halogen, for example, fluorine, chlorine, bromine, or iodine. In some preferred embodiments, the leaving group is chlorine. In some preferred embodiments, the leaving group is p-nitrophenol. In some preferred embodiments, the leaving group is pentafluorophenol.

In another aspect, the present application discloses a conjugate compound or a pharmaceutically acceptable salt thereof, wherein the conjugate compound has the following structure:

W^{a}-L¹-(EG)n¹-(D)n⁵-(CH₂)n²-(B)n⁶-(AA)n³-(CH₂CH₂-Q)n⁴-L²-W^{b},

wherein,
L¹ is 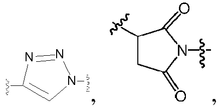 -S-S-, or a bond;
EG is -CH₂CH₂O- or -OCH₂CH₂-; n¹ is an integer of 0-6;
n² is an integer of 0-8;
D is -C(=O)-NH-S(=O)₂-NH-; n⁵ is 0 or 1;
B is each independently -C(=O)-, 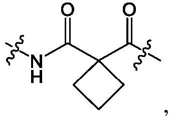 -NH-CH₂CH₂-C(=O)-, -C(=O)-NH-, -CH(CH₃)-CH₂-O-, -CH₂CH₂O-, -NH-C(=O)-, or -NH-; n⁶ is an integer of 0-3;
AA is each independently an amino acid residue; n³ is an integer of 0-5;
Q is each independently -C(=O)-, -O-, or a bond; n⁴ is an integer of 0-3;
L² is 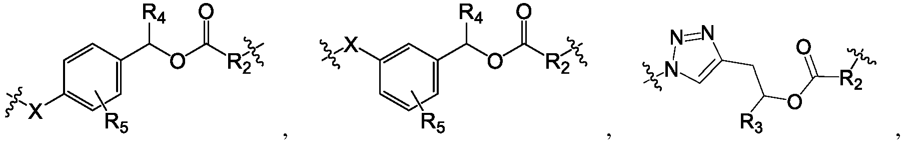 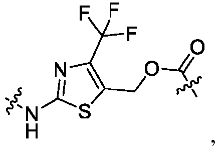 -NH-CH₂-OH, or a bond;
wherein X is a bond, -NH-, -C(=O)-, oxygen, or 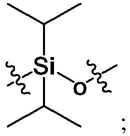
R₂ is a bond, -O-CH₂-, or -NH-CH₂CH₂-NH-C(=O)-;
R₃ is hydrogen or 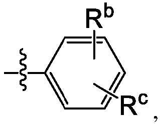 wherein R^{b} and R^{c} are each independently hydrogen, nitro, 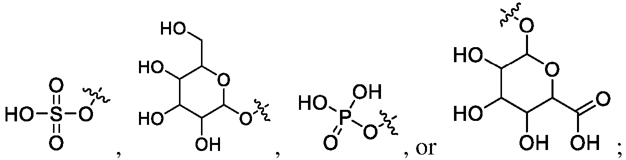
R₄ is hydrogen or C₁₋₈ alkyl;
R₅ is hydrogen, amino, nitro, phosphate group, -OR^{a}, or 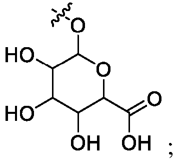 wherein R^{a} is methyl, ethyl, propyl, or isopropyl;
W^{a} and W^{b} are each independently a payload or a targeted molecule capable of binding to a cell surface protein.

In some embodiments, EG is -CH₂CH₂O-. In some embodiments, n¹ is 2. In some embodiments, n¹ is 0. In some embodiments, n² is 1. In some embodiments, n² is 4. In some embodiments, n² is 5. In some embodiments, n⁵ is 0. In some embodiments, n⁶ is 1. In some embodiments, n⁶ is 3. In some embodiments, n¹ is 2, n² is 1, n³ and n⁴ are 0, and L² is a bond.

In some embodiments, AA is each independently an amino acid residue formed by Val, Cit, Glu, Leu, Lys, Ala, Gly, Phe, Gln, Pro, or Asn. In some embodiments, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Cit-, -Val-Leu-Lys-, -Val-Cit-Pro-Gly, -Gly-Asn-Asn-, and -Ala-Ala-Asn-.

In some embodiments, when n⁴ is 1, Q is -C(=O)-; when n⁴ is 2, Q are -O- and -C(=O)-, respectively; when n⁴ is 3, Q are -O-, -O-, and a bond, respectively. In some embodiments, n⁴ is 0.

In some embodiments, L² is a bond, 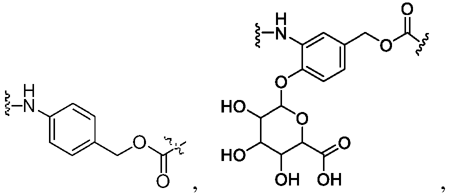 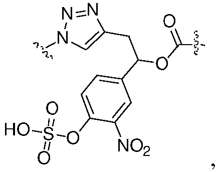 -NH-CH₂-O-, 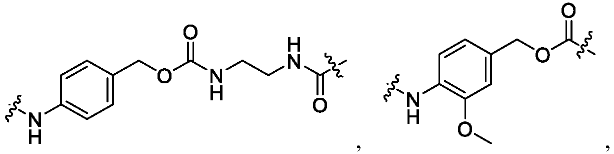 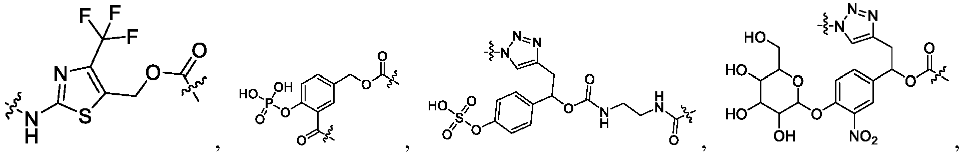 or

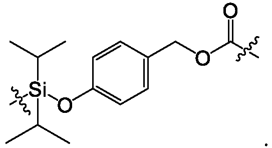

In some embodiments, the compound has the following structure:

W^{a}-L¹-(EG)n¹-(D)n⁵-(CH₂)n²-(B)n⁶-(AA)n³-(CH₂CH₂-Q)n⁴-L²-W^{b}

wherein,
L¹ is 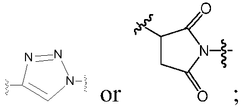
EG is -CH₂CH₂O-; n¹ is an integer of 2-4;
D is -C(=O)-NH-S(=O)₂-NH-; n⁵ is 0 or 1;
n² is an integer of 0-5;
B is -C(=O)-, -C(=O)-NH-, -CH₂CH₂O-, -NH-CH₂CH₂-C(=O)-, or -NH-; n⁶ is an integer of 0-3;
AA is each independently an amino acid residue; n³ is an integer of 0-3;
Q is each independently -C(=O)-, -O-, or a bond; n⁴ is an integer of 0-3;
L² is 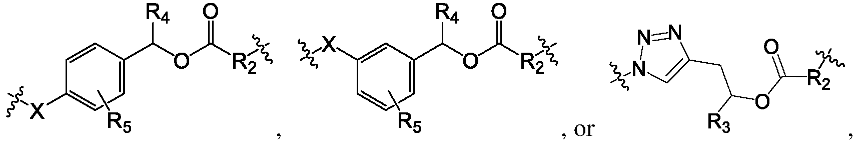
wherein X is a bond, -NH-, -C(=O)-, or 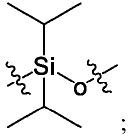
R₂ is a bond;
R₃ is 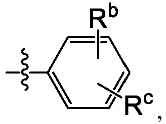 wherein R^{b} and R^{c} are each independently hydrogen, nitro, or 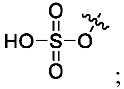
R₄ is hydrogen;
R₅ is hydrogen, -OCH₃, or 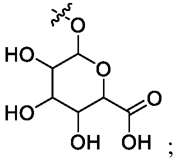
W^{a} and W^{b} are each independently a payload or a targeted molecule capable of binding to a cell surface protein.

In some embodiments, L² is

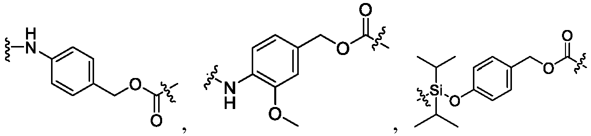

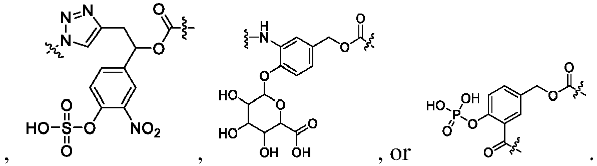

In some embodiments, when n⁵ is 1, n² and n⁴ are both 0.

In some embodiments, L¹ is 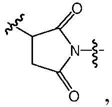 and n¹, n⁵, and n⁴ are all 0.

In some embodiments, L¹ is 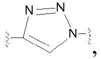 and n² is 1 or 2.

In some embodiments, AA is each independently an amino acid residue formed by Val, Cit, Glu, or Ala. In some embodiments, -(AA)n³- is a peptide residue selected from the following group: -Val-Cit-, -Glu-Val-Cit-, -Ala-Cit-, and -Val-Ala-.

In some embodiments, the compound has the following structure:

W^{a}-L¹-(EG)n¹-(CH₂)n²-(C=O)-(AA)n³-L²-W^{b}

wherein,
L¹ is 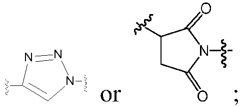
EG is -CH₂CH₂O-; n¹ is an integer of 0-4;
n² is an integer of 0-5;
AA is each independently an amino acid residue; n³ is an integer of 1-4;
L² is 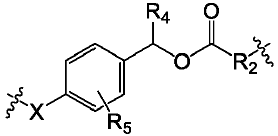 or a bond,
wherein X is -NH-;
R₂ is a bond;
R₄ is hydrogen;
R₅ is hydrogen or -OCH₃;
W^{a} and W^{b} are each independently a payload or a targeted molecule capable of binding to a cell surface protein.

In some embodiments, L¹ is 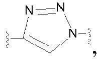 n¹ is 2, n² is 1, and L² is

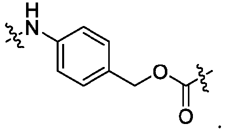

In some embodiments, L¹ is 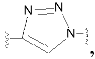 n¹ is 2, n² is 1, and L² is a bond.

In some embodiments, L¹ is 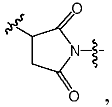 n¹ is 0, n² is 5, and L² is

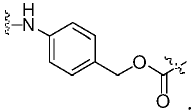

In some embodiments, L¹ is 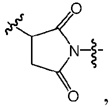 n¹ is 0, n² is 5, and L² is a bond.

In some embodiments, the present application discloses a conjugate compound having a structure selected from the following group: 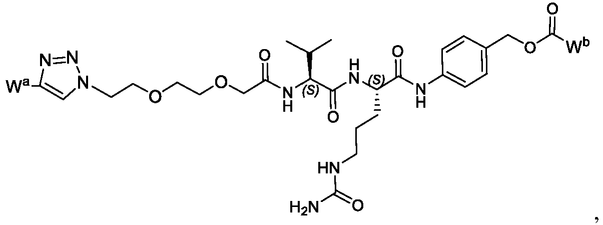 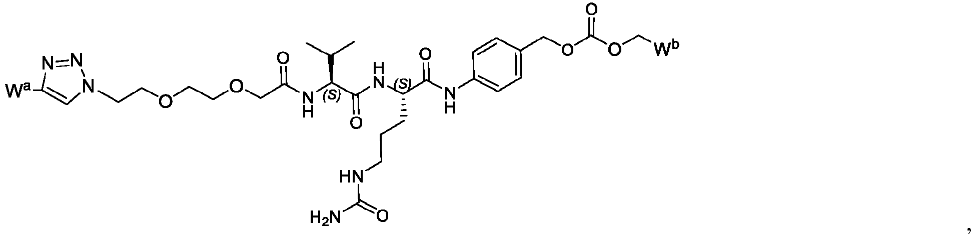 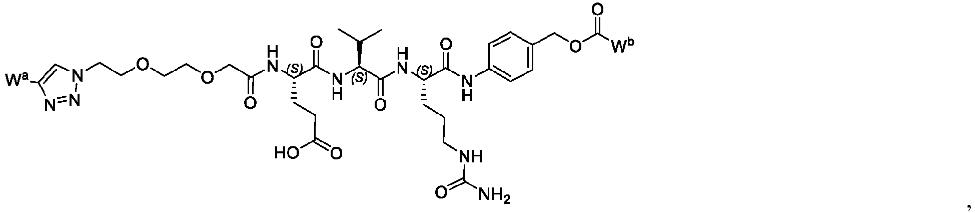 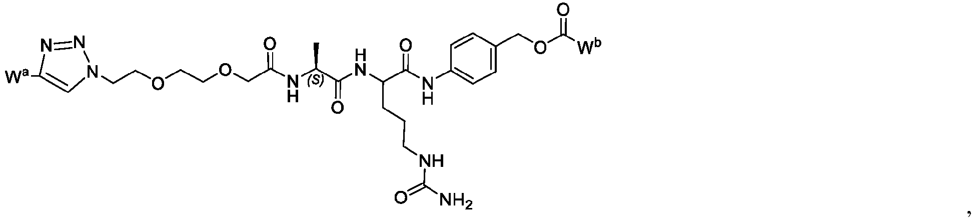 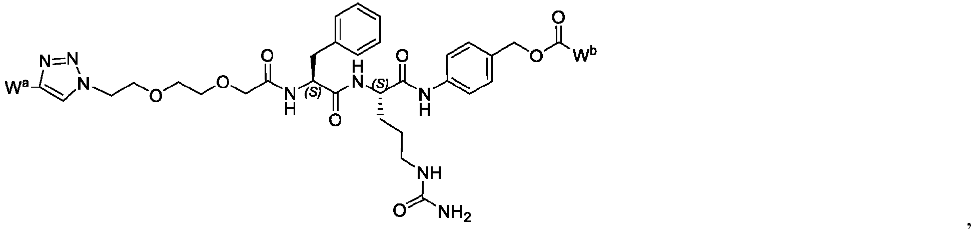 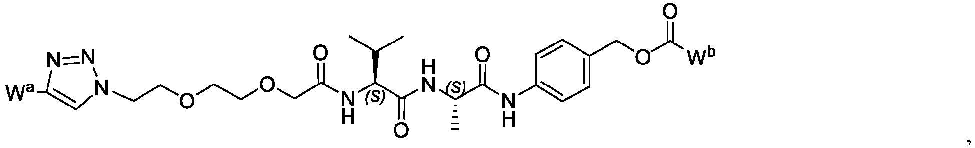 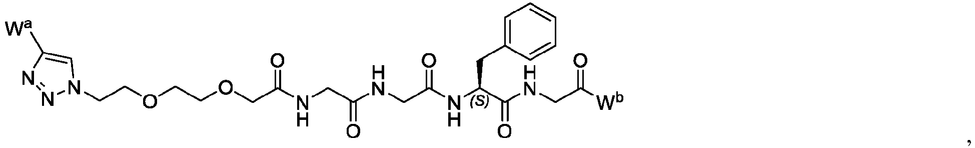 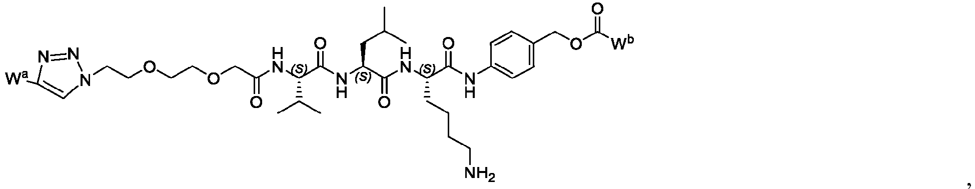 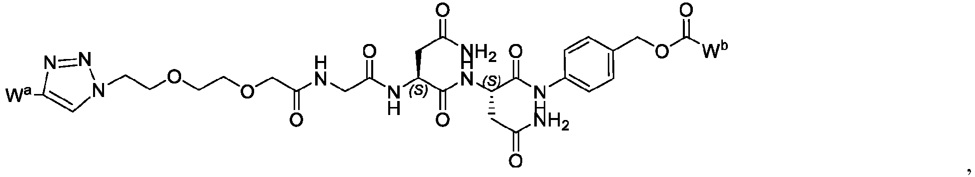 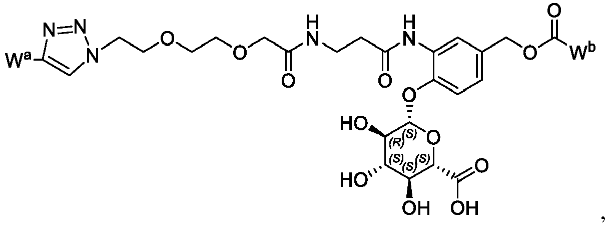 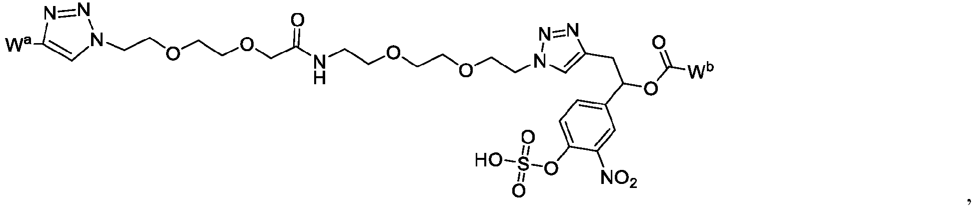 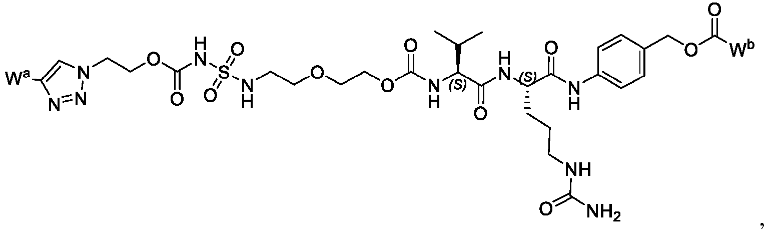 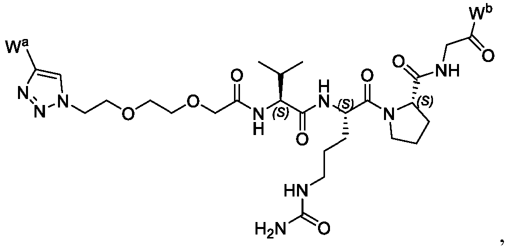 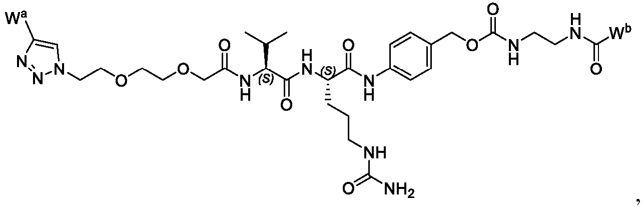 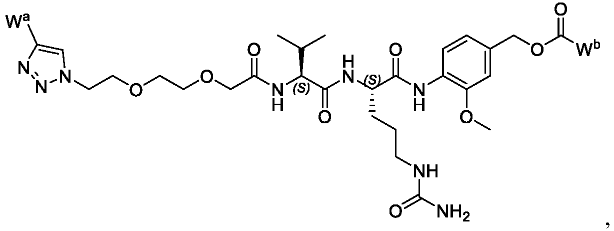 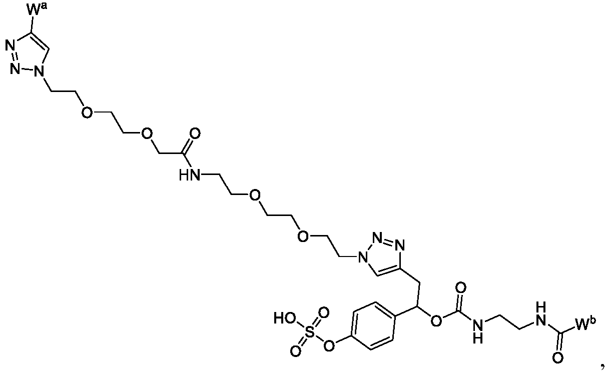 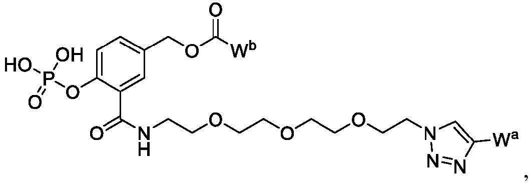 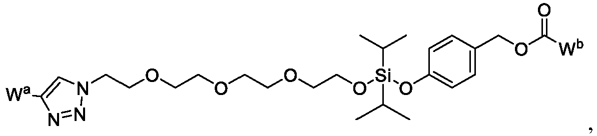 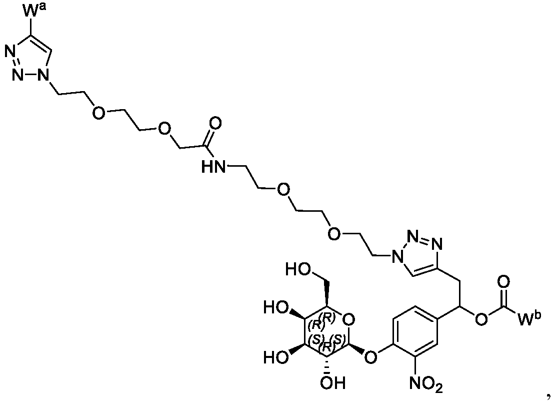 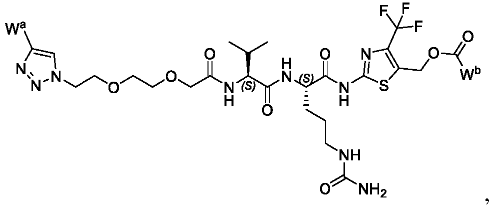 and 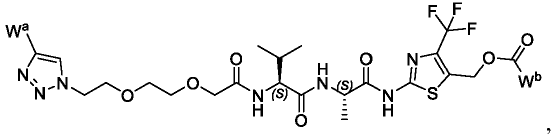 wherein W^{a} and W^{b} are each independently a payload or a targeted molecule capable of binding to a cell surface protein.

In another aspect, the present application provides a conjugate compound or a pharmaceutically acceptable salt thereof, wherein the conjugate compound is 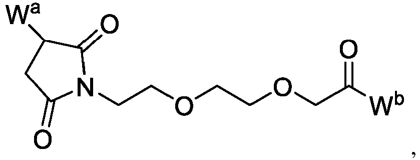 wherein W^{a} and W^{b} are each independently a payload or a targeted molecule capable of binding to a cell surface protein.

In another aspect, the present application provides a conjugate compound or a pharmaceutically acceptable salt thereof, wherein the conjugate compound is 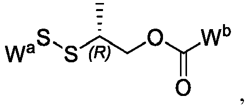 wherein W^{a} and W^{b} are each independently a payload or a targeted molecule capable of binding to a cell surface protein.

In another aspect, the present application provides a proteolysis targeting chimera or a pharmaceutically acceptable salt thereof, wherein the proteolysis targeting chimera compound has the following structures: 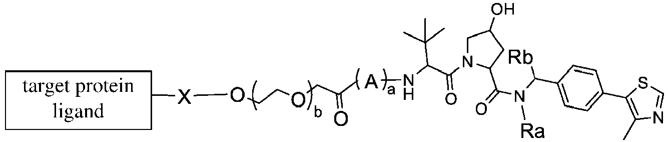 or 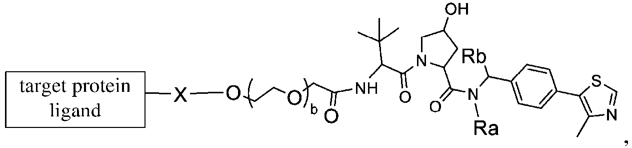 wherein,
Ra and Rb are each independently hydrogen, alkyl, NH₂, OH, or halogen;
A is each independently an amino acid residue;
a is 0 or 1;
b is an integer of 0-5; 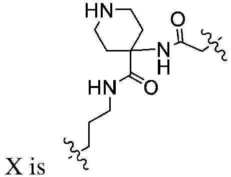 or a bond;
the target protein ligand is

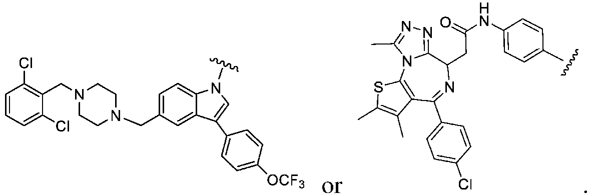

In some embodiments, Ra is hydrogen. In some embodiments, Rb is hydrogen. In some embodiments, A is an amino acid residue formed by Lys. In some embodiments, a is 1. In some embodiments, a is 0. In some embodiments, b is 1. In some embodiments, b is 2. In some embodiments, b is 3. In some embodiments, b is 4. In some embodiments, b is 5.

In some embodiments, the proteolysis targeting chimera has a structure selected from the following group:

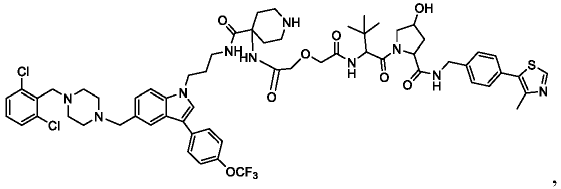

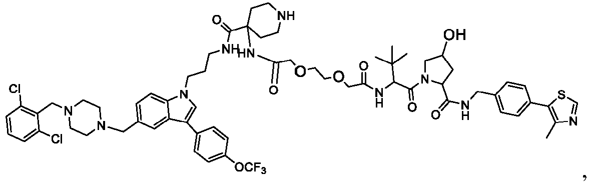

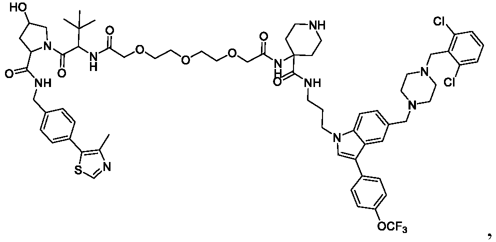

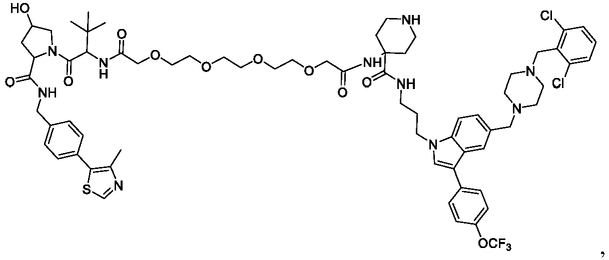

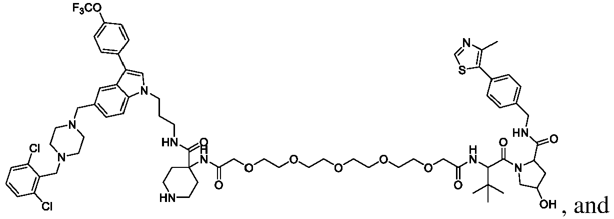

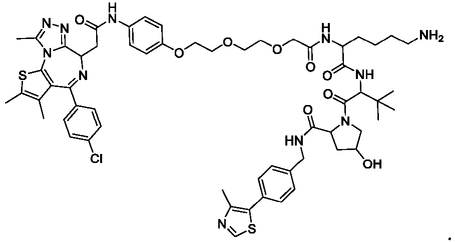

The present application will be described in more detail below by way of specific examples. The following examples are provided for illustrative purposes only and are not intended to limit the present invention in any way. Those skilled in the art will readily recognize that various non-critical parameters may be changed or modified to generate substantially identical results.

### Examples

The following examples are intended to better illustrate the present invention and should not be construed as limiting the scope of the present invention. All of the specific compositions, materials and methods described below, in their entirety or in part, fall within the scope of the present invention. The specific compositions, materials, and methods are not intended to limit the present invention, but merely to illustrate particular embodiments within the scope of the present invention. Those skilled in the art may develop equivalent compositions, materials, and methods without creative efforts and without departing from the scope of the present invention. It will be appreciated that various modifications may be made to the method of the present disclosure, which still fall within the scope of the present disclosure. The inventors intend to include such variations within the scope of the present disclosure.

### Example 1 Preparation of conjugate compounds

### 1. Preparation of proteolysis targeting chimeras

### 1.1 Preparation of CR202CC, CR202CF, CR202CI, CR202CL, and CR202CP

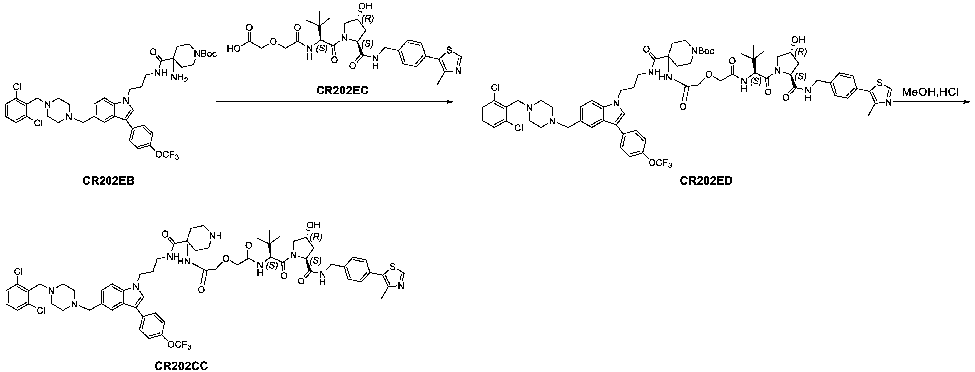

**CR202EC** (240.0 mg, 0.44 mmol; for the synthetic route, see J. Med. Chem. 2020, 63, 17, 9977-9989, which is incorporated herein by reference) was added to DMF (5 mL), before HATU (175.0 mg, 0.46 mmol) and DIPEA (166.0 µL, 0.50 mmol) were added at 0-5 °C. After stirring for half an hour, **CR202EB** (342.0 mg, 0.42 mmol; for the synthetic route, see paragraph [0227] in WO2015/184349A2, which is incorporated herein by reference) was added. The reaction system was incubated for 2 h until the reaction was completed. The reaction mixture was extracted with water (25 mL) and ethyl acetate (25 mL), and the organic phase was dried over anhydrous sodium sulfate and purified by column chromatography to give a pale yellow solid **CR202ED** (14.0 mg). **CR202ED** (14.0 mg, 0.01 mmol) was added to methanol (200 µL) before a 3 M HCl/methanol solution (50 µL) was added at 0-5 °C. The reaction system was incubated at 20-25 °C for 1 h until the reaction was completed, and dried to give **CR202CC** (13.0 mg).

The proteolysis targeting chimeras **CR202CF, CR202CI, CR202CL,** and **CR202CP** were obtained by similar procedures to the above method.

### 1.2 Preparation of CR20274

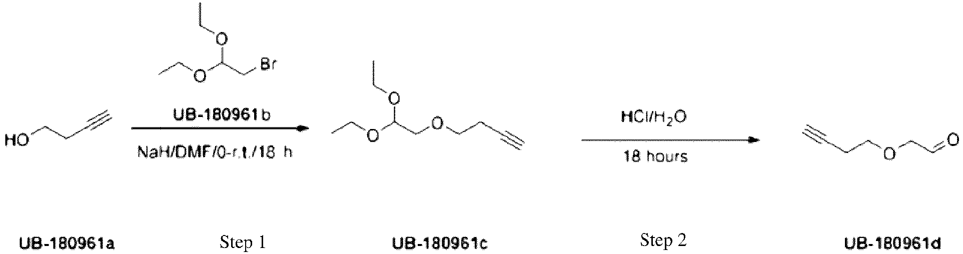

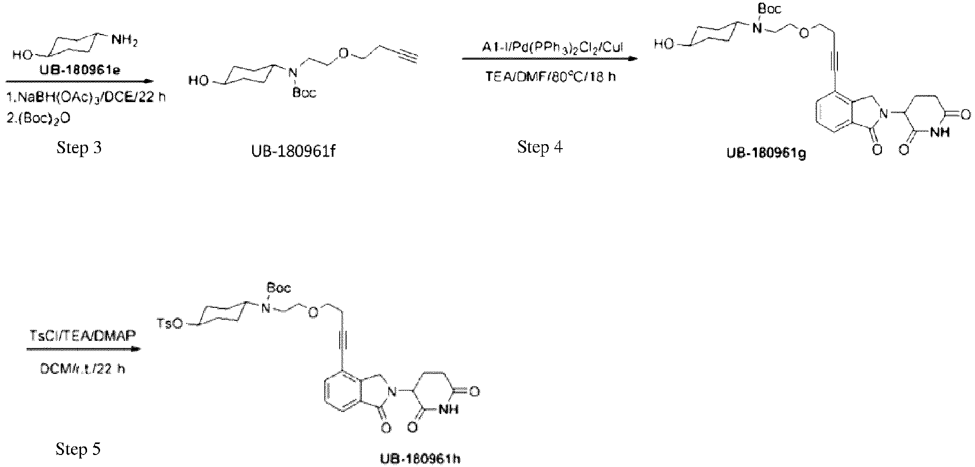

### Synthesis of UB-180961h

The preparation of compound UB-180961h is disclosed in WO2021205391A1, which is incorporated herein by reference.

**UB-180961a** (20 g, 71.1 mmol) was dissolved in anhydrous DMF (80 mL) and the mixture was cooled to 0 °C, before NaH (16.8 g, 107 mmol) was added. Half an hour later, **UB-180961b** (21.1 g, 107 mmol) was dissolved in anhydrous DMF (20 mL) and the mixture was added dropwise to the reaction system. The reaction system was incubated overnight at room temperature. Ice water (100 mL) was added to the reaction mixture and the mixture was extracted thrice with EtOAc (60 mL). The organic phases were combined and separated by column chromatography (PE/EtOAc = 0-100%) to give a colorless oil **UB-180961c** (25 g, 47% yield). Compound **UB-180961c** (15 g, 285.4 mmol) was dissolved in water (40 mL) before concentrated HCl (10 mL) was added. The reaction system was incubated overnight at room temperature. The reaction mixture was extracted thrice with DCM (50 mL), and the organic phase was dried over anhydrous sodium sulfate and concentrated to give a colorless oil **UB-180961d** (7.6 g).

**UB-180961d** (7.8 g, 68 mmol) and **UB-180961e** (7.6 g, 68 mmol) were dissolved in DCE (100 mL) and the reaction system was incubated at room temperature for 1 h. NaBH(OAc)₃ (29.6 g, 136 mmol) was added and the reaction system was incubated at room temperature overnight. TEA (5 mL, sat) and Boc₂O (6 g, 23.8 mmol) were added and the reaction system was incubated at room temperature for 18 h. The reaction mixture was extracted with EtOAc (15 mL) twice, and the organic phase was dried over anhydrous Na₂SO₄ and separated by column chromatography (PE/EtOAc = 0-100%) to give a yellow oil **UB-180961f** (4.6 g, 57% yield). **UB-180961f** (4.6 g, 15 mmol), A1-I(3.7 g, 10 mmol), Pd(PPh₃)₂Cl₂ (701 mg, 1 mmol), CuI (190 mg, 1 mmol), TEA (4.2 mL, 30 mmol) were dissolved in anhydrous DMF (120 mL), and the reaction system was incubated overnight at 80 °C. The reaction mixture was extracted twice with EtOAc (15 mL), and the organic phase was dried over anhydrous Na₂SO₄ and separated by column chromatography (PE/EtOAc = 0-100%) to give a yellow solid **UB-180961g** (3.6 g, 57% yield).

**UB-180961g** (3.6 g, 33.8 mmol), TEA (10.3 g, 10.2 mmol), and DMAP (12.4 g, 10.2 mmol) were dissolved in anhydrous DMF (140 mL), before TsCl (14.6 g, 7.7 mmol) was added at 0 °C. The reaction system was heated to 30 °C and incubated for 15 h. The reaction mixture was extracted twice with DCM (50 mL), and the organic phase was concentrated and separated by column chromatography (PE/EtOAc = 0-100%) to give a white solid **UB-180961h** (3.6 g, 86% yield).

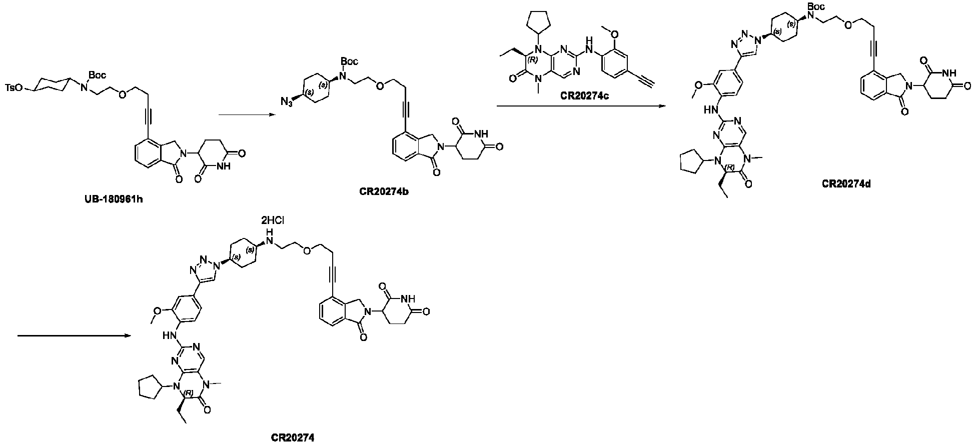

### Synthesis of CR20274b

**UB-180961h** (48.4g, 1.0 eq) was dissolved in DMF (484 mL, 10 V), before the system was purged with N₂. CsF (31.2 g, 3 eq) and TMSN₃ (azidotrimethylsilane, 23.7 g, 3 eq) were sequentially added before the internal temperature was raised to 80 °C, and the reaction system was stirred for 4.5 h. The reaction mixture was dropwise added to a saturated aqueous NaHCO₃ solution (4.8 L) to precipitate a solid. The solid was dissolved in EA (ethyl acetate, 968 mL), and the solution was washed with saturated brine (484 mL). The organic phase was dried and concentrated to give a crude product, which was purified by column chromatography (DCM:MeOH = 45:1) to give a white solid **CR20274b** (31.1 g, 95.6% purity, 78.7% yield), LCMS: [M+H]⁺ = 579.1.

### Synthesis of CR20274d

**CR20274b** (30.8 g, 1.0 eq) and **CR20274c** (21.7 g, 1.0 eq) were dissolved in tert-butanol (900 mL, 30 v). Water (45.0 mL, 15 v), CuSO₄ (8.49 g, 1.0 eq), and sodium ascorbate (21.1 g, 2.0 eq) were added before the system was purged with nitrogen thrice. The temperature was controlled at 25-30 °C, and the reaction system was incubated for 3.1 h. DCM (900 mL) and saturated brine (300 mL) were added into the reaction mixture and the phases were separated. The aqueous phase was extracted with DCM (900 mL) once, and the organic phase was collected, dried, concentrated, purified by column chromatography (eluent: DCM/MeOH = 200/1-50/1), and concentrated at reduced pressure to give the product **CR20274d** (41.5 g, 78.8% yield).

### Synthesis of CR20274

**CR20274d** was dissolved in DCM (3 mL) before 0.5 mL of HCl/dioxane (4 M) was added. The reaction system was incubated at room temperature for 1 h. The reaction mixture was concentrated, washed with diethyl ether (5 mL) thrice, and filtered to give a white solid **CR20274** (98.1% purity, MS:[M+H]⁺=884.6).

### 1.3 Preparation of CR202EE

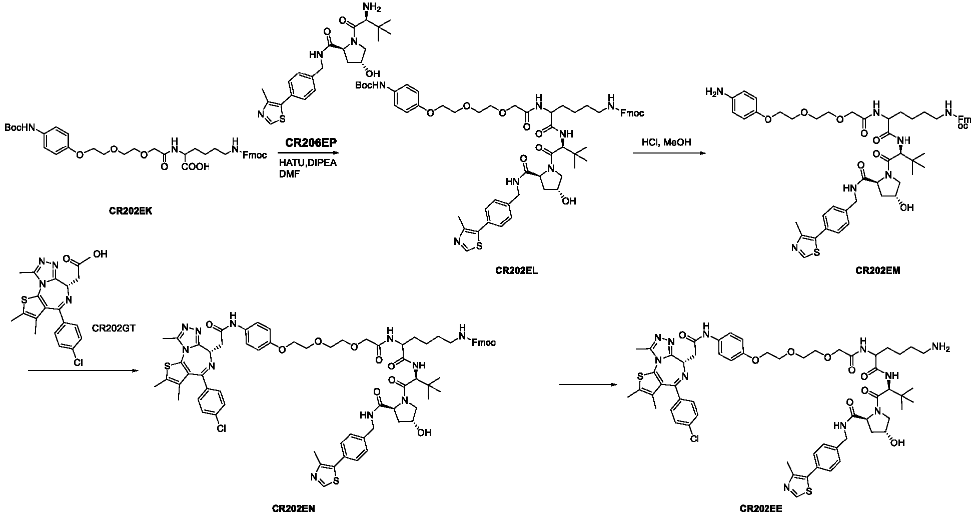

### Synthesis of CR202EM

**CR206EP** was added to DMF (5 mL), before HATU and DIPEA were added at 0-5 °C. After stirring for half an hour, **CR202EK** was added. The reaction system was incubated for 2 h until the reaction was completed. The reaction mixture was extracted with water (25 mL) and ethyl acetate (25 mL), and the organic phase was dried over anhydrous sodium sulfate, concentrated, and purified by column chromatography to give **CR202EL. CR202EL** was added to methanol (200 µL) before a 3 M HCl/methanol solution (50 µL) was added at 0-5 °C. The reaction system was incubated at 20-25 °C for 1 h, and concentrated to give **CR202EM.**

### Synthesis of CR202EN

**CR206GT** was added to DMF (5 mL), before HATU and DIPEA were added at 0-5 °C. After stirring for half an hour, **CR202EM** was added. The reaction system was incubated for 2 h until the reaction was completed. The reaction mixture was extracted with water (25 mL) and ethyl acetate (25 mL), and the organic phase was dried over anhydrous sodium sulfate, concentrated, and purified by column chromatography to give **CR202EN.**

### Synthesis of CR202EE

**CR205EN** (320.0 mg, 0.23 mmol) was added to a 20% piperidine/DMF solution (3.0 mL). The reaction system was incubated at room temperature for 1 h until the reaction was completed. Water (15 mL) was added, and the mixture was extracted with dichloromethane (15 mL). The extract was concentrated and purified by column chromatography to give **CR202EE** (181.0 mg).

### 1.4 Synthesis of CR202Z5, CR202ES, CR202Z7, CR202Z8, CR202Z9, CR202Z1, CR202Z2, CR202Z3, CR202Z4, CR202Y2, and CR202Y4 (source: MCE, Cat. No. HY-114312)

The synthetic route of **CR202Z5** can be found in Communications Biology, (2018) 1: 100 for the synthesis of ARCC-4. The synthetic route of **CR202ES** can be found in PNAS, 113 (26) 7124-7129 for the synthesis of ARV-771. The synthetic route of **CR202Z7** can be found in J. Med. Chem. 2019, 62, 3, 1420-1442 for the synthesis of ERD-308. The synthetic route of **CR202Z8** can be found in ChemMedChem, 2020, 15, 17-25 for the synthesis of GNE-987. The synthetic route of **CR202Z9** can be found in J. Med. Chem. 2019, 62, 2, 941-964 for the synthesis of ARD-69. The synthetic route of **CR202Z1** can be found in Patent No. CN111892577A for the synthesis of ARV-110. The synthetic route of **CR202Z2** can be found in Patent No. US2021/0139458A1 for the synthesis of ARV-471. The synthetic route of **CR202Z3** can be found in Chemistry & Biology, 2015, 22, 755-763 for the synthesis of ARV-825. The synthetic route of **CR202Z4** can be found in ACS Med Chem Lett. 2019, 11, 1855-1862 for the synthesis of FC-11. The synthetic route of **CR202Y2** can be found in Cancer Res. 2019, 79, 251-262 for the synthesis of MD-224. **CR202Y4** was purchased from MCE (Cat. No. HY-114312). The documents are incorporated herein by reference in their entireties.

### 2. Preparation of targeted molecules

### 2.1 Synthesis of L-01, L-02, and L-04

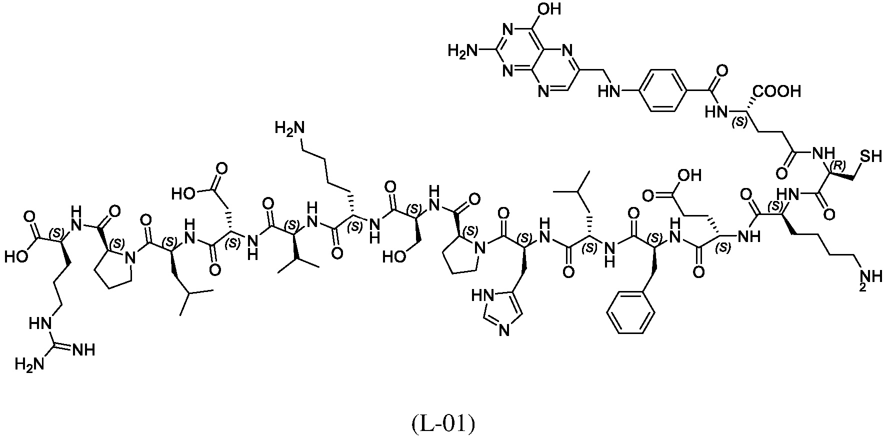

The targeted molecule L-01 targeting TRPV6 and folate receptor was synthesized in solid phase using chemical Fmoc-amino resin. The resin was loaded into a solid-phase reaction column before DMF was added. Nitrogen was introduced into the solvent to swell the resin for 30 min. Fmoc-Arg-OH, DCC (dicyclohexylcarbodiimide), and DMAP were added, and the reaction system was incubated at 25 °C for 3 h. The ends were capped with acetic anhydride and pyridine for 1 h, and the mixture was washed with DMF thrice. The Fmoc-protecting group on the resin was removed by DBLK, and the mixture was washed with DMF 5 times. Proper amounts of Fmoc-Pro-OH and HOBt were dissolved in DMF. DIC was added to the above solution in an ice-water bath at 0 °C, and the mixture was mixed to activate for 5 min. The solution was added to the above reaction column and incubated for 3 h. The solvent was removed, and the resin in the reaction column was washed thrice. The Fmoc-protecting group was removed by DBLK. The above procedures were repeated to sequentially couple Fmoc-Leu-OH, Fmoc-Asp(OtBu)-OH, Fmoc-Val-OH, Fmoc-Lys(Boc)OH, Fmoc-Ser(tBu)-OH, Fmoc-Pro-OH, Fmoc-His(Trt)-OH, Fmoc-Leu-OH, Fmoc-Phe-OH, Fmoc-Glu(OtBu)-OH, Fmoc-Lys(Boc)-OH, Fmoc-Cys(Trt)-OH, Fmoc-Glu-OtBu, and pteroic acid according to the structure to give a peptide resin. The peptide resin was cleaved with a cleaving buffer and filtered. The resultant solution was collected and poured into MTBE to precipitate a solid. The solid was washed with MTBE to give a crude product, which was subjected to Pre-HPLC to give L-01.

### L-02 and L-04 can be obtained by procedures similar to those above.

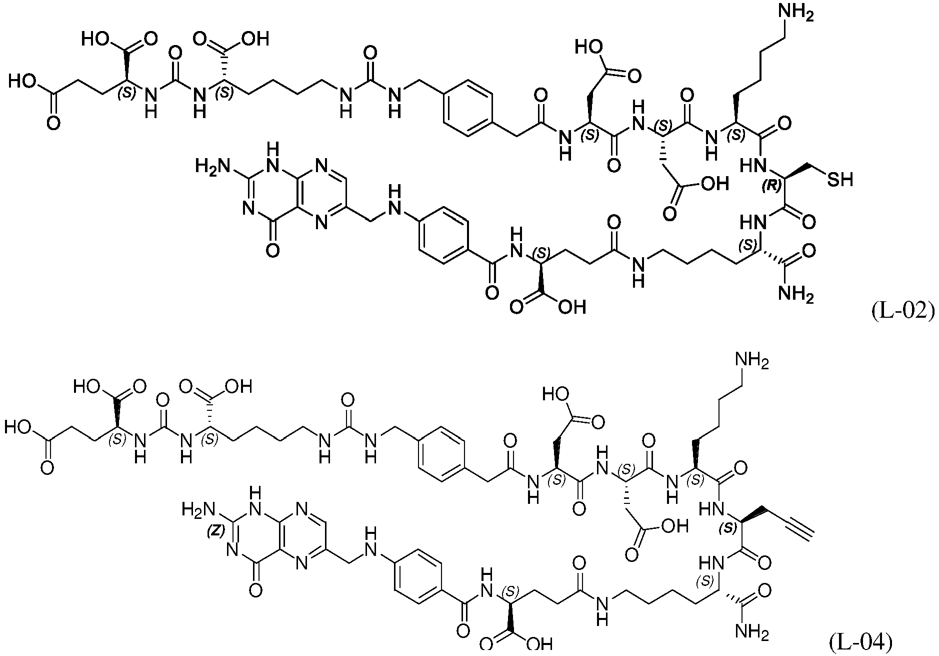

### 2.2 Synthesis of L-03

### Structure of CR211D6:

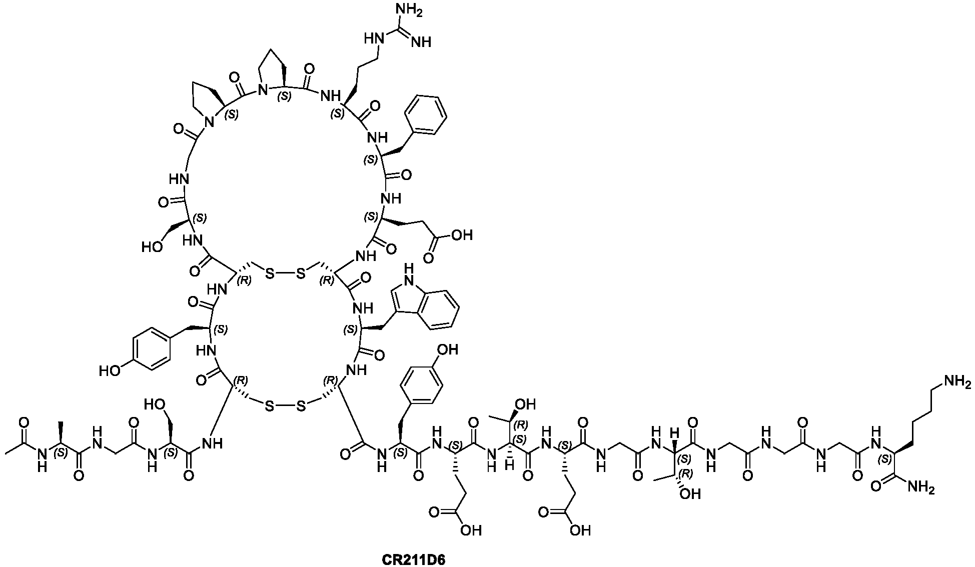

### Procedures:

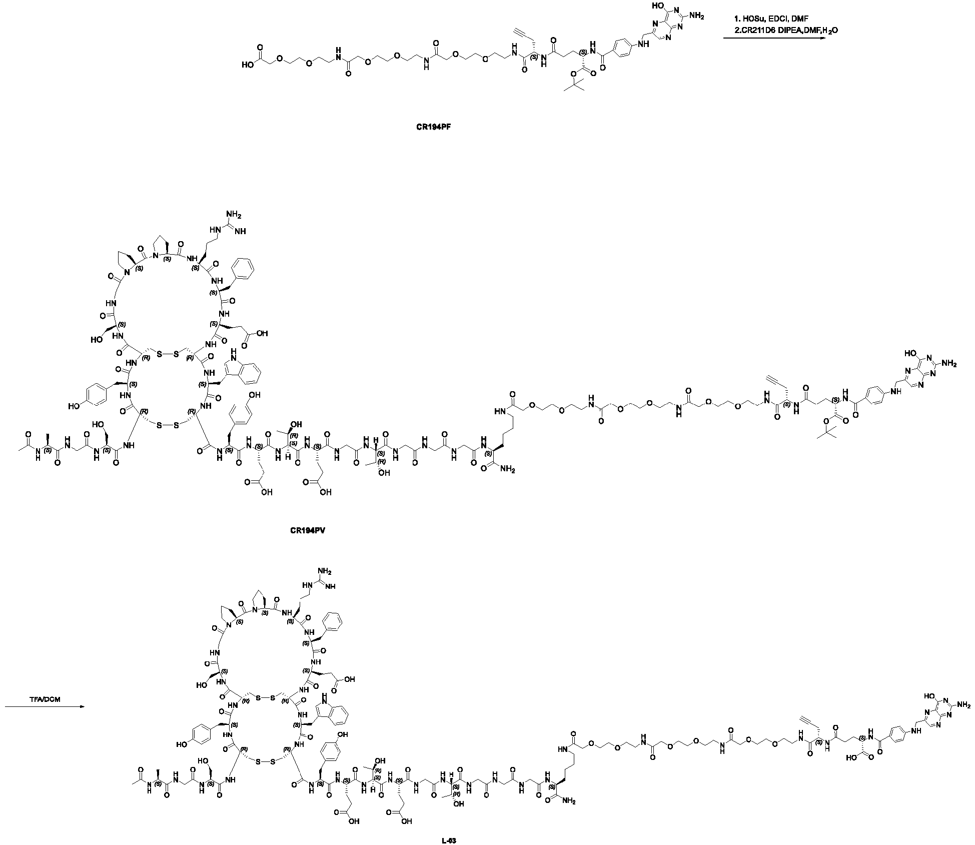

For the synthesis of **CR211D6,** see Nature Medicine, 2015, 21, 955-961 (), which is incorporated herein by reference in its entirety.

**CR194PF** (150.0 mg, 145.9 µmol) was added to DMF (3.0 mL) in a nitrogen atmosphere away from light for dissolving. The reaction system was stirred at room temperature (25.5 °C), before HOSu (25.2 mg, 219.0 µmol) and EDCI (42.0 mg, 219.0 µmol) were sequentially added and DIPEA was added dropwise to adjust the pH to 8. The reaction system was incubated at room temperature for 1 h before EDCI (42.0 mg, 219.0 µmol) was added, and the system was then incubated for another 1 h. The reaction mixture was added dropwise to MTBE (30.0 mL). The supernatant was discarded, and the residue was washed with MTBE (5.0 mL × 2), and dried in vacuo at 20 °C to give a yellow oil. The yellow oil was dissolved in DMF (1.0 mL) (i.e., to give an activated **CR194PF** ester solution) for later use.

**CR211D6** (284.0 mg, 102.13 µmol) was added to DMF/water (2.0 mL/1.0 mL) in a nitrogen atmosphere away from light for dissolving. The reaction system was stirred at room temperature (25.9 °C), before DIPEA was added dropwise to adjust the pH to 8. The activated **CR194PF** ester solution was then added dropwise, and the reaction system was incubated at room temperature for 1 h. The reaction mixture was purified by reverse-phase preparative liquid chromatography and lyophilized to give **CR194PV** (50.0 mg).

**CR194PV** (56.2 mg, 14.8 µmmol) was added to TFA/DCM (1.4 mL/1.4 mL) in a nitrogen atmosphere away from light for dissolving. The reaction system was stirred at room temperature (22.7 °C) for 1 h, diluted with MTBE (10.0 mL), and dried with nitrogen, and the residue was washed with MTBE (5.0 mL) and dried with nitrogen to give **L-03.**

### 2.3 Synthesis of L-05

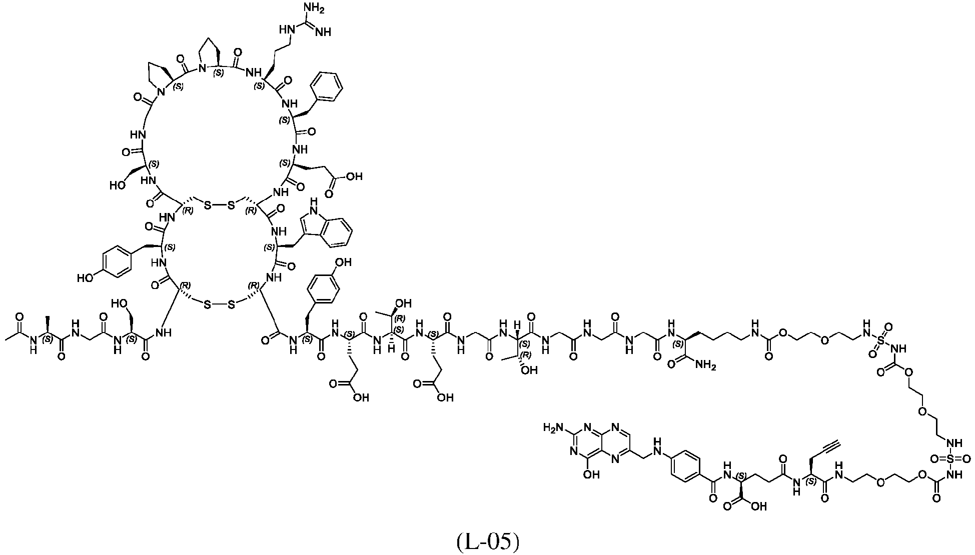

### Procedures:

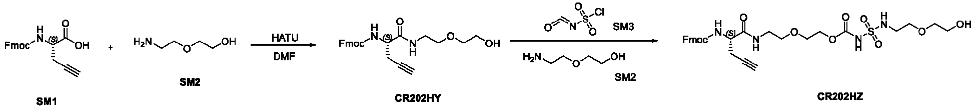

**SM1** (Fmoc-L-Pra-OH, 20.00 g, 60 mmol) was dissolved in DMF (150 mL). The reaction system was purged with nitrogen thrice and cooled to -8 °C, before HATU (27.40 g, 72 mmol, 1.2 eq) and **SM2** (7.56 g, 72 mmol) were added. DIEA (15.50 g, 120 mmol) was dissolved in DMF (10 mL) and the solution was added dropwise to the system. The reaction system was stirred at 0 °C for 1 h. The starting material SM1 was monitored by HPLC until substantially depleted. The reaction system was added to ethyl acetate (250 mL) and water (800 mL) for extraction. The phases were separated, and the aqueous phase was extracted twice with appropriate amounts of EA. The organic phases were combined and dried over an appropriate amount of anhydrous sodium sulfate for 1 h. The mixture was filtered, concentrated at reduced pressure, dried, and purified by column chromatography (DCM:MeOH = 100:1) to give a white solid **CR202HY** (26.51 g, 98.51% purity, 100% yield). [M+H]⁺=423.4. ¹H-NMR (400 MHz, CDCl₃) δ 7.81 (d, J = 7.5 Hz, 2H), 7.64 (d, J = 7.3 Hz, 2H), 7.45 (t, J = 7.4 Hz, 2H), 7.36 (t, J = 7.4 Hz, 2H), 6.94 (s, 1H), 5.76 (s, 1H), 4.49 (dd, J = 18.1, 11.0 Hz, 2H), 4.26 (t, J = 6.8 Hz, 1H), 3.86 - 3.69 (m, 2H), 3.66 - 3.44 (m, 6H), 2.66 (dd, J = 71.1, 60.5 Hz, 4H).

**SM3** (13.58 g, 96 mmol, 1.6 eq) was added into anhydrous DCM (600 mL) in a nitrogen atmosphere. The reaction system was cooled to 0 °C before a solution of **CR202HY** (25.80 g, 60 mmol) in DCM (200 mL) was slowly and dropwise added. The reaction system was incubated at 10 °C for 30 min, and a sample was taken and quenched by ammonia-acetonitrile. The reaction was monitored by on-line HPLC until completion. Triethylamine (18.22 g, 180 mmol) was added and the pH measurement was 8. Finally, a solution of **SM2** (9.47 g, 90 mmol) in DCM (10 mL) was added dropwise at 5 °C, and after the addition, the reaction system was stirred at 10 °C for 30 min until on-line HPLC monitoring indicated a substantial completion of the reaction. The reaction system was added to dichloromethane (500 mL) and water (200 mL) for extraction. The phases were separated, and the aqueous phase was extracted twice with 150 mL of dichloromethane. The organic phases were combined and dried over an appropriate amount of anhydrous sodium sulfate for 1 h. The mixture was filtered, concentrated at reduced pressure, dried, purified by column chromatography (DCM:EA = 1:1, containing 1% of methanol and 0.5% of acetic acid), and concentrated to give a foamy solid **CR202HZ** (24.00 g, 95.82 % purity, 63.3% yield). [M+H]⁺=633.14.

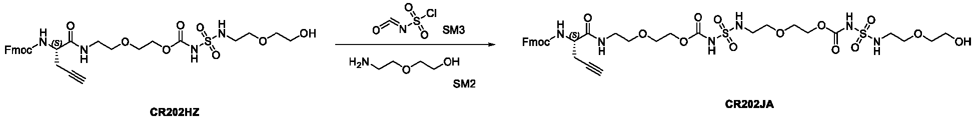

**CR202JA** was prepared by similar procedures to those for preparing **CR202HZ** from **CR202HY.**

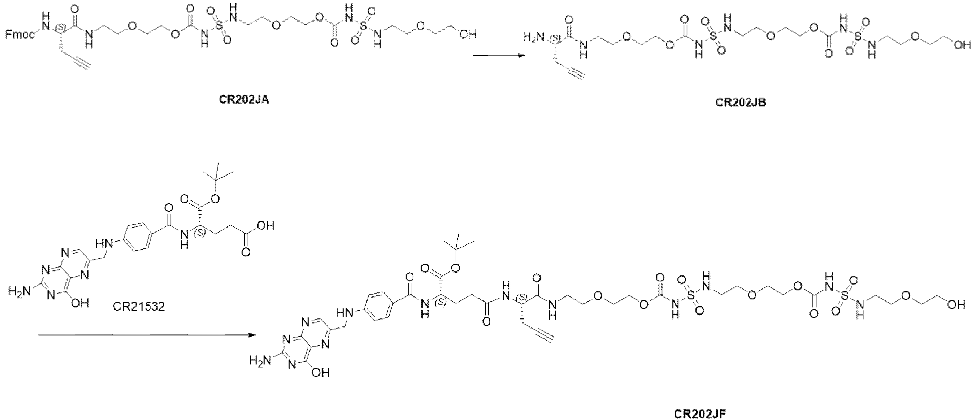

**CR202JA** (6.15 g, 7.29 mmol) was dissolved in DMF (100 mL). The reaction system was purged with nitrogen thrice and cooled to -5 °C before DBU (2.10 g, 13.68 mmol) was added dropwise. After the addition, the reaction system was stirred at 15 °C for 1 to 4 h, until on-line HPLC monitoring indicated a substantial completion of the reaction. A solution of **CR21532** (3.63 g, 7.29 mmol) in DMSO (80 mL) was added, and HATU (4.99 g, 13.12 mmol) was then added. The reaction system was incubated at 15 °C for 30 min, until on-line HPLC monitoring indicated a substantial completion of the reaction. The reaction was terminated, and the crude product was purified (acetonitrile/0.1% TFA-H₂O) and lyophilized to give a yellow-brown solid **CR202JF** (2.55 g, 83.16% purity, 31.87% yield). [M+H]⁺=1100.9.

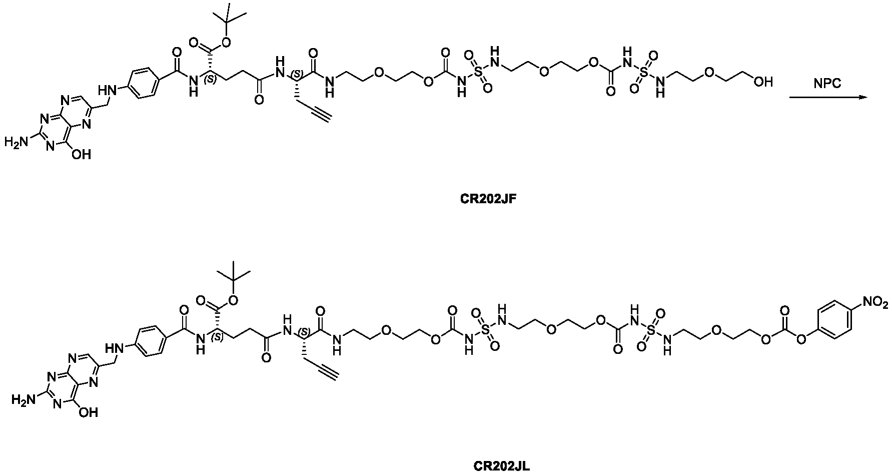

**CR202JF** (1.26 g, 1.14 mmol) was dissolved in anhydrous DMF (15 mL). Spherical molecular sieves were added, and the system was dried for 30 min and filtered. The filtrate was transferred to a 250-mL jacketed flask before anhydrous TEA (0.92 g, 9.12 mmol, pH 8-9) was added. The system was purged with nitrogen thrice before bis(*p*-nitrophenyl) carbonate (NPC, 0.6859 g, 2.3 mmol) was added at 25 °C. The reaction system was stirred by a magnetic stirrer for 8 to 10 h, and the reaction was terminated. 200 mL of MTBE was added to precipitate a solid, and the mixture was centrifuged to give a yellow solid **CR202JL** (1.28 g, 60.86% purity, 87.50% yield). [M+H]⁺=1265.52.

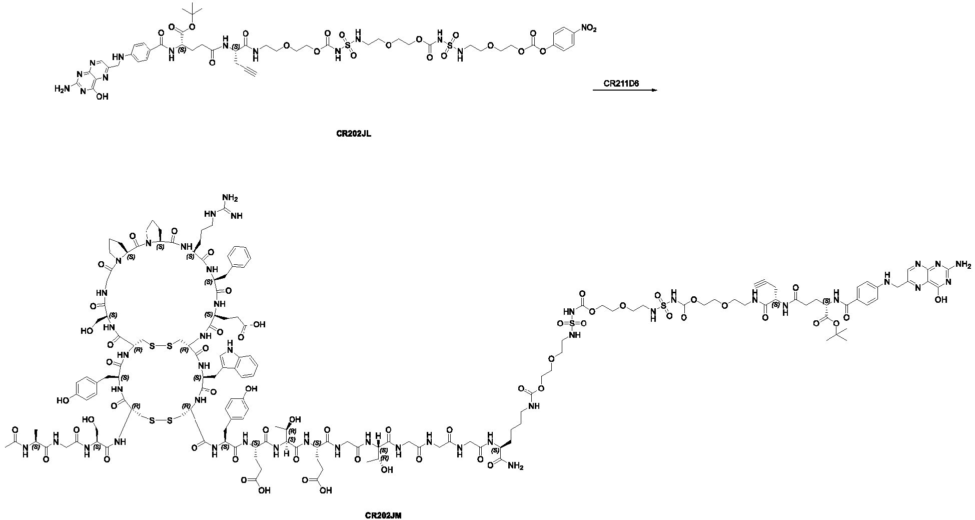

**CR202JL** (600.0 mg, 0.474 mmol) was dissolved in DMF (40 mL), before **CR211D6** (1039.5 mg, 0.474 mmol) and HOBT (115.5 mg, 0.711 mmol) were added. The system was purged with nitrogen thrice, and DIEA (125.9 mg, 0.948 mmol) was added. The reaction temperature was controlled at 25 °C, and the reaction system was stirred with a magnetic stirrer for 3 to 5 h, until on-line HPLC monitoring indicated the completion of the reaction. The reaction was terminated, and 300 mL of MTBE was added to precipitate a solid. The mixture was stirred for 10 min and centrifuged to give a yellow solid **CR202JM** (1.26 g, 60.41% purity, 68.11% yield). [M4+H]⁺=978.09.

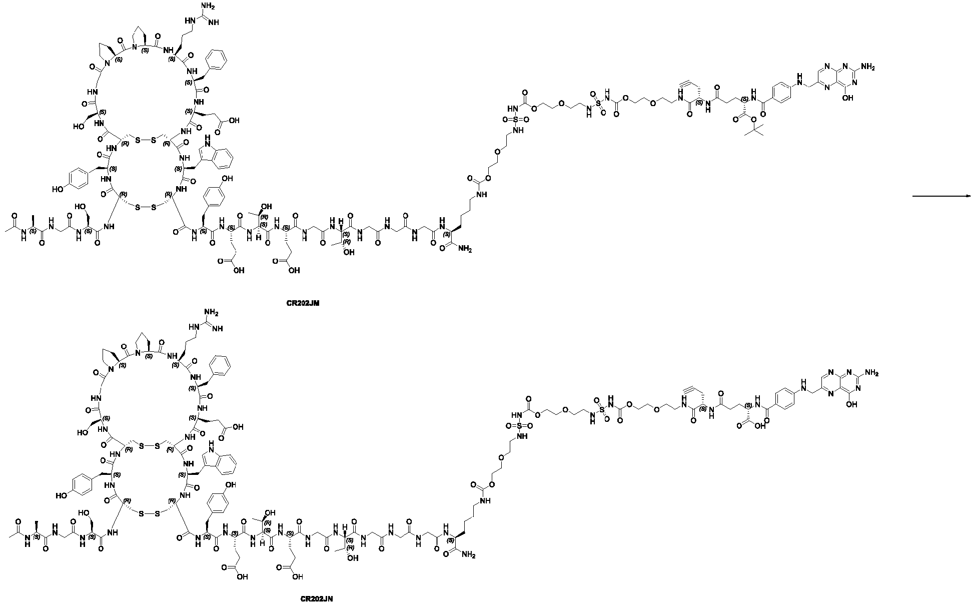

40% TFA/DCM (40 mL) was added to a 100-mL jacketed flask. The reaction system was cooled to -10 °C before **CR202JM** (1.26 g, 0.317 mmol) was added in a nitrogen atmosphere. The system was then slowly warmed to 18 °C and stirred for 2 to 3 h, until on-line HPLC monitoring indicated the completion of the reaction. The reaction was terminated. The reaction mixture was added to 300 mL of cold MTBE to precipitate a solid, and the mixture was stirred for 10 min and centrifuged to give a crude product. The crude product was purified by preparative chromatography and lyophilized to give a yellow solid **CR202JN** (i.e., **L-05,** 160.9 mg, 90.87% purity, 26.67% yield). [M/3+H]+=1285.5.

### 3. Preparation of linkers

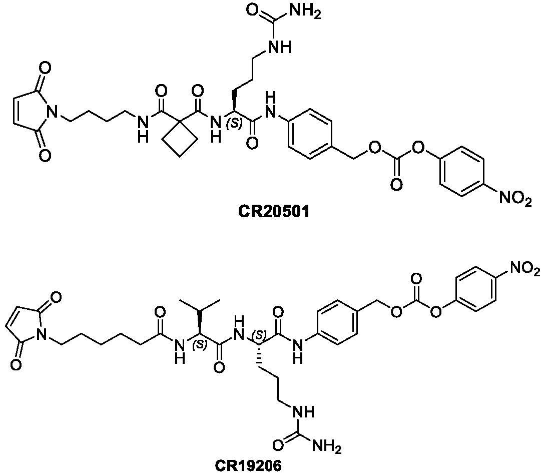

The synthetic route of the linker **CR20501** can be found in J. Med. Chem. 2018, 61, 989-1000. The synthetic route of **CR19206** can be found in Int. J. Mol. Sci. 2017, 18, 1860. The documents are incorporated herein by reference in their entireties. Other linker compounds herein can be synthesized by similar methods as described above.

The polypeptide moiety in the linker of the present invention can be prepared by referring to solid-phase synthesis of the targeted molecule.

### 4. Preparation of conjugate compounds

### 4.1 Preparation of CR202F0

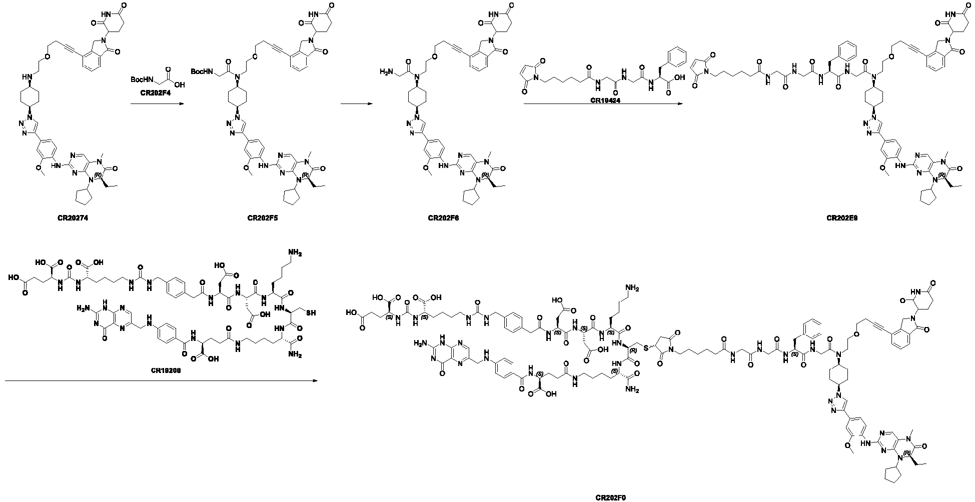

In a nitrogen atmosphere at 0 to 10 °C, **CR202F4** (13.5 mg, 0.077 mmol), HATU (29.3 mg, 0.077 mmol), and DIPEA (10.0 mg, 0.077 mmol) were sequentially added into DCM (1.0 mL), and the reaction system was stirred with a magnetic stirrer for 0.5 h. Free **CR20274** (45.5 mg, 0.05 mmol) was added, and the reaction system was stirred with a magnetic stirrer for dissolving and incubated at 20 to 25 °C for 4 h in a nitrogen atmosphere, until on-line HPLC monitoring indicated the completion of the reaction. Ice water (10.0 mL) and DCM (10.0 mL) were added to the reaction mixture for extraction. The phases were separated, and the organic phase was washed sequentially with saturated citric acid (10.0 mL), saturated sodium bicarbonate (10.0 mL), and saturated brine (10.0 mL), and dried over anhydrous sodium sulfate to give 56.0 mg of a yellow solid (containing **CR202F5**). The crude product was directly used in the next step.

The crude product (56.0 mg, 0.0054 mmol) containing **CR202F5** was added into DCM (2.0 mL). In a nitrogen atmosphere, the reaction system was stirred with a magnetic stirrer for dissolving with the temperature controlled at 0 to 10 °C. TFA (0.5 mL) was dropwise added, and the reaction system was incubated for 1 h, until on-line HPLC monitoring indicated the completion of the reaction. The reaction mixture was concentrated before DCM (10.0 mL) and saturated sodium bicarbonate (2.0 mL) were added. The mixture was then extracted and the phases were separated. The aqueous phase was extracted with DCM (5.0 mL × 2), and the organic phases were combined and dried over anhydrous sodium sulfate to give 49.7 mg of a gray solid (containing **CR202F6**).

In a nitrogen atmosphere at 0 to 10 °C, **CR19424** (29.9 mg, 0.063 mmol), HATU (24.1 mg, 0.063 mmol), and DIPEA (8.2 mg, 0.063 mmol) were sequentially added into DMF (2.0 mL), and the reaction system was stirred with a magnetic stirrer for 0.5 h. Free **CR202F6** (39.7 mg, 0.042 mmol) was added, and the reaction system was stirred with a magnetic stirrer for dissolving and incubated at 20 to 25 °C for 1 h in a nitrogen atmosphere, until on-line HPLC monitoring indicated the completion of the reaction. The reaction mixture was purified by preparative liquid chromatography and lyophilized to give 21.1 mg of a white solid (containing **CR202E9**).

In a nitrogen atmosphere at 20 to 25 °C away from light, **CR202E9** (17.0 mg, 0.012 mmol) and **CR19208** (27.8 mg, 0.018 mmol) were sequentially added into DMF (2.5 mL), and the reaction system was stirred with a magnetic stirrer, adjusted to pH 8 by dropwise adding DIPEA, and incubated for 1 h, until on-line HPLC monitoring indicated the completion of the reaction. The reaction mixture was purified by preparative liquid chromatography and lyophilized to give 15.1 mg of a white solid **(CR202F0).** MS: [M+4H]⁴⁺/4=730.58.

### 4.2 Preparation of CR202F2

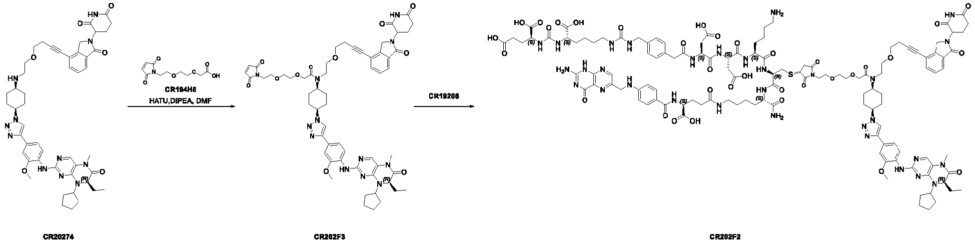

In a nitrogen atmosphere at 0 to 10 °C, **CR194H8** (167.9 mg, 0.068 mmol), HATU (25.8 mg, 0.068 mmol), and DIPEA (13.2 mg, 0.10 mmol) were sequentially added into DMF (0.6 mL), and the reaction system was stirred with a magnetic stirrer for 0.5 h. Free **CR20274** (30.0 mg, 0.034 mmol) was added, and the reaction system was stirred with a magnetic stirrer for dissolving and incubated at 25 °C for 2 h in a nitrogen atmosphere, until on-line HPLC monitoring indicated the completion of the reaction. DMF (2 mL) was added to the reaction system, and when protected from light, **CR19208** (155.2 mg, 0.10 mmol) was added at 15 to 25 °C. The system was stirred for dissolving, adjusted to pH 8 by dropwise adding DIPEA, and incubated for 1.5 h, until on-line HPLC monitoring indicated the completion of the reaction. The reaction mixture was purified by preparative liquid chromatography and lyophilized to give **CR202F2** (25.5 mg). MS: [M+4H]^{4+/}4=658.78.

### 4.3 Preparation of CR20293, CR202CE, CR202CH, CR202CK, CR202CN, CR202CR, CR20214, CR20282, CR20285, CR20287, CR20295, and CR20297

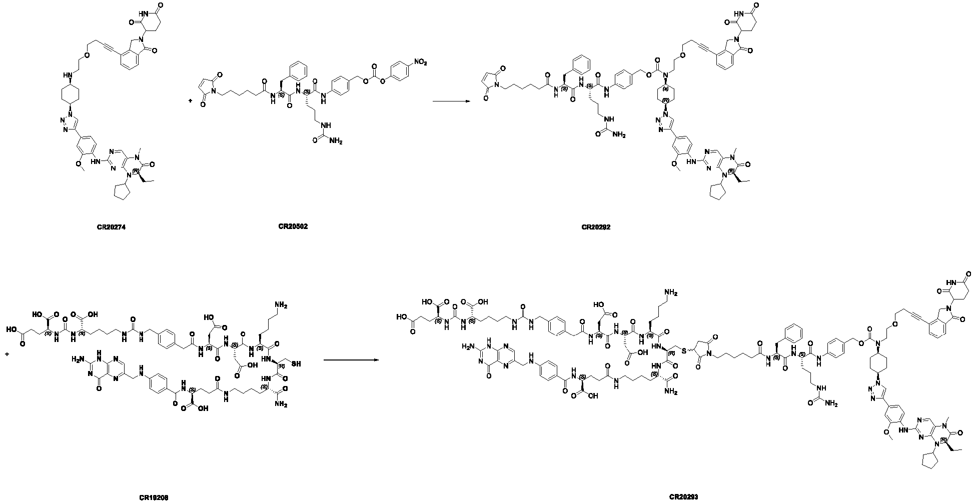

**CR20502** was prepared using a method similar to **CR19206.**

**CR20274** (151.3 mg, 0.171 mmol), **CR20502** (277.3 mg, 0.353 mmol), and HOBt (5.7 mg, 0.042 mmol) were sequentially added into DMF (3.0 mL), and the reaction system was stirred with a magnetic stirrer for dissolving and incubated at 25 °C for 24 h in a nitrogen atmosphere, until on-line HPLC monitoring indicated the completion of the reaction. DMF (6 mL) was added to the reaction system, and when protected from light, **CR19208** (775.0 mg, 0.509 mmol) was added at 15 to 25 °C. The system was stirred for dissolving, adjusted to pH 8 by dropwise adding DIPEA, and incubated for 2 h. A sample of 20 µL was taken and diluted with acetonitrile/water (400 µL). The reaction was monitored by on-line HPLC until completion. The reaction mixture was purified by preparative liquid chromatography to give **CR20293** (175.2 mg, 95.85% purity, 33.7% yield).

The other conjugate compounds CR202CE, CR202CH, CR202CK, CR202CN, CR202CR, CR20214, CR20282, CR20285, CR20287, CR20295, and CR20297 were prepared using a method similar to CR20293.

### 4.4 Synthesis of CR202EZ

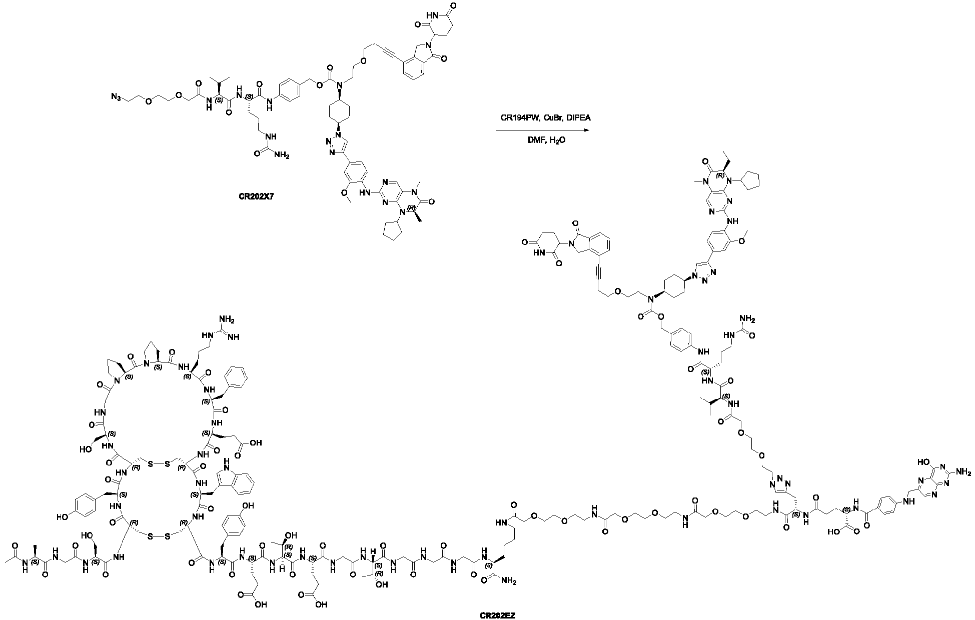

The crude **L-03 (CR194PW)** was dissolved in DMF/water (2.0 mL/0.7 mL) before **CR202X7** (21.8 mg, 14.8 µmol) was added for dissolving. In a nitrogen atmosphere away from light at room temperature (25.9 °C), the reaction system was stirred and adjusted to pH 8 by dropwise adding DIPEA before copper(I) bromide (2.1 mg, 14.8 µmol) was added. The reaction system was incubated at room temperature for 1 h, and the reaction mixture was purified by reverse-phase preparative liquid chromatography and lyophilized to give **CR202EZ** (5.0 mg, 6.7% yield). [M+5H]⁵⁺/5=1040.06.

### 4.5 Synthesis of CR202W5, CR202W6, and CR202W9

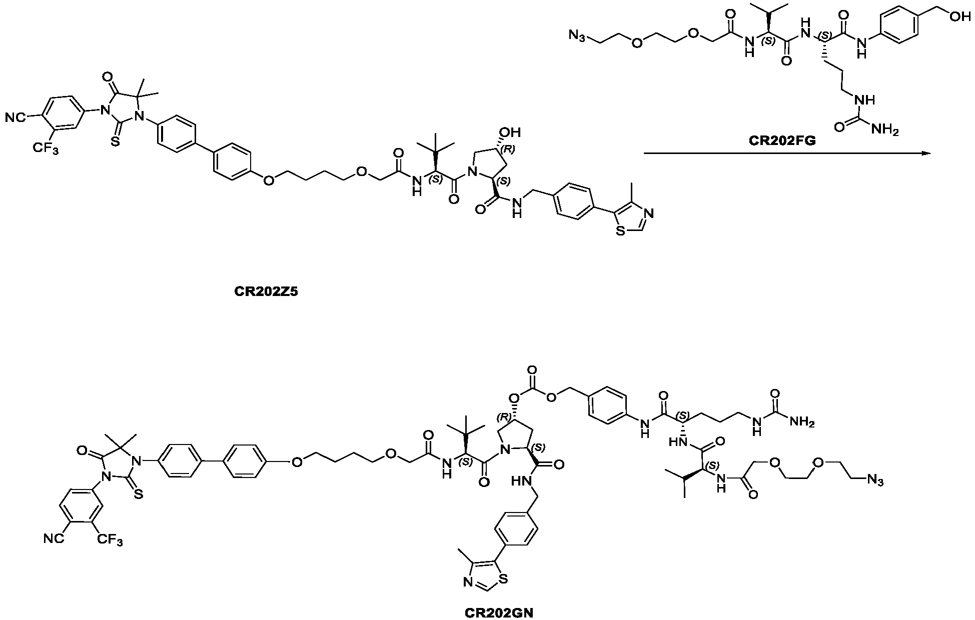

**CR202Z5** (100.0 mg, 97.7 µmol), DCM (2 mL), and 4A molecular sieves (200.0 mg) were added to a 25-mL jacketed single-neck flask R1. The reaction system was cooled to 0 °C before triethylamine (34.5 mg, 342.0 µmol) was added. The reaction system was then stirred for 2 h. A solution of triphosgene (14.5 mg, 48.9 µmol) in DCM (0.1 mL) was added dropwise at 0 °C and the reaction system was incubated for 5 min. Simultaneously, DMF (0.5 mL), **CR202FG** (54 mg, 97.7 µmol), DMAP (12 mg, 97.7 µmol), and TEA (10 mg, 97.7 µmol) were added to a 25-mL jacketed single-neck flask R2, and the reaction system in R2 was stirred for dissolving before 4A molecular sieves (0.1 g) were added. The reaction system was then cooled to 0 °C. In a nitrogen atmosphere, the mixture in R1 was added into R2 dropwise, and the reaction system was stirred for 0.5 h until the completion of the reaction. The reaction in R2 was quenched by adding water (1 mL). The reaction mixture was purified by reverse-phase preparative liquid chromatography to give **CR202GN** (white powder, m = 90 mg, 91.4% purity, 58.4% yield). LCMS: [M+2]²⁺/2=800.9.

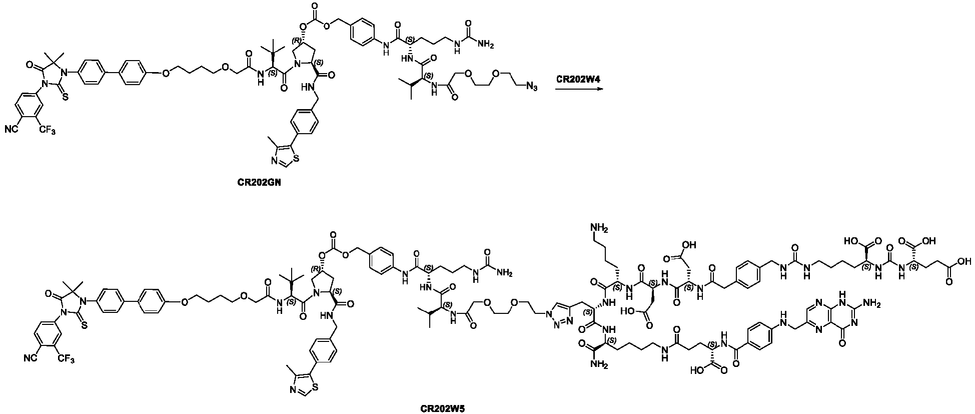

**CR202GN** (75 mg, 46.9 µmol) and **L-04 (CR202W4)** (106.5 mg, 70.3 µmol) were dissolved with DMF (4.5 mL) and water (1.5 mL) away from light. In a nitrogen atmosphere, the system was adjusted to pH 8 by dropwise adding DIPEA, and CuBr (6.7 mg, 46.9 µmol) was added. The reaction system was incubated until on-line HPLC monitoring indicated the completion of the reaction. The reaction mixture was purified by reverse-phase preparative liquid chromatography to give **CR202W5** (white powder, 70.0 mg, 100% purity, 47.9% yield). LCMS: [M-3H]³⁻/3=1037.0.

**CR202W6** and **CR202W9** were synthesized by similar procedures.

### 4.6 Synthesis of CR202X1 and CR202W7

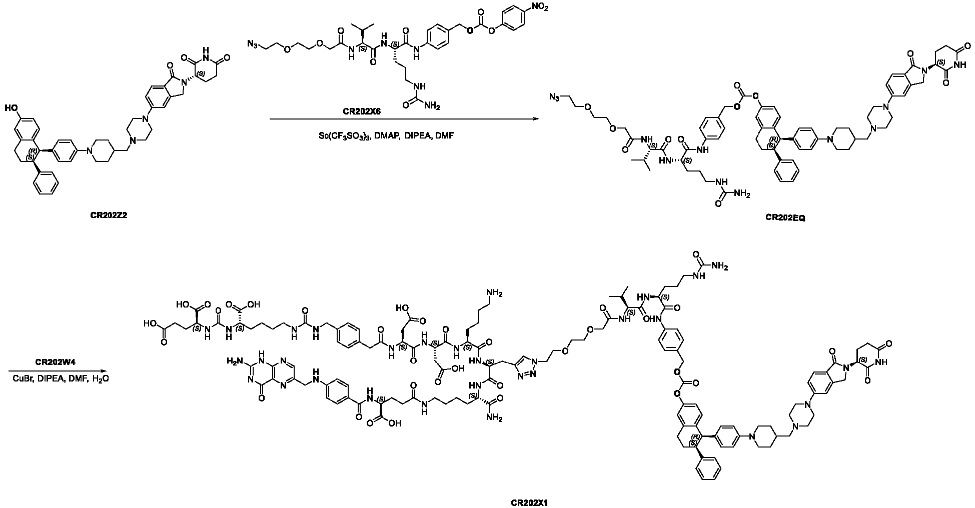

**CR202Z2** (100.0 mg, 138.0 µmol) and DMAP (20.0 mg, 166.0 µmol) were added to DMF (3.6 mL). In a nitrogen atmosphere at -10 °C, scandium trifluoromethanesulfonate (18.0 mg, 41.4 µmol), DIPEA (18.0 mg, 138.0 µmol), and **CR202X6** (129.0 mg, 179.2 µmol) were added sequentially while stirring, and DMF (1.0 mL) was added for dissolving. The reaction system was incubated for 48 h, and the reaction mixture was purified by reverse-phase preparative liquid chromatography to give a white solid **CR202EQ** (140.0 mg, 78.2% yield). [M+H]⁺=1300.5.

**CR202X1** was synthesized similarly to **CR202W5.**

**CR202W7** was synthesized similarly.

### 4.7 Synthesis of CR202W8

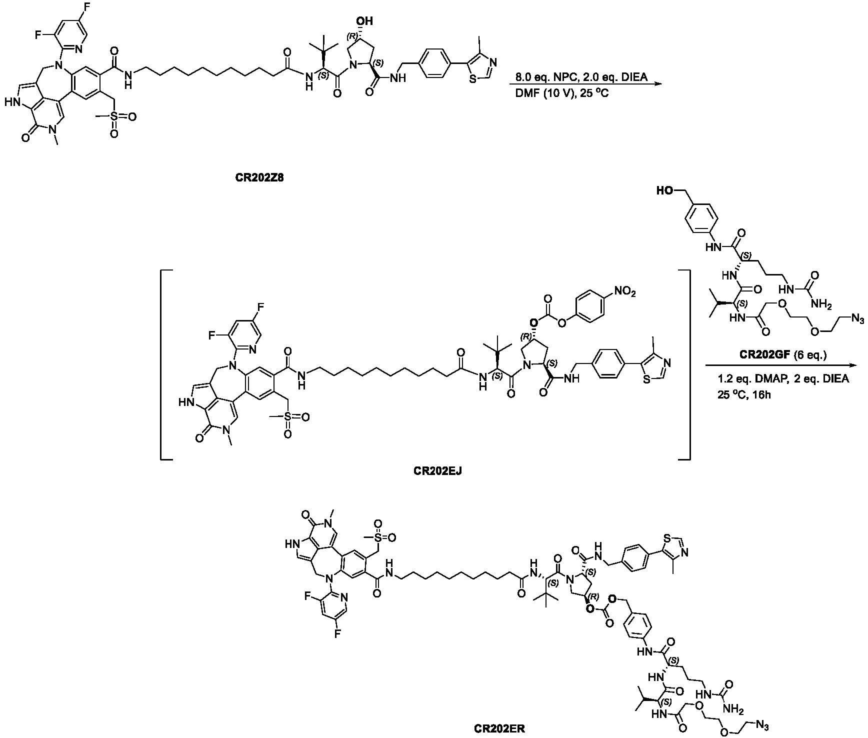

**CR202Z8** (30 mg, 27.4 µmol) and NPC (67 mg, 220 µmol) were dissolved in DMF (0.3 mL) with magnetic stirring in a nitrogen atmosphere. DIEA (7.1 mg, 54.9 µmol) was added and the reaction system was stirred at 25 °C for 15 h. **CR202GF** (45 mg, 81.7 µmol), DMAP (4 mg, 32.7 µmol), and DIEA (7.1 mg, 54.9 µmol) were added, and the reaction system was stirred at 25 °C for 16 h, until on-line detection indicated the completion of the reaction. The reaction mixture was purified by preparative chromatography to give a white solid **CR202ER** (29 mg, 98.24% purity, 63% yield). LCMS: [M+2H]²⁺/2 = 837.5.

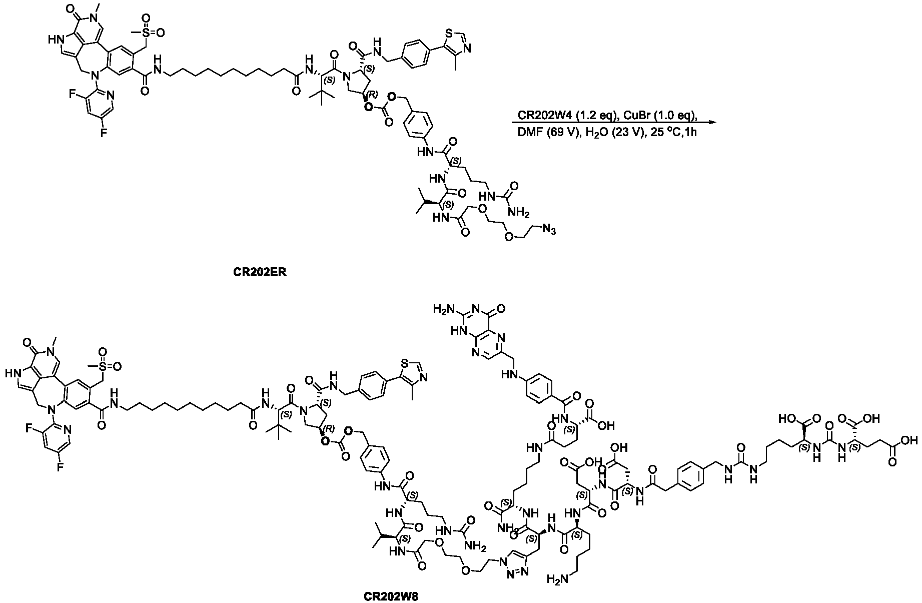

**CR202ER** (50 mg, 29.9 µmol) and **CR202W4** (54 mg, 35.6 µmol) were dissolved in DMF (2.5 mL) and water (0.8 mL) away from light with magnetic stirring in a nitrogen atmosphere. The reaction system was adjusted to pH 8 by adding DIEA (130 µL) before CuBr (4.3 mg, 29.9 µmol) was added. The reaction system was then incubated at 25 °C for 1 h, until on-line detection indicated the completion of the reaction. The reaction mixture was purified by preparative liquid chromatography to give **CR202W8** (33 mg, 41% yield). LCMS: [M+3H]³⁺/3 = 1063.1.

### 4.8 Synthesis of CR202V4

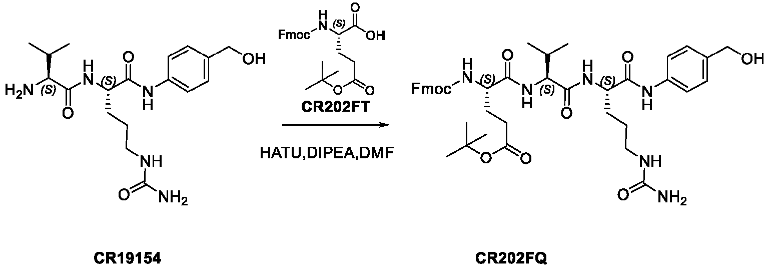

**CR202FT** (6.73 g, 15.81 mmol) was dissolved in DMF (50 mL) with magnetic stirring. The reaction system was then cooled to 0 °C before HATU (6.61 g, 17.39 mmol) was added. The reaction system was stirred at 0 °C for 10 min. **CR19154** (6.00 g, 15.8 mmol) was added. A proper amount of DIEA (2.25 g, 17.39 mmol) was dissolved in DMF (10 mL) and the solution was added dropwise to the system. The reaction system was stirred at 0 °C for 1 h. The reaction was monitored until the depletion of the starting materials. The reaction mixture was added to ethyl acetate (180 mL), and the mixture was stirred for 0.5 h, centrifuged, washed thrice with ethyl acetate, centrifuged, and incubated in an oven overnight (35 °C) to give a yellow solid **CR202FQ** (11.93 g, 80% purity, 76.7% yield). [M+H]⁺=787.47.

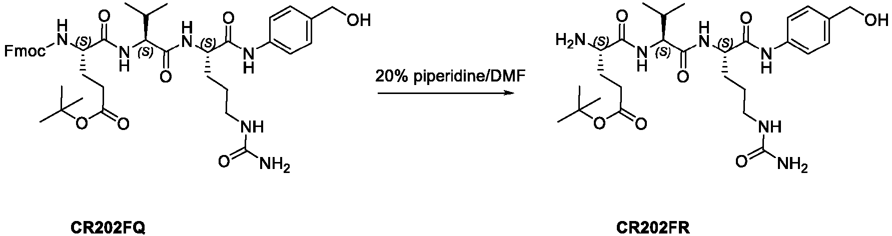

**CR202FQ** (6.5 g, 8.26 mmol) was dissolved in methanol (70 mL) with magnetic stirring. A 20% piperidine/DMF solution (12.3 g, 28.91 mmol) was added and the reaction system was stirred at 50 °C overnight. The reaction was monitored by LCMS until the depletion of the starting materials. Methanol was removed by rotary evaporation. The residue was extracted twice with ethyl acetate (50 mL × 2), and the mixture was concentrated by rotary evaporation to about 15 mL. Methyl tert-butyl ether (200 mL) was added and a white solid was precipitated. The mixture was filtered, and the filter cake was rinsed with methyl tert-butyl ether (20 mL × 2), and dried *in vacuo* to give an off-white solid **CR202FR** (4.09 g, 91% purity, 79.8% yield).

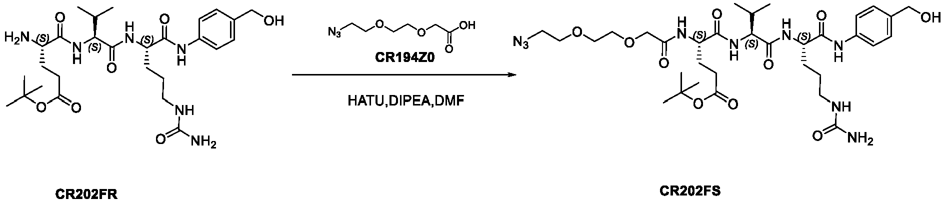

**CR194Z0** (1.51 g, 7.97 mmol) was dissolved in DMF (30 mL) with magnetic stirring. The reaction system was then cooled to 0 °C before HATU (3.03 g, 7.97 mmol) was added. The reaction system was stirred at 0 °C for 10 min. **CR202FR** (4.09 g, 7.24 mmol) was added. A proper amount of DIEA (1.03 g, 7.97 mmol) was dissolved in DMF (10 mL) and the solution was added dropwise to the system. The reaction system was stirred at 0 °C for 1 h. The reaction was monitored by LCMS until the depletion of the starting materials. The reaction mixture was added to ethyl acetate (150 mL), and the mixture was stirred overnight. A solid was precipitated, centrifuged, washed once with methyl tert-butyl ether, and dried to give a pale yellow solid **CR202FS** (3.7 g, 79.6% purity, 69.5% yield). [M+H]⁺=736.51.

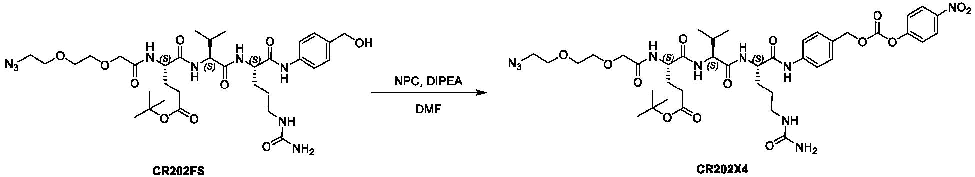

**CR202FS** (1.7 g, 2.3103 mmol) was dissolved in DMF (20 mL) with magnetic stirring. NPC (1.4 g, 4.6206 mmol) and DIEA (447.9 mg, 3.4655 mmol) were added, and the reaction system was stirred at room temperature for 1 h. The reaction mixture was purified by reverse-phase preparative liquid chromatography to give a white solid **CR202X4** (1.77 g, 99.28% purity, 85.04% yield). ¹H-NMR (400 MHz, DMSO) δ 10.16 (s, 1H), 8.38-8.30 (m, 2H), 8.25 (d, *J =* 7.2 Hz, 1H), 7.96 (d, *J* = 8.6 Hz, 1H), 7.75 (d, *J* = 8.2 Hz, 1H), 7.68 (d, *J* = 8.5 Hz, 2H), 7.64-7.55 (m, 2H), 7.44 (d, *J =* 8.6 Hz, 2H), 6.06 (s, 0H), 5.27 (s, 2H), 4.51-4.37 (m, 3H), 4.24 (dd, *J* = 8.4, 6.9 Hz, 1H), 3.96 (s, 2H), 3.70-3.58 (m, 6H), 3.49-3.39 (m, 2H), 3.03 (ddd, *J* = 38.8, 13.1, 6.5 Hz, 2H), 2.29-2.19 (m, 2H), 1.98 (ddd, *J* = 20.3, 12.7, 6.0 Hz, 2H), 1.85-1.67 (m, 2H), 1.55 (ddd, *J* = 21.1, 11.2, 5.0 Hz, 2H), 1.41 (s, 10H), 0.87 (dd, *J* = 14.8, 6.8 Hz, 6H). [M+H]⁺=901.46.

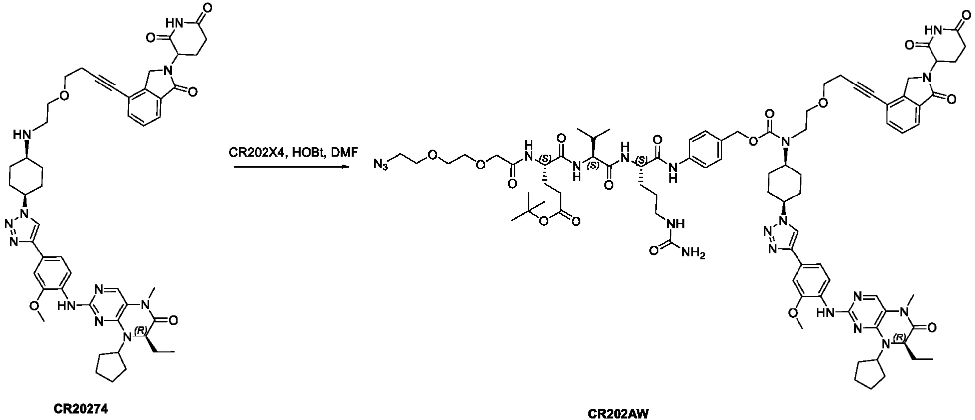

Free **CR20274:** CR20274 hydrochloride (902.8 mg, 0.94 mmol) was added to DCM (50.0 mL) and methanol (10.0 mL) with magnetic stirring until the solid was completely dissolved. At 5 to 10 °C, a 10% sodium bicarbonate aqueous solution (20 mL) was dropwise added. The reaction system was stirred for 30 min before DCM (50 mL), methanol (5 mL), and water (20 mL) were added. The reaction mixture was extracted (with a thick emulsion layer), and the aqueous phase was extracted twice, each time with a mixed DCM (50 mL)/methanol (5 mL) solution. The organic phases were combined, dried over anhydrous sodium sulfate, and concentrated *in vacuo* at 30 °C to give a solid free **CR20274** (910.4 mg, 92.04% purity, 100.0% yield), which was used directly in the subsequent steps without purification.

Free **CR20274** (60.0 mg, 0.068 mmol), **CR202X4** (150.1 mg, 0.170 mmol), and HOBt (1.8 mg, 0.014 mmol) were sequentially added into DMF (1.5 mL), and the reaction system was stirred with a magnetic stirrer for dissolving and incubated at 25 °C for 24 h in a nitrogen atmosphere. DIPEA (12 µL, 0.068 mmol) was added, and the reaction system was further incubated for 20 h, until on-line HPLC monitoring indicated the completion of the reaction. The reaction mixture was directly purified by preparative chromatography to give **CR202AW** (64.9 mg, 92.96% purity, 43.6% yield). [M+2H]⁺/2=823.8.

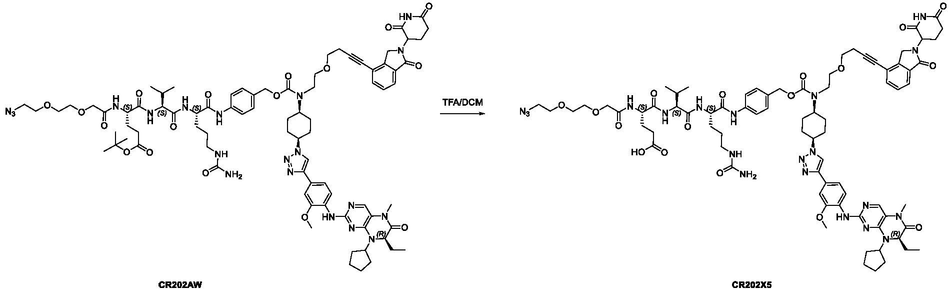

**CR202AW** (50.0 mg, 0.03 mmol) was added into DCM (2.0 mL). In a nitrogen atmosphere at 15 °C, TFA (0.2 mL) was dropwise added, and the reaction system was incubated for 3 h, until on-line HPLC monitoring indicated the completion of the reaction. At an external temperature of 10 °C, saturated NaHCO₃ was dropwise added to adjust the reaction mixture to pH 7 to 8. The mixture was concentrated to remove DCM, and a small amount of solid was precipitated. The solid was dissolved in acetonitrile (4 mL), and the mixture was purified by reverse-phase preparative liquid chromatography to give **CR202X5** (20.6 mg, 84.20% purity, 42.9% yield).

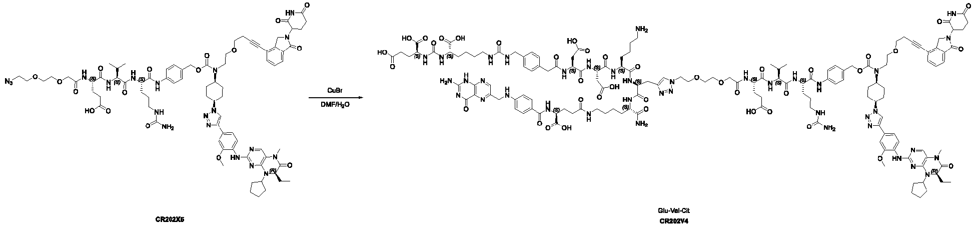

In a 25-mL jacketed flask, **CR202X5** (16.0 mg, 10.1 µmol) and **CR202W4** (18.3 mg, 12.1 µmol) were dissolved in DMF (1 mL) and water (0.3 mL) with magnetic stirring in a nitrogen atmosphere away from light. The reaction system was adjusted to pH 8 by adding DIEA (250 µL) before CuBr (1.5 mg, 1.0 eq.) was added. The reaction system was then incubated at 25 °C for 2 h. The reaction mixture was purified by preparative liquid chromatography to give **CR202V4** (5.7 mg, 92.26% purity, 13.9% yield). [M+3H]³⁺/3=1035.98.

### 4.9 Synthesis of CR202V5, CR202V6, CR202Z6, and CR202V7

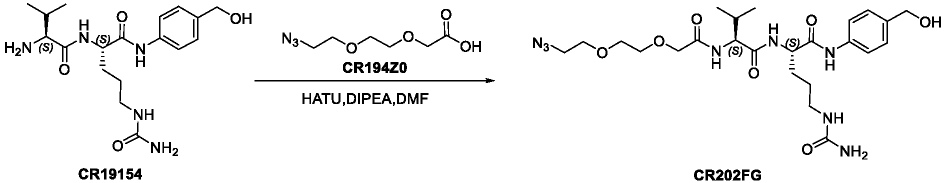

**CR202Z0** (3.00 g, 15.81 mmol), HATU (6.01 g, 15.81 mmol), DIPEA (2.73 g, 21.08 mmol), and **CR19154** (4.00 g, 10.54 mmol) were sequentially added into DMF (40 mL). In a nitrogen atmosphere at 25 °C, the reaction system was incubated for 2 h until on-line HPLC monitoring indicated the completion of the reaction. The reaction mixture was directly purified by reverse-phase preparative liquid chromatography to give **CR202FG** (1.88 g, 43.2% yield). [M+H]⁺ =551.31.

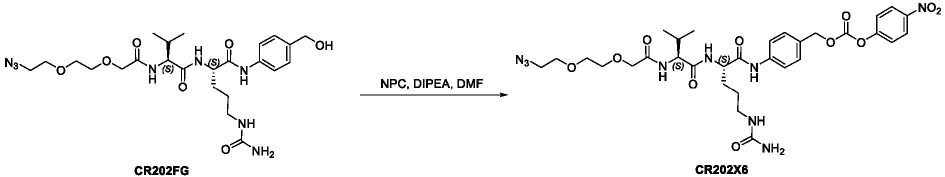

**CR202FG** (1.48 g, 2.68 mmol) and NPC (1.63 g, 5.36 mmol) were sequentially added into DMF (7.5 mL) before DIEA (519.7 mg, 4.02 mmol) was dropwise added. The reaction system was stirred with a magnetic stirrer and incubated for 2 h in a nitrogen atmosphere, until on-line HPLC monitoring indicated the completion of the reaction. The reaction mixture was directly purified by reverse-phase preparative liquid chromatography to give **CR202X6** (2.07 g, 100.0% purity, 100% yield). ¹H-NMR (400 MHz, DMSO) δ 10.17 (s, 1H), 8.40 (d, *J* = 7.3 Hz, 1H), 8.34 (d, *J* = 9.1 Hz, 3H), 7.68 (d, *J* = 8.5 Hz, 2H), 7.63-7.57 (m, 2H), 7.49 (d, *J =* 9.0 Hz, 1H), 7.44 (d, *J* = 8.5 Hz, 3H), 6.04 (s, 1H), 5.27 (s, 2H), 4.42 (d, *J* = 5.3 Hz, 1H), 4.35 (dd, *J* = 8.8, 6.6 Hz, 1H), 3.99 (s, 2H), 3.73-3.58 (m, 8H), 3.43 (t, *J* = 4.9 Hz, 2H), 3.02 (d, *J* = 21.0 Hz, 2H), 2.03 (dd, *J* = 13.4, 6.7 Hz, 1H), 1.79-1.54 (m, 2H), 1.42 (dd, *J* = 21.6, 14.8 Hz, 2H), 0.91 (d, *J* = 6.7 Hz, 3H), 0.85 (d, *J* = 6.7 Hz, 3H). [M+H]⁺ =716.30.

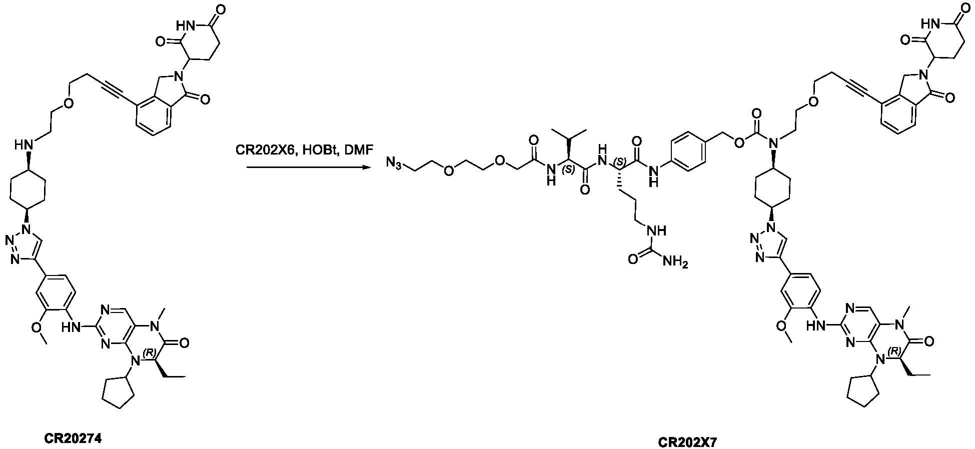

Free **CR20274:** CR20274 hydrochloride (902.8 mg, 0.94 mmol) was added to DCM (50.0 mL) and methanol (10.0 mL) with magnetic stirring until the solid was completely dissolved. At 5 to 10 °C, a 10% sodium bicarbonate aqueous solution (20 mL) was dropwise added. The reaction system was stirred for 30 min before DCM (50 mL), methanol (5 mL), and water (20 mL) were added. The reaction mixture was extracted (with a thick emulsion layer), and the aqueous phase was extracted twice, each time with a mixed DCM (50 mL)/methanol (5 mL) solution. The organic phases were combined, dried over anhydrous sodium sulfate, and concentrated *in vacuo* at 30 °C to give a solid **CR20274** (910.4 mg, 100.0% yield, 92.04% purity), which was directly used in the next step without purification.

Free **CR20274** (200.0 mg, 0.23 mmol), **CR202X6** (404.7 mg, 0.57 mmol), and HOBt (6.1 mg, 0.045 mmol) were sequentially added into DMF (5.0 mL), and the reaction system was stirred with a magnetic stirrer for dissolving and incubated at 25 °C for 24 h in a nitrogen atmosphere, until on-line HPLC monitoring indicated the completion of the reaction. The reaction mixture was directly purified by reverse-phase preparative liquid chromatography to give **CR202X7** (134.0 mg, 98.32% purity, 40.6% yield). [M+2H]²⁺ =730.99.

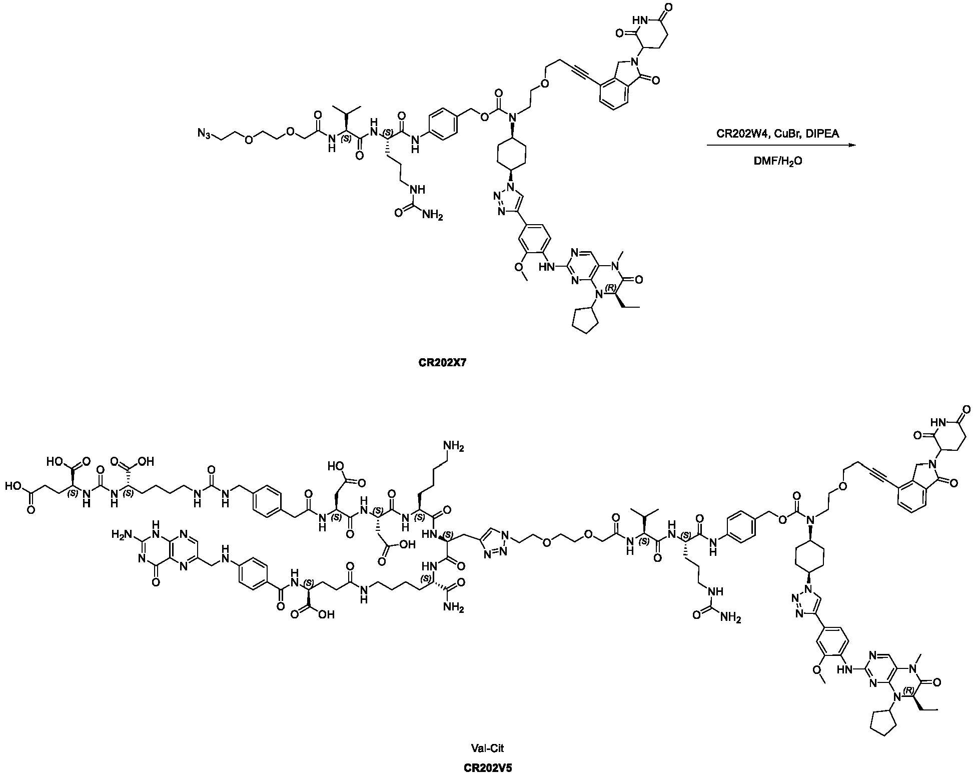

**CR202X7** (130.0 mg, 0.089 mmol) and **CR202W4** (161.8 mg, 0.107 mmol) were dissolved in DMF (7.8 mL) and water (2.6 mL) with magnetic stirring in a nitrogen atmosphere away from light. The reaction system was adjusted to pH 8 by adding DIEA (250 µL) before CuBr (12.7 mg, 0.089 mmol) was added. The reaction system was then incubated at 25 °C for 2 h, until on-line detection indicated the completion of the reaction. The reaction mixture was purified by reverse-phase preparative liquid chromatography to give **CR202V5** (76.6 mg, 95.70% purity, 28.9% yield). MS: [M+4H]⁴⁺/4=745.02.

CR202V6, CR202Z6, and CR202V7 were synthesized similarly.

### 4.10 Synthesis of CR202V8

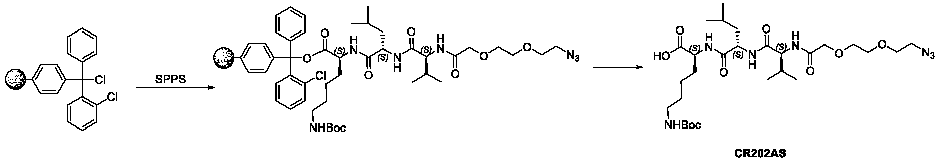

### Synthesis of CR202AS

Resin swelling: A proper amount of 2-CTC resin (50.00 g, 32.5 mmol) was added to anhydrous DCM (500 mL). The system was stirred (120 rpm), purged with nitrogen, and incubated at 25 °C for 30 min for resin swelling.

Amino acid coupling: Fmoc-Lys(boc)-OH (45.68 g, 97.5 mmol) and DIEA (21.00 g, 162.5 mmol) were added and the reaction system was further incubated for 3 h.

End capping: The reaction system was transferred to a solid-phase synthesis reactor. The liquid was drained, and the resin was washed thrice with DMF (500 mL × 3). Then DMF (500 mL) was added, and the system was purged with nitrogen before methanol (10.41 g, 325 mmol) and DIEA (12.60 g, 97.5 mmol) were added. The reaction system was incubated at 25 °C for 1 h. The liquid was drained and the resin was washed 5 times with DMF (500 mL × 5).

Deprotection: A 20% piperidine solution (500 mL) was added. The reaction system was purged with nitrogen and incubated at 25 °C for 20 min. Several pieces of resin were taken, washed with ethanol thrice, added to ninhydrin chromogenic agent, and heated at 110 °C for 2 min until the resin turned purple. The reaction was terminated. The liquid was drained and the resin was washed 5 times with DMF (500 mL × 5).

Amino acid coupling: Proper amounts of Fmoc-Leu-OH (17.33 g, 48.8 mmol) and HOBT (6.59 g, 48.8 mmol) were added to DMF (250 mL). The mixture was stirred with a magnetic stirrer for dissolving before DIC (6.15 g, 48.98 mmol) was added. The mixture was stirred for 3 min, transferred to a reactor, and incubated at 25 °C for 1 h. The reaction was monitored by resin sampling. When the heated resin turned white, the reaction was terminated. The liquid was drained and the resin was washed thrice with DMF (500 mL × 3). Fmoc-Val-OH and N₃-PEG₂-CH₂-COOH were sequentially added for coupling to the resin.

Resin shrinkage: The resin was washed twice with DCM (500 mL × 2), and MeOH was added to shrink the resin. The mixture was dried and weighed 66.00 g.

Cleavage: 22.00 g of resin was added to 240 mL of prepared cleavage solution (72 mL of trifluoroethanol diluted to 240 mL with DCM). The reaction system was stirred at 25 °C for 3 h in a nitrogen atmosphere.

Post-treatment: The resin was removed by filtration at reduced pressure and washed once with a small amount of dichloromethane. The filtrate was directly concentrated to give 8.08 g of an oily liquid **CR202AS,** which was solidified by storage at -20 °C (91.08% HPLC purity, 118.0% yield; reason for overweight: the product had trifluoroethanol remaining).

### Synthesis of CR202AY

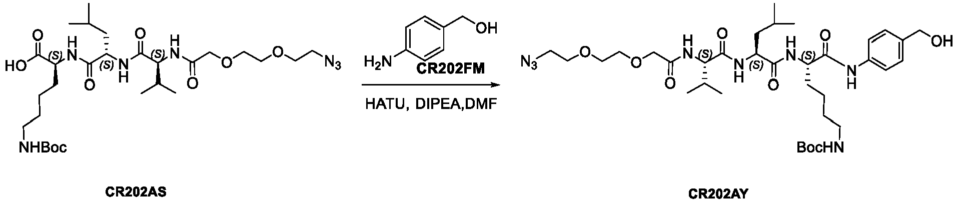

**CR202AS** (3.51 g, 5.6 mmol) and HATU (2.97 g, 7.8 mmol) were added sequentially to a mixed solvent of DMF (33 mL), EA (ethyl acetate, 18 mL), and water (3.5 mL). The mixture was stirred with a magnetic stirrer in a nitrogen atmosphere and cooled to 0 °C before a p-aminobenzyl alcohol solution (1.03 g, 8.4 mmol; in EA (7 mL) and DMF (1.8 mL)) and a DIEA solution (1.3 mL, 7.8 mmol; in EA (7 mL)) were dropwise added sequentially. The reaction system was incubated at 0 °C for 1 h, until on-line HPLC monitoring indicated the completion of the reaction. The reaction mixture was added to purified water (200 mL) and extracted with EA (100 mL × 3). The organic phases were combined, washed sequentially with purified water (100 mL) and saturated brine (100 mL), dried over anhydrous sodium sulfate, and concentrated to give a yellow oily liquid **CR202AY** (4.1466 g, 86.48% HPLC purity, 96.5% yield).

### Synthesis of CR202CU

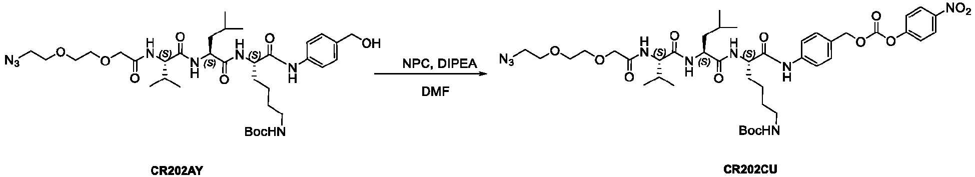

**CR202AY** (2.0783 g, 2.8 mmol) and di-p-nitrophenyl carbonate (1.5486 g, 5.1 mmol) were added to DCM (30 mL). The reaction system was stirred with a magnetic stirrer until complete dissolution before DIEA (0.93 mL, 5.7 mmol) was added. The reaction system was incubated in a nitrogen atmosphere at 25 °C for 3 h. The reaction was monitored by on-line HPLC until completion. The reaction mixture was concentrated at 25 °C and purified by preparative liquid chromatography to give a white solid **CR202Z1** (1.3924 g, 95.00% HPLC purity, 52.8% yield).

### Synthesis of CR202AZ

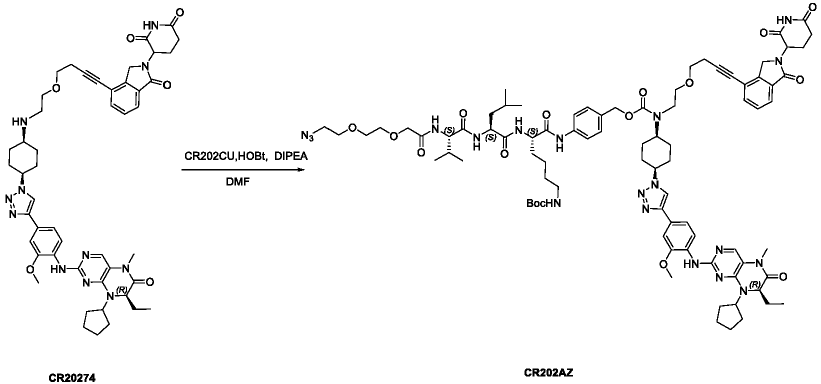

**CR20274** (150 mg, 0.17 mmol) and **CR202CU** (275.0 mg, 0.31 mmol) were sequentially added into DMF (3.0 mL), and the reaction system was stirred with a magnetic stirrer until complete dissolution. HOBT (4.6 mg, 0.034 mmol) and DIEA (40 mg, 0.26 mmol, 1.5 eq) were added, and the reaction system was incubated at 25 °C for 18 h in a nitrogen atmosphere. The reaction was monitored by on-line HPLC until completion, and the reaction mixture was purified by preparative liquid chromatography to give a white solid **CR202AZ** (170.0 mg, 98.89% HPLC purity, 60.9% yield). [M+2H]²⁺/2=823.46.

### Synthesis of CR202BA

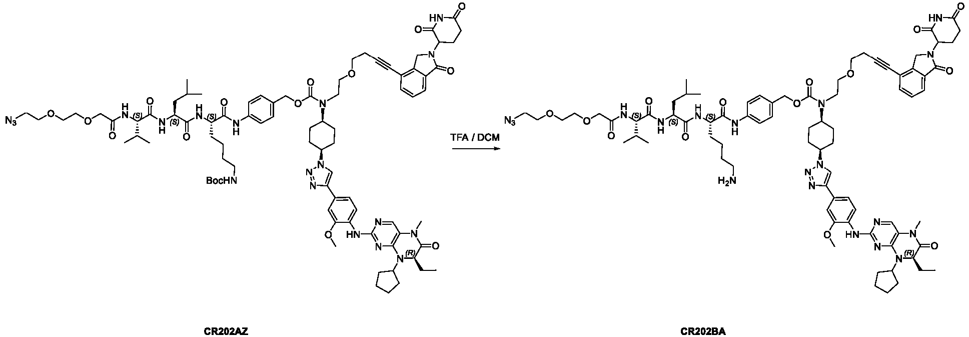

The **CR202AZ** (170.0 mg, 0.10 mmol) was added into DCM (4.0 mL). In a nitrogen atmosphere at an external temperature of 15 °C, TFA (0.4 mL) was dropwise added, and the reaction system was incubated for 2.5 h, until on-line HPLC monitoring indicated the completion of the reaction. At an external temperature of 0 °C, the reaction mixture was adjusted to pH 7 to 8 by dropwise adding a saturated sodium bicarbonate solution. At 15 °C, the mixture was concentrated to remove DCM, and the residue was dissolved in acetonitrile (10 mL). The reaction mixture was purified by preparative liquid chromatography to give a white solid **CR202BA** (34.0 mg, 89.36% HPLC purity, 21.4% yield). [M+2H]²⁺/2=773.3.

### Synthesis of CR202V8

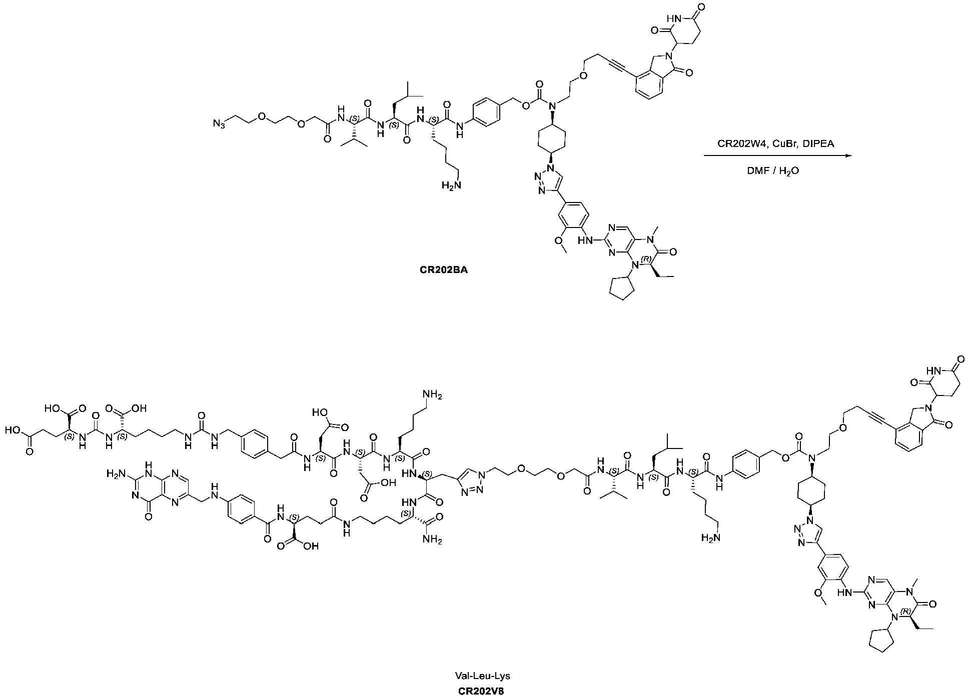

**CR202BA** (55.0 mg, 0.036 mmol) and **CR202W4** (64.7 mg, 0.43 mmol) were dissolved in DMF (4.0 mL) and water (1.3 mL) with magnetic stirring in a nitrogen atmosphere away from light. The reaction system was adjusted to pH 8 by adding DIEA before CuBr (5.1 mg, 0.036 mmol) was added. The reaction system was then incubated at 25 °C for 2 h, until on-line detection indicated the completion of the reaction. The reaction mixture was purified by preparative liquid chromatography and desalted to give **CR202V8** (22.5 mg, 95.03% purity, 20.8% yield). [M+3H]³⁺/3=1020.48.

### 4.11 Synthesis of CR202V9

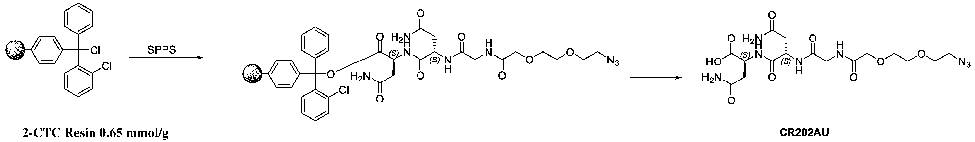

### Synthesis of CR202AU

Resin swelling: A proper amount of 2-CTC resin (50.00 g, 32.5 mmol) was added to anhydrous DCM (500 mL). The system was stirred, purged with nitrogen, and incubated at 25 °C for 30 min for resin swelling.

Amino acid coupling: Fmoc-Asn(trt)-OH (58.18 g, 97.5 mmol) and DIEA (21.00 g, 162.5 mmol) were added and the reaction system was further incubated for 3 h.

End capping: The reaction system was transferred to a solid-phase synthesis reactor. The liquid was drained, and the resin was washed thrice with DMF (500 mL × 3). Then DMF (500 mL) was added, and the system was purged with nitrogen before methanol (10.41 g, 325 mmol) and DIEA (12.60 g, 97.5 mmol) were added. The reaction system was incubated at 25 °C for 1 h. The liquid was drained and the resin was washed 5 times with DMF (500 mL × 5).

Deprotection: A 20% piperidine solution (500 mL) was added. The reaction system was purged with nitrogen and incubated at 25 °C for 20 min. Several pieces of resin were taken, washed with ethanol thrice, added to ninhydrin chromogenic agent, and heated at 110 °C for 2 min until the resin turned purple. The reaction was terminated. The liquid was drained and the resin was washed 5 times with DMF (500 mL × 5).

Amino acid coupling: Proper amounts of Fmoc-Asn(trt)-OH (17.33 g, 48.8 mmol) and HOBT (6.59 g, 48.8 mmol) were added to DMF (250 mL). The mixture was stirred with a magnetic stirrer for dissolving before DIC (6.15 g, 48.98 mmol) was added. The mixture was stirred for 3 min, transferred to a reactor, and incubated at 25 °C for 1 h. The reaction was monitored by resin sampling. When the heated resin turned white, the reaction was terminated. The liquid was drained and the resin was washed thrice with DMF (500 mL × 3). Fmoc-Gly-OH and N₃-PEG₂-CH₂-COOH were sequentially added for coupling to the resin.

Resin shrinkage: The resin was washed twice with DCM (500 mL × 2), and MeOH was added to shrink the resin. The mixture was dried and weighed 96.42 g.

Cleavage: 32.14 g of resin was added to a prepared cleavage solution (300 mL; DCM:TFA:Tis=4.9:4.9:0.2) at 5 °C. The reaction system was stirred at 25 °C for 3 h in a nitrogen atmosphere.

Post-treatment: The resin was removed by filtration at reduced pressure and washed once with a small amount of dichloromethane. The filtrate was directly concentrated to remove DCM and added to 600 mL of pre-cooled MTBE to precipitate a white solid. The mixture was stirred for 10 min and filtered. The solid was washed twice with MTBE (100 mL × 2) and dried to give 5.21 g of a white solid CR202AU (92.71% HPLC purity, 101.6% yield; reason for overweight: the product had TFA remaining).

### Synthesis of CR202AV

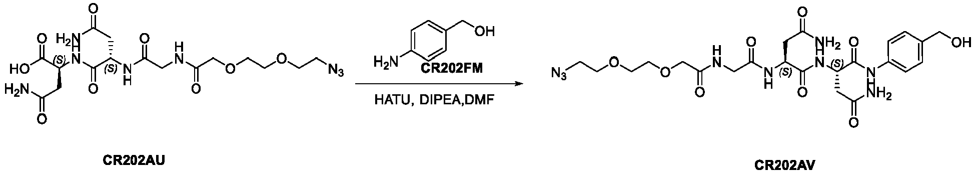

**CR202AU** (2.50 g, 5.3 mmol) and *p*-aminobenzyl alcohol (0.54 g, 4.4 mmol) were added to DMF (12 mL). The reaction system was stirred with a magnetic stirrer until complete dissolution before HATU (2.51 g, 6.6 mmol) and DIEA (2.20 mL, 13.2 mmol) were added. The reaction system was incubated at 25 °C for 1.5 h. The reaction was monitored by on-line HPLC until completion. The reaction mixture was added to pre-cooled MTBE (60 mL) to precipitate a white solid. The mixture was stirred with a magnetic stirrer for 10 min and filtered. The solid was washed twice with MTBE (20 mL × 2) and dried to give a white solid **CR202AV** (1.3194 g, 87.79% purity, 51.9% yield).

### Synthesis of CR202CV

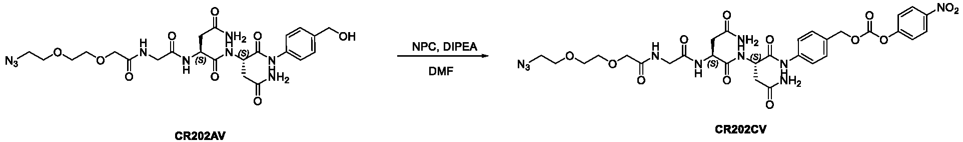

**CR202AV** (1.28 g, 2.2 mmol) and di-p-nitrophenyl carbonate (1.21 g, 4.0 mmol) were added to DMF (10 mL). The reaction system was stirred with a magnetic stirrer until complete dissolution before DIEA (0.73 mL, 4.4 mmol) was added. The reaction system was incubated in a nitrogen atmosphere at 25 °C for 2 h. The reaction was monitored by on-line HPLC until completion. The reaction mixture was added to MTBE (60 mL) to precipitate a white solid. The mixture was stirred for 10 min and filtered. The solid was washed twice with MTBE (20 mL × 2) and dried *in vacuo* at 30 °C to give a white solid **CR202CV** (1.19 g, 87.40% HPLC purity, 84.8% yield).

### Synthesis of CR202BD

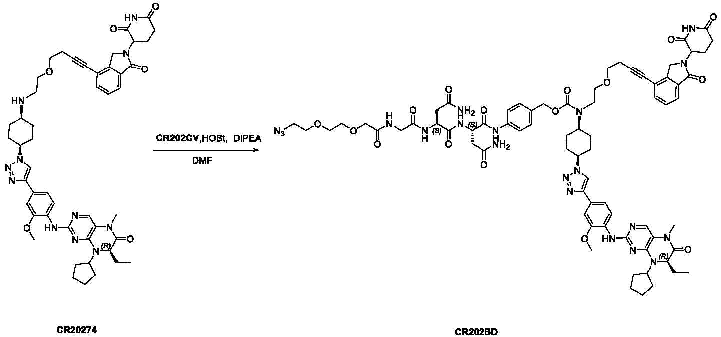

**CR20274** (51.2 mg, 56.6 µmol) and **CR202CV** (75.8 mg, 101.8 µmol) were added to DMF (1 mL) with magnetic stirring for dissolving. HOBT (1.5 mg, 11.3 µmol) and DIEA (15 µL, 84.8 µmol) were added, and the reaction system was stirred at 25 °C for 18 h in a N₂ atmosphere. The reaction was monitored by on-line HPLC until completion. The reaction mixture was purified by preparative liquid chromatography to give a white solid **CR202BD** (68.6 mg, 91.5% purity, 81.7% yield).

### Synthesis of CR202V9

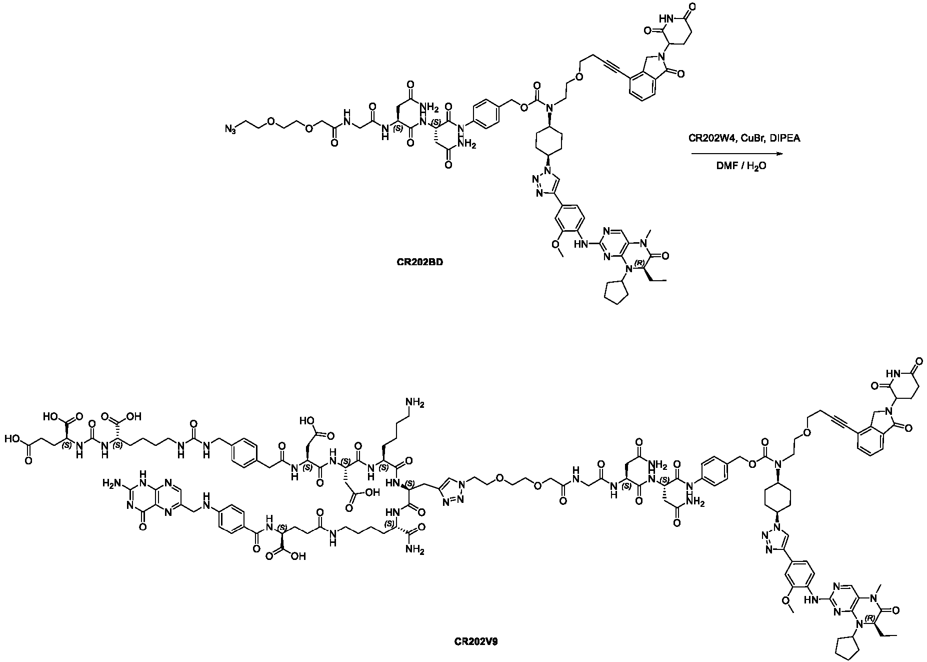

**CR202BD** (58.0 mg, 39.0 µmol) and **CR202W4** (70.8 mg, 46.7 µmol) were dissolved in DMF (3.6 mL) and water (1.2 mL) with magnetic stirring in a nitrogen atmosphere away from light. The reaction system was adjusted to pH 8 by adding DIEA before CuBr (5.6 mg, 39.0 µmol) was added. The reaction system was then incubated at 25 °C for 2 h, until on-line detection indicated the completion of the reaction. The reaction mixture was purified by preparative liquid chromatography to give **CR202V9** (37.4 mg, 94.90% purity, 31.6% yield). [M+4]⁴⁺/4=751.89.

### 4.12 Synthesis of CR202AL

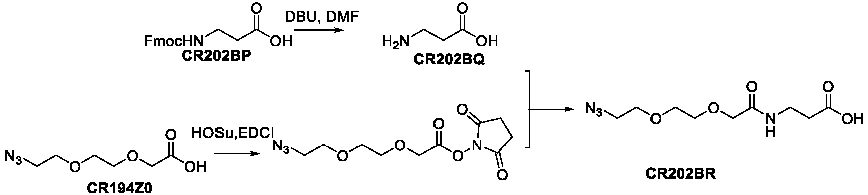

Synthesis of activated **CR202Z0** ester: **CR202Z0** (3.00 g, 15.9 mmol) was added to DCM (30 mL), and in a nitrogen atmosphere, EDCI (4.57 g, 23.8 mmol) and HOSu (2.74 g, 23.8 mmol) were sequentially added in portions at an external temperature of 0 °C. After the addition, the mixture was naturally warmed to room temperature and incubated at room temperature for 2 h. The reaction mixture was poured into ice water (100 mL) and extracted with DCM (50 mL × 2). The organic phases were combined, dried over anhydrous sodium sulfate, and concentrated to give 4.6 g of a yellow oil.

Synthesis of **CR202BQ: CR202BP** (5.90 g, 19.1 mmol) was added to DMF (30 mL) with magnetic stirring for dissolving. DBU (2.40 g, 15.9 mmol) was added, and the reaction system was incubated at room temperature for 3 h before DIPEA (4.5 mL) was added. Activated **CR202Z0** ester (4.6 g) was added dropwise to the reaction system, and the reaction system was incubated at room temperature for 2 h. The reaction mixture was poured into ice water (250 mL) and extracted with n-heptane (100 mL × 2). The aqueous phase was adjusted to pH 2 with 6 N hydrochloric acid and extracted with DCM (100 mL × 2). The organic phases were combined and dried over anhydrous sodium sulfate to give a yellow oil **CR202BR** (2.30 g, 56.1% yield). ¹H-NMR (400 MHz, CDCl₃) δ 7.36 (s, 1H), 4.06 (s, 2H), 3.82-3.67 (m, 6H), 3.62 (dd, *J* = 12.1, 6.0 Hz, 2H), 3.52-3.41 (m, 2H), 2.67 (t, *J* = 6.0 Hz, 2H). MS: [M+H]⁺=261.4.

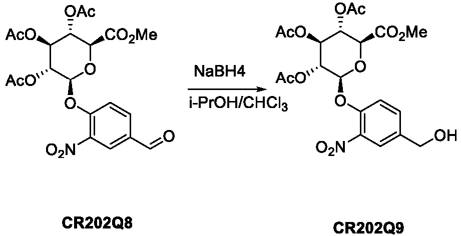

**CR202Q8** (3.30 g, 6.8 mmol) was added to isopropanol (15 mL) and chloroform (55 mL). In a nitrogen atmosphere at an external temperature of 0 °C, sodium borohydride (389.1 mg, 10.2 mmol) was added in portions, and the reaction system was incubated for 3.5 h, until TLC (n-heptane:EA = 1:1, Rf = 0.5) monitoring indicated the completion of the reaction. The reaction mixture was poured into ice water (150 mL) before 6 N hydrochloric acid (2 mL) was added dropwise. The mixture was extracted with DCM (100 mL × 2), and the organic phases were combined, dried over anhydrous sodium sulfate, and concentrated to give 4.6 g of crude product. A mixture of EA:n-heptane (7.5 mL:7.5 mL) was added, and the mixture was triturated overnight at room temperature, filtered, rinsed with a mixture of EA:n-heptane (1 mL:1 mL), and dried *in vacuo* to give an off-white solid **CR202Q9** (3.60 g, 83.7% yield). ¹H-NMR (400 MHz, CDCl₃) δ 7.86 (d, *J* = 2.0 Hz, 1H), 7.58 (dd, *J* = 8.5, 2.1 Hz, 1H), 7.40 (d, *J* = 8.5 Hz, 1H), 5.43-5.30 (m, 3H), 5.23 (d, *J = 6.8* Hz, 1H), 4.77 (s, 2H), 4.24 (d, *J =* 8.9 Hz, 1H), 3.79 (s, 3H), 2.17 (s, 3H), 2.10 (d, *J* = 3.4 Hz, 6H). MS: [M+H]⁺=475.2.

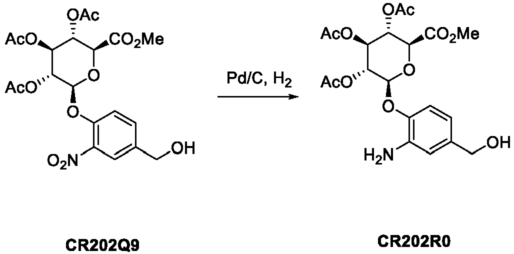

**CR202Q9** (2.85 g, 5.9 mmol) was added to methanol (100 mL) before 10% Pd/C (0.30 g, water content: 50%) was added. The system was purged with hydrogen thrice and hydrogenated at room temperature for 4 h at normal pressure, until TLC monitoring indicated the completion of the reaction. The reaction mixture was filtered, concentrated, rinsed with a mixture of EA:n-heptane (2 mL:4 mL), triturated at room temperature for 16 h, filtered, rinsed with a mixture of EA:n-heptane (1:2, 2 mL × 2), and dried *in vacuo* to give an off-white solid **CR202R0** (2.60 g, 91.63% purity, 87.8% yield). ¹H-NMR (400 MHz, CDCl₃) δ 6.94 (d, *J* = 8.2 Hz, 1H), 6.77 (d, *J =* 1.7 Hz, 1H), 6.70 (dd, *J* = 8.2, 1.7 Hz, 1H), 5.42-5.31 (m, 3H), 5.06 (d, *J* = 7.3 Hz, 1H), 4.59 (s, 2H), 4.20 (d, *J* = 9.3 Hz, 1H), 3.80 (s, 3H), 2.14-2.08 (m, 9H). MS: [M+H]⁺=456.3.

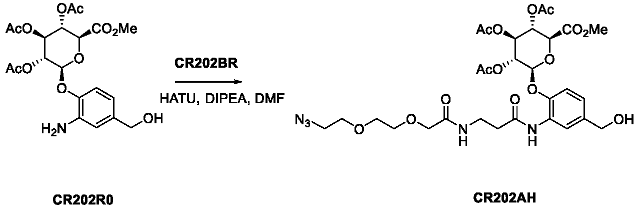

**CR202BR** (1.82 g, 6.9 mmol) was added to DMF (24 mL). In a nitrogen atmosphere at an external temperature of 0 °C, DIPEA (1.38 g, 10.6 mmol), HATU (2.62 g, 6.9 mmol), and **CR202R0** (2.40 g, 5.3 mmol) were added sequentially. After the addition, the reaction system was naturally warmed to room temperature (16.0 °C) and incubated at room temperature for 2.5 h, until on-line HPLC monitoring indicated the completion of the reaction. The reaction mixture was poured into ice water (200 mL) and extracted with EA (100 mL × 3). The organic phases were combined, washed with saturated brine (100 mL × 2), dried over anhydrous sodium sulfate, and concentrated to give 5.4 g of a crude product. The crude product was purified by preparative chromatography to give a foamy solid **CR202AH** (2.18 g, 97.52% purity, 54.8% yield). ¹H-NMR (400 MHz, CDCl₃) δ 8.43 (s, 1H), 8.08 (s, 1H), 7.47 (s, 1H), 7.10 (d, *J* = 7.0 Hz, 1H), 6.97 (d, *J =* 8.3 Hz, 1H), 5.45 (t, *J* = 9.5 Hz, 1H), 5.35 (dt, *J* = 14.0, 7.7 Hz, 2H), 5.09 (d, *J* = 7.6 Hz, 1H), 4.68 (s, 2H), 4.23 (d, *J* = 9.7 Hz, 1H), 4.06 (s, 2H), 3.80 (s, 3H), 3.71 (d, *J* = 5.1 Hz, 8H), 3.44 (t, *J* = 4.9 Hz, 2H), 2.76 (t, *J* = 5.6 Hz, 2H), 2.18-2.04 (m, 9H). MS: [M+H]⁺=698.3.

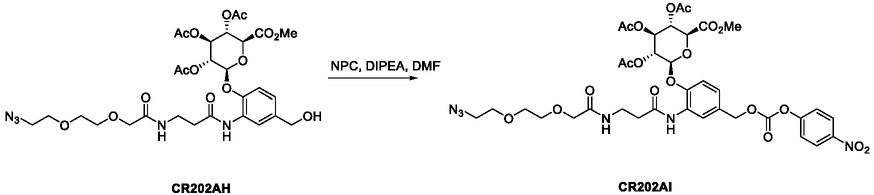

**CR202AH** (1.10 g, 1.58 mmol) was dissolved in DMF (10.0 mL). In a nitrogen atmosphere, di-p-nitrophenol carbonate (960.0 mg, 3.15 mmol) and DIPEA (306.0 mg, 2.37 mmol) were added sequentially. The reaction system was incubated at room temperature for 3 h, until on-line HPLC monitoring indicated the completion of the reaction. The reaction mixture was purified by preparative chromatography to give a white solid **CR202AI** (1.10 g, 99.85% purity, 80.9% yield). ¹H-NMR (400 MHz, CDCl₃) δ 8.56 (d, *J* = 1.9 Hz, 1H), 8.35-8.28 (m, 2H), 8.13 (s, 1H), 7.57 (s, 1H), 7.45-7.39 (m, 2H), 7.17 (dd, *J* = 8.4, 2.0 Hz, 1H), 7.01 (d, *J* = 8.4 Hz, 1H), 5.47 (t, *J* = 9.5 Hz, 1H), 5.39-5.31 (m, 2H), 5.27 (s, 2H), 5.13 (d, J= 7.6 Hz, 1H), 4.25 (d, *J* = 9.7 Hz, 1H), 4.10 (s, 2H), 3.80 (s, 3H), 3.75-3.68 (m, 8H), 3.47-3.41 (m, 2H), 2.79 (t, *J* = 6.1 Hz, 2H), 2.14-2.08 (m, 9H). MS: [M+H]⁺=863.3.

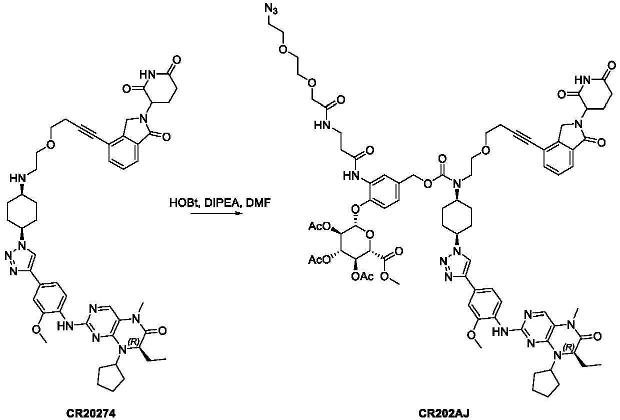

Free **CR20274:** CR20274 hydrochloride (2.8 g, 2.93 mmol, 1.0 eq.) was dissolved in DCM (100.0 mL) and methanol (20.0 mL). The reaction system was cooled to 5 to 10 °C. A proper amount of NaHCO₃ (2.0 g, 23.8 mmol) was dissolved in water (35 mL) and the mixture was added dropwise to the solution of CR20274 in DCM/MeOH. The mixture was stirred at 5 to 10 °C for 3 min and the phases were separated. The aqueous phase was extracted with DCM/MeOH (10/1, 50 mL × 2). The organic phases were combined, dried over anhydrous sodium sulfate, and filtered. The filtrate was concentrated *in vacuo* at 30 °C and dried to give a pale yellow solid free **CR20274** (2.5 g, 98.02% purity, 97% yield), which was used directly in the subsequent steps.

Free **CR20274** (150.0 mg, 0.17 mmol), **CR202AI** (292.8 mg,0.34 mmol), HOBt (4.6 mg, 0.034 mmol), and DIPEA (32.9 mg, 0.25 mmol) were sequentially added into DMF (4.0 mL), and the reaction system was stirred with a magnetic stirrer for dissolving and incubated at 25 °C for 20 h in a nitrogen atmosphere. DIPEA (8 µL, 0.045 mmol) was added, and the reaction system was further incubated for 24 h, until on-line HPLC monitoring indicated the completion of the reaction. The reaction mixture was directly purified by preparative liquid chromatography to give **CR202AJ** (218.1 mg, 99.56% purity, 54.5% yield).

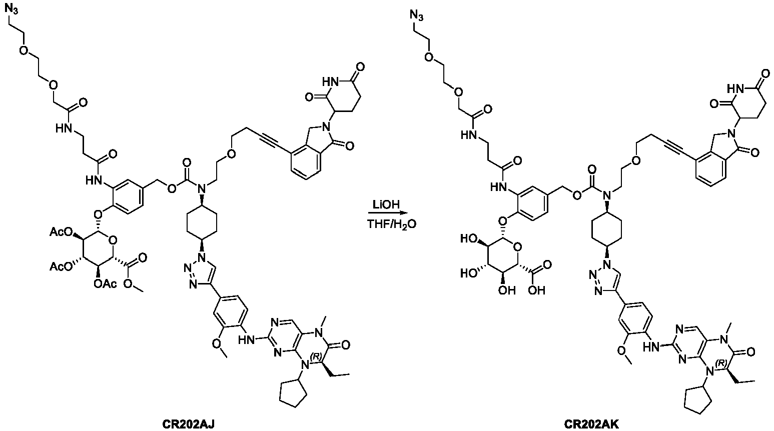

**CR202AJ** (210.0 mg, 130.0 µmol) was added to THF (21 mL) for dissolving. A 0.1 M aqueous LiOH solution (7.2 mL, 718.0 µmol) was added dropwise at an external temperature of 0 to 5 °C, and the reaction system was incubated for 1 h. The reaction mixture was adjusted to pH 6 to 7 by dropwise adding a 5% TFA aqueous solution and concentrated at reduced pressure to remove THF. The residue was purified by preparative liquid chromatography to give **CR202AK** (35.0 mg, 94.84% purity, 17.8% yield).

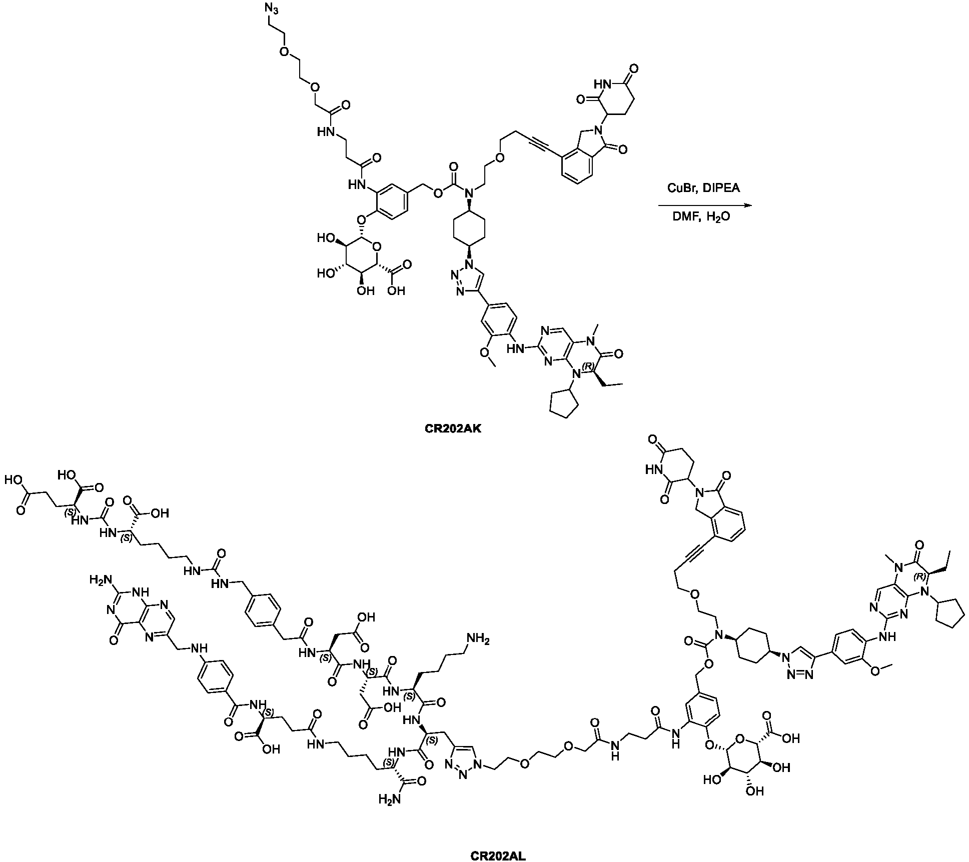

In a 25-mL jacketed flask, **CR202AK** (85.0 mg, 57.8 µmol, 1.0 eq.) and **CR202W4** (104.9 mg, 69.4 µmol, 1.2 eq.) were dissolved in DMF (6.4 mL) and water (2.5 mL) with magnetic stirring in a nitrogen atmosphere away from light. The reaction system was adjusted to pH 8 by adding DIEA before CuBr (8.3 mg, 57.8 µmol, 1.0 eq.) was added. The reaction system was then incubated at 25 °C for 2 h, until on-line detection indicated the completion of the reaction. The reaction mixture was purified by preparative liquid chromatography and desalted in purified water to give **CR202AL** (23.7 mg, 94.48% purity, 13.9% yield). [M+4H]⁴⁺/4=746.40.

### 4.13 Synthesis of CR202W1

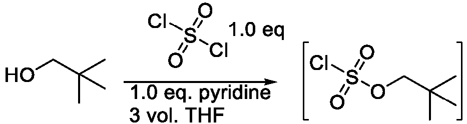

Sulfonyl chloride (9.65 g, 71.5 mmol, 1.0 eq.) was diluted with anhydrous THF (15 mL) in a 100-mL three-necked flask. The reaction system was stirred with a magnetic stirrer in a nitrogen atmosphere and cooled to -70 °C. Neopentyl alcohol (6.3 g, 71.5 mmol) and pyridine (5.66 g, 71.5 mmol) were dissolved in anhydrous THF (30 mL), and the mixture was dropwise and slowly added over 1 h into the three-necked flask. After the addition, the reaction system was naturally warmed to room temperature and stirred for 2 h. The solid was removed by filtration to give **neopentyl sulfurochloridate.**

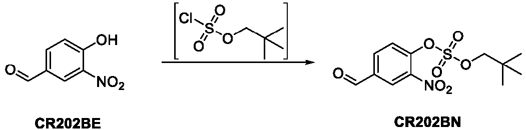

In a 250-mL three-necked flask, **CR202BE** (6 g, 35.9 mmol), TEA (7.27 g, 71.8 mmol), and DMAP (4.4 g, 36.0 mmol) were dissolved in THF (120 mL), and the mixture was cooled to 0 °C in a nitrogen atmosphere. A solution of *Neopentyl sulfurochloridate* in THF (71.5 mmol) was added dropwise. The reaction system was stirred at 0 °C for 1 h and the reaction was quenched by adding 1 M HCl (150 mL) and ethyl acetate (150 mL). The phases were separated, and the organic phase was washed sequentially with saturated NaHCO₃ (100 mL) and 10% Na₂SO₄ (100 mL), dried over anhydrous Na₂SO₄, and filtered. The filtrate was concentrated and subjected to silica gel column chromatography (n-heptane/ethyl acetate = 10/1 to 5/1) to give a pale yellow solid **CR202BN** (6.4 g, 98.49% purity, 50% yield). H-NMR (400 MHz, DMSO-*d*₆) δ 10.09 (s, 1H), 8.76 (d, *J* = 2.0 Hz, 1H), 8.39 (dd, *J* = 8.4 Hz, 2.0 Hz, 1H), 7.96 (d, *J =* 8.4 Hz, 1H), 4.36 (s, 2H), 0.97 (s, 9H).

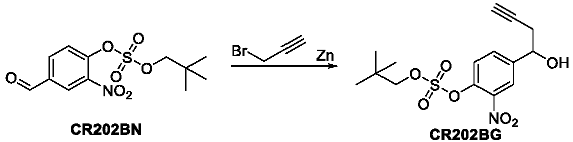

In a 100-mL jacketed flask, **CR202BN** (3 g, 9.45 mmol) and bromopropyne (1.69 g, 14.2 mmol) were dissolved in THF (40 mL), and in a nitrogen atmosphere, the mixture was cooled to - 10 °C before a zinc powder (927 mg, 14.2 mmol) was added. The condensation system was turned off, and the reaction system was naturally warmed to room temperature (10 to 20 °C) and stirred overnight, until on-line detection indicated the completion of the reaction. The reaction was quenched with saturated NH₄Cl (200 mL), and the reaction mixture was extracted with ethyl acetate (150 mL × 2). The organic phases were combined, washed sequentially with water (150 mL) and 10% Na₂SO₄ (150 mL), dried over anhydrous Na₂SO₄, and filtered. The filtrate was concentrated and subjected to column chromatography (n-heptane/ethyl acetate = 6/1 to 4/1) to give a yellow oil **CR202BG** (5.0 g, 94.86% purity, 64% yield). H-NMR (400 MHz, CDCl₃) δ 8.09 (d, *J =* 2.0 Hz, 1H), 7.71 (dd, *J* = 8.4 Hz, 2.0 Hz, 1H), 7.61 (d, *J =* 8.4 Hz, 1H), 4.97 (t, *J =* 6.4 Hz, 1H), 4.22 (s, 2H), 2.73-2.60 (m, 2H), 2.13 (t, J = 6.4 Hz, 1H), 1.02 (s, 9H).

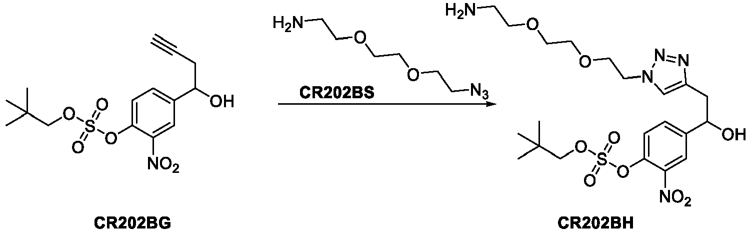

**CR202BG** (5 g, 14.0 mmol) and **CR202BS** (2.44 g, 14.0 mmol) were dissolved in DMF (50 mL) and water (15 mL) in a nitrogen atmosphere. The reaction system was adjusted to pH 8 by adding DIEA (2.3 mL). CuBr (2.0 g, 13.9 mmol) was added and the reaction system was stirred at 25 °C for 1 h, until on-line detection indicated the completion of the reaction. The reaction mixture was purified by preparative liquid chromatography to give a colorless oil **CR202BH** (7.2 g, 86.69% purity, 59% yield). LCMS: [M+H]+ = 532.3.

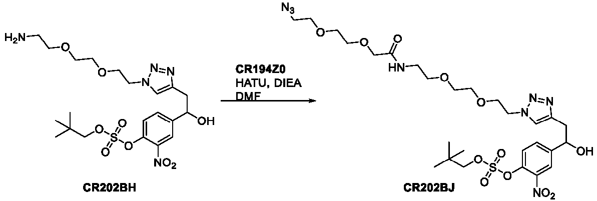

**CR202BH** (7.2 g, 9.48 mmol), **CR194Z0** (1.8 g, 9.52 mmol), and DIEA (3.65 g, 28.2 mmol) were dissolved in DMF (50 mL) in a nitrogen atmosphere. HATU (3.6 g, 9.47 mmol) was added. The reaction system was stirred at 14 °C for 2 h, until on-line detection indicated the completion of the reaction. Water (150 mL) was added, and the mixture was extracted with ethyl acetate (100 mL × 2). The organic phases were combined, washed sequentially with water (100 mL) and 10% Na₂SO₄ (100 mL), dried over anhydrous Na₂SO₄, and filtered. The filtrate was concentrated and purified by column chromatography (DCM/MeOH = 30/1 to 20/1) to give a yellow oil **CR202BJ** (6.4 g, 94.11% purity, 90% yield). [M+H]⁺ = 703.4 (4.43 min). H-NMR (400 MHz, DMSO-*d*₆) δ 8.09 (d, *J* = 2.0 Hz, 1H), 7.80 - 7.78 (m, 2H), 7.64 (d, *J =* 8.4 Hz, 1H), 7.61 (t, *J =* 5.6 Hz, 1H), 5.00 (t, *J =* 5.6 Hz, 1H), 4.46 (t, *J =* 5.6 Hz, 2H), 4.29 (s, 2H), 3.88 (s, 2H), 3.76 (t, *J* = 5.2 Hz, 2H), 3.62 - 3.59 (m, 6H), 3.51 - 3.39 (m, 9H), 3.26 (q, *J* = 6.0 Hz, 2H), 3.02 (d, *J* = 6.0 Hz, 2H), 0.96 (s, 9H).

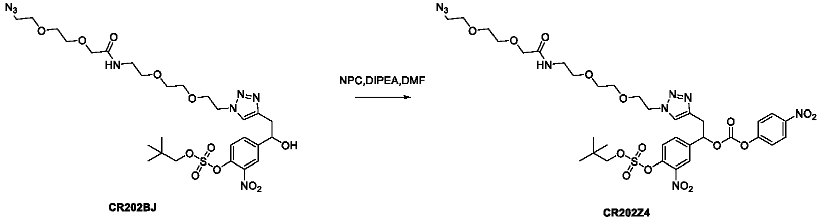

**CR202BJ** (6.4 g, 9.11 mmol), NPC (5.5 g, 18.1 mmol), and DIEA (1.77 g, 13.7 mmol) were dissolved in DMF (30 mL) in a nitrogen atmosphere. The reaction system was stirred at 25 °C for 1.5 h, until on-line detection indicated the completion of the reaction. The reaction mixture was purified by preparative liquid chromatography to give a colorless gum **CR202Z4** (6.0 g, 97.24% purity, 72% yield). [M+H]⁺ = 868.3. H-NMR (400 MHz, DMSO-*d*₆) δ 8.30 (d, *J* = 9.2 Hz, 2H), 8.25 (d, *J =* 2.0 Hz, 1H), 7.94 (dd, *J* = 8.4 Hz, 2.0 Hz, 1H), 7.86 (s, 1H), 7.74 (d, *J* = 8.8 Hz, 1H), 7.60 (t, *J* = 5.6 Hz, 1H), 7.52 (d, *J* = 9.2 Hz, 2H), 6.17 (t, *J* = 6.4 Hz, 1H), 4.48 (t, *J* = 4.8 Hz, 2H), 4.31 (s, 2H), 3.87 (s, 2H), 3.76 (t, *J =* 4.8 Hz, 2H), 3.61 - 3.58 (m, 6H), 3.46 - 3.36 (m, 10H), 3.24 (q, *J =* 6.0 Hz, 2H), 0.95 (s, 9H).

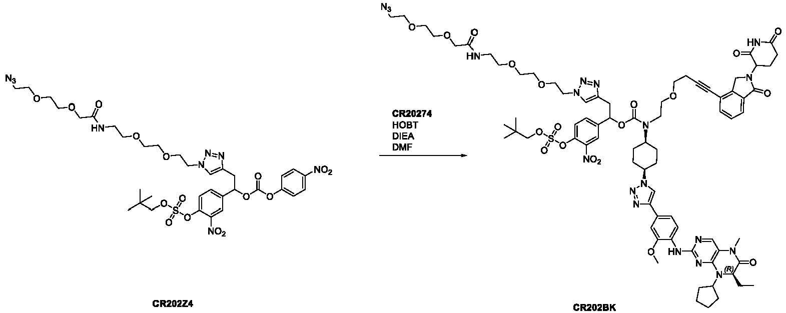

**CR20274** (100 mg, 113 µmol.), **CR202Z4** (196 mg, 226 µmol), HOBT (3 mg, 22.2 µmol), and DIEA (29.2 mg, 226 µmol) were dissolved in DMF (1.5 mL) in a nitrogen atmosphere. The reaction system was stirred at 25 °C for 21 h, until on-line detection indicated the completion of the reaction. The reaction mixture was purified by preparative liquid chromatography to give **CR202BK** (82 mg, 95.21% purity, 53% yield). LCMS: [M/2+H]⁺ = 806.9 (RT=5.45 min).

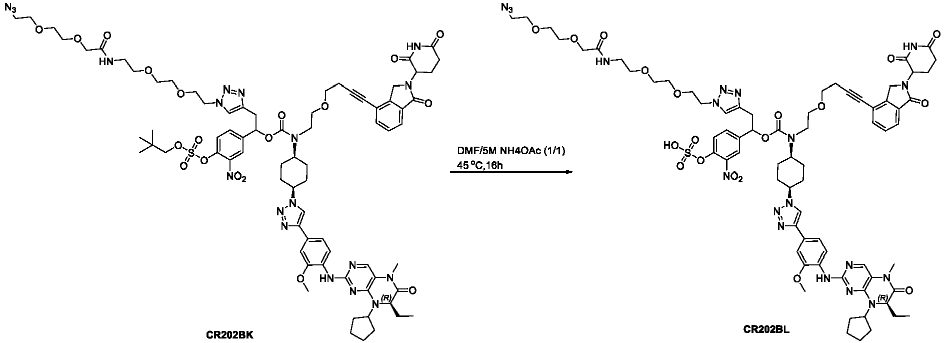

**CR202BK** (117 mg, 101 µmol.) was dissolved in DMF (3 mL) before a 5 M aqueous NH₄OAc solution (3 mL) was added in a nitrogen atmosphere. The reaction system was stirred at 45 °C for 17 h, until on-line detection indicated the completion of the reaction. The reaction mixture was purified by preparative liquid chromatography to give **CR202BL** (78 mg, 99.54% purity, 70% yield). LCMS: [M-H]⁻ = 1540.8.

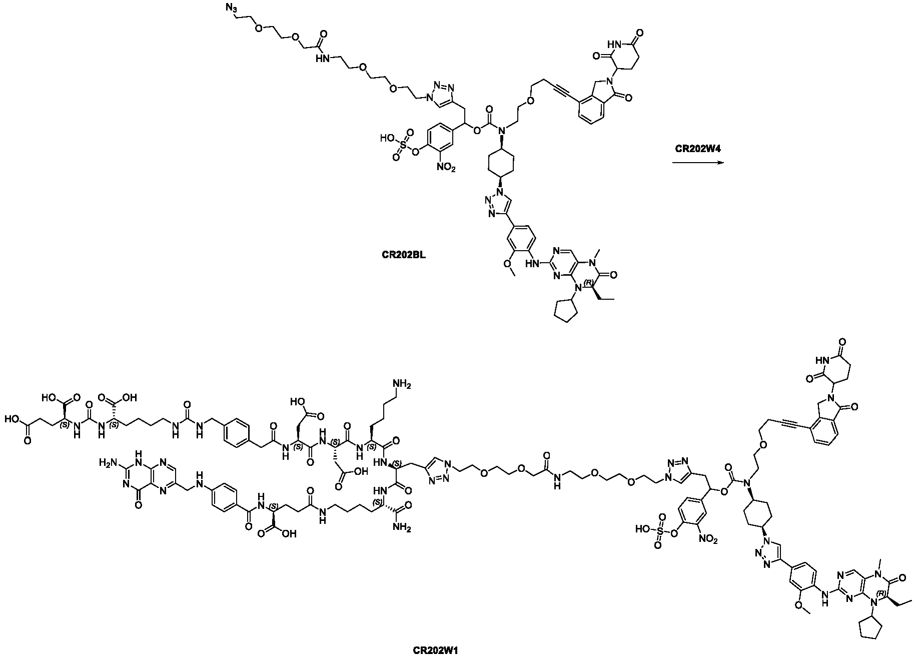

**CR202BL** (70 mg, 45.4 µmol) and **CR202W4** (83 mg, 54.8 µmol) were dissolved in DMF (6 mL) and water (2 mL) with magnetic stirring in a nitrogen atmosphere away from light. The reaction system was adjusted to pH 8 by adding DIEA (250 µL) before CuBr (6.5 mg, 45.4 µmol) was added. The reaction system was then incubated at 25 °C for 1 h, before **CR202W4** (18 mg, 11.9 µmol), DIEA (100 µL), and CuBr (3.2 mg, 22.7 µmol) were added. The reaction system was incubated at 25 °C for another 1 h, until on-line detection indicated the completion of the reaction. The reaction mixture was purified by preparative liquid chromatography and desalted in purified water to give **CR202W1** (33 mg, 94.46% purity, 19% yield). LCMS: [M+4H]⁴⁺/4 = 764.94.

### 4.14 Synthesis of CR202GM

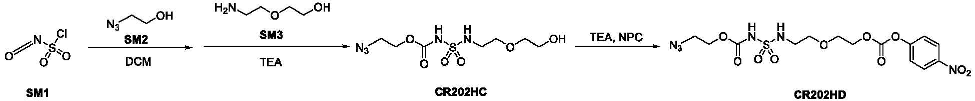

**SM1** (500 mg, 3.53 mmol, 1.0 eq.) was dissolved in DCM (50.0 mL), and the reaction system was cooled to 0 °C in a nitrogen atmosphere. **SM2** (307 mg, 3.53 mmol, 1.0 eq.) was added, and the reaction system was stirred at 0 °C for 30 min. TEA (714 mg, 7.06 mmol, 2.0 eq.) and **SM3** (371 mg, 3.53 mmol, 1.0 eq.) were added, and the reaction system was stirred at 5 °C for 1 h. The reaction was monitored by on-line HPLC until completion. NPC (2.15 g, 7.07 mmol, 2.0 eq.) and TEA (358 mg, 3.54 mmol, 1.0 eq.) were added, and the reaction system was stirred at 25 °C for 3 h. The reaction mixture was concentrated and subjected to silica gel column chromatography (n-heptane/ethyl acetate/acetic acid = 50/50/1) to give a colorless viscous oil **CR202HD** (1.2 g, 98.47% purity, 74% yield). LCMS: [M+H]⁺ = 463.4. ¹H-NMR (400 MHz, CDCl₃) *δ* 8.30 - 8.26 (m, 2H), 8.02 (s, 1H), 7.43 - 7.40 (m, 2H), 5.72 (t, J = 6.0 Hz, 1H), 4.46 - 4.44 (m, 2H), 4.33 (t, *J* = 4.8 Hz, 2H), 3.79 - 3.77 (m, 2H), 3.67 (t, *J* = 4.8 Hz, 2H), 3.53 (t, *J* = 4.8 Hz, 2H), 3.35 (q, *J* = 4.8 Hz, 2H).

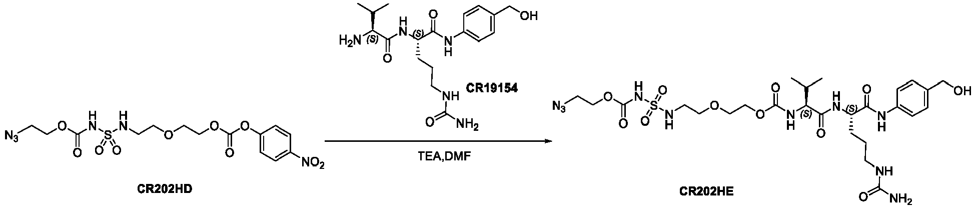

**CR202HD** (402 mg, 0.869 mmol, 1.1 eq.) was dissolved in DMF (3 mL) before **CR19154** (300 mg, 0.791 mmol, 1.0 eq.) and TEA (240 mg, 2.37 mmol, 3.0 eq.) were added. The reaction system was stirred at 25 °C for 17 h. The reaction was monitored by on-line HPLC until completion. The reaction mixture was concentrated and purified by column chromatography (DCM/MeOH/AcOH = 100/10/1) to give a white foamy solid **CR202HE** (560 mg, 100% purity, 90% yield). LCMS: [M+H]⁺ = 703.4. ¹H-NMR (DMSO-d6, 400 MHz): δ 9.92 (s, 1H), 8.04 (d, J = 7.6 Hz, 1H), 7.54 (d, J = 8.4 Hz, 2H), 7.28 (d, J = 8.4 Hz, 1H), 7.22 (d, J = 8.8 Hz, 2H), 5.98 (t, J = 5.6 Hz, 1H), 5.40 (s, 2H), 5.08 (s, 1H), 4.42 - 4.37 (m, 3H), 4.07 - 4.03 (m, 4H), 3.88 (dd, J = 8.4 Hz, 6.8 Hz, 1H), 3.56 - 3.54 (m, 2H), 3.48 - 3.46 (m, 4H), 3.01 - 2.93 (m, 4H), 2.01 - 1.92 (m, 1H), 1.74 - 1.30 (m, 4H), 0.87 (d, J = 6.8 Hz, 3H), 0.83 (d, J = 6.8 Hz, 3H).

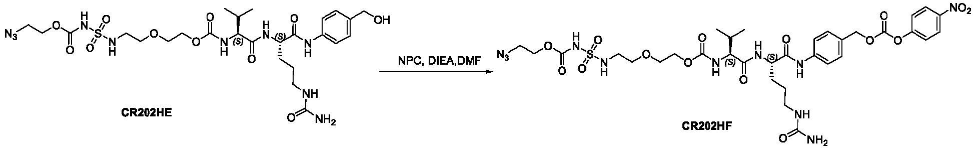

**CR202HE** (460 mg, 1.21 mmol, 1.0 eq.) was dissolved in DMF (6 mL) before NPC (405 mg, 1.33 mmol, 1.1 eq.) and DIEA (235 mg, 1.82 mmol, 1.5 eq.) were added. The reaction system was stirred at 25 °C for 17 h. The reaction was monitored by on-line HPLC until completion. The reaction mixture was directly purified by reverse-phase preparative liquid chromatography to give a white solid **CR202HF** (230 mg, 98.59% purity, 37% yield). LCMS: [M+H]⁺ = 868.3. ¹H-NMR (DMSO-d6, 400 MHz): δ 11.37 (s, 1H), 10.14 (s, 1H), 8.36 - 8.32 (m, 2H), 8.13 (d, J = 7.6 Hz, 1H), 7.84 (t, J = 5.6 Hz, 1H), 7.68 (d, J = 8.4 Hz, 2H), 7.61 - 7.58 (m, 2H), 7.44 (d, J = 8.4 Hz, 2H), 7.24 (d, J = 8.8 Hz, 1H), 6.03 (s, 1H), 5.27 (s, 2H), 4.47 - 4.42 (m, 1H), 4.25 (t, J = 4.8 Hz, 2H), 4.14 - 4.05 (m, 2H), 3.95 - 3.91 (m, 1H), 3.61 - 3.59 (m, 4H), 3.52 - 3.51 (m, 2H), 3.13 - 2.96 (m, 4H), 2.04 - 1.96 (m, 1H), 1.78 - 1.58 (m, 2H), 1.54 - 1.34 (m, 2H), 0.90 (d, J = 6.8 Hz, 3H), 0.86 (d, J = 6.8 Hz, 3H).

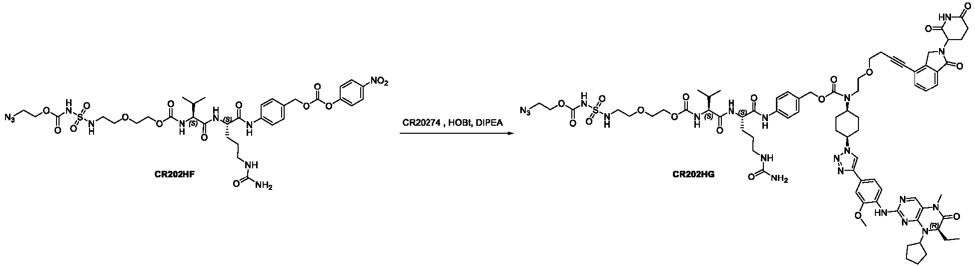

**CR202HF** (200.0 mg, 0.23 mmol), **CR20274** (free form, 220.0 mg, 0.23 mmol), HOBt (6.4 mg, 0.05 mmol), and DIPEA (89.0 mg, 0.69 mmol) were sequentially added into DMF (4.0 mL), and the reaction system was incubated at room temperature (26.0 °C) for 16 h in a nitrogen atmosphere, until on-line HPLC monitoring indicated the completion of the reaction. The reaction mixture was purified by preparative HPLC (mobile phases: 0.1% TFA/H₂O and 0.1% TFA/ACN) and lyophilized to give **CR202HG** (130.0 mg, 97.81% purity, 35.0 % yield). LCMS: [M+H]⁺=1612.93.

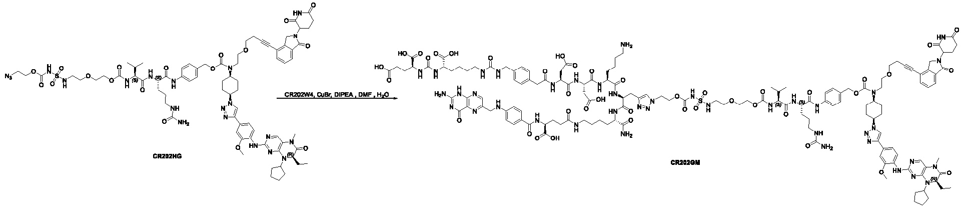

In a nitrogen atmosphere, **CR202HG** (120.0 mg, 0.074 mmol) and **CR202W4** (134.0 mg, 0.088 mmol) were added to a mixed solvent of DMF (6 mL) and water (2 mL). The reaction system was stirred away from light for dissolving and adjusted to pH 8 by dropwise adding DIPEA before CuBr (13.0 mg, 0.074 mmol) was added. The reaction system was incubated at room temperature (25.0 °C) for 2.5 h, until on-line HPLC monitoring indicated the completion of the reaction. The reaction mixture was directly purified by preparative HPLC (mobile phase: 0.1% TFA/H₂O and 0.1% TFA/ACN). The product purified by preparative HPLC was desalted with purified water and lyophilized to give **CR202GM** (52.1 mg, 95.83% purity, 22.4% yield). LCMS: [M+4H]⁴⁺=782.49.

### 4.15 Synthesis of CR202HJ

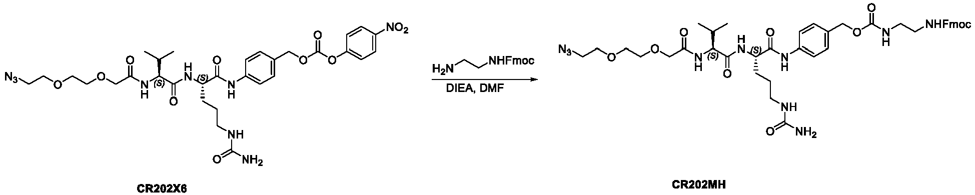

**CR202X6** (1.00 g, 1.39 mmol), Fmoc-ethylenediamine (0.39 g, 1.39 mmol), and DMF (10 mL) were sequentially added to a 100-mL single-neck flask, and the mixture was stirred until complete dissolution. DIEA (0.22 g, 1.67 mmol) was added, and the reaction system was incubated at 20.7 °C for 0.5 h, until on-line detection indicated the completion of the reaction. The reaction mixture was poured into pre-cooled MTBE (80 mL; 0 to 10 °C). The mixture was stirred at room temperature for 5 min and centrifuged, and the precipitated solid was washed with cold MTBE (8 mL × 5) to give a white solid **CR202MH** (1.12 g, 93.66% purity, 93.8% yield).

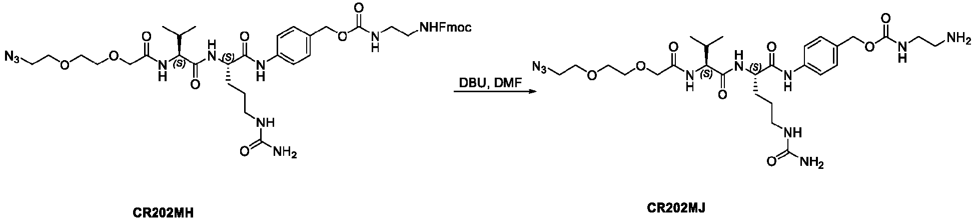

**CR202MH** (1.08 g, 1.26 mmol) was added into DMF (7 mL) before a solution of DBU (0.19 g, 1.26 mmol) in DMF (3 mL) was dropwise added. The reaction system was incubated at 22.2 °C for 0.5 h, until on-line detection indicated the completion of the reaction. The reaction mixture was purified by preparative HPLC (mobile phases: 0.1% TFA/H₂O and 0.1% TFA/ACN) and lyophilized to give **CR202MJ** (913.7 mg, 99.8% purity).

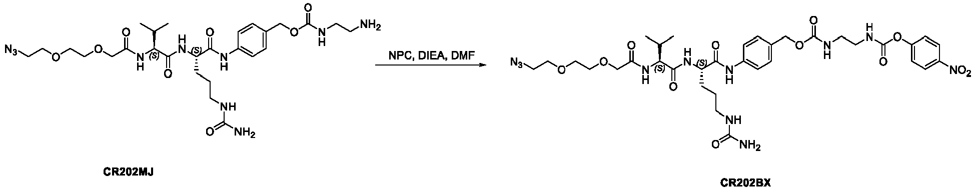

**CR202MJ** (0.83 g, 1.30 mmol) and NPC (0.79 g, 2.60 mmol) were added into DMF (8 mL) until complete dissolution, before DIEA (0.25 g, 1.95 mmol) was added. The reaction system was incubated at room temperature for 0.5 h, until on-line detection indicated the completion of the reaction. The reaction mixture was purified by preparative HPLC and lyophilized to give **CR202BX** (254.9 mg, 96.46% purity, 24.5% yield). LCMS: [M+1H]⁺=802.4. ¹H-NMR (400 MHz, DMSO) δ 10.06 (s, 1H), 8.36 (d, J = 7.4 Hz, 1H), 8.28 (d, J = 7.2 Hz, 2H), 8.10 (s, 1H), 7.61 (d, J = 8.5 Hz, 2H), 7.47 (d, J = 9.0 Hz, 1H), 7.43 (d, J = 9.1 Hz, 2H), 7.32 (d, J = 8.4 Hz, 2H), 6.01 (s, 1H), 5.43 (s, 1H), 4.99 (s, 2H), 4.42 (dd, J = 13.4, 7.8 Hz, 1H), 4.38-4.32 (m, 1H), 3.99 (s, 2H), 3.70-3.61 (m, 6H), 3.45-3.42 (m, 3H), 3.17 (s, 4H), 3.01 (dd, J = 22.5, 9.5 Hz, 2H), 2.07-1.97 (m, 1H), 1.77-1.56 (m, 2H), 1.53-1.32 (m, 2H), 0.90 (d, J = 6.8 Hz, 3H), 0.85 (d, J = 6.8 Hz, 3H).

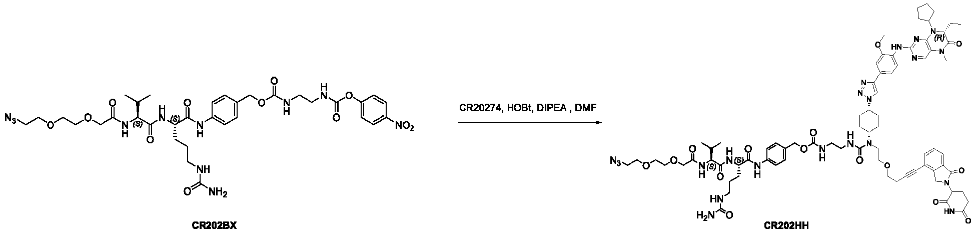

**CR202BX** (190.0 mg, 0.24 mmol), **CR20274** (free form, 251.4 mg, 0.28 mmol), HOBt (6.4 mg, 0.05 mmol), and DIPEA (91.9 mg, 0.71 mmol) were sequentially added into DMF (3.8 mL), and the reaction system was incubated at room temperature for 8 h in a nitrogen atmosphere. The reaction mixture was purified by preparative HPLC (mobile phases: 0.1% TFA/H₂O and 0.1% TFA/ACN) and lyophilized to give **CR202HH** (230.0 mg, 98.27% purity, 59.7% yield). LCMS: [M+H]⁺=1546.63.

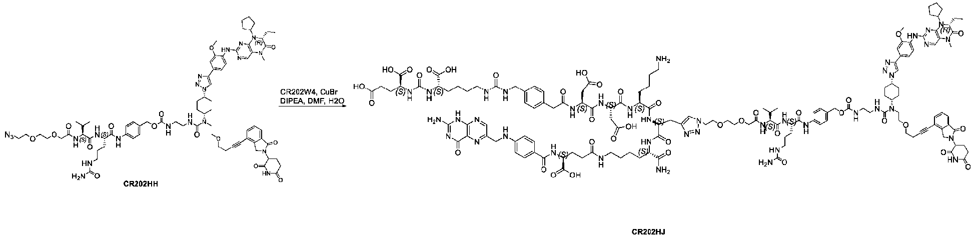

In a nitrogen atmosphere, **CR202HH** (210.0 mg, 0.14 mmol) and **CR202W4** (240.0 mg, 0.16 mmol) were added to a mixed solvent of DMF (12 mL) and water (4 mL). The reaction system was stirred away from light for dissolving and adjusted to pH 8 by dropwise adding DIPEA before CuBr (18.9 mg, 0.14 mmol) was added. The reaction system was incubated at room temperature (25.0 °C) for 5 h, until on-line detection indicated the completion of the reaction. The reaction mixture was directly purified by preparative HPLC (mobile phase: 0.1% TFA/H₂O and 0.1% TFA/ACN). The product purified by preparative HPLC was desalted with purified water and lyophilized to give **CR202HJ** (215.7 mg, 96.69% purity, 47.3% yield). LCMS: [M+3H]³⁺=1021.20.

### 4.16 Synthesis of CR202FU

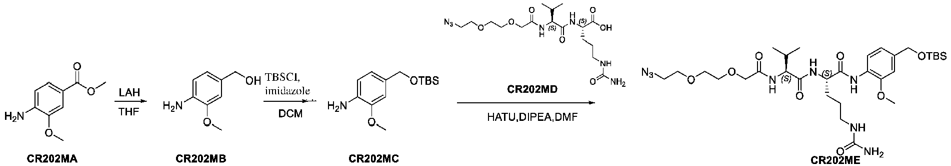

In a nitrogen atmosphere, lithium aluminum hydride (0.47 g, 12.24 mmol) was added to THF (3 mL), and the reaction mixture was cooled to 0 °C. A solution of **CR202MA** (1.50 g, 8.28 mmol) in THF (15 mL) was added dropwise, and the reaction system was incubated at 0 °C for 2 h, until on-line TLC monitoring indicated the completion of the reaction. The reaction mixture was poured into ice water (5 mL), before a 15% sodium hydroxide solution (1 mL) was added dropwise. The mixture was stirred at room temperature for 15 min and extracted with ethyl acetate (15 mL × 3). The organic phases were combined, dried over anhydrous sodium sulfate, and filtered. The filtrate was concentrated, and the crude product was purified by column chromatography (PE/EA = 1:1) to give **CR202MB** (1.15 g, 95.03% purity, 86.15% yield). LCMS: [M+H] ⁺=154.6.

In a nitrogen atmosphere, **CR202MB** (1.15 g, 7.50 mmol) was added to DCM (12 mL), before imidazole (1.53 g, 22.52 mmol) and TBSCl (1.69 g, 11.26 mmol) were sequentially added. The reaction system was incubated at room temperature for 2 h, until on-line TLC monitoring indicated the completion of the reaction. The reaction mixture was poured into ice water (5 mL) to quench the reaction. The mixture was extracted with DCM (15 mL × 3). The organic phases were combined, dried over anhydrous sodium sulfate, and filtered. The filtrate was concentrated, and the crude product was purified by column chromatography (DCM/MeOH = 10:1) to give **CR202MC** (1.50 g, 99.15% purity, 74.07% yield). ¹H-NMR (400 MHz, DMSO) δ 6.73 (s, 1H), 6.62 (d, J = 7.9 Hz, 1H), 6.57 (d, J = 7.9 Hz, 1H), 4.60 (s, 2H), 4.54 (s, 2H), 3.74 (s, 3H), 0.88 (s, 9H), 0.05 (s, 6H).

**CR202MD** (1.66 g, 3.73 mmol) was added to DMF (15 mL), before HATU (1.99 g, 5.22 mmol) was added at 25 °C. The reaction system was stirred for 15 min before **CR202MC** (1.50 g, 5.59 mmol) and DIEA (0.67 g, 5.22 mmol) were added. The reaction was monitored by on-line detection until completion. The reaction mixture was poured into ice water (75 mL) and extracted with DCM (30 mL × 3). The organic phases were combined, dried over anhydrous sodium sulfate and filtered. The filtrate was concentrated to give a crude product **CR202ME,** which was directly used in the next step. LCMS: [M+H]⁺ = 695.6.

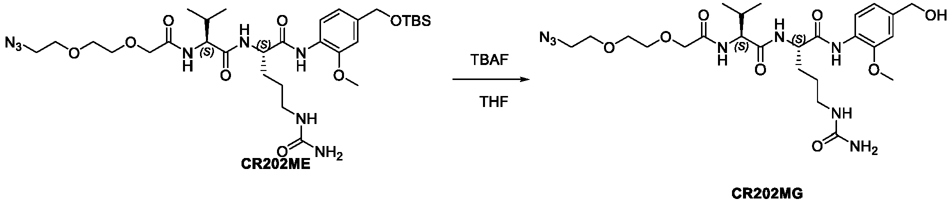

**CR202ME** (2.78 g, 3.73 mmol) was added to THF (30 mL), TBAF (2.09 g, 7.46 mmol) was added, the reaction system was incubated at 25 °C for 1 h, until on-line TLC monitoring indicated the completion of the reaction. The reaction mixture was concentrated, and the crude product **CR202MG** was used directly for the next reaction.

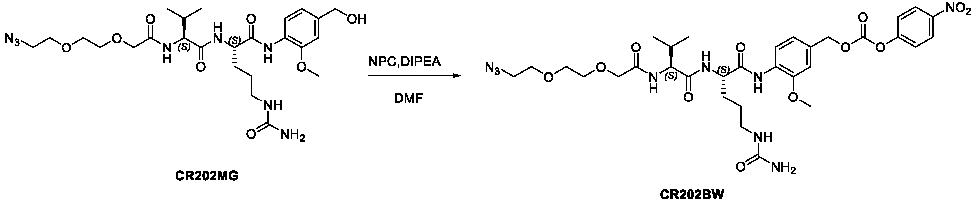

The crude product **CR202MG** was added to DMF (30 mL) for complete dissolution. Di(p-nitrophenol) carbonate (NPC, 2.26 g, 7.44 mmol) and DIPEA (0.72 g, 5.58 mmol) were added and the reaction system was incubated at 20 to 25 °C for 1 h, until on-line detection indicated the completion of the reaction. The reaction mixture was purified by preparative HPLC and lyophilized to give a white solid **CR202BW** (1.35 g, 98.5% purity, 48.7% yield). LCMS: [M+H]⁺ = 746.6; ¹H-NMR (400 MHz, DMSO) δ 9.17 (s, 1H), 8.46-8.48 (d, 1H), 8.31-8.33 (d, 1H), 8.05-8.07 (d, 1H), 7.57-7.59 (d, 1H), 7.46-7.49 (d, 1H), 7.17 (s, 1H), 7.02-7.07 (d, 1H), 6.00 (bs, 1H), 5.26 (s, 2H), 4.49-4.54 (m, 2H), 4.33-4.37 (dd, 2H), 3.97 (s, 2H), 3.86 (s, 3H), 3.62-3.65 (m, 6H), 3.40-3.43(t, 2H), 2.97-3.00 (t, 2H), 2.01-2.03(m, 1H), 1.59-1.61 (m, 2H), 1.42-1.45 (m, 2H), 0.88-0.90 (d, 3H), 0.82-0.84 (d, 3H).

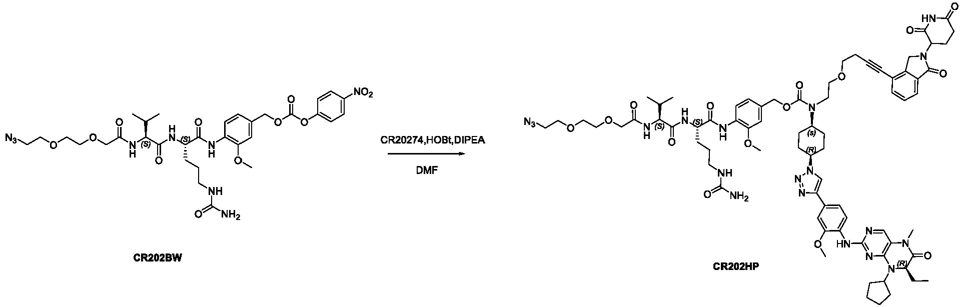

In a nitrogen atmosphere, **CR202BW** (250.1 mg, 0.34 mmol), **CR20274** (free form, 326.0 mg, 0.37 mmol), HOBt (9.0 mg, 0.07 mmol), and DIPEA (140.0 mg, 1.02 mmol) were sequentially added into DMF (5.0 mL), and the reaction system was incubated at room temperature for 16 h. The reaction mixture was purified by preparative HPLC (mobile phases: 0.1% TFA/H₂O and 0.1% TFA/ACN) and lyophilized to give **CR202HP** (236.2 mg, 96.14% purity, 47.3% yield). LCMS: [M+H]⁺=1490.96.

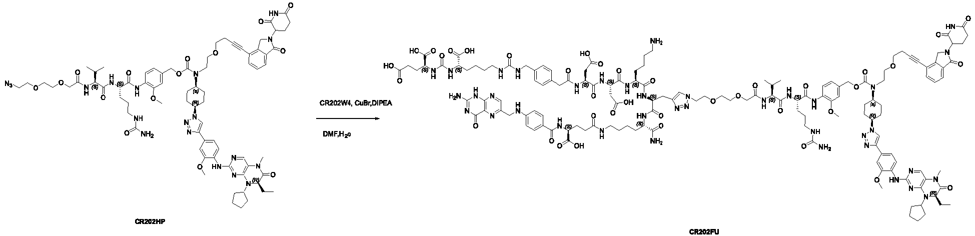

In a nitrogen atmosphere, **CR202HP** (211.9 mg, 0.15 mmol) and **CR202W4** (268.0 mg, 0.148 mmol) were added to a mixed solvent of DMF (12 mL) and water (4 mL). The reaction system was stirred away from light for dissolving and adjusted to pH 8 by dropwise adding DIPEA before CuBr (6.4 mg, 0.05 mmol) was added. The reaction system was incubated at room temperature (23.7 °C) for 5 h. The reaction mixture was directly purified by preparative HPLC (mobile phase: 0.1% TFA/H₂O and 0.1% TFA/ACN). The product purified by preparative HPLC was desalted with purified water and lyophilized to give **CR202FU** (132.4 mg, 98.67% purity, 28.5% yield). LCMS: [M₊4H]⁴⁺=751.89.

### 4.17 Synthesis of CR202GQ

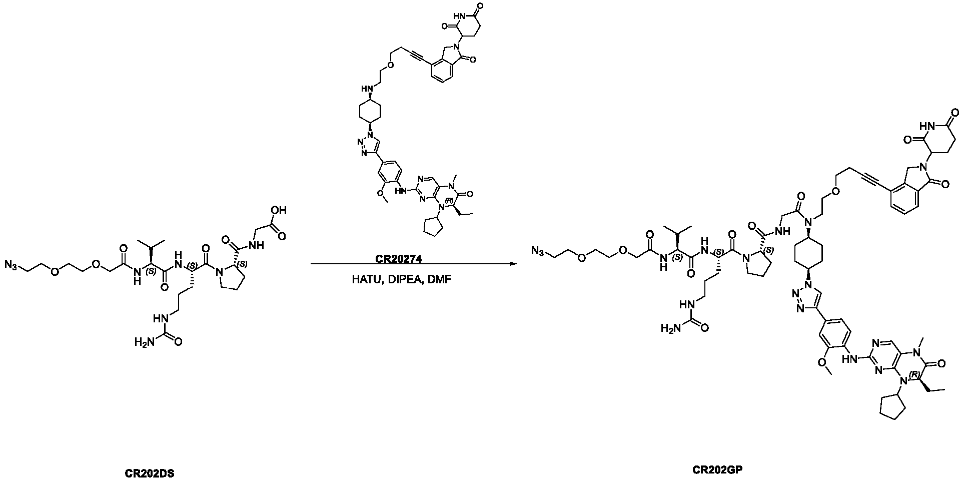

The synthesis of **CR202DS** was conducted by reference to the preparation of Ligand-01: LCMS: [M+H]⁺=600.4; ¹H-NMR (400 MHz, DMSO) δ 8.29 (d, J = 7.2 Hz, 1H), 8.10 (t, J = 5.8 Hz, 1H), 7.41 (d, J = 9.1 Hz, 1H), 4.49-4.40 (m, 1H), 4.35-4.23 (m, 2H), 3.94 (s, 2H), 3.78 (dd, J = 17.5, 6.0 Hz, 1H), 3.73-3.54 (m, 9H), 3.45-3.37 (m, 2H), 2.95 (s, 2H), 2.08-1.78 (m, 5H), 1.73-1.63 (m, 1H), 1.56-1.32 (m, 3H), 0.81 (dd, J = 19.2, 6.7 Hz, 6H)

In a nitrogen atmosphere, **CR202DS** (350.0 mg, 0.58 mmol) was added to DMF (4.0 mL), and the mixture was stirred until complete dissolution and then cooled to 5 °C before HATU (220.5 mg, 0.58 mmol) and DIPEA (224.9 mg, 1.74 mmol) were added. The reaction system was incubated for 0.5 h before **CR20274** (free form, 541.8 mg, 0.61 mmol) was added. The reaction system was naturally warmed to room temperature (27.0 °C) and incubated for 16 h. The reaction mixture was purified by preparative HPLC (mobile phases: 0.1% TFA/H₂O and 0.1% TFA/ACN) and lyophilized to give **CR202GP** (204.9 mg, 91.88% purity, 23.8% yield). LCMS: [M+H]⁺=1465.32.

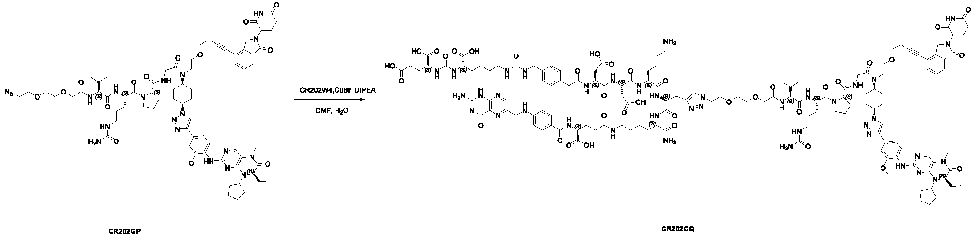

In a nitrogen atmosphere, **CR202GP** (200.0 mg, 0.14 mmol) and **CR202W4** (249.5 mg, 0.16 mmol) were added to a mixed solvent of DMF (12 mL) and water (4 mL). The reaction system was stirred away from light for dissolving, naturally warmed to room temperature (23.0 °C), and adjusted to pH 8 by dropwise adding DIPEA before CuBr (20.0 mg, 0.14 mmol) was added. The reaction system was incubated for 1.5 h. The reaction mixture was directly purified by preparative HPLC (mobile phase: 0.1% TFA/H₂O and 0.1% TFA/ACN). The product purified by preparative HPLC was desalted with purified water and lyophilized to give **CR202GQ** (156.7 mg, 95.61% purity, 36.6% yield). LCMS: [M+4H]⁴⁺=745.93.

### 4.18 Synthesis of CR202LB

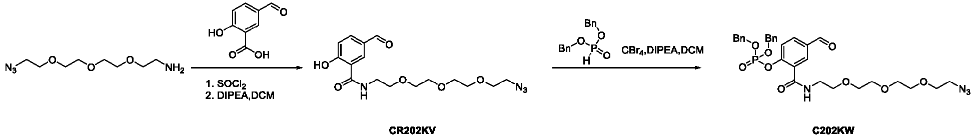

5-Formyl salicylic acid (5.03 g, 2.96 mmol) was added to thionyl chloride (75 mL) and the reaction system was incubated in an oil bath (82 °C) overnight. The reaction mixture was then cooled to 50 °C and concentrated *in vacuo* at 35 °C. The mixture was extracted with anhydrous THF (30 mL × 3) and evaporated thrice to remove residual thionyl chloride, so as to give 5-formyl salicyl chloride (yellow solid).

Azido-triethyleneglycol-amine (4.30 g, 1.97 mmol) was added to THF (80 mL). The reaction system was cooled to 2 °C, before DIPEA (10 mL) was added. 5-Formyl salicyloyl chloride was dissolved in anhydrous THF (20 mL), and the mixture was cooled to 2 to 10 °C and added dropwise to the reaction system (pH 5 to 6) before 1 mL of DIPEA (pH 8) was added. The reaction system was warmed to 20 °C and stirred overnight. Water (80 mL) was added to the reaction mixture, and the resultant mixture was extracted with DCM (100 mL × 3). The organic phases were combined, dried over anhydrous sodium sulfate, and filtered. The filtrate was concentrated to give a crude oil product. The crude product was purified by silica gel column chromatography (DCM:EtOAc = 10:1 to 2:1) to give **CR202KV** (0.8 g, 90% TLC purity, 15% yield). LC-MS: [M+H]⁺:367.06. ¹H-NMR (400 MHz, DMSO) δ 9.71 (s, 1H), 8.37 (d, J = 2.1 Hz, 1H), 7.78 (dd, J = 8.6, 2.2 Hz, 1H), 6.89 (d, J = 8.6 Hz, 1H), 3.60 - 3.48 (m, 10H), 3.46 (dd, J = 7.3, 4.2 Hz, 4H), 3.38-3.26 (m, 2H).

**CR194KV** (0.80 g, 2.18 mmol) was added to DCM (40 mL). The reaction system was stirred for dissolving and cooled to 0 °C in an ice-salt bath before DIPEA (1.0 mL, 5.45 mmol) and CBr₄ (2.79 g, 8.73 mmol) were added. The reaction system was stirred until complete dissolution. Dibenzyl phosphite (1.14 g, 4.36 mmol) was added dropwise, and the ice-salt bath was removed after the addition. The reaction system was controlled at an internal temperature of 5 °C in an ice bath. A 5% aqueous NaH₄PO₄ solution (30 mL) was added dropwise, and the reaction system was extracted with DCM (50 mL × 3). The organic phases were combined, dried over anhydrous sodium sulfate, and filtered. The filtrate was concentrated *in vacuo* at 35 °C to give an orange oil. The crude product was purified by column chromatography (DCM:EtOAc = 10:1 to 2:1) to give **CR202KW** (1.3 g, 60% yield). [M+H]⁺:627.19; ¹H-NMR (400 MHz, CDCl₃) δ 9.96 (s, 1H), 8.34 (s, 1H), 7.85 (dd, J = 8.5, 2.1 Hz, 1H), 7.46 (d, J = 8.5 Hz, 1H), 7.30 (dt, J = 10.0, 5.1 Hz, 10H), 7.20 (s, 1H), 5.12 (d, J = 7.5 Hz, 4H), 3.63-3.51 (m, 14H), 3.30 (t, J = 5.0 Hz, 2H).

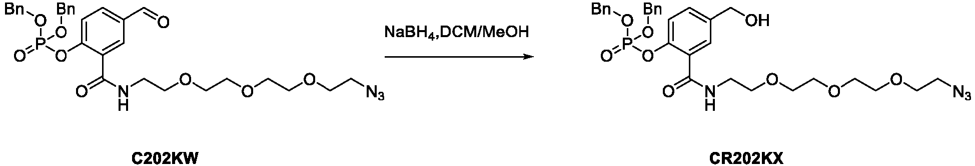

**CR202KW** (1.30 g, 2.07 mmol) was added to a mixed DCM/MeOH solution (50 mL, V/V = 1/1) until complete dissolution. The internal temperature was controlled at 0 °C in an ice-salt bath, before NaBH₄ (0.16 g, 4.14 mmol) was added in portions. The reaction system was stirred at 0 °C for 30 min before water (50 mL) and DCM (50 mL) were added. The organic phase was separated, and the aqueous phase was extracted with DCM (50 mL × 2). The organic phases were combined, dried over anhydrous sodium sulfate, and filtered. The filtrate was concentrated *in vacuo* at 35 °C to give a crude **CR202KX** product (1.29 g, 85.30% purity, 99.2% yield). The crude product was directly used in the next step without purification. LC-MS: [M+H]⁺:629.14; ¹H-NMR (400 MHz, CDCl₃) δ 7.78 (s, 1H), 7.37-7.26 (m, 13H), 5.10 (d, J = 8.5 Hz, 4H), 4.66 (s, 2H), 3.57 (d, J = 11.9 Hz, 4H), 3.30 (t, J = 5.0 Hz, 2H).

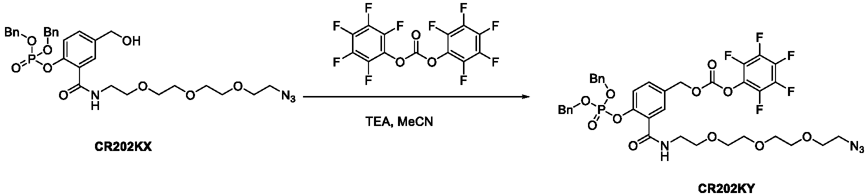

**CR202KX** (1.09 g, 1.73 mmol) was added to acetonitrile (20 mL) until complete dissolution, and the system was cooled in an ice bath. TEA (0.25 mL, 1.73 mmol) was added, before a solution of bis(pentafluorophenyl) carbonate (1.06 g, 2.6 mmol) in acetonitrile (2.5 mL) was added dropwise to the reaction system. The ice bath was removed and the reaction system was stirred for 1.5 h. After the completion of the reaction, the reaction mixture was cooled in an ice bath and extracted with saturated aqueous sodium bicarbonate (30 mL) and ethyl acetate (70 mL). The organic phase was collected. The organic phase was washed with water (once, 40 mL) and saturated sodium chloride solution (30 mL), dried over anhydrous sodium sulfate, and filtered. The filtrate was concentrated *in vacuo* at 30 °C to give an oil. The oil was purified by column chromatography (n-heptane:ethyl acetate = 10:1 to 1:1) to give **CR202KY** (1.02 g, 96.53% purity, 70.3% yield). LC-MS: [M+H]⁺:839.12, H-NMR:¹H-NMR (400 MHz, CDCl₃) δ 7.91 (s, 1H), 7.43-7.26 (m, 12H), 5.28 (s, 2H), 5.11 (d, J = 8.9 Hz, 4H), 3.66-3.47 (m, 14H), 3.30 (t, J = 5.0 Hz, 2H).

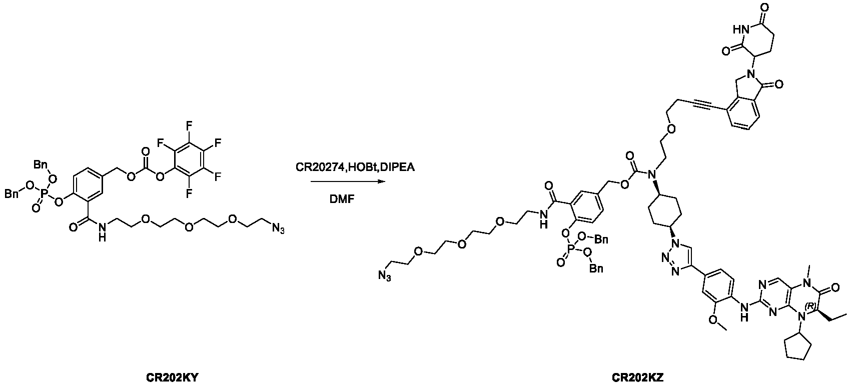

**CR20274** (0.57 g, 0.65 mmol) was dissolved in DMF (10 mL), and the system was cooled in an ice bath. HOBT (88.0 mg, 0.65 mmol) and DIPEA (140 µL, 0.78 mmol) were added sequentially. **CR202KY** (0.66 g, 0.78 mmol) was dissolved in DMF (2.5 mL), and the mixture was added dropwise to the reaction system. After the addition, the ice bath was removed, and the reaction system was stirred for 1 h. The reaction mixture was purified by preparative chromatography and lyophilized to give **CR202KZ** (0.79 g, 93.01% purity, 80.0% yield). LC-MS: [M+2H]²⁺:769.92.

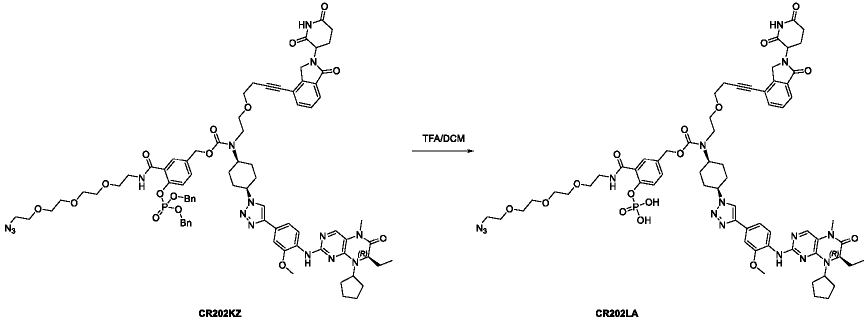

A 50% solution of TFA in dichloromethane (14 mL, V/V = 1/1) was cooled to 0 °C before **CR202KZ** (0.40 g, 0.26 mmol) was added. The mixture was stirred until complete dissolution, warmed to 24 °C, and stirred for 2 h. The reaction mixture was concentrated *in vacuo* at 27 °C to give **CR202LA** (0.36 g), which was directly used in the next reaction according to the theoretical yield. LC-MS: [M+2]²⁺:680.25.

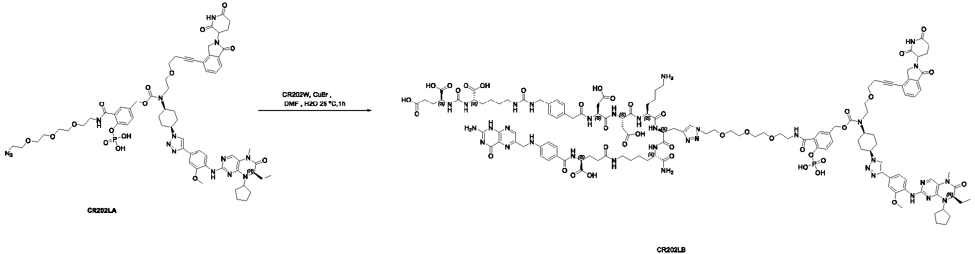

**CR202LA** (0.36 g, 0.26 mmol) was added to a DMF:H₂O (12 mL, V/V = 1/1) mixed solvent, and the system was cooled to 4 °C before DIPEA (1.7 mL) was added dropwise. The reaction system was adjusted to pH 8, and **CR202W4** (0.39 g, 0.26 mmol) and CuBr (37.1 mg, 10.26 mmol) were sequentially added. The reaction system was warmed to an inner temperature of 24.2 °C and stirred for 1.5 h, until on-line HPLC monitoring indicated the completion of the reaction. The reaction mixture was purified by preparative HPLC and lyophilized to give **CR202LB** (0.29 g, 98.34% purity, 38.8% two-step yield).

### 4.19 Synthesis of CR202HV

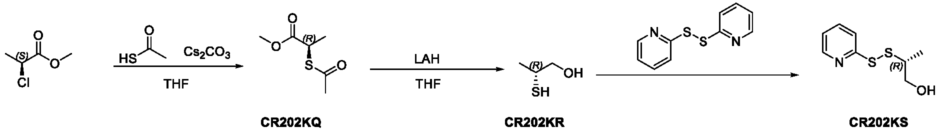

In a nitrogen atmosphere, cesium carbonate (53.18 g, 179.48 mmol) was added to THF (290 mL), before thioacetic acid (13.66 g, 179.48 mmol) was added with stirring. The reaction system was incubated at 25 °C for 30 min before methyl (s)-(-)-2-chloropropionate (20.00 g, 163.16 mmol) was added, and then incubated for another 1 h. The reaction mixture was filtered. The filtrate was added to water (1.2 L), and the mixture was extracted twice with MTBE (1 L × 2). The organic phases were combined, dried over anhydrous sodium sulfate, and filtered. The filtrate was concentrated to give **CR202KQ** (24.34 g, 90.09% purity, 92.00% yield). [M+H] ⁺=163.11. ¹H-NMR (400 MHz, CDCl₃) δ 4.16 (q, J = 7.3 Hz, 1H), 3.66 (s, 4H), 2.28 (s, 3H), 1.43 (d, J = 7.3 Hz, 3H).

In a nitrogen atmosphere, LiAlH₄ (9.36 g, 246.64 mmol) was added to THF (150 mL), and the mixture was stirred for dissolving and cooled to 0 °C. Meanwhile, **CR202KQ** (10.00 g, 61.65 mmol) was dissolved in THF (50 mL), and the solution was slowly added dropwise to the LiAlH₄ solution. After the addition, the reaction system was warmed to room temperature and incubated for 1.5 h, until on-line HPLC monitoring indicated the completion of the reaction. The reaction mixture was then cooled to 0 °C before a 2 M HCl solution was slowly and dropwise added until no bubbles were generated. The system was stirred for another 15 min and filtered. The filter cake was washed once with THF (100 mL), and the (THF) filtrates were combined and discarded. The filter cake was washed with methanol (300 mL) and the mixture was filtered. This procedure was repeated, and the organic phases were combined, dried over anhydrous sodium sulfate, filtered, and concentrated to give **CR202KR** (9.0104 g, 92.21% purity, 158.63% yield; note: the yield over 100% was attributed to excessive methanol residue).

2,2'-Dithiodipyridine (10.37 g, 47.07 mmol) was added to methanol (40 mL) before **CR202KR** (8.6763 g, 94.14 mmol) was added. The reaction system was incubated at 25 °C for 2 h, until on-line HPLC monitoring indicated the completion of the reaction. The reaction mixture was concentrated to give a crude product. The crude product was purified by column chromatography (n-heptane/EA = 6/1) to give **CR202KS** (2.5298 g, 94.35% purity, 26.7% yield). LCMS: [M+H]⁺ =201.97. ¹H-NMR (400 MHz, DMSO) δ 8.43 (m, 1H), 7.81 (m, 2H), 7.22 (td, J = 5.3, 2.8 Hz, 1H), 5.08 (t, J = 5.7 Hz, 1H), 3.46 (m, 2H), 3.05 (dd, J = 13.0, 6.5 Hz, 1H), 1.22 (t, J = 6.5 Hz, 3H).

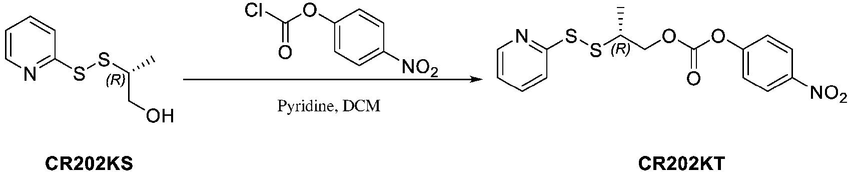

**CR202KS** (1.20 g, 5.96 mmol) was added to DCM (12 mL) before p-nitrophenyl chloroformate (1.44 g, 7.14 mmol) and pyridine (0.49 g, 6.26 mmol) were added. The reaction system was incubated at 25 °C for 1 h, until on-line HPLC monitoring indicated the completion of the reaction. The reaction mixture was concentrated to give a crude product. The crude product was purified by column chromatography (n-heptane/EA = 10/1) to give **CR202KT** (2.01 g, 87.42% purity, 92.2% yield). LCMS: [M+H]⁺ =366.98; ¹H-NMR (400 MHz, DMSO) δ 8.44 (d, J = 4.5 Hz, 1H), 8.31 (m, 2H), 7.81 (m, 2H), 7.55 (m, 2H), 7.24 (ddd, J = 6.5, 4.8, 1.5 Hz, 1H), 4.34 (qd, J = 11.2, 6.0 Hz, 2H), 3.46 (dt, J = 13.1, 6.5 Hz, 1H), 1.33 (d, J = 7.0 Hz, 3H).

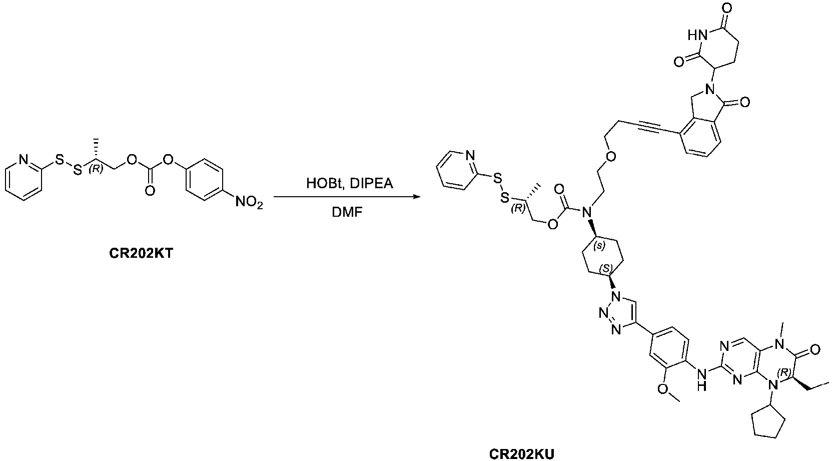

**CR202KT** (314.0 mg, 0.86 mmol) was added to DMF (5 mL) before **CR20274** (500.0 mg, 0.57 mmol), HOBT (76.4 mg, 0.57 mmol), and DIPEA (146.0 mg, 1.13 mmol) were added. The reaction system was incubated at 25 °C for 16 h, until on-line detection monitoring indicated the completion of the reaction. The reaction mixture was purified by preparative HPLC and lyophilized to give **CR202KU** (0.4567 g, 88.69% purity, 72.69% yield).

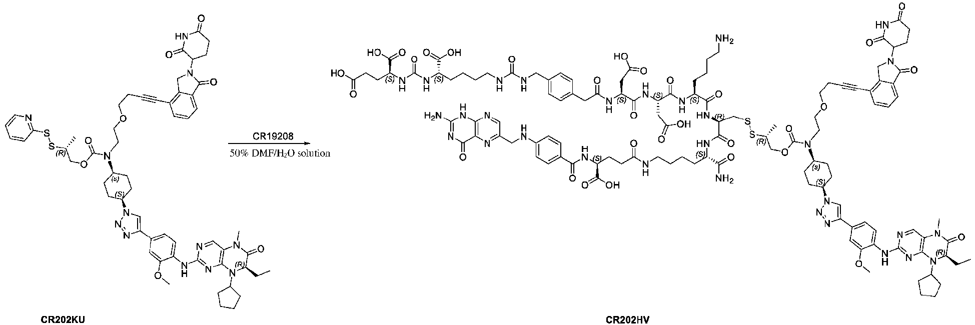

In a nitrogen atmosphere, **CR19208** (205.5 mg, 0.13 mmol) was added to a 50% DMF/H₂O solution (8 mL, V/V = 1/1) before a 0.1 M NaHCO₃ solution was added dropwise, until the pH of the reaction system was about 7. **CR202KU** (150.0 mg, 0.13 mmol) was dissolved in THF (3 mL) and added dropwise to the reaction system (turbidity was observed). 10 mL of the 50% DMF/H₂O solution, 10 mL of DMF, and 5 mL of 0.1 M NaHCO₃ solution were added, and the reaction system turned clear. The reaction system was then incubated at 25 °C for 0.5 h, until on-line HPLC monitoring indicated the completion of the reaction. The reaction mixture was purified by preparative HPLC (mobile phase: 0.1% TFA/H₂O and 0.1% TFA/ACN) and lyophilized to give **CR202HV** (99.5 mg, 98.73 % purity, 29.26% yield). LCMS: [M+3H]³⁺=841.76.

### 4.20 Synthesis of CR202GC

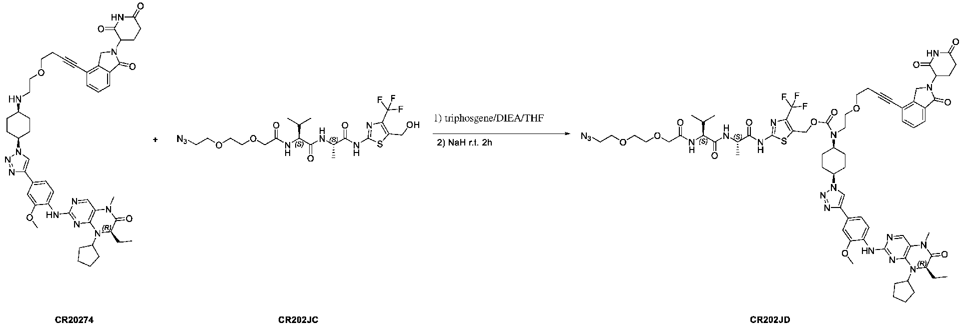

In a nitrogen atmosphere, **CR20274** (313.9 mg, 0.3551 mmol) followed by THF (10.0 mL) was added to a 25-mL single-neck flask, and the reaction system was cooled to 0 °C. Triphosgene (42.2 mg, 0.1420 mmol) was added, and a white solid was precipitated. DIEA was added dropwise until the solid was completely dissolved, and the mixture was incubated at 0 °C for 40 min. **CR202JC** (191.6 mg, 0.3551 mmol) and THF (2.0 mL) were added to another 25-mL single-neck flask. The mixture was cooled to 0 °C before NaH (85.2 mg, 2.1306 mmol) was added, and stirred until no more bubbles were generated. The solution of **CR20274** was added to the solution of **CR202JC.** After the addition, the reaction system was slowly warmed to room temperature for 30 min, until on-line HPLC monitoring indicated the completion of the reaction. The reaction mixture was purified by preparative HPLC to give 44.2 mg of a white solid **CR202JD** (80.42% purity, 8.6% yield).

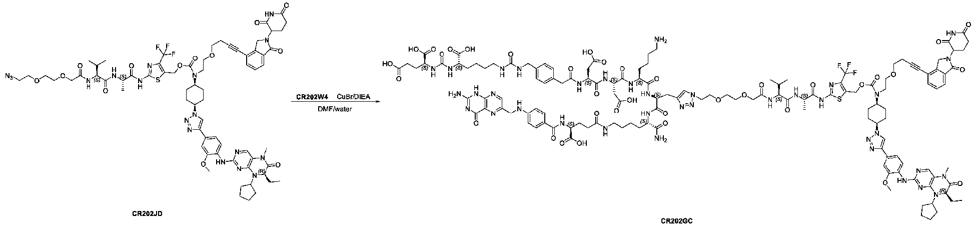

Proper amounts of **CR202JD** (40.0 mg, 0.0276 mmol, 1.0 eq.) and **CR202W4** (41.8 mg, 0.0276 mmol, 1.0 eq.) were added into a 50-mL jacketed flask before a DMF/H₂O solution (2.0 mL, V/V = 3/1) was added away from light with the temperature controlled at 25 °C. The reaction system was adjusted to pH 8 by dropwise adding DIEA before CuBr (4.5 mg, 0.0276 mmol, 1.0 eq.) was added. The reaction system was stirred at 25 °C for 10 min. 20 µL of the reaction mixture was diluted to 300 µL with ACN/MeOH/H₂O until on-line HPLC monitoring indicated the completion of the reaction. The reaction mixture was refrigerated at 2 to 8 °C for 2 h and purified by preparative HPLC to give a white solid **CR202GC** (22.0 mg, 98.53% purity).

### 4.21 Synthesis of CR202JG

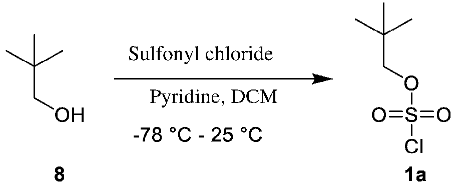

**Compound 8** (20.0 g, 226 mmol, 24.4 mL, 1.40 eq.) and pyridine (12.8 g, 162 mmol, 13.1 mL, 1.00 eq.) were dissolved in DCM (200 mL) with temperature controlled at -78 °C to -60 °C. The mixture was slowly and dropwise added to a solution of sulfonyl chloride (21.8 g, 162 mmol, 16.2 mL, 1.00 eq.) in DCM (200 mL), and the reaction system was incubated at room temperature for 10 h. The reaction mixture was concentrated to give a yellow oil, which was distilled to give a colorless oil **compound 1a** (10.5 g, crude). ¹H NMR: (400 MHz, CDCl₃) δ: 4.18 (s, 2H), 1.06 (s, 9H).

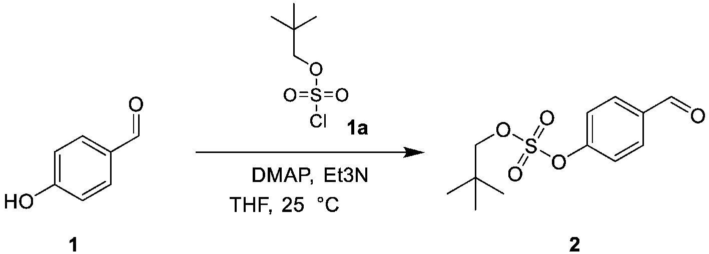

**Compound 1a** (5.00 g, 40.9 mmol, 1.00 eq.), DMAP (5.00 g, 40.9 mmol, 1.00 eq.), and Et₃N (8.29 g, 81.8 mmol, 11.4 mL, 2.00 eq.) were dissolved in THF (70.0 mL), and a solution of **compound 1** (9.55 g, 51.1 mmol, 1.25 eq.) in THF (35.0 mL) was added dropwise to the above solution. The reaction system was incubated at room temperature for 12 h. The reaction was quenched by adding 10% citric acid (500 mL), and the mixture was extracted with ethyl acetate (200 mL × 2). The organic phases were combined, washed with saturated brine (200 mL), dried over anhydrous sodium sulfate, and filtered. The filtrate was concentrated to give a yellow oil, which was purified by column chromatography (petroleum ether/ethyl acetate = 10/1 to 5/1) to give a colorless oil **compound 2** (9.00 g, 32.7 mmol, 99.0% purity, 79.9% yield). ¹H NMR: (CDCl₃, 400 MHz) δ 9.94 (s, 1H), 7.91 - 7.87 (m, 2H), 7.41 (dd, J = 10.8 Hz, 2.40 Hz, 2H), 4.04 (s, 2H), 0.90 (s, 9H).

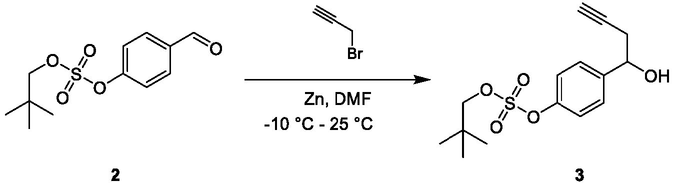

**Compound 2** (9.00 g, 32.7 mmol, 1.00 eq.) and 3-bromopropyne (7.30 g, 49.0 mmol, 5.29 mL, 80% purity, 1.50 eq.) were dissolved in DMF (135 mL) before Zn (3.44 g, 52.6 mmol, 1.61 eq.) was added at -10 °C. The reaction system was warmed to room temperature and incubated for 12 h. The reaction was quenched by adding aqueous ammonium chloride (400 mL), and the reaction mixture was extracted with ethyl acetate (250 mL × 3). The organic phases were combined, dried over anhydrous magnesium sulfate, and filtered. The filtrate was concentrated, and the crude product was purified by column chromatography (PE/EA = 6/1) to give a yellow oil **compound 3** (10.0 g, crude). LCMS: [M+H]⁺ = 313.1; ¹H NMR: (CDCl₃, 400 MHz) δ 7.46 (d, J = 8.80 Hz, 2H), 7.32 - 7.28 (m, 2H), 4.90 (t, J = 6.40 Hz, 1H), 4.09 (s, 2H), 2.65 - 2.62 (m, 2H), 2.09 (t, J = 2.80 Hz, 1H), 1.01 (s, 9H).

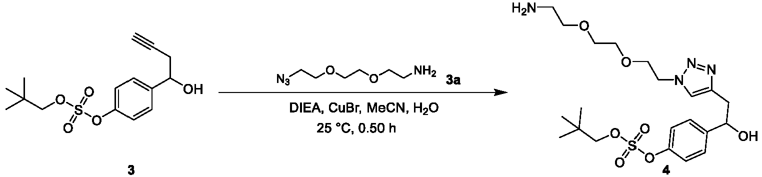

**Compound 3** (6.87 g, 19.5 mmol, 1.00 eq.) and **compound 3a** (3.41 g, 19.5 mmol, 1.00 eq.) were dissolved in acetonitrile (60 mL) and water (18.0 mL) before DIEA (2.03 g, 15.6 mmol, 2.73 mL, 0.80 eq.) and CuBr (2.81 g, 19.6 mmol, 596 µL, 1.00 eq.) were added at room temperature. The reaction system was incubated at room temperature for 0.5 h. The reaction mixture was acidified by adding acetic acid to give a blue solution, which was purified by preparative HPLC (0.05% TFA system) to give **compound 4** (10.7 g, 17.5 mmol, 98.6% purity, 89.6% yield, TFA salt). LCMS: [M+H]⁺ = 487.2; ¹H NMR: (CDCl₃, 400 MHz) δ 8.01 (br s, 3H), 7.46 (br s, 2H), 7.25 (s, 2H), 4.49 - 4.46 (m, 2H), 4.10 (s, 2H), 3.85 - 3.55 (m, 10H), 3.11 (s, 3H), 1.01 (s, 9H).

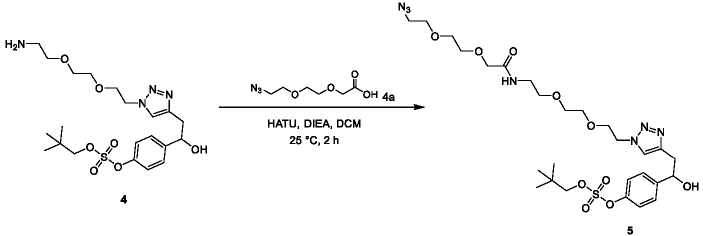

**Compound 4** (6.30 g, 10.3 mmol, 1.00 eq., TFA salt), **compound 4a** (1.62 g, 8.58 mmol, 0.83 eq.), and DIPEA (4.01 g, 31.0 mmol, 5.40 mL, 3.00 eq.) were dissolved in DMF (30.0 mL) before HATU (3.93 g, 10.3 mmol, 1.00 eq.) was added at room temperature. The reaction system was incubated at room temperature for 2 h. The reaction mixture was purified by preparative HPLC (0.01% TFA system) to give **compound 5** (3.30 g, 4.95 mmol, 98.6% purity, 47.8% yield). LCMS: [M+H]⁺ = 658.4; ¹H NMR: (CDCl₃, 400 MHz) δ 7.51 (s, 1H), 7.39 (d, J = 8.40 Hz, 2H), 7.21 - 7.20 (m, 2H), 7.04 (s, 1H), 5.00 (dd, J = 8.80 Hz, 3.20 Hz, 1H), 4.46 - 4.43 (m, 2H), 4.01 (s, 2H), 3.89 (s, 2H), 3.78 - 3.59 (m, 2H), 3.58 - 3.32 (m, 16H), 3.04 - 2.99 (m, 2H), 0.94 (s, 9H).

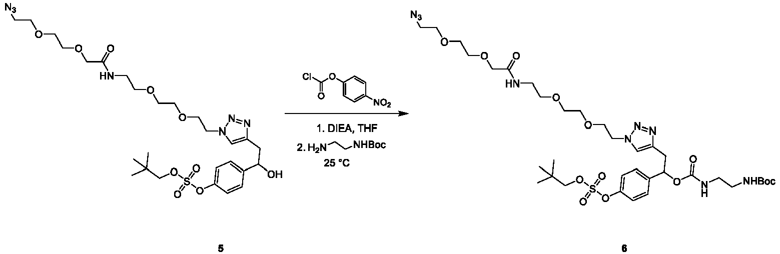

**Compound 5** (3.50 g, 5.32 mmol, 1.00 eq.) and DIPEA (2.75 g, 21.3 mmol, 3.71 mL, 4.00 eq.) were dissolved in THF (35.0 mL) before p-nitrophenyl chloroformate (2.25 g, 11.2 mmol, 2.10 eq.) was added at room temperature. The reaction system was incubated at room temperature for 1 h before N-tert-butoxycarbonyl-1,2-ethylenediamine (1.71 g, 10.6 mmol, 1.67 mL, 2.00 eq.) was added. The reaction system was further incubated at room temperature for 2 h. The reaction mixture was purified by preparative HPLC (0.01% TFA system) to give **compound 6** (3.50 g, 4.00 mmol, 96.4% purity, 75.1% yield). LCMS: [M+H]+ = 844.4; ¹H NMR: (CDCl₃, 400 MHz) δ 7.43 (s, 1H), 7.35 (d, J = 8.80 Hz, 2H), 7.24 - 7.22 (m, 2H), 7.09 (s, 1H), 5.92 - 5.89 (m, 1H), 5.77 (s, 1H), 5.18 (s, 1H), 4.48 - 4.46 (m, 2H), 4.06 (s, 2H), 3.98 (s, 2H), 3.80 - 3.66 (m, 2H), 3.65 - 3.55 (m, 14H), 3.53 (d, J = 8.40 Hz, 2H), 3.37 - 3.19 (m, 6H), 1.40 (s, 9H), 0.98 (s, 9H).

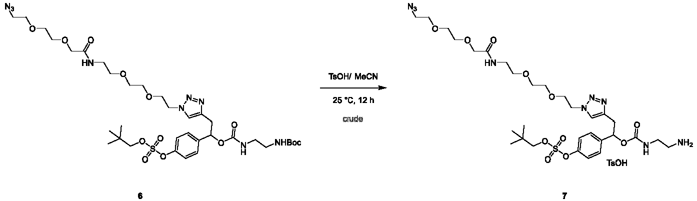

**Compound 6** (3.50 g, 4.00 mmol, 1.00 eq.) was dissolved in acetonitrile (44.0 mL) before TsOH (2.41 g, 13.9 mmol, 3.50 eq.) was added at room temperature. The reaction system was incubated at room temperature for 12 h. The reaction mixture was concentrated to dryness to give a yellow oil **compound 7** (4.00 g, crude), which was directly used in the next step.

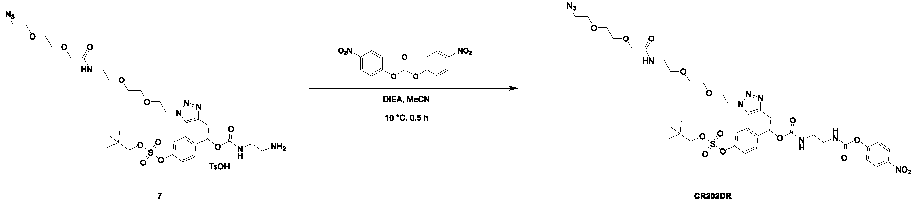

**Compound 7** (2.00 g, 2.18 mmol, 1.00 eq., crude) and DIEA (1.13 g, 8.73 mmol, 1.52 mL, 4.00 eq.) were dissolved in acetonitrile (20 mL) before di(*p*-nitrophenol) carbonate (531 mg, 1.75 mmol, 0.80 eq.) was added at 10 °C. The reaction system was incubated at 10 °C for 0.5 h. The reaction mixture was purified by preparative HPLC (0.01% TFA system) to give a colorless oil **CR202DR** (1.05 g, 1.14 mmol, 52.2% yield, 98.7% purity). LCMS: [M+H]⁺ = 909.3; ¹H NMR: (CDCl₃, 400 MHz) δ 8.23 (d, J = 8.80 Hz, 2H), 7.48(s, 1H), 7.38 (d, J = 8.40 Hz, 2H), 7.31 - 7.25 (m, 4H), 7.16 (s, 1H), 6.46 (s, 1H), 5.97 (s, 2H), 4.48 (t, J = 4.80 Hz, 2H), 4.10 (s, 2H), 4.01 (s, 2H), 3.82 - 3.67 (m, 2H), 3.58 - 3.26 (m, 22H), 1.01(s, 9H).

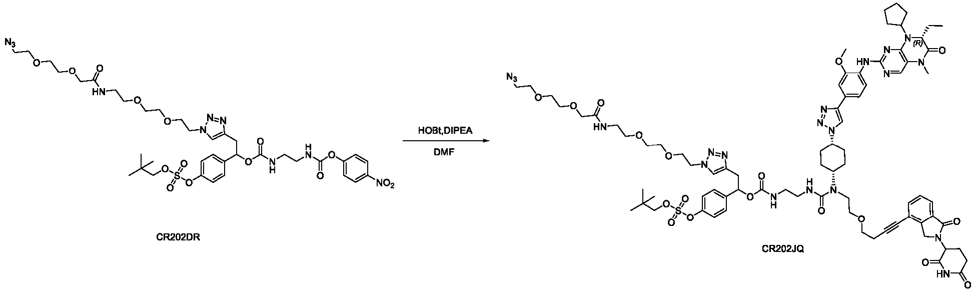

**CR202DR** (350 mg, 385 µmol) was dissolved in DMF (7 mL), and the system was cooled in an ice bath. In a nitrogen atmosphere, **CR20274** (340 mg, 385 µmol), HOBT (10 mg, 77 µmol), and DIEA (100 mg, 770 µmol) were added, and the reaction system was stirred at 25 °C for 6 h. The reaction mixture was poured into 70 mL of MTBE. The lower phase is an oil phase, and the clear upper phase was discarded. 50 mL of MTBE was added to the oil with stirring. The oil was partially solidified to give a semi-solid crude **CR202JQ** product (78.22% purity), which was directly used in the next reaction. LCMS: [M+H]⁺ = 1654.2.

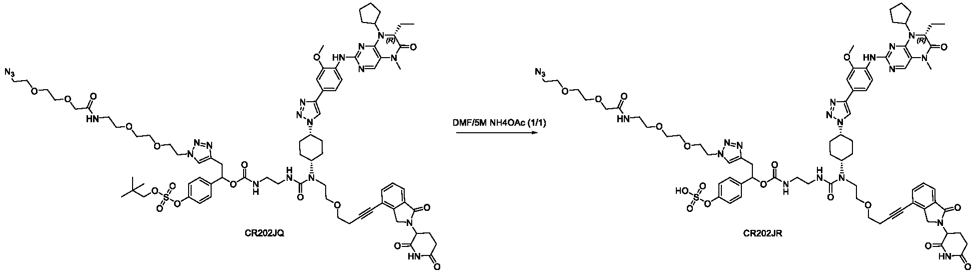

In a nitrogen atmosphere, **CR202JQ** obtained in the previous step was dissolved in DMF (7.5 mL) in a 100-mL recovery flask, before a 5 M aqueous NH₄OAc solution (7.5 mL) was added. The reaction system was stirred at 55 °C for 16 h, until on-line detection indicated the completion of the reaction. The reaction mixture was purified by preparative HPLC to give **CR202JR** (219.3 mg, 85.83% purity, 55.2% yield). [M+H]⁺ = 1504.07.

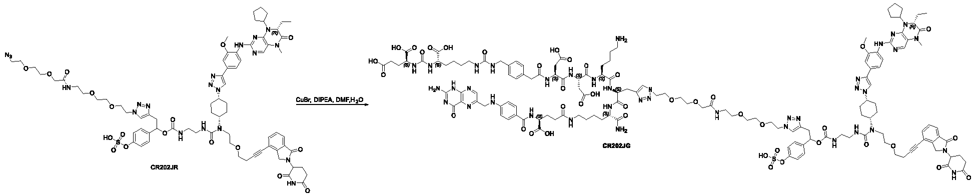

In a nitrogen atmosphere, **CR202JR** (151.0 mg, 94.7 µmol) and **CR202W4** (170.2 mg, 94.7 µmol) were added to a 25-mL recovery flask, before DMF (7.5 mL) and water (7.5 mL) were added. The system was stirred away from light until complete dissolution. The reaction system was adjusted to pH 8 by adding DIEA before CuBr (13.6 mg, 94.7 µmol) was added. The reaction system was then incubated at 25 °C for 2.5 h, until on-line detection indicated the completion of the reaction. The reaction mixture was purified by preparative HPLC to give **CR202JG** (62.3 mg, 96.78% purity, 21.2% yield). LCMS: [M-2H]²⁻ = 1547.85.

### 4.22 Synthesis of CR202HT

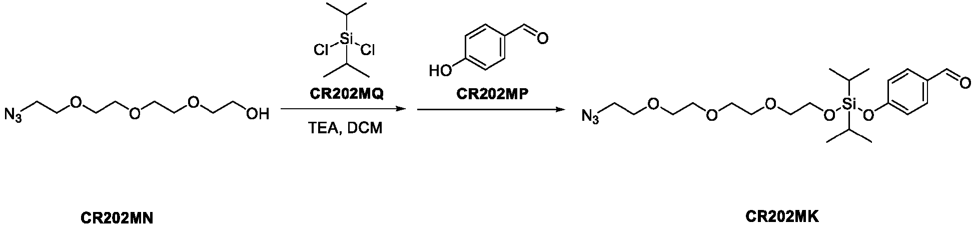

In a nitrogen atmosphere, TEA (2.54 g, 25.08 mmol) was added to DCM (20 mL). The system was cooled to 0 °C, before **CR202MQ** (1.95 g, 10.53 mmol) was added dropwise. The syringe was rinsed with DCM (4 mL), and the reaction system was incubated for 0.5 h. **CR202MN**/DCM (2.20 g/8 mL, 10.03 mmol) was added dropwise with a syringe pump. After 1 h of addition, the reaction system was incubated for 1 h, and **CR202MP**/DCM (1.35 g/5 mL, 11.24 mmol) was added dropwise. After 10 min of addition, the reaction system was incubated at 0 °C for 1 h. DCM was removed by concentration, before EA/n-heptane (V/V = 1/4, 60 mL) was added for dilution. The mixture was washed with purified water thrice (60 mL × 3). The organic phase was dried over anhydrous sodium sulfate and filtered. The filtrate was concentrated *in vacuo* at 35 °C and purified by column chromatography (n-heptane/EA = 100/0 to 8/1) to give a pale yellow oil **CR202MK** (1.59 g, about 80% purity, 54.9% yield). ¹H-NMR (400 MHz, CDCl₃) δ 9.93 (s, 1H), 7.84 (d, J = 8.6 Hz, 2H), 7.15 (d, J = 8.5 Hz, 2H), 4.02 (t, J = 5.2 Hz, 2H), 3.70 (m, 12H), 3.42 (m, 2H), 1.23 (m, 2H), 1.13 (dd, J = 7.1, 3.4 Hz, 12H).

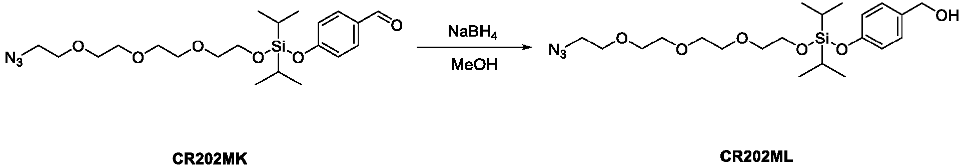

**CR202MK** (112.8 mg, 0.25 mmol) was added to methanol (1.1 mL) before sodium borohydride (10.0 mg, 0.25 mmol) was added at an external temperature of -4.0 °C. The reaction was monitored by on-line TLC until completion. Purified water (2.0 mL) was added dropwise to quench the reaction, and EA (3.0 mL) was added for extraction. The organic phase was dried over anhydrous sodium sulfate, filtered, and concentrated *in vacuo* at 35 °C to give a pale yellow oil **CR202ML** (181.5 mg). ¹H-NMR (400 MHz, CDCl₃) δ 7.27 (d, J = 8.5 Hz, 2H), 6.99 (d, J = 8.5 Hz, 2H), 4.65 (s, 2H), 4.00 (t, J = 5.4 Hz, 2H), 3.70 (m, 12H), 3.41 (m, 2H), 1.19 (m, 2H), 1.11 (dd, J = 6.9, 2.5 Hz, 12H).

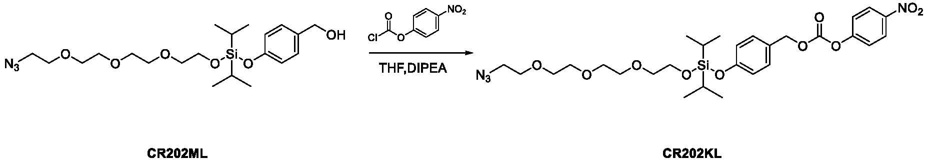

**CR202ML** (170.0 mg, 0.373 mmol) was added to THF (3.0 mL), and the mixture was stirred to form a suspension. In a nitrogen atmosphere, DIPEA (120.5 mg, 0.933 mmol) and p-nitrophenol chloroformate (112.8 mg, 0.559 mmol) were added, and the reaction system was incubated at room temperature (26.2 °C) for 6 h. DCM (1.2 mL) was added until the reaction system was clear, and the reaction was further incubated for 44 h, until on-line HPLC monitoring indicated a substantial completion of the reaction. EA (5.0 mL) and purified water (5.0 mL) were added for extraction. The extract was washed twice with purified water (5 mL × 2). The organic phase was dried over anhydrous sodium sulfate, filtered, and concentrated *in vacuo.* The crude product was purified by preparative HPLC (mobile phase: acetonitrile and water) and lyophilized to give an oil **CR202KL** (219.0 mg, 98.23% purity, 61.8% yield).

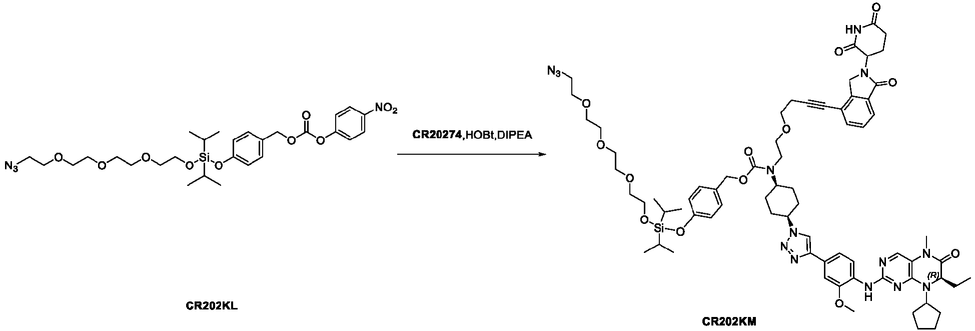

In a nitrogen atmosphere, **CR202KL** (2170 mg, 0.35 mmol), **CR20274** (370.8 mg, 0.42 mmol), and HOBt (10.1 mg, 0.07 mmol) were added to DMF (4.0 mL) before DIPEA (90.5 mg, 0.70 mmol) was added. The reaction system was incubated at room temperature (24.9 °C) for 7 h, until on-line detection indicated the completion of the reaction. The reaction mixture was purified by preparative HPLC (mobile phase: acetonitrile and water) and lyophilized to give **CR202KM** (107.7 mg, 95.0% purity, 22.4% yield).

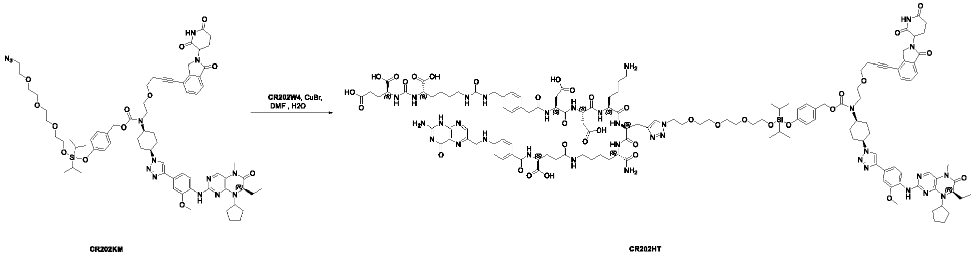

**CR202KM** (107.0 mg, 0.078 mmol) was dissolved in DMF (6.5 mL), before purified water (3.5 mL) was added. The reaction system turned turbid. **CR202W4** (142.5 mg, 0.095 mmol) was added away from light. The system was purged with nitrogen thrice and stirred at an external temperature of 25 °C until the system was clear. The system was adjusted to pH 8 by dropwise adding DIPEA (100 µL), before copper(I) bromide (11.2 mg, 0.078 mmol) was added. The reaction system was incubated for 2 h, until on-line detection indicated the completion of the reaction. The reaction mixture was purified by preparative HPLC (mobile phase: 15 nM sodium acetate/water-acetonitrile system; desalting: acetonitrile-water system) and lyophilized to give **CR202HT** (26.1 mg, 88.29% purity, 16.0 mg, 73.74% purity). LCMS: [M+3H]³⁺=960.92.

### 4.23 Synthesis of CR202HU

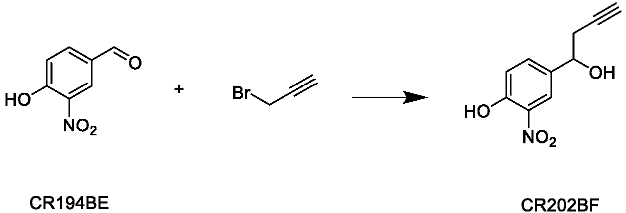

Zinc powder (9.03 g, 0.138 mol) was charged into a 500-mL single-neck flask, before anhydrous THF (150 mL) was added and propargyl bromide (16.41 g, 0.138 mol) was added dropwise. The system was stirred for 30 min. **CR202BE** (9.98 g,0.060 mol) was dissolved in anhydrous THF (150 mL). The solution was added dropwise to the reaction system at -10 °C to - 5 °C. After the dropwise addition, the ice bath was removed and the reaction system was incubated for 15 min, until the reaction mixture gelled. The reaction was monitored by on-line HPLC. The reaction mixture was cooled to 0 °C before 100 mL of saturated ammonium chloride was added dropwise and 100 mL of water was added. The reaction mixture was transferred to a separatory funnel, before 300 mL of ethyl acetate was added for two extractions. The organic phases were combined, dried over anhydrous sodium sulfate, and filtered. The filtrate was concentrated *in vacuo* at 35 °C to give a brown oil. The crude product was purified by column chromatography (n-heptane:ethyl acetate = 20:1 to 2:1) to give **CR202BF** (12.00 g, 91.14% purity, 97.6% yield). ¹H-NMR (400 MHz, CDCl₃) δ 10.53 (s, 1H), 8.13 (d, J = 1.9 Hz, 1H), 7.62 (dd, J = 8.7, 2.0 Hz, 1H), 7.14 (d, J = 8.7 Hz, 1H), 4.86 (dd, J = 9.5, 6.2 Hz, 1H), 2.71 - 2.55 (m, 2H), 2.48 (d, J = 3.6 Hz, 1H), 2.11 - 2.04 (m, 1H).

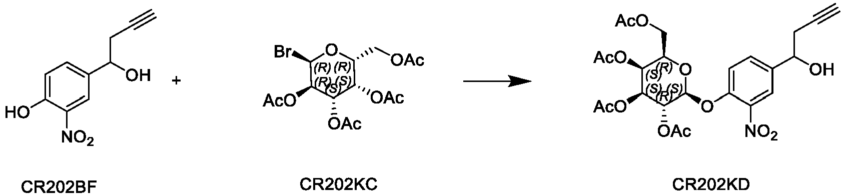

Silver carbonate (13.24 g, 48.03 mmol) was added to a 250 mL single-neck flask before acetonitrile (130 mL) was added. The system was cooled in an ice-salt bath. HMTTA (2.09 g, 9.09 mmol) was added, and the system was stirred for 7 min. **CR202BF** (2.70 g, 12.98 mmol) and **CR202KC** (6.94 g, 16.88 mmol) were added, and the ice-salt bath was removed. The reaction system was incubated for 1 h, with the reaction being monitored by on-line HPLC. The reaction mixture was filtered to remove silver carbonate, before ethyl acetate (150 mL) and water (100 mL) were added to the filtrate. The organic phase was separated, and the aqueous phase was extracted with ethyl acetate (100 mL). The organic phases were combined, washed with saturated sodium chloride (100 mL), dried over anhydrous sodium sulfate, and filtered (celite + silica gel + celite triple filtration). The filtrate was concentrated *in vacuo* at 28 °C after filtration and purified by column chromatography (n-heptane:ethyl acetate =4:1 to 1:2) to give **CR202KD** (10.00 g, 120.9% yield (containing ethyl acetate)). ¹H-NMR (400 MHz, CDCl₃) δ 7.83 (dd, J = 9.1, 2.1 Hz, 1H), 7.54 (td, J = 9.3, 2.2 Hz, 1H), 7.32 (d, J = 8.6 Hz, 1H), 5.51 (dd, J = 10.5, 7.9 Hz, 1H), 5.44 (d, J = 2.8 Hz, 1H), 5.06 (ddd, J = 9.2, 8.5, 1.9 Hz, 2H), 4.88 (t, J = 6.2 Hz, 1H), 4.30 - 4.19 (m, 1H), 4.18 - 3.98 (m, 2H), 2.71 - 2.54 (m, 2H), 2.16 (s, 3H), 2.13 - 2.07 (m,3H), 2.04 (s, 3H), 2.01 (s, 3H).

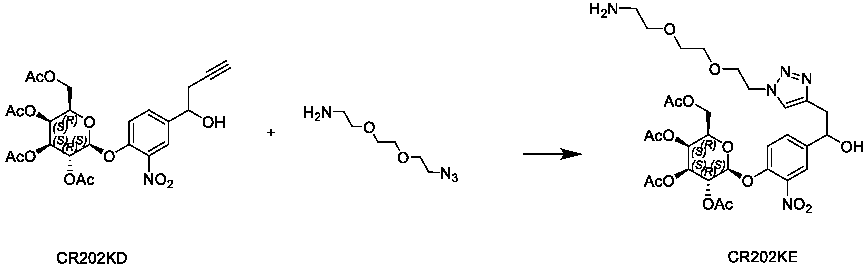

**CR202KD** (9.50 g, 0.018 mol) was dissolved in acetonitrile/H₂O (280 mL, acetonitrile:H₂O = 3:1). The system was cooled to 3 °C in an ice-salt bath before N₃-(PEG)₂-NH₂ (3.08 g, 0.018 mol) was added. CuBr (2.54 g, 0.018 mol) was then added and the reaction system was incubated at 3 °C for 20 min with the reaction being monitored by on-line HPLC. 5% TFA (60 mL) was added dropwise to the reaction mixture. The reaction mixture was adjusted to pH 5, purified by preparative chromatography, and lyophilized to give **CR202KE** (11.02 g, 93.38% purity, 115.7% yield (containing reactant). LC-MS: [M+H]⁺: 712.17.

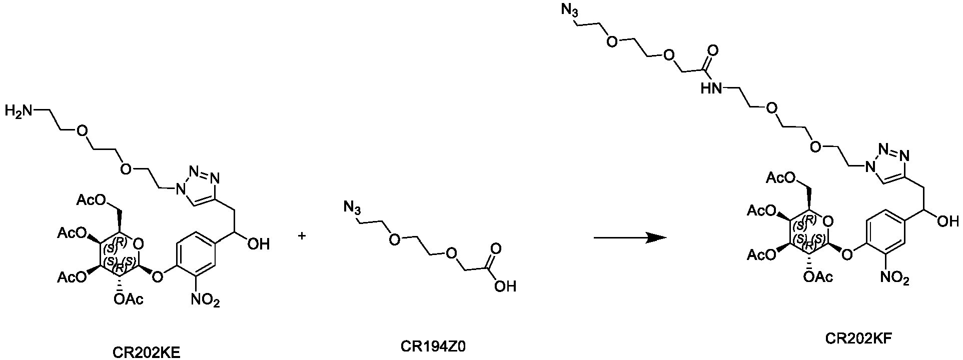

**CR202KE** (10.00 g, 0.014 mol) was added into a 500-mL single-neck flask before anhydrous DMAc (100 mL) was added. The system was cooled to an internal temperature of 4 °C in an ice bath before **CR202Z0** (2.66 g, 0.014 mol) and HATU (5.34 g, 0.014 mol) were added. DIPEA (11 mL, 0.063 mol) was added dropwise, and the reaction system was incubated in an ice bath for 25 min, until on-line detection indicated the completion of the reaction. 300 mL of water was added dropwise to the reaction mixture with temperature controlled at <20 °C. The mixture was extracted with ethyl acetate (200 mL × 2). The organic phases were combined, washed with saturated brine (50 mL × 4), dried over anhydrous sodium sulfate, and filtered. The filtrate was concentrated *in vacuo* at 25 °C to give a crude product, which was purified by column chromatography (n-heptane:ethyl acetate = 4:1; DCM:MeOH = 100:1 to 30:1) to give **CR202KF** (6.20 g, 90.02% purity, 61.4% yield). ¹H-NMR (400 MHz, CDCl₃) δ 7.81 (s, 1H), 7.73 (d, J = 4.3 Hz, 1H), 7.56 (d, J = 8.8 Hz, 1H), 7.31 (d, J = 8.6 Hz, 1H), 7.13 (s, 1H), 5.51 (dd, J = 10.3, 8.1 Hz, 1H), 5.44 (d, J = 3.2 Hz, 1H), 5.14 - 5.00 (m, 3H), 4.61 - 4.50 (m, 2H), 4.22 (dd, J = 11.2, 7.0 Hz, 1H), 4.15 (dd, J = 11.6, 6.6 Hz, 1H), 3.94 (s, 2H), 3.84 (t, J = 4.8 Hz, 2H), 3.70 - 3.61 (m, 6H), 3.58 (s, 4H), 3.51 (t, J = 5.3 Hz, 2H), 3.48 - 3.41 (m, 2H), 3.41 - 3.35 (m, 2H), 3.16 (dd, J = 15.1, 3.2 Hz, 1H), 3.03 (dd, J = 15.0, 8.8 Hz, 3H), 2.16 (s, 3H), 2.10 (s, 3H), 2.05 (s, 3H), 1.99 (s, 3H).

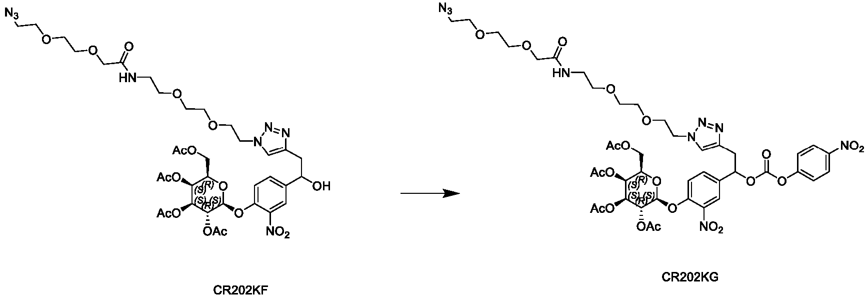

**CR202KF** (2.00 g, 2.27 mmol) was dissolved in anhydrous DMAc (60 mL) before NPC (1.38 g, 4.53 mmol) was added. Then DIPEA (1.2 mL, 6.81 mmol) was added, and the reaction system was stirred for 17 h. 300 mL of an acetonitrile/0.1% TFA water = 1/1 reagent was dropwise added with the temperature controlled at <20 °C. The mixture was purified by preparative chromatography and lyophilized to give **CR202KG** (1.65 g, 98.3% purity, 67.5% yield). LC-MS: [M+H]⁺:1048.29.

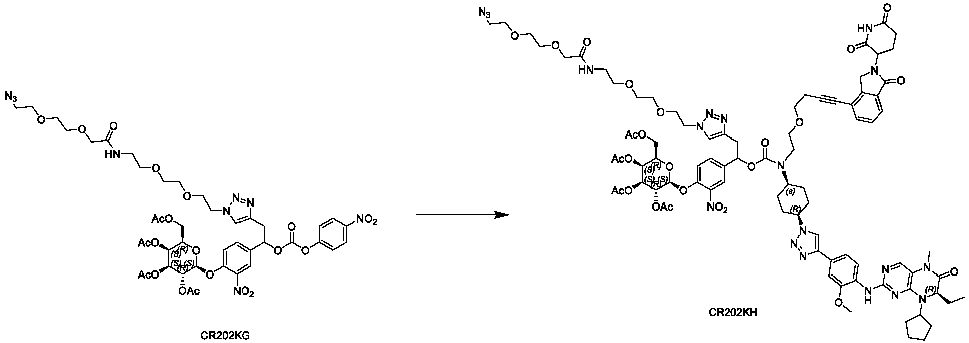

**CR202KG** (0.82 g, 0.76 mmol) was dissolved in anhydrous DMAc (13 mL). The reaction was cooled to an internal temperature of 10 °C in an ice bath, before **CR20274** (0.74 g, 0.84 mmol) and HOBT (0.1032 g, 0.76 mmol) were added. Then DIPEA (0.16 mL) was added, the ice bath was removed, and the reaction system was stirred for 3.5 h. The reaction mixture was purified and lyophilized to give **CR202KH** (1.0781 g, 98.23% purity, 78.7% yield). LC-MS: [M+2]²⁺: 897.21.

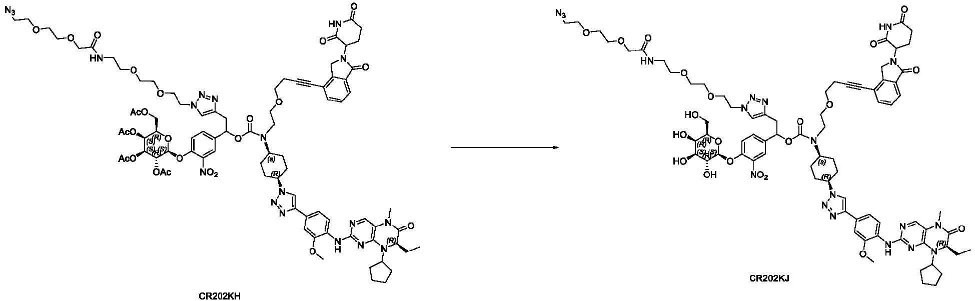

**CR202KH** (0.50 g, 0.28 mmol) was dissolved in methanol (50 mL). The system was cooled to 10 °C before 0.1 M LiOH (25 mL) was dropwise added. The reaction system was incubated at 10 to 15 °C for 25 min, until on-line detection indicated the completion of the reaction. The reaction mixture was adjusted to pH 5 by dropwise adding 5% AcOH, purified, and lyophilized to give **CR202KJ** (0.24 g, 98.84% purity, 45.3% yield). LC-MS: [M+2]²⁺: 813.18.

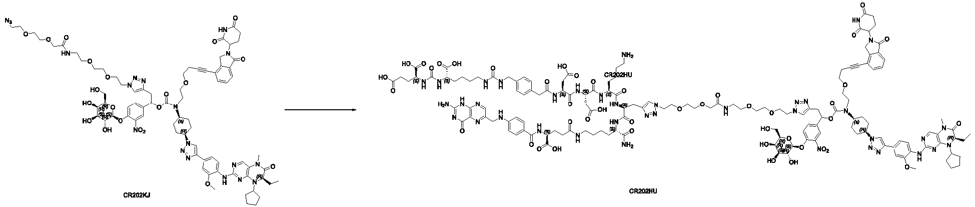

**CR202KJ** (0.22 g, 0.135 mmol) and CR202W4 (0.25 g, 0.162 mmol) were dissolved in DMF:H₂O = 1:1 (6.6 mL). At 20 °C, the system was adjusted to pH 8.0 by dropwise adding DIPEA. CuBr (20.1 mg, 0.135 mmol) was added, and the reaction system was stirred for 1.5 h, until on-line HPLC indicated the completion of the reaction. The reaction mixture was cooled to 0 °C and adjusted to pH 3 by dropwise adding 2% TFA. The mixture was purified by preparative chromatography to give **CR202HU** (0.11 g, 95.3% purity, 26.2% yield).

### 4.24 Synthesis of CR202HR

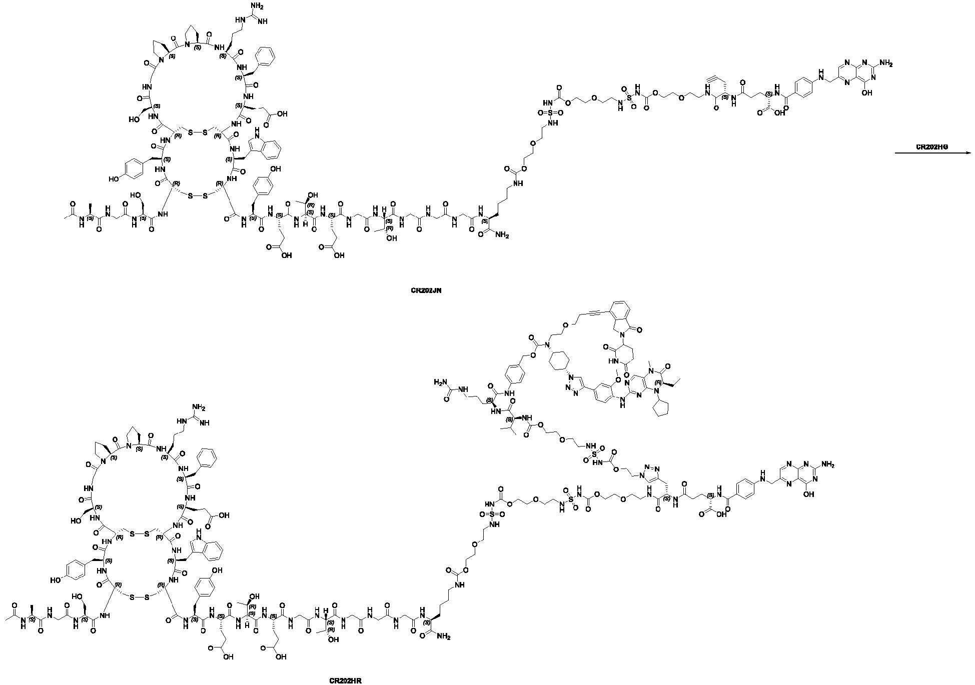

**CR202JN** (520.0 mg, 0.135 mmol) was dissolved in DMF/water (9/1, 40 mL), before **CR202HG** (250.0 mg, 0.155 mmol) and CuBr (48.4 mg, 0.337 mmol) was added. The reaction system was purged with nitrogen thrice, adjusted to pH 7, and stirred with a magnetic stirrer for 3 to 5 h with the reaction temperature controlled at 25 °C, until on-line HPLC monitoring indicated the completion of the reaction. The reaction was terminated, and the reaction mixture was purified by preparative chromatography and lyophilized to give a yellow solid powder **CR202HR** (358.6 mg, 97.78% purity, 40.75% yield). [M/4+H]⁺=1367.50.

### 4.25 Synthesis of CR202MT

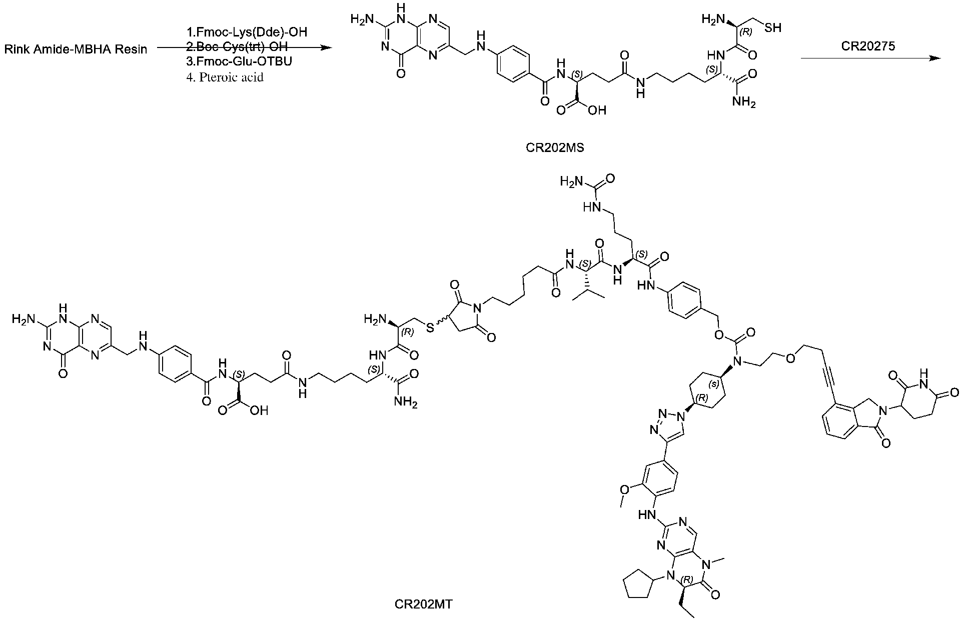

A yellow solid **CR202MS** was prepared using a method similar to L-01.

**CR20275** was prepared using a method similar to **CR20282.**

**CR20275** (150.1 mg, 0.10 mmol) and **CR202MS** (81.2 mg, 0.12 mmol) were sequentially added into 50% ACN/H₂O (15 mL). The reaction system was adjusted to pH 4 to 6 by adding 10 mmol/L Na₂HPO₄, and incubated at 25 °C for 1 h, until on-line HPLC monitoring indicated the completion of the reaction. The reaction mixture was directly purified by preparative chromatography to give **CR202MT** (90.0 mg, 93.46% purity, 41.3% yield).

### Example 2 Solubility study

General procedures for solubility study: PBS (pH 7.2 to 7.4) was added to the conjugate compounds (1.0 mg) and the proteolysis targeting chimera alone in portions at 0.05 mL each time. The mixture was vortexed for 2 min until the solid was dissolved. The solubility was calculated as: C = m/V, C: concentration, m: sample weight, V: PBS total volume.

**Table 1. Solubility**

| Compound | CR202V5 | CR202V6 | CR202V7 | CR202W4 | CR20282 | CR20293 | CR20295 | CR20297 |
|---|---|---|---|---|---|---|---|---|
| Concentration, mg/mL | 2.8 | 1.9 | 1.5 | 2.8 | 13.4 | 20.6 | 26.4 | 10.6 |

| Compound | CR202Z6 | CR202V4 | CR202V8 | CR202V9 | CR202AL | CR202W1 | CR20274 | |
|---|---|---|---|---|---|---|---|---|
| Concentration, mg/mL | 2.7 | 8.0 | 7.5 | 15.8 | 5.7 | 8.1 | < 0.9 | |

Conclusion: As can be seen from the above table, the solubilities of the conjugate compounds are significantly higher than that of the unconjugated proteolytic degradation chimera (i.e., CR20274).

### Example 3 Enzymatic cleavage study

### 1. Cathepsin B cleavage study for CR20282, CR20293, CR20295, and CR20297

Sample information (cleavage study): CR20282, CR20293, CR20295, CR20297, cathepsin B-human liver (source: Sigma), cysteine (source: Sigma), sodium acetate, and EDTA (Shanghai Lingfeng).

Procedures: The conjugate compounds were dissolved in acetonitrile-water (1:1, containing 0.1% TFA) to prepare 1 mg/mL stock solutions; 30 µL of the 1 mg/mL stock solution was added to 570 µL of 50 mM NaOAc (containing 1 mM EDTA and 10 mM cysteine), and the mixture was mixed well; 7 µL of cathepsin B (0.05 U/µL) was added, and the mixture was gently mixed evenly; the reaction system was transferred into a 37 °C thermostat for 0, 1, 2, 4, or 8 h of incubation; samples were taken at each time point for HPLC analysis.

Results and analysis: As can be seen from Tables 2 and 3, with the cleavage of cathepsin B, the agents exhibited good payload release rates.

**Table 2. Principal component residue of conjugate compounds after cleavage**

| **Time** | **CR20282** | **CR20293** | **CR20295** | **CR20297** |
|---|---|---|---|---|
| 0 h | 87% | 100% | 100% | 100% |
| 1 h | 17% | 76% | 46% | 55% |
| 2 h | 8% | 46% | 22% | 27% |
| 4 h | 2% | 27% | 8% | 10% |
| 8 h | 0% | 7% | 0% | 0% |

**Table 3. CR20274 release rate of conjugate compounds after cleavage**

| **Time point** | **CR20282** | **CR20293** | **CR20295** | **CR20297** |
|---|---|---|---|---|
| 0 h | 9% | 0% | 0% | 0% |
| 1 h | 79 % | 17% | 15% | 21% |
| 2 h | 86% | 38% | 16% | 40% |
| 4 h | 89% | 59% | 18% | 55% |
| 8 h | 86% | 64% | 24% | 61% |

### 2. Cathepsin B cleavage rate study for CR202V4, CR202V5, CR202V6, CR202V7, and CR202Z6

Sample information (cleavage study): CR202V4, CR202V5, CR202V6, CR202V7, CR202Z6, cathepsin B-human liver (source: Sigma), cysteine (source: Sigma), sodium acetate, and EDTA (source: Shanghai Lingfeng).

### Procedures:

1) The conjugate compounds were prepared into 500 µg/mL stock solutions with 0.1% TFA-acetonitrile water (1:1);
2) 60 µL of 500 µg/mL compound stock solution was added to 536 µL of 50 mM NaOAc (containing 1 mM EDTA + 10 mM Cys), before 4 µL of cathepsin B (CB enzyme) was added, and the mixture was well mixed by gentle shaking.
3) The cleavage reaction system in step 2) was transferred to a thermostatic incubator at 37 °C for 0 h, 1 h, 2 h, 4 h, or 24 h of incubation. Samples were taken and loaded at various time points for LC-MS analysis.

Results and analysis: As can be seen from Tables 4 and 5, with the cleavage of CB enzyme, the agents exhibited good payload release rates.

**Table 4. Principal component residue of compounds after cleavage**

| **Time** | **CR202V4** | **CR202V5** | **CR202V6** | **CR202V7** | **CR202Z6** |
|---|---|---|---|---|---|
| 0 h | 100.00% | 100.00% | 100.00% | 100.00% | 100.00% |
| 1 h | 0.00% | 2.37% | 11.20% | 30.64% | 6.89% |
| 2 h | 0.00% | 0% | 2.66% | 13.69% | 0.58% |
| 4 h | 0.00% | 0% | 0% | 3.70% | 0.41% |
| 24 h | 0.00% | 0% | 0% | 0% | 0% |

**Table 5. CR20274 release rate of compounds after cleavage**

| Time | **CR202V4** | **CR202V5** | **CR202V6** | **CR202V7** | **CR202Z6** |
|---|---|---|---|---|---|
| 0 h | 0% | 0% | 0% | 0% | 0% |
| 1 h | 100% | 100% | 6% | 57% | 100% |
| 2 h | 100% | 100% | 88% | 89% | 100% |
| 4 h | 100% | 100% | 100% | 100% | 100% |
| 24 h | 100% | 100% | 100% | 100% | 100% |

### 3. Cleavage rate study for CR202V8 and CR202W1

The compound structure stability of conjugate compounds CR202V8 (containing plasmin-cleavable groups) and CR202W1 (containing sulfatase-cleavable groups) was observed by cleavage with plasmin or sulfatase.

### Procedures:

1) The compounds were formulated into 500 µg/mL stock solutions with PBS (pH 7.4);
2) 60 µL of 500 µg/mL compound stock solution was added to 420 µL PBS (pH 7.4), before 60 µL of 0.2 U/mL plasmin (or 60 µL of 0.5 U/mL sulfatase) was added, and the mixture was well mixed by gentle shaking;
3) The cleavage reaction system in step 2) was transferred to a thermostatic incubator at 37 °C for 0 h, 1 h, 2 h, 4 h, or 24 h of incubation. Samples were taken and loaded at various time points for LC-MS analysis.

Results and analysis: As can be seen from Tables 6 and 7, CR202V8 and CR202W1 exhibited good payload release rates. CR202V8 has a higher payload release rate compared to CR202W1.

**Table 6. Principal component residue of compounds after cleavage**

| **Time** | CR202V8 | CR202W1 |
|---|---|---|
| 0 h | 8% | 97% |
| 1 h | 1% | 89% |
| 2 h | 1% | 80% |
| 4 h | 0% | 69% |
| 24 h | 0% | 37% |

**Table 7. CR20274 release rate of compounds after cleavage**

| **Time** | CR202V8 | CR202W1 |
|---|---|---|
| 0 h | 22% | 0% |
| 1 h | 100% | 9% |
| 2 h | 100% | 15% |
| 4 h | 100% | 27% |
| 24 h | 100% | 56% |

### 4. β-Glucosidase cleavage rate study for CR202AL

The compound structure stability of conjugate compound CR202AL was observed by cleavage with β-glucosidase, and the presence of other cleaved fragments was observed to determine the presence of other cleavage sites.

### Procedures:

1) CR202AL was formulated into a 500 µg/mL stock solution with PBS (pH 6.8);
2) 100 µL of 500 µg/mL compound stock solution was added to 775 µL of PBS (pH = 6.8) before 125 µL of 100 U/mL β-glucosidase was added, and the mixture was well mixed by gentle shaking.
3) The cleavage reaction system in step 2) was transferred to a thermostatic incubator at 25 °C for 0 h, 1 h, 2 h, 4 h, or 24 h of incubation. Samples were taken and loaded at various time points for LC-MS analysis.

Results and analysis: As can be seen from Tables 8 and 9, with the cleavage of β-glucosidase, CR202AL exhibited a good payload release rate.

**Table 8. Principal component residue of CR202AL after cleavage**

| **Time** | CR202AL |
|---|---|
| 0 h | 100.00% |
| 1 h | 26.70% |
| 2 h | 9.75% |
| 4 h | 4.87% |
| 24 h | 0.00% |

**Table 9. CR20274 release rate of CR202AL after cleavage**

| **Time** | CR202AL |
|---|---|
| 0 h | 5.16% |
| 1 h | 107.33% |
| 2 h | 146.03% |
| 4 h | 146.54% |
| 24 h | 100.80% |

### Example 4 Plasma stability study

### 1. Plasma stability study of CR20282, CR20285, CR20293, CR20295, and CR20297

Sample information: CR20282, CR20285, CR20293, CR20295, CR20297, methanol, and mouse plasma.

Procedures: The conjugate compounds were dissolved in acetonitrile-water (1:1) or acetonitrile-water (1:1, containing 0.1% TFA) to prepare 1 mg/mL stock solutions; 10 µL of the 1 mg/mL stock solution was added to 90 µL of plasma, and the mixture was gently mixed well. The plasma drug samples were shaken and incubated in a 37 °C thermostat for 0, 0.5, 1, 2, 4, 6, or 24 h; at the time points, the samples were taken out and 300 µL of methanol solution (containing 10 ng/mL of terfenadine) was added for protein precipitation; the mixture was centrifuged (12 min, 4 °C, 12000 rpm) and the supernatant was collected, and preserved in a -80 °C refrigerator; after collection, the samples were loaded for LC-MS analysis.

Results and analysis: As can be seen from Tables 10 and 11, the conjugate compounds are stable in plasma, with less payload release in plasma and a lower rate.

**Table 10. Principal component residue of compounds in plasma stability study**

| **Time** | **CR20282** | **CR20285** | **CR20293** | **CR20295** | **CR20297** |
|---|---|---|---|---|---|
| 0 h | 86.91% | 80.03% | 83.13% | 92.38% | 85.14% |
| 0.5 h | 78.98% | 73.49% | 75.57% | 85.39% | 87.68% |
| 1 h | 80.58% | 70.41% | 75.83% | 72.91% | 77.46% |
| 2 h | 67.29% | 68.31% | 67.76% | 71.15% | 74.50% |
| 4 h | 54.93% | 63.85% | 69.79% | 64.95% | 79.71% |
| 6 h | 55.92% | 60.08% | 70.46% | 41.82% | 43.25% |
| 24 h | 15.25% | 26.68% | 45.43% | 20.02% | 22.96% |

**Table 11. CR20274 release rate of compounds in plasma stability study**

| **Time** | **CR20282** | **CR20285** | **CR20293** | **CR20295** | **CR20297** |
|---|---|---|---|---|---|
| 0 h | 0.01% | 0.20% | 0.00% | 0.02% | 0.05% |
| 0.5 h | 0.27% | 0.56% | 0.17% | 0.09% | 0.13% |
| 1 h | 0.36% | 0.52% | 0.32% | 0.10% | 0.15% |
| 2 h | 0.41% | 0.80% | 0.52% | 0.19% | 0.38% |
| 4 h | 0.50% | 1.39% | 0.67% | 0.31% | 0.47% |
| 6 h | 0.65% | 1.51% | 0.95% | 0.19% | 0.38% |
| 24 h | 0.84% | 3.09% | 3.38% | 0.71% | 1.34% |

### 2. Plasma stability study of CR202V4, CR202V5, CR202V6, CR202V7, CR202V8, CR202V9, CR202Z6, CR202W1, CR202AL, and CR20282

Sample information: CR202V4, CR202V5, CR202V6, CR202V7, CR202V8, CR202V9, CR202Z6, CR202W1, CR202AL, CR20282, methanol, and mouse plasma.

### Procedures:

1) The conjugate compounds were prepared into 1 mg/mL stock solutions with 0.1% TFA-acetonitrile water (1:1);
2) 10 µL of 1 mg/mL compound stock solution was added into 90 µL of fresh plasma, and the mixture was well mixed by gentle shaking, and transferred to a thermostatic incubator at 37 °C for 0 h, 0.5 h, 1 h, 2 h, 4 h, 6 h, or 24 h of incubation;
3) at the time points, samples were taken out and 300 µL of methanol solution (containing 10 ng/mL of terfenadine) was added for protein precipitation;
4) the mixture was centrifuged (13 min, 4 °C, 12000 rpm) and the supernatant was collected, and preserved in a -80 °C refrigerator; after collection, the samples were loaded for LC-MS analysis.

Results and analysis: As can be seen from Tables 12 and 13, the conjugate compounds are stable in plasma, with less payload release in plasma and a lower rate.

**Table 12. Principal component residue of compounds in plasma stability study**

| **Time** | **Residual rate of principal component (CR202V4)** | **Residual rate of principal component (CR202V5)** | **Residual rate of principal component (CR202V6)** | **Residual rate of principal component (CR202V7)** | **Residual rate of principal component (CR202V8)** | **Residual rate of principal component (CR202V9)** | **Residual rate of principal component (CR202Z6)** | **Residual rate of principal component (CR202W1)** | **Residual rate of principal component (CR202AL)** | **Residual rate of principal component (CR20282)** |
|---|---|---|---|---|---|---|---|---|---|---|
| 0h | 84.13% | 94.75% | 91.24% | 88.59% | 78.37% | 99.22% | 105.34% | 105.30% | 76.04% | 109.94% |
| 0.5h | 75.91% | 100.98% | 87.89% | 80.93% | 50.90% | 99.85% | 113.62% | 89.44% | 69.77% | 95.98% |
| 1h | 74.31% | 94.88% | 87.91% | 86.63% | 59.93% | 98.41% | 111.67% | 102.61% | 78.07% | 82.50% |
| 2h | 78.19% | 94.66% | 87.59% | 75.50% | 49.50% | 89.57% | 80.38% | 105.09% | 75.61% | 83.10% |
| 4h | 61.27% | 90.56% | 82.48% | 63.89% | 41.21% | 67.13% | 88.43% | 107.80% | 74.87% | 74.33% |
| 6h | 70.38% | 80.42% | 80.40% | 66.20% | 32.46% | 66.29% | 98.63% | 84.83% | 57.09% | 66.72% |
| 24h | 1.36% | -1.03% | 0.00% | -2.63% | -2.40% | 0.62% | 4.82% | -2.67% | 1.20% | -2.82% |

**Table 13. CR20274 release rate of compounds**

| **Time** | **CR20274 release rate of CR202V4 (actual release/ theoretical release)** | **CR20274 release rate of CR202V5 (actual release/ theoretical release)** | **CR20274 release rate of CR202V6 (actual release/ theoretical release)** | **CR20274 release rate of CR202V7 (actual release/ theoretical release)** | **CR20274 release rate of CR202V8 (actual release/ theoretical release)** | **CR20274 release rate of CR202V9 (actual release/ theoretical release)** | **CR20274 release rate of CR202Z6 (actual release/ theoretical release)** | **CR20274 release rate of CR202W1 (actual release/ theoretical release)** | **CR20274 release rate of CR202AL (actual release/ theoretical release)** | **CR20274 release rate of CR20282 (actual release/ theoretical release)** |
|---|---|---|---|---|---|---|---|---|---|---|
| 0h | -0.12% | 0.02% | 0.03% | -0.02% | 0.06% | 0.11% | 0.05% | 0.68% | 0.01% | 0.00% |
| 0.5h | 0.04% | 0.17% | 0.07% | 0.15% | 1.94% | 0.17% | 0.22% | 0.63% | 0.47% | 0.34% |
| 1h | 0.11% | 0.25% | 0.15% | 0.24% | 5.56% | 0.22% | 0.34% | 0.83% | 0.86% | 0.48% |
| 2h | 0.17% | 0.37% | 0.26% | 0.28% | 7.07% | 0.21% | 0.25% | 0.80% | 0.96% | 0.84% |
| 4h | 0.30% | 0.94% | 0.47% | 0.37% | 14.20% | 0.25% | 0.37% | 0.96% | 2.60% | 1.17% |
| 6h | 0.42% | 0.97% | 0.52% | 0.49% | 14.58% | 0.30% | 0.48% | 0.88% | 3.69% | 1.60% |
| 24h | 2.62% | 3.92% | 2.47% | 3.25% | 105.58% | 1.46% | 1.95% | 2.82% | 28.17% | 8.18% |

### Example 5 Expansion inhibition study of different tumor cells by conjugate compound CR20214

Information on cell lines

**Table 14**

| **Cell line** | **Type** |
|---|---|
| A549 | Human non-small cell lung cancer cell |
| HCC1954 | Human ductal carcinoma cell |
| HCT-116 | Human colorectal cancer cell |
| SK-BR-3 | Human breast adenocarcinoma cell |
| U87MG | Human brain astroblastoma cell |
| NCI-H460 | Human large cell lung cancer cell |
| RT4 | Human bladder transitional cell papilloma cell |
| KB | Human oral epidermoid carcinoma cell |
| SCLC-21H | Human small cell lung cancer cell |
| LNCaP | Human prostate cancer cell |
| Hela | Human cervical cancer cell |
| 293T | Human embryonic kidney cell |
| 293T-FOLR1 | Human embryonic kidney cell overexpressing FOLR1 |
| 293T-FOLR1/PSMA | Human embryonic kidney cell overexpressing FOLR1 and PSMA |

Major reagents: 1640 folic acid-free medium, fetal bovine serum, penicillin-streptomycin solution, L-glutamine, and CCK8.

### Procedures

### 1) Cell plating

Cells were prepared in advance, trypsinized, collected, counted, diluted to 2 × 10⁴ cells/mL in a complete cell culture medium, and plated in 96-well plates. The diluted cell suspension was added at 100 µL/well, and negative and blank control wells were set on each plate. The 96-well plates with the added cells were incubated overnight at 37 °C in a 5% CO₂ incubator.

### 2) Dilution and treatment

Samples were serially diluted with medium (serially 5-fold diluted from 10 µM) to 9 concentrations. The medium in the plates was discarded, and the drug solutions were added at 100 µL per well to a 96-well plate in triplicate. Negative and blank controls were set. The plates were incubated at 37 °C for 72 h in a 5% CO₂ incubator.

### 3) Chromogenesis and plate reading

A CCK-8 developing solution was added at 10 µL/well (10% of the liquid volume in the wells). The plates were incubated at 37 °C for a proper time (the OD value was controlled within the range of 1.0-2.5 as possible). The plate covers were removed, and the 96-well plates were loaded on a microplate reader (Molecular Devices SpectraMax iD5) to read the measurement at 450 nm.

### 4) Data processing

Four-parameter curves were fitted using GraphPad Prism 8.0.2 to give IC₅₀ values.

### 5) Results and analysis

Results: As shown in Table 15, CR20214 exerts a killing effect on various tumor cells.

**Table 15. IC₅₀ of compounds (nM)**

| **Cell line** | **CR20214** |
|---|---|
| A549 | 1603 |
| HCC1954 | 3968 |
| HCT-116 | 1117 |
| SK-BR-3 | 1489 |
| U87MG | 907.3 |
| NCI-H460 | 1595 |
| RT4 | 275.5 |
| KB | 422.2 |
| SCLC-21H | 1795 |
| LNCaP | 52.23 |
| Hela | 950.2 |
| 293T | 2023 |
| 293T-FOLR1 | 692.7 |
| 293T-FOLR1/PSMA | 845.2 |

### Example 6 Expansion inhibition study of different tumor cells by conjugate compounds CR20282, CR20285, CR20287, CR20293, CR20295, CR20297, and CR202F0

Information on cell lines

**Table 16**

| **Cell line** | **Type** | **FOLR1/PSMA expression** |
|---|---|---|
| LNCaP | Human prostate cancer cell | PSMA overexpression |
| 22RV1 | Human prostate cancer cell | PSMA overexpression |
| C4-2 | Human prostate cancer cell | PSMA overexpression |
| MDA-MB-231-FOLR1 | Human breast cancer cell stably expressing FOLR1 | FOLR1 overexpression |
| MDA-MB-231-PSMA | Human breast cancer cell stably expressing PSMA | PSMA overexpression |
| 293T-PSMA | Human embryonic kidney cell | PSMA overexpression |

Major reagents: 1640 folic acid-free medium, fetal bovine serum, penicillin-streptomycin solution, L-glutamine, and CCK8.

### Procedures

### 1) Cell plating

Cells were prepared in advance, trypsinized, collected, counted, diluted to 2 × 10⁴ cells/mL in a complete cell culture medium, and plated in 96-well plates. The diluted cell suspension was added at 100 µL/well, and negative and blank control wells were set on each plate. The 96-well plates with the added cells were incubated overnight at 37 °C in a 5% CO₂ incubator.

### 2) Dilution and treatment

Samples were serially diluted with medium (serially 5-fold diluted from 10 µM) to 9 concentrations. The medium in the plates was discarded, and the drug solutions were added at 100 µL per well to a 96-well plate in triplicate. Negative and blank controls were set. The plates were incubated at 37 °C for 72 h in a 5% CO₂ incubator.

### 3) Chromogenesis and plate reading

A CCK-8 developing solution was added at 10 µL/well (10% of the liquid volume in the wells). The plates were incubated at 37 °C for a proper time (the OD value was controlled within the range of 1.0-2.5 as possible). The plate covers were removed, and the 96-well plates were loaded on a microplate reader (Molecular Devices SpectraMax iD5) to read the measurement at 450 nm.

### 4) Data processing

Four-parameter curves were fitted using GraphPad Prism 8.0.2 to give IC₅₀ values.

### 5) Results and analysis

Results: As shown in Table 17, CR20282, CR20285, CR20293, CR20295, and CR20297 have strong killing effects on various tumor cells expressing FOLR1 and PSMA.

**Table 17. IC₅₀ of compounds (nM)**

| IC₅₀ (nM) | LNCap | 22RV1 | C4-2 | MDA-MB-231-FOLR1 | MDA-MB-231-PSMA | 293T-PSMA |
|---|---|---|---|---|---|---|
| CR20282 | 73.95 | 98.49 | 212.4 | 206.9 | 374.3 | 85.64 |
| CR20295 | 10.58 | 3.239 | 5.074 | 13.89 | 8.695 | 85.64 |
| CR20293 | 37.91 | 16.43 | 77.98 | 94.15 | 70.63 | 3267 |
| CR20297 | 178 | 71.89 | 128.8 | 258.2 | 313.9 | 5827 |
| CR20285 | 44.59 | 17.53 | 40.67 | 49.2 | 85.81 | 6047 |
| CR202F0 | 1483 | 367.7 | 1121 | 10808 | 5951 | 12661 |
| CR20287 | 1259 | 449 | 882.5 | 1630 | 2040 | 3314 |

### Example 7 Expansion inhibition study of different tumor cells by compound CR20274 and conjugate compound CR20282

Information on cell lines

**Table 18**

| **Cell line** | **Type** | **FOLR1/PSMA expression** |
|---|---|---|
| HepG2 | Human liver cancer cell | No detectable FOLR1 or PSMA expression |
| PC-3 | Human prostate cancer cell | No detectable FOLR1 or PSMA expression |
| A549 | Human non-small cell lung cancer cell | No detectable FOLR1 or PSMA expression |
| NCI-H460 | Human large cell lung cancer cell | No detectable FOLR1 or PSMA expression |

Major reagents: 1640 folic acid-free medium, fetal bovine serum, penicillin-streptomycin solution, L-glutamine, and CCK8.

### Procedures

### 1) Cell plating

Cells were prepared in advance, trypsinized, collected, counted, diluted to 2 × 10⁴ cells/mL in a complete cell culture medium, and plated in 96-well plates. The diluted cell suspension was added at 100 µL/well, and negative and blank control wells were set on each plate. The 96-well plates with the added cells were incubated overnight at 37 °C in a 5% CO₂ incubator.

### 2) Dilution and treatment

Samples were serially diluted with medium (serially 5-fold diluted from 10 µM) to 9 concentrations. The medium in the plates was discarded, and the drug solutions were added at 100 µL per well to a 96-well plate in triplicate. Negative and blank controls were set. The plates were incubated at 37 °C for 72 h in a 5% CO₂ incubator.

### 3) Chromogenesis and plate reading

A CCK-8 developing solution was added at 10 µL/well (10% of the liquid volume in the wells). The plates were incubated at 37 °C for a proper time (the OD value was controlled within the range of 1.0-2.5 as possible). The plate covers were removed, and the 96-well plates were loaded on a microplate reader (Molecular Devices SpectraMax iD5) to read the measurement at 450 nm.

### 4) Data processing

Four-parameter curves were fitted using GraphPad Prism 8.0.2 to give IC₅₀ values.

### 5) Results and analysis

Results: Are shown in Table 19, CR20274 possesses a strong killing effect on cells with no PSMA or FOLR1 expression, while CR20282 has almost no killing effect on cells with no PSMA or FOLR1 expression. This indicates that when FOLR1 and PSMA are absent on the cell membrane surface, the targeting delivery of dual-ligand PROTACs is blocked, suggesting that dual-ligand PROTACs have better targeting properties than unconjugated PROTAC drugs.

**Table 19. IC₅₀ of compounds (nM)**

| **IC₅₀ (nM)** | **HepG2** | **PC-3** | **A549** | **NCI-H460** |
|---|---|---|---|---|
| CR20274 | 106.8 | 103.2 | 124.2 | 314.2 |
| CR20282 | / | / | / | / |

### Example 8 Expansion inhibition study of different tumor cells by conjugate compounds CR202V4, CR202V5, CR202V6, CR202V7, CR202V9, CR202W1, CR202Z6, CR202AL, CR202GM, CR202GQ, CR202FU, CR202HJ, CR202JG, CR202HU, CR202HV, and CR202V8

Information on cell lines: 22RV1 (human prostate cancer cell), C4-2 (human prostate cancer cell), and MDA-MB-231 (human breast cancer cell)

Major reagents: 1640 folic acid-free medium, fetal bovine serum, penicillin-streptomycin solution, L-glutamine, and CellTiter-Glo^{®} Luminescent Cell Viability Assay reagent.

### Procedures:

### 1) Cell plating

Cells were prepared in advance, trypsinized, collected, counted, diluted to 1.5 × 10⁴ cells/mL in a complete cell culture medium, and plated in 96-well plates. The diluted cell suspension was added at 100 µL/well, and negative and blank control wells were set on each plate. The 96-well plates with the added cells were incubated overnight at 37 °C in a 5% CO₂ incubator.

### 2) Dilution and treatment

Samples were serially diluted with medium (serially 3-fold diluted from 10 µM) to 9 concentrations. The medium in the plates was discarded, and the drug solutions were added at 100 µL per well to a 96-well plate in triplicate. Negative and blank controls were set. The plates were incubated at 37 °C for 72 h in a 5% CO₂ incubator.

### 3) Cell viability assay by CellTiter-Glo luminescence method

The CellTiter-Glo buffer was thawed and warmed to room temperature, and the CellTiter-Glo substrate was warmed to room temperature. The CellTiter-Glo buffer was added to the flask containing CellTiter-Glo substrate to dissolve the substrate, thereby preparing a CellTiter-Glo working solution, which was then vortexed slowly to complete dissolution. The cell culture plates were equilibrated at room temperature for 30 min, before the CellTiter-Glo working solution was added at 50 µL/well (about half the volume of cell culture medium in each well). The plate was covered by aluminum foil to keep out of light. The culture plate was shaken on an orbital shaker for 2 min to induce cell lysis. The culture plate was left to stand at room temperature for 10 min to stabilize the luminescence signals. The luminescence signal was detected on a microplate reader (Molecular Devices SpectraMax iD5).

### 4) Data processing

Four-parameter curves were fitted using GraphPad Prism 8.0.2 to give IC₅₀ values.

### 5) Results and analysis

Results: As shown in Table 20, conjugate compounds CR202V4, CR202V5, CR202V6, CR202V7, CR202V9, CR202W1, CR202Z6, CR202AL, CR202GM, CR202GQ, CR202FU, CR202HJ, CR202JG, CR202HU, CR202HV, and CR202V8 have good tumor inhibitory effects on various tumor cells.

**Table 20. IC₅₀ of compounds (nM)**

| Compound | Cell line | | |
|---|---|---|---|
| | 22RV1 | C4-2 | MDA-MB-231 |
| CR202V4 | 221.8 | 84.7 | 233.7 |
| CR202V5 | 779.3 | 143.8 | 412.2 |
| CR202V6 | 3.0 | 4.6 | 2.7 |
| CR202V7 | 103.3 | 37.1 | 128.1 |
| CR202V9 | 118.0 | 44.0 | 324.5 |
| CR202W1 | 111.5 | 43.2 | 120.2 |
| CR202Z6 | 370.4 | 272.5 | 344.4 |
| CR202AL | 6.3 | 0.5 | 2.5 |
| CR202GM | 286.7 | / | 512.1 |
| CR202GQ | 3162 | / | 4475 |
| CR202FU | 468.7 | / | 791.9 |
| CR202HJ | 1215 | / | 3653 |
| CR202JG | 1098 | / | 3779 |
| CR202HU | 1.52 | / | 5.5 |
| CR202HV | 154.6 | / | 14.19 |
| CR202V8 | 20.47 | / | 24.07 |

| | | | |
|---|---|---|---|
| Note: "/" denotes no detection. | | | |

### Example 9 Expansion inhibition study of different tumor cells by conjugate compounds CR202W5, CR202W8, CR202W9 and CR202W6, and unconjugated proteolytic degradation chimeras CR202Z5, CR202Z8, CR202Z9 and CR202ES

Information on cell lines: LNCaP (human prostate cancer cell) and VCaP (human prostate cancer cell)

Major reagents: 1640 folic acid-free medium, fetal bovine serum, penicillin-streptomycin solution, L-glutamine, and CellTiter-Glo^{®} Luminescent Cell Viability Assay reagent.

### Procedures:

### 6) Cell plating

Cells were prepared in advance, trypsinized, collected, counted, diluted to 1.5 × 10⁴ cells/mL in a complete cell culture medium, and plated in 96-well plates. The diluted cell suspension was added at 100 µL/well, and negative and blank control wells were set on each plate. The 96-well plates with the added cells were incubated overnight at 37 °C in a 5% CO₂ incubator.

### 7) Dilution and treatment

Samples were serially diluted with medium (serially 3-fold diluted from 10 µM) to 9 concentrations. The medium in the plates was discarded, and the drug solutions were added at 100 µL per well to a 96-well plate in triplicate. Negative and blank controls were set. The plates were incubated at 37 °C for 72 h in a 5% CO₂ incubator.

### 8) Cell viability assay by CellTiter-Glo luminescence method

The CellTiter-Glo buffer was thawed and warmed to room temperature, and the CellTiter-Glo substrate was warmed to room temperature. The CellTiter-Glo buffer was added to the flask containing CellTiter-Glo substrate to dissolve the substrate, thereby preparing a CellTiter-Glo working solution, which was then vortexed slowly to complete dissolution. The cell culture plates were equilibrated at room temperature for 30 min, before the CellTiter-Glo working solution was added at 50 µL/well (about half the volume of cell culture medium in each well). The plate was covered by aluminum foil to keep out of light. The culture plate was shaken on an orbital shaker for 2 min to induce cell lysis. The culture plate was left to stand at room temperature for 10 min to stabilize the luminescence signals. The luminescence signal was detected on a microplate reader (Molecular Devices SpectraMax iD5).

### 9) Data processing

Four-parameter curves were fitted using GraphPad Prism 8.0.2 to give IC₅₀ values.

### 10) Results and analysis

Results: As shown in Table 21, compared with unconjugated proteolytic degradation chimeras CR202Z5, CR202Z8, CR202Z9, and CR202ES, conjugate compounds CR202W5, CR202W8, CR202W9, and CR202W6 have comparable or even better killing effects on various tumor cells.

**Table 21. IC₅₀ of compounds (nM)**

| Compound | Cell line | |
|---|---|---|
| | LNCaP | VCaP |
| CR202Z5 | 373.1 | 182.9 |
| CR202W5 | 442.9 | 75.3 |
| CR202Z8 | 1.5 | 1.5 |
| CR202W8 | 5.3 | 2.7 |
| CR202Z9 | 417.7 | 12.4 |
| CR202W9 | 72 | 180 |
| CR202ES | 8.7 | 10.9 |
| CR202W6 | 12.2 | 4.7 |

### Example 10 Expansion inhibition study of different tumor cells by compounds CR202CC, CR202CF, and CR202CI

Information on cell lines

**Table 22**

| **Cell line** | **Type** | **FOLR1/PSMA expression** |
|---|---|---|
| LNCaP | Human prostate cancer cell | PSMA overexpression |
| SK-OV-3 | Human ovarian cancer cell | FOLR1 overexpression |

Major reagents: 1640 folic acid-free medium, fetal bovine serum, penicillin-streptomycin solution, L-glutamine, and CCK8.

### Procedures

### 1) Cell plating

Cells were prepared in advance, trypsinized, collected, counted, diluted to 2 × 10⁴ cells/mL in a complete cell culture medium, and plated in 96-well plates. The diluted cell suspension was added at 100 µL/well, and negative and blank control wells were set on each plate. The 96-well plates with the added cells were incubated overnight at 37 °C in a 5% CO₂ incubator.

### 2) Dilution and treatment

Samples were serially diluted with medium (serially 5-fold diluted from 10 µM) to 9 concentrations. The medium in the plates was discarded, and the drug solutions were added at 100 µL per well to a 96-well plate in triplicate. Negative and blank controls were set. The plates were incubated at 37 °C for 72 h in a 5% CO₂ incubator.

### 3) Chromogenesis and plate reading

A CCK-8 developing solution was added at 10 µL/well (10% of the liquid volume in the wells). The plates were incubated at 37 °C for a proper time (the OD value was controlled within the range of 1.0-2.5 as possible). The plate covers were removed, and the 96-well plates were loaded on a microplate reader (Molecular Devices SpectraMax iD5) to read the measurement at 450 nm.

### 4) Data processing

Four-parameter curves were fitted using GraphPad Prism 8.0.2 to give IC₅₀ values.

### 5) Results and analysis

Results: As shown in Table 23, CR202CC, CR202CF, and CR202CI have strong killing effects on various tumor cells expressing FOLR1 and PSMA.

**Table 23. IC₅₀ of compounds (nM)**

| IC₅₀ (nM) | LNCap | SK-OV-3 |
|---|---|---|
| CR202CC | 0.3 | / |
| CR202CF | / | 31.5 |
| CR202CI | / | 0.3 |

### Examples 11 Assay of target protein degradation activity for CR20214, CR20295, CR202V6, and CR202AL

### Information on cell lines: MDA-MB-231 (human breast cancer cell) and 22RV1 (human prostate cancer cell)

Major reagents and consumables:

| Name | Manufacturer | Cat. No. |
|---|---|---|
| PBS(1×) | gibco | C100105OOBT |
| RIPA Lysis and Extraction Buffer | Thermo | 89901 |
| Protease Inhibitor Cocktail | bimake | B14001 |
| 4×Laemmli sample buffer | BIO-RAD | 161-0747 |
| Precision Plus Protein^{™} | BIO-RAD | 161-0377 |
| Tris-Glycine Transfer Buffer (pH8.3, 10×) | CoWin | CW0044S |
| TBS (pH8.0, 10×) | CoWin | CW0042 |
| Nonfat Dry Milk | CST | #9999 |
| GAPDH(D4C6R)Mouse mAb | CST | #97166 |
| Anti-mouse IgG, HRP-linked Antibody | CST | #7076 |
| Anti-rabbit IgG, HRP-linked Antibody | CST | #7074 |
| ExpressPlus^{™} PAGE Gels,10×8, 4-20%, 15 wells | Genscript | M42015C |
| MOPS Running Bffer Liquid(20×) | Genscript | M00680-500 |
| Clc-PARPity Max^{™} Western ECL Substrate | BIO-RAD | 1705062 |
| BCA protein quantification kit | TIANGEN | PA115 |

### Procedures:

### Extraction of protein samples:

1) A proper amount of RIPA lysis buffer was added to the Protease Inhibitor Cocktail (1:100) a few minutes before use.
2) The medium of the cells was discarded. The cells were collected by cell scraper, centrifuged, and washed twice with pre-cooled PBS. The lysis buffer was added at 300 µL per dish. The centrifuge tube was placed on ice and mixed by pipetting for 5 min to allow the lysis buffer to fully contact the cells.
3) After 30 min of lysis, the lysate was collected, transferred to a centrifuge tube, and centrifuged for 15 min at 12000× g to pelletize the cell debris.
4) The supernatant was transferred to a new centrifuge tube, for subsequent Western Blot.

### BCA concentration determination:

1) 100 µL of mercaptoethanol was added to 900 µL of 4× Laemmli sample buffer right before use.
2) Sample dilution: The lysis buffer, PBS, and 4× loading buffer were well mixed according to a protein amount of 20 to 40 µg/well and a sample loading amount of 15 to 20 µL/well.
3) Denaturing: The sample was heated for 5 min at 90 to 95 °C.

### ExpressPlus^{™} PAGE electrophoresis:

A proper amount of protein sample solution was added into each well. The marker protein solution was added to the selected wells as the electrophoresis mark. The electrophoresis was conducted at a constant voltage of 140 V until bromophenol blue ran to the bottom of the gel.

### Membrane transfer:

1) A nitrocellulose membrane was treated with methanol for 5 to 10 seconds and with purified water for 1 to 2 min, and the nitrocellulose membrane and thick filter papers were then equilibrated with a membrane-transferring buffer solution for 30 min separately;
2) The gel and the nitrocellulose membrane were clamped between two thick filter papers to form a "sandwich", which was transferred to a wet membrane transfer instrument and processed at a constant current of 300 mA for 2 h;
3) After the membrane transfer, the membrane was taken out by forceps, and marked for the convenience of the operator.

### Blocking:

The nitrocellulose membrane was washed with 1× TBST, immersed in a 5% Nonfat Dry Milk blocking buffer prepared in 1× TBST buffer, and incubated for 2 h at 20 to 25 °C. The membrane was then washed twice with 1× TBST buffer and once with 1× TBS solution for 5 min each.

### Incubation with primary antibody:

After blocking, the prepared primary antibody solution and the nitrocellulose membrane were added into the hybridization bag and incubated overnight at 4 °C. The membrane was then washed twice with 1× TBST buffer and once with 1× TBS solution for 5 min each.

### Incubation with secondary antibody:

The nitrocellulose membrane was co-incubated with the diluted secondary antibody for 1.5 h. The excess antibody solution was discarded, and the membrane was then washed twice with 1× TBST buffer and once with 1× TBS solution for 5 min each.

### Chromogenesis and exposure:

The antibody marked by HRP was coupled with an enhanced chemiluminescence reagent for chromogenesis, and the exposure was conducted via an X-ray film. The positions of the antigen-antibody (primary antibody)-antibody (secondary antibody) complex on the nitrocellulose membrane were determined by means of development, washing, and fixation. The bands on the X-ray film were scanned with a gel imaging system with GAPDH as the reference.

Results and analysis: As shown by the Western Blot results (see FIGs. 2-4), CR20214, CR20295, CR202V6, and CR202AL can rapidly degrade target proteins. Among these, CR20214 is capable of almost completely degrading BRD4 and PLK1 at 100 nM over 6 h (FIG. 2). CR20295 is capable of almost completely degrading BRD4 target protein at 100 nM over 6 h (FIG. 3A); at 10 nM, CR20295 is capable of almost completely degrading BRD4 over 24 h (FIG. 3B). At 3 nM, CR202V6 and CR202AL are capable of almost completely degrading BRD4 target protein over 6 h (FIG. 4A), showing excellent target protein degradation activity; they still retain strong degradation activity at 24 h (FIG. 4B).

### Example 12. Pharmacodynamic assessment in BALB/c nude mouse model with human liver cancer HepG2 cell subcutaneous xenograft tumor

Objective: Pre-clinical assessment of the efficacy of CR202 series of compounds in BALB/c nude mouse model with human liver cancer HepG2 cell subcutaneous xenograft tumor with no FOLR1 or PSMA receptor expression.

Procedures: 0.1 mL (10 × 10⁶ cells) of HepG2 cells was subcutaneously inoculated on the right back of each mouse, and the mice were randomized into a modeling control group and treatment groups after the mean tumor volume was approximately 100-150 mm³. The treatment was started on the first day of grouping. Before the start of administration, the animals were weighed, and the tumor volume was measured with a vernier caliper. After the grafting of tumor cells, the routine monitoring included the effect on tumor growth and the effect of treatment on the normal behavior of the animals, specifically the activity, food and water intake, weight gain or loss, the eyes, the hair, and other abnormal conditions. After the administration was started, the body weight and tumor size of the mice were measured periodically. The calculation formula for tumor volume: Tumor volume V (mm³) = 1/2 × (a × b²) (where a denotes the long diameter and b notes the short diameter). The calculation formula for the tumor growth inhibition, TGI (%), is TGI% = (1 - T/C) × 100% (T and C are the mean tumor volume of the treatment group and control group, respectively, at a particular time point). The grouping and design of the study are shown in Table 24 below:

**Table 24. Grouping and dosing regimens in HepG2 animal model study**

| **Group** | **Number of animals** | **Test compound** | **Dose (mg/kg)** | **Route of administration** | **Dosing volume (mL/kg)** | **Scheduled administration cycle** |
|---|---|---|---|---|---|---|
| 1 | 6 | Control | -- | -- | -- | -- |
| 2 | 6 | CR20274 | 4 | iv | 10 | biw×3w |
| 3 | 6 | CR20282 | 12.5 | iv | 10 | biw×3w |
| 4 | 6 | CR20282 | 12.5 | iv | 10 | qw×3w |

| | | | | | | |
|---|---|---|---|---|---|---|
| Note: biw×3w denotes dosing twice a week for 3 weeks (unless otherwise indicated hereinafter); qw×3w denotes dosing once a week for 3 weeks (unless otherwise indicated hereinafter). | | | | | | |

Results and analysis: The tumor volume of the negative control group reached 1726.90 mm³ on day 36 after grouping. Test compound **CR20274** exhibited no tumor growth inhibition at the dosage of 4 mg/kg, biw×3, and the mean tumor volume reached 1329.84 mm³ on day 36 after grouping with a TGI value of 22.99% and no significant difference compared with the negative control group (P = 0.382). Test compound **CR20282** exhibited no tumor growth inhibition at the dosages of 12.5 mg/kg, biw×3 and 12.5 mg/kg, qw×3, with no significant difference compared with the negative control group (P = 0.701). See FIGs. 5A-5B for details.

Test compounds CR20274 and CR20282 exhibited no anti-tumor effect on the tumor growth of human liver cancer model HepG2 tumor-bearing mice under the planned regimens. The human liver cancer HepG2 mouse model is a model with no FOLR1 or PSMA expression, and the results suggest that the dual-ligand PROTAC compounds have no significant anti-tumor effects in an animal model without corresponding target protein expression.

### Example 13. Pharmacodynamic assessment in human breast cancer MDA-MB-231 cell subcutaneous xenograft tumor model

Objective: Pre-clinical pharmacodynamic study of test compounds CR20274, CR20282, and CR20285 in BALB/c nude mouse model with human breast cancer MDA-MB-231 cell subcutaneous xenograft.

Procedures: MDA-MB-231 tumor mass was grafted subcutaneously at the right axilla of the mice, and the mice were randomized after the mean tumor volume was 200 mm³. After administration, the animals were monitored daily for health conditions and death, and the routine examinations included observation of the effect on tumor growth and the effect of the treatment on the daily performance of the animals, such as behavioral activities, food and water intake, weight changes (measured once every other day), appearance, or other abnormal conditions. Whether the tumor growth was inhibited or delayed or the tumor was cured was also observed. The tumor volume was measured periodically. The tumor volume was calculated using the following formula: V = 1/2 × (a × b²), where a and b denote the long diameter and short diameter of the tumor, respectively. The calculation formula for the tumor growth inhibition, TGI (%), is TGI% = (1 - T/C) × 100% (T and C are the mean tumor volume of the treatment group and control group, respectively, at a particular time point). The grouping and regimens of the study are shown in Table 25 below:

**Table 25. Grouping and dosing regimens in MDA-MB-231 animal model study**

| **Group** | **Number of animals** | **Test compound** | **Dose (mg/kg)** | **Route of administration** | **Scheduled administration cycle** |
|---|---|---|---|---|---|
| 1 | 6 | Control | N/A | N/A | N/A |
| 2 | 6 | CR20274 | 4 | iv | biw×3 |
| 3 | 6 | CR20282 | 12.5 | iv | qw×3 |
| 4 | 6 | CR20282 | 12.5 | iv | biw×3 |
| 5 | 6 | CR20282 | 6 | iv | biw×3 |
| 6 | 6 | CR20285 | 12.5 | iv | qw×3 |
| 7 | 6 | CR20285 | 6 | iv | biw×3 |

Results and analysis: The change in tumor volume in the human breast cancer MDA-MB-231 tumor-bearing mouse model is shown in FIG. 6A, and the change from baseline over time in body weight in the human breast cancer MDA-MB-231 tumor-bearing mouse model is shown in FIG. 6B. **CR20282** 12.5 mg/kg biw×3 has a significant anti-tumor effect on tumor growth and good safety in the human breast cancer MDA-MB-231 xenograft mouse model. **CR20282** 12.5 mg/kg qw×3, **CR20285** 12.5 mg/kg qw×3, **CR20285** 6 mg/kg biw×3, **CR20282** 3 mg/kg biw×3, and **CR20285** 3 mg/kg biw×3 also possess certain anti-tumor effects on the tumors in the human breast cancer MDA-MB-231 xenograft mouse model. **CR20274** 4 mg/kg biw×3 has no anti-tumor effect on the tumors in the human breast cancer MDA-MB-231 xenograft mouse model. The results indicate that the conjugate compounds containing the targeted molecule have significantly improved anti-tumor effects compared with the proteolysis targeting chimera.

### Example 14. Pharmacodynamic assessment in BALB/c nude mouse model with human prostate cancer 22RV1 cell subcutaneous xenograft tumor

Objective: Pre-clinical pharmacodynamic study of compounds for treating BALB/c nude mouse model with 22RV1 cell subcutaneous xenograft tumor (overexpressing PSMA).

Procedures: 22RV1 tumor mass was grafted subcutaneously at the right back of the mice, and the mice were randomized after the mean tumor volume was 150 mm³. After administration, the animals were monitored daily for health conditions and death, and the routine examinations included observation of the effect on tumor growth and the effect of the treatment on the daily performance of the animals, such as behavioral activities, food and water intake, weight changes (measured once every other day), appearance, or other abnormal conditions. Whether the tumor growth was inhibited or delayed or the tumor was cured was also observed. The tumor volume was measured periodically. The tumor volume was calculated using the following formula: V = 1/2 × (a × b²), where a and b denote the long diameter and short diameter of the tumor, respectively. The grouping and regimens of the study are shown in Table 26 below:

**Table 26. Route of administration, dosage, and regimen in 22RV1 animal model**

| **Group** | **Number of animals** | **Test compound** | **Dose (mg/kg)** | **Route of administration** | **Dosing volume (mL/kg)** | **Scheduled administration cycle** |
|---|---|---|---|---|---|---|
| 1 | 6 | Control | -- | -- | -- | -- |
| 2 | 6 | CR20274 | 4 | iv | 10 | biw×3 |
| 3 | 6 | CR20282 | 12.5 | iv | 10 | biw×3 |
| 4 | 6 | CR20293 | 12.5 | iv | 10 | biw×3 |
| 5 | 6 | CR20295 | 12.5 | iv | 10 | biw×3 |
| 6 | 6 | CR20297 | 12.5 | iv | 10 | biw×3 |

Results and analysis: The change from baseline over time in body weight in the human prostate cancer 22RV1 tumor-bearing mouse model is shown in FIG. 7A. In this study, the test compounds at different doses are well tolerated in the tumor-bearing BALB/c nude mice, with a growing trend in the body weight of the mice and no death during the study.

The change in tumor volume in the human prostate cancer 22RV1 tumor-bearing mouse model is shown in FIG. 7B. The mean tumor volume of the negative control group reached 1861.79 mm³ on day 31 after grouping. Test compound **CR20274** exhibited no tumor growth inhibition at the dosage of 4 mg/kg, biw×3, and the mean tumor volume reached 1274.47 mm³ on day 31 after grouping with a TGI value of 31.55% and no significant difference compared with the negative control group (P > 0.05). Test compound **CR20282** exhibited very significant tumor growth inhibition at the dosage of 12.5 mg/kg, biw×3, and the mean tumor volume reached 21.22 mm³ on day 31 after grouping with a TGI value of 98.86% and a significant difference compared with the negative control group (P < 0.001). Test compound **CR20293** exhibited very significant tumor growth inhibition at the dosage of 12.5 mg/kg, biw×3, and the mean tumor volume reached 46.32 mm³ on day 31 after grouping with a TGI value of 97.51% and a significant difference compared with the negative control group (P < 0.001). Test compound **CR20295** exhibited very significant tumor growth inhibition at the dosage of 12.5 mg/kg, biw×3, and the mean tumor volume reached 86.32 mm³ on day 31 after grouping with a TGI value of 95.36% and a significant difference compared with the negative control group (P < 0.001). Test compound **CR20297** exhibited very significant tumor growth inhibition at the dosage of 12.5 mg/kg, biw×3, and the tumors completely regressed on day 31 after grouping with a TGI value of 100.00% and a significant difference compared with the negative control group (P < 0.001).

It can be seen that the test compounds CR20282, CR20293, CR20295, and CR20297 exhibited significant anti-tumor effects against tumor growth in the human prostate cancer 22RV1 tumor-bearing mouse model compared with the control group and CR20274; the compounds are well tolerated in the tumor-bearing BALB/c nude mice, with a growing trend in the body weight of the mice and no death during the study.

### Example 15. Pharmacodynamic assessment in NCG nude mouse model with human prostate cancer C4-2 cell subcutaneous xenograft tumor

Objective: Pre-clinical assessment of the efficacy of compounds for treating NCG mouse model with C4-2 cell subcutaneous xenograft tumor (overexpressing PSMA).

Procedures: C4-2 cells were subcutaneously grafted at the right back of the mice, and the mice were randomized when the mean tumor volume reached 100-150 mm³. After the administration, the routine monitoring included the effect on tumor growth and the effect of treatment on the normal behavior of the animals, specifically the activity, food and water intake, weight gain or loss, the eyes, the hair, and other abnormal conditions. Clinical symptoms observed during the study were recorded in the raw data. After the administration was started, the body weight and tumor size of the mice were measured periodically. The tumor volume was calculated using the following formula: V = 1/2 × (a × b²), where a and b denote the long diameter and short diameter of the tumor, respectively. The grouping and regimens of the study are shown in Table 27 below:

**Table 27. Route of administration, dosage, and regimen in C4-2 animal model**

| **Group** | **Number of animals** | **Test compound** | **Dose (mg/kg)** | **Route of administration** | **Dosing volume (mL/kg)** | **Scheduled administration cycle** |
|---|---|---|---|---|---|---|
| 1 | 5 | Control | -- | -- | -- | -- |
| 2 | 5 | CR20274 | 4 | *i.v* | 10 | biw×3w |
| 3 | 5 | CR20282 | 12.5 | *i.v* | 10 | biw×3w |
| | 5 | CR20285 | 12.5 | *i.v* | 10 | biw×3w |
| 4 5 | 5 | CR20293 | 12.5 | *i.v* | 10 | biw×3w |
| 6 | 5 | CR20295 | 12.5 | *i.v* | 10 | biw×3w |
| 7 | 5 | CR20297 | 12.5 | *i.v* | 10 | biw×3w |

Results and analysis: The change from baseline over time in body weight in the human prostate cancer C4-2 tumor-bearing mouse model is shown in FIG. 8A. In this study, the test compounds at different doses are well tolerated in the tumor-bearing BALB/c nude mice, with a growing trend in the body weight of the mice and substantially no death during the study.

The change from baseline over time in body weight in the human prostate cancer C4-2 tumor-bearing mouse model is shown in FIG. 8B. The mean tumor volume of the negative control group reached 1748.61 mm³ on day 23 after grouping. Test compound **CR20274** exhibited no tumor growth inhibition at the dosage of 4 mg/kg, biw×3, and the mean tumor volume reached 2198.07 mm³ on day 23 after grouping with a TGI value of -25.70% and no significant difference compared with the negative control group (P > 0.05). Test compound **CR20282** exhibited certain tumor growth inhibition at the dosage of 12.5 mg/kg, biw×3, and the mean tumor volume reached 557.12 mm³ on day 23 after grouping with a TGI value of 68.14% and a significant difference compared with the negative control group (P < 0.01). Test compound **CR20293** exhibited certain tumor growth inhibition at the dosage of 12.5 mg/kg, biw×3, and the mean tumor volume reached 778.70 mm³ on day 23 after grouping with a TGI value of 55.47% and a significant difference compared with the negative control group (P < 0.05). Test compound **CR20295** exhibited certain tumor growth inhibition at the dosage of 12.5 mg/kg, BIW×3, and the mean tumor volume reached 415.38 mm³ on day 23 after grouping with a TGI value of 76.25% and a significant difference compared with the negative control group (P < 0.001). Test compound **CR20297** exhibited excellent tumor growth inhibition at the dosage of 12.5 mg/kg, biw×3, and the mean tumor volume reached 300.72 mm³ on day 23 after grouping with a TGI value of 82.80% and a significant difference compared with the negative control group (P < 0.001).

In conclusion, the test compounds CR20282, CR20293, CR20295, and CR20297 exhibited good anti-tumor effects on the tumor growth and safety in the human prostate cancer C4-2 mouse model compared with the control group and CR20274.

### Example 16. Pharmacodynamic assessment in BALB/c nude mouse model with HT-29 cell subcutaneous xenograft Tumor

Objective: Pre-clinical assessment of the efficacy of CR202 series of compounds for treating BALB/c nude mouse model with HT-29 cell subcutaneous xenograft tumor.

Procedures: HT-29 cells were subcutaneously grafted at the right back of the mice, and the mice were randomized when the mean tumor volume reached 100-150 mm³. After the administration, the routine monitoring included the effect on tumor growth and the effect of treatment on the normal behavior of the animals, specifically the activity, food and water intake, weight gain or loss, the eyes, the hair, and other abnormal conditions. Clinical symptoms observed during the study were recorded in the raw data. After the administration was started, the body weight and tumor size of the mice were measured periodically. The tumor volume was calculated using the following formula: V = 1/2 × (a × b²), where a and b denote the long diameter and short diameter of the tumor, respectively. The grouping and regimens of the study are shown in Table 28 below:

**Table 28. Route of administration, dosage, and regimen in HT-29 animal model**

| **Group** | **Number of animals** | **Test compound** | **Dose (mg/kg)** | **Route of administration** | **Scheduled administration cycle** |
|---|---|---|---|---|---|
| 1 | 6 | Control | -- | -- | -- |
| 2 | 6 | CR20274 | 2 | *i.v* | biw×3w |
| 3 | 6 | CR20274 | 4 | *i.v* | biw×3w |
| 4 | 6 | CR20282 | 6 | *i.v* | biw×3w |
| 5 | 6 | CR20282 | 12.5 | *i.v* | biw×3w |
| 6 | 6 | CR202EZ | 10.4 | *i.v* | biw×3w |
| 7 | 6 | CR202EZ | 21.6 | *i.v* | biw×3w |
| 8 | 6 | CR202HR | 10.9 | *i.v* | biw×3w |
| 9 | 6 | CR202HR | 22.7 | *i.v* | biw×3w |

Results and analysis: In this study, CR20282, CR202EZ, and CR202HR exhibited good tumor inhibition and safety at both low and high doses compared with CR20274 at an equimolar dose (see FIGs. 9A-9B). The compounds were well tolerated in the mice of the treatment groups, with a growing trend in the body weight of the mice and no abnormal death during the study. The mean tumor volume of the negative control group reached 2079.64 mm³ on day 25 after grouping, and the mice were euthanized.

Test compound CR20274 exhibited certain tumor growth inhibition at the dosage of 2 mg/kg, BIW×3, and the mean tumor volume reached 1575.84 mm³ on day 25 after grouping with a TGI value of 24.23% and no significant difference compared with the negative control group (P > 0.05).

Test compound CR20274 exhibited certain tumor growth inhibition at the dosage of 4 mg/kg, BIW×3, and the mean tumor volume reached 1316.64 mm³ on day 25 after grouping with a TGI value of 36.69% and no significant difference compared with the negative control group (P > 0.01).

Test compound CR20282 exhibited certain tumor growth inhibition at the dosage of 6 mg/kg, BIW×3, and the mean tumor volume reached 971.53 mm³ on day 25 after grouping with a TGI value of 53.28% and a significant difference compared with the negative control group (P < 0.001).

Test compound CR20282 exhibited certain tumor growth inhibition at the dosage of 12.5 mg/kg, BIW×3, and the mean tumor volume reached 670.36 mm³ on day 25 after grouping with a TGI value of 67.77% and a significant difference compared with the negative control group (P < 0.001).

Test compound CR202EZ exhibited no tumor growth inhibition at the dosage of 10.4 mg/kg, BIW×3, and the mean tumor volume reached 1082.77 mm³ on day 25 after grouping with a TGI value of 47.93% and a significant difference compared with the negative control group (P < 0.001).

Test compound CR202EZ exhibited certain tumor growth inhibition at the dosage of 21.6 mg/kg, BIW×3, and the mean tumor volume reached 691.438 mm³ on day 25 after grouping with a TGI value of 66.75% and a significant difference compared with the negative control group (P < 0.001).

Test compound CR202HR exhibited excellent tumor growth inhibition at the dosage of 10.9 mg/kg, BIW×3, and the mean tumor volume reached 946.05 mm³ on day 25 after grouping with a TGI value of 54.51% and a significant difference compared with the negative control group (P < 0.001).

Test compound CR202HR exhibited excellent tumor growth inhibition at the dosage of 22.7 mg/kg, BIW×3, and the mean tumor volume reached 614.73 mm³ on day 25 after grouping with a TGI value of 70.44% and a significant difference compared with the negative control group (P < 0.001).

In conclusion, test compounds CR20282, CR202EZ, and CR202HR exhibited good anti-tumor effects on tumor growth in the human colorectal cancer HT-29 subcutaneous xenograft mouse model, which are significantly dose-related.

### Example 17. Pharmacodynamic assessment in BALB/c nude male mouse model with human prostate cancer 22Rv-1 cell subcutaneous xenograft

Objective: Pre-clinical anti-tumor effect assessment of CR202 series of compounds in BALB/c nude male mouse model with human prostate cancer 22Rv-1 cell subcutaneous xenograft.

Procedures: 22Rv-1 (CL-00458) cells were cultured in a PRMI1640 medium containing 10% fetal bovine serum. The 22Rv-1 cells in the exponential phase were collected and resuspended in PBS to an appropriate concentration for subcutaneous tumor grafting in nude mice. The mice were subcutaneously grafted at the right back with 1 × 10⁷ 22Rv-1 cells, which were resuspended in a mixture of PBS and matrigel (1:1, 0.1 mL/mouse). The growth of the tumors was observed periodically. When the mean volume of the tumors reached 100-150 mm³, the mice were randomized according to the tumor size. After the administration, the routine monitoring included the effect on tumor growth and the effect of treatment on the normal behavior of the animals, specifically the activity, food and water intake, weight gain or loss, the eyes, the hair, and other abnormal conditions. Clinical symptoms observed during the study were recorded in the raw data. After the administration was started, the body weight and tumor size of the mice were measured periodically. The tumor volume was calculated using the following formula: V = 1/2 × (a × b²), where a and b denote the long diameter and short diameter of the tumor, respectively. The grouping and regimens of the study are shown in Table 29 below:

**Table 29. Route of administration, dosage, and regimen in 22Rv-1 animal model**

| **Group** | **Number of animals** | **Test compound** | **Dose (mg/kg)** | **Route of administration** | **Scheduled administration cycle** |
|---|---|---|---|---|---|
| 1 | 6 | Control | -- | -- | -- |
| 2 | 6 | CR202LB | 2.87 | i.v | biw×3w |
| 3 | 6 | CR202HT | 2.88 | i.v | biw×3w |

Results and analysis: In this study, the change in tumor volume in the human prostate cancer 22Rv-1 tumor-bearing mouse model is shown in FIG. 10A, and the change from baseline over time in body weight in the human prostate cancer 22Rv-1 tumor-bearing mouse model is shown in FIG. 10B. It can be seen that both CR202LB and CR202HT possess good tumor growth inhibition rates and safety. The compounds were well tolerated in the mice of the treatment groups, with a growing trend in the body weight of the mice and no abnormal death during the study. The mean tumor volume of the negative control group reached 1651.17 mm³ on day 29 after grouping, and the mice were euthanized.

Test compound CR202LB exhibited certain tumor growth inhibition at the dosage of 2.87 mg/kg, BIW×3, and the mean tumor volume reached 969.46 mm³ on day 29 after grouping with a TGI value of 41.29% and a significant difference compared with the negative control group (P < 0.001).

Test compound CR202HT exhibited certain tumor growth inhibition at the dosage of 2.88 mg/kg, BIW×3, and the mean tumor volume reached 690.37 mm³ on day 29 after grouping with a TGI value of 58.19% and a significant difference compared with the negative control group (P < 0.001).

In conclusion, test compounds CR202LB and CR202HT exhibited good safety and certain anti-tumor effects on the tumor growth in the human prostate cancer 22Rv-1 subcutaneous xenograft mouse model.

Other conjugate compounds of the present application were tested for their tumor inhibitory effects similarly. The results show that other conjugate compounds of the present application also have certain tumor inhibitory effects superior to that of the unconjugated compound.

### Example 18. Single-dose PK assessment of compounds in BALB/c nude male mouse model with human colorectal cancer HT-29 cell subcutaneous xenograft

Objective: Single-dose PK assessment of compounds CR20214 and CR20274 in BALB/c nude male mouse model with human colorectal cancer HT-29 cell subcutaneous xenograft.

Procedures: HT-29 cells were grafted subcutaneously on the right back of the mice (purchased from GemPharmatech Co., Ltd., weighing 20-25 g, male, aged 45-60 days), and the mice were randomized when the mean tumor volume reached about 300 mm³. 6.25 mg/kg of CR20214 and 2 mg/kg of CR20274 were given to the HT-29 tumor-bearing mice in a single dose by tail vein injection. The tumors were taken at 0.833, 0.25, 0.5, 1, 2, 4, 8, 24, 48 and 72 h post-dose, ground, and homogenized. The homogenate was extracted, and after centrifugation, the supernatants were injected into a triple quadrupole tandem mass spectrometer to determine the CR20274 content in the tumors. The results are shown in Table 30.

**Table 30. Concentration of CR20214 and CR20274 in tumor tissue (nmol/g)**

| **Time (h)** | **Concentration of CR20274 released by CR20214 in tumors (C1)** | **Concentration of CR20274 in tumors (C2)** | **C1/C2** |
|---|---|---|---|
| **0.0833** | 0.044 | 0.227 | 0.19 |
| **0.25** | 0.060 | 0.206 | 0.29 |
| **0.5** | 0.164 | 0.222 | 0.74 |
| **1** | 0.108 | 0.191 | 0.57 |
| **2** | 0.191 | 0.180 | 1.06 |
| **4** | 0.265 | 0.164 | 1.62 |
| **8** | 0.353 | 0.154 | 2.29 |
| **24** | 0.271 | 0.104 | 2.61 |
| **48** | 0.166 | 0.064 | 2.59 |
| **72** | 0.133 | 0.044 | 3.02 |

Results and analysis: at equimolar doses, CR20214 has a higher concentration than CR20274 in tumor tissues, indicating that CR20214 has better targeting ability.

## Claims

1. A conjugate compound or a pharmaceutically acceptable salt thereof, comprising: a targeted molecule comprising at least two ligands specifically binding to a cell surface protein and a payload linked to the targeted molecule, wherein the payload is a proteolysis targeting chimera.

2. The conjugate compound or the pharmaceutically acceptable salt thereof according to claim 1, wherein the targeted molecule binds to the following cell surface proteins: FOLR1, TRPV6, PSMA, LHRH, EGFR, Her2, Trop2, Her3, Claudin18.2, c-Met, or any combination thereof.

3. The conjugate compound or the pharmaceutically acceptable salt thereof according to claim 1 or 2, wherein the targeted molecule binds to the following cell surface proteins: FOLR1, TRPV6, PSMA, c-Met, or any combination thereof.

4. The conjugate compound or the pharmaceutically acceptable salt thereof according to any one of claims 1-3, wherein the targeted molecule binds to the following cell surface protein combinations: FOLR1 and TRPV6; FOLR1 and PSMA; TRPV6 and PSMA; TRPV6 and c-Met; FOLR1 and c-Met; PSMA and c-Met; FOLR1, TRPV6, and PSMA; FOLR1, TRPV6, and e-Met; FOLR1, PSMA, and e-Met; TRPV6, PSMA, and c-Met; or FOLR1, PSMA, TRPV6, and c-Met.

5. The conjugate compound or the pharmaceutically acceptable salt thereof according to any one of claims 1-4, wherein the targeted molecule comprises two ligands respectively binding to FOLR1 and TRPV6, FOLR1 and PSMA, TRPV6 and PSMA, FOLR1 and c-Met, PSMA and c-Met, or TRPV6 and c-Met.

6. The conjugate compound or the pharmaceutically acceptable salt thereof according to any one of claims 2-5, wherein the ligand binding to FOLR1 comprises folic acid or an analog thereof and pteroic acid; preferably the folic acid analog is selected from: 5-methyltetrahydrofolic acid, 5-formyltetrahydrofolic acid, 10-formyltetrahydrofolic acid, methotrexate, 5,10-formyltetrahydrofolic acid, 5,10-methenyltetrahydrofolic acid, aminopterin, and raltitrexed.

7. The conjugate compound or the pharmaceutically acceptable salt thereof according to any one of claims 2-6, wherein the ligand binding to TRPV6 comprises the amino acid sequence set forth in SEQ ID NO: 1, the amino acid sequence set forth in SEQ ID NO: 2, or an amino acid sequence having at least 80%, 85%, 90%, 95%, 96%, 97%, 98%, or 99% sequence identity to SEQ ID NO: 1 or SEQ ID NO: 2.

8. The conjugate compound or the pharmaceutically acceptable salt thereof according to any one of claims 2-7, wherein the ligand binding to PSMA comprises a peptide, an antibody, or a small molecule.

9. The conjugate compound or the pharmaceutically acceptable salt thereof according to any one of claims 2-8, wherein the ligand binding to PSMA is selected from the following structures: 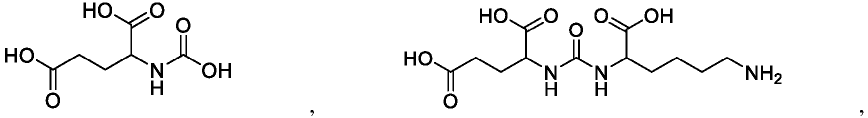 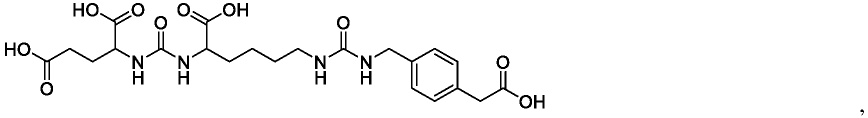 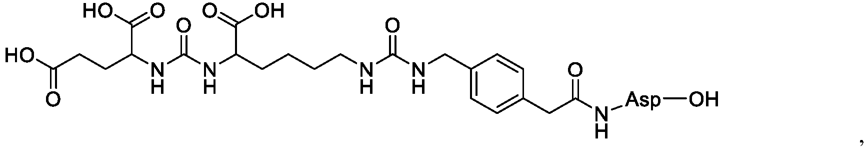 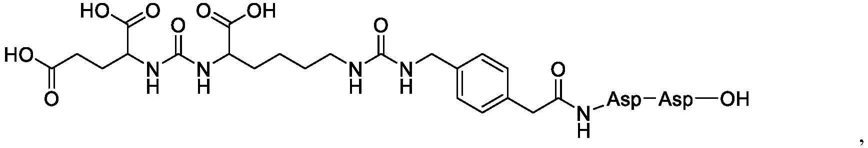 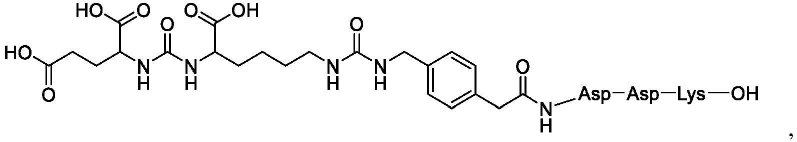 and

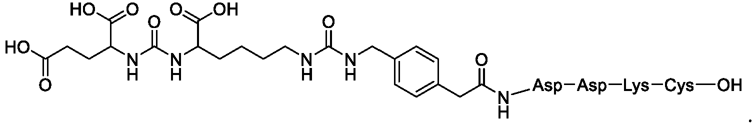

10. The conjugate compound or the pharmaceutically acceptable salt thereof according to any one of claims 2-9, wherein the ligand binding to c-MET comprises the amino acid sequence set forth in SEQ ID NO: 3: Cys^{a}-X1-Cys^{c}-X2-Gly-Pro-Pro-X3-Phe-Glu-Cys^{d}-Trp-Cys^{b}-Tyr-X4-X5-X6; X1 is Asn, His, or Tyr; X2 is Gly, Ser, Thr, or Asn; X3 is Thr or Arg; X4 is Ala, Asp, Glu, Gly, or Ser; X5 is Ser or Thr; X6 is Asp or Glu; Cys^{a-d} are cysteine residues; preferably, residues Cys^{a} and Cys^{b}, as well as residues Cys^{c} and Cys^{d}, are separately cyclized to form two independent disulfide bonds.

11. The conjugate compound or the pharmaceutically acceptable salt thereof according to any one of claims 2-10, wherein the ligand binding to c-MET comprises the amino acid sequence set forth in SEQ ID NO: 4: Ala-Gly-Ser-Cys^{a}-Tyr-Cys^{c}-Ser-Gly-Pro-Pro-Arg-Phe-Glu-Cys^{d}-Trp-Cys^{b}-Tyr-Glu-Thr-Glu-Gly-Thr-Gly-Gly-Gly-Lys; Cys^{a-d} are cysteine residues; preferably, residues Cys^{a} and Cys^{b}, as well as residues Cys^{c} and Cys^{d}, are separately cyclized to form two independent disulfide bonds; optionally, the carboxyl of the terminal lysine may be amidated; optionally, the alanine at the N-terminus may be acetylated.

12. The conjugate compound or the pharmaceutically acceptable salt thereof according to any one of claims 2-11, wherein the ligand binding to c-MET comprises an amino acid sequence having at least 80%, 85%, 90%, 95%, 96%, 97%, 98%, or 99% sequence identity to SEQ ID NO: 4; Cys^{a-d} are cysteine residues; preferably, residues Cys^{a} and Cys^{b} and residues Cys^{c} and Cys^{d} are separately cyclized to form two independent disulfide bonds.

13. The conjugate compound or the pharmaceutically acceptable salt thereof according to any one of claims 1-12, wherein the ligands are linked to each other directly or via a spacer region; preferably the linkage is by means of a dehydration reaction.

14. The conjugate compound or the pharmaceutically acceptable salt thereof according to claim 13, wherein the spacer region comprises an amino acid or an amino acid combination selected from the following group: Lys, Cys, Lys-Cys, Cys-Cys, Lys-Cys-Lys, Arg-Arg, Ala-Ser-Asn, Ala-Ala-Ala, Ser-Ser-Arg, Pro-Arg, Asp-Asp-Lys-Cys, and Pro-Leu-Gly; optionally, the amino acid may be amidated.

15. The conjugate compound or the pharmaceutically acceptable salt thereof according to claim 14, wherein Lys is amidated, e.g., to 2,6-diaminohexanamide.

16. The conjugate compound or the pharmaceutically acceptable salt thereof according to claim 13, wherein the spacer region is formed by one or more identical or different compounds selected from the following group by means of a dehydration reaction: 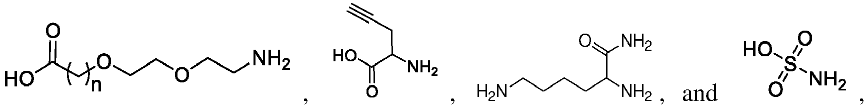 wherein n is 0 or 1.

17. The conjugate compound or the pharmaceutically acceptable salt thereof according to any one of claims 1-16, wherein the targeted molecule having the following structures is linked to the payload to form the conjugate compound: 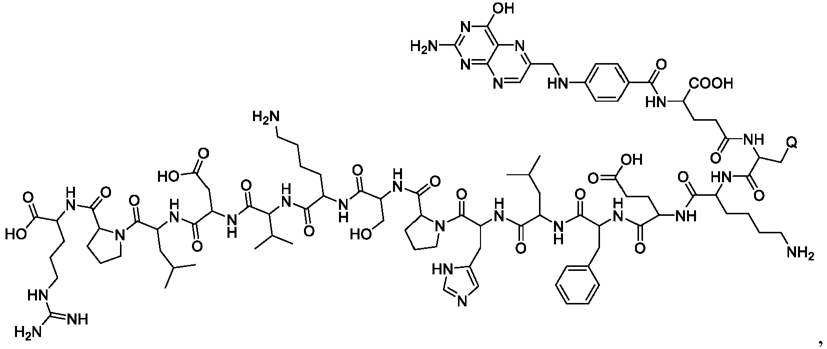 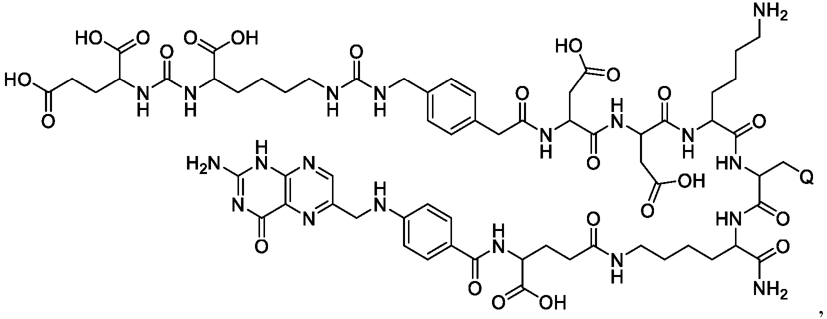 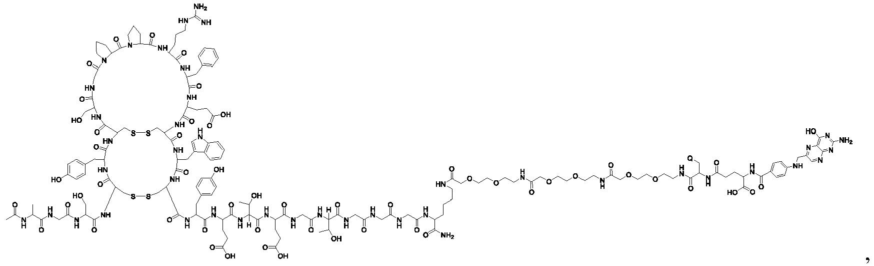 or 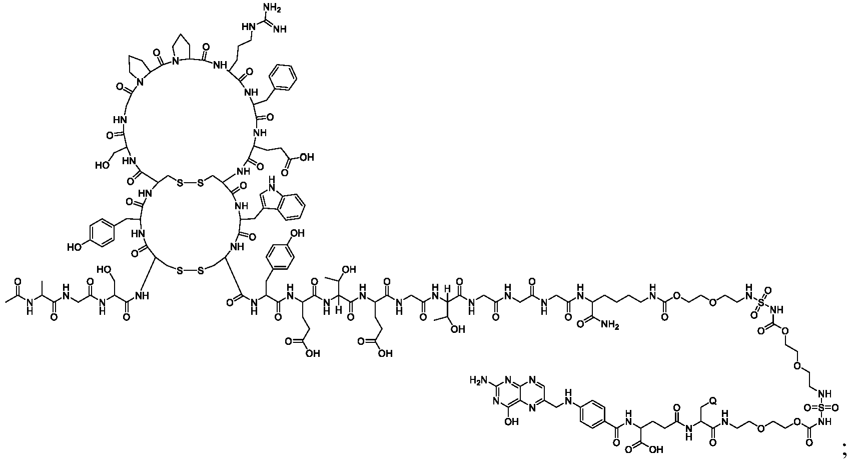 wherein Q is an active group, and the targeted molecule is linked to the payload through the active group Q; preferably, the active group Q is alkynyl or sulfhydryl; preferably, the targeted molecule has the following structures: 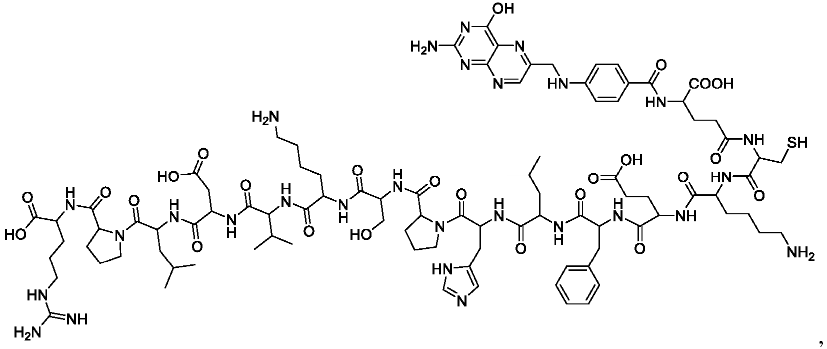 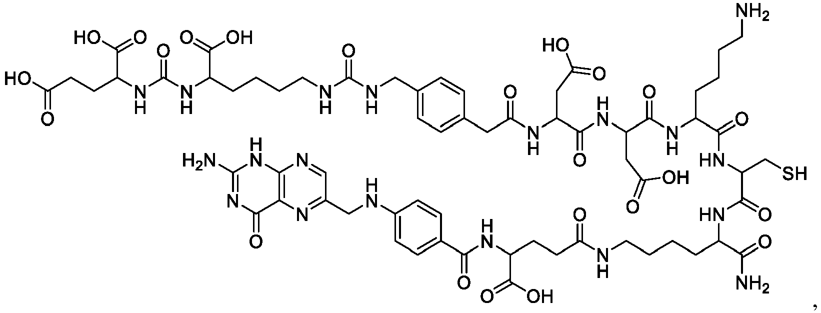 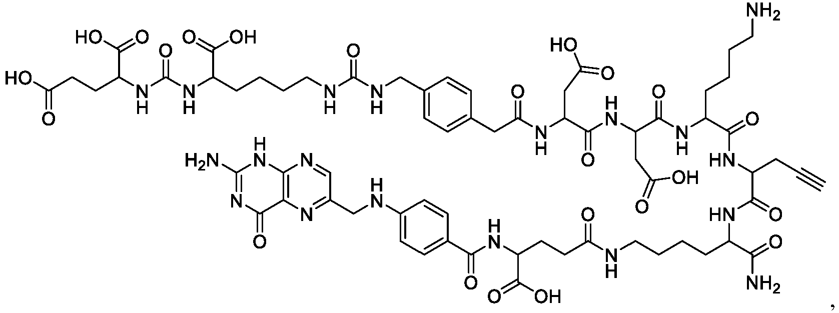 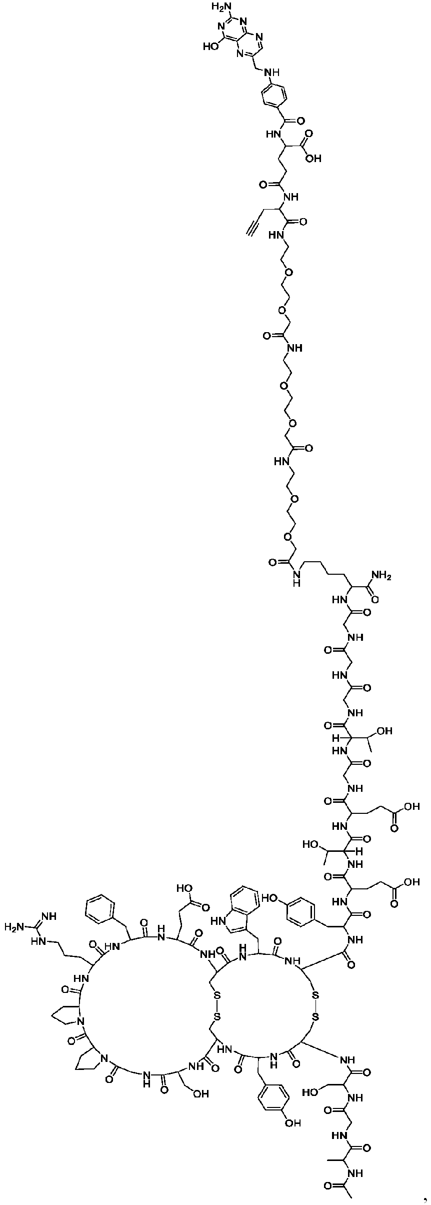 or

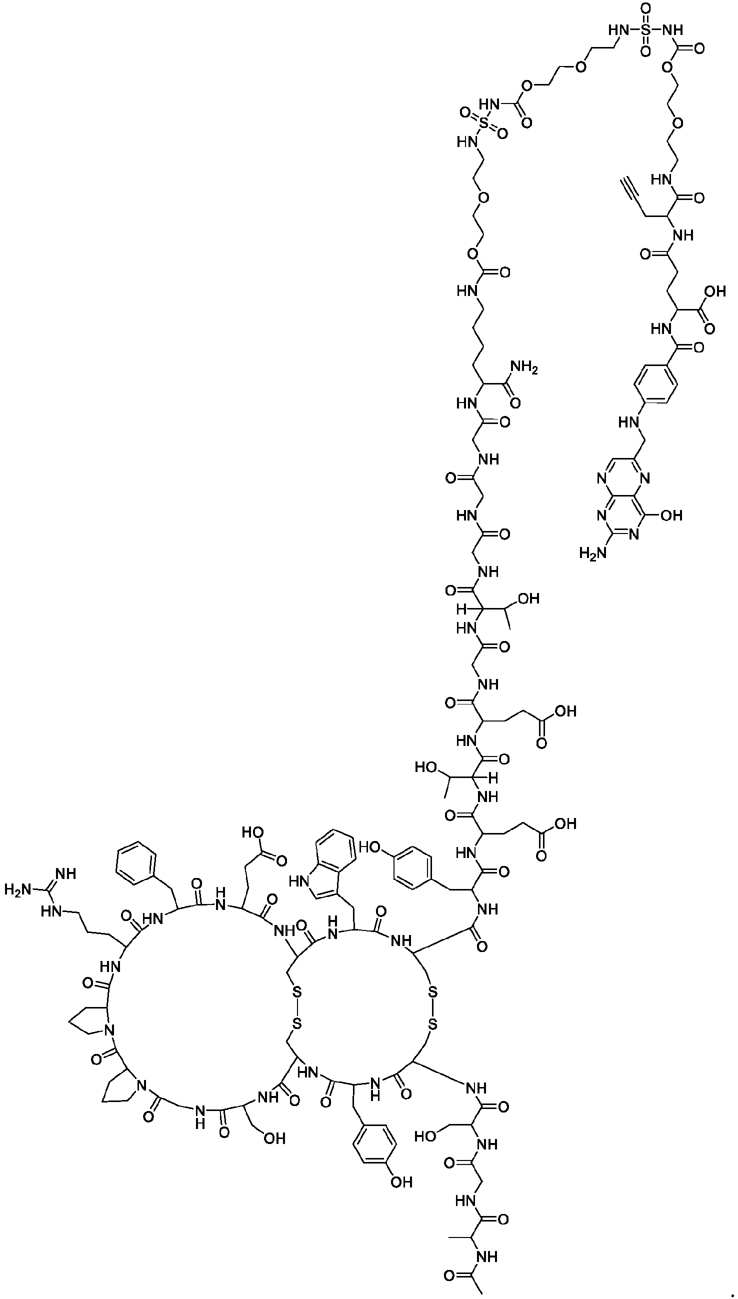

18. The conjugate compound or the pharmaceutically acceptable salt thereof according to any one of claims 1-17, wherein the proteolysis targeting chimera having the structure represented by formula (I) is linked to the targeted molecule to form the conjugate compound:
(T_{P})ₙ-L-T_{E3} (I),
wherein,
T_{P} is a target protein ligand moiety for binding to a target protein, wherein n is 1, 2, or 3;
L is a linking moiety for linking T_{P} to T_{E3};
T_{E3} is an E3 ligase ligand moiety for binding to E3 ligase.

19. The conjugate compound or the pharmaceutically acceptable salt thereof according to claim 18, wherein the E3 ligase ligand binds to VHL (Von Hippel-Lindau) protein, CRBN (cereblon) protein, MDM2 protein, or XIAP protein; preferably, the E3 ligase ligand is a VHL ligand or a CRBN ligand.

20. The conjugate compound or the pharmaceutically acceptable salt thereof according to claim 19, wherein the VHL ligand is VHL-L or an analog thereof, and the CRBN ligand is lenalidomide, pomalidomide, thalidomide, or an analog thereof.

21. The conjugate compound or the pharmaceutically acceptable salt thereof according to any one of claims 18-20, wherein the proteolysis targeting chimera has a structure selected from the following group: 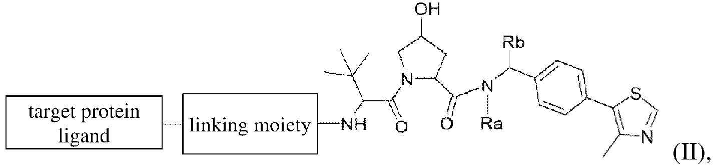 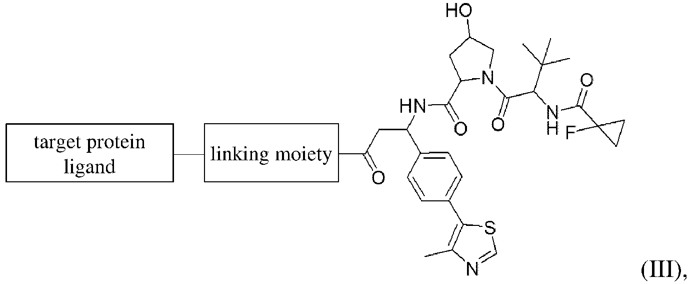 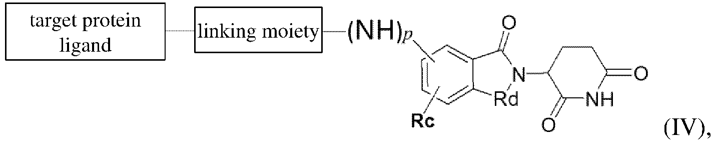 or 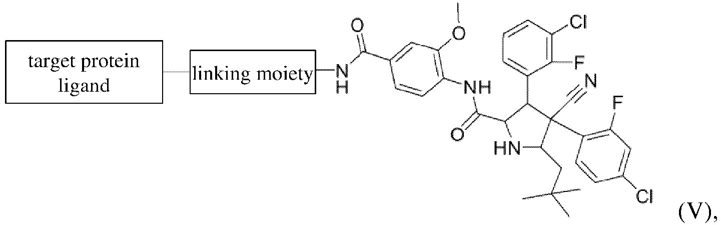 wherein,
Ra, Rb and Rc are each independently hydrogen, alkyl, NH₂, OH, or halogen; preferably the alkyl is methyl, and the halogen is fluorine;
Rd is CH₂, C=O, or C=S;
p is 0 or 1;
the target protein ligand can bind to a specific target protein;
the target protein ligand is linked to the E3 ligase group through the linking moiety.

22. The conjugate compound or the pharmaceutically acceptable salt thereof according to any one of claims 18-21, wherein the target protein is a nuclear receptor (e.g., AR and ER), a kinase-like protein (e.g., RIPK2, BCR-ABL, EGFR, HER2, c-Met, TBK1, CDK2/4/6/9, ALK, Akt, CK2, ERK1/2, FLT3, PI3K, BTK, and FAK), AKT, PAN-BET protein, BET protein (e.g., BRD2, BRD3, BRD4, and BRD6), BLK, BRAF1, BCL-xl, ERRα, FKBP12, FRS2α, JAK1, JAK3, IKZF1, IRAK4, MDM2, MetAP2, PLK1, PSK-J3, RAS, TACC3, Tau, TrkB, MDM2, or any combination thereof.

23. The conjugate compound or the pharmaceutically acceptable salt thereof according to any one of claims 18-22, wherein the target protein is AR protein, ER protein, RAS protein, BRD4 protein, PLK1 protein, FAK protein, MDM2 protein, or any combination thereof.

24. The conjugate compound or the pharmaceutically acceptable salt thereof according to any one of claims 18-23, wherein the target protein ligand moiety has a structure selected from: 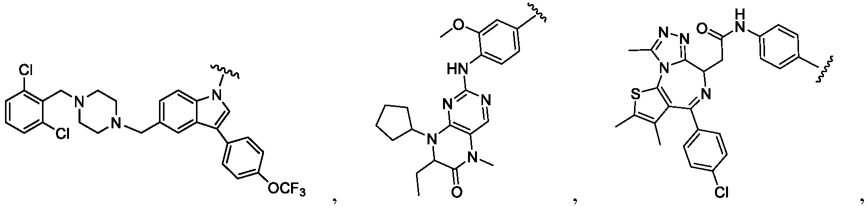 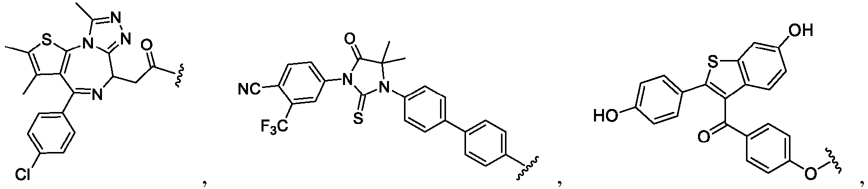 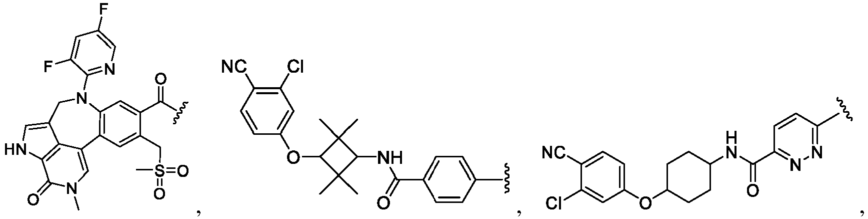 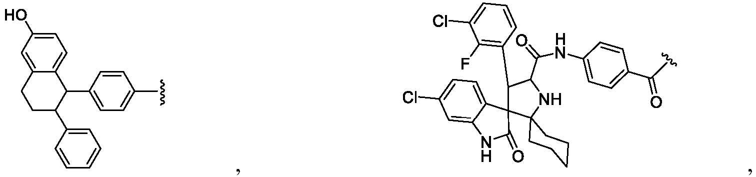 and

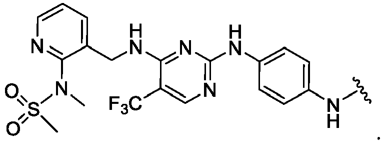

25. The conjugate compound or the pharmaceutically acceptable salt thereof according to any one of claims 18-24, wherein the linking moiety has a structure selected from the following group: 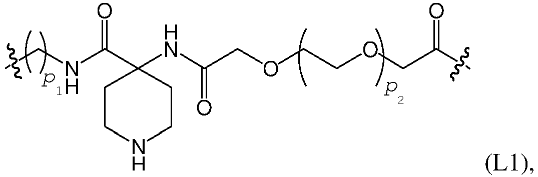 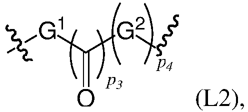 or 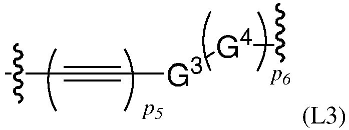 wherein,
p₁ is an integer of 0-4;
p₂ is an integer of 0-6;
p₃, p₄, p₅, and p₆ are each independently 0 or 1;
G¹ is a 4-14 membered linear alkylene, optionally 2-5 carbon atoms of the 4-14 membered linear alkylene may be substituted by -O-, -NH-, -N(C₁₋₆ alkyl)-, or -C≡C-; G² is an amino acid residue, wherein preferably G² is a Lys residue;
G³ is selected from the following group: 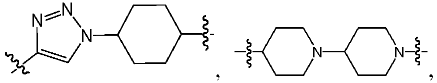 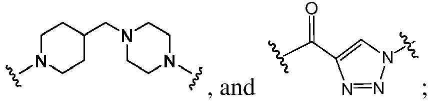
G⁴ is a 5-9 membered linear alkylene, optionally 2-4 carbon atoms of the 5-9 membered linear alkylene may be substituted by -O-, -NH-, -N(C₁₋₆ alkyl)-, or -C≡C-.

26. The conjugate compound or the pharmaceutically acceptable salt thereof according to any one of claims 18-25, wherein the linking moiety has a structure selected from the following group:

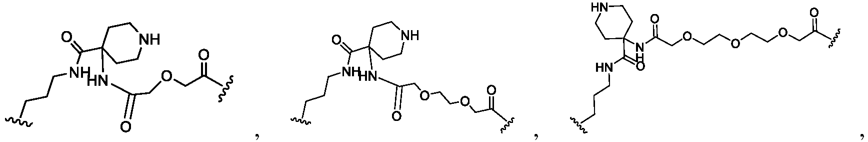

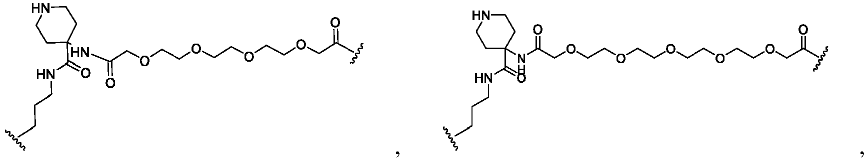

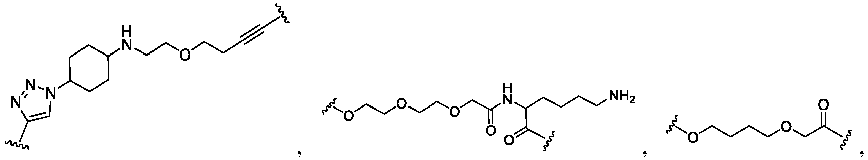

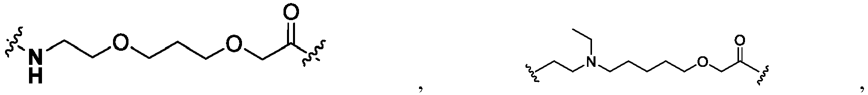

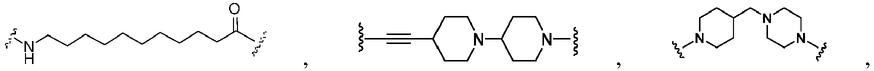

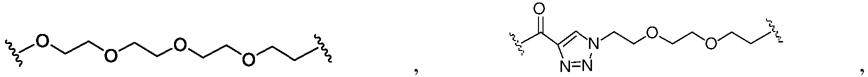

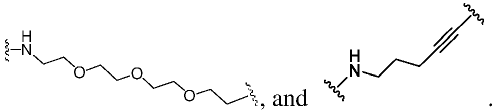

27. The conjugate compound or the pharmaceutically acceptable salt thereof according to any one of claims 18-26, wherein the proteolysis targeting chimera has the structures shown below: 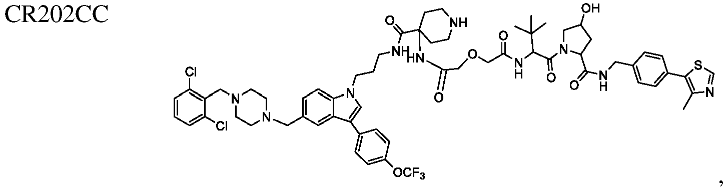 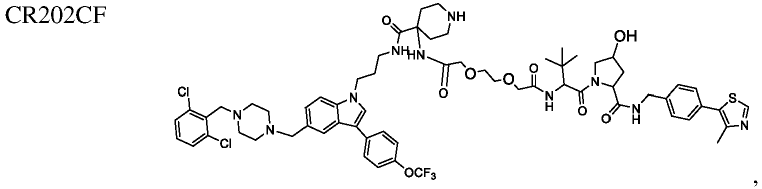 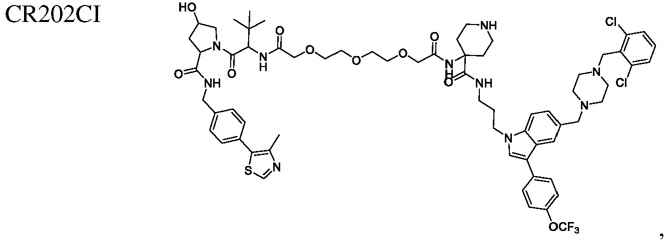 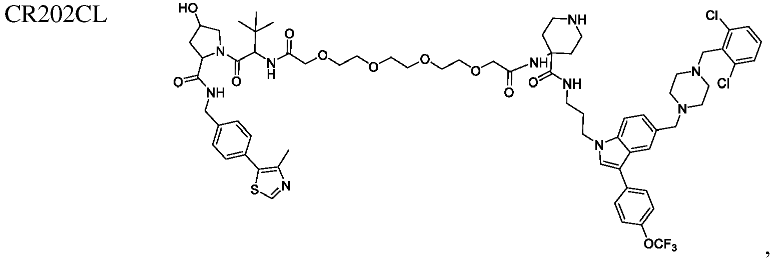 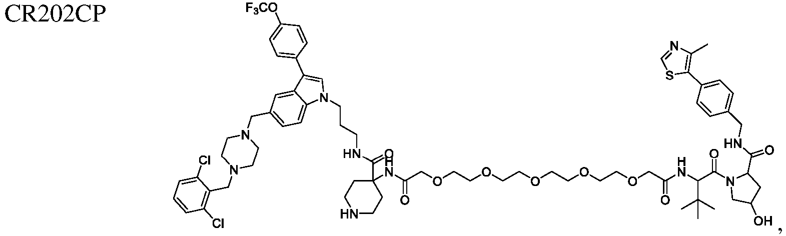 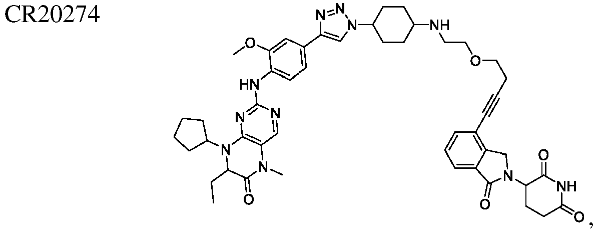 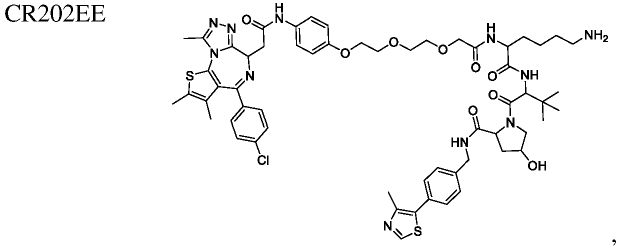 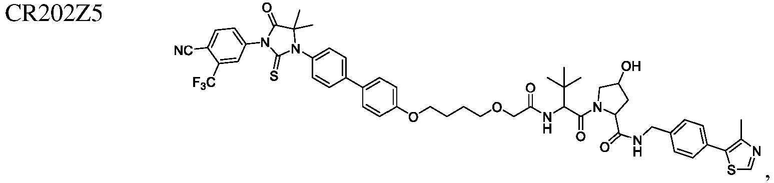 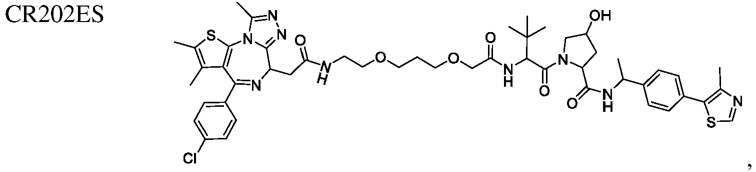 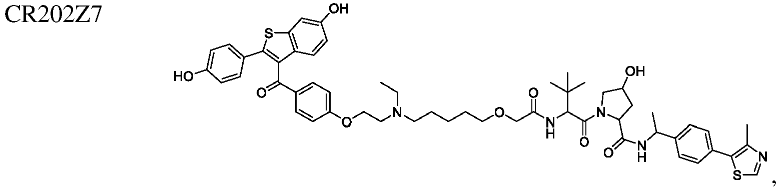 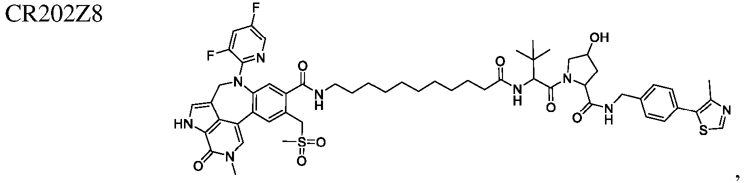 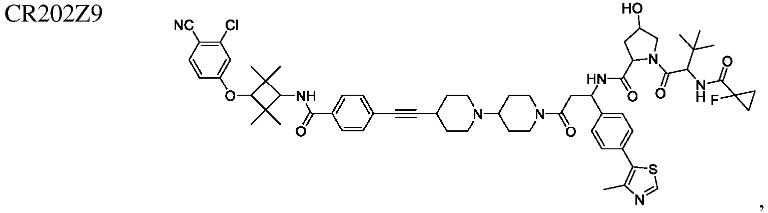 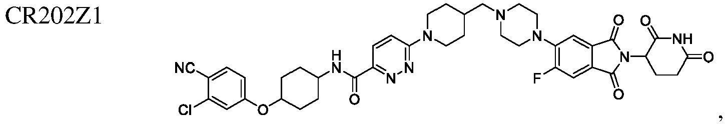 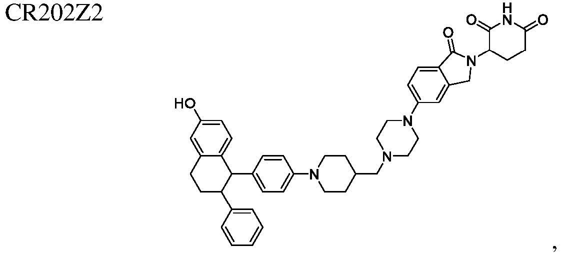 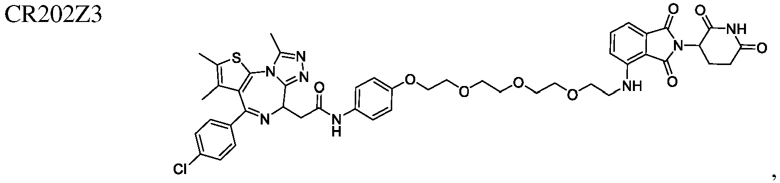 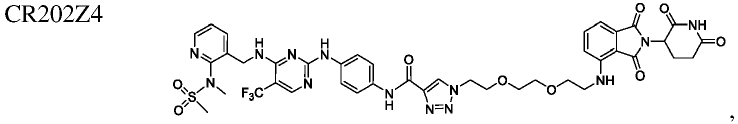 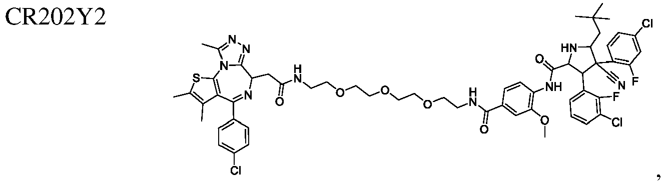 or

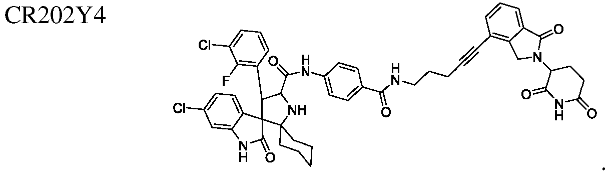

28. The conjugate compound or the pharmaceutically acceptable salt thereof according to any one of claims 1-27, wherein the payload is linked to the targeted molecule directly, or the payload is linked to the targeted molecule via a linker.

29. The conjugate compound or the pharmaceutically acceptable salt thereof according to any one of claims 1-28, wherein the linkage between the payload and the targeted molecule, the payload and the linker, or the linker and the targeted molecule is accomplished by a chemical reaction; preferably, the linkage between the targeted molecule and the linker is accomplished by a click reaction.

30. The conjugate compound or the pharmaceutically acceptable salt thereof according to any one of claims 1-29, wherein the conjugate compound has the following structure
W¹-L¹-(EG)n¹-(D)n⁵-(CH₂)n²-(B)n⁶-(AA)n³-(CH₂CH₂-Q)n⁴-L²-W²,
wherein,
L¹ is 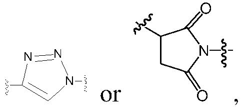 -S-S-, or a bond;
EG is -CH₂CH₂O- or -OCH₂CH₂-; n¹ is an integer of 0-6;
n² is an integer of 0-8;
D is -C(=O)-NH-S(=O)₂-NH-; n⁵ is 0 or 1;
B is each independently -C(=O)-, 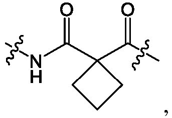 -NH-CH₂CH₂-C(=O)-, -C(=O)-NH-, -CH(CH₃)-CH₂-O-, -CH₂CH₂O-, -NH-C(=O)-, or -NH-; n⁶ is an integer of 0-3;
AA is each independently an amino acid residue; n³ is an integer of 0-5;
Q is each independently -C(=O)-, -O-, or a bond; n⁴ is an integer of 0-3;
L² is 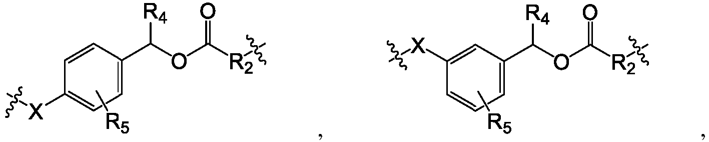 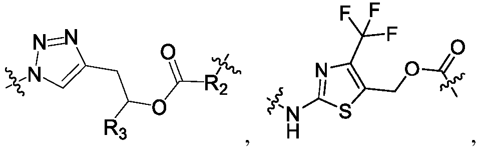 -NH-CH₂-O-, or a bond;
wherein X is a bond, -NH-, -C(=O)-, oxygen, or 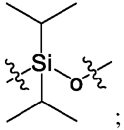
R₂ is a bond, -O-CH₂-, or -NH-CH₂CH₂-NH-C(=O)-;
R₃ is hydrogen or 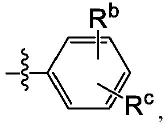 wherein R^{b} and R^{c} are each independently hydrogen, nitro, 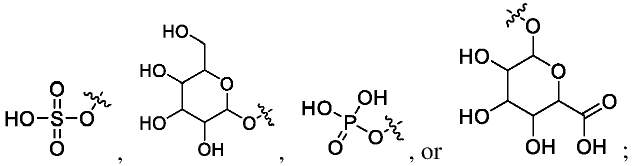
R₄ is hydrogen or C₁₋₈ alkyl;
R₅ is hydrogen, amino, nitro, phosphate group, -OR^{a}, or 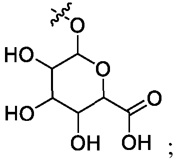 wherein R^{a} is methyl, ethyl, propyl, or isopropyl;
W¹ is the targeted molecule capable of binding to the cell surface protein, and W² is the payload;
preferably, L² is a bond, 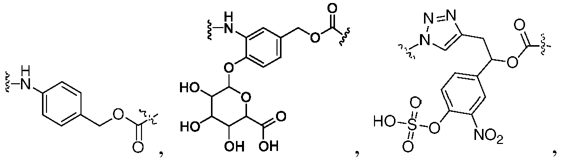 -NH-CH₂-O-, 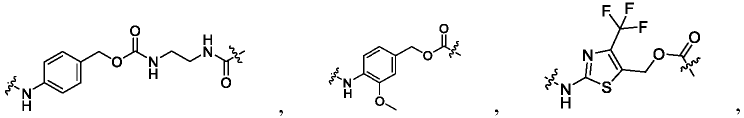 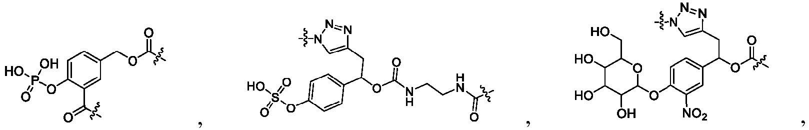 or 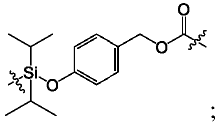
preferably, EG is -CH₂CH₂O-;
preferably, n¹ is 2 or 0;
preferably, n² is 1, 4, or 5;
preferably, n⁵ is 0;
preferably, n⁶ is 1 or 3;
preferably, n¹ is 2, n² is 1, n³ and n⁴ are 0, and L² is a bond;
preferably, AA is each independently an amino acid residue formed by Val, Cit, Glu, Leu, Lys, Ala, Gly, Phe, Gln, Pro, or Asn;
preferably, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Cit-, -Val-Leu-Lys-, Val-Cit-Pro-Gly-, -Gly-Asn-Asn-, and Ala-Ala-Asn-;
preferably, when n⁴ is 1, Q is -C(=O)-;
preferably, when n⁴ is 2, Q are -O- and -C(=O)-, respectively;
preferably, when n⁴ is 3, Q are -O-, -O-, and a bond, respectively; or
preferably, n⁴ is 0.

31. The conjugate compound or the pharmaceutically acceptable salt thereof according to any one of claims 1-30, wherein the conjugate compound has the following structure
W¹-L¹-(EG)n¹-(D)n⁵-(CH₂)n²-(B)n⁶-(AA)n³-(CH₂CH₂-Q)n⁴-L²-W²
wherein,
L¹ is 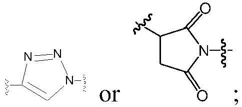
EG is -CH₂CH₂O-; n¹ is an integer of 0-4;
D is -C(=O)-NH-S(=O)₂-NH-; n⁵ is 0 or 1;
n² is an integer of 0-5;
B is -C(=O)-, -C(=O)-NH-, -CH₂CH₂O-, -NH-CH₂CH₂-C(=O)-, or -NH-; n⁶ is an integer of 0-3;
AA is each independently an amino acid residue; n³ is an integer of 0-3;
Q is each independently -C(=O)-, -O-, or a bond; n⁴ is an integer of 0-3;
L² is 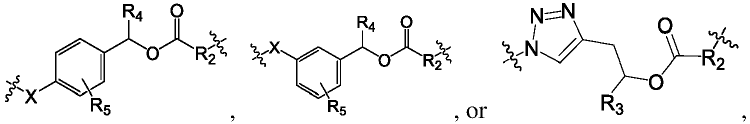
wherein X is a bond, -NH-, -C(=O)-, or 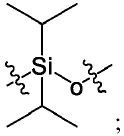
R₂ is a bond;
R₃ is 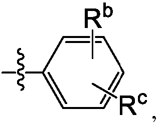 wherein R^{b} and R^{c} are each independently hydrogen, nitro, or 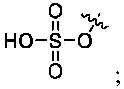
R₄ is hydrogen;
R₅ is hydrogen, -OCH₃, or 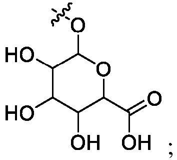
W¹ is the targeted molecule capable of binding to the cell surface protein, and W² is the payload;
preferably, L² is 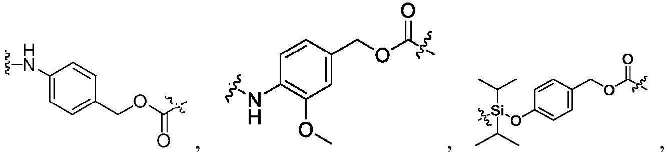 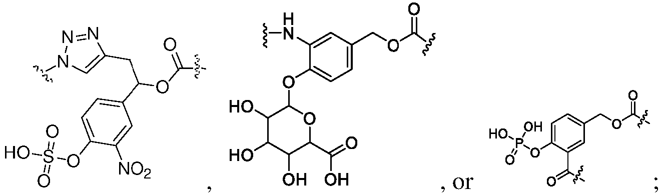
preferably, L¹ is 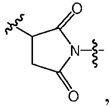 and n¹, n⁵, and n⁴ are all 0;
preferably, L¹ is 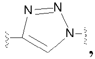 and n² is 1 or 2;
preferably, when n⁵ is 1, n² and n⁴ are both 0;
preferably, AA is each independently an amino acid residue formed by Val, Cit, Glu, or Ala; or
preferably, -(AA)n³- is a peptide residue selected from the following group: -Val-Cit-, -Glu-Val-Cit-, -Ala-Cit-, and -Val-Ala-.

32. The conjugate compound or the pharmaceutically acceptable salt thereof according to any one of claims 1-31, wherein the conjugate compound has the following structure
W¹-L¹-(EG)n¹-(CH₂)n²-(C=O)-(AA)n³-L²-W²
wherein,
L¹ is 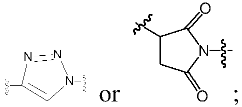
EG is -CH₂CH₂O-; n¹ is an integer of 0-4;
n² is an integer of 0-5;
AA is each independently an amino acid residue; n³ is an integer of 1-4;
L² is 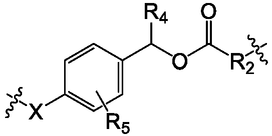 or a bond,
wherein X is -NH-; R₂ is a bond; R₄ is hydrogen; R₅ is hydrogen or -OCH₃;
W¹ is the targeted molecule capable of binding to the cell surface protein, and W² is the payload;
preferably, L¹ is 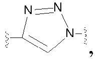 n¹ is 2, n² is 1, and L² is 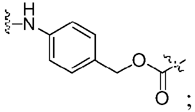
preferably, L¹ is 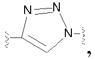 n¹ is 2, n² is 1, and L² is a bond;
preferably, L¹ is 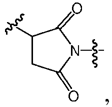 n¹ is 0, n² is 5, and L² is 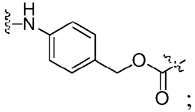
preferably, L¹ is 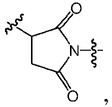 n¹ is 0, n² is 5, and L² is a bond;
preferably, AA is each independently Val, Cit, Glu, or Ala amino acid residue; or
preferably, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Val-Leu-Lys-, and -Gly-Asn-Asn-.

33. The conjugate compound or the pharmaceutically acceptable salt thereof according to any one of claims 28-32, wherein the linker comprises a linking module 1 and a linking module 2; optionally the linking module 1 is linked to the linking module 2 via a bond; optionally, the linking module 1 is linked to the linking module 2 via a chemical group.

34. The conjugate compound or the pharmaceutically acceptable salt thereof according to any one of claims 28-32, wherein the linker comprises a linking module 2; preferably, the linking module 2 may be enzymatically cleavable or acid-labile.

35. The conjugate compound or the pharmaceutically acceptable salt thereof according to any one of claims 28-32, wherein the linker comprises a linking module 1 and a group binding to the linking module 1; optionally, the group binding to the linking module 1 is a PEG chain, 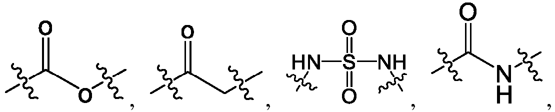 C₁-C₈ alkyl chain, -NH-, -O-, -C(O)-, or any combination thereof;
preferably, the PEG chain is PEG1 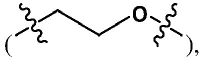 PEG2 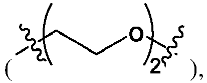 or PEG3 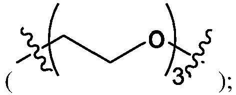
optionally, the group binding to the linking module 1 is

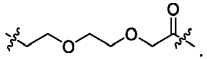

36. The conjugate compound or the pharmaceutically acceptable salt thereof according to any one of claims 33-35, wherein the linking module 1 is maleimidocaproyl 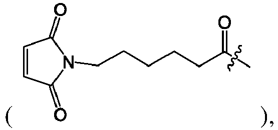 maleimido 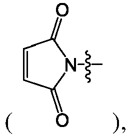 or azido ( 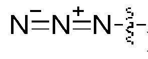 ).

37. The conjugate compound or the pharmaceutically acceptable salt thereof according to any one of claims 33-36, wherein the linking module 2, before linked to the targeted molecule, the linking module 1, the chemical group, or the payload, has a structure comprising the following groups: a CB enzyme-cleavable group, a plasmin-cleavable group, a legumain-cleavable group, a β-glucuronidase-cleavable group, a sulfatase-cleavable group, a phosphatase-cleavable group, a β-galactosidase-cleavable group, a glutathione enzyme-cleavable group, or an acid-labile group.

38. The conjugate compound or the pharmaceutically acceptable salt thereof according to claim 33, wherein the chemical group is a PEG chain, 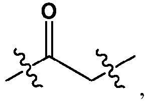 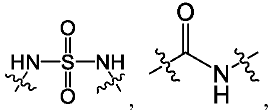 C₁-C₈ alkyl chain, -NH-, -O-, -C(O)-, 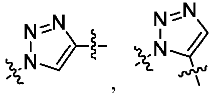 , or any combination thereof;
preferably, the PEG chain is PEG1, PEG2, or PEG3;
preferably, the chemical group is 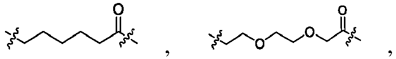 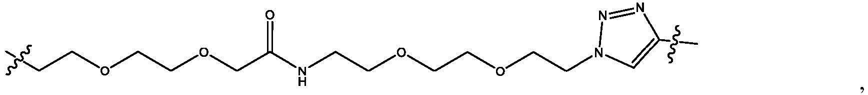 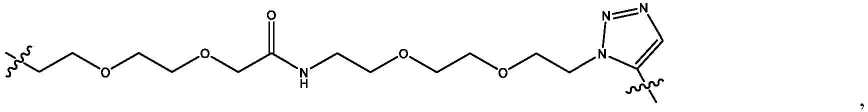 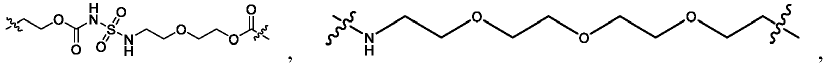 or

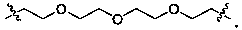

39. The conjugate compound or the pharmaceutically acceptable salt thereof according to claim 37, wherein:
the CB enzyme-cleavable group comprises an amino acid or a combination of an amino acid and a self-decomposing fragment, and the amino acid is Val-Cit, Gly-Gly-Phe-Gly, CycloBut-Cit, Phe-Cit, Val-Ala, Glu-Val-Cit, Ala-Cit, Val-Cit-Pro, or Val-Cit-Pro-Gly;
the plasmin-cleavable group comprises an amino acid or a combination of an amino acid and a self-decomposing fragment, and the amino acid is Val-Leu-Lys;
the legumain-cleavable group comprises an amino acid or a combination of an amino acid and a self-decomposing fragment, and the amino acid is selected from Gly-Asn-Asn or Ala-Ala-Asn;
the β-glucuronidase-cleavable group is 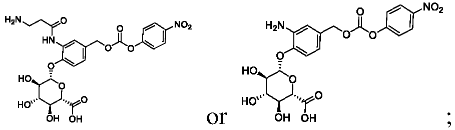
the sulfatase-cleavable group is 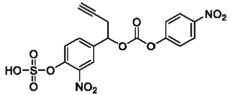 or 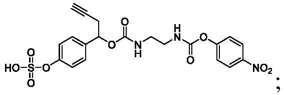
the phosphatase-cleavable group is 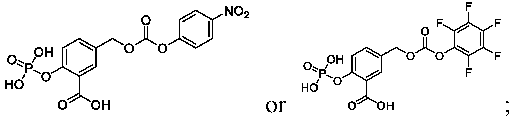
the β-galactosidase-cleavable group is 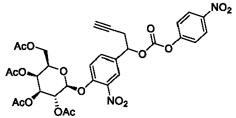 or 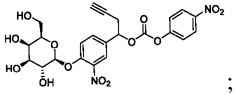
the glutathione enzyme-cleavable group is 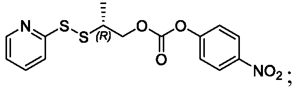
the acid-labile group is 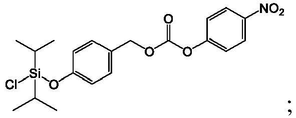
optionally, the self-decomposing fragment is PAB and a derivative thereof, 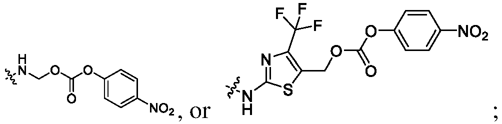
preferably, the PAB is 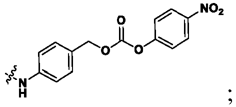
preferably, the PAB derivative is

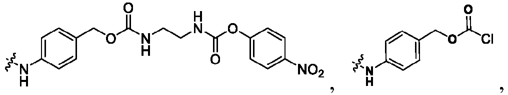

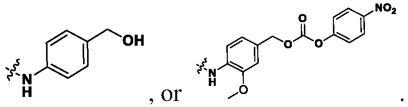

40. The conjugate compound or the pharmaceutically acceptable salt thereof according to claim 39, wherein the linking module 2, upon operative linkage to the linking module 1, the chemical group, the targeted molecule, or the payload, has the following structures:

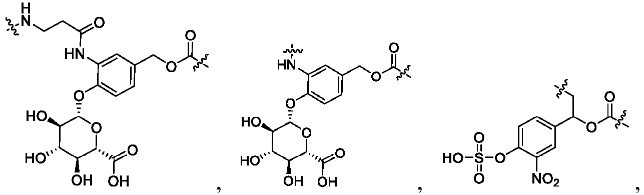

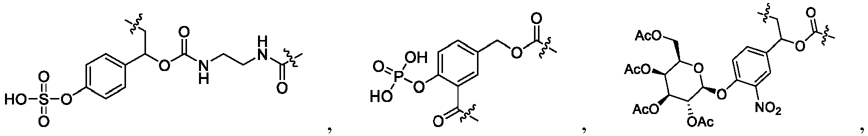

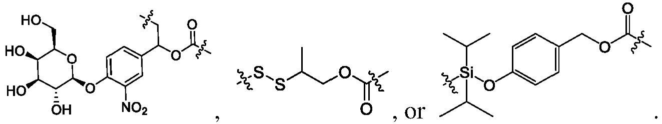

41. The conjugate compound or the pharmaceutically acceptable salt thereof according to any one of claims 1-40, wherein the conjugate compound has the following structures: 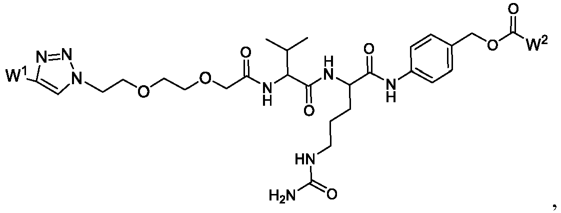 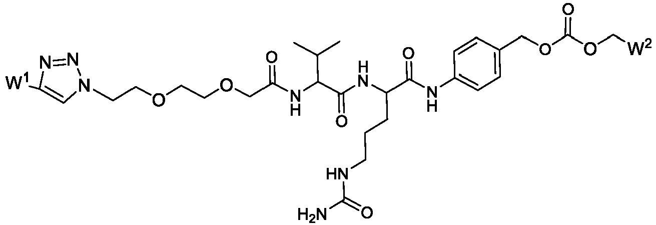 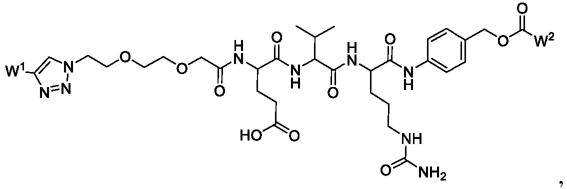 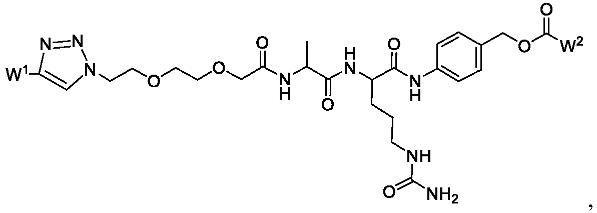 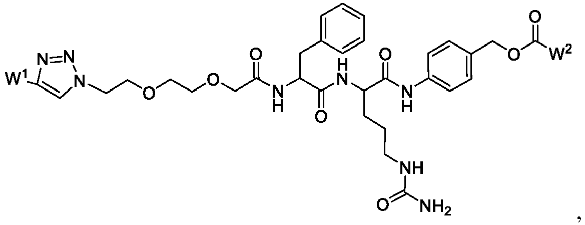 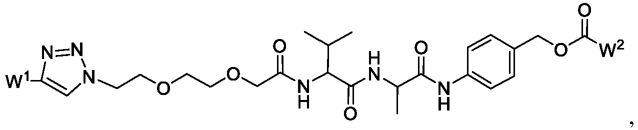 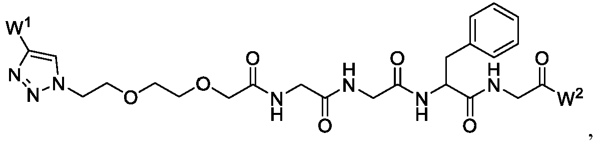 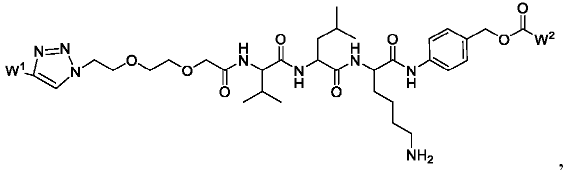 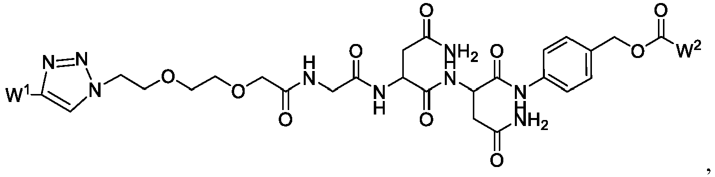 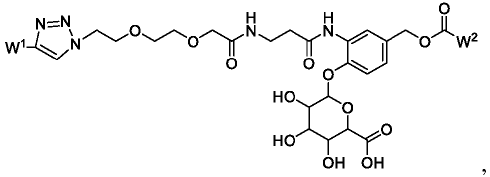 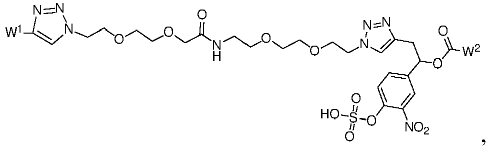 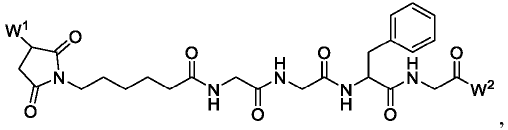 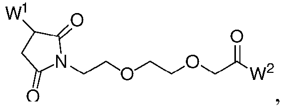 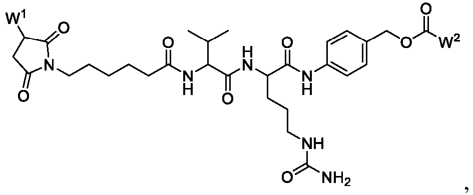 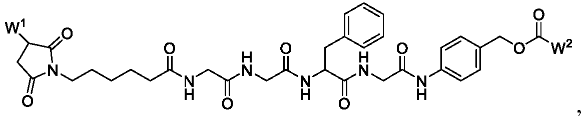 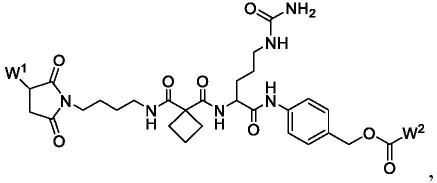 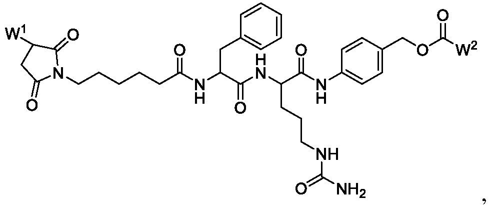 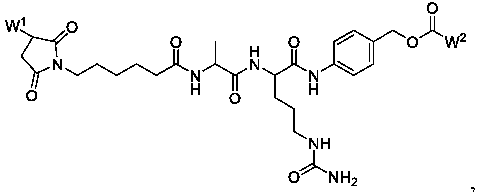 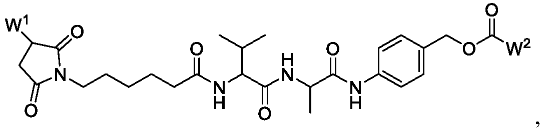 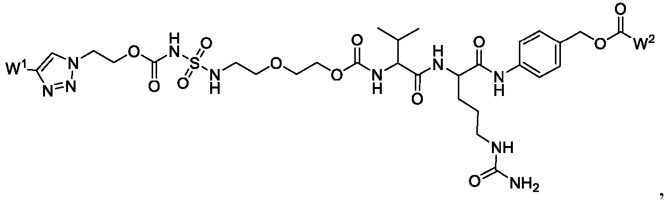 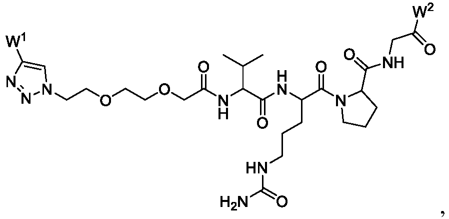 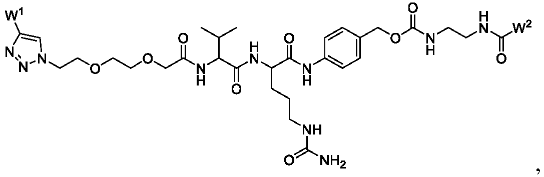 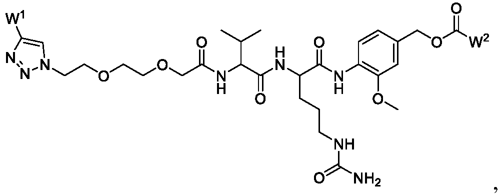 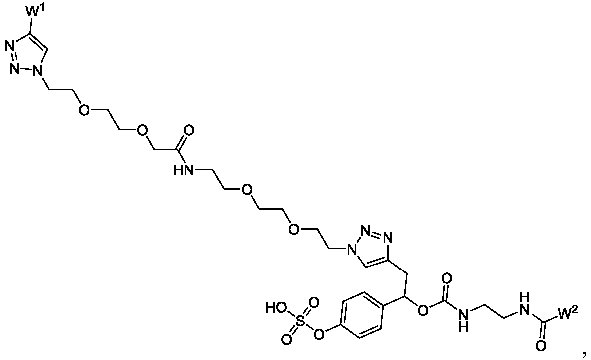 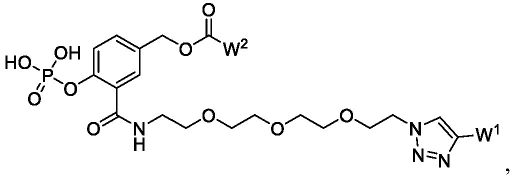 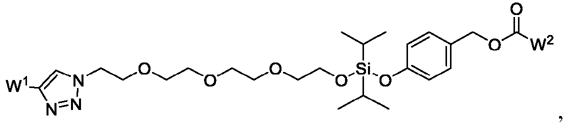 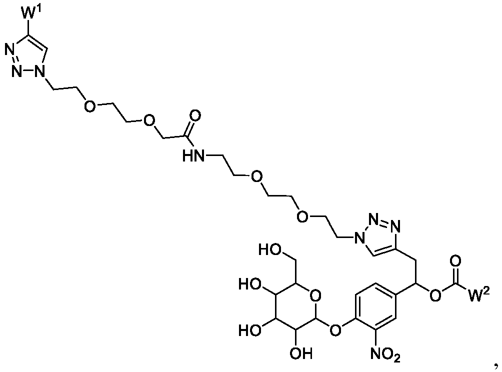 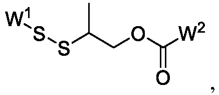 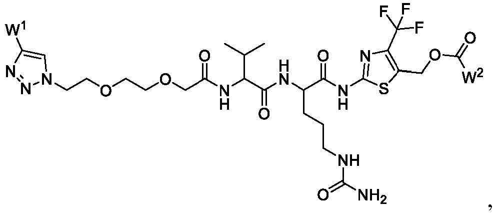 or 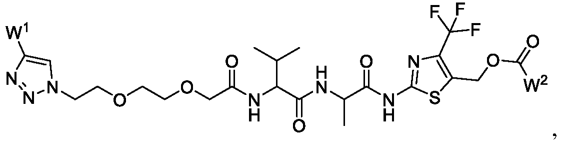 wherein W¹ is the targeted molecule capable of binding to the cell surface protein, and W² is the payload.

42. The conjugate compound or the pharmaceutically acceptable salt thereof according to any one of claims 1-41, wherein the conjugate compound has the following structures:
| | |
|---|---|
| CR202CE | 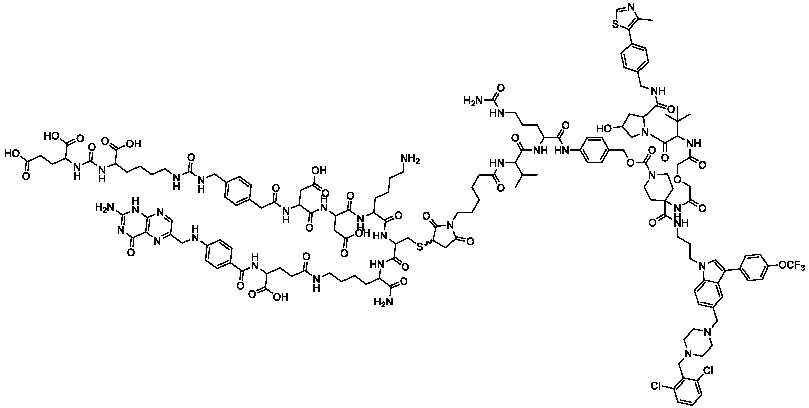 |
| CR202CH | 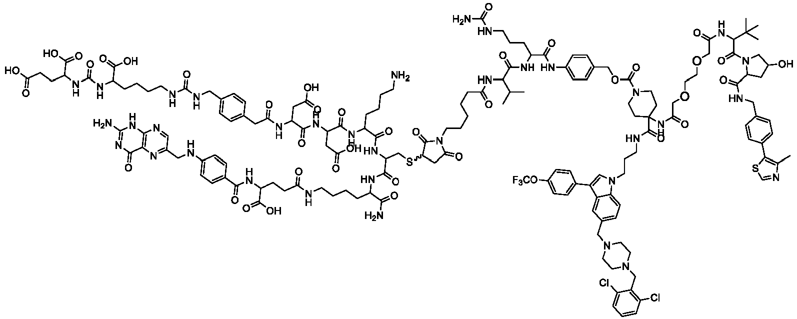 |
| CR202CK | 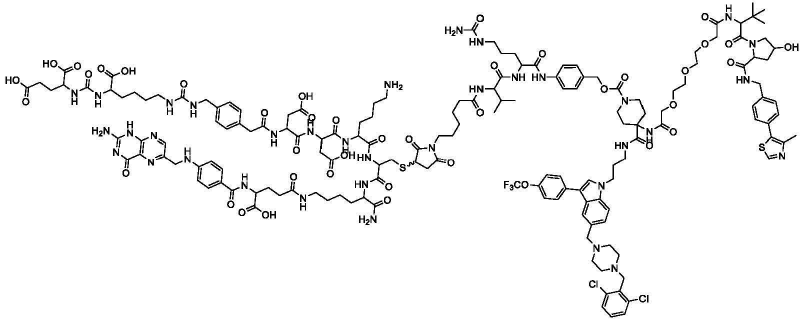 |
| | |
|---|---|
| CR202CN | 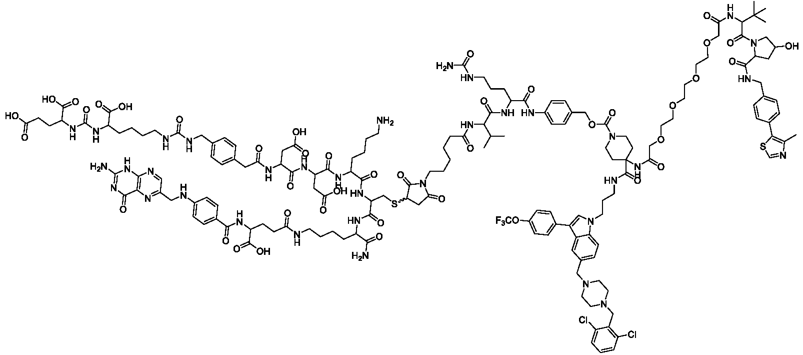 |
| CR202CR | 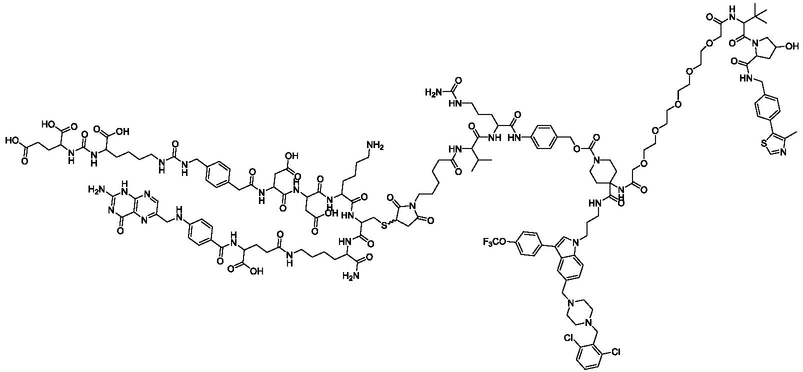 |
| CR202F0 | 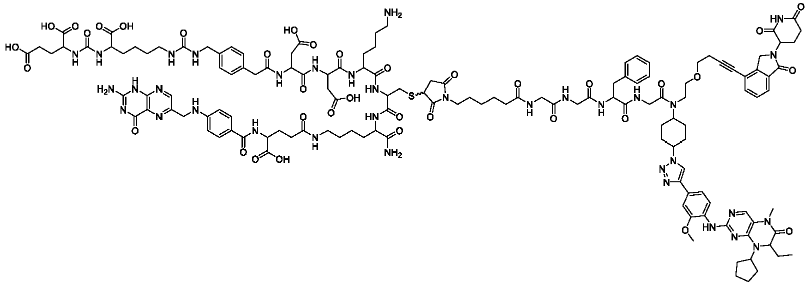 |
| CR202F2 | 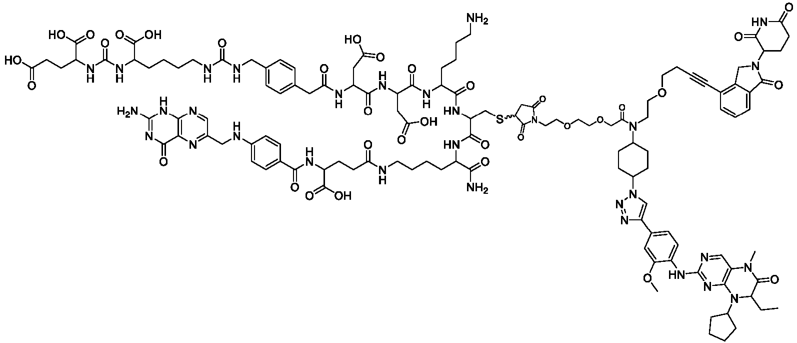 |
| | |
|---|---|
| CR20214 | 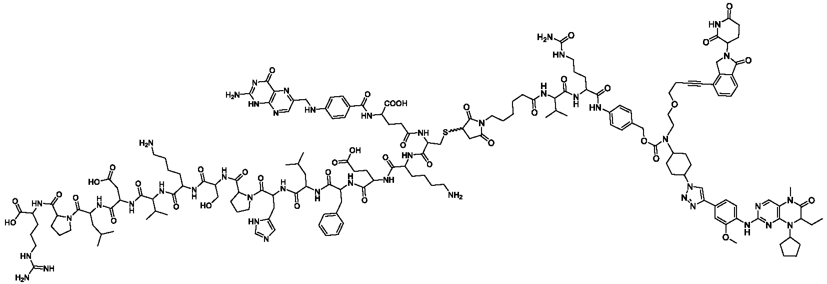 |
| CR20282 | 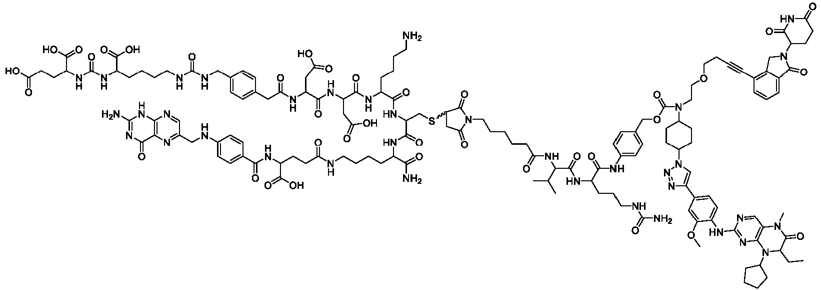 |
| CR20285 | 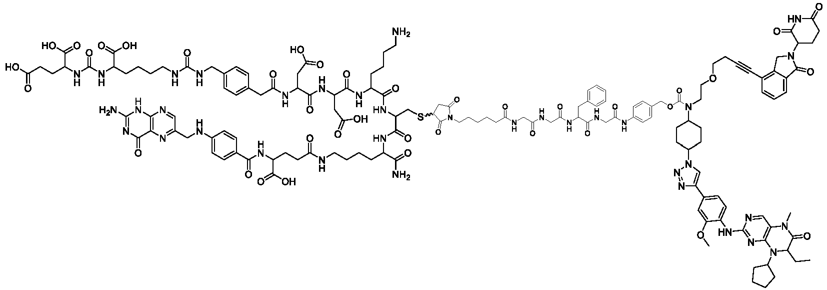 |
| CR20287 | 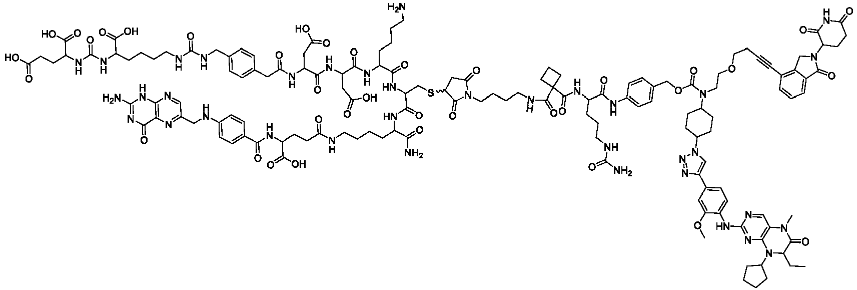 |
| | |
|---|---|
| CR20293 | 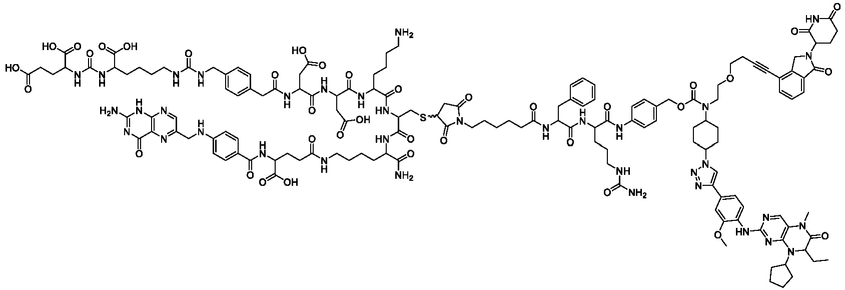 |
| CR20295 | 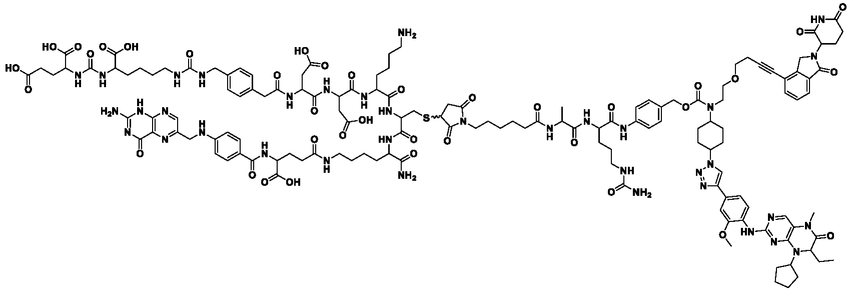 |
| CR20297 | 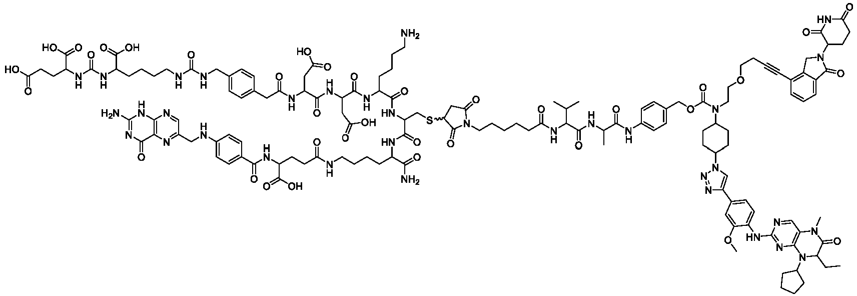 |
| CR202EF | 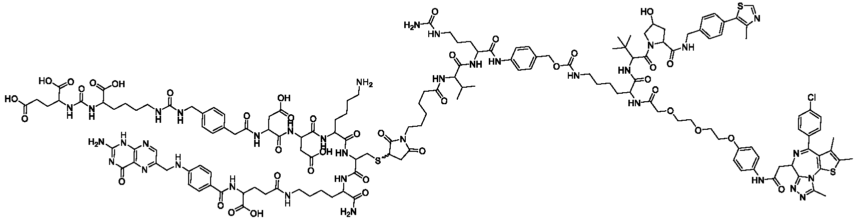 |
| CR202W5 | 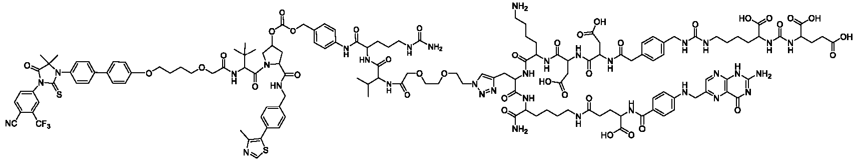 |
| | |
|---|---|
| CR202W6 | 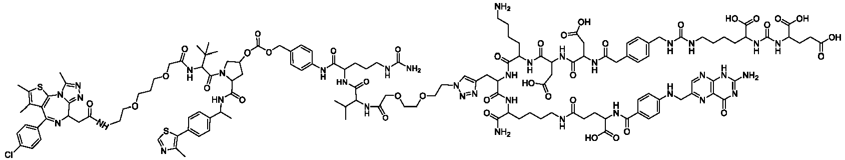 |
| CR202W7 | 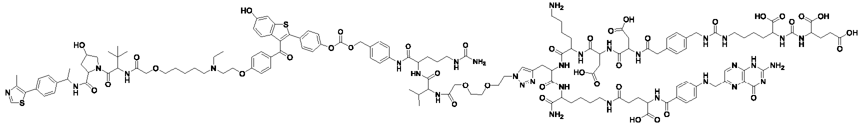 |
| CR202W8 | 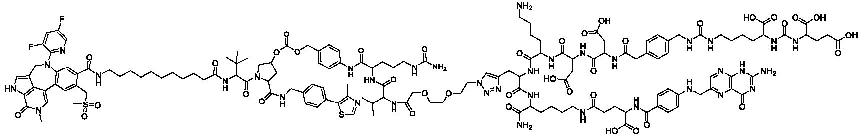 |
| CR202W9 | 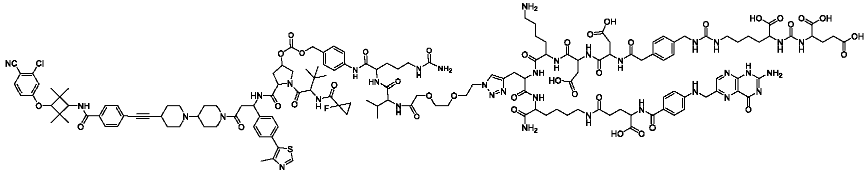 |
| CR202X0 | 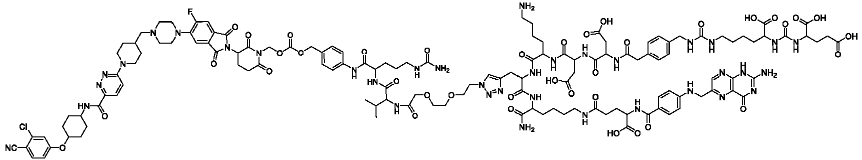 |
| CR202Xl | 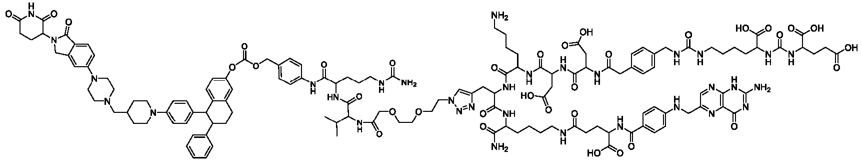 |
| CR202X2 | 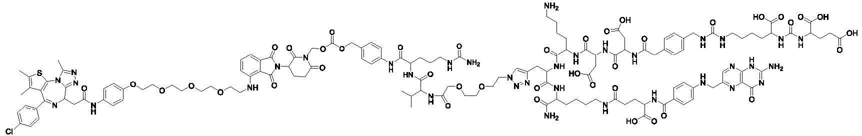 |
| CR202X3 | 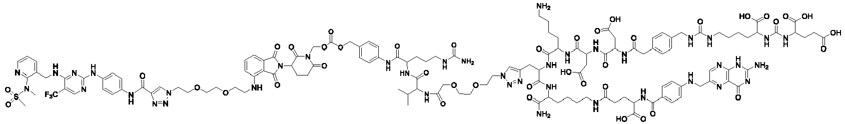 |
| | |
|---|---|
| CR202Y3 | 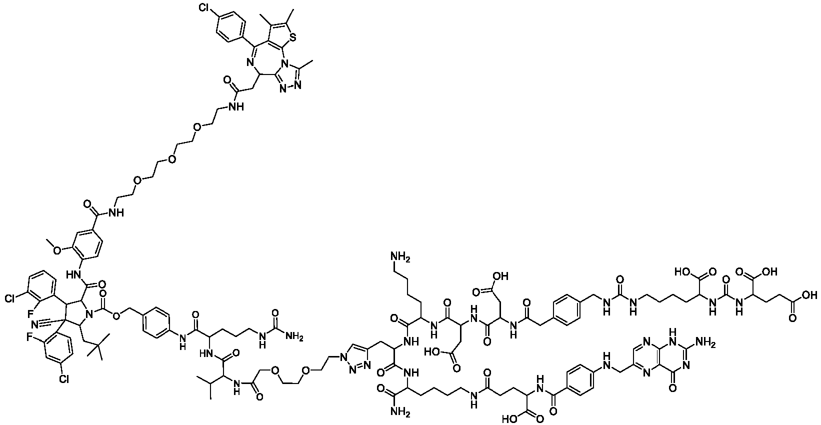 |
| CR202Y5 | 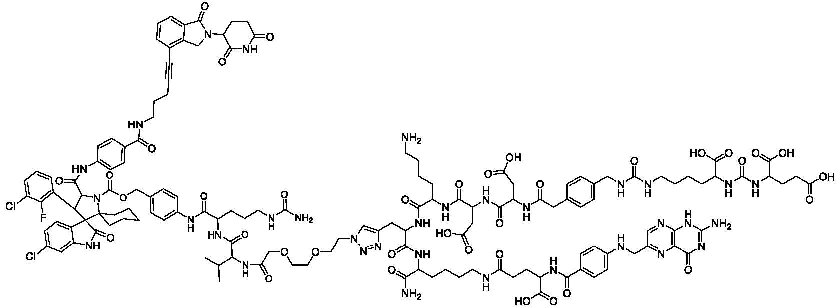 |
| CR202V4 | 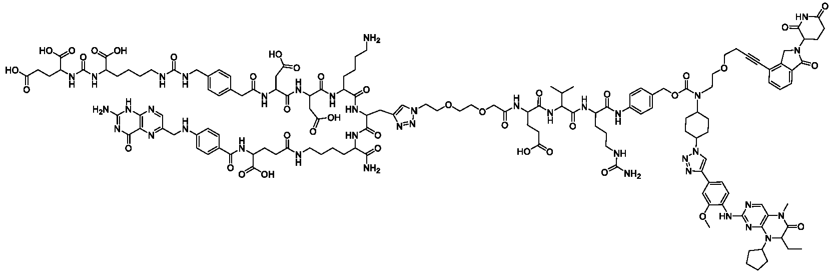 |
| CR202V5 | 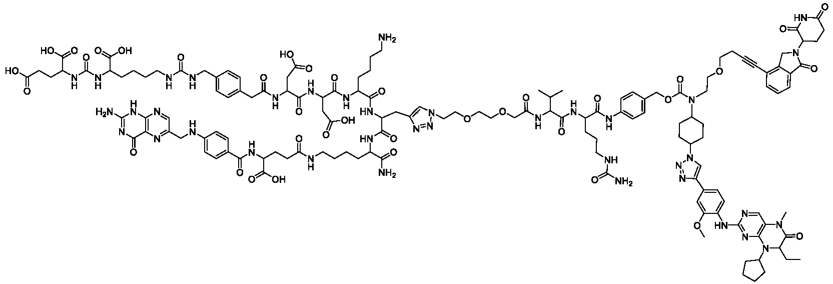 |
| | |
|---|---|
| CR202V6 | 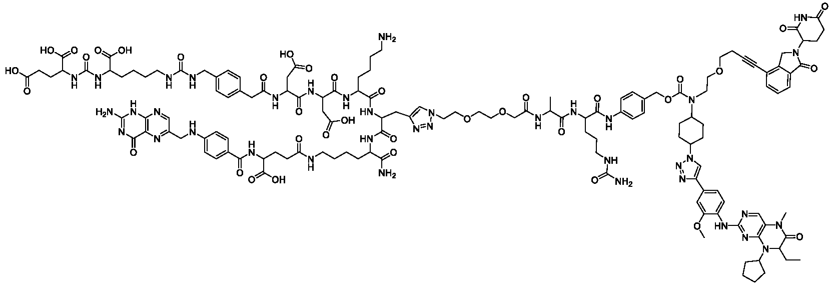 |
| CR202V7 | 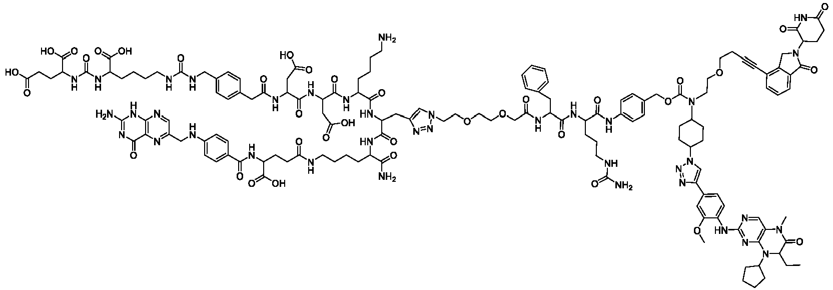 |
| CR202Z6 | 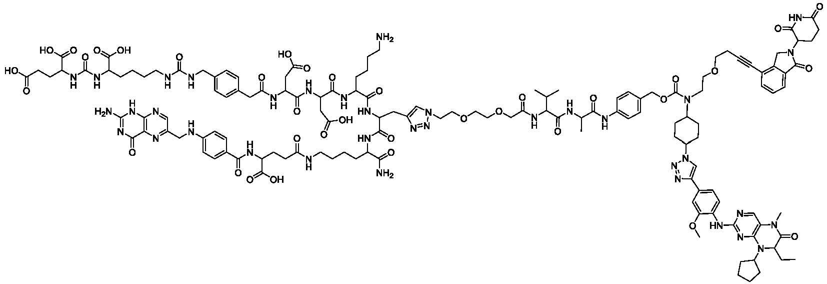 |
| CR202V8 | 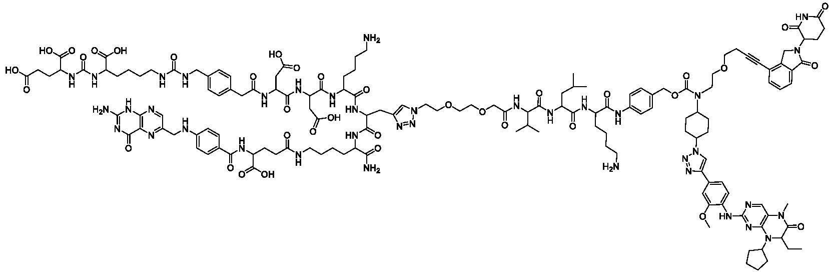 |
| | |
|---|---|
| CR202V9 | 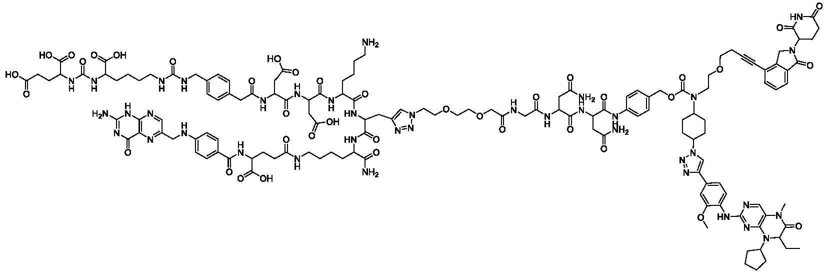 |
| CR202AL | 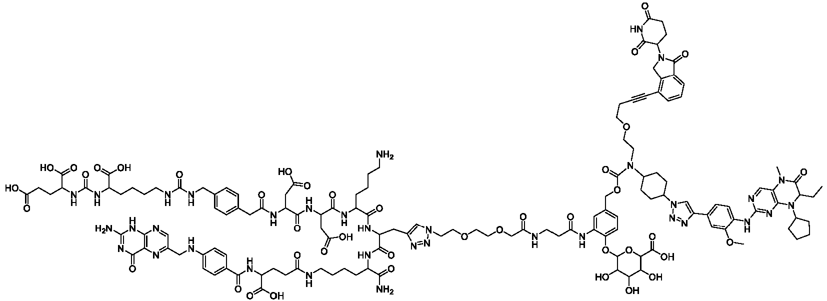 |
| CR202W1 | 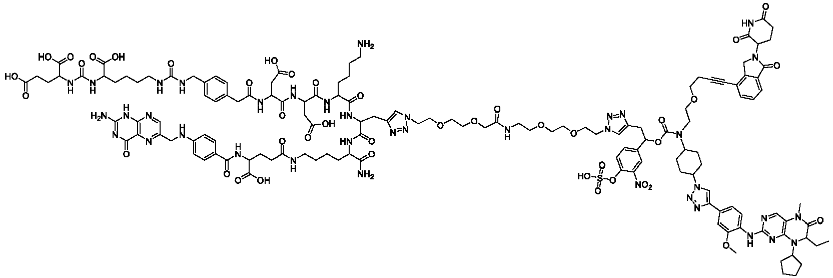 |
| CR202GM | 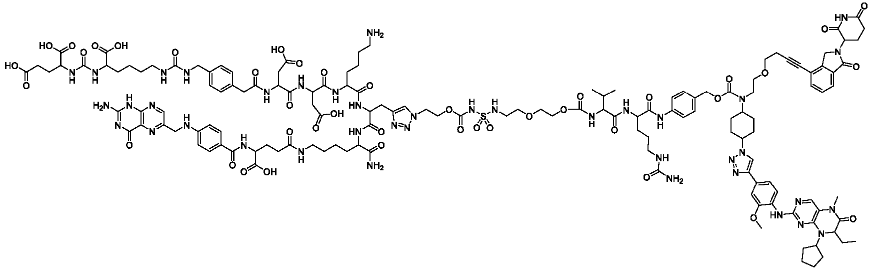 |
| | |
|---|---|
| CR202GQ | 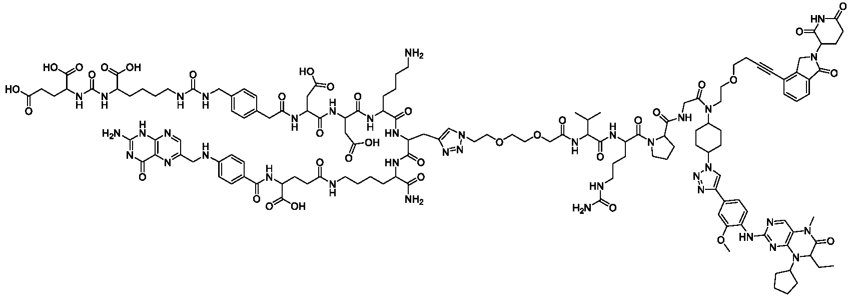 |
| CR202HJ | 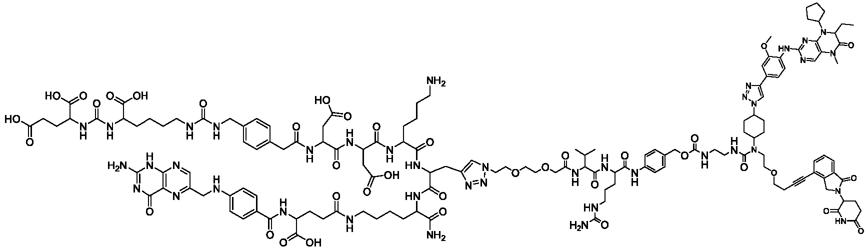 |
| CR202FU | 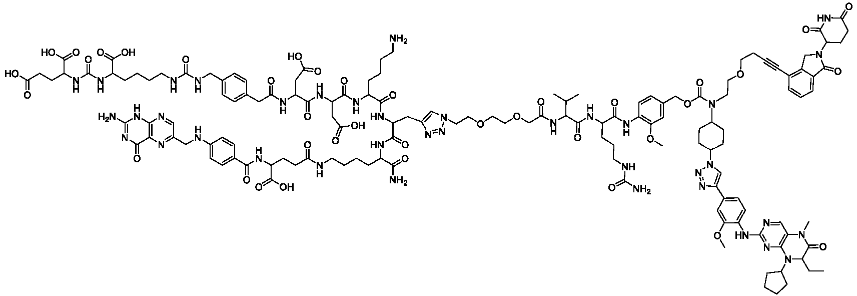 |
| CR202JG | 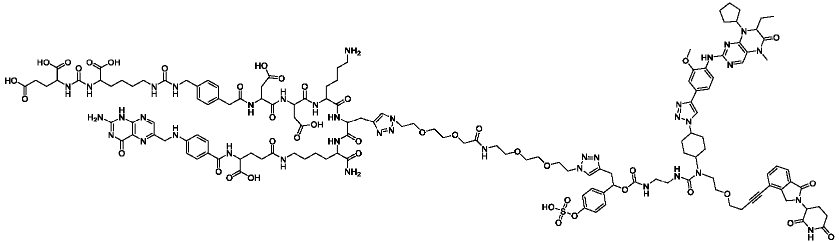 |
| | |
|---|---|
| CR202LB | 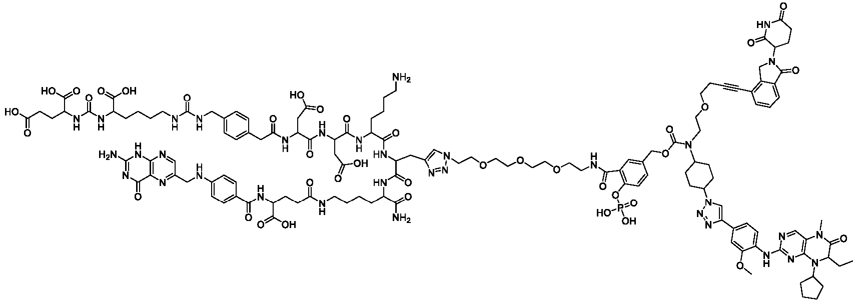 |
| CR202HT | 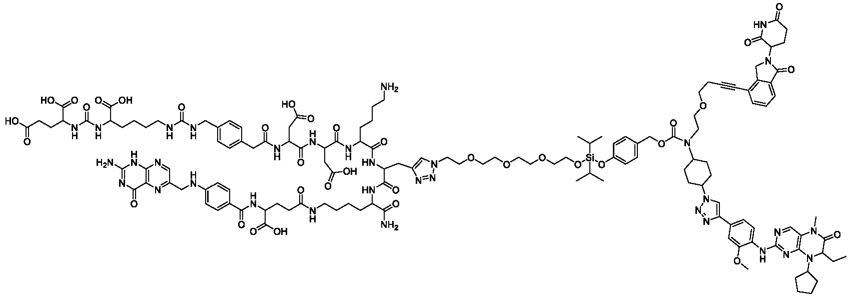 |
| CR202HU | 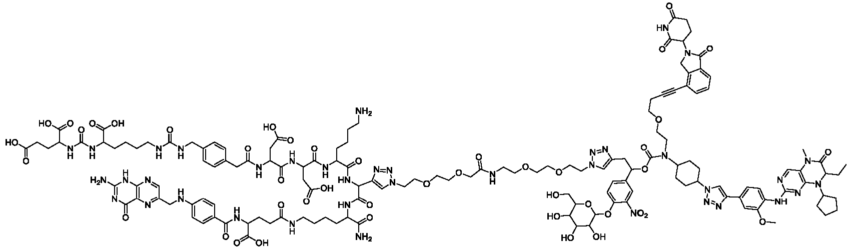 |
| CR202HV | 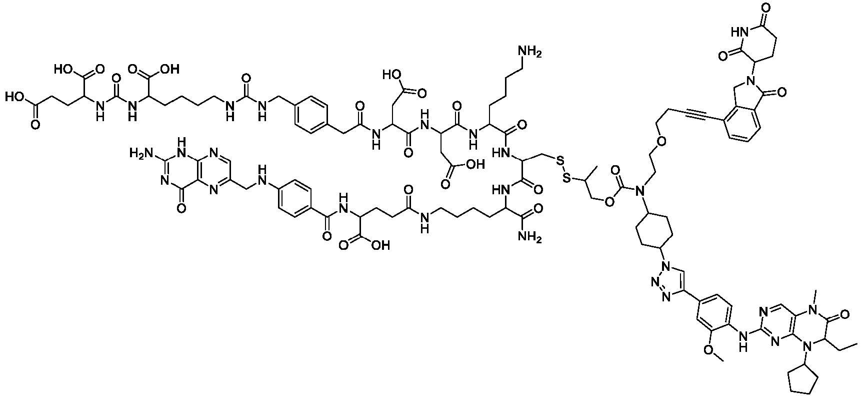 |
| | |
|---|---|
| CR202FX | 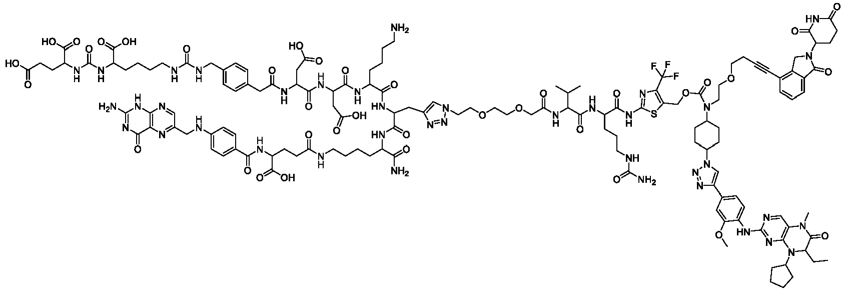 |
| CR202HR | 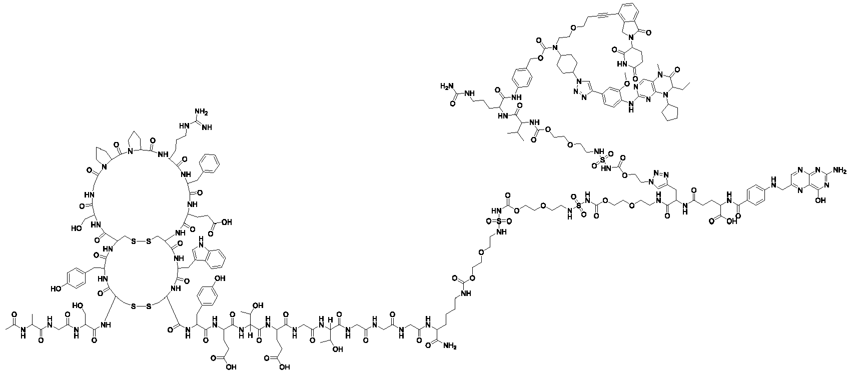 |
| CR202GC | 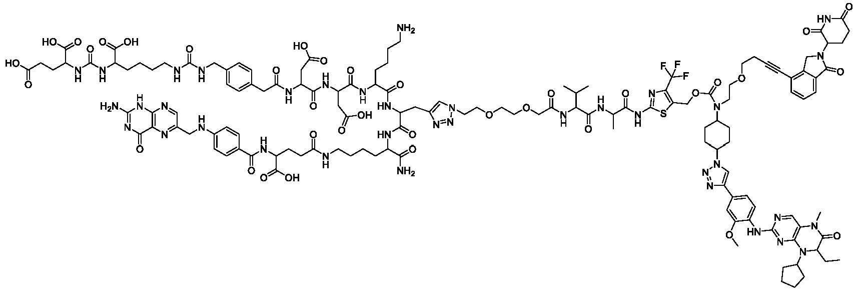 |
| CR202MT | 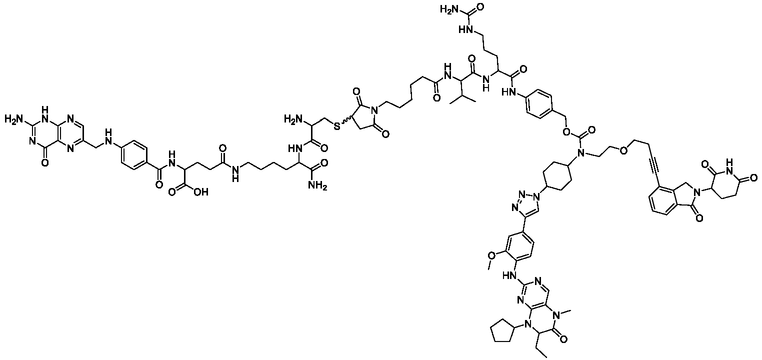 |
| | |
|---|---|
| CR202EZ | 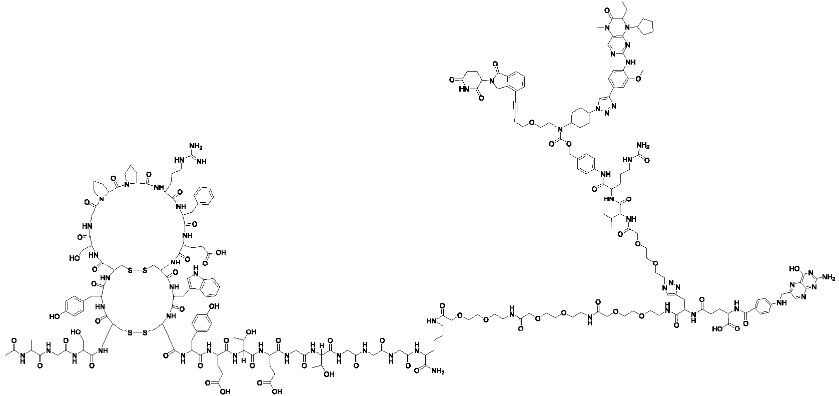 |

43. A pharmaceutical composition, comprising the conjugate compound or the pharmaceutically acceptable salt thereof according to any one of claims 1-42, and a pharmaceutically acceptable carrier.

44. The pharmaceutical composition according to claim 43, wherein the composition is for intravenous, subcutaneous, oral, intramuscular, or intraventricular administration.

45. A method for treating a disease in a subject, comprising administering to the subject a therapeutically effective amount of the conjugate compound or the pharmaceutically acceptable salt thereof according to any one of claims 1-42 or the pharmaceutical composition according to claim 43 or 44.

46. The method according to claim 45, wherein the disease is selected from the following group: a cancer, an immune disease, a cardiovascular disease, a metabolic disease, and a neurological disease.

47. The method according to claim 46, wherein the cancer is **characterized by** cancer cells overexpressing FOLR1, TRPV6, PSMA, or c-MET.

48. The method according to claim 47, wherein the cancer is **characterized by** cancer cells overexpressing FOLR1 and TRPV6, FOLR1 and c-MET, c-MET and TRPV6, c-MET and PSMA, PSMA and TRPV6, or FOLR1 and PSMA.

49. The method according to any one of claims 45-48, wherein the disease is **characterized by** expression of the target protein or abnormal expression of the target protein in the subject.

50. The method according to claim 46, wherein the cancer is selected from the following group: human breast ductal carcinoma, human brain astroblastoma, transitional cell papilloma of the bladder, prostate cancer, breast cancer, lung cancer, kidney cancer, leukemia, ovarian cancer, gastric cancer, cervical cancer, uterine cancer, endometrial cancer, liver cancer, colon cancer, thyroid cancer, pancreatic cancer, colorectal cancer, esophageal cancer, testicular cancer, skin cancer, lymphoma, and multiple myeloma.

51. The method according to claim 46, wherein the immune disease is an autoimmune disease; optionally, the autoimmune disease is selected from the following group: connective tissue disorder, systemic sclerosis, rheumatoid arthritis, and systemic lupus erythematosus.

52. The method according to claim 46, wherein the cardiovascular disease is selected from the following group: angina pectoris, myocardial infarction, stroke, heart attack, hypertensive heart disease, rheumatic heart disease, cardiomyopathy, cardiac arrhythmia, and congenital heart disease.

53. The method according to claim 46, wherein the metabolic disease is selected from the following group: diabetes, gout, obesity, hypoglycemia, hyperglycemia, and dyslipidemia.

54. The method according to claim 46, wherein the neurological disease is selected from the following group: Alzheimer's disease, Parkinson's disease, Huntington's disease, head injury, multiple sclerosis, vertigo, coma, and epilepsy.

55. The method according to any one of claims 45-54, further comprising administering one or more therapeutic agents in combination with the conjugate compound or the pharmaceutically acceptable salt thereof or the pharmaceutical composition.

56. Use of the conjugate compound or the pharmaceutically acceptable salt thereof according to any one of claims 1-42, or the pharmaceutical composition according to claim 43 or 44 in preparing a medicament for treating a disease in a subject.

57. A compound or a salt thereof, wherein the compound has the following structure:
L^{a}-(EG)n¹-(D)n⁵-(CH₂)n²-(B)n⁶-(AA)n³-(CH₂CH₂-Q)n⁴-L^{b},
wherein,
L^{a} is 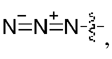 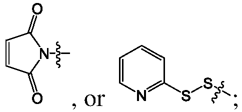
EG is -CH₂CH₂O- or -OCH₂CH₂-; n¹ is an integer of 0-6;
n² is an integer of 0-8;
D is -C(=O)-NH-S(=O)₂-NH-; n⁵ is 0 or 1;
B is -C(=O)-, 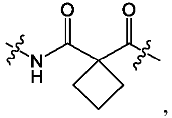 -NH-CH₂CH₂-C(=O)-, -C(=O)-NH-, -CH(CH₃)-CH₂-O-, -CH₂CH₂O-, -NH-C(=O)-, or -NH-; n⁶ is an integer of 0-3;
AA is each independently an amino acid residue; n³ is an integer of 0-5;
Q is each independently -C(=O)-, -O-, or a bond; n⁴ is an integer of 0-3;
L^{b} is 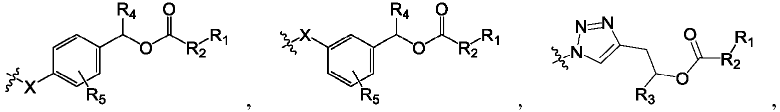 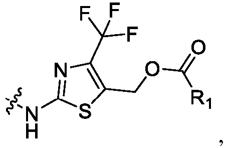 -OH, or -NH-CH₂-OH;
wherein X is a bond, -NH-, -C(=O)-, oxygen, or 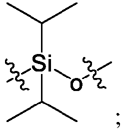
R₂ is a bond, -O-CH₂-, or -NH-CH₂CH₂-NH-C(=O)-;
R₃ is hydrogen or 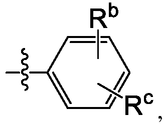 wherein R^{b} and R^{c} are each independently hydrogen, nitro, 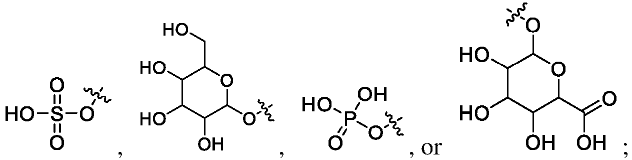
R₄ is hydrogen or C₁₋₈ alkyl;
R₅ is hydrogen, amino, nitro, phosphate group, -OR^{a}, or 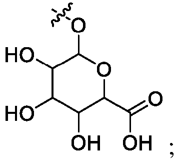 wherein R^{a} is methyl, ethyl, propyl, or isopropyl;
R₁ is a leaving group, preferably R₁ is halogen (e.g., chlorine), 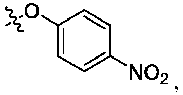 or 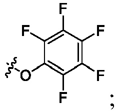
preferably, L^{b} is 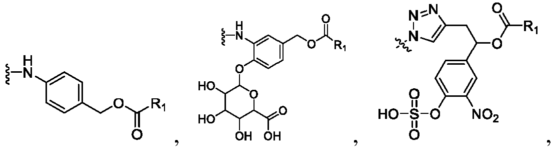 -OH, -NH-CH₂-OH, 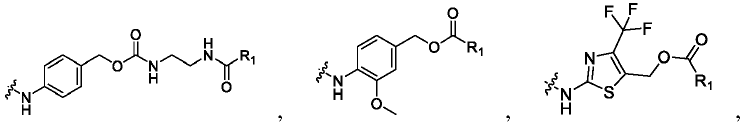 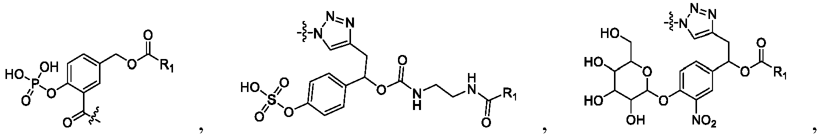 or 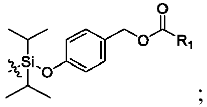
preferably, EG is -CH₂CH₂O-;
preferably, n¹ is 0 or 2;
preferably, n² is 1, 4, or 5;
preferably, n⁵ is 0;
preferably, n⁶ is 1 or 3;
preferably, n¹ is 2, n² is 1, n³ and n⁴ are 0, and L^{b} is -OH;
preferably, AA is each independently an amino acid residue formed by Val, Cit, Glu, Leu, Lys, Ala, Gly, Phe, Gln, Pro, or Asn;
preferably, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Cit-, -Val-Leu-Lys-, -Val-Cit-Pro-Gly-, -Gly-Asn-Asn-, and -Ala-Ala-Asn-;
preferably, when n⁴ is 1, Q is -C(=O)-;
preferably, when n⁴ is 2, Q are -O- and -C(=O)-, respectively;
preferably, when n⁴ is 3, Q are -O-, -O-, and a bond, respectively; or
preferably, n⁴ is 0.

58. The compound or the salt thereof according to claim 57, wherein the compound has the following structure:
L^{a}-(EG)n¹-(D)n⁵-(CH₂)n²-(B)n⁶-(AA)n³-(CH₂CH₂-Q)n⁴-L^{b}
wherein,
L^{a} is 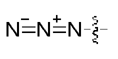 or 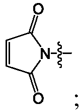
EG is -CH₂CH₂O-; n¹ is an integer of 2-4;
D is -C(=O)-NH-S(=O)₂-NH-; n⁵ is 0 or 1;
n² is an integer of 0-5;
B is -C(=O)-, -C(=O)-NH-, -CH₂CH₂O-, -NH-CH₂CH₂-C(=O)-, or -NH-; n⁶ is an integer of 0-3;
AA is each independently an amino acid residue; n³ is an integer of 0-3;
Q is each independently -C(=O)-, -O-, or a bond; n⁴ is an integer of 0-3;
L^{b} is -OH, 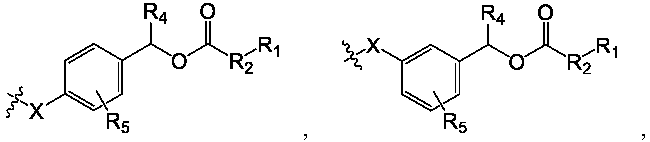 or 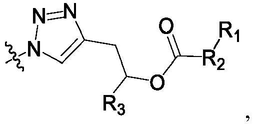
wherein X is a bond, -NH-, -C(=O)-, or
R₂ is a bond; 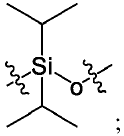
R₃ is 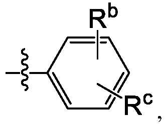 wherein R^{b} and R^{c} are each independently hydrogen, nitro, or 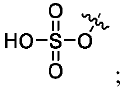
R₄ is hydrogen;
R₅ is hydrogen, -OCH₃, or 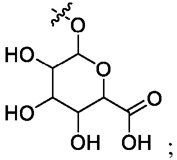
R₁ is a leaving group, preferably R₁ is halogen (e.g., chlorine), 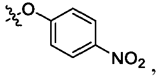 or 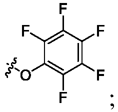
preferably, L^{b} is -OH, 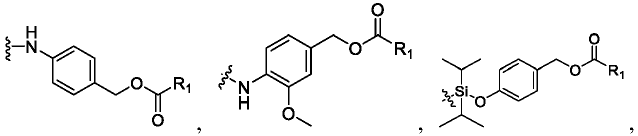 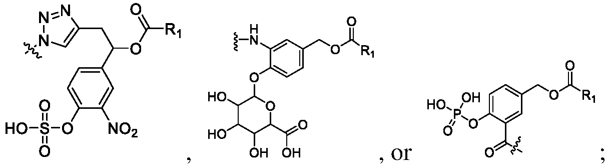
preferably, when n⁵ is 1, n² and n⁴ are both 0;
preferably, L^{a} is 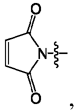 and n¹, n⁵, and n⁴ are all 0;
preferably, L^{a} is 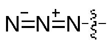 , and n² is 1 or 2;
preferably, AA is each independently an amino acid residue formed by Val, Cit, Glu, or Ala; or
preferably, -(AA)n³- is a peptide residue selected from the following group: -Val-Cit-, -Glu-Val-Cit-, -Ala-Cit-, and -Val-Ala-.

59. The compound or the salt thereof according to claim 57 or 58, wherein the compound has the following structure:
L^{a}-(EG)n¹-(CH₂)n²-(C=O)-(AA)n³-L^{b}
wherein,
L^{a} is 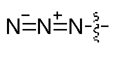 or 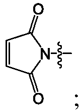
EG is -CH₂CH₂O-; n¹ is an integer of 0-4;
n² is an integer of 0-5;
AA is each independently an amino acid residue; n³ is an integer of 0-3;
L^{b} is 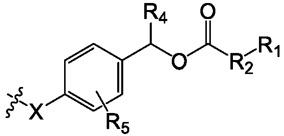 or -OH,
wherein X is -NH-; R₂ is a bond; R₄ is hydrogen; R₅ is hydrogen or -OCH₃;
R₁ is a leaving group, preferably R₁ is halogen (e.g., chlorine), 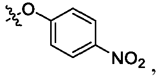 or 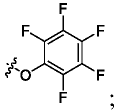
preferably, L^{a} is 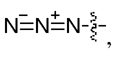 , n¹ is 2, n² is 1, and L^{b} is 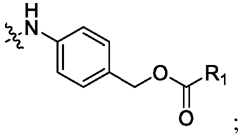
preferably, L^{a} is 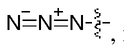 n¹ is 2, n² is 1, and L^{b} is -OH;
preferably, L^{a} is 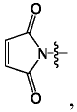 n¹ is 0, n² is 5, and L^{b} is 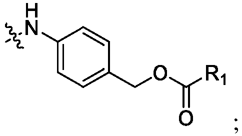
preferably, L^{a} is 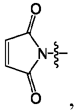 n¹ is 0, n² is 5, and L^{b} is -OH;
preferably, AA is each independently Val, Cit, Glu, or Ala amino acid residue; or
preferably, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Val-Leu-Lys-, and -Gly-Asn-Asn-.

60. The compound or the salt thereof according to any one of claims 57-59, wherein the compound has a structure selected from the following group: 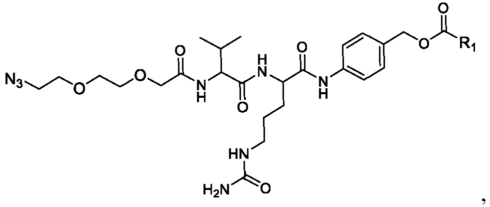 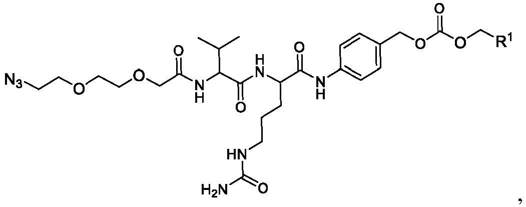 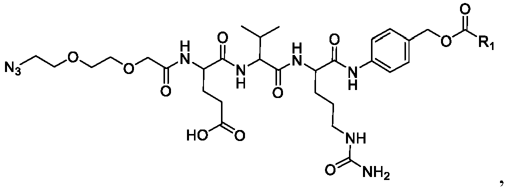 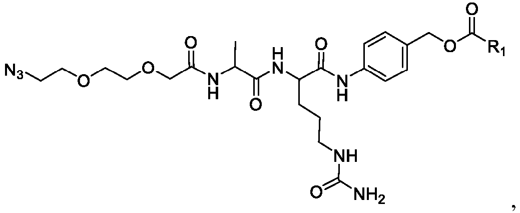 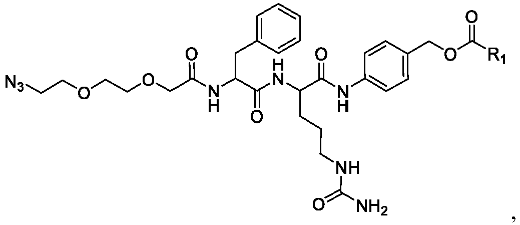 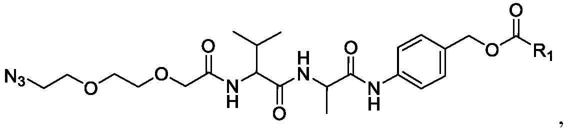 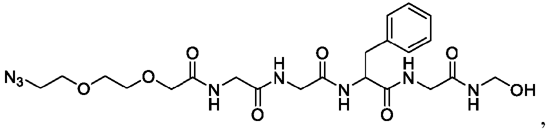 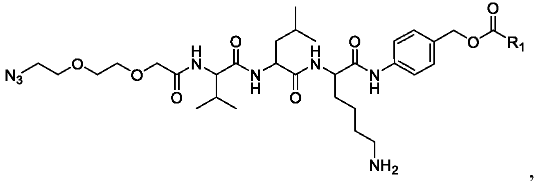 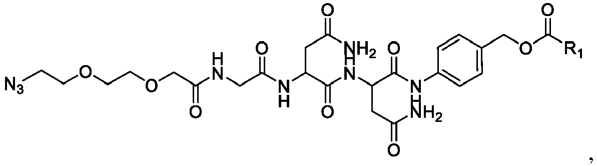 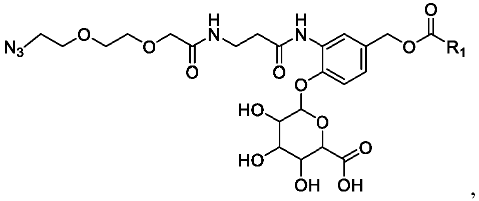 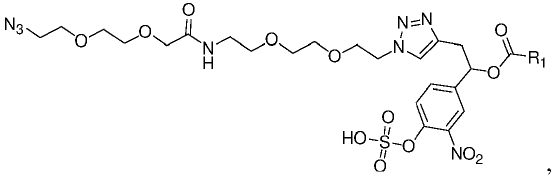 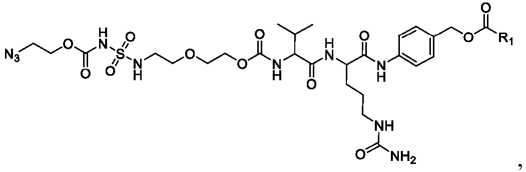 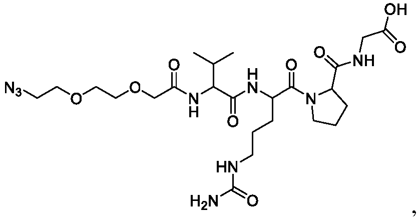 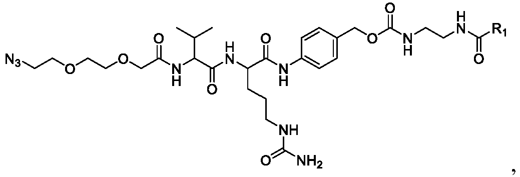 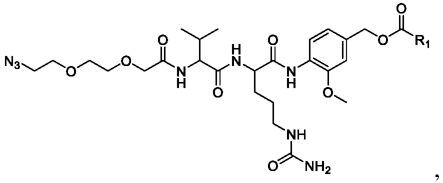 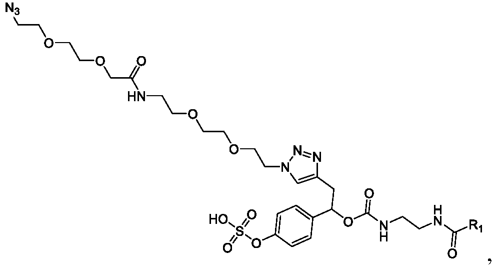 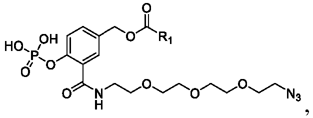 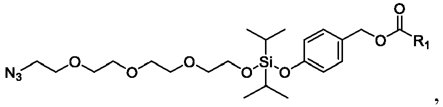 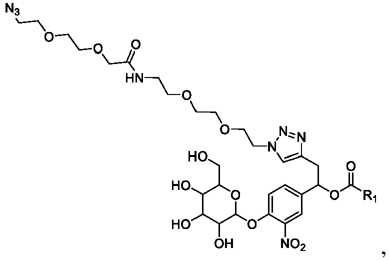 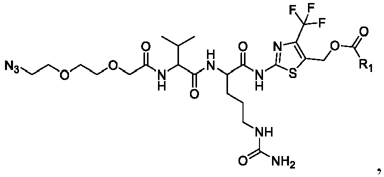 and 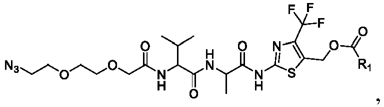 wherein R₁ is a leaving group, preferably R₁ is halogen (e.g., chlorine), 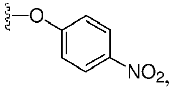 or

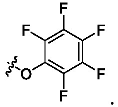

61. A compound or a salt thereof, wherein the compound is

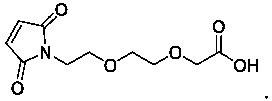

62. A conjugate compound or a pharmaceutically acceptable salt thereof, wherein the conjugate compound has the following structure:
W^{a}-L¹-(EG)n¹-(D)n⁵-(CH₂)n²-(B)n⁶-(AA)n³-(CH₂CH₂-Q)n⁴-L²-W^{b},
wherein,
L¹ is 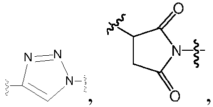 -S-S-, or a bond;
EG is -CH₂CH₂O- or -OCH₂CH₂-; n¹ is an integer of 0-6;
n² is an integer of 0-8;
D is -C(=O)-NH-S(=O)₂-NH-; n⁵ is 0 or 1;
B is each independently -C(=O)-, 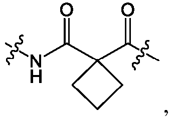 -NH-CH₂CH₂-C(=O)-, -C(=O)-NH-, -CH(CH₃)-CH₂-O-, -CH₂CH₂O-, -NH-C(=O)-, or -NH-; n⁶ is an integer of 0-3;
AA is each independently an amino acid residue; n³ is an integer of 0-5;
Q is each independently -C(=O)-, -O-, or a bond; n⁴ is an integer of 0-3;
L² is 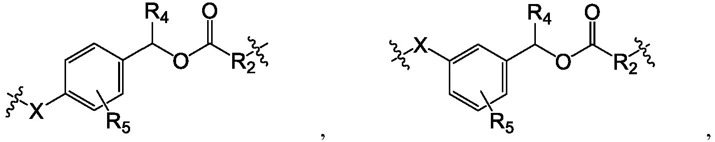 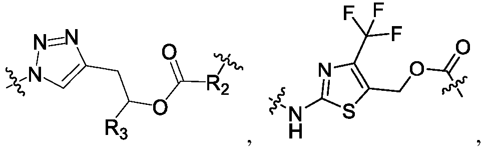 -NH-CH₂-OH, or a bond;
wherein X is a bond, -NH-, -C(=O)-, oxygen, or 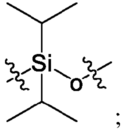
R₂ is a bond, -O-CH₂-, or -NH-CH₂CH₂-NH-C(=O)-;
R₃ is hydrogen or 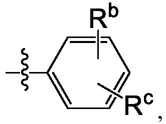 wherein R^{b} and R^{c} are each independently hydrogen, nitro, 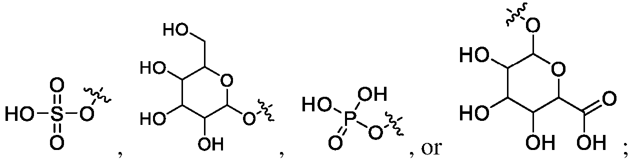
R₄ is hydrogen or C₁₋₈ alkyl;
R₅ is hydrogen, amino, nitro, phosphate group, -OR^{a}, or 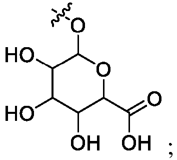 wherein R^{a} is methyl, ethyl, propyl, or isopropyl;
W^{a} and W^{b} are each independently a payload or a targeted molecule capable of binding to a cell surface protein;
preferably, L² is a bond, 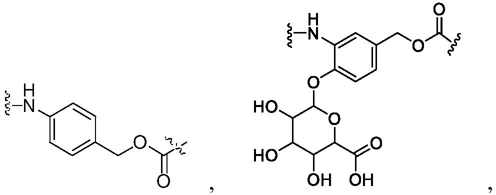 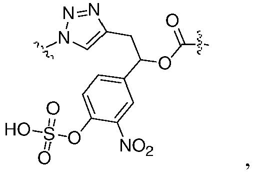 -NH-CH₂-O-, 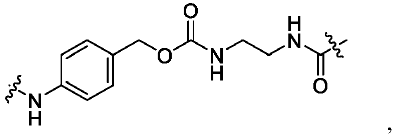 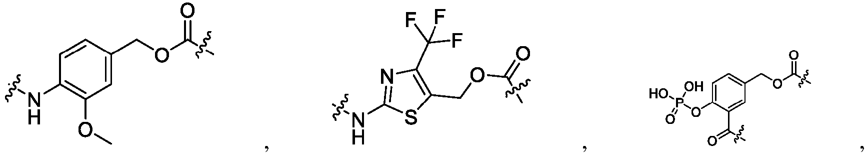 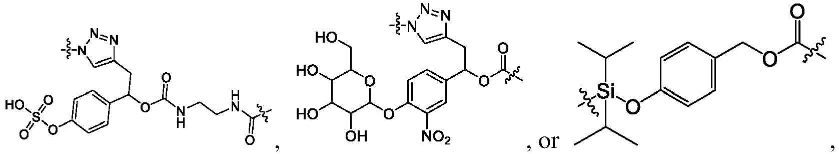
preferably, EG is -CH₂CH₂O-;
preferably, n¹ is 0 or 2;
preferably, n² is 1, 4, or 5;
preferably, n⁵ is 0;
preferably, n⁶ is 1 or 3;
preferably, n¹ is 2, n² is 1, n³ and n⁴ are 0, and L² is a bond;
preferably, AA is each independently an amino acid residue formed by Val, Cit, Glu, Leu, Lys, Ala, Gly, Phe, Gln, Pro, or Asn;
preferably, -(AA)n³- is a peptide residue selected from the following group: -Val-Cit-, -Glu-Val-Cit-, -Ala-Cit-, -Phe-Cit-, -Val-Ala-, -Gly-Gly-Phe-Gly-, -Cit-, -Val-Leu-Lys-, -Val-Cit-Pro-Gly-, -Gly-Asn-Asn-, and -Ala-Ala-Asn-;
preferably, when n⁴ is 1, Q is -C(=O)-;
preferably, when n⁴ is 2, Q are -O- and -C(=O)-, respectively;
preferably, when n⁴ is 3, Q are -O-, -O-, and a bond, respectively; or
preferably, n⁴ is 0.

63. The conjugate compound or the pharmaceutically acceptable salt thereof according to claim 62, wherein the conjugate compound has the following structure:
W^{a}-L¹-(EG)n¹-(D)n⁵-(CH₂)n²-(B)n⁶-(AA)n³-(CH₂CH₂-Q)n⁴-L²-W^{b}
wherein,
L¹ is 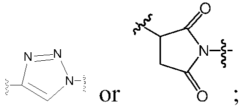
EG is -CH₂CH₂O-; n¹ is an integer of 2-4;
D is -C(=O)-NH-S(=O)₂-NH-; n⁵ is 0 or 1;
n² is an integer of 0-5;
B is -C(=O)-, -C(=O)-NH-, -CH₂CH₂O-, -NH-CH₂CH₂-C(=O)-, or -NH-; n⁶ is an integer of 0-3;
AA is each independently an amino acid residue; n³ is an integer of 0-3;
Q is each independently -C(=O)-, -O-, or a bond; n⁴ is an integer of 0-3;
L² is 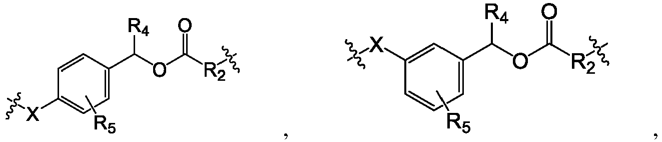 or 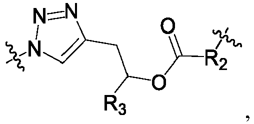
wherein X is a bond, -NH-, -C(=O)-, or 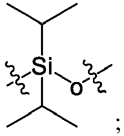
R₂ is a bond;
R₃ is 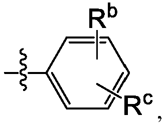 wherein R^{b} and R^{c} are each independently hydrogen, nitro, or 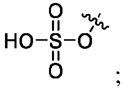
R₄ is hydrogen;
R₅ is hydrogen, -OCH₃, or 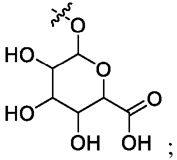
W^{a} and W^{b} are each independently a payload or a targeted molecule capable of binding to a cell surface protein;
preferably, L² is 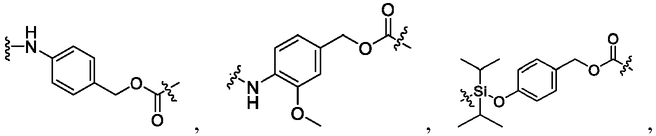 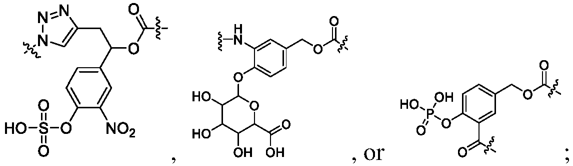
preferably, when n⁵ is 1, n² and n⁴ are both 0;
preferably, L¹ is 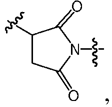 and n¹, n⁵, and n⁴ are all 0;
preferably, L¹ is 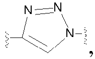 and n² is 1 or 2;
preferably, AA is each independently an amino acid residue formed by Val, Cit, Glu, or Ala; or
preferably, -(AA)n³- is a peptide residue selected from the following group: -Val-Cit-, -Glu-Val-Cit-, -Ala-Cit-, and -Val-Ala-.

64. The conjugate compound or the pharmaceutically acceptable salt thereof according to claim 61 or 62, wherein the conjugate compound has the following structure:
W^{a}-L¹-(EG)n¹-(CH₂)n²-(C=O)-(AA)n³-L²-W^{b}
wherein,
L¹ is 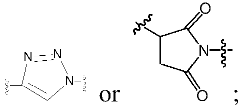
EG is -CH₂CH₂O-; n¹ is an integer of 0-4; preferably
n² is an integer of 1-5;
AA is each independently an amino acid residue; n³ is an integer of 1-4;
L² is 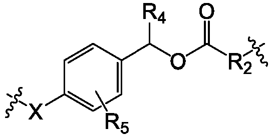 or a bond,
wherein X is -NH-; R₂ is a bond; R₄ is hydrogen; R₅ is hydrogen or -OCH₃;
W^{a} and W^{b} are each independently a payload or a targeted molecule capable of binding to a cell surface protein;
preferably, L¹ is 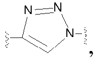 n¹ is 2, n² is 1, and L² is 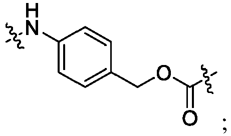
preferably, L¹ is 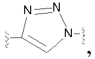 n¹ is 2, n² is 1, and L² is a bond;
preferably, L¹ is 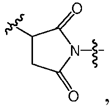 n¹ is 0, n² is 5, and L² is 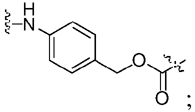
preferably, L¹ is 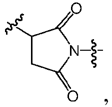 n¹ is 0, n² is 5, and L² is a bond.

65. The conjugate compound or the pharmaceutically acceptable salt thereof according to any one of claims 52-64, wherein the compound has a structure selected from the following group: 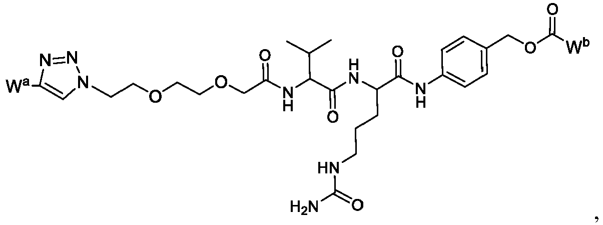 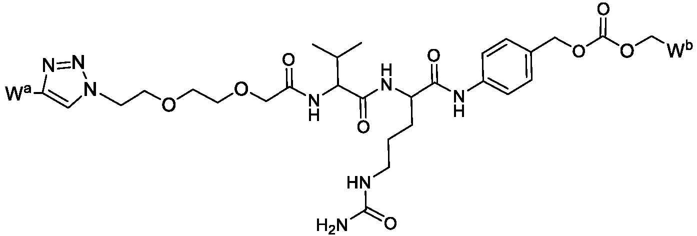 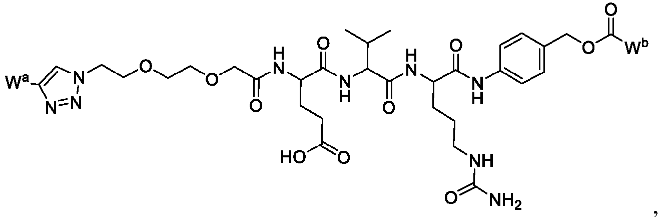 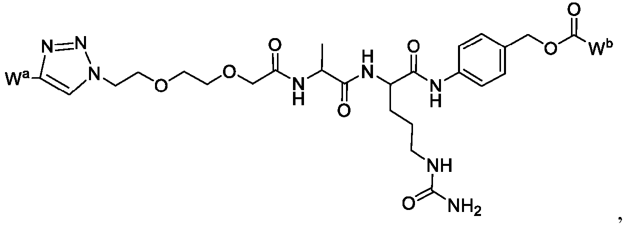 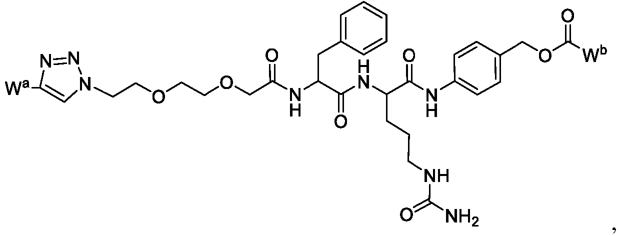 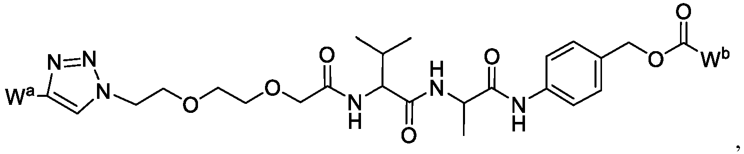 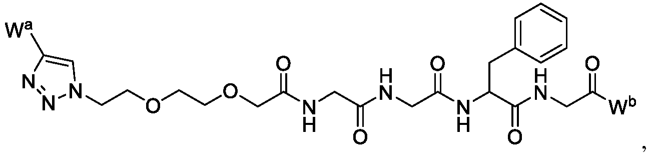 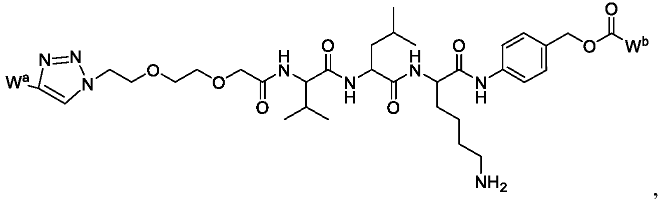 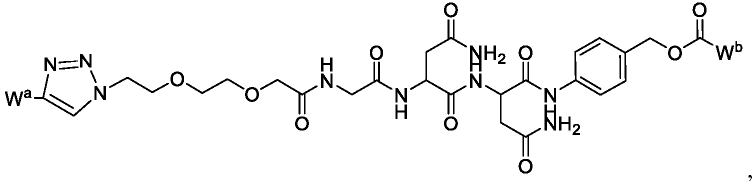 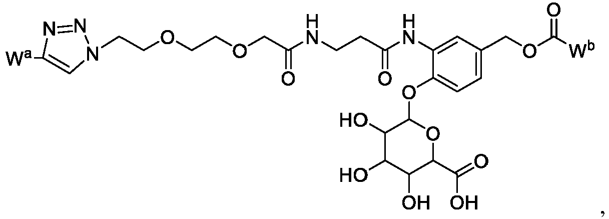 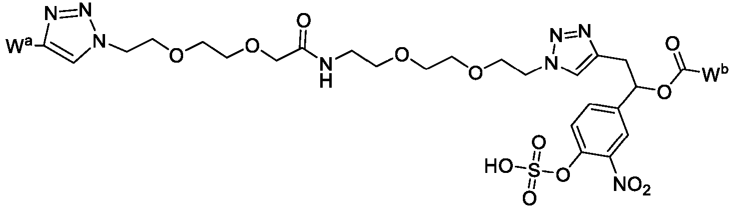 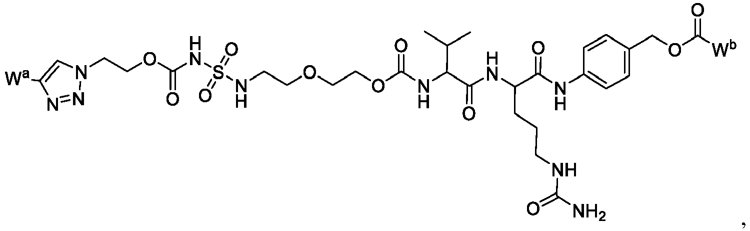 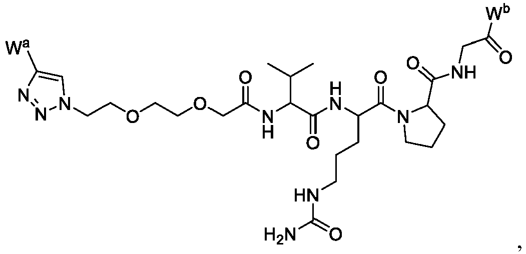 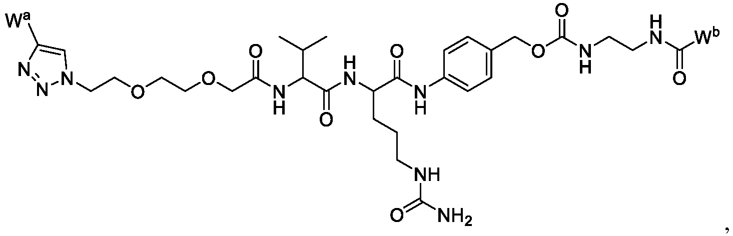 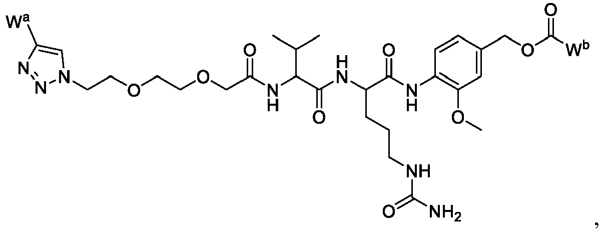 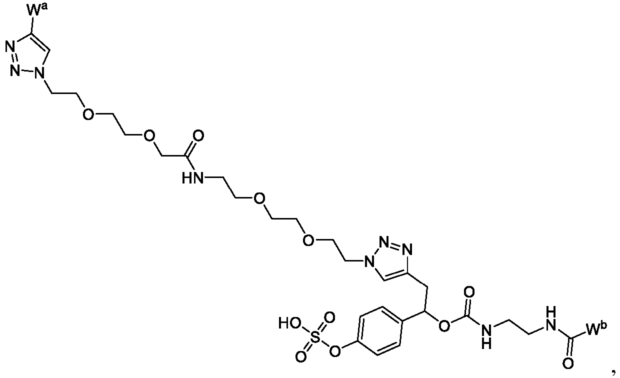 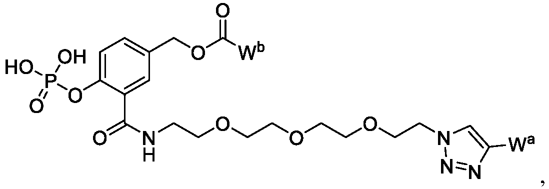 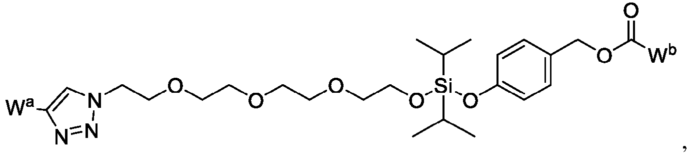 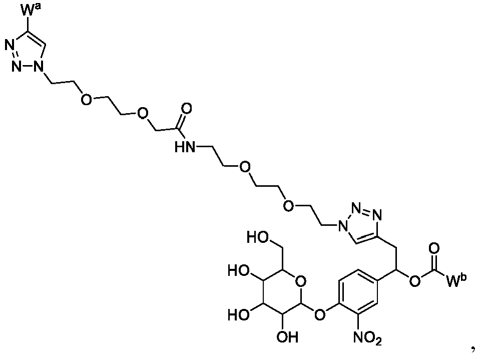 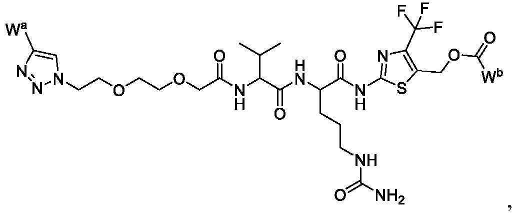 and 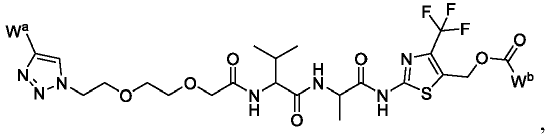 wherein W^{a} and W^{b} are each independently a payload or a targeted molecule capable of binding to a cell surface protein.

66. A conjugate compound or a pharmaceutically acceptable salt thereof, wherein the conjugate compound is 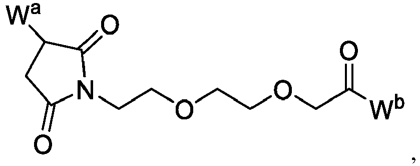 wherein W^{a} and W^{b} are each independently a payload or a targeted molecule capable of binding to a cell surface protein.

67. A conjugate compound or a pharmaceutically acceptable salt thereof, wherein the conjugate compound is 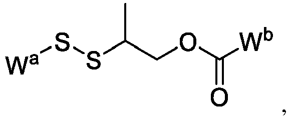 wherein W^{a} and W^{b} are each independently a payload or a targeted molecule capable of binding to a cell surface protein.

68. A proteolysis targeting chimera or a pharmaceutically acceptable salt thereof, wherein the proteolysis targeting chimera compound has the following structures: 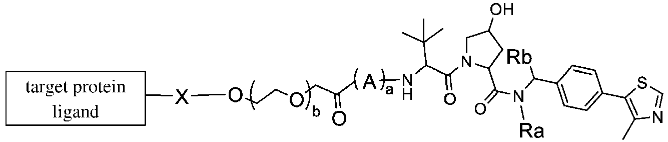 or 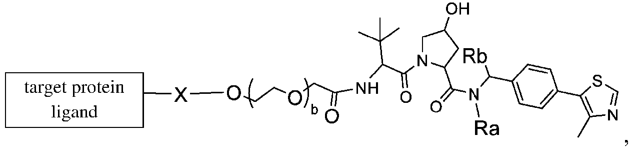 wherein,
Ra and Rb are each independently hydrogen, C₁₋₄ alkyl, NH₂, OH, or halogen;
A is an amino acid residue; a is 0 or 1;
b is an integer of 0-5; 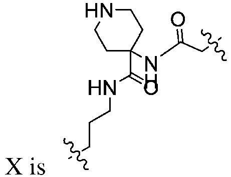 or a bond;
the target protein ligand is

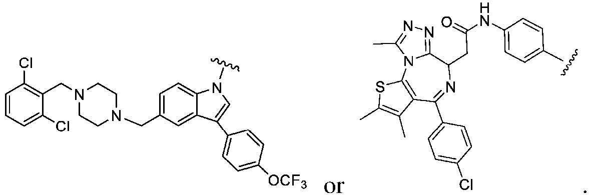

69. The proteolysis targeting chimera or the pharmaceutically acceptable salt thereof according to claim 68, wherein Ra is hydrogen.

70. The proteolysis targeting chimera or the pharmaceutically acceptable salt thereof according to claim 68, wherein Rb is hydrogen.

71. The proteolysis targeting chimera or the pharmaceutically acceptable salt thereof according to claim 68, wherein A is a Lys-formed amino acid residue.

72. The proteolysis targeting chimera or the pharmaceutically acceptable salt thereof according to claim 68, wherein the proteolysis targeting chimera compound has a structure selected from the following group:
